(19) Europäisches Patentamt
European Patent Office
Office européen des brevets

(11) EP 2 216 403 A2

(12) EUROPEAN PATENT APPLICATION

(43) Date of publication:
**11.08.2010 Bulletin 2010/32**

(21) Application number: **10001560.1**

(22) Date of filing: **02.02.2007**

(51) Int Cl.:
***C12N 15/00*** *(2006.01)* ***C12N 9/16*** *(2006.01)*
***C12N 9/18*** *(2006.01)* ***C12N 9/20*** *(2006.01)*
***C12P 21/06*** *(2006.01)*

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**HR MK**

(30) Priority: **02.02.2006 US 764486 P**

(62) Document number(s) of the earlier application(s) in accordance with Art. 76 EPC:
**07763087.9 / 1 987 142**

(71) Applicant: **Verenium Corporation**
**San Diego, CA 92121 (US)**

(72) Inventors:
- **Mathur, Eric J.**
**Carlsbad, CA 92009 (US)**
- **Callen, Walter**
**San Diego, CA 92122 (US)**
- **Fielding, Roderick**
**San Diego, CA 92117 (US)**

(74) Representative: **Wakerley, Helen Rachael**
**Reddie & Grose**
**16 Theobalds Road**
**London**
**WC1X 8PL (GB)**

Remarks:
This application was filed on 16-02-2010 as a divisional application to the application mentioned under INID code 62.

# (54) Esterases and related nucleic acids and methods

(57) The invention provides hydrolases, polynucleotides encoding them, and methods of making and using these polynucleotides and polypeptides. In one aspect, the invention is directed to polypeptides, e.g., enzymes, having a hydrolase activity, e.g., an esterase, acylase, lipase, phospholipase (e.g. phosphplipase A, B, C and D activity, patatin activity, lipid acyl hydrolase (LAH) activity) or protease activity, including thermostable and thermotolerant hydrolase activity, and polynucleotides encoding these enzymes, and making and using these polynucleotides and polypeptides. The hydrolase activities of the polypeptides and peptides of the invention include esterase activity, lipase activity (hydrolysis of lipids), acidolysis reactions (to replace an esterified fatty acid with a free fatty acid), transesterification reactions (exchange of fatty acids between triglycerides), ester synthesis, ester interchange reactions, phospholipase activity and protease activity (hydrolysis of peptide bonds). The polypeptides of the invention can be used in a variety of pharmaceutical, agricultural and industrial contexts, including the manufacture of cosmetics and nutraceuticals. In another aspect, the polypeptides of the invention are used to synthesize enantiomerically pure chiral products.

EP 2 216 403 A2

## Description

TECHNICAL FIELD

This invention relates to molecular and cellular biology and biochemistry. In one aspect, the invention provides hydrolases, polynucleotides encoding them, and methods of making and using these polynucleotides and polypeptides. In one aspect, the invention is directed to polypeptides, e.g., enzymes, having a hydrolase activity, e.g., an esterase, acylase, lipase, phospholipase or protease activity, including thermostable and thermotolerant hydrolase activity, and polynucleotides encoding these enzymes, and making and using these polynucleotides and polypeptides. The hydrolase activities of the polypeptides and peptides of the invention include esterase activity, lipase activity (hydrolysis of lipids), acidolysis reactions (to replace an esterified fatty acid with a free fatty acid), transesterification reactions (exchange of fatty acids between triglycerides), ester synthesis, ester interchange reactions, phospholipase activity (e.g., phospholipase A, B, C and D activity, patatin activity, lipid acyl hydrolase (LAH) activity) and protease activity (hydrolysis of peptide bonds). The polypeptides of the invention can be used in a variety of pharmaceutical, agricultural and industrial contexts, including the manufacture of cosmetics and nutraceuticals. In another aspect, the polypeptides of the invention are used to synthesize enantiomerically pure chiral products.

In one aspect, the polypeptides of the invention are used in the biocatalytic synthesis of structured lipids (lipids that contain a defined set of fatty acids distributed in a defined manner on the glycerol backbone), including cocoa butter alternatives (CBA), lipids containing poly-unsaturated fatty acids (PUFAs), diacylglycerides, e.g., 1,3-diacyl glycerides (DAGs), monoglycerides, e.g., 2-monoglycerides (MAGs) and triacylglycerides (TAGs). In one aspect, the polypeptides of the invention are used to modify oils, such as fish, animal and vegetable oils, and lipids, such as poly-unsaturated fatty acids. The hydrolases of the invention having lipase activity can modify oils by hydrolysis, alcoholysis, esterification, transesterification and/or interesterification. The methods of the invention can use lipases with defined regio-specificity or defined chemoselectivity in biocatalytic synthetic reactions.

Additionally, the polypeptides of the invention can be used in food processing, brewing, bath additives, alcohol production, peptide synthesis, enantioselectivity, hide preparation in the leather industry, waste management and animal degradation, silver recovery in the photographic industry, medical treatment, silk degumming, biofilm degradation, biomass conversion to ethanol, biodefense, antimicrobial agents and disinfectants, personal care and cosmetics, biotech reagents, in increasing starch yield from corn wet milling and pharmaceuticals such as digestive aids and anti-inflammatory (anti-phlogistic) agents.

BACKGROUND

The major industrial applications for hydrolases, e.g., esterases, lipases, phospholipases and proteases, include the detergent industry, where they are employed to decompose fatty materials in laundry stains into easily removable hydrophilic substances; the food and beverage industry where they are used in the manufacture of cheese, the ripening and flavoring of cheese, as antistaling agents for bakery products, and in the production of margarine and other spreads with natural butter flavors; in waste systems; and in the pharmaceutical industry where they are used as digestive aids.

Oils and fats an important renewable raw material for the chemical industry. They are available in large quantities from the processing of oilseeds from plants like rice bran oil, rapeseed (canola), sunflower, olive, palm or soy. Other sources of valuable oils and fats include fish, restaurant waste, and rendered animal fats. These fats and oils are a mixture oftriglycerides or lipids, i.e. fatty acids (FAs) esterified on a glycerol scaffold. Each oil or fat contains a wide variety of different lipid structures, defined by the FA content and their regiochemical distribution on the glycerol backbone. These properties of the individual lipids determine the physical properties of the pure triglyceride. Hence, the triglyceride content of a fat or oil to a large extent determines the physical, chemical and biological properties of the oil. The value of lipids increases greatly as a function of their purity. High purity can be achieved by fractional chromatography or distillation, separating the desired triglyceride from the mixed background of the fat or oil source. However, this is costly and yields are often limited by the low levels at which the triglyceride occurs naturally. In addition, the purity of the product is often compromised by the presence of many structurally and physically or chemically similar triglycerides in the oil.

An alternative to purifying triglycerides or other lipids from a natural source is to synthesize the lipids. The products of such processes are called structured lipids because they contain a defined set of fatty acids distributed in a defined manner on the glycerol backbone. The value of lipids also increases greatly by controlling the fatty acid content and distribution within the lipid. Lipases can be used to affect such control.

Phospholipases are enzymes that hydrolyze the ester bonds of phospholipids. Corresponding to their importance in the metabolism of phospholipids, these enzymes are widespread among prokaryotes and eukaryotes. The phospholipases affect the metabolism, construction and reorganization of biological membranes and are involved in signal cascades. Several types of phospholipases are known which differ in their specificity according to the position of the bond attacked in the phospholipid molecule. Phospholipase A1 (PLA1) removes the 1-position fatty acid to produce free fatty acid and 1-lyso-2-acylphospholipid. Phospholipase A2 (PLA2) removes the 2-position fatty acid to produce free fatty acid and 1-acyl-2-lysophospholipid. PLA1 and PLA2 enzymes can be intra- or extra-cellular, membrane-bound or soluble. Intracellular PLA2 is found in almost every mammalian cell. Phospholipase C (PLC) removes the phosphate moiety to produce 1,2 diacylglycerol and phospho base. Phospholipase D (PLD) produces 1,2-diacylglycerophosphate and base group. PLC and PLD are important in cell function and signaling. Patatins are another type of phospholipase thought to work as a PLA.

In general, enzymes, including hydrolases such as esterases, lipases and proteases, are active over a narrow range of environmental conditions (temperature, pH, etc.), and many are highly specific for particular substrates. The narrow range of activity for a given enzyme limits its applicability and creates a need for a selection of enzymes that (a) have similar activities but are active under different conditions or (b) have different substrates. For instance, an enzyme capable of catalyzing a reaction at 50°C may be so inefficient at 35°C, that its use at the lower temperature will not be feasible. For this reason, laundry detergents generally contain a selection of proteolytic enzymes, allowing the detergent to be used over a broad range of wash temperature and pH. In view of the specificity of enzymes and the growing use of hydrolases in industry, research, and medicine, there is an ongoing need in the art for new enzymes and new enzyme inhibitors.

## SUMMARY

The invention provides polypeptides, for example, enzymes and catalytic antibodies, having a hydrolase activity, e.g., an esterase, acylase, lipase, phospholipase or protease activity, including thermostable and thermotolerant hydrolase activities, and enantiospecific activities, and polynucleotides encoding these polypeptides, including vectors, host cells, transgenic plants and non-human animals, and methods for making and using these polynucleotides and polypeptides.

The invention provides isolated, synthetic or recombinant nucleic acids comprising a nucleic acid sequence having at least about 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or more, or complete (100%) sequence identity to an exemplary nucleic acid of the invention, including SEQ ID NO:1, SEQ ID NO:3, SEQ ID NO:5, SEQ ID NO: 7, SEQ ID NO:9, SEQ ID NO:11, SEQ ID NO:13, SEQ ID NO:15, SEQ ID NO:17, SEQ ID NO:19, SEQ ID NO:21, SEQ ID NO:23, SEQ ID NO:25, SEQ ID NO:27, SEQ ID NO:29, SEQ ID NO:31, SEQ ID NO:33, SEQ ID NO:35, SEQ ID NO: 37, SEQ ID NO:39, SEQ ID NO:41, SEQ ID NO:43, SEQ ID NO:45, SEQ ID NO:47, SEQ ID NO:49, SEQ ID NO:51, SEQ ID NO:53, SEQ ID NO:55, SEQ ID NO:57, SEQ ID NO:59, SEQ ID NO:61, SEQ ID NO:63, SEQ ID NO:65, SEQ ID NO:67, SEQ ID NO:69, SEQ ID NO:71, SEQ ID NO:73, SEQ ID NO:75, SEQ ID NO:77, SEQ ID NO:79, SEQ ID NO: 81, SEQ ID NO:83, SEQ ID NO:85, SEQ ID NO:87, SEQ ID NO:89, SEQ ID NO:91, SEQ ID NO:93, SEQ ID NO:95, SEQ ID NO:97, SEQ ID NO:99, SEQ ID NO:101, SEQ ID NO:103, SEQ ID NO:105, SEQ ID NO:107, SEQ ID NO:109, SEQ ID NO:111, SEQ ID NO:113, SEQ ID NO:115, SEQ ID NO:117, SEQ ID NO:119, SEQ ID NO:121, SEQ ID NO: 123, SEQ ID NO:125, SEQ ID NO:127, SEQ ID NO:129, SEQ ID NO:131, SEQ ID NO:133, SEQ ID NO:135, SEQ ID NO:137, SEQ ID NO:139, SEQ ID NO:141, SEQ ID NO:143, SEQ ID NO:145, SEQ ID NO:147, SEQ ID NO:149, SEQ ID NO:151, SEQ ID NO:153, SEQ ID NO:155, SEQ ID NO:157, SEQ ID NO:159, SEQ ID NO:161, SEQ ID NO:163 and SEQ ID NO:165; over a region of at least about 10, 15, 20, 25, 30, 35, 40, 45, 50, 55, 60, 65, 70, 75, 100, 125, 150, 175, 200, 250, 300, 350, 400, 450, 500, 550, 600, 650, 700, 750, 800, 850, 900, 950, 1000, 1050, 1100, 1150, 1200, 1250, 1300, 1350, 1400, 1450, 1500, 1550 or more, residues, wherein the nucleic acid encodes at least one polypeptide having a hydrolase activity, or the nucleic acid encodes a polypeptide that can generate antibody that specifically binds to an exemplary polypeptide sequence of the invention, including SEQ ID NO:2, SEQ ID NO:4, SEQ ID NO:6, SEQ ID NO:8, SEQ ID NO:10, SEQ ID NO:12, SEQ ID NO:14, SEQ ID NO:16, SEQ ID NO:18, SEQ ID NO:20, SEQ ID NO:22, SEQ ID NO:24, SEQ ID NO:26, SEQ ID NO:28, SEQ ID NO:30, SEQ ID NO:32, SEQ ID NO:34, SEQ ID NO:36, SEQ ID NO: 38, SEQ ID NO:40, SEQ ID NO:42, SEQ ID NO:44, SEQ ID NO:46, SEQ ID NO:48, SEQ ID NO:50, SEQ ID NO:52, SEQ ID NO:54, SEQ ID NO:56, SEQ ID NO:58, SEQ ID NO:60, SEQ ID NO:62, SEQ ID NO:64, SEQ ID NO:66, SEQ ID NO:68, SEQ ID NO:70, SEQ ID NO:72, SEQ ID NO:74, SEQ ID NO:76, SEQ ID NO:78, SEQ ID NO:80, SEQ ID NO: 82, SEQ ID NO:84, SEQ ID NO:86, SEQ ID NO:88, SEQ ID NO:90, SEQ ID NO:92, SEQ ID NO:94, SEQ ID NO:96, SEQ ID NO:98, SEQ ID NO:100, SEQ ID NO:102, SEQ ID NO:104, SEQ ID NO:106, SEQ ID NO:108, SEQ ID NO:110, SEQ ID NO:112, SEQ ID NO:114, SEQ ID NO:116, SEQ ID NO:118, SEQ ID NO:120, SEQ ID NO:122, SEQ ID NO: 124, SEQ ID NO:126, SEQ ID NO:128, SEQ ID NO:130, SEQ ID NO:132, SEQ ID NO:134, SEQ ID NO:136, SEQ ID NO:138, SEQ ID NO:140, SEQ ID NO:142, SEQ ID NO:143, SEQ ID NO:146, SEQ ID NO:148, SEQ ID NO:150, SEQ ID NO:152, SEQ ID NO:154, SEQ ID NO:156, SEQ ID NO:158, SEQ ID NO:160, SEQ ID NO:162, SEQ ID NO:164 and/or SEQ ID NO:166; or a polypeptide of the invention can generate a humoral immune response to make an anti-hydrolase antibody. In alternative embodiments the hydrolase activity comprises an esterase, acylase, lipase, phospholipase or protease activity. The sequence identities can be determined by analysis with a sequence comparison algorithm or by a visual inspection. Exemplary nucleic acids of the invention include isolated, synthetic or recombinant nucleic acids comprising a nucleic acid sequence as set forth in SEQ ID NO:1, SEQ ID NO:3, SEQ ID NO:5, SEQ ID NO:7, SEQ ID NO:9, SEQ ID NO:11, SEQ ID NO:13, SEQ ID NO:15, SEQ ID NO:17, SEQ ID NO:19, SEQ ID NO:21, SEQ ID NO:23, SEQ ID NO:25, SEQ ID NO:27, SEQ ID NO:29, SEQ ID NO:31, SEQ ID NO:33, SEQ ID NO:35, SEQ ID NO: 37, SEQ ID NO:39, SEQ ID NO:41, SEQ ID NO:43, SEQ ID NO:45, SEQ ID NO:47, SEQ ID NO:49, SEQ ID NO:51, SEQ ID NO:53, SEQ ID NO:55, SEQ ID NO:57, SEQ ID NO:59, SEQ ID NO:61, SEQ ID NO:63, SEQ ID NO:65, SEQ ID NO:67, SEQ ID NO:69, SEQ ID NO:71, SEQ ID NO:73, SEQ ID NO:75, SEQ ID NO:77, SEQ ID NO:79, SEQ ID NO: 81, SEQ ID NO:83, SEQ ID NO:85, SEQ ID NO:87, SEQ ID NO:89, SEQ ID NO:91, SEQ ID NO:93, SEQ ID NO:95, SEQ ID NO:97, SEQ ID NO:99, SEQ ID NO:101, SEQ ID NO:103, SEQ ID NO:105, SEQ ID NO:107, SEQ ID NO:109, SEQ ID NO:111, SEQ ID NO:113, SEQ ID NO:115, SEQ ID NO:117, SEQ ID NO:119, SEQ ID NO:121, SEQ ID NO: 123, SEQ ID NO:125, SEQ ID NO:127, SEQ ID NO:129, SEQ ID NO:131, SEQ ID NO:133, SEQ ID NO:135, SEQ ID NO:137, SEQ ID NO:139, SEQ ID NO:141, SEQ ID NO:143, SEQ ID NO:145, SEQ ID NO:147, SEQ ID NO:149, SEQ ID NO:151, SEQ ID NO:153, SEQ ID NO:155, SEQ ID NO:157, SEQ ID NO:159, SEQ ID NO:161, SEQ ID NO:163 and/or SEQ ID NO:165, and subsequences thereof, e.g., at least about 10, 15, 20, 25, 30, 35, 40, 45, 50, 75, 100, 150, 200, 250, 300, 350, 400, 450, 500, 550, 600, 650, 700, 750, 800, 850, 900, 950, 1000, 1050, 1100, 1150, 1200, 1250, 1300, 1350, 1400, 1450, 1500 or more residues in length, or over the full length of a gene or transcript. All of these polynucleotides (nucleic acids) and oligonucleotides are considered polynucleotides and oligonucleotides of the invention.

Exemplary nucleic acids of the invention also include isolated, synthetic or recombinant nucleic acids encoding a polypeptide encoded by a nucleic acid of the invention, e.g., a sequence as set forth in SEQ ID NO:2 (encoded, e.g., by SEQ ID NO:1), SEQ ID NO:4 (encoded, e.g., by SEQ ID NO:3), SEQ ID NO:6 (encoded, e.g., by SEQ ID NO:5), SEQ ID NO:8 (encoded, e.g., by SEQ ID NO:7), SEQ ID NO:10 (encoded, e.g., by SEQ ID NO:9), SEQ ID NO:12 (encoded, e.g., by SEQ ID NO:11), SEQ ID NO:14 (encoded, e.g., by SEQ ID NO:13), SEQ ID NO:16 (encoded, e.g., by SEQ ID NO:15), SEQ ID NO:18 (encoded, e.g., by SEQ ID NO:17), SEQ ID NO:20 (encoded, e.g., by SEQ ID NO: 19), SEQ ID NO:22 (encoded, e.g., by SEQ ID NO:21), SEQ ID NO:24 (encoded, e.g., by SEQ ID NO:23), SEQ ID NO: 26 (encoded, e.g., by SEQ ID NO:25), SEQ ID NO:28 (encoded, e.g., by SEQ ID NO:27), SEQ ID NO:30 (encoded, e.g., by SEQ ID NO:29), SEQ ID NO:32 (encoded, e.g., by SEQ ID NO:11), SEQ ID NO:34 (encoded, e.g., by SEQ ID NO:33), SEQ ID NO:36 (encoded, e.g., by SEQ ID NO:35), SEQ ID NO:38 (encoded, e.g., by SEQ ID NO:37), SEQ ID NO:40 (encoded, e.g., by SEQ ID NO:39), SEQ ID NO:42 (encoded, e.g., by SEQ ID NO:41), SEQ ID NO:44 (encoded, e.g., by SEQ ID NO:43), SEQ ID NO:46 (encoded, e.g., by SEQ ID NO:45), SEQ ID NO:48 (encoded, e.g., by SEQ ID NO:47), SEQ ID NO:50 (encoded, e.g., by SEQ ID NO:49), SEQ ID NO:52 (encoded, e.g., by SEQ ID NO:51), SEQ ID NO:54 (encoded, e.g., by SEQ ID NO:53), SEQ ID NO:56 (encoded, e.g., by SEQ ID NO:55), SEQ ID NO:58 (encoded, e.g., by SEQ ID NO:57), SEQ ID NO:60 (encoded, e.g., by SEQ ID NO:59), SEQ ID NO:62 (encoded, e.g., by SEQ ID NO:61), SEQ ID NO:64 (encoded, e.g., by SEQ ID NO:63), SEQ ID NO:66 (encoded, e.g., by SEQ ID NO:65), SEQ ID NO:68 (encoded, e.g., by SEQ ID NO:67), SEQ ID NO:70 (encoded, e.g., by SEQ ID NO:69), SEQ ID NO:72 (encoded, e.g., by SEQ ID NO:71), SEQ ID NO:74 (encoded, e.g., by SEQ ID NO:73), SEQ ID NO:76 (encoded, e.g., by SEQ ID NO:75), SEQ ID NO:78 (encoded, e.g., by SEQ ID NO:77), SEQ ID NO:80 (encoded, e.g., by SEQ ID NO:79), SEQ ID NO:82 (encoded, e.g., by SEQ ID NO:81), SEQ ID NO:84 (encoded, e.g., by SEQ ID NO:83), SEQ ID NO:86 (encoded, e.g., by SEQ ID NO:85), SEQ ID NO:88 (encoded, e.g., by SEQ ID NO:87), SEQ ID NO:90 (encoded, e.g., by SEQ ID NO:89), SEQ ID NO:92 (encoded, e.g., by SEQ ID NO:91), SEQ ID NO:94 (encoded, e.g., by SEQ ID NO:93), SEQ ID NO:96 (encoded, e.g., by SEQ ID NO:95), SEQ ID NO:98 (encoded, e.g., by SEQ ID NO:97) or SEQ ID NO:100 (encoded, e.g., by SEQ ID NO:99), etc. through to SEQ ID NO:166 (encoded, e.g., by SEQ ID NO:165) and subsequences thereof and variants thereof. In one aspect, the polypeptide has a hydrolase activity, e.g., an esterase, acylase, lipase, phospholipase or protease activity. In one aspect, the hydrolase activity is a regioselective and/or chemoselective activity.

In one aspect, the invention also provides hydrolase-encoding nucleic acids with a common novelty in that they are derived from mixed cultures. The invention provides hydrolase-encoding nucleic acids isolated from mixed cultures comprising a nucleic acid of the invention, e.g., a nucleic acid having a sequence at least about 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or more, or complete (100%) sequence identity to an exemplary nucleic acid of the invention over a region of at least about 10, 20, 30, 40, 50, 60, 70, 75, 100, 125, 150, 200, 250, 300, 350, 400, 450, 500, 550, 600, 650, 700, 750, 800, 850, 900, 950, 1000, 1050, 1100, 1150, 1200, 1250, 1300, 1350, 1400, 1450, 1500, 1550 or more, residues, wherein the nucleic acid encodes at least one polypeptide having a hydrolase activity, and the sequence identities are determined by analysis with a sequence comparison algorithm or by a visual inspection. In one aspect, the invention provides hydrolase-encoding nucleic acids isolated from mixed cultures comprising a nucleic acid of the invention.

In one aspect, the invention also provides hydrolase-encoding nucleic acids with a common novelty in that they are derived from environmental sources, e.g., mixed environmental sources. In one aspect, the invention provides hydrolase-encoding nucleic acids isolated from environmental sources, e.g., mixed environmental sources, comprising a nucleic acid sequence having at least about 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or more, or complete (100%) sequence identity to an exemplary nucleic acid of the invention over a region of at least about 10, 15, 20, 25, 30, 35, 40, 45, 50, 55, 60, 65, 70, 75, 100, 125, 150, 175, 200, 250, 300, 350, 400, 450, 500, 550, 600, 650, 700, 750, 800, 850, 900, 950, 1000, 1050, 1100, 1150, 1200, 1250, 1300, 1350, 1400, 1450, 1500, 1550 or more, residues, wherein the nucleic acid encodes at least one polypeptide having a hydrolase activity, and the sequence identities are determined by analysis with a sequence comparison algorithm or by a visual inspection. In one aspect, the invention provides hydrolase-encoding nucleic acids isolated from environmental sources, e.g., mixed environmental sources, comprising a nucleic acid of the invention.

In one aspect, the sequence comparison algorithm is a BLAST version 2.2.2 algorithm where a filtering setting is set to blastall -p blastp -d "nr pataa" -F F, and all other options are set to default.

Another aspect of the invention is an isolated, synthetic or recombinant nucleic acid including at least 10 consecutive bases of a nucleic acid sequence of the invention, sequences substantially identical thereto, and the sequences complementary thereto.

In one aspect, the lipase activity comprises hydrolyzing a triacylglycerol (TAG), a diacylglycerol (DAG) or a monoacylglycerol (MAG). The lipase activity can comprise hydrolyzing a triacylglycerol to a diacylglycerol and a free fatty acid, or, hydrolyzing a triacylglycerol to a monoacylglycerol and free fatty acids, or, hydrolyzing a diacylglycerol to a monoacylglycerol and free fatty acids, or, hydrolyzing a monoacylglycerol to a free fatty acid and a glycerol. The lipase activity can comprise synthesizing a tryacylglycerol from a diacylglycerol or a monoacylglycerol and free fatty acids. The lipase activity can comprise synthesizing 1,3-dipalmitoyl-2-oleoylglycerol (POP), 1,3-distearoyl-2-oleoylglycerol (SOS), 1-palmitoyl-2-oleoyl-3-stearoylglycerol (POS) or 1-oleoyl-2,3-dimyristoylglycerol (OMM), long chain polyunsaturated fatty acids, arachidonic acid, docosahexaenoic acid (DHA) or eicosapentaenoic acid (EPA). The lipase activity can be triacylglycerol (TAG), diacylglycerol (DAG) or monoacylglycerol (MAG) position - specific. The lipase activity can be Sn2-specific, Sn1- or Sn3-specific. The lipase activity can be fatty acid specific. The lipase activity can comprise modifying oils by hydrolysis, alcoholysis, esterification, transesterification or interesterification. The lipase activity can be regio-specific or chemoselective. The lipase activity can comprise synthesis of enantiomerically pure chiral products. The lipase activity can comprise synthesis ofumbelliferyl fatty acid (FA) esters.

In one aspect, the isolated, synthetic or recombinant nucleic acid encodes a polypeptide having a hydrolase activity, e.g., an esterase, acylase, lipase, phospholipase or protease activity, which is thermostable. The polypeptide can retain activity under conditions comprising a temperature range of between about 37°C to about 95°C; between about 55°C to about 85°C, between about 70°C to about 95°C, or, between about 90°C to about 95°C.

In another aspect, the isolated, synthetic or recombinant nucleic acid encodes a polypeptide having a hydrolase activity, e.g., an esterase, acylase, lipase, phospholipase or protease activity, which is thermotolerant. The polypeptide can retain activity after exposure to a temperature in the range from greater than 37°C to about 95°C or anywhere in the range from greater than 55°C to about 85°C. In one aspect, the polypeptide retains activity after exposure to a temperature in the range from greater than 90°C to about 95°C at pH 4.5.

The invention provides isolated, synthetic or recombinant nucleic acids comprising a sequence that hybridizes under stringent conditions to a nucleic acid of the invention, e.g., an exemplary nucleic acid of the invention comprising the sequence of SEQ ID NO:1, SEQ ID NO:3, SEQ ID NO:5, SEQ ID NO:7, SEQ ID NO:9, SEQ ID NO:11, SEQ ID NO:13, SEQ ID NO:15, SEQ ID NO:17, SEQ ID NO:19, SEQ ID NO:21, SEQ ID NO:23, SEQ ID NO:25, SEQ ID NO:27, SEQ ID NO:29, SEQ ID NO:31, SEQ ID NO:33, SEQ ID NO:35, SEQ ID NO:37, SEQ ID NO:39, SEQ ID NO:41, SEQ ID NO:43, SEQ ID NO:45, SEQ ID NO:47, SEQ ID NO:49, SEQ ID NO:51, SEQ ID NO:53, SEQ ID NO:55, SEQ ID NO:57, SEQ ID NO:59, SEQ ID NO:61, SEQ ID NO:63, SEQ ID NO:65, SEQ ID NO:67, SEQ ID NO:69, SEQ ID NO:71, SEQ ID NO:73, SEQ ID NO:75, SEQ ID NO:77, SEQ ID NO:79, SEQ ID NO:81, SEQ ID NO:83, SEQ ID NO:85, SEQ ID NO:87, SEQ ID NO:89, SEQ ID NO:91, SEQ ID NO:93, SEQ ID NO:95, SEQ ID NO:97, SEQ ID NO:99, SEQ ID NO:101, SEQ ID NO:103, SEQ ID NO:105, SEQ ID NO:107, SEQ ID NO:109, SEQ ID NO:111, SEQ ID NO:113, SEQ ID NO:115, SEQ ID NO:117, SEQ ID NO:119, SEQ ID NO:121, SEQ ID NO:123, SEQ ID NO:125, SEQ ID NO: 127, SEQ ID NO:129, SEQ ID NO:131, SEQ ID NO:133, SEQ ID NO:135, SEQ ID NO:137, SEQ ID NO:139, SEQ ID NO:141, SEQ ID NO:143, SEQ ID NO:145, SEQ ID NO:147, SEQ ID NO:149, SEQ ID NO:151, SEQ ID NO:153, SEQ ID NO:155, SEQ ID NO:157, SEQ ID NO:159, SEQ ID NO:161, SEQ ID NO:163 and/or SEQ ID NO:165, or fragments or subsequences thereof. In one aspect, the nucleic acid encodes a polypeptide having a hydrolase activity, e.g., an esterase, acylase, lipase, phospholipase or protease activity. The nucleic acid can be at least about 10, 15, 20, 25, 30, 35, 40, 45, 50, 55, 60, 65, 70, 75, 100, 125, 150, 175, 200, 250, 300, 350, 400, 450, 500, 550, 600, 650, 700, 750, 800, 850, 900, 950, 1000, 1050, 1100, 1150, 1200, 1250, 1300, 1350, 1400, 1450, 1500 or more residues in length or the full length of the gene or transcript. In one aspect, the stringent conditions include a wash step comprising a wash in 0.2X SSC at a temperature of about 65°C for about 15 minutes.

The invention provides a nucleic acid probe, e.g., a probe for identifying a nucleic acid encoding a polypeptide having a hydrolase activity, e.g., an esterase, acylase, lipase, phospholipase or protease activity, wherein the probe comprises at least about 10, 15, 20, 25, 30, 35, 40, 45, 50, 55, 60, 65, 70, 75, 80, 85, 90, 95, 100, 150, 200, 250, 300, 350, 400, 450, 500, 550, 600, 650, 700, 750, 800, 850, 900, 950, 1000 or more, consecutive bases of a sequence of the invention, or fragments or subsequences thereof, wherein the probe identifies the nucleic acid by binding or hybridization. The probe can comprise an oligonucleotide comprising at least about 10 to 50, about 20 to 60, about 30 to 70, about 40 to 80, or about 60 to 100 consecutive bases of a sequence comprising a sequence of the invention, or fragments or subsequences thereof. The probe can comprise an oligonucleotide comprising at least about 10 to 50, about 20 to 60, about 30 to 70, about 40 to 80, or about 60 to 100 consecutive bases of a nucleic acid sequence of the invention, or a subsequence thereof.

The invention provides an amplification primer sequence pair for amplifying a nucleic acid encoding a polypeptide having a hydrolase activity, e.g., an esterase, acylase, lipase, phospholipase or protease activity, wherein the primer pair is capable of amplifying a nucleic acid comprising a sequence of the invention, or fragments or subsequences thereof. One or each member of the amplification primer sequence pair can comprise an oligonucleotide comprising at least about 10 to 50 consecutive bases of the sequence.

The invention provides methods of amplifying a nucleic acid encoding a polypeptide having a hydrolase activity, e.g., an esterase, acylase, lipase, phospholipase or protease activity, comprising amplification of a template nucleic acid with an amplification primer sequence pair capable of amplifying a nucleic acid sequence of the invention, or fragments or subsequences thereof.

The invention provides expression cassettes comprising a nucleic acid of the invention or a subsequence thereof. In one aspect, the expression cassette can comprise the nucleic acid that is operably linked to a promoter. The promoter can be a viral, bacterial, mammalian or plant promoter. In one aspect, the plant promoter can be a potato, rice, corn, wheat, tobacco or barley promoter. The promoter can be a constitutive promoter. The constitutive promoter can comprise CaMV35S. In another aspect, the promoter can be an inducible promoter. In one aspect, the promoter can be a tissue-specific promoter or an environmentally regulated or a developmentally regulated promoter. Thus, the promoter can be, e.g., a seed-specific, a leaf-specific, a root-specific, a stem-specific or an abscission-induced promoter. In one aspect, the expression cassette can further comprise a plant or plant virus expression vector.

The invention provides cloning vehicles comprising an expression cassette (e.g., a vector) of the invention or a nucleic acid of the invention. The cloning vehicle can be a viral vector, a plasmid, a phage, a phagemid, a cosmid, a fosmid, a bacteriophage or an artificial chromosome. The viral vector can comprise an adenovirus vector, a retroviral vector or an adeno-associated viral vector. The cloning vehicle can comprise a bacterial artificial chromosome (BAC), a plasmid, a bacteriophage P1-derived vector (PAC), a yeast artificial chromosome (YAC), or a mammalian artificial chromosome (MAC).

The invention provides transformed cell comprising a nucleic acid of the invention or an expression cassette (e.g., a vector) of the invention, or a cloning vehicle of the invention. In one aspect, the transformed cell can be a bacterial cell, a mammalian cell, a fungal cell, a yeast cell, an insect cell or a plant cell. In one aspect, the plant cell can be a potato, wheat, rice, corn, tobacco or barley cell.

The invention provides transgenic non-human animals comprising a nucleic acid of the invention or an expression cassette (e.g., a vector) of the invention. In one aspect, the animal is a mouse.

The invention provides transgenic plants comprising a nucleic acid of the invention or an expression cassette (e.g., a vector) of the invention. The transgenic plant can be a corn plant, a potato plant, a tomato plant, a wheat plant, an oilseed plant, a rapeseed plant, a soybean plant, a rice plant, a barley plant or a tobacco plant

The invention provides transgenic seeds comprising a nucleic acid of the invention or an expression cassette (e.g., a vector) of the invention. The transgenic seed can be rice, a corn seed, a wheat kernel, an oilseed, a rapeseed, a soybean seed, a palm kernel, a sunflower seed, a sesame seed, a peanut or a tobacco plant seed.

The invention provides an antisense oligonucleotide comprising a nucleic acid sequence complementary to or capable of hybridizing under stringent conditions to a nucleic acid of the invention. The invention provides methods of inhibiting the translation of a hydrolase message in a cell comprising administering to the cell or expressing in the cell an antisense oligonucleotide comprising a nucleic acid sequence complementary to or capable of hybridizing under stringent conditions to a nucleic acid of the invention.

The invention provides an isolated, synthetic or recombinant polypeptide comprising an amino acid sequence having at least about 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91 %, 92%, 93%, 94%, 95%, 96%, 97%, 98%, 99%, or more or complete (100%) sequence identity to an exemplary polypeptide or peptide of the invention over a region of at least about 10, 15, 20, 25, 30, 35, 40, 45, 50, 55, 60, 65, 70, 75, 100, 125, 150, 175, 200, 250, 300, 350, 400, 450, 500, 550, 600, 650, 700, 750, 800, 850, 900, 950, 1000, 1050, 1100, 1150, 1200, 1250, 1300, 1350, 1400, 1450, 1500, 1550 or more residues, or over the full length of the polypeptide. In one aspect, the sequence identities are determined by analysis with a sequence comparison algorithm or by a visual inspection. Exemplary polypeptide or peptide sequences of the invention include SEQ ID NO: 2, SEQ ID NO:4, SEQ ID NO:6, SEQ ID NO:8, SEQ ID NO:10, SEQ ID NO:12, SEQ ID NO:14, SEQ ID NO:16, SEQ ID NO:18, SEQ ID NO:20, SEQ ID NO:22, SEQ ID NO:24, SEQ ID NO:26, SEQ ID NO:28, SEQ ID NO:30, SEQ ID NO: 32, SEQ ID NO:34, SEQ ID NO:36, SEQ ID NO:38, SEQ ID NO:40, SEQ ID NO:42, SEQ ID NO:44, SEQ ID NO:46, SEQ ID NO:48, SEQ ID NO:50, SEQ ID NO:52, SEQ ID NO:54, SEQ ID NO:56, SEQ ID NO:58, SEQ ID NO:60, SEQ ID NO:62, SEQ ID NO:64, SEQ ID NO:66, SEQ ID NO:68, SEQ ID NO:70, SEQ ID NO:72, SEQ ID NO:74, SEQ ID NO: 76, SEQ ID NO:78, SEQ ID NO:80, SEQ ID NO:82, SEQ ID NO:84, SEQ ID NO:86, SEQ ID NO:88, SEQ ID NO:90, SEQ ID NO:92, SEQ ID NO:94, SEQ ID NO:96, SEQ ID NO:98, SEQ ID NO:100, SEQ ID N0:102, SEQ ID NO:104, SEQ ID NO:106, SEQ ID NO:108, SEQ ID NO:110, SEQ ID NO:112, SEQ ID NO:114, SEQ ID NO:116, SEQ ID NO: 118, SEQ ID NO:120, SEQ ID NO:122, SEQ ID NO:124, SEQ ID NO:126, SEQ ID NO:128, SEQ ID NO:130, SEQ ID NO:132, SEQ ID NO:134, SEQ ID NO:136, SEQ ID NO:138, SEQ ID NO:140, SEQ ID NO:142, SEQ ID NO:143, SEQ ID NO:146, SEQ ID NO:148, SEQ ID NO:150, SEQ ID NO:152, SEQ ID NO:154, SEQ ID NO:156, SEQ ID NO:158, SEQ ID NO:160, SEQ ID NO:162, SEQ ID NO:164 and/or SEQ ID NO:166, and subsequences thereof and variants thereof, e.g., at least about 10, 15, 20, 25, 30, 35, 40, 45, 50, 75, 100, 150, 200, 250, 300, 350, 400, 450, 500, 550, 600, 650, 700, 750, 800, 850, 900, 950,1000, 1050, 1100, 1150, 1200, 1250, 1300, 1350, 1400, 1450, 1500 or more residues in length, or over the full length of an enzyme. Exemplary polypeptide or peptide sequences of the invention include sequences encoded by a nucleic acid of the invention. Polypeptides or peptides of the invention can include polypeptides or peptides specifically bound by an antibody of the invention. In one aspect, a polypeptide of the invention has at least one hydrolase activity, e.g., an esterase, acylase, lipase, phospholipase or protease activity. In one aspect, a polypeptide of the invention can generate antibody that specifically binds to an exemplary polypeptide or peptide sequences of the invention: SEQ ID NO:2, SEQ ID NO:4, SEQ ID NO:6, SEQ ID NO:8, SEQ ID NO:10, SEQ ID NO:12, SEQ ID NO:14, SEQ ID NO:16, SEQ ID NO:18, SEQ ID NO:20, SEQ ID NO:22, SEQ ID NO:24, SEQ ID NO:26, SEQ ID NO:28, SEQ ID NO:30, SEQ ID NO:32, SEQ ID NO:34, SEQ ID NO:36, SEQ ID NO:38, SEQ ID NO:40, SEQ ID NO:42, SEQ ID NO: 44, SEQ ID NO:46, SEQ ID NO:48, SEQ ID NO:50, SEQ ID NO:52, SEQ ID NO:54, SEQ ID NO:56, SEQ ID NO:58, SEQ ID NO:60, SEQ ID NO:62, SEQ ID NO:64, SEQ ID NO:66, SEQ ID NO:68, SEQ ID NO:70, SEQ ID NO:72, SEQ ID NO:74, SEQ ID NO:76, SEQ ID NO:78, SEQ ID NO:80, SEQ ID NO:82, SEQ ID NO:84, SEQ ID NO:86, SEQ ID NO: 88, SEQ ID NO:90, SEQ ID NO:92, SEQ ID NO:94, SEQ ID NO:96, SEQ ID NO:98, SEQ ID NO:100, SEQ ID NO: 102, SEQ ID NO: 104, SEQ ID NO: 106, SEQ ID NO:108, SEQ ID NO:110, SEQ ID NO:112, SEQ ID NO:114, SEQ ID NO: 116, SEQ ID NO:118, SEQ ID NO:120, SEQ ID NO:122, SEQ ID NO:124, SEQ ID NO:126, SEQ ID NO:128, SEQ ID NO:130, SEQ ID NO:132, SEQ ID NO:134, SEQ ID NO:136, SEQ ID NO:138, SEQ ID NO:140, SEQ ID NO:142, SEQ ID NO:143, SEQ ID NO:146, SEQ ID NO:148, SEQ ID NO:150, SEQ ID NO:152, SEQ ID NO:154, SEQ ID NO:156, SEQ ID NO:158, SEQ ID NO:160, SEQ ID NO:162, SEQ ID NO:164 and/or SEQ ID NO:166. In one aspect, the activity is a regioselective and/or chemoselective activity. All of these polypeptides and peptides are considered polypeptides and peptides of the invention.

Another aspect of the invention is an isolated, synthetic or recombinant polypeptide or peptide including at least 10 consecutive bases of a polypeptide or peptide sequence of the invention, sequences substantially identical thereto, and the sequences complementary thereto.

In one aspect, the lipase activity comprises hydrolyzing a triacylglycerol (TAG), a diacylglycerol (DAG) or a monoacylglycerol (MAG). The lipase activity can comprise hydrolyzing a triacylglycerol to a diacylglycerol and a free fatty acid, or, hydrolyzing a triacylglycerol to a monoacylglycerol and free fatty acids, or, hydrolyzing a diacylglycerol to a monoacylglycerol and free fatty acids, or, hydrolyzing a monoacylglycerol to a free fatty acid and a glycerol. The lipase activity can comprise synthesizing a tryacylglycerol from a diacylglycerol or a monoacylglycerol and free fatty acids. The lipase activity can comprise synthesizing 1,3-dipalmitoyl-2-oleoylglycerol (POP), 1,3-distearoyl-2-oleoylglycerol (SOS), 1-palmitoyl-2-oleoyl-3-stearoylglycerol (POS) or 1-oleoyl-2,3-dimyristoylglycerol (OMM), long chain polyunsaturated fatty acids, arachidonic acid, docosahexaenoic acid (DHA) or eicosapentaenoic acid (EPA). The lipase activity can be triacylglycerol (TAG), diacylglycerol (DAG) or monoacylglycerol (MAG) position - specific. The lipase activity can be Sn2-specific, Sn1- or Sn3-specific. The lipase activity can be fatty acid specific. The lipase activity can comprise modifying oils by hydrolysis, alcoholysis, esterification, transesterification or interesterification. The lipase activity can be regio-specific or chemoselective. The lipase activity can comprise synthesis of enantiomerically pure chiral products. The lipase activity can comprise synthesis of umbelliferyl fatty acid (FA) esters.

In one aspect, the hydrolase activity can be thermostable. The polypeptide can retain a hydrolase activity under conditions comprising a temperature range of between about 37°C to about 95°C, between about 55°C to about 85°C, between about 70°C to about 95°C, or between about 90°C to about 95°C. In another aspect, the hydrolase activity can be thermotolerant. The polypeptide can retain a hydrolase activity after exposure to a temperature in the range from greater than 37°C to about 95°C, or in the range from greater than 55°C to about 85°C. In one aspect, the polypeptide can retain a hydrolase activity after exposure to a temperature in the range from greater than 90°C to about 95°C at pH 4.5.

In one aspect, the isolated, synthetic or recombinant polypeptide can comprise the polypeptide of the invention that lacks a signal sequence. In one aspect, the isolated, synthetic or recombinant polypeptide can comprise the polypeptide of the invention comprising a heterologous signal sequence (signal peptide), such as a heterologous hydrolase or non-hydrolase signal sequence. In one aspect, the invention provides chimeric proteins comprising a first domain comprising a signal sequence of the invention and at least a second domain.

In one aspect, the isolated, synthetic or recombinant polypeptide can be (comprise) a fusion protein, and can comprise any heterologous moiety (domain) or sequence. The second domain (or moiety) can comprise an enzyme, a tag, an epitope, a binding domain and the like. The enzyme can be a hydrolase (e.g., a hydrolase of the invention, or, another hydrolase).

In one aspect, the hydrolase activity comprises a specific activity at about 37°C in the range from about 100 to about 1000 units per milligram of protein. In another aspect, the hydrolase activity comprises a specific activity from about 500 to about 750 units per milligram of protein. Alternatively, the hydrolase activity comprises a specific activity at 37°C in the range from about 500 to about 1200 units per milligram of protein. In one aspect, the hydrolase activity comprises a specific activity at 37°C in the range from about 750 to about 1000 units per milligram of protein. In another aspect, the thermotolerance comprises retention of at least half of the specific activity of the hydrolase at 37°C after being heated to the elevated temperature. Alternatively, the thermotolerance can comprise retention of specific activity at 37°C in the range from about 500 to about 1200 units per milligram of protein after being heated to the elevated temperature.

The invention provides the isolated, synthetic or recombinant polypeptide of the invention, wherein the polypeptide comprises at least one glycosylation site. In one aspect, glycosylation can be an N-linked glycosylation. In one aspect, the polypeptide can be glycosylated after being expressed in a *P. pastoris* or a *S. pombe*.

In one aspect, the polypeptide can retain a hydrolase activity under conditions comprising about pH 6.5, pH 6, pH 5.5, pH 5, pH 4.5 or pH 4. In another aspect, the polypeptide can retain a hydrolase activity under conditions comprising about pH 7, pH 7.5 pH 8.0, pH 8.5, pH 9, pH 9.5, pH 10, pH 10.5 or pH 11.

The invention provides protein preparations comprising a polypeptide of the invention, wherein the protein preparation comprises a liquid, a solid or a gel.

The invention provides heterodimers comprising a polypeptide of the invention and a second domain. In one aspect, the second domain can be a polypeptide and the heterodimer can be a fusion protein. In one aspect, the second domain can be an epitope or a tag. In one aspect, the invention provides homodimers comprising a polypeptide of the invention.

The invention provides immobilized polypeptides having a hydrolase activity, wherein the polypeptide comprises a polypeptide of the invention, a polypeptide encoded by a nucleic acid of the invention, or a polypeptide comprising a polypeptide of the invention and a second domain. In one aspect, the polypeptide can be immobilized on a cell, a metal, a resin, a polymer, a ceramic, a glass, a microelectrode, a graphitic particle, a bead, a gel, a plate, an array or a capillary tube.

The invention provides arrays comprising an immobilized nucleic acid of the invention. The invention provides arrays comprising an antibody of the invention.

The invention provides isolated, synthetic or recombinant antibodies that specifically bind to a polypeptide of the invention or to a polypeptide encoded by a nucleic acid of the invention. The antibody can be a monoclonal or a polyclonal antibody. The invention provides hybridomas comprising an antibody of the invention, e.g., an antibody that specifically binds to a polypeptide of the invention or to a polypeptide encoded by a nucleic acid of the invention.

The invention provides food supplements for an animal comprising a polypeptide of the invention, e.g., a polypeptide encoded by the nucleic acid of the invention. In one aspect, the polypeptide in the food supplement can be glycosylated. The invention provides edible enzyme delivery matrices comprising a polypeptide of the invention, e.g., a polypeptide encoded by the nucleic acid of the invention. In one aspect, the delivery matrix comprises a pellet. In one aspect, the polypeptide can be glycosylated. In one aspect, the hydrolase activity is thermotolerant. In another aspect, the hydrolase activity is thermostable.

The invention provides method of isolating or identifying a polypeptide having a hydrolase activity comprising the steps of: (a) providing an antibody of the invention; (b) providing a sample comprising polypeptides; and (c) contacting the sample of step (b) with the antibody of step (a) under conditions wherein the antibody can specifically bind to the polypeptide, thereby isolating or identifying a polypeptide having a hydrolase activity.

The invention provides methods of making an anti-hydrolase antibody comprising administering to a non-human animal a nucleic acid of the invention or a polypeptide of the invention or subsequences thereof in an amount sufficient to generate a humoral immune response, thereby making an anti-hydrolase antibody. The invention provides methods of making an anti-hydrolase immune comprising administering to a non-human animal a nucleic acid of the invention or a polypeptide of the invention or subsequences thereof in an amount sufficient to generate an immune response.

The invention provides methods of producing a recombinant polypeptide comprising the steps of: (a) providing a nucleic acid of the invention operably linked to a promoter; and (b) expressing the nucleic acid of step (a) under conditions that allow expression of the polypeptide, thereby producing a recombinant polypeptide. In one aspect, the method can further comprise transforming a host cell with the nucleic acid of step (a) followed by expressing the nucleic acid of step (a), thereby producing a recombinant polypeptide in a transformed cell.

The invention provides methods for identifying a polypeptide having a hydrolase activity comprising the following steps: (a) providing a polypeptide of the invention; or a polypeptide encoded by a nucleic acid of the invention; (b) providing a hydrolase substrate; and (c) contacting the polypeptide or a fragment or variant thereof of step (a) with the substrate of step (b) and detecting a decrease in the amount of substrate or an increase in the amount of a reaction product, wherein a decrease in the amount of the substrate or an increase in the amount of the reaction product detects a polypeptide having a hydrolase activity. In alternative aspects, the substrate can be a poly-unsaturated fatty acid (PUFA), a diacylglyceride, e.g., a 1,3-diacyl glyceride (DAG), a monoglyceride, e.g., 2-monoglyceride (MAG) or a triacylglyceride (TAG).

The invention provides methods for identifying a hydrolase substrate comprising the following steps: (a) providing a polypeptide of the invention; or a polypeptide encoded by a nucleic acid of the invention; (b) providing a test substrate; and (c) contacting the polypeptide of step (a) with the test substrate of step (b) and detecting a decrease in the amount of substrate or an increase in the amount of reaction product, wherein a decrease in the amount of the substrate or an increase in the amount of a reaction product identifies the test substrate as a hydrolase substrate.

The invention provides methods of determining whether a test compound specifically binds to a polypeptide comprising the following steps: (a) expressing a nucleic acid or a vector comprising the nucleic acid under conditions permissive for translation of the nucleic acid to a polypeptide, wherein the nucleic acid comprises a nucleic acid of the invention, or, providing a polypeptide of the invention; (b) providing a test compound; (c) contacting the polypeptide with the test compound; and (d) determining whether the test compound of step (b) specifically binds to the polypeptide.

The invention provides methods for identifying a modulator of a hydrolase activity comprising the following steps: (a) providing a polypeptide of the invention or a polypeptide encoded by a nucleic acid of the invention; (b) providing a test compound; (c) contacting the polypeptide of step (a) with the test compound of step (b) and measuring an activity of the hydrolase, wherein a change in the hydrolase activity measured in the presence of the test compound compared to the activity in the absence of the test compound provides a determination that the test compound modulates the hydrolase activity. In one aspect, the hydrolase activity can be measured by providing a hydrolase substrate and detecting a decrease in the amount of the substrate or an increase in the amount of a reaction product, or, an increase in the amount of the substrate or a decrease in the amount of a reaction product. A decrease in the amount of the substrate or an increase in the amount of the reaction product with the test compound as compared to the amount of substrate or reaction product without the test compound identifies the test compound as an activator of hydrolase activity. An increase in the amount of the substrate or a decrease in the amount of the reaction product with the test compound as compared to the amount of substrate or reaction product without the test compound identifies the test compound as an inhibitor of hydrolase activity.

The invention provides computer systems comprising a processor and a data storage device wherein said data storage device has stored thereon a polypeptide sequence or a nucleic acid sequence of the invention (e.g., a polypeptide encoded by a nucleic acid of the invention). In one aspect, the computer system can further comprise a sequence comparison algorithm and a data storage device having at least one reference sequence stored thereon. In another aspect, the sequence comparison algorithm comprises a computer program that indicates polymorphisms. In one aspect, the computer system can further comprise an identifier that identifies one or more features in said sequence. The invention provides computer readable media having stored thereon a polypeptide sequence or a nucleic acid sequence of the invention. The invention provides methods for identifying a feature in a sequence comprising the steps of: (a) reading the sequence using a computer program which identifies one or more features in a sequence, wherein the sequence comprises a polypeptide sequence or a nucleic acid sequence of the invention; and (b) identifying one or more features in the sequence with the computer program. The invention provides methods for comparing a first sequence to a second sequence comprising the steps of: (a) reading the first sequence and the second sequence through use of a computer program which compares sequences, wherein the first sequence comprises a polypeptide sequence or a nucleic acid sequence of the invention; and (b) determining differences between the first sequence and the second sequence with the computer program. The step of determining differences between the first sequence and the second sequence can further comprise the step of identifying polymorphisms. In one aspect, the method can further comprise an identifier that identifies one or more features in a sequence. In another aspect, the method can comprise reading the first sequence using a computer program and identifying one or more features in the sequence.

The invention provides methods for isolating or recovering a nucleic acid encoding a polypeptide having a hydrolase activity from an environmental sample comprising the steps of: (a) providing an amplification primer sequence pair for amplifying a nucleic acid encoding a polypeptide having a hydrolase activity, wherein the primer pair is capable of amplifying a nucleic acid of the invention; (b) isolating a nucleic acid from the environmental sample or treating the environmental sample such that nucleic acid in the sample is accessible for hybridization to the amplification primer pair; and, (c) combining the nucleic acid of step (b) with the amplification primer pair of step (a) and amplifying nucleic acid from the environmental sample, thereby isolating or recovering a nucleic acid encoding a polypeptide having a hydrolase activity from an environmental sample. One or each member of the amplification primer sequence pair can comprise an oligonucleotide comprising at least about 10 to 50 consecutive bases of a sequence of the invention.

The invention provides methods for isolating or recovering a nucleic acid encoding a polypeptide having a hydrolase activity from an environmental sample comprising the steps of: (a) providing a polynucleotide probe comprising a nucleic acid of the invention or a subsequence thereof; (b) isolating a nucleic acid from the environmental sample or treating the environmental sample such that nucleic acid in the sample is accessible for hybridization to a polynucleotide probe of step (a); (c) combining the isolated nucleic acid or the treated environmental sample of step (b) with the polynucleotide probe of step (a); and (d) isolating a nucleic acid that specifically hybridizes with the polynucleotide probe of step (a), thereby isolating or recovering a nucleic acid encoding a polypeptide having a hydrolase activity from an environmental sample. The environmental sample can comprise a water sample, a liquid sample, a soil sample, an air sample or a biological sample. In one aspect, the biological sample can be derived from a bacterial cell, a protozoan cell, an insect cell, a yeast cell, a plant cell, a fungal cell or a mammalian cell.

The invention provides methods of generating a variant of a nucleic acid encoding a polypeptide having a hydrolase activity comprising the steps of: (a) providing a template nucleic acid comprising a nucleic acid of the invention; and (b) modifying, deleting or adding one or more nucleotides in the template sequence, or a combination thereof, to generate a variant of the template nucleic acid. In one aspect, the method can further comprise expressing the variant nucleic acid to generate a variant hydrolase polypeptide. The modifications, additions or deletions can be introduced by a method comprising error-prone PCR, shuffling, oligonucleotide-directed mutagenesis, assembly PCR, sexual PCR mutagenesis, *in vivo* mutagenesis, cassette mutagenesis, recursive ensemble mutagenesis, exponential ensemble mutagenesis, site-specific mutagenesis, gene reassembly, gene site saturated mutagenesis (GSSM), synthetic ligation reassembly (SLR) or a combination thereof. In another aspect, the modifications, additions or deletions are introduced by a method comprising recombination, recursive sequence recombination, phosphothioate-modified DNA mutagenesis, uracil-containing template mutagenesis, gapped duplex mutagenesis, point mismatch repair mutagenesis, repair-deficient host strain mutagenesis, chemical mutagenesis, radiogenic mutagenesis, deletion mutagenesis, restriction-selection mutagenesis, restriction-purification mutagenesis, artificial gene synthesis, ensemble mutagenesis, chimeric nucleic acid multimer creation and a combination thereof.

In one aspect, the method can be iteratively repeated until a hydrolase having an altered or different activity or an altered or different stability from that of a polypeptide encoded by the template nucleic acid is produced. In one aspect, the variant hydrolase polypeptide is thermotolerant, and retains some activity after being exposed to an elevated temperature. In another aspect, the variant hydrolase polypeptide has increased glycosylation as compared to the hydrolase encoded by a template nucleic acid. Alternatively, the variant hydrolase polypeptide has a hydrolase activity under a high temperature, wherein the hydrolase encoded by the template nucleic acid is not active under the high temperature. In one aspect, the method can be iteratively repeated until a hydrolase coding sequence having an altered codon usage from that of the template nucleic acid is produced. In another aspect, the method can be iteratively repeated until a hydrolase gene having higher or lower level of message expression or stability from that of the template nucleic acid is produced.

The invention provides methods for modifying codons in a nucleic acid encoding a polypeptide having a hydrolase activity to increase its expression in a host cell, the method comprising the following steps: (a) providing a nucleic acid of the invention encoding a polypeptide having a hydrolase activity; and, (b) identifying a non-preferred or a less preferred codon in the nucleic acid of step (a) and replacing it with a preferred or neutrally used codon encoding the same amino acid as the replaced codon, wherein a preferred codon is a codon over-represented in coding sequences in genes in the host cell and a non-preferred or less preferred codon is a codon under-represented in coding sequences in genes in the host cell, thereby modifying the nucleic acid to increase its expression in a host cell.

The invention provides methods for modifying codons in a nucleic acid encoding a polypeptide having a hydrolase activity; the method comprising the following steps: (a) providing a nucleic acid of the invention; and, (b) identifying a codon in the nucleic acid of step (a) and replacing it with a different codon encoding the same amino acid as the replaced codon, thereby modifying codons in a nucleic acid encoding a hydrolase.

The invention provides methods for modifying codons in a nucleic acid encoding a polypeptide having a hydrolase activity to increase its expression in a host cell, the method comprising the following steps: (a) providing a nucleic acid of the invention encoding a hydrolase polypeptide; and, (b) identifying a non-preferred or a less preferred codon in the nucleic acid of step (a) and replacing it with a preferred or neutrally used codon encoding the same amino acid as the replaced codon, wherein a preferred codon is a codon over-represented in coding sequences in genes in the host cell and a non-preferred or less preferred codon is a codon under-represented in coding sequences in genes in the host cell, thereby modifying the nucleic acid to increase its expression in a host cell.

The invention provides methods for modifying a codon in a nucleic acid encoding a polypeptide having a hydrolase activity to decrease its expression in a host cell, the method comprising the following steps: (a) providing a nucleic acid of the invention; and (b) identifying at least one preferred codon in the nucleic acid of step (a) and replacing it with a non-preferred or less preferred codon encoding the same amino acid as the replaced codon, wherein a preferred codon is a codon over-represented in coding sequences in genes in a host cell and a non-preferred or less preferred codon is a codon under-represented in coding sequences in genes in the host cell, thereby modifying the nucleic acid to decrease its expression in a host cell. In one aspect, the host cell can be a bacterial cell, a fungal cell, an insect cell, a yeast cell, a plant cell or a mammalian cell.

The invention provides methods for producing a library of nucleic acids encoding a plurality of modified hydrolase active sites or substrate binding sites, wherein the modified active sites or substrate binding sites are derived from a first nucleic acid comprising a sequence encoding a first active site or a first substrate binding site the method comprising the following steps: (a) providing a first nucleic acid encoding a first active site or first substrate binding site, wherein the first nucleic acid sequence comprises a sequence that hybridizes under stringent conditions to a nucleic acid of the invention, and the nucleic acid encodes a hydrolase active site or a hydrolase substrate binding site; (b) providing a set of mutagenic oligonucleotides that encode naturally-occurring amino acid variants at a plurality of targeted codons in the first nucleic acid; and, (c) using the set of mutagenic oligonucleotides to generate a set of active site-encoding or substrate binding site-encoding variant nucleic acids encoding a range of amino acid variations at each amino acid codon that was mutagenized, thereby producing a library of nucleic acids encoding a plurality of modified hydrolase active sites or substrate binding sites. In one aspect, the method comprises mutagenizing the first nucleic acid of step (a) by a method comprising an optimized directed evolution system, gene site-saturation mutagenesis (GSSM), synthetic ligation reassembly (SLR), error-prone PCR, shuffling, oligonucleotide-directed mutagenesis, assembly PCR, sexual PCR mutagenesis, in vivo mutagenesis, cassette mutagenesis, recursive ensemble mutagenesis, exponential ensemble mutagenesis, site-specific mutagenesis, gene reassembly, gene site saturated mutagenesis (GSSM), synthetic ligation reassembly (SLR) and a combination thereof. In another aspect, the method comprises mutagenizing the first nucleic acid of step (a) or variants by a method comprising recombination, recursive sequence recombination, phosphothioate-modified DNA mutagenesis, uracil-containing template mutagenesis, gapped duplex mutagenesis, point mismatch repair mutagenesis, repair-deficient host strain mutagenesis, chemical mutagenesis, radiogenic mutagenesis, deletion mutagenesis, restriction-selection mutagenesis, restriction-purification mutagenesis, artificial gene synthesis, ensemble mutagenesis, chimeric nucleic acid multimer creation and a combination thereof.

The invention provides methods for making a small molecule comprising the following steps: (a) providing a plurality of biosynthetic enzymes capable of synthesizing or modifying a small molecule, wherein one of the enzymes comprises a hydrolase enzyme encoded by a nucleic acid of the invention; (b) providing a substrate for at least one of the enzymes of step (a); and (c) reacting the substrate of step (b) with the enzymes under conditions that facilitate a plurality of biocatalytic reactions to generate a small molecule by a series of biocatalytic reactions. The invention provides methods for modifying a small molecule comprising the following steps: (a) providing a hydrolase enzyme, wherein the enzyme comprises a polypeptide of the invention, or, a polypeptide encoded by a nucleic acid of the invention, or a subsequence thereof; (b) providing a small molecule; and (c) reacting the enzyme of step (a) with the small molecule of step (b) under conditions that facilitate an enzymatic reaction catalyzed by the hydrolase enzyme, thereby modifying a small molecule by a hydrolase enzymatic reaction. In one aspect, the method can comprise a plurality of small molecule substrates for the enzyme of step (a), thereby generating a library of modified small molecules produced by at least one enzymatic reaction catalyzed by the hydrolase enzyme. In one aspect, the method can comprise a plurality of additional enzymes under conditions that facilitate a plurality of biocatalytic reactions by the enzymes to form a library of modified small molecules produced by the plurality of enzymatic reactions. In another aspect, the method can further comprise the step of testing the library to determine if a particular modified small molecule which exhibits a desired activity is present within the library. The step of testing the library can further comprise the steps of systematically eliminating all but one of the biocatalytic reactions used to produce a portion of the plurality of the modified small molecules within the library by testing the portion of the modified small molecule for the presence or absence of the particular modified small molecule with a desired activity, and identifying at least one specific biocatalytic reaction that produces the particular modified small molecule of desired activity.

The invention provides methods for determining a functional fragment of a hydrolase enzyme comprising the steps of: (a) providing a hydrolase enzyme, wherein the enzyme comprises a polypeptide of the invention, or a polypeptide encoded by a nucleic acid of the invention, or a subsequence thereof; and (b) deleting a plurality of amino acid residues from the sequence of step (a) and testing the remaining subsequence for a hydrolase activity, thereby determining a functional fragment of a hydrolase enzyme. In one aspect, the hydrolase activity is measured by providing a hydrolase substrate and detecting a decrease in the amount of the substrate or an increase in the amount of a reaction product.

The invention provides methods for whole cell engineering of new or modified phenotypes by using real-time metabolic flux analysis, the method comprising the following steps: (a) making a modified cell by modifying the genetic composition of a cell, wherein the genetic composition is modified by addition to the cell of a nucleic acid of the invention; (b) culturing the modified cell to generate a plurality of modified cells; (c) measuring at least one metabolic parameter of the cell by monitoring the cell culture of step (b) in real time; and, (d) analyzing the data of step (c) to determine if the measured parameter differs from a comparable measurement in an unmodified cell under similar conditions, thereby identifying an engineered phenotype in the cell using real-time metabolic flux analysis. In one aspect, the genetic composition of the cell can be modified by a method comprising deletion of a sequence or modification of a sequence in the cell, or, knocking out the expression of a gene. In one aspect, the method can further comprise selecting a cell comprising a newly engineered phenotype. In another aspect, the method can comprise culturing the selected cell, thereby generating a new cell strain comprising a newly engineered phenotype.

The invention provides methods of increasing thermotolerance or thermostability of a hydrolase polypeptide, the method comprising glycosylating a hydrolase polypeptide, wherein the polypeptide comprises at least thirty contiguous amino acids of a polypeptide of the invention; or a polypeptide encoded by a nucleic acid sequence of the invention, thereby increasing the thermotolerance or thermostability of the hydrolase polypeptide. In one aspect, the hydrolase specific activity can be thermostable or thermotolerant at a temperature in the range from greater than about 37°C to about 95°C.

The invention provides methods for overexpressing a recombinant hydrolase polypeptide in a cell comprising expressing a vector comprising a nucleic acid comprising a nucleic acid of the invention or a nucleic acid sequence of the invention, wherein the sequence identities are determined by analysis with a sequence comparison algorithm or by visual inspection, wherein overexpression is effected by use of a high activity promoter, a dicistronic vector or by gene amplification of the vector.

The invention provides detergent compositions comprising a polypeptide of the invention or a polypeptide encoded by a nucleic acid of the invention, wherein the polypeptide comprises a hydrolase activity, e.g., an esterase, acylase, lipase, phospholipase or protease activity. In one aspect, the hydrolase can be a nonsurface-active hydrolase. In another aspect, the hydrolase can be a surface-active hydrolase.

The invention provides methods for washing an object comprising the following steps: (a) providing a composition comprising a polypeptide having a hydrolase activity, e.g., an esterase, acylase, lipase, phospholipase or protease activity, wherein the polypeptide comprises: a polypeptide of the invention or a polypeptide encoded by a nucleic acid of the invention; (b) providing an object; and (c) contacting the polypeptide of step (a) and the object of step (b) under conditions wherein the composition can wash the object.

The invention provides methods of making a transgenic plant comprising the following steps: (a) introducing a heterologous nucleic acid sequence into the cell, wherein the heterologous nucleic sequence comprises a nucleic acid sequence of the invention, thereby producing a transformed plant cell; and (b) producing a transgenic plant from the transformed cell. In one aspect, the step (a) can further comprise introducing the heterologous nucleic acid sequence by electroporation or microinjection of plant cell protoplasts. In another aspect, the step (a) can further comprise introducing the heterologous nucleic acid sequence directly to plant tissue by DNA particle bombardment. Alternatively, the step (a) can further comprise introducing the heterologous nucleic acid sequence into the plant cell DNA using an *Agrobacterium tumefaciens* host. In one aspect, the plant cell can be a potato, corn, rice, wheat, tobacco, or barley cell.

The invention provides methods of expressing a heterologous nucleic acid sequence in a plant cell comprising the following steps: (a) transforming the plant cell with a heterologous nucleic acid sequence operably linked to a promoter, wherein the heterologous nucleic sequence comprises a nucleic acid of the invention; (b) growing the plant under conditions wherein the heterologous nucleic acids sequence is expressed in the plant cell.

The invention provides signal sequences comprising or consisting of a peptide having a subsequence of a polypeptide of the invention. The invention provides a chimeric protein comprising a first domain comprising a signal sequence of the invention and at least a second domain. The protein can be a fusion protein. The second domain can comprise an enzyme. The enzyme can be a hydrolase.

The invention provides method for biocatalytic synthesis of a structured lipid comprising the following steps: (a) providing a hydrolase of the invention; (b) providing a composition comprising a triacylglyceride (TAG); (c) contacting the polypeptide of step (a) with the composition of step (b) under conditions wherein the polypeptide hydrolyzes an acyl residue at the Sn2 position of the triacylglyceride (TAG), thereby producing a 1,3-diacylglyceride (DAG); (d) providing an R1 ester; (e) providing an R1-specific hydrolase, and (f) contacting the 1,3-DAG of step (c) with the R1 ester of step (d) and the R1-specific hydrolase of step (e) under conditions wherein the R1-specific hydrolase catalyzes esterification of the Sn2 position, thereby producing the structured lipid. The hydrolase can be an Sn2-specific lipase. The structured lipid can comprise a cocoa butter alternative (CBA), a synthetic cocoa butter, a natural cocoa butter, 1,3-dipalmitoyl-2-oleoylglycerol (POP), 1,3-distearoyl-2-oleoylglycerol (SOS), 1-palmitoyl-2-oleoyl-3-stearoylglycerol (POS) or 1-oleoyl-2,3-dimyristoylglycerol (OMM).

The invention provides a method for biocatalytic synthesis of a structured lipid comprising the following steps: (a) providing a hydrolase of the invention; (b) providing a composition comprising a triacylglyceride (TAG); (c) contacting the polypeptide of step (a) with the composition of step (b) under conditions wherein the polypeptide hydrolyzes an acyl residue at the Sn1 or Sn3 position of the triacylglyceride (TAG), thereby producing a 1,2-DAG or 2,3-DAG; and (d) promoting of acyl migration in the 1,2-DAG or 2,3-DAG of the step (c) under kinetically controlled conditions, thereby producing a 1,3-DAG. The method can further comprise providing an R1 ester and an R1-specific lipase, and contacting the 1,3-DAG of step (d) with the R1 ester and the R1-specific lipase under conditions wherein the R1-specific lipase catalyzes esterification of the Sn2 position, thereby producing a structured lipid. The lipase can be an Sn1 or an Sn3-specific lipase. The structured lipid can comprise a cocoa butter alternative (CBA), a synthetic cocoa butter, a natural cocoa butter, 1,3-dipalmitoyl-2-oleoylglycerol (POP), 1,3-distearoyl-2-oleoylglycerol (SOS), 1-palmitoyl-2-oleoyl-3-stearoylglycerol (POS) or 1-oleoyl-2,3-dimyristoylglycerol (OMM). In one aspect of the method, step (d) further comprises using ion exchange resins. The kinetically controlled conditions can comprise non-equilibrium conditions resulting in production of an end product having greater than a 2:1 ratio of 1,3-DAG to 2,3-DAG. The composition of step (b) can comprise a fluorogenic fatty acid (FA). The composition of step (b) can comprise an umbelliferyl FA ester. The end product can be enantiomerically pure.

The invention provides a method for preparation of an optical isomer of a propionic acid from a racemic ester of the propionic acid comprising the following steps: (a) providing a hydrolase of the invention, wherein the hydrolase is stereoselective for an optical isomer of the propionic acid; (b) providing racemic esters; (c) contacting the polypeptide of step (a) with the racemic esters of step (b) wherein the polypeptide can selectively catalyze the hydrolysis of the esters of step (b), thereby producing the optical isomer of the propionic acid. The optical isomer of the propionic acid can comprise an S(+) of 2-(6-methoxy-2-naphthyl) propionic acid and the racemic esters comprises racemic (R,S) esters of 2-(6-methoxy-2-naphthyl) propionic acid.

The invention provides a method for stereoselectively hydrolyzing racemic mixtures of esters of 2-substituted acids comprising the following steps: (a) providing a hydrolase of the invention, wherein the hydrolase is stereoselective; (b) providing a composition comprising a racemic mixture of esters of 2-substituted acids; and (c) contacting the polypeptide of step (a) with the composition of step (b) under conditions wherein the polypeptide of step (b) can selectively hydrolyze the esters. The hydrolase can be immobilized. The 2-substituted acid can comprise a 2-aryloxy substituted acid, an R-2-(4-hydroxyphenoxy)propionic acid or a 2-arylpropionic acid. The 2-substituted acid can comprise a ketoprofen.

The invention provides a method for oil or fat modification comprising the following steps: (a) providing a hydrolase of the invention; (b) providing an oil or fat, and (c) contacting the hydrolase of step (a) with the oil or fat of step (b) under conditions wherein the hydrolase can modify the oil or fat. The modification can comprise a hydrolase-catalyzed hydrolysis of the fat or oil. The hydrolysis can be a complete or a partial hydrolysis of the fat or oil. The oil can comprise a glycerol ester of a polyunsaturated fatty acid, or a fish, animal, or vegetable oil. The vegetable oil can comprise an olive, canola, sunflower, palm, soy or lauric oil or rice bran oil.

The invention provides a method for hydrolysis of polyunsaturated fatty acid (PUFA) esters comprising the following steps: (a) providing a hydrolase of the invention; (b) providing composition comprising a polyunsaturated fatty acid ester, and (c) contacting the hydrolase with the composition of step (b) under conditions wherein the hydrolase can hydrolyze the polyunsaturated fatty acid (PUFA) ester. The invention provides a method of selective hydrolysis of polyunsaturated fatty acids esters over saturated fatty acid esters comprising the following steps: (a) providing a hydrolase of the invention, wherein the hydrolase has a lipase activity and selectively hydrolyzes polyunsaturated fatty acid (PUFA) esters; (b) providing a composition comprising a mixture of polyunsaturated and saturated esters; and (c) contacting the polypeptide of step (a) with the composition of step (b) under conditions wherein the polypeptide can selectively catalyze the hydrolysis of polyunsaturated fatty acids esters.

The invention provides a method for preparing a food or a feed additive comprising polyunsaturated fatty acids (PUFA) comprising the following steps: (a) providing a hydrolase of the invention, wherein the hydrolase selectively hydrolyzes polyunsaturated fatty acid (PUFA) esters; (b) providing a composition comprising a PUFA ester; and (c) contacting the polypeptide of step (a) with the composition of step (b) under conditions wherein the polypeptide can selectively catalyze the hydrolysis of polyunsaturated fatty acid esters thereby producing the PUFA-containing food or feed additive.

The invention provides a method for treatment of latex comprising the following steps: (a) providing a hydrolase of the invention, wherein the polypeptide has selectivity for a saturated ester over an unsaturated ester, thereby converting the saturated ester to its corresponding acid and alcohol; (b) providing a latex composition comprising saturated and unsaturated esters; (c) contacting the hydrolase of step (a) with the composition of step (b) under conditions wherein the polypeptide can selectively hydrolyze saturated esters, thereby treating the latex. The ethyl propionate can be selectively hydrolyzed over ethyl acrylate. The latex composition of step (b) can comprise polymers containing acrylic, vinyl and unsaturated acid monomers, alkyl acrylate monomers, methyl acrylate, ethyl acrylate, propyl acrylate and butyl acrylate, acrylate acids, acrylic acid, methacrylic acid, crotonic acid, itaconic acid and mixtures thereof. The latex composition can be a hair fixative. The conditions of step (c) can comprise a pH in the range from about pH 4 to pH 8 and a temperature in the range from about $20^0$ to about $50^0$C.

The invention provides a method for refining a lubricant comprising the following steps: (a) providing a composition comprising a hydrolase of the invention; (b) providing a lubricant; and (c) treating the lubricant with the hydrolase under conditions wherein the hydrolase can selective hydrolyze oils in the lubricant, thereby refining it. The lubricant can be a hydraulic oil.

The invention provides a method of treating a fabric comprising the following steps: (a) providing a composition comprising a hydrolase of the invention, wherein the hydrolase can selectively hydrolyze carboxylic esters; (b) providing a fabric; and (c) treating the fabric with the hydrolase under condition wherein the hydrolase can selectively hydrolyze carboxylic esters thereby treating the fabric. The treatment of the fabric can comprise improvement of the hand and drape of the final fabric, dyeing, obtaining flame retardancy, obtaining water repellency, obtaining optical brightness, or obtaining resin finishing. The fabric can comprise cotton, viscose, rayon, lyocell, flax, linen, ramie, all blends thereof, or blends thereof with polyesters, wool, polyamides acrylics or polyacrylics. The invention provides a fabric, yarn or fiber comprising a hydrolase of the invention, which can be adsorbed, absorbed or immobilized on the surface of the fabric, yarn or fiber.

The invention provides a method for removing or decreasing the amount of a food or oil stain comprising contacting a hydrolase of the invention with the food or oil stain under conditions wherein the hydrolase can hydrolyze oil or fat in the stain. The hydrolase can have an enhanced stability to denaturation by surfactants and to heat deactivation. The hydrolase can have a detergent or a laundry solution.

The invention provides a dietary composition comprising a hydrolase of the invention. The dietary composition can further comprise a nutritional base comprising a fat. The hydrolase can be activated by a bile salt. The dietary composition can further comprising a cow's milk-based infant formula. The hydrolase can hydrolyze long chain fatty acids. The invention provides a method of reducing fat content in milk or vegetable-based dietary compositions comprising the following steps: (a) providing a composition comprising a hydrolase of the invention; (b) providing a composition comprising a milk or a vegetable oil, and (c) treating the composition of step (b) with the hydrolase under conditions wherein the hydrolase can hydrolyze the oil or fat in the composition, thereby reducing its fat content. The invention provides a dietary composition for a human or non-ruminant animals comprising a nutritional base, wherein the base comprises a fat and no or little hydrolase, and an effective amount of a hydrolase as set forth in claim 56 to increase fat absorption and growth of human or non-ruminant animal.

The invention provides a method of catalyzing an interesterification reaction to produce new triglycerides comprising the following steps: (a) providing a composition comprising a hydrolase of the invention, wherein the hydrolase can catalyze an interesterification reaction; (b) providing a mixture of triglycerides and free fatty acids; (c) treating the composition of step (b) with the hydrolase under conditions wherein the hydrolase can catalyze exchange of free fatty acids with the acyl groups of triglycerides, thereby producing new triglycerides enriched in the added fatty acids. The hydrolase can be an Snl,3-specific lipase. The invention provides a transesterification method for preparing a margarine oil having a low trans- acid and a low intermediate chain fatty acid content, comprising the following steps: (a) providing a transesterification reaction mixture comprising a stearic acid source material selected from the group consisting of stearic acid, stearic acid monoesters of low molecular weight monohydric alcohols and mixtures thereof, (b) providing a liquid vegetable oil; (c) providing a hydrolase of the invention, wherein the polypeptide comprises a 1,3-specific lipase activity; (d) transesterifying the stearic acid source material and the vegetable oil triglyceride, to substantially equilibrate the ester groups in the 1-, 3- positions of the glyceride component with non-glyceride fatty acid components of the reaction mixture, (e) separating transesterified free fatty acid components from glyceride components of the transesterification mixture to provide a transesterified margarine oil product and a fatty acid mixture comprising fatty acids, fatty acid monoesters or mixtures thereof released from the vegetable oil, and (f) hydrogenating the fatty acid mixture.

The invention provides a method for making a composition comprising 1-palmitoyl-3-stearoyl-2-monoleine (POSt) and 1,3-distearoyl-2-monoleine (StOSt) comprising providing a lipase as set forth in claim 56, wherein the lipase is capable of 1,3-specific lipase-catalyzed interesterification of 1,3-dipalmitoyl-2-monoleine (POP) with stearic acid or tristearin, to make a product enriched in the 1-palmitoyl-3-stearoyl-2-monoleine (POSt) or 1,3-distearoyl-2-monoleine (StOSt).

The invention provides a method for ameliorating or preventing lipopolysaccharide (LPS)-mediated toxicity comprising administering to a patient a pharmaceutical composition comprising a polypeptide of the invention. The invention provides a method for detoxifying an endotoxin comprising contacting the endotoxin with a polypeptide of the invention. The invention provides a method for deacylating a 2' or a 3' fatty acid chain from a lipid A comprising contacting the lipid A with a polypeptide of the invention.

The details of one or more embodiments of the invention are set forth in the accompanying drawings and the description below. Other features, objects, and advantages of the invention will be apparent from the description and drawings, and from the claims.

All publications, patents, patent applications, GenBank sequences and ATCC deposits, cited herein are hereby expressly incorporated by reference for all purposes.

## DESCRIPTION OF DRAWINGS

The following drawings are illustrative of embodiments of the invention and are not meant to limit the scope of the invention as encompassed by the claims.

The patent or application file contains at least one drawing executed in color. Copies of this patent or patent application publication with color drawing(s) will be provided by the Office upon request and payment of the necessary fee.

Figure 1 is a block diagram of a computer system.

Figure 2 is a flow diagram illustrating one aspect of a process for comparing a new nucleotide or protein sequence with a database of sequences in order to determine the homology levels between the new sequence and the sequences in the database.

Figure 3 is a flow diagram illustrating one aspect of a process in a computer for determining whether two sequences are homologous.

Figure 4 is a flow diagram illustrating one aspect of an identifier process 300 for detecting the presence of a feature in a sequence.

Figure 5 illustrates an exemplary method of the invention to test for lipase activity, a colorimetric lipase assay, as described in Example 1, below.

Figure 6 illustrates an exemplary method of the invention using an Sn2 regio-specific lipase in the synthesis of structured lipids.

Figure 7 illustrates an exemplary method of the invention, a "Forced Migration Methodology" for the structured synthesis of lipids, as described in detail in Example 2, below.

Figure 8 illustrates an exemplary method comprising use of lipases of the invention to synthesize cocoa butter alternatives, as described in detail below.

Figure 9A and figure 9B illustrate exemplary methods of the invention comprising synthesizing PUFA-containing sTAGs (Figure 9A, top) and 2-PUFA sMAGs and purified PUFAs (Figure 9B, bottom).

Figure 10 illustrates an exemplary method comprising a coupled enzyme assay, as discussed in detail, below.

Figure 11 illustrates an exemplary growth-kill assay, as discussed in Example 6, below.

Figure 12 illustrates data of various esterification reactions in the synthesis of 1,3-DCy, as discussed in Example 7, below.

Figure 13 summarizes data showing the effect of substrate ratio on esterification between glycerol and caprylic acid, as discussed in Example 7, below.

Figure 14 summarizes data of various synthesis of 1,3-dilaurin, as discussed in Example 7, below.

Figure 15 summarizes the effect of substrate ration on esterification of glycerol and lauric acid in n-hexane, as discussed in Example 7, below.

Figure 16 summarizes the synthesis of 1,3-dipalmitin, as discussed in Example 7, below.

Figure 17 summarizes data for the esterification of glycerol and palmitic (C16:O) or stearic (C18:0) acid, as discussed in Example 7, below.

Figure 18 shows data from alcoholysis reaction, as discussed in Example 7, below.

Figure 19 illustrates data from the hydrolysis of trilaurin, as discussed in Example 7, below.

Figure 20 shows the effect of trilaurin:water ratio on hydrolysis of trilaurin, as discussed in Example 7, below.

Figure 21 summarizes data showing the effect of organic solvents on hydrolysis of trilaurin, as discussed in Example 7, below.

Figure 22 illustrates data from the alcoholysis and hydrolysis of coconut oil in organic solvent, as discussed in Example 7, below.

Figure 23 shows the effect of oleic acid on acyl migration of 1,2-dipalmitin in n-hexane at room temperature, as discussed in Example 7, below.

Figure 24 shows the effect of the amount of anion exchanger on acyl migration of 1,2-dipalmitin in n-hexane, as discussed in Example 7, below.

Figure 25 shows data from the esterification of 1,3-dicaprylin and oleic acid vinyl ester in n-hexane by an immobilized lipase from a *Pseudomonas* sp. (Amano PS-D, Amano Enzyme USA, Elgin, IL), as discussed in Example 7, below.

Figure 26 shows data from the esterification of 1,3-DG and oleic acid or oleic acid vinyl ester in n-hexane by an immobilized lipase from a *Pseudomonas* sp. (Amano PS-D, Amano Enzyme USA, Elgin, IL), as discussed in Example 7, below.

Figure 27 illustrates an exemplary forced migration reaction of the invention, as discussed below.

Figure 28 illustrates an exemplary synthesis of a triglyceride mixture composed of POS (Palmitic-Oleic-Stearic), POP (Palmitic-Oleic-Palmitic) and SOS (Stearic-Oleic-Stearic) from glycerol, as discussed below.

Figure 29 illustrates an exemplary synthesis where stearate and palmitate are mixed together to generate mixtures of DAGs which are subsequently acylated with oleate to give components of cocoa butter equivalents, as discussed below.

Figure 30 schematically illustrates data from a two enzyme system of the invention, as described in Example 10, below.

Like reference symbols in the various drawings indicate like elements.

DETAILED DESCRIPTION

In one aspect, the invention provides hydrolases, polynucleotides encoding them, and methods of making and using these polynucleotides and polypeptides. In one aspect, the invention is directed to polypeptides, e.g., enzymes, having a hydrolase activity, e.g., an esterase, acylase, lipase, phospholipase or protease activity, including thermostable and thermotolerant hydrolase activity, and polynucleotides encoding these enzymes, and making and using these polynucleotides and polypeptides. The hydrolase activities of the polypeptides and peptides of the invention include esterase activity, lipase activity (hydrolysis of lipids), acidolysis reactions (to replace an esterified fatty acid with a free fatty acid), transesterification reactions (exchange of fatty acids between triglycerides), ester synthesis, ester interchange reactions, phospholipase activity (e.g., phospholipase A, B, C and D activity, patatin activity, lipid acyl hydrolase (LAH) activity) and protease activity (hydrolysis of peptide bonds). The polypeptides of the invention can be used in a variety of pharmaceutical, agricultural and industrial contexts, including the manufacture of cosmetics and nutraceuticals. In another aspect, the polypeptides of the invention are used to synthesize enantiomerically pure chiral products. The polypeptides of the invention can be used in a variety of pharmaceutical, agricultural and industrial contexts, including the manufacture of cosmetics and nutraceuticals.

Enzymes of the invention can be highly selective catalysts. They can have the ability to catalyze reactions with stereo-, regio-, and chemo- selectivities not possible in conventional synthetic chemistry. Enzymes of the invention can be versatile. In various aspects, they can function in organic solvents, operate at extreme pHs (for example, high pHs and low pHs) extreme temperatures (for example, high temperatures and low temperatures), extreme salinity levels (for example, high salinity and low salinity), and catalyze reactions with compounds that are structurally unrelated to their natural, physiological substrates.

*Hydrolases of the invention having lipase activity*

In one aspect, the polypeptides of the invention have lipase activity and can be used as lipases, e.g., in the biocatalytic synthesis of structured lipids (lipids that contain a defined set of fatty acids distributed in a defined manner on the glycerol backbone), including cocoa butter alternatives, poly-unsaturated fatty acids (PUFAs), 1,3-diacyl glycerides (DAGs), 2-monoglycerides (MAGs) and triacylglycerides (TAGs), such as 1,3-dipalmitoyl-2-oleoylglycerol (POP), 1,3-distearoyl-2-oleoylglycerol (SOS), 1-palmitoyl-2-oleoyl-3-stearoylglycerol (POS) or 1-oleoyl-2,3-dimyristoylglycerol (OMM), long chain polyunsaturated fatty acids such as arachidonic acid, docosahexaenoic acid (DHA) and eicosapentaenoic acid (EPA).

In one aspect, the invention provides an exemplary synthesis (using lipases of the invention) of a triglyceride mixture composed of POS (Palmitic-Oleic-Stearic), POP (Palmitio-Oleic-Palmitic) and SOS (Stearic-Oleic-Stearic) from glycerol, as illustrated in Figure 28. This exemplary synthesis uses free fatty acids versus fatty acid esters. In one aspect, this reaction can be performed in one pot with sequential addition of fatty acids using crude glycerol and free fatty acids and fatty acid esters. In one aspect, stearate and palmitate are mixed together to generate mixtures ofDAGs. In one aspect, the diacylglycerides are subsequently acylated with oleate to give components of cocoa butter equivalents, as illustrated in Figure 29. In alternative aspects, the proportions of POS, POP and SOS can be varied according to: stearate to palmitate ratio; selectivity of enzyme for palmitate versus stearate; or enzyme enantioselectivity (could alter levels of POS/SOP). One-pot synthesis of cocoa butter equivalents or other cocoa butter alternatives is possible using this aspect of the invention.

In one aspect, lipases that exhibit regioselectivity and/or chemoselectivity are used in the structure synthesis of lipids or in the processing of lipids. Thus, the methods of the invention use lipases with defined regio-specificity or defined chemoselectivity (e.g., a fatty acid specificity) in a biocatalytic synthetic reaction. For example, the methods of the invention can use lipases with SN1, SN2 and/or SN3 regio-specificity, or combinations thereof. In one aspect, the methods of the invention use lipases that exhibit regioselectivity for the 2-position of a triacylglyceride (TAG). This SN2 regioselectivity can be used in the synthesis of a variety of structured lipids, e.g., triacylglycerides (TAGs), including 1,3-DAGs and components of cocoa butter, as illustrated in Figure 6.

The methods and compositions (lipases) of the invention can be used in the biocatalytic synthesis of structured lipids, and the production of nutraceuticals (e.g., polyunsaturated fatty acids and oils), various foods and food additives (e.g., emulsifiers, fat replacers, margarines and spreads), cosmetics (e.g., emulsifiers, creams), pharmaceuticals and drug delivery agents (e.g., liposomes, tablets, formulations), and animal feed additives (e.g., polyunsaturated fatty acids, such as linoleic acids) comprising lipids made by the structured synthesis methods of the invention or processed by the methods of the invention

In one aspect, lipases of the invention can act on fluorogenic fatty acid (FA) esters, e.g., umbelliferyl FA esters. In one aspect, profiles of FA specificities of lipases made or modified by the methods of the invention can be obtained by measuring their relative activities on a series of umbelliferyl FA esters, such as palmitate, stearate, oleate, laurate, PUFA, butyrate.

The methods and compositions (lipases) of the invention can be used to synthesize enantiomerically pure chiral products. In one aspect, the methods and compositions (lipases) of the invention can be used to prepare a D-amino acid and corresponding esters from a racemic mix. For example, D-aspartic acid can be prepared from racemic aspartic acid. In one aspect, optically active D-homophenylalanine and/or its esters are prepared. The enantioselectively synthesized D-homophenylalanine can be starting material for many drugs, such as Enalapril, Lisinopril, and Quinapril, used in the treatment of hypertension and congestive heart failure. The D-aspartic acid and its derivatives made by the methods and compositions of the invention can be used in pharmaceuticals, e.g., for the inhibition of arginiosuccinate synthetase to prevent or treat sepsis or cytokine-induced systemic hypotension or as immunosuppressive agents. The D-aspartic acid and its derivatives made by the methods and compositions of the invention can be used as taste modifying compositions for foods, e.g., as sweeteners (e.g., ALITAME™). For example, the methods and compositions (lipases) of the invention can be used to synthesize an optical isomer S(+) of 2-(6-methoxy-2-naphthyl) propionic acid from a racemic (R,S) ester of 2-(6-methoxy-2-naphthyl) propionic acid (see, e.g., U.S. Patent No. 5,229,280).

In one aspect, the methods and compositions (lipases) of the invention can be used to for stereoselectively hydrolyzing racemic mixtures of esters of 2-substituted acids, e.g., 2-aryloxy substituted acids, such as R-2-(4-hydroxyphenoxy)propionic acid, 2-arylpropionic acid, ketoprofen to synthesize enantiomerically pure chiral products. See, e.g., U.S. Patent No. 5,108,916.

In one aspect, the lipase of the invention for these reactions is immobilized, e.g., as described below. In alternative aspects, the methods of the invention do not require an organic solvent, can proceed with relatively fast reaction rates; and do not require a protective group for the amino acid. See, e.g., U.S. Patent No. 5,552,317; 5,834,259.

The methods and compositions (lipases) of the invention can be used to hydrolyze oils, such as fish, animal and vegetable oils, and lipids, such as poly-unsaturated fatty acids. In one aspect, the polypeptides of the invention are used process fatty acids (such as poly-unsaturated fatty acids), e.g., fish oil fatty acids, for use in or as a feed additive. Addition of poly-unsaturated fatty acids PUFAs to feed for dairy cattle has been demonstrated to result in improved fertility and milk yields. Fish oil contains a high level of PUFAs (see Table 2, below) and therefore is a potentially inexpensive source for PUFAs as a starting material for the methods of the invention. The biocatalytic methods of the invention can process fish oil under mild conditions, thus avoiding harsh conditions utilized in some processes. Harsh conditions may promote unwanted isomerization, polymerization and oxidation of the PUFAs. In one aspect, the methods of the invention comprise lipase-catalyzed total hydrolysis of fish-oil or selective hydrolysis of PUFAs from fish oil to provide a mild alternative that would leave the high-value PUFAs intact. In one aspect, the methods further comprise hydrolysis of lipids by chemical or physical splitting of the fat.

Table 2: Fatty acid composition of a variety of fats and oils. Fatty acid content of fats (%):

| | 14:0 | 16:0 | 16:1 | 18:0 | 18:1 | 18:2 | 18:3 | 18:4 | 20:x | 22:x |
|---|---|---|---|---|---|---|---|---|---|---|
| Tallow | 3.7 | 24.9 | 4.2 | 18.9 | 36 | 3.1 | | | | |
| Lard | | 23.8 | 2.7 | 13.5 | 41.2 | 10.2 | 1.0 | | | |
| Canola Oil | | 4.0 | | 1.8 | 56.1 | 20.3 | 9.3 | | 2.4 | 1.0 |
| Soybean Oil | | 10.3 | | 3.8 | 22.8 | 51 | 6.8 | | 0.2 | |
| Palm Oil | | 48.6 | | 4.1 | 36.6 | 9.1 | 0.3 | | 0.1 | |
| Corn Oil | | 10.9 | | 1.8 | 24.2 | 58 | 0.7 | | | |
| Fish Oil | 7.2 | 16.7 | 11.1 | 3.2 | 10.2 | 1.4 | 2.4 | 3.5 | 16.4 | 16.1 |

In one aspect, the lipases and methods of the invention are used for the total hydrolysis of fish oil. Lipases can be screened for their ability to catalyze the total hydrolysis of fish oil under different conditions using, e.g., a method comprising a coupled enzyme assay, as illustrated in Figure 10, to detect the release of glycerol from lipids. This assay has been validated in the presence of lipid emulsions and retains sensitivity under these conditions. In alternative aspects, a single or multiple lipases are used to catalyze the total splitting of the fish oil. Several lipases of the invention may need to be used, owing to the presence of the PUFAs. In one aspect, a PUFA-specific lipase of the invention is combined with a general lipase to achieve the desired effect.

The methods and compositions (lipases) of the invention can be used to catalyze the partial or total hydrolysis of other oils, e.g. olive oils, that do not contain PUFAs.

The methods and compositions (lipases) of the invention can be used to catalyze the hydrolysis of PUFA glycerol esters. These methods can be used to make feed additives. In one aspect, lipases of the invention catalyze the release of PUFAs from simple esters and fish oil. Standard assays and analytical methods can be utilized.

The methods and compositions (lipases) of the invention can be used to selectively hydrolyze saturated esters over unsaturated esters into acids or alcohols. The methods and compositions (lipases) of the invention can be used to treat latexes for a variety of purposes, e.g., to treat latexes used in hair fixative compositions to remove unpleasant odors. The methods and compositions (lipases) of the invention can be used in the treatment of a lipase deficiency in an animal, e.g., a mammal, such as a human. The methods and compositions (lipases) of the invention can be used to prepare lubricants, such as hydraulic oils. The methods and compositions (lipases) of the invention can be used in making and using detergents. The methods and compositions (lipases) of the invention can be used in processes for the chemical finishing of fabrics, fibers or yarns. In one aspect, the methods and compositions (lipases) of the invention can be used for obtaining flame retardancy in a fabric using, e.g., a halogen-substituted carboxylic acid or an ester thereof, i.e. a fluorinated, chlorinated or bromated carboxylic acid or an ester thereof. In one aspect, the invention provides methods of generating lipases from environmental libraries.

*Hydrolases of the invention having esterase or acylase activity*

In one aspect, the hydrolase activity of the invention comprises an acylase or an esterase activity. In one aspect, the hydrolysis activity comprises hydrolyzing a lactone ring or acylating an acyl lactone or a diol lactone. In one aspect, the hydrolysis activity comprises an esterase activity. In one aspect, the esterase activity comprises hydrolysis of ester groups to organic acids and alcohols. In one aspect, the esterase activity comprises feruloyl esterase activity. In one aspect, the esterase activity comprises a lipase activity. In alternative aspects, the esterase activities of the enzymes of the invention include lipase activity (in the hydrolysis of lipids), acidolysis reactions (to replace an esterified fatty acid with a free fatty acid), transesterification reactions (exchange of fatty acids between triglycerides), ester synthesis and ester interchange reactions. The enzymes of the invention can also be utilized in organic synthesis reactions in the manufacture of medicaments, pesticides or intermediates thereof.

In one aspect, the polypeptides of the invention have esterase or acylase activity and can be used, e.g., to hydrolyze a lactone ring or acylate an acyl lactone or a diol lactone. In one aspect, the hydrolysis activity of a polypeptide of the invention comprises an esterase activity. In one aspect, the esterase activity comprises hydrolysis of ester groups to organic acids and alcohols. In one aspect, the esterase activity comprises feruloyl esterase activity. In one aspect, the esterase activity comprises a lipase activity. In alternative aspects, the esterase activities of the enzymes of the invention include lipase activity (in the hydrolysis of lipids), acidolysis reactions (to replace an esterified fatty acid with a free fatty acid), transesterification reactions (exchange of fatty acids between triglycerides), ester synthesis and ester interchange reactions. The enzymes of the invention can also be utilized in organic synthesis reactions in the manufacture of medicaments, pesticides or intermediates thereof. The esterase activities of the polypeptides and peptides of the invention include lipase activity (in the hydrolysis of lipids), acidolysis reactions (to replace an esterified fatty acid with a free fatty acid), transesterification reactions (exchange of fatty acids between triglycerides), ester synthesis and ester interchange reactions. The polypeptides and peptides of the invention can also be utilized in organic synthesis reactions in the manufacture of medicaments, pesticides or intermediates thereof.

*Hydrolases of the invention having protease activity*

In one aspect, the invention provides polypeptides having a protease activity, polynucleotides encoding the polypeptides, and methods for making and using these polynucleotides and polypeptides. In one aspect, the proteases of the invention are used to catalyze the hydrolysis of peptide bonds. The proteases of the invention can be used to make and/or process foods or feeds, textiles, detergents and the like. The proteases of the invention can be used in pharmaceutical compositions and dietary aids.

The protease preparations of the invention (including those for treating or processing feeds or foods, treating fibers and textiles, waste treatments, plant treatments, and the like) can further comprise one or more enzymes, for example, hydrolases of this invention or other hydrolases, pectate lyases, cellulases (endo-beta-1,4-glucanases), beta-glucanases (endo-beta-1,3(4)-glucanases), lipases, cutinases, peroxidases, laccases, amylases, glucoamylases, pectinases, reductases, oxidases, phenoloxidases, ligninases, pullulanases, arabinanases, hemicellulases, mannanases, xyloglucanases, xylanases, esterases of the invention or other esterases, pectin acetyl esterases, rhamnogalacturonan acetyl esterases, polygalacturonases, rhamnogalacturonases, galactanases, pectin lyases, pectin methylesterases, cellobiohydrolases, transglutaminases; or mixtures thereof.

A polypeptide can be routinely assayed for protease activity (e.g., tested to see if the protein is within the scope of the invention) by any method, e.g., protease activity can be assayed by the hydrolysis of casein in zymograms, the release of fluorescence from gelatin, or the release of p-nitroanalide from various small peptide substrates (these and other exemplary protease assays are set forth in the Examples, below).

*Hydrolases of the invention having phospholipase activity*

In one aspect, the invention provides polypeptides having a phospholipase activity. The phospholipases of the invention can have phospholipase A, B, C, D, a lipid acyl hydrolase (LAH), or patatin enzyme activity. The phospholipases of the invention can efficiently cleave glycerolphosphate ester linkage in oils, such as vegetable oils, e.g., oilseed phospholipids, to generate a water extractable phosphorylated base and a diglyceride. In alternative aspects, the phospholipases of the invention can cleave glycerolphosphate ester linkages in phosphatidylcholine, phosphatidylethanolamine, phosphatidylserine and sphingomyelin.

In one aspect, the phospholipases of the invention are used in various vegetable oil processing steps, such as in vegetable oil extraction, particularly, in the removal of "phospholipid gums" in a process called "oil degumming," as described herein. The production of vegetable oils from various sources, such as rice bran, soybeans, rapeseed, peanut, sesame, sunflower and corn. The phospholipase enzymes of the invention can be used in place of PLA, e.g., phospholipase A2, in any vegetable oil processing step. A phospholipase of the invention (e.g., phospholipase A, B, C, D, patatin enzymes) can be used for enzymatic degumming of vegetable oils because the phosphate moiety is soluble in water and easy to remove. The diglyceride product will remain in the oil and therefore will reduce losses. The PLCs of the invention can be used in addition to or in place of PLA1s and PLA2s in commercial oil degumming, such as in the ENZYMAX® process, where phospholipids are hydrolyzed by PLA1 and PLA2.

In alternative aspects, enzymes of the invention have phosphatidylinositol-specific phospholipase C (PI-PLC) activity, phosphatidylcholine-specific phospholipase C activity, phosphatidic acid phosphatase activity, phospholipase A activity and/or patatin-related phospholipase activity. These enzymes can be used alone or in combination each other or with other enzymes of the invention, or other enzymes. In one aspect, the invention provides methods wherein these enzymes (including phosphatidylinositol-specific phospholipase C, phosphatidylcholine-specific phospholipase C, phosphatidic acid phosphatase, phospholipase A and/or patatin-related phospholipases of the invention) are used alone or in combination in the degumming of oils, e.g., rice bran oil, vegetable oils, e.g., high phosphorous oils, such as soybean, corn, canola and sunflower oils.

These enzymes and processes of the invention can be used to achieve a more complete degumming of high phosphorous oils, in particular, rice bran, soybean, corn, canola, and sunflower oils. Upon cleavage by PI-PLC, phosphatidylinositol is converted to diacylglycerol and phosphoinositol. The diacylglycerol partitions to the aqueous phase (improving oil yield) and the phosphoinositol partitions to the aqueous phase where it is removed as a component of the heavy phase during centrifugation. An enzyme of the invention, e.g., a PI-PLC of the invention, can be incorporated into either a chemical or physical oil refining process.

In one aspect, hydrolases, e.g., PLC phospholipases, of the invention utilize a variety of phospholipid substrates including phosphatidylcholine, phosphatidylethanolamine, phosphatidylserine, phosphatidylinositol, and phosphatidic acid. In addition, these enzymes can have varying degrees of activity on the lysophospholipid forms of these phospholipids. In various aspects, PLC enzymes of the invention may show a preference for phosphatidylcholine and phosphatidylethanolamine as substrates.

In one aspect, hydrolases, e.g., phosphatidylinositol PLC phospholipases, of the invention utilize a variety of phospholipid substrates including phosphatidylcholine, phosphatidylethanolamine, phosphatidylserine, phosphatidylinositol, and phosphatidic acid. In addition, these enzymes can have varying degrees of activity on the lysophospholipid forms of these phospholipids. In various aspects, phosphatidylinositol PLC enzymes of the invention may show a preference for phosphatidylinositol as a substrate.

In one aspect, hydrolases, e.g., patatin enzymes, of the invention utilize a variety of phospholipid substrates including phosphatidylcholine, phosphatidylethanolamine, phosphatidylserine, phosphatidylinositol, and phosphatidic acid. In addition, these enzymes can have varying degrees of activity on the lysophospholipid forms of these phospholipids. In various aspects, patatins of the invention are based on a conservation of amino acid sequence similarity. In various aspects, these enzymes display a diverse set of biochemical properties and may perform reactions characteristic of PLA1, PLA2, PLC, or PLD enzyme classes.

In one aspect, hydrolases, e.g., PLD phospholipases, of the invention utilize a variety of phospholipid substrates including phosphatidylcholine, phosphatidylethanolamine, phosphatidylserine, phosphatidylinositol, and phosphatidic acid. In addition, these enzymes can have varying degrees of activity on the lysophospholipid forms of these phospholipids. In one aspect, these enzymes are useful for carrying out transesterification reactions to produce structured phospholipids.

Generating and Manipulating Nucleic Acids

The invention provides nucleic acids, including expression cassettes such as expression vectors, encoding the polypeptides (e.g., hydrolases, antibodies) of the invention. The invention also includes methods for discovering new hydrolase sequences using the nucleic acids of the invention. Also provided are methods for modifying the nucleic acids of the invention by, e.g., synthetic ligation reassembly, optimized directed evolution system and/or saturation mutagenesis.

The nucleic acids of the invention can be made, isolated and/or manipulated by, e.g., cloning and expression of cDNA libraries, amplification of message or genomic DNA by PCR, and the like. In practicing the methods of the invention, homologous genes can be modified by manipulating a template nucleic acid, as described herein. The invention can be practiced in conjunction with any method or protocol or device known in the art, which are well described in the scientific and patent literature.

*General Techniques*

The nucleic acids used to practice this invention, whether RNA, iRNA, antisense nucleic acid, cDNA, genomic DNA, vectors, viruses or hybrids thereof, may be isolated from a variety of sources, genetically engineered, amplified, and/or expressed/ generated recombinantly. Recombinant polypeptides generated from these nucleic acids can be individually isolated or cloned and tested for a desired activity. Any recombinant expression system can be used, including bacterial, mammalian, yeast, insect or plant cell expression systems.

Alternatively, these nucleic acids can be synthesized in vitro by well-known chemical synthesis techniques, as described in, e.g., Adams (1983) J. Am. Chem. Soc. 105:661; Belousov (1997) Nucleic Acids Res. 25:3440-3444; Frenkel (1995) Free Radic. Biol. Med. 19:373-380; Blommers (1994) Biochemistry 33:7886-7896; Narang (1979) Meth. Enzymol. 68:90; Brown (1979) Meth. Enzymol. 68:109; Beaucage (1981) Tetra. Lett. 22:1859; U.S. Patent No. 4,458,066.

Techniques for the manipulation of nucleic acids, such as, e.g., subcloning, labeling probes (e.g., random-primer labeling using Klenow polymerase, nick translation, amplification), sequencing, hybridization and the like are well described in the scientific and patent literature, see, e.g., Sambrook, ed., MOLECULAR CLONING: A LABORATORY MANUAL (2ND ED.), Vols. 1-3, Cold Spring Harbor Laboratory, (1989); CURRENT PROTOCOLS IN MOLECULAR BIOLOGY, Ausubel, ed. John Wiley & Sons, Inc., New York (1997); LABORATORY TECHNIQUES IN BIOCHEMISTRY AND MOLECULAR BIOLOGY: HYBRIDIZATION WITH NUCLEIC ACID PROBES, Part I. Theory and Nucleic Acid Preparation, Tijssen, ed. Elsevier, N.Y. (1993).

Another useful means of obtaining and manipulating nucleic acids used to practice the methods of the invention is to clone from genomic samples, and, if desired, screen and re-clone inserts isolated or amplified from, e.g., genomic clones or cDNA clones. Sources of nucleic acid used in the methods of the invention include genomic or cDNA libraries contained in, e.g., mammalian artificial chromosomes (MACs), see, e.g., U.S. Patent Nos. 5,721,118; 6,025,155; human artificial chromosomes, see, e.g., Rosenfeld (1997) Nat. Genet. 15:333-335; yeast artificial chromosomes (YAC); bacterial artificial chromosomes (BAC); P1 artificial chromosomes, see, e.g., Woon (1998) Genomics 50:306-316; P1-derived vectors (PACs), see, e.g., Kern (1997) Biotechniques 23:120-124; cosmids, recombinant viruses, phages or plasmids.

In one aspect, a nucleic acid encoding a polypeptide of the invention is assembled in appropriate phase with a leader sequence capable of directing secretion of the translated polypeptide or fragment thereof.

The invention provides fusion proteins and nucleic acids encoding them. A polypeptide of the invention can be fused to a heterologous peptide or polypeptide, such as N-terminal identification peptides which impart desired characteristics, such as increased stability or simplified purification. Peptides and polypeptides of the invention can also be synthesized and expressed as fusion proteins with one or more additional domains linked thereto for, e.g., producing a more immunogenic peptide, to more readily isolate a recombinantly synthesized peptide, to identify and isolate antibodies and antibody-expressing B cells, and the like. Detection and purification facilitating domains include, e.g., metal chelating peptides such as polyhistidine tracts and histidine-tryptophan modules that allow purification on immobilized metals, protein A domains that allow purification on immobilized immunoglobulin, and the domain utilized in the FLAGS extension/ affinity purification system (Immunex Corp, Seattle WA). The inclusion of a cleavable linker sequences such as Factor Xa or enterokinase (Invitrogen, San Diego CA) between a purification domain and the motif-comprising peptide or polypeptide to facilitate purification. For example, an expression vector can include an epitope-encoding nucleic acid sequence linked to six histidine residues followed by a thioredoxin and an enterokinase cleavage site (see e.g., Williams (1995) Biochemistry 34:1787-1797; Dobeli (1998) Protein Expr. Purif. 12:404-414). The histidine residues facilitate detection and purification while the enterokinase cleavage site provides a means for purifying the epitope from the remainder of the fusion protein. Technology pertaining to vectors encoding fusion proteins and application of fusion proteins are well described in the scientific and patent literature, see e.g., Kroll (1993) DNA Cell. Biol., 12:441-53.

The invention provides "nucleic acids" or "nucleic acid sequences" that can comprise (include) an oligonucleotide, nucleotide, polynucleotide, or to a fragment of any of these, to DNA or RNA (e.g., mRNA, rRNA, tRNA, RNAi) of genomic or synthetic origin which may be single-stranded or double-stranded and may represent a sense or antisense strand, to peptide nucleic acid (PNA), or to any DNA-like or RNA-like material, natural or synthetic in origin, including, e.g., RNAi (double-stranded "interfering" RNA), ribonucleoproteins (e.g., iRNPs). The invention provides nucleic acids, i.e., oligonucleotides, containing known analogues of natural nucleotides. The invention provides nucleic-acid-like structures with synthetic backbones, see e.g., Mata (1997) Toxicol. Appl. Pharmacol. 144:189-197; Strauss-Soukup (1997) Biochemistry 36:8692-8698; Samstag (1996) Antisense Nucleic Acid Drug Dev 6:153-156.

The invention provides "genes" that can comprise (include) a nucleic acid sequence (e.g., a sequence of the invention) comprising a segment of DNA involved in producing a transcription product (e.g., a message), which in turn is translated to produce a polypeptide chain, or regulates gene transcription, reproduction or stability. Genes can include, inter alia, regions preceding and following the coding region, such as leader and trailer, promoters and enhancers, as well as, where applicable, intervening sequences (introns) between individual coding segments (exons). A "coding sequence of' or a "sequence encodes" a particular polypeptide or protein, is a nucleic acid sequence which is transcribed and translated into a polypeptide or protein when placed under the control of appropriate regulatory sequences. A promoter sequence can be "operably linked to" a coding sequence when RNA polymerase which initiates transcription at the promoter will transcribe the coding sequence into mRNA, as discussed further, below.

The term "recombinant" can mean that the nucleic acid is adjacent to a "backbone" nucleic acid to which it is not adjacent in its natural environment. In one aspect, nucleic acids represent 5% or more of the number of nucleic acid inserts in a population of nucleic acid "backbone molecules." "Backbone molecules" according to the invention include nucleic acids such as expression vectors, self-replicating nucleic acids, viruses, integrating nucleic acids, and other vectors or nucleic acids used to maintain or manipulate a nucleic acid insert of interest. In one aspect, the enriched nucleic acids represent 15%, 20%, 30%, 40%, 50%, 60%, 70%, 80%, 90% or more of the number of nucleic acid inserts in the population of recombinant backbone molecules. "Recombinant" polypeptides or proteins refer to polypeptides or proteins produced by recombinant DNA techniques; e.g., produced from cells transformed by an exogenous DNA construct encoding the desired polypeptide or protein. "Synthetic" polypeptides or protein are those prepared by chemical synthesis, as described in further detail, below.

"Oligonucleotide" can include either a single stranded polydeoxynucleotide or two complementary polydeoxynucleotide strands which may be chemically synthesized. Such synthetic oligonucleotides have no 5' phosphate and thus will not ligate to another oligonucleotide without adding a phosphate with an ATP in the presence of a kinase. A synthetic oligonucleotide will ligate to a fragment that has not been dephosphorylated. The term "variant" can include polynucleotides or polypeptides of the invention modified at one or more base pairs, codons, introns, exons, or amino acid residues (respectively) yet still retain the biological activity of a hydrolase of the invention. Variants can be produced by any number of means included methods such as, for example, error-prone PCR, shuffling, oligonucleotide-directed mutagenesis, assembly PCR, sexual PCR mutagenesis, in vivo mutagenesis, cassette mutagenesis, recursive ensemble mutagenesis, exponential ensemble mutagenesis, site-specific mutagenesis, gene reassembly, GSSM and any combination thereof. Techniques for producing variant hydrolases having activity at a pH or temperature, for example, that is different from a wild-type hydrolase, are included herein.

The term "saturation mutagenesis" or "GSSM" includes a method that uses degenerate oligonucleotide primers to introduce point mutations into a polynucleotide, as described in detail, below. The term "optimized directed evolution system" or "optimized directed evolution" includes a method for reassembling fragments of related nucleic acid sequences, e.g., related genes, and explained in detail, below. The term "synthetic ligation reassembly" or "SLR" includes a method of ligating oligonucleotide fragments in a non-stochastic fashion, and explained in detail, below.

*Transcriptional and translational control sequences*

The invention provides nucleic acid (e.g., DNA, iRNA) sequences of the invention operatively linked to expression (e.g., transcriptional or translational) control sequence(s), e.g., promoters or enhancers, to direct or modulate RNA synthesis/expression. The expression control sequence can be in an expression vector. Exemplary bacterial promoters include lacI, lacZ, T3, T7, gpt, lambda PR, PL and trp. Exemplary eukaryotic promoters include CMV immediate early, HSV thymidine kinase, early and late SV40, LTRs from retrovirus, and mouse metallothionein I.

Promoters suitable for expressing a polypeptide in bacteria include the *E. coli* lac or trp promoters, the lacI promoter, the lacZ promoter, the T3 promoter, the T7 promoter, the gpt promoter, the lambda PR promoter, the lambda PL promoter, promoters from operons encoding glycolytic enzymes such as 3-phosphoglycerate kinase (PGK), and the acid phosphatase promoter. Eukaryotic promoters include the CMV immediate early promoter, the HSV thymidine kinase promoter, heat shock promoters, the early and late SV40 promoter, LTRs from retroviruses, and the mouse metallothionein-I promoter. Other promoters known to control expression of genes in prokaryotic or eukaryotic cells or their viruses may also be used.

*Tissue-Specific Plant Promoters*

The invention provides expression cassettes that can be expressed in a tissue-specific manner, e.g., that can express a hydrolase of the invention in a tissue-specific manner. The invention also provides plants or seeds that express a hydrolase of the invention in a tissue-specific manner. The tissue-specificity can be seed specific, stem specific, leaf specific, root specific, fruit specific and the like.

The term "plant" includes whole plants, plant parts (e.g., leaves, stems, flowers, roots, etc.), plant protoplasts, seeds and plant cells and progeny of same. The class of plants which can be used in the method of the invention is generally as broad as the class of higher plants amenable to transformation techniques, including angiosperms (monocotyledonous and dicotyledonous plants), as well as gymnosperms. It includes plants of a variety of ploidy levels, including polyploid, diploid, haploid and hemizygous states. As used herein, the term "transgenic plant" includes plants or plant cells into which a heterologous nucleic acid sequence has been inserted, e.g., the nucleic acids and various recombinant constructs (e.g., expression cassettes) of the invention.

The invention provides nucleic acids comprising a sequence of the invention operably linked to a "promoter" that comprises (includes) all sequences capable of driving transcription of a coding sequence in a cell, e.g., a plant cell. Thus, promoters used in the constructs of the invention include *cis*-acting transcriptional control elements and regulatory sequences that are involved in regulating or modulating the timing and/or rate of transcription of a gene. For example, a promoter can be a *cis*-acting transcriptional control element, including an enhancer, a promoter, a transcription terminator, an origin of replication, a chromosomal integration sequence, 5' and 3' untranslated regions, or an intronic sequence, which are involved in transcriptional regulation. These cis-acting sequences typically interact with proteins or other biomolecules to carry out (turn on/off, regulate, modulate, etc.) transcription. "Constitutive" promoters are those that drive expression continuously under most environmental conditions and states of development or cell differentiation. "Inducible" or "regulatable" promoters direct expression of the nucleic acid of the invention under the influence of environmental conditions or developmental conditions. Examples of environmental conditions that may affect transcription by inducible promoters include anaerobic conditions, elevated temperature, drought, or the presence of light.

"Tissue-specific" promoters are transcriptional control elements that are only active in particular cells or tissues or organs, e.g., in plants or animals. Tissue-specific regulation may be achieved by certain intrinsic factors which ensure that genes encoding proteins specific to a given tissue are expressed. Such factors are known to exist in mammals and plants so as to allow for specific tissues to develop.

In one aspect, a constitutive promoter such as the CaMV 35S promoter can be used for expression in specific parts of the plant or seed or throughout the plant. For example, for overexpression of a hydrolase of the invention, a plant promoter fragment can be employed which will direct expression of a nucleic acid in some or all tissues of a plant, e.g., a regenerated plant. Such "constitutive" promoters and are active under most environmental conditions and states of development or cell differentiation. Examples of constitutive promoters include the cauliflower mosaic virus (CaMV) 35S transcription initiation region, the 1'- or 2'- promoter derived from T-DNA of *Agrobacterium tumefaciens*, and other transcription initiation regions from various plant genes known to those of skill. Such genes include, e.g., *ACT11* from *Arabidopsis* (Huang (1996) Plant Mol. Biol. 33:125-139); *Cat3* from *Arabidopsis* (GenBank No. U43147, Zhong (1996) Mol. Gen. Genet. 251:196-203); the gene encoding stearoyl-acyl carrier protein desaturase from *Brassica napus* (Genbank No. X74782, Solocombe (1994) Plant Physiol. 104:1167-1176); *GPc1* from maize (GenBank No. X15596; Martinez (1989) J. Mol. Biol 208:551-565); the *Gpc2* from maize (GenBank No. U45855, Manjunath (1997) Plant Mol. Biol. 33: 97-112); plant promoters described in U.S. Patent Nos. 4,962,028; 5,633,440.

The invention uses tissue-specific or constitutive promoters derived from viruses which can include, e.g., the tobamovirus subgenomic promoter (Kumagai (1995) Proc. Natl. Acad. Sci. USA 92:1679-1683; the rice tungro bacilliform virus (RTBV), which replicates only in phloem cells in infected rice plants, with its promoter which drives strong phloem-specific reporter gene expression; the cassava vein mosaic virus (CVMV) promoter, with highest activity in vascular elements, in leaf mesophyll cells, and in root tips (Verdaguer (1996) Plant Mol. Biol. 31:1129-1139).

Alternatively, the plant promoter may direct expression of a hydrolase-expressing nucleic acid in a specific tissue, organ or cell type (*i.e.* tissue-specific promoters) or may be otherwise under more precise environmental or developmental control or under the control of an inducible promoter. Examples of environmental conditions that may affect transcription include anaerobic conditions, elevated temperature, the presence of light, or sprayed with chemicals/hormones. For example, the invention incorporates the drought-inducible promoter of maize (Busk (1997) supra); the cold, drought, and high salt inducible promoter from potato (Kirch (1997) Plant Mol. Biol. 33:897 909).

Tissue-specific promoters can promote transcription only within a certain time frame of developmental stage within that tissue. See, e.g., Blazquez (1998) Plant Cell 10:791-800, characterizing the *Arabidopsis* LEAFY gene promoter. See also Cardon (1997) Plant J 12:367-77, describing the transcription factor SPL3, which recognizes a conserved sequence motif in the promoter region of the *A. thaliana* floral meristem identity gene AP1; and Mandel (1995) Plant Molecular Biology, Vol. 29, pp 995-1004, describing the meristem promoter eIF4. Tissue specific promoters which are active throughout the life cycle of a particular tissue can be used. In one aspect, the nucleic acids of the invention are operably linked to a promoter active primarily only in cotton fiber cells. In one aspect, the nucleic acids of the invention are operably linked to a promoter active primarily during the stages of cotton fiber cell elongation, e.g., as described by Rinehart (1996) supra. The nucleic acids can be operably linked to the Fb12A gene promoter to be preferentially expressed in cotton fiber cells (Ibid). See also, John (1997) Proc. Natl. Acad. Sci. USA 89:5769-5773; John, et al., U.S. Patent Nos. 5,608,148 and 5,602,321, describing cotton fiber-specific promoters and methods for the construction of transgenic cotton plants. Root-specific promoters may also be used to express the nucleic acids of the invention. Examples of root-specific promoters include the promoter from the alcohol dehydrogenase gene (DeLisle (1990) Int. Rev. Cytol. 123: 39-60). Other promoters that can be used to express the nucleic acids of the invention include, e.g., ovule-specific, embryo-specific, endosperm-specific, integument-specific, seed coat-specific promoters, or some combination thereof; a leaf-specific promoter (see, e.g., Busk (1997) Plant J. 11:1285 1295, describing a leaf-specific promoter in maize); the ORF13 promoter from *Agrobacterium rhizogenes* (which exhibits high activity in roots, see, e.g., Hansen (1997) supra); a maize pollen specific promoter (see, e.g., Guerrero (1990) Mol. Gen. Genet. 224:161 168); a tomato promoter active during fruit ripening, senescence and abscission of leaves and, to a lesser extent, of flowers can be used (see, e.g., Blume (1997) Plant J. 12:731 746); a pistil-specific promoter from the potato SK2 gene (see, e.g., Ficker (1997) Plant Mol. Biol. 35:425 431); the Blec4 gene from pea, which is active in epidermal tissue of vegetative and floral shoot apices of transgenic alfalfa making it a useful tool to target the expression of foreign genes to the epidermal layer of actively growing shoots or fibers; the ovule-specific BEL1 gene (see, e.g., Reiser (1995) Cell 83:735-742, GenBank No. U39944); and/or, the promoter in Klee, U.S. Patent No. 5,589,583, describing a plant promoter region is capable of conferring high levels of transcription in meristematic tissue and/or rapidly dividing cells.

Alternatively, plant promoters which are inducible upon exposure to plant hormones, such as auxins, are used to express the nucleic acids of the invention. For example, the invention can use the auxin-response elements E1 promoter fragment (AuxREs) in the soybean (*Glycine max* L.) (Liu (1997) Plant Physiol. 115:397-407); the auxin-responsive *Arabidopsis* GST6 promoter (also responsive to salicylic acid and hydrogen peroxide) (Chen (1996) Plant J. 10: 955-966); the auxin-inducible parC promoter from tobacco (Sakai (1996) 37:906-913); a plant biotin response element (Streit (1997) Mol. Plant Microbe Interact. 10:933-937); and, the promoter responsive to the stress hormone abscisic acid (Sheen (1996) Science 274:1900-1902).

The nucleic acids of the invention can also be operably linked to plant promoters which are inducible upon exposure to chemicals reagents which can be applied to the plant, such as herbicides or antibiotics. For example, the maize In2-2 promoter, activated by benzenesulfonamide herbicide safeners, can be used (De Veylder (1997) Plant Cell Physiol. 38:568-577); application of different herbicide safeners induces distinct gene expression patterns, including expression in the root, hydathodes, and the shoot apical meristem. Coding sequence can be under the control of, *e.g.*, a tetracycline-inducible promoter, *e.g.*, as described with transgenic tobacco plants containing the *Avena sativa* L. (oat) arginine decarboxylase gene (Masgrau (1997) Plant J. 11:465-473); or, a salicylic acid-responsive element (Stange (1997) Plant J. 11:1315-1324). Using chemically- (e.g., hormone- or pesticide-) induced promoters, *i.e.*, promoter responsive to a chemical which can be applied to the transgenic plant in the field, expression of a polypeptide of the invention can be induced at a particular stage of development of the plant. Thus, the invention also provides for transgenic plants containing an inducible gene encoding for polypeptides of the invention whose host range is limited to target plant species, such as corn, rice, barley, wheat, potato or other crops, inducible at any stage of development of the crop.

Tissue-specific plant promoters may drive expression of operably linked sequences in tissues other than the target tissue. Thus, a tissue-specific promoter is one that drives expression preferentially in the target tissue or cell type, but may also lead to some expression in other tissues as well.

The nucleic acids of the invention can also be operably linked to plant promoters which are inducible upon exposure to chemicals reagents. These reagents include, e.g., herbicides, synthetic auxins, or antibiotics which can be applied, e.g., sprayed, onto transgenic plants. Inducible expression of the hydrolase-producing nucleic acids of the invention will allow the grower to select plants with the optimal starch / sugar ratio. The development of plant parts can thus controlled. In this way the invention provides the means to facilitate the harvesting of plants and plant parts. For example, in various embodiments, the maize In2-2 promoter, activated by benzenesulfonamide herbicide safeners, is used (De Veylder (1997) Plant Cell Physiol. 38:568-577); application of different herbicide safeners induces distinct gene expression patterns, including expression in the root, hydathodes, and the shoot apical meristem. Coding sequences of the invention are also under the control of a tetracycline-inducible promoter, e.g., as described with transgenic tobacco plants containing the *Avena sativa* L. (oat) arginine decarboxylase gene (Masgrau (1997) Plant J. 11:465-473); or, a salicylic acid-responsive element (Stange (1997) Plant J. 11:1315-1324).

If proper polypeptide expression is desired, a polyadenylation region at the 3'-end of the coding region should be included. The polyadenylation region can be derived from the natural gene, from a variety of other plant genes, or from genes in the *Agrobacterial* T-DNA.

*Expression vectors and cloning vehicles*

The invention provides expression vectors and cloning vehicles comprising nucleic acids of the invention, e.g., sequences encoding the hydrolases and antibodies of the invention.

The invention provides "expression cassette" comprising a nucleotide sequence which is capable of affecting expression of a structural gene (i.e., a protein coding sequence, such as a hydrolase of the invention) in a host compatible with such sequences. Expression cassettes include at least a promoter operably linked with the polypeptide coding sequence; and, optionally, with other sequences, e.g., transcription termination signals. Additional factors necessary or helpful in effecting expression may also be used, e.g., enhancers. "Operably linked" as used herein refers to linkage of a promoter upstream from a DNA sequence such that the promoter mediates transcription of the DNA sequence. Thus, expression cassettes also include plasmids, expression vectors, recombinant viruses, any form of recombinant "naked DNA" vector, and the like. A "vector" comprises a nucleic acid which can infect, transfect, transiently or permanently transduce a cell. It will be recognized that a vector can be a naked nucleic acid, or a nucleic acid complexed with protein or lipid. The vector optionally comprises viral or bacterial nucleic acids and/or proteins, and/or membranes (e.g., a cell membrane, a viral lipid envelope, etc.). Vectors include, but are not limited to replicons (e.g., RNA replicons, bacteriophages) to which fragments of DNA may be attached and become replicated. Vectors thus include, but are not limited to RNA, autonomous self-replicating circular or linear DNA or RNA (e.g., plasmids, viruses, and the like, see, e.g., U.S. Patent No. 5,217,879), and includes both the expression and non-expression plasmids. Where a recombinant microorganism or cell culture is described as hosting an "expression vector" this includes both extra-chromosomal circular and linear DNA and DNA that has been incorporated into the host chromosome(s). Where a vector is being maintained by a host cell, the vector may either be stably replicated by the cells during mitosis as an autonomous structure, or is incorporated within the host's genome.

Expression vectors and cloning vehicles of the invention can comprise viral particles, baculovirus, phage, plasmids, phagemids, cosmids, fosmids, bacterial artificial chromosomes, viral DNA (e.g., vaccinia, adenovirus, foul pox virus, pseudorabies and derivatives of SV40), P1-based artificial chromosomes, yeast plasmids, yeast artificial chromosomes, and any other vectors specific for specific hosts of interest (such as bacillus, Aspergillus and yeast). Vectors of the invention can include chromosomal, non-chromosomal and synthetic DNA sequences. Large numbers of suitable vectors are known to those of skill in the art, and are commercially available. Exemplary vectors are include: bacterial: pQE vectors (Qiagen), pBluescript plasmids, pNH vectors, (lambda-ZAP vectors (Stratagene); ptrc99a, pKK223-3, pDR540, pRIT2T (Pharmacia); Eukaryotic: pXT1, pSG5 (Stratagene), pSVK3, pBPV, pMSG, pSVLSV40 (Pharmacia). However, any other plasmid or other vector may be used so long as they are replicable and viable in the host. Low copy number or high copy number vectors may be employed with the present invention.

The expression vector may comprise a promoter, a ribosome binding site for translation initiation and a transcription terminator. The vector may also include appropriate sequences for amplifying expression. Mammalian expression vectors can comprise an origin of replication, any necessary ribosome binding sites, a polyadenylation site, splice donor and acceptor sites, transcriptional termination sequences, and 5' flanking non-transcribed sequences. In some aspects, DNA sequences derived from the SV40 splice and polyadenylation sites may be used to provide the required non-transcribed genetic elements.

In one aspect, the expression vectors contain one or more selectable marker genes to permit selection of host cells containing the vector. Such selectable markers include genes encoding dihydrofolate reductase or genes conferring neomycin resistance for eukaryotic cell culture, genes conferring tetracycline or ampicillin resistance in E. coli, and the S. cerevisiae TRP1 gene. Promoter regions can be selected from any desired gene using chloramphenicol transferase (CAT) vectors or other vectors with selectable markers.

Vectors for expressing the polypeptide or fragment thereof in eukaryotic cells may also contain enhancers to increase expression levels. Enhancers are cis-acting elements of DNA, usually from about 10 to about 300 bp in length that act on a promoter to increase its transcription. Examples include the SV40 enhancer on the late side of the replication origin bp 100 to 270, the cytomegalovirus early promoter enhancer, the polyoma enhancer on the late side of the replication origin, and the adenovirus enhancers.

A DNA sequence may be inserted into a vector by a variety of procedures. In general, the DNA sequence is ligated to the desired position in the vector following digestion of the insert and the vector with appropriate restriction endonucleases. Alternatively, blunt ends in both the insert and the vector may be ligated. A variety of cloning techniques are known in the art, e.g., as described in Ausubel and Sambrook. Such procedures and others are deemed to be within the scope of those skilled in the art.

The vector may be in the form of a plasmid, a viral particle, or a phage. Other vectors include chromosomal, non-chromosomal and synthetic DNA sequences, derivatives of SV40; bacterial plasmids, phage DNA, baculovirus, yeast plasmids, vectors derived from combinations of plasmids and phage DNA, viral DNA such as vaccinia, adenovirus, fowl pox virus, and pseudorabies. A variety of cloning and expression vectors for use with prokaryotic and eukaryotic hosts are described by, e.g., Sambrook.

Particular bacterial vectors which may be used include the commercially available plasmids comprising genetic elements of the well known cloning vector pBR322 (ATCC 37017), pKK223-3 (Pharmacia Fine Chemicals, Uppsala, Sweden), GEM1 (Promega Biotec, Madison, WI, USA) pQE70, pQE60, pQE-9 (Qiagen), pD10, psiX174 pBluescript II KS, pNH8A, pNH16a, pNH18A, pNH46A (Stratagene), ptrc99a, pKK223-3, pKK233-3, DR540, pRIT5 (Pharmacia), pKK232-8 and pCM7. Particular eukaryotic vectors include pSV2CAT, pOG44, pXT1, pSG (Stratagene) pSVK3, pBPV, pMSG, and pSVL (Pharmacia). However, any other vector may be used as long as it is replicable and viable in the host cell.

The nucleic acids of the invention can be expressed in expression cassettes, vectors or viruses and transiently or stably expressed in plant cells and seeds. One exemplary transient expression system uses episomal expression systems, e.g., cauliflower mosaic virus (CaMV) viral RNA generated in the nucleus by transcription of an episomal minichromosome containing supercoiled DNA, see, e.g., Covey (1990) Proc. Natl. Acad. Sci. USA 87:1633-1637. Alternatively, coding sequences, i.e., all or sub-fragments of sequences of the invention can be inserted into a plant host cell genome becoming an integral part of the host chromosomal DNA. Sense or antisense transcripts can be expressed in this manner. A vector comprising the sequences (e.g., promoters or coding regions) from nucleic acids of the invention can comprise a marker gene that confers a selectable phenotype on a plant cell or a seed. For example, the marker may encode biocide resistance, particularly antibiotic resistance, such as resistance to kanamycin, G418, bleomycin, hygromycin, or herbicide resistance, such as resistance to chlorosulfuron or Basta.

Expression vectors capable of expressing nucleic acids and proteins in plants are well known in the art, and can include, *e.g.*, vectors from *Agrobacterium* spp., potato virus X (see, *e.g.*, Angell (1997) EMBO J. 16:3675-3684), tobacco mosaic virus (see, *e.g.*, Casper (1996) Gene 173:69-73), tomato bushy stunt virus (see, *e.g.*, Hillman (1989) Virology 169:42-50), tobacco etch virus (see, *e.g.*, Dolja (1997) Virology 234:243-252), bean golden mosaic virus (see, *e.g.*, Morinaga (1993) Microbiol Immunol. 37:471-476), cauliflower mosaic virus (see, *e.g.*, Cecchini (1997) Mol. Plant Microbe Interact. 10:1094-1101), maize Ac/Ds transposable element (see, *e.g.*, Rubin (1997) Mol. Cell. Biol. 17: 6294-6302; Kunze (1996) Curr. Top. Microbiol. Immunol. 204:161-194), and the maize suppressor-mutator (Spm) transposable element (see, *e.g.*, Schlappi (1996) Plant Mol. Biol. 32:717-725); and derivatives thereof.

In one aspect, the expression vector can have two replication systems to allow it to be maintained in two organisms, for example in mammalian or insect cells for expression and in a prokaryotic host for cloning and amplification. Furthermore, for integrating expression vectors, the expression vector can contain at least one sequence homologous to the host cell genome. It can contain two homologous sequences which flank the expression construct. The integrating vector can be directed to a specific locus in the host cell by selecting the appropriate homologous sequence for inclusion in the vector. Constructs for integrating vectors are well known in the art.

Expression vectors of the invention may also include a selectable marker gene to allow for the selection of bacterial strains that have been transformed, e.g., genes which render the bacteria resistant to drugs such as ampicillin, chloramphenicol, erythromycin, kanamycin, neomycin and tetracycline. Selectable markers can also include biosynthetic genes, such as those in the histidine, tryptophan and leucine biosynthetic pathways.

*Host cells and transformed cells*

The invention also provides a transformed cell comprising a nucleic acid sequence of the invention, e.g., a sequence encoding a hydrolase or an antibody of the invention, or a vector of the invention. The host cell may be any of the host cells familiar to those skilled in the art, including prokaryotic cells, eukaryotic cells, such as bacterial cells, fungal cells, yeast cells, mammalian cells, insect cells, or plant cells. Enzymes of the invention can be expressed in any host cell, e.g., any bacterial cell, any yeast cell, e.g., *Pichia pastoris, Saccharomyces cerevisiae or Schizosaccharomyces pombe*. Exemplary bacterial cells include *E. coli, Lactococcus lactis, Streptomyces, Bacillus subtilis, Bacillus cereus, Salmonella typhimurium* or any species within the genera *Bacillus, Streptomyces* and *Staphylococcus*. Exemplary insect cells include *Drosophila S2* and *Spodoptera Sf9*. Exemplary animal cells include CHO, COS or Bowes melanoma or any mouse or human cell line. The selection of an appropriate host is within the abilities of those skilled in the art. Techniques for transforming a wide variety of higher plant species are well known and described in the technical and scientific literature. See, e.g., Weising (1988) Ann. Rev. Genet. 22:421-477, U.S. Patent No. 5,750,870.

The vector may be introduced into the host cells using any of a variety of techniques, including transformation, transfection, transduction, viral infection, gene guns, or Ti-mediated gene transfer. Particular methods include calcium phosphate transfection, DEAE-Dextran mediated transfection, lipofection, or electroporation (Davis, L., Dibner, M., Battey, I., Basic Methods in Molecular Biology, (1986)).

Where appropriate, the engineered host cells can be cultured in conventional nutrient media modified as appropriate for activating promoters, selecting transformants or amplifying the genes of the invention. Following transformation of a suitable host strain and growth of the host strain to an appropriate cell density, the selected promoter may be induced by appropriate means (e.g., temperature shift or chemical induction) and the cells may be cultured for an additional period to allow them to produce the desired polypeptide or fragment thereof.

In one aspect, the nucleic acids or vectors of the invention are introduced into the cells for screening, thus, the nucleic acids enter the cells in a manner suitable for subsequent expression of the nucleic acid. The method of introduction is largely dictated by the targeted cell type. Exemplary methods include $CaPO_4$ precipitation, liposome fusion, lipofection (e.g., LIPOFECTIN™), electroporation, viral infection, etc. The candidate nucleic acids may stably integrate into the genome of the host cell (for example, with retroviral introduction) or may exist either transiently or stably in the cytoplasm (i.e. through the use of traditional plasmids, utilizing standard regulatory sequences, selection markers, etc.). As many pharmaceutically important screens require human or model mammalian cell targets, retroviral vectors capable of transfecting such targets are preferred.

Cells can be harvested by centrifugation, disrupted by physical or chemical means, and the resulting crude extract is retained for further purification. Microbial cells employed for expression of proteins can be disrupted by any convenient method, including freeze-thaw cycling, sonication, mechanical disruption, or use of cell lysing agents. Such methods are well known to those skilled in the art. The expressed polypeptide or fragment thereof can be recovered and purified from recombinant cell cultures by methods including ammonium sulfate or ethanol precipitation, acid extraction, anion or cation exchange chromatography, phosphocellulose chromatography, hydrophobic interaction chromatography, affinity chromatography, hydroxylapatite chromatography and lectin chromatography. Protein refolding steps can be used, as necessary, in completing configuration of the polypeptide. If desired, high performance liquid chromatography (HPLC) can be employed for final purification steps.

Various mammalian cell culture systems can also be employed to express recombinant protein. Examples of mammalian expression systems include the COS-7 lines of monkey kidney fibroblasts and other cell lines capable of expressing proteins from a compatible vector, such as the C127, 3T3, CHO, HeLa and BHK cell lines.

The constructs in host cells can be used in a conventional manner to produce the gene product encoded by the recombinant sequence. Depending upon the host employed in a recombinant production procedure, the polypeptides produced by host cells containing the vector may be glycosylated or may be non-glycosylated. Polypeptides of the invention may or may not also include an initial methionine amino acid residue.

Cell-free translation systems can also be employed to produce a polypeptide of the invention. Cell-free translation systems can use mRNAs transcribed from a DNA construct comprising a promoter operably linked to a nucleic acid encoding the polypeptide or fragment thereof. In some aspects, the DNA construct may be linearized prior to conducting an in vitro transcription reaction. The transcribed mRNA is then incubated with an appropriate cell-free translation extract, such as a rabbit reticulocyte extract, to produce the desired polypeptide or fragment thereof.

The expression vectors can contain one or more selectable marker genes to provide a phenotypic trait for selection of transformed host cells such as dihydrofolate reductase or neomycin resistance for eukaryotic cell culture, or such as tetracycline or ampicillin resistance in *E. coli*.

*Amplification of Nucleic Acids*

In practicing the invention, nucleic acids encoding the polypeptides of the invention, or modified nucleic acids, can be reproduced by, e.g., amplification. The invention provides amplification primer sequence pairs for amplifying nucleic acids encoding a hydrolase, e.g., an esterase, acylase, lipase, phospholipase or protease, where the primer pairs are capable of amplifying nucleic acid sequences including the exemplary SEQ ID NO:1, SEQ ID NO:3, SEQ ID NO:5, SEQ ID NO:7, SEQ ID NO:9, SEQ ID NO:11, SEQ ID NO:13, SEQ ID NO:15, SEQ ID NO:17, SEQ ID NO:19, SEQ ID NO:21, SEQ ID NO:23, SEQ ID NO:25, SEQ ID NO:27, SEQ ID NO:29, SEQ ID NO:31, SEQ ID NO:33, SEQ ID NO:35, SEQ ID NO:37, SEQ ID NO:39, SEQ ID NO:41, SEQ ID NO:43, SEQ ID NO:45, SEQ ID NO:47, SEQ ID NO:49, SEQ ID NO:51, SEQ ID NO:53, SEQ ID NO:55, SEQ ID NO:57, SEQ ID NO:59, SEQ ID NO:61, SEQ ID NO:63, SEQ ID NO:65, SEQ ID NO:67, SEQ ID NO:69, SEQ ID NO:71, SEQ ID NO:73, SEQ ID NO:75, SEQ ID NO:77, SEQ ID NO:79, SEQ ID NO:81, SEQ ID NO:83, SEQ ID NO:85, SEQ ID NO:87, SEQ ID NO:89, SEQ ID NO:91, SEQ ID NO:93, SEQ ID NO:95, SEQ ID NO:97, SEQ ID NO:99, SEQ ID NO:101, SEQ ID NO:103, SEQ ID NO:105, SEQ ID NO:107, SEQ ID NO:109, SEQ ID NO:111, SEQ ID NO:113, SEQ ID NO:115, SEQ ID NO:117, SEQ ID NO:119, SEQ ID NO:121, SEQ ID NO:123, SEQ ID NO:125, SEQ ID NO:127, SEQ ID NO:129, SEQ ID NO:131, SEQ ID NO:133, SEQ ID NO:135, SEQ ID NO:137, SEQ ID NO:139, SEQ ID NO:141, SEQ ID NO:143, SEQ ID NO:145, SEQ ID NO:147, SEQ ID NO:149, SEQ ID NO:151, SEQ ID NO:153, SEQ ID NO:155, SEQ ID NO:157, SEQ ID NO:159, SEQ ID NO:161, SEQ ID NO:163 and/or SEQ ID NO:165. One of skill in the art can design amplification primer sequence pairs for any part of or the full length of these sequences.

Amplification reactions can also be used to quantify the amount of nucleic acid in a sample (such as the amount of message in a cell sample), label the nucleic acid (e.g., to apply it to an array or a blot), detect the nucleic acid, or quantify the amount of a specific nucleic acid in a sample. In one aspect of the invention, message isolated from a cell or a cDNA library are amplified. The skilled artisan can select and design suitable oligonucleotide amplification primers. Amplification methods are also well known in the art, and include, e.g., polymerase chain reaction, PCR (see, e.g., PCR PROTOCOLS, A GUIDE TO METHODS AND APPLICATIONS, ed. Innis, Academic Press, N.Y. (1990) and PCR STRATEGIES (1995), ed. Innis, Academic Press, Inc., N.Y., ligase chain reaction (LCR) (see, e.g., Wu (1989) Genomics 4:560; Landegren (1988) Science 241:1077; Barringer (1990) Gene 89:117); transcription amplification (see, e.g., Kwoh (1989) Proc. Natl. Acad. Sci. USA 86:1173); and, self-sustained sequence replication (see, e.g., Guatelli (1990) Proc. Natl. Acad. Sci. USA 87:1874); Q Beta replicase amplification (see, e.g., Smith (1997) J. Clin. Microbiol. 35:1477-1491), automated Q-beta replicase amplification assay (see, e.g., Burg (1996) Mol. Cell. Probes 10:257-271) and other RNA polymerase mediated techniques (e.g., NASBA, Cangene, Mississauga, Ontario); see also Berger (1987) Methods Enzymol. 152:307-316; Sambrook; Ausubel; U.S. Patent Nos. 4,683,195 and 4,683,202; Sooknanan (1995) Biotechnology 13:563-564.

The invention also provides amplification primer pairs comprising sequences of the invention, for example, wherein the primer pair comprises a first member having a sequence as set forth by about the first (the 5') 12, 13, 14, 15, 16, 17, 18, 19, 20, 21, 22, 23, 24, 25, 26, 27, 28, 29, 30, 31, 32, 33, 34, 35, 36, 37, 38, 39 or 40 or more residues of a nucleic acid of the invention, and a second member having a sequence as set forth by about the first (the 5') 12, 13, 14, 15, 16, 17, 18, 19, 20, 21, 22, 23, 24, 25, 26, 27, 28, 29, 30, 31, 32, 33, 34, 35, 36, 37, 38, 39 or 40 or more residues of the complementary strand of the first member.

Determining the degree of sequence identity

The invention provides nucleic acids having at least nucleic acid, or complete (100%) sequence identity (homology) to a nucleic acid of the invention, e.g., an exemplary nucleic acid of the invention (e.g., having a sequence as set forth in SEQ ID NO:1, SEQ ID NO:3, SEQ ID NO:5, SEQ ID NO:7, SEQ ID NO:9, SEQ ID NO:11, etc.); and polypeptides having at least 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or more, or complete (100%) sequence identity (homology) to a polypeptide of the invention, e.g., an exemplary polypeptide having a sequence as set forth in SEQ ID NO:2, SEQ ID NO:4, SEQ ID NO:6, SEQ ID NO:8, SEQ ID NO:10, SEQ ID NO:12, etc. In alternative aspects, the sequence identity (homology) can be over a region of at least about 5, 10, 20, 30, 40, 50, 100, 150, 200, 250, 300, 350, 400, 450, 500, 550, 600, 650, 700, 750, 800, 850, 900, 950, 1000, or more consecutive residues, or the full length of the nucleic acid or polypeptide. The extent of sequence identity (homology) may be determined using any computer program and associated parameters, including those described herein, such as BLAST 2.2.2. or FASTA version 3.0t78, with the default parameters.

The phrase "substantially identical" in the context of two nucleic acids or polypeptides, can refer to two or more sequences that have, e.g., at least about 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or more, nucleotide or amino acid residue sequence identity (homology), when compared and aligned for maximum correspondence, as measured using one any known sequence comparison algorithm, as discussed in detail below, or by visual inspection. In alternative aspects, the invention provides nucleic acid and polypeptide sequences having substantial identity to a nucleic acid of the invention, e.g., an exemplary sequence of the invention, over a region of at least about 10, 20, 30, 40, 50, 100, 150, 200, 250, 300, 50, 400, 450, 500, 550, 600, 650, 700, 750, 800, 850, 900, 950, 1000 residues, or a region ranging from between about 50 residues to the full length of the nucleic acid or polypeptide. Nucleic acid sequences of the invention can be substantially identical over the entire length of a polypeptide coding region.

Homologous sequences also include RNA sequences in which uridines replace the thymines in the nucleic acid sequences. The homologous sequences may be obtained using any of the procedures described herein or may result from the correction of a sequencing error. It will be appreciated that the nucleic acid sequences as set forth herein can be represented in the traditional single character format (see, e.g., Stryer, Lubert. Biochemistry, 3rd Ed., W. H Freeman & Co., New York) or in any other format which records the identity of the nucleotides in a sequence.

Various sequence comparison programs identified herein are used in this aspect of the invention. Protein and/or nucleic acid sequence identities (homologies) may be evaluated using any of the variety of sequence comparison algorithms and programs known in the art. Such algorithms and programs include, but are not limited to, TBLASTN, BLASTP, FASTA, TFASTA, and CLUSTALW (Pearson and Lipman, Proc. Natl. Acad. Sci. USA 85(8):2444-2448, 1988; Altschul et al., J. Mol. Biol. 215(3):403-410, 1990; Thompson et al., Nucleic Acids Res. 22(2):4673-4680, 1994; Higgins et al., Methods Enzymol. 266:383-402, 1996; Altschul et al., J. Mol. Biol. 215(3):403-410, 1990; Altschul et al., Nature Genetics 3:266-272, 1993).

Homology or identity can be measured using sequence analysis software (e.g., Sequence Analysis Software Package of the Genetics Computer Group, University of Wisconsin Biotechnology Center, 1710 University Avenue, Madison, WI 53705). Such software matches similar sequences by assigning degrees of homology to various deletions, substitutions and other modifications. The terms "homology" and "identity" in the context of two or more nucleic acids or polypeptide sequences, refer to two or more sequences or subsequences that are the same or have a specified percentage of amino acid residues or nucleotides that are the same when compared and aligned for maximum correspondence over a comparison window or designated region as measured using any number of sequence comparison algorithms or by manual alignment and visual inspection. For sequence comparison, one sequence can act as a reference sequence (e.g., an exemplary nucleic acid or polypeptide sequence of the invention) to which test sequences are compared. When using a sequence comparison algorithm, test and reference sequences are entered into a computer, subsequence coordinates are designated, if necessary, and sequence algorithm program parameters are designated. Default program parameters can be used, or alternative parameters can be designated. The sequence comparison algorithm then calculates the percent sequence identities for the test sequences relative to the reference sequence, based on the program parameters.

A "comparison window", as used herein, includes reference to a segment of any one of the numbers of contiguous residues. For example, in alternative aspects of the invention, continugous residues ranging anywhere from 20 to the full length of an exemplary polypeptide or nucleic acid sequence of the invention, are compared to a reference sequence of the same number of contiguous positions after the two sequences are optimally aligned. If the reference sequence has the requisite sequence identity to an exemplary polypeptide or nucleic acid sequence of the invention, e.g., in alternative aspects, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or more, or complete (100%) sequence identity to an exemplary polypeptide or nucleic acid sequence of the invention, that sequence is within the scope of the invention. In alternative embodiments, subsequences ranging from about 20 to 600, about 50 to 200, and about 100 to 150 are compared to a reference sequence of the same number of contiguous positions after the two sequences are optimally aligned. Methods of alignment of sequence for comparison are well known in the art. Optimal alignment of sequences for comparison can be conducted, e.g., by the local homology algorithm of Smith & Waterman, Adv. Appl. Math. 2:482, 1981, by the homology alignment algorithm of Needleman & Wunsch, J. Mol. Biol. 48:443, 1970, by the search for similarity method of person & Lipman, Proc. Nat'l. Acad. Sci. USA 85:2444, 1988, by computerized implementations of these algorithms (GAP, BESTFIT, FASTA, and TFASTA in the Wisconsin Genetics Software Package, Genetics Computer Group, 575 Science Dr., Madison, WI), or by manual alignment and visual inspection. Other algorithms for determining homology or identity include, for example, in addition to a BLAST program (Basic Local Alignment Search Tool at the National Center for Biological Information), ALIGN, AMAS (Analysis of Multiply Aligned Sequences), AMPS (Protein Multiple Sequence Alignment), ASSET (Aligned Segment Statistical Evaluation Tool), BANDS, BESTSCOR, BIOSCAN (Biological Sequence Comparative Analysis Node), BLIMPS (BLocks IMProved Searcher), FASTA, Intervals & Points, BMB, CLUSTAL V, CLUSTAL W, CONSENSUS, LCONSENSUS, WCONSENSUS, Smith-Waterman algorithm, DARWIN, Las Vegas algorithm, FNAT (Forced Nucleotide Alignment Tool), Framealign, Framesearch, DYNAMIC, FILTER, FSAP (Fristensky Sequence Analysis Package), GAP (Global Alignment Program), GENAL, GIBBS, GenQuest, ISSC (Sensitive Sequence Comparison), LALIGN (Local Sequence Alignment), LCP (Local Content Program), MACAW (Multiple Alignment Construction & Analysis Workbench), MAP (Multiple Alignment Program), MBLKP, MBLKN, PIMA (Pattern-Induced Multi-sequence Alignment), SAGA (Sequence Alignment by Genetic Algorithm) and WHAT-IF. Such alignment programs can also be used to screen genome databases to identify polynucleotide sequences having substantially identical sequences. A number of genome databases are available, for example, a substantial portion of the human genome is available as part of the Human Genome Sequencing Project (Gibbs, 1995). Several genomes have been sequenced, e.g., M. genitalium (Fraser et al., 1995), M. jannaschii (Bult et al., 1996), H. influenzae (Fleischmann et al., 1995), E. coli (Blattner et al., 1997), and yeast (S. cerevisiae) (Mewes et al., 1997), and D. melanogaster (Adams et al., 2000). Significant progress has also been made in sequencing the genomes of model organism, such as mouse, C. elegans, and Arabadopsis sp. Databases containing genomic information annotated with some functional information are maintained by different organization, and are accessible via the internet.

BLAST, BLAST 2.0 and BLAST 2.2.2 algorithms are also used to practice the invention. They are described, e.g., in Altschul (1977) Nuc. Acids Res. 25:3389-3402; Altschul (1990) J. Mol. Biol. 215:403-410. Software for performing BLAST analyses is publicly available through the National Center for Biotechnology Information. This algorithm involves first identifying high scoring sequence pairs (HSPs) by identifying short words of length W in the query sequence, which either match or satisfy some positive-valued threshold score T when aligned with a word of the same length in a database sequence. T is referred to as the neighborhood word score threshold (Altschul (1990) supra). These initial neighborhood word hits act as seeds for initiating searches to find longer HSPs containing them. The word hits are extended in both directions along each sequence for as far as the cumulative alignment score can be increased. Cumulative scores are calculated using, for nucleotide sequences, the parameters M (reward score for a pair of matching residues; always >0). For amino acid sequences, a scoring matrix is used to calculate the cumulative score. Extension of the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T, and X determine the sensitivity and speed of the alignment. The BLASTN program (for nucleotide sequences) uses as defaults a wordlength (W) of 11, an expectation (E) of 10, M=5, N=-4 and a comparison of both strands. For amino acid sequences, the BLASTP program uses as defaults a wordlength of 3, and expectations (E) of 10, and the BLOSUM62 scoring matrix (see Henikoff & Henikoff (1989) Proc. Natl. Acad. Sci. USA 89:10915) alignments (B) of 50, expectation (E) of 10, M=5, N= -4, and a comparison of both strands. The BLAST algorithm also performs a statistical analysis of the similarity between two sequences (see, e.g., Karlin & Altschul (1993) Proc. Natl. Acad. Sci. USA 90:5873). One measure of similarity provided by BLAST algorithm is the smallest sum probability (P(N)), which provides an indication of the probability by which a match between two nucleotide or amino acid sequences would occur by chance. For example, a nucleic acid is considered similar to a references sequence if the smallest sum probability in a comparison of the test nucleic acid to the reference nucleic acid is less than about 0.2, more preferably less than about 0.01, and most preferably less than about 0.001. In one aspect, protein and nucleic acid sequence homologies are evaluated using the Basic Local Alignment Search Tool ("BLAST"). For example, five specific BLAST programs can be used to perform the following task: (1) BLASTP and BLAST3 compare an amino acid query sequence against a protein sequence database; (2) BLASTN compares a nucleotide query sequence against a nucleotide sequence database; (3) BLASTX compares the six-frame conceptual translation products of a query nucleotide sequence (both strands) against a protein sequence database; (4) TBLASTN compares a query protein sequence against a nucleotide sequence database translated in all six reading frames (both strands); and, (5) TBLASTX compares the six-frame translations of a nucleotide query sequence against the six-frame translations of a nucleotide sequence database. The BLAST programs identify homologous sequences by identifying similar segments, which are referred to herein as "high-scoring segment pairs," between a query amino or nucleic acid sequence and a test sequence which is preferably obtained from a protein or nucleic acid sequence database. High-scoring segment pairs are preferably identified (i.e., aligned) by means of a scoring matrix, many of which are known in the art. Preferably, the scoring matrix used is the BLOSUM62 matrix (Gonnet et al., Science 256:1443-1445, 1992; Henikoffand Henikoff, Proteins 17:49-61, 1993). Less preferably, the PAM or PAM250 matrices may also be used (see, e.g., Schwartz and Dayhoff, eds., 1978, Matrices for Detecting Distance Relationships: Atlas of Protein Sequence and Structure, Washington: National Biomedical Research Foundation).

In one aspect of the invention, to determine if a nucleic acid has the requisite sequence identity to be within the scope of the invention, the NCBI BLAST 2.2.2 programs is used, default options to blastp. There are about 38 setting options in the BLAST 2.2.2 program. In this exemplary aspect of the invention, all default values are used except for the default filtering setting (i.e., all parameters set to default except filtering which is set to OFF); in its place a "-F F" setting is used, which disables filtering. Use of default filtering often results in Karlin-Altschul violations due to short length of sequence.

The default values used in this exemplary aspect of the invention include:

"Filter for low complexity: ON
Word Size: 3
Matrix: Blosum62
Gap Costs: Existence:11
Extension:1"

Other default settings are: filter for low complexity OFF, word size of 3 for protein, BLOSUM62 matrix, gap existence penalty of -11 and a gap extension penalty of -1. An exemplary NCBI BLAST 2.2.2 program setting is set forth in Example 1, below. Note that the "-W" option defaults to 0. This means that, if not set, the word size defaults to 3 for proteins and 11 for nucleotides.

<u>Computer systems and computer program products</u>

To determine and identify sequence identities, structural homologies, motifs and the like *in silico*, the sequence of the invention can be stored, recorded, and manipulated on any medium which can be read and accessed by a computer. Accordingly, the invention provides computers, computer systems, computer readable mediums, computer programs products and the like recorded or stored thereon the nucleic acid and polypeptide sequences of the invention. As used herein, the terms "computer," "computer program" and "processor" are used in their broadest general contexts and incorporate all such devices, as described in detail, below. As used herein, the words "recorded" and "stored" refer to a process for storing information on a computer medium. A skilled artisan can readily adopt any known methods for recording information on a computer readable medium to generate manufactures comprising one or more of the nucleic acid and/or polypeptide sequences of the invention.

Another aspect of the invention is a computer readable medium having recorded thereon at least one nucleic acid and/or polypeptide sequence of the invention. Computer readable media include magnetically readable media, optically readable media, electronically readable media and magnetic/optical media. For example, the computer readable media may be a hard disk, a floppy disk, a magnetic tape, CD-ROM, Digital Versatile Disk (DVD), Random Access Memory (RAM), or Read Only Memory (ROM) as well as other types of other media known to those skilled in the art.

Aspects of the invention include systems (e.g., internet based systems), particularly computer systems, which store and manipulate the sequences and sequence information described herein. One example of a computer system 100 is illustrated in block diagram form in Figure 1. As used herein, "a computer system" refers to the hardware components, software components, and data storage components used to analyze a nucleotide or polypeptide sequence of the invention. The computer system 100 can include a processor for processing, accessing and manipulating the sequence data. The processor 105 can be any well-known type of central processing unit, such as, for example, the Pentium III from Intel Corporation, or similar processor from Sun, Motorola, Compaq, AMD or International Business Machines. The computer system 100 is a general purpose system that comprises the processor 105 and one or more internal data storage components 110 for storing data, and one or more data retrieving devices for retrieving the data stored on the data storage components. A skilled artisan can readily appreciate that any one of the currently available computer systems are suitable.

In one aspect, the computer system 100 includes a processor 105 connected to a bus which is connected to a main memory 115 (preferably implemented as RAM) and one or more internal data storage devices 110, such as a hard drive and/or other computer readable media having data recorded thereon. The computer system 100 can further include one or more data retrieving device 118 for reading the data stored on the internal data storage devices 110. The data retrieving device 118 may represent, for example, a floppy disk drive, a compact disk drive, a magnetic tape drive, or a modem capable of connection to a remote data storage system (e.g., via the internet) etc. In some embodiments, the internal data storage device 110 is a removable computer readable medium such as a floppy disk, a compact disk, a magnetic tape, etc. containing control logic and/or data recorded thereon. The computer system 100 may advantageously include or be programmed by appropriate software for reading the control logic and/or the data from the data storage component once inserted in the data retrieving device. The computer system 100 includes a display 120 which is used to display output to a computer user. It should also be noted that the computer system 100 can be linked to other computer systems 125a-c in a network or wide area network to provide centralized access to the computer system 100. Software for accessing and processing the nucleotide or amino acid sequences of the invention can reside in main memory 115 during execution. In some aspects, the computer system 100 may further comprise a sequence comparison algorithm for comparing a nucleic acid sequence of the invention. The algorithm and sequence(s) can be stored on a computer readable medium. A "sequence comparison algorithm" refers to one or more programs which are implemented (locally or remotely) on the computer system 100 to compare a nucleotide sequence with other nucleotide sequences and/or compounds stored within a data storage means. For example, the sequence comparison algorithm may compare the nucleotide sequences of the invention stored on a computer readable medium to reference sequences stored on a computer readable medium to identify homologies or structural motifs.

The parameters used with the above algorithms may be adapted depending on the sequence length and degree of homology studied. In some aspects, the parameters may be the default parameters used by the algorithms in the absence of instructions from the user. Figure 2 is a flow diagram illustrating one aspect of a process 200 for comparing a new nucleotide or protein sequence with a database of sequences in order to determine the homology levels between the new sequence and the sequences in the database. The database of sequences can be a private database stored within the computer system 100, or a public database such as GENBANK that is available through the Internet. The process 200 begins at a start state 201 and then moves to a state 202 wherein the new sequence to be compared is stored to a memory in a computer system 100. As discussed above, the memory could be any type of memory, including RAM or an internal storage device. The process 200 then moves to a state 204 wherein a database of sequences is opened for analysis and comparison. The process 200 then moves to a state 206 wherein the first sequence stored in the database is read into a memory on the computer. A comparison is then performed at a state 210 to determine if the first sequence is the same as the second sequence. It is important to note that this step is not limited to performing an exact comparison between the new sequence and the first sequence in the database. Well-known methods are known to those of skill in the art for comparing two nucleotide or protein sequences, even if they are not identical. For example, gaps can be introduced into one sequence in order to raise the homology level between the two tested sequences. The parameters that control whether gaps or other features are introduced into a sequence during comparison are normally entered by the user of the computer system. Once a comparison of the two sequences has been performed at the state 210, a determination is made at a decision state 210 whether the two sequences are the same. Of course, the term "same" is not limited to sequences that are absolutely identical. Sequences that are within the homology parameters entered by the user will be marked as "same" in the process 200. If a determination is made that the two sequences are the same, the process 200 moves to a state 214 wherein the name of the sequence from the database is displayed to the user. This state notifies the user that the sequence with the displayed name fulfills the homology constraints that were entered. Once the name of the stored sequence is displayed to the user, the process 200 moves to a decision state 218 wherein a determination is made whether more sequences exist in the database. If no more sequences exist in the database, then the process 200 terminates at an end state 220. However, if more sequences do exist in the database, then the process 200 moves to a state 224 wherein a pointer is moved to the next sequence in the database so that it can be compared to the new sequence. In this manner, the new sequence is aligned and compared with every sequence in the database. It should be noted that if a determination had been made at the decision state 212 that the sequences were not homologous, then the process 200 would move immediately to the decision state 218 in order to determine if any other sequences were available in the database for comparison. Accordingly, one aspect of the invention is a computer system comprising a processor, a data storage device having stored thereon a nucleic acid sequence of the invention and a sequence comparer for conducting the comparison. The sequence comparer may indicate a homology level between the sequences compared or identify structural motifs, or it may identify structural motifs in sequences which are compared to these nucleic acid codes and polypeptide codes. Figure 3 is a flow diagram illustrating one embodiment of a process 250 in a computer for determining whether two sequences are homologous. The process 250 begins at a start state 252 and then moves to a state 254 wherein a first sequence to be compared is stored to a memory. The second sequence to be compared is then stored to a memory at a state 256. The process 250 then moves to a state 260 wherein the first character in the first sequence is read and then to a state 262 wherein the first character of the second sequence is read. It should be understood that if the sequence is a nucleotide sequence, then the character would normally be either A, T, C, G or U. If the sequence is a protein sequence, then it can be a single letter amino acid code so that the first and sequence sequences can be easily compared. A determination is then made at a decision state 264 whether the two characters are the same. If they are the same, then the process 250 moves to a state 268 wherein the next characters in the first and second sequences are read. A determination is then made whether the next characters are the same. If they are, then the process 250 continues this loop until two characters are not the same. If a determination is made that the next two characters are not the same, the process 250 moves to a decision state 274 to determine whether there are any more characters either sequence to read. If there are not any more characters to read, then the process 250 moves to a state 276 wherein the level of homology between the first and second sequences is displayed to the user. The level of homology is determined by calculating the proportion of characters between the sequences that were the same out of the total number of sequences in the first sequence. Thus, if every character in a first 100 nucleotide sequence aligned with an every character in a second sequence, the homology level would be 100%.

Alternatively, the computer program can compare a reference sequence to a sequence of the invention to determine whether the sequences differ at one or more positions. The program can record the length and identity of inserted, deleted or substituted nucleotides or amino acid residues with respect to the sequence of either the reference or the invention. The computer program may be a program which determines whether a reference sequence contains a single nucleotide polymorphism (SNP) with respect to a sequence of the invention, or, whether a sequence of the invention comprises a SNP of a known sequence. Thus, in some aspects, the computer program is a program which identifies SNPs. The method may be implemented by the computer systems described above and the method illustrated in Figure 3. The method can be performed by reading a sequence of the invention and the reference sequences through the use of the computer program and identifying differences with the computer program.

In other aspects the computer based system comprises an identifier for identifying features within a nucleic acid or polypeptide of the invention. An "identifier" refers to one or more programs which identifies certain features within a nucleic acid sequence. For example, an identifier may comprise a program which identifies an open reading frame (ORF) in a nucleic acid sequence. Figure 4 is a flow diagram illustrating one aspect of an identifier process 300 for detecting the presence of a feature in a sequence. The process 300 begins at a start state 302 and then moves to a state 304 wherein a first sequence that is to be checked for features is stored to a memory 115 in the computer system 100. The process 300 then moves to a state 306 wherein a database of sequence features is opened. Such a database would include a list of each feature's attributes along with the name of the feature. For example, a feature name could be "Initiation Codon" and the attribute would be "ATG". Another example would be the feature name "TAATAA Box" and the feature attribute would be "TAATAA". An example of such a database is produced by the University of Wisconsin Genetics Computer Group. Alternatively, the features may be structural polypeptide motifs such as alpha helices, beta sheets, or functional polypeptide motifs such as enzymatic active sites, helix-turn-helix motifs or other motifs known to those skilled in the art. Once the database of features is opened at the state 306, the process 300 moves to a state 308 wherein the first feature is read from the database. A comparison of the attribute of the first feature with the first sequence is then made at a state 310. A determination is then made at a decision state 316 whether the attribute of the feature was found in the first sequence. If the attribute was found, then the process 300 moves to a state 318 wherein the name of the found feature is displayed to the user. The process 300 then moves to a decision state 320 wherein a determination is made whether move features exist in the database. If no more features do exist, then the process 300 terminates at an end state 324. However, if more features do exist in the database, then the process 300 reads the next sequence feature at a state 326 and loops back to the state 310 wherein the attribute of the next feature is compared against the first sequence. If the feature attribute is not found in the first sequence at the decision state 316, the process 300 moves directly to the decision state 320 in order to determine if any more features exist in the database. Thus, in one aspect, the invention provides a computer program that identifies open reading frames (ORFs).

A polypeptide or nucleic acid sequence of the invention may be stored and manipulated in a variety of data processor programs in a variety of formats. For example, a sequence can be stored as text in a word processing file, such as MicrosoftWORD or WORDPERFECT or as an ASCII file in a variety of database programs familiar to those of skill in the art, such as DB2, SYBASE, or ORACLE. In addition, many computer programs and databases may be used as sequence comparison algorithms, identifiers, or sources of reference nucleotide sequences or polypeptide sequences to be compared to a nucleic acid sequence of the invention. The programs and databases used to practice the invention include, but are not limited to: MacPattern (EMBL), DiscoveryBase (Molecular Applications Group), GeneMine (Molecular Applications Group), Look (Molecular Applications Group), MacLook (Molecular Applications Group), BLAST and BLAST2 (NCBI), BLASTN and BLASTX (Altschul et al, J. Mol. Biol. 215: 403, 1990), FASTA (Pearson and Lipman, Proc. Natl. Acad. Sci. USA, 85: 2444, 1988), FASTDB (Brutlag et al. Comp. App. Biosci. 6:237-245, 1990), Catalyst (Molecular Simulations Inc.), Catalyst/SHAPE (Molecular Simulations Inc.), Cerius2.DBAccess (Molecular Simulations Inc.), HypoGen (Molecular Simulations Inc.), Insight II, (Molecular Simulations Inc.), Discover (Molecular Simulations Inc.), CHARMm (Molecular Simulations Inc.), Felix (Molecular Simulations Inc.), DelPhi, (Molecular Simulations Inc.), QuanteMM, (Molecular Simulations Inc.), Homology (Molecular Simulations Inc.), Modeler (Molecular Simulations Inc.), ISIS (Molecular Simulations Inc.), Quanta/Protein Design (Molecular Simulations Inc.), WebLab (Molecular Simulations Inc.), WebLab Diversity Explorer (Molecular Simulations Inc.), Gene Explorer (Molecular Simulations Inc.), SeqFold (Molecular Simulations Inc.), the MDL Available Chemicals Directory database, the MDL Drug Data Report data base, the Comprehensive Medicinal Chemistry database, Derwent's World Drug Index database, the BioByteMasterFile database, the Genbank database, and the Genseqn database. Many other programs and data bases would be apparent to one of skill in the art given the present disclosure.

Motifs which may be detected using the above programs include sequences encoding leucine zippers, helix-turn-helix motifs, glycosylation sites, ubiquitination sites, alpha helices, and beta sheets, signal sequences encoding signal peptides which direct the secretion of the encoded proteins, sequences implicated in transcription regulation such as homeoboxes, acidic stretches, enzymatic active sites, substrate binding sites, and enzymatic cleavage sites.

Hybridization of nucleic acids

The invention provides isolated, synthetic or recombinant nucleic acids that hybridize under stringent conditions to nucleic acid of the invention, e.g., an exemplary sequence of the invention, e.g., a sequence as set forth in SEQ ID NO:1, SEQ ID NO:3, SEQ ID NO:5, SEQ ID NO:7, SEQ ID NO:9, SEQ ID NO:11, etc., and subsequences thereof, or a nucleic acid that encodes a polypeptide of the invention. The stringent conditions can be highly stringent conditions, medium stringent conditions, low stringent conditions, including the high and reduced stringency conditions described herein.

In alternative embodiments, nucleic acids of the invention as defined by their ability to hybridize under stringent conditions can be between about five residues and the full length of nucleic acid of the invention; e.g., they can be at least 5, 10, 15, 20, 25, 30, 35, 40, 50, 55, 60, 65, 70, 75, 80, 90, 100, 150, 200, 250, 300, 350, 400, 450, 500, 550, 600, 650, 700, 750, 800, 850, 900, 950, 1000, or more, residues in length. Nucleic acids shorter than full length are also included. These nucleic acids can be useful as, e.g., hybridization probes, labeling probes, PCR oligonucleotide probes, iRNA, antisense or sequences encoding antibody binding peptides (epitopes), motifs, active sites and the like.

In one aspect, nucleic acids of the invention are defined by their ability to hybridize under high stringency comprises conditions of about 50% formamide at about 37°C to 42°C. In one aspect, nucleic acids of the invention are defined by their ability to hybridize under reduced stringency comprising conditions in about 35% to 25% formamide at about 30°C to 35°C.

Alternatively, nucleic acids of the invention are defined by their ability to hybridize under high stringency comprising conditions at 42°C in 50% formamide, 5X SSPE, 0.3% SDS, and a repetitive sequence blocking nucleic acid, such as cos-1 or salmon sperm DNA (e.g., 200 n/ml sheared and denatured salmon sperm DNA). In one aspect, nucleic acids of the invention are defined by their ability to hybridize under reduced stringency conditions comprising 35% formamide at a reduced temperature of 35°C.

Following hybridization, the filter may be washed with 6X SSC, 0.5% SDS at 50°C. These conditions are considered to be "moderate" conditions above 25% formamide and "low" conditions below 25% formamide. A specific example of "moderate" hybridization conditions is when the above hybridization is conducted at 30% formamide. A specific example of "low stringency"'hybridization conditions is when the above hybridization is conducted at 10% formamide.

The temperature range corresponding to a particular level of stringency can be further narrowed by calculating the purine to pyrimidine ratio of the nucleic acid of interest and adjusting the temperature accordingly. Nucleic acids of the invention are also defined by their ability to hybridize under high, medium, and low stringency conditions as set forth in Ausubel and Sambrook. Variations on the above ranges and conditions are well known in the art. Hybridization conditions are discussed further, below.

The above procedure may be modified to identify nucleic acids having decreasing levels of homology to the probe sequence. For example, to obtain nucleic acids of decreasing homology to the detectable probe, less stringent conditions may be used. For example, the hybridization temperature may be decreased in increments of 5°C from 68°C to 42°C in a hybridization buffer having a $Na^+$ concentration of approximately 1M. Following hybridization, the filter may be washed with 2X SSC, 0.5% SDS at the temperature of hybridization. These conditions are considered to be "moderate" conditions above 50°C and "low" conditions below 50°C. A specific example of "moderate" hybridization conditions is when the above hybridization is conducted at 55°C. A specific example of "low stringency" hybridization conditions is when the above hybridization is conducted at 45°C.

Alternatively, the hybridization may be carried out in buffers, such as 6X SSC, containing formamide at a temperature of 42°C. In this case, the concentration of formamide in the hybridization buffer may be reduced in 5% increments from 50% to 0% to identify clones having decreasing levels of homology to the probe. Following hybridization, the filter may be washed with 6X SSC, 0.5% SDS at 50°C. These conditions are considered to be "moderate" conditions above 25% formamide and "low" conditions below 25% formamide. A specific example of "moderate" hybridization conditions is when the above hybridization is conducted at 30% formamide. A specific example of "low stringency" hybridization conditions is when the above hybridization is conducted at 10% formamide.

However, the selection of a hybridization format is not critical - it is the stringency of the wash conditions that set forth the conditions which determine whether a nucleic acid is within the scope of the invention. Wash conditions used to identify nucleic acids within the scope of the invention include, e.g.: a salt concentration of about 0.02 molar at pH 7 and a temperature of at least about 50°C or about 55°C to about 60°C; or, a salt concentration of about 0.15 M NaCl at 72°C for about 15 minutes; or, a salt concentration of about 0.2X SSC at a temperature of at least about 50°C or about 55°C to about 60°C for about 15 to about 20 minutes; or, the hybridization complex is washed twice with a solution with a salt concentration of about 2X SSC containing 0.1% SDS at room temperature for 15 minutes and then washed twice by 0.1X SSC containing 0.1% SDS at 68oC for 15 minutes; or, equivalent conditions. See Sambrook, Tijssen and Ausubel for a description of SSC buffer and equivalent conditions.

These methods may be used to isolate nucleic acids of the invention.

Oligonucleotides probes and methods for using them

The invention also provides nucleic acid probes for identifying nucleic acids encoding a polypeptide with a hydrolase activity, e.g., an esterase, acylase, lipase, phospholipase or protease activity. In one aspect, the probe comprises at least 10 consecutive bases of a nucleic acid of the invention. Alternatively, a probe of the invention can be at least about 5, 6, 7, 8, 9, 10, 15, 20, 25, 30, 35, 40, 45, 50, 60, 70, 80, 90, 100, 110, 120, 130, 150, 160, 170, 180, 190, 200 or more, or about 10 to 50, about 20 to 60 about 30 to 70, consecutive bases of a sequence as set forth in a nucleic acid of the invention. The probes identify a nucleic acid by binding and/or hybridization. The probes can be used in arrays of the invention, see discussion below, including, e.g., capillary arrays. The probes of the invention can also be used to isolate other nucleic acids or polypeptides.

The probes of the invention can be used to determine whether a biological sample, such as a soil sample, contains an organism having a nucleic acid sequence of the invention (e.g., a hydrolase-encoding nucleic acid) or an organism from which the nucleic acid was obtained. In such procedures, a biological sample potentially harboring the organism from which the nucleic acid was isolated is obtained and nucleic acids are obtained from the sample. The nucleic acids are contacted with the probe under conditions which permit the probe to specifically hybridize to any complementary sequences present in the sample. Where necessary, conditions which permit the probe to specifically hybridize to complementary sequences may be determined by placing the probe in contact with complementary sequences from samples known to contain the complementary sequence, as well as control sequences which do not contain the complementary sequence. Hybridization conditions, such as the salt concentration of the hybridization buffer, the formamide concentration of the hybridization buffer, or the hybridization temperature, may be varied to identify conditions which allow the probe to hybridize specifically to complementary nucleic acids (see discussion on specific hybridization conditions).

If the sample contains the organism from which the nucleic acid was isolated, specific hybridization of the probe is then detected. Hybridization may be detected by labeling the probe with a detectable agent such as a radioactive isotope, a fluorescent dye or an enzyme capable of catalyzing the formation of a detectable product. Many methods for using the labeled probes to detect the presence of complementary nucleic acids in a sample are familiar to those skilled in the art. These include Southern Blots, Northern Blots, colony hybridization procedures, and dot blots. Protocols for each of these procedures are provided in Ausubel and Sambrook.

Alternatively, more than one probe (at least one of which is capable of specifically hybridizing to any complementary sequences which are present in the nucleic acid sample), may be used in an amplification reaction to determine whether the sample contains an organism containing a nucleic acid sequence of the invention (e.g., an organism from which the nucleic acid was isolated). In one aspect, the probes comprise oligonucleotides. In one aspect, the amplification reaction may comprise a PCR reaction. PCR protocols are described in Ausubel and Sambrook (see discussion on amplification reactions). In such procedures, the nucleic acids in the sample are contacted with the probes, the amplification reaction is performed, and any resulting amplification product is detected. The amplification product may be detected by performing gel electrophoresis on the reaction products and staining the gel with an intercalator such as ethidium bromide. Alternatively, one or more of the probes may be labeled with a radioactive isotope and the presence of a radioactive amplification product may be detected by autoradiography after gel electrophoresis.

Probes derived from sequences near the 3' or 5' ends of a nucleic acid sequence of the invention can also be used in chromosome walking procedures to identify clones containing additional, e.g., genomic sequences. Such methods allow the isolation of genes which encode additional proteins of interest from the host organism.

In one aspect, nucleic acid sequences of the invention are used as probes to identify and isolate related nucleic acids. In some aspects, the so-identified related nucleic acids may be cDNAs or genomic DNAs from organisms other than the one from which the nucleic acid of the invention was first isolated. In such procedures, a nucleic acid sample is contacted with the probe under conditions which permit the probe to specifically hybridize to related sequences. Hybridization of the probe to nucleic acids from the related organism is then detected using any of the methods described above.

"Hybridization" refers to the process by which a nucleic acid strand joins with a complementary strand through base pairing. Hybridization reactions can be sensitive and selective so that a particular sequence of interest can be identified even in samples in which it is present at low concentrations. Stringent conditions can be defined by, for example, the concentrations of salt or formamide in the prehybridization and hybridization solutions, or by the hybridization temperature, and are well known in the art. For example, stringency can be increased by reducing the concentration of salt, increasing the concentration of formamide, or raising the hybridization temperature, altering the time of hybridization, as described in detail, below. In alternative aspects, nucleic acids of the invention are defined by their ability to hybridize under various stringency conditions (e.g., high, medium, and low), as set forth herein.

In nucleic acid hybridization reactions, the conditions used to achieve a particular level of stringency will vary, depending on the nature of the nucleic acids being hybridized. For example, the length, degree of complementarity, nucleotide sequence composition (e.g., GC v. AT content), and nucleic acid type (e.g., RNA v. DNA) of the hybridizing regions of the nucleic acids can be considered in selecting hybridization conditions. An additional consideration is whether one of the nucleic acids is immobilized, for example, on a filter. Hybridization may be carried out under conditions of low stringency, moderate stringency or high stringency. As an example of nucleic acid hybridization, a polymer membrane containing immobilized denatured nucleic acids is first prehybridized for 30 minutes at 45°C in a solution consisting of 0.9 M NaCl, 50 mM $NaH_2PO4$, pH 7.0, 5.0 mM $Na_2EDTA$, 0.5% SDS, 10X Denhardt's, and 0.5 mg/ml polyriboadenylic acid. Approximately 2 X 107 cpm (specific activity 4-9 X 108 cpm/ug) of 32P end-labeled oligonucleotide probe are then added to the solution. After 12-16 hours of incubation, the membrane is washed for 30 minutes at room temperature (RT) in 1X SET (150 mM NaCl, 20 mM Tris hydrochloride, pH 7.8, 1 mM Na2EDTA) containing 0.5% SDS, followed by a 30 minute wash in fresh 1X SET at Tm-10°C for the oligonucleotide probe. The membrane is then exposed to autoradiographic film for detection of hybridization signals.

By varying the stringency of the hybridization conditions used to identify nucleic acids, such as cDNAs or genomic DNAs, which hybridize to the detectable probe, nucleic acids having different levels of homology to the probe can be identified and isolated. Stringency may be varied by conducting the hybridization at varying temperatures below the melting temperatures of the probes. The melting temperature, Tm, is the temperature (under defined ionic strength and pH) at which 50% of the target sequence hybridizes to a perfectly complementary probe. Very stringent conditions are selected to be equal to or about 5°C lower than the Tm for a particular probe. The melting temperature of the probe may be calculated using the following exemplary formulas. For probes between 14 and 70 nucleotides in length the melting temperature (Tm) is calculated using the formula: Tm=81.5+16.6(log [Na+])+0.41 (fraction G+C)-(600/N) where N is the length of the probe. If the hybridization is carried out in a solution containing formamide, the melting temperature may be calculated using the equation: Tm=81.5+16.6(log [Na+])+0.41(fraction G+C)-(0.63% formamide)-(600/N) where N is the length of the probe. Prehybridization may be carried out in 6X SSC, 5X Denhardt's reagent, 0.5% SDS, 100 μg denatured fragmented salmon sperm DNA or 6X SSC, 5X Denhardt's reagent, 0.5% SDS, 100 μg denatured fragmented salmon sperm DNA, 50% formamide. Formulas for SSC and Denhardt's and other solutions are listed, e.g., in Sambrook.

In one aspect, hybridization is conducted by adding the detectable probe to the prehybridization solutions listed above. Where the probe comprises double stranded DNA, it is denatured before addition to the hybridization solution. The filter is contacted with the hybridization solution for a sufficient period of time to allow the probe to hybridize to cDNAs or genomic DNAs containing sequences complementary thereto or homologous thereto. For probes over 200 nucleotides in length, the hybridization may be carried out at 15-25°C below the Tm. For shorter probes, such as oligonucleotide probes, the hybridization may be conducted at 5-10°C below the Tm. In one aspect, hybridizations in 6X SSC are conducted at approximately 68°C. In one aspect, hybridizations in 50% formamide containing solutions are conducted at approximately 42°C. All of the foregoing hybridizations would be considered to be under conditions of high stringency.

In one aspect, following hybridization, the filter is washed to remove any non-specifically bound detectable probe. The stringency used to wash the filters can also be varied depending on the nature of the nucleic acids being hybridized, the length of the nucleic acids being hybridized, the degree of complementarity, the nucleotide sequence composition (e.g., GC v. AT content), and the nucleic acid type (e.g., RNA v. DNA). Examples of progressively higher stringency condition washes are as follows: 2X SSC, 0.1% SDS at room temperature for 15 minutes (low stringency); 0.1X SSC, 0.5% SDS at room temperature for 30 minutes to I hour (moderate stringency); 0.1X SSC, 0.5% SDS for 15 to 30 minutes at between the hybridization temperature and 68°C (high stringency); and 0.15M NaCl for 15 minutes at 72°C (very high stringency). A final low stringency wash can be conducted in 0.1X SSC at room temperature. The examples above are merely illustrative of one set of conditions that can be used to wash filters. One of skill in the art would know that there are numerous recipes for different stringency washes.

Nucleic acids which have hybridized to the probe can be identified by autoradiography or other conventional techniques. The above procedure may be modified to identify nucleic acids having decreasing levels of homology to the probe sequence. For example, to obtain nucleic acids of decreasing homology to the detectable probe, less stringent conditions may be used. For example, the hybridization temperature may be decreased in increments of 5°C from 68°C to 42°C in a hybridization buffer having a Na+ concentration of approximately 1M. Following hybridization, the filter may be washed with 2X SSC, 0.5% SDS at the temperature of hybridization. These conditions are considered to be "moderate" conditions above 50°C and "low" conditions below 50°C. An example of "moderate" hybridization conditions is when the above hybridization is conducted at 55°C. An example of "low stringency" hybridization conditions is when the above hybridization is conducted at 45°C.

Alternatively, the hybridization may be carried out in buffers, such as 6X SSC, containing formamide at a temperature of 42°C. In this case, the concentration of formamide in the hybridization buffer may be reduced in 5% increments from 50% to 0% to identify clones having decreasing levels of homology to the probe. Following hybridization, the filter may be washed with 6X SSC, 0.5% SDS at 50°C. These conditions are considered to be "moderate" conditions above 25% formamide and "low" conditions below 25% formamide. A specific example of "moderate" hybridization conditions is when the above hybridization is conducted at 30% formamide. A specific example of "low stringency" hybridization conditions is when the above hybridization is conducted at 10% formamide.

These probes and methods of the invention can be used to isolate, or identify (e.g., using an array), nucleic acids having a sequence with at least about 950%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or more, sequence identity to a nucleic acid sequence of the invention comprising at least about 10, 15, 20, 25, 30, 35, 40, 50, 75, 100, 150, 200, 250, 300, 350, 400, 500, 550, 600, 650, 700, 750, 800, 850, 900, 950, 1000, or more consecutive bases thereof, and the sequences complementary thereto. Homology may be measured using an alignment algorithm, as discussed herein. For example, the homologous polynucleotides may have a coding sequence which is a naturally occurring allelic variant of one of the coding sequences described herein. Such allelic variants may have a substitution, deletion or addition of one or more nucleotides when compared to a nucleic acid of the invention.

Additionally, the probes and methods of the invention may be used to isolate, or identify (e.g., using an array), nucleic acids which encode polypeptides having at least about 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or more sequence identity (homology) to a polypeptide of the invention comprising at least 5, 10, 15, 20, 25, 30, 35, 40, 50, 75, 100, or 150 or more consecutive amino acids thereof as determined using a sequence alignment algorithm, e.g., such as the FASTA version 3.0t78 algorithm with the default parameters, or a BLAST 2.2.2 program with exemplary settings as set forth herein.

Inhibiting Expression of Hydrolases

The invention further provides for nucleic acids complementary to (e.g., antisense sequences to) the nucleic acid sequences of the invention, e.g., hydrolase-encoding sequences. Antisense sequences are capable of inhibiting the transport, splicing or transcription of hydrolase-encoding genes. The inhibition can be effected through the targeting of genomic DNA or messenger RNA. The inhibition can be effected using DNA, e.g., an inhibitory ribozyme, or an RNA, e.g., a double-stranded iRNA, comprising a sequence of the invention. The transcription or function of targeted nucleic acid can be inhibited, for example, by hybridization and/or cleavage. The invention provides a set of inhibitors comprising oligonucleotides capable of binding hydrolase gene and/or message, in either case preventing or inhibiting the production or function of hydrolase. The association can be through sequence specific hybridization. Another useful class of inhibitors includes oligonucleotides which cause inactivation or cleavage of hydrolase message. The oligonucleotide can have enzyme activity which causes such cleavage, such as ribozymes. The oligonucleotide can be chemically modified or conjugated to an enzyme or composition capable of cleaving the complementary nucleic acid. One may screen a pool of many different such oligonucleotides for those with the desired activity.

*Antisense Oligonucleotides*

The invention provides antisense oligonucleotides capable of binding hydrolase message which can inhibit hydrolase activity by targeting mRNA or genomic DNA. Strategies for designing antisense oligonucleotides are well described in the scientific and patent literature, and the skilled artisan can design such hydrolase oligonucleotides using the novel reagents of the invention. For example, gene walking/ RNA mapping protocols to screen for effective antisense oligonucleotides are well known in the art, see, e.g., Ho (2000) Methods Enzymol. 314:168-183, describing an RNA mapping assay, which is based on standard molecular techniques to provide an easy and reliable method for potent antisense sequence selection. See also Smith (2000) Eur. J. Pharm. Sci. 11:191-198.

In one aspect, recombinantly generated, or, isolated naturally occurring nucleic acids are used as antisense oligonucleotides. The antisense oligonucleotides can be of any length; for example, in alternative aspects, the antisense oligonucleotides are between about 5 to 100, about 10 to 80, about 15 to 60, about 18 to 40. The antisense oligonucleotides can be single stranded or double-stranded RNA or DNA. The optimal length can be determined by routine screening. The antisense oligonucleotides can be present at any concentration. The optimal concentration can be determined by routine screening. A wide variety of synthetic, non-naturally occurring nucleotide and nucleic acid analogues are known which can address this potential problem. For example, peptide nucleic acids (PNAs) containing non-ionic backbones, such as N-(2-aminoethyl) glycine units can be used. Antisense oligonucleotides having phosphorothioate linkages can also be used, as described in WO 97/03211; WO 96/39154; Mata (1997) Toxicol Appl Pharmacol 144:189-197; Antisense Therapeutics, ed. Agrawal (Humana Press, Totowa, N.J., 1996). Antisense oligonucleotides having synthetic DNA backbone analogues provided by the invention can also include phosphoro-dithioate, methylphosphonate, phosphoramidate, alkyl phosphotriester, sulfamate, 3'-thioacetal, methylene(methylimino), 3'-N-carbamate, and morpholino carbamate nucleic acids, as described above.

Combinatorial chemistry methodology can be used to create vast numbers of oligonucleotides that can be rapidly screened for specific oligonucleotides that have appropriate binding affinities and specificities toward any target, such as the sense and antisense hydrolase sequences of the invention (see, e.g., Gold (1995) J. of Biol. Chem. 270: 13581-13584).

*Inhibitory Ribozymes*

The invention provides for with ribozymes capable of binding hydrolase message that can inhibit hydrolase activity by targeting mRNA. Strategies for designing ribozymes and selecting the hydrolase-specific antisense sequence for targeting are well described in the scientific and patent literature, and the skilled artisan can design such ribozymes using the novel reagents of the invention. Ribozymes act by binding to a target RNA through the target RNA binding portion of a ribozyme which is held in close proximity to an enzymatic portion of the RNA that cleaves the target RNA. Thus, the ribozyme recognizes and binds a target RNA through complementary basepairing, and once bound to the correct site, acts enzymatically to cleave and inactivate the target RNA. Cleavage of a target RNA in such a manner will destroy its ability to direct synthesis of an encoded protein if the cleavage occurs in the coding sequence. After a ribozyme has bound and cleaved its RNA target, it is typically released from that RNA and so can bind and cleave new targets repeatedly.

In some circumstances, the enzymatic nature of a ribozyme can be advantageous over other technologies, such as antisense technology (where a nucleic acid molecule simply binds to a nucleic acid target to block its transcription, translation or association with another molecule) as the effective concentration of ribozyme necessary to effect a therapeutic treatment can be lower than that of an antisense oligonucleotide. This potential advantage reflects the ability of the ribozyme to act enzymatically. Thus, a single ribozyme molecule is able to cleave many molecules of target RNA. In addition, a ribozyme is typically a highly specific inhibitor, with the specificity of inhibition depending not only on the base pairing mechanism of binding, but also on the mechanism by which the molecule inhibits the expression of the RNA to which it binds. That is, the inhibition is caused by cleavage of the RNA target and so specificity is defined as the ratio of the rate of cleavage of the targeted RNA over the rate of cleavage of non-targeted RNA. This cleavage mechanism is dependent upon factors additional to those involved in base pairing. Thus, the specificity of action of a ribozyme can be greater than that of antisense oligonucleotide binding the same RNA site.

The enzymatic ribozyme RNA molecule can be formed in a hammerhead motif, but may also be formed in the motif of a hairpin, hepatitis delta virus, group I intron or RnaseP-like RNA (in association with an RNA guide sequence). Examples of such hammerhead motifs are described by Rossi (1992) Aids Research and Human Retroviruses 8:183; hairpin motifs by Hampel (1989) Biochemistry 28:4929, and Hampel (1990) Nuc. Acids Res. 18:299; the hepatitis delta virus motif by Perrotta (1992) Biochemistry 31:16; the RNaseP motif by Guerrier-Takada (1983) Cell 35:849; and the group I intron by Cech U.S. Pat. No. 4,987,071. The recitation of these specific motifs is not intended to be limiting; those skilled in the art will recognize that an enzymatic RNA molecule of this invention has a specific substrate binding site complementary to one or more of the target gene RNA regions, and has nucleotide sequence within or surrounding that substrate binding site which imparts an RNA cleaving activity to the molecule.

*RNA interference (RNAi)*

In one aspect, the invention provides an RNA inhibitory molecule, a so-called "RNAi" molecule, comprising a hydrolase enzyme sequence of the invention. The RNAi molecule can comprise a double-stranded RNA (dsRNA) molecule, e.g., siRNA and/or miRNA. The RNAi molecule, e.g., siRNA and/or miRNA, can inhibit expression of a hydrolase enzyme gene. In one aspect, the RNAi molecule, e.g., siRNA and/or miRNA, is about 11, 12, 13, 14, 15, 16, 17, 18, 19, 20, 21, 22, 23, 24, 25, 26, 27, 28, 29 or 30 or more duplex nucleotides in length. While the invention is not limited by any particular mechanism of action, the RNAi can enter a cell and cause the degradation of a single-stranded RNA (ssRNA) of similar or identical sequences, including endogenous mRNAs.

While the invention is not limited by any particular mechanism of action, when a cell is exposed to double-stranded RNA (dsRNA), mRNA from the homologous gene is selectively degraded by a process called RNA interference (RNAi). A possible basic mechanism behind RNAi is the breaking of a double-stranded RNA (dsRNA) matching a specific gene sequence into short pieces called short interfering RNA, which trigger the degradation of mRNA that matches its sequence. In one aspect, the RNAi's of the invention are used in gene-silencing therapeutics, see, e.g., Shuey (2002) Drug Discov. Today 7:1040-1046. In one aspect, the invention provides methods to selectively degrade RNA using the RNAi's molecules, e.g., siRNA and/or miRNA, of the invention. The process may be practiced *in vitro, ex vivo* or *in vivo*. In one aspect, the RNAi molecules of the invention can be used to generate a loss-of-function mutation in a cell, an organ or an animal. Methods for making and using RNAi molecules, e.g., siRNA and/or miRNA, for selectively degrade RNA are well known in the art, see, e.g., U.S. Patent No. 6,506,559; 6,511,824; 6,515,109; 6,489,127.

<u>Modification of Nucleic Acids</u>

The invention provides methods of generating variants of the nucleic acids of the invention, e.g., those encoding a hydrolase or an antibody of the invention. These methods can be repeated or used in various combinations to generate hydrolases or antibodies having an altered or different activity or an altered or different stability from that of a hydrolase or antibody encoded by the template nucleic acid. These methods also can be repeated or used in various combinations, e.g., to generate variations in gene /message expression, message translation or message stability. In another aspect, the genetic composition of a cell is altered by, e.g., modification of a homologous gene ex vivo, followed by its reinsertion into the cell.

A nucleic acid of the invention can be altered by any means. For example, random or stochastic methods, or, non-stochastic, or "directed evolution," methods, see, e.g., U.S. Patent No. 6,361,974. Methods for random mutation of genes are well known in the art, see, e.g., U.S. Patent No. 5,830,696. For example, mutagens can be used to randomly mutate a gene. Mutagens include, e.g., ultraviolet light or gamma irradiation, or a chemical mutagen, e.g., mitomycin, nitrous acid, photoactivated psoralens, alone or in combination, to induce DNA breaks amenable to repair by recombination. Other chemical mutagens include, for example, sodium bisulfite, nitrous acid, hydroxylamine, hydrazine or formic acid. Other mutagens are analogues of nucleotide precursors, e.g., nitrosoguanidine, 5-bromouracil, 2-aminopurine, or acridine. These agents can be added to a PCR reaction in place of the nucleotide precursor thereby mutating the sequence. Intercalating agents such as proflavine, acriflavine, quinacrine and the like can also be used.

Any technique in molecular biology can be used, e.g., random PCR mutagenesis, see, e.g., Rice (1992) Proc. Natl. Acad. Sci. USA 89:5467-5471; or, combinatorial multiple cassette mutagenesis, see, e.g., Crameri (1995) Biotechniques 18:194-196. Alternatively, nucleic acids, e.g., genes, can be reassembled after random, or "stochastic," fragmentation, see, e.g., U.S. Patent Nos. 6,291,242; 6,287,862; 6,287,861; 5,955,358; 5,830,721; 5,824,514; 5,811,238; 5,605,793. In alternative aspects, modifications, additions or deletions are introduced by error-prone PCR, shuffling, oligonucleotide-directed mutagenesis, assembly PCR, sexual PCR mutagenesis, in vivo mutagenesis, cassette mutagenesis, recursive ensemble mutagenesis, exponential ensemble mutagenesis, site-specific mutagenesis, gene reassembly, gene site saturated mutagenesis (GSSM), synthetic ligation reassembly (SLR), recombination, recursive sequence recombination, phosphothioate-modified DNA mutagenesis, uracil-containing template mutagenesis, gapped duplex mutagenesis, point mismatch repair mutagenesis, repair-deficient host strain mutagenesis, chemical mutagenesis, radiogenic mutagenesis, deletion mutagenesis, restriction-selection mutagenesis, restriction-purification mutagenesis, artificial gene synthesis, ensemble mutagenesis, chimeric nucleic acid multimer creation, and/or a combination of these and other methods.

The following publications describe a variety of recursive recombination procedures and/or methods which can be incorporated into the methods of the invention: Stemmer (1999) "Molecular breeding of viruses for targeting and other clinical properties" Tumor Targeting 4:1-4; Ness (1999) Nature Biotechnology 17:893-896; Chang (1999) "Evolution of a cytokine using DNA family shuffling" Nature Biotechnology 17:793-797; Minshull (1999) "Protein evolution by molecular breeding" Current Opinion in Chemical Biology 3:284-290; Christians (1999) "Directed evolution of thymidine kinase for AZT phosphorylation using DNA family shuffling" Nature Biotechnology 17:259-264; Crameri (1998) "DNA shuffling of a family of genes from diverse species accelerates directed evolution" Nature 391:288-291; Crameri (1997) "Molecular evolution of an arsenate detoxification pathway by DNA shuffling," Nature Biotechnology 15:436-438; Zhang (1997) "Directed evolution of an effective fucosidase from a galactosidase by DNA shuffling and screening" Proc. Natl. Acad. Sci. USA 94:4504-4509; Patten et al. (1997) "Applications of DNA Shuffling to Pharmaceuticals and Vaccines" Current Opinion in Biotechnology 8:724-733; Crameri et al. (1996) "Construction and evolution of antibody-phage libraries by DNA shuffling" Nature Medicine 2:100-103; Gates et al. (1996) "Affinity selective isolation of ligands from peptide libraries through display on a lac repressor 'headpiece dimer'" Journal of Molecular Biology 255:373-386; Stemmer (1996) "Sexual PCR and Assembly PCR" In: The Encyclopedia of Molecular Biology. VCH Publishers, New York. pp.447-457; Crameri and Stemmer (1995) "Combinatorial multiple cassette mutagenesis creates all the permutations of mutant and wildtype cassettes" BioTechniques 18:194-195; Stemmer et al. (1995) "Single-step assembly of a gene and entire plasmid form large numbers of oligodeoxyribonucleotides" Gene, 164:49-53; Stemmer (1995) "The Evolution of Molecular Computation" Science 270: 1510; Stemmer (1995) "Searching Sequence Space" Bio/Technology 13:549-553; Stemmer (1994) "Rapid evolution of a protein in vitro by DNA shuffling" Nature 370:389-391; and Stemmer (1994) "DNA shuffling by random fragmentation and reassembly: In vitro recombination for molecular evolution." Proc. Natl. Acad. Sci. USA 91: 10747-10751.

Mutational methods of generating diversity include, for example, site-directed mutagenesis (Ling et al. (1997) "Approaches to DNA mutagenesis: an overview" Anal Biochem. 254(2): 157-178; Dale et al. (1996) "Oligonucleotide-directed random mutagenesis using the phosphorothioate method" Methods Mol. Biol. 57:369-374; Smith (1985) "In vitro mutagenesis" Ann. Rev. Genet. 19:423-462; Botstein & Shortle (1985) "Strategies and applications of in vitro mutagenesis" Science 229:1193-1201; Carter (1986) "Site-directed mutagenesis" Biochem. J. 237:1-7; and Kunkel (1987) "The efficiency of oligonucleotide directed mutagenesis" in Nucleic Acids & Molecular Biology (Eckstein, F. and Lilley, D. M. J. eds., Springer Verlag, Berlin)); mutagenesis using uracil containing templates (Kunkel (1985) "Rapid and efficient site-specific mutagenesis without phenotypic selection" Proc. Natl. Acad. Sci. USA 82:488-492; Kunkel et al. (1987) "Rapid and efficient site-specific mutagenesis without phenotypic selection" Methods in Enzymol. 154,367-382; and Bass et al. (1988) "Mutant Trp repressors with new DNA-binding specificities" Science 242:240-245); oligonucleotide-directed mutagenesis (Methods in Enzymol. 100: 468-500 (1983); Methods in Enzymol. 154: 329-350 (1987); Zoller & Smith (1982) "Oligonucleotide-directed mutagenesis using M13-derived vectors: an efficient and general procedure for the production of point mutations in any DNA fragment" Nucleic Acids Res. 10:6487-6500; Zoller & Smith (1983) "Oligonucleotide-directed mutagenesis of DNA fragments cloned into M13 vectors" Methods in Enzymol. 100:468-500; and Zoller & Smith (1987) Oligonucleotide-directed mutagenesis: a simple method using two oligonucleotide primers and a single-stranded DNA template" Methods in Enzymol. 154:329-350); phosphorothioate-modified DNA mutagenesis (Taylor et al. (1985) "The use of phosphorothioate-modified DNA in restriction enzyme reactions to prepare nicked DNA" Nucl. Acids Res. 13: 8749-8764; Taylor et al. (1985) "The rapid generation of oligonucleotide-directed mutations at high frequency using phosphorothioate-modified DNA" Nucl. Acids Res. 13: 8765-8787 (1985); Nakamaye (1986) "Inhibition of restriction endonuclease Nci I cleavage by phosphorothioate groups and its application to oligonucleotide-directed mutagenesis" Nucl. Acids Res. 14: 9679-9698; Sayers et al. (1988) "Y-T Exonucleases in phosphorothioate-based oligonucleotide-directed mutagenesis" Nucl. Acids Res. 16:791-802; and Sayers et al. (1988) "Strand specific cleavage ofphosphorothioate-containing DNA by reaction with restriction endonucleases in the presence of ethidium bromide" Nucl. Acids Res. 16: 803-814); mutagenesis using gapped duplex DNA (Kramer et al. (1984) "The gapped duplex DNA approach to oligonucleotide-directed mutation construction" Nucl. Acids Res. 12: 9441-9456; Kramer & Fritz (1987) Methods in Enzymol. "Oligonucleotide-directed construction of mutations via gapped duplex DNA" 154: 350-367; Kramer et al. (1988) "Improved enzymatic in vitro reactions in the gapped duplex DNA approach to oligonucleotide-directed construction of mutations" Nucl. Acids Res. 16: 7207; and Fritz et al. (1988) "Oligonucleotide-directed construction of mutations: a gapped duplex DNA procedure without enzymatic reactions in vitro" Nucl. Acids Res. 16: 6987-6999).

Additional protocols used in the methods of the invention include point mismatch repair (Kramer (1984) "Point Mismatch Repair" Cell 38:879-887), mutagenesis using repair-deficient host strains (Carter et al. (1985) "Improved oligonucleotide site-directed mutagenesis using M13 vectors" Nucl. Acids Res. 13: 4431-4443; and Carter (1987) "Improved oligonucleotide-directed mutagenesis using M13 vectors" Methods in Enzymol. 154: 382-403), deletion mutagenesis (Eghtedarzadeh (1986) "Use of oligonucleotides to generate large deletions" Nucl. Acids Res. 14: 5115), restriction-selection and restriction-selection and restriction-purification (Wells et al. (1986) "Importance of hydrogen-bond formation in stabilizing the transition state of subtilisin" Phil. Trans. R. Soc. Lond. A 317: 415-423), mutagenesis by total gene synthesis (Nambiar et al. (1984) "Total synthesis and cloning of a gene coding for the ribonuclease S protein" Science 223: 1299-1301; Sakamar and Khorana (1988) "Total synthesis and expression of a gene for the a-subunit of bovine rod outer segment guanine nucleotide-binding protein (transducin)" Nucl. Acids Res. 14: 6361-6372; Wells et al. (1985) "Cassette mutagenesis: an efficient method for generation of multiple mutations at defined sites" Gene 34: 315-323; and Grundstrom et al. (1985) "Oligonucleotide-directed mutagenesis by microscale 'shot-gun' gene synthesis" Nucl. Acids Res. 13: 3305-3316), double-strand break repair (Mandecki (1986); Arnold (1993) "Protein engineering for unusual environments" Current Opinion in Biotechnology 4:450-455. "Oligonucleotide-directed double-strand break repair in plasmids of Escherichia coli: a method for site-specific mutagenesis" Proc. Natl. Acad. Sci. USA, 83:7177-7181). Additional details on many of the above methods can be found in Methods in Enzymology Volume 154, which also describes useful controls for trouble-shooting problems with various mutagenesis methods.

Additional protocols used in the methods of the invention include those discussed in U.S. Patent Nos. 5,605,793 to Stemmer (Feb. 25, 1997), "Methods for In Vitro Recombination;" U.S. Pat. No. 5,811,238 to Stemmer et al. (Sep. 22, 1998) "Methods for Generating Polynucleotides having Desired Characteristics by Iterative Selection and Recombination;" U.S. Pat. No. 5,830,721 to Stemmer et al. (Nov. 3, 1998), "DNA Mutagenesis by Random Fragmentation and Reassembly;" U.S. Pat. No. 5,834,252 to Stemmer, et al. (Nov. 10, 1998) "End-Complementary Polymerase Reaction;" U.S. Pat. No. 5,837,458 to Minshull, et al. (Nov. 17, 1998), "Methods and Compositions for Cellular and Metabolic Engineering;" WO 95/22625, Stemmer and Crameri, "Mutagenesis by Random Fragmentation and Reassembly;" WO 96/33207 by Stemmer and Lipschutz "End Complementary Polymerase Chain Reaction;" WO 97/20078 by Stemmer and Crameri "Methods for Generating Polynucleotides having Desired Characteristics by Iterative Selection and Recombination;" WO 97/35966 by Minshull and Stemmer, "Methods and Compositions for Cellular and Metabolic Engineering;" WO 99/41402 by Punnonen et al. "Targeting of Genetic Vaccine Vectors;" WO 99/41383 by Punnonen et al. "Antigen Library Immunization;" WO 99/41369 by Punnonen et al. "Genetic Vaccine Vector Engineering;" WO 99/41368 by Punnonen et al. "Optimization of Immunomodulatory Properties of Genetic Vaccines;" EP 752008 by Stemmer and Crameri, "DNA Mutagenesis by Random Fragmentation and Reassembly;" EP 0932670 by Stemmer "Evolving Cellular DNA Uptake by Recursive Sequence Recombination;" WO 99/23107 by Stemmer et al., "Modification of Virus Tropism and Host Range by Viral Genome Shuffling," WO 99/21979 by Apt et al., "Human Papillomavirus Vectors;" WO 98/31837 by del Cardayre et al. "Evolution of Whole Cells and Organisms by Recursive Sequence Recombination;" WO 98/27230 by Patten and Stemmer, "Methods and Compositions for Polypeptide Engineering;" WO 98/27230 by Stemmer et al., "Methods for Optimization of Gene Therapy by Recursive Sequence Shuffling and Selection," WO 00/00632, "Methods for Generating Highly Diverse Libraries," WO 00/09679, "Methods for Obtaining in Vitro Recombined Polynucleotide Sequence Banks and Resulting Sequences," WO 98/42832 by Arnold et al., "Recombination of Polynucleotide Sequences Using Random or Defined Primers," WO 99/29902 by Arnold et al., "Method for Creating Polynucleotide and Polypeptide Sequences," WO 98/41653 by Vind, "An in Vitro Method for Construction of a DNA Library," WO 98/41622 by Borchert et al., "Method for Constructing a Library Using DNA Shuffling," and WO 98/42727 by Pati and Zarling, "Sequence Alterations using Homologous Recombination."

Protocols that can be used to practice the invention (providing details regarding various diversity generating methods) are described, e.g., in U.S. Patent application serial no. (USSN) 09/407,800, "SHUFFLING OF CODON ALTERED GENES" by Patten et al. filed Sep. 28, 1999; "EVOLUTION OF WHOLE CELLS AND ORGANISMS BY RECURSIVE SEQUENCE RECOMBINATION" by del Cardayre et al., United States Patent No. 6,379,964; "OLIGONUCLEOTIDE MEDIATED NUCLEIC ACID RECOMBINATION" by Crameri et al., United States Patent Nos. 6,319,714; 6,368,861; 6,376,246; 6,423,542; 6,426,224 and PCT/US00/01203; "USE OF CODON-VARIED OLIGONUCLEOTIDE SYNTHESIS FOR SYNTHETIC SHUFFLING" by Welch et al., United States Patent No. 6,436,675; "METHODS FOR MAKING CHARACTER STRINGS, POLYNUCLEOTIDES & POLYPEPTIDES HAVING DESIRED CHARACTERISTICS" by Selifonov et al., filed Jan. 18, 2000, (PCT/US00/01202) and, e.g. "METHODS FOR MAKING CHARACTER STRINGS, POLYNUCLEOTIDES & POLYPEPTIDES HAVING DESIRED CHARACTERISTICS" by Selifonov et al., filed Jul. 18,2000 (U.S. Ser. No. 09/618,579); "METHODS OF POPULATING DATA STRUCTURES FOR USE IN EVOLUTIONARY SIMULATIONS" by Selifonov and Stemmer, filed Jan. 18, 2000 (PCT/US00/01138); and "SINGLE-STRANDED NUCLEIC ACID TEMPLATE-MEDIATED RECOMBINATION AND NUCLEIC ACID FRAGMENT ISOLATION" by Affholter, filed Sep. 6, 2000 (U.S. Ser. No. 09/656,549); and United States Patent Nos. 6,177,263; 6,153,410.

Non-stochastic, or "directed evolution," methods include, e.g., saturation mutagenesis (GSSM), synthetic ligation reassembly (SLR), or a combination thereof are used to modify the nucleic acids of the invention to generate hydrolases with new or altered properties (e.g., activity under highly acidic or alkaline conditions, high temperatures, and the like). Polypeptides encoded by the modified nucleic acids can be screened for an activity before testing for proteolytic or other activity. Any testing modality or protocol can be used, e.g., using a capillary array platform. See, e.g., U.S. Patent Nos. 6,361,974; 6,280,926; 5,939,250.

*Gene Site Saturation mutagenesis, or, GSSM*

The invention also provides methods for making enzyme using Gene Site Saturation mutagenesis, or, GSSM, as described herein, and also in U.S. Patent Nos. 6,171,820 and 6,579,258. In one aspect of the invention, non-stochastic gene modification, a "directed evolution process," is used to generate hydrolases and antibodies with new or altered properties. Variations of this method have been termed "gene site-saturation mutagenesis," "site-saturation mutagenesis," "saturation mutagenesis" or simply "GSSM." It can be used in combination with other mutagenization processes. See, e.g., U.S. Patent Nos. 6,171,820; 6,238,884.

In one aspect, GSSM comprises providing a template polynucleotide and a plurality of oligonucleotides, wherein each oligonucleotide comprises a sequence homologous to the template polynucleotide, thereby targeting a specific sequence of the template polynucleotide, and a sequence that is a variant of the homologous gene; generating progeny polynucleotides comprising non-stochastic sequence variations by replicating the template polynucleotide with the oligonucleotides, thereby generating polynucleotides comprising homologous gene sequence variations.

sIn one aspect, codon primers containing a degenerate N,N,G/T sequence are used to introduce point mutations into a polynucleotide, so as to generate a set of progeny polypeptides in which a full range of single amino acid substitutions is represented at each amino acid position, e.g., an amino acid residue in an enzyme active site or ligand binding site targeted to be modified. These oligonucleotides can comprise a contiguous first homologous sequence, a degenerate N,N,G/T sequence, and, optionally, a second homologous sequence. The downstream progeny translational products from the use of such oligonucleotides include all possible amino acid changes at each amino acid site along the polypeptide, because the degeneracy of the N,N,G/T sequence includes codons for all 20 amino acids. In one aspect, one such degenerate oligonucleotide (comprised of, e.g., one degenerate N,N,G/T cassette) is used for subjecting each original codon in a parental polynucleotide template to a full range of codon substitutions. In another aspect, at least two degenerate cassettes are used - either in the same oligonucleotide or not, for subjecting at least two original codons in a parental polynucleotide template to a full range of codon substitutions. For example, more than one N,N,G/T sequence can be contained in one oligonucleotide to introduce amino acid mutations at more than one site. This plurality ofN,N, G/T sequences can be directly contiguous, or separated by one or more additional nucleotide sequence(s). In another aspect, oligonucleotides serviceable for introducing additions and deletions can be used either alone or in combination with the codons containing an N,N,G/T sequence, to introduce any combination or permutation of amino acid additions, deletions, and/or substitutions.

In one aspect, simultaneous mutagenesis of two or more contiguous amino acid positions is done using an oligonucleotide that contains contiguous N,N,G/T triplets, i.e. a degenerate (N,N,G/T)n sequence. In another aspect, degenerate cassettes having less degeneracy than the N,N,G/T sequence are used. For example, it may be desirable in some instances to use (e.g. in an oligonucleotide) a degenerate triplet sequence comprised of only one N, where said N can be in the first second or third position of the triplet. Any other bases including any combinations and permutations thereof can be used in the remaining two positions of the triplet. Alternatively, it may be desirable in some instances to use (e.g. in an oligo) a degenerate N,N,N triplet sequence.

In one aspect, use of degenerate triplets (e.g., N,N,G/T triplets) allows for systematic and easy generation of a full range of possible natural amino acids (for a total of 20 amino acids) into each and every amino acid position in a polypeptide (in alternative aspects, the methods also include generation of less than all possible substitutions per amino acid residue, or codon, position). For example, for a 100 amino acid polypeptide, 2000 distinct species (i.e. 20 possible amino acids per position X 100 amino acid positions) can be generated. Through the use of an oligonucleotide or set of oligonucleotides containing a degenerate N,N,G/T triplet, 32 individual sequences can code for all 20 possible natural amino acids. Thus, in a reaction vessel in which a parental polynucleotide sequence is subjected to saturation mutagenesis using at least one such oligonucleotide, there are generated 32 distinct progeny polynucleotides encoding 20 distinct polypeptides. In contrast, the use of a non-degenerate oligonucleotide in site-directed mutagenesis leads to only one progeny polypeptide product per reaction vessel. Nondegenerate oligonucleotides can optionally be used in combination with degenerate primers disclosed; for example, nondegenerate oligonucleotides can be used to generate specific point mutations in a working polynucleotide. This provides one means to generate specific silent point mutations, point mutations leading to corresponding amino acid changes, and point mutations that cause the generation of stop codons and the corresponding expression of polypeptide fragments.

In one aspect, each saturation mutagenesis reaction vessel contains polynucleotides encoding at least 20 progeny polypeptide (e.g., hydrolases, e.g., esterases, acylases, lipases, phospholipases or proteases) molecules such that all 20 natural amino acids are represented at the one specific amino acid position corresponding to the codon position mutagenized in the parental polynucleotide (other aspects use less than all 20 natural combinations). The 32-fold degenerate progeny polypeptides generated from each saturation mutagenesis reaction vessel can be subjected to clonal amplification (e.g. cloned into a suitable host, e.g., E. coli host, using, e.g., an expression vector) and subjected to expression screening. When an individual progeny polypeptide is identified by screening to display a favorable change in property (when compared to the parental polypeptide, such as increased proteolytic activity under alkaline or acidic conditions), it can be sequenced to identify the correspondingly favorable amino acid substitution contained therein.

In one aspect, upon mutagenizing each and every amino acid position in a parental polypeptide using saturation mutagenesis as disclosed herein, favorable amino acid changes may be identified at more than one amino acid position. One or more new progeny molecules can be generated that contain a combination of all or part of these favorable amino acid substitutions. For example, if 2 specific favorable amino acid changes are identified in each of 3 amino acid positions in a polypeptide, the permutations include 3 possibilities at each position (no change from the original amino acid, and each of two favorable changes) and 3 positions. Thus, there are 3 x 3 x 3 or 27 total possibilities, including 7 that were previously examined - 6 single point mutations (i.e. 2 at each of three positions) and no change at any position.

In another aspect, site-saturation mutagenesis can be used together with another stochastic or non-stochastic means to vary sequence, e.g., synthetic ligation reassembly (see below), shuffling, chimerization, recombination and other mutagenizing processes and mutagenizing agents. This invention provides for the use of any mutagenizing process (es), including saturation mutagenesis, in an iterative manner.

*Synthetic Ligation Reassembly (SLR)*

The invention provides a non-stochastic gene modification system termed "synthetic ligation reassembly," or simply "SLR," a "directed evolution process," to generate polypeptides, e.g., hydrolase enzymes or antibodies of the invention, with new or altered properties. SLR is a method of ligating oligonucleotide fragments together non-stochastically. This method differs from stochastic oligonucleotide shuffling in that the nucleic acid building blocks are not shuffled, concatenated or chimerized randomly, but rather are assembled non-stochastically. See, e.g., U.S. Patent Nos. 6,773,900; 6,740,506; 6,713,282; 6,635,449; 6,605,449; 6,537,776.

In one aspect, SLR comprises the following steps: (a) providing a template polynucleotide, wherein the template polynucleotide comprises sequence encoding a homologous gene; (b) providing a plurality of building block polynucleotides, wherein the building block polynucleotides are designed to cross-over reassemble with the template polynucleotide at a predetermined sequence, and a building block polynucleotide comprises a sequence that is a variant of the homologous gene and a sequence homologous to the template polynucleotide flanking the variant sequence; (c) combining a building block polynucleotide with a template polynucleotide such that the building block polynucleotide cross-over reassembles with the template polynucleotide to generate polynucleotides comprising homologous gene sequence variations.

SLR does not depend on the presence of high levels of homology between polynucleotides to be rearranged. Thus, this method can be used to non-stochastically generate libraries (or sets) of progeny molecules comprised of over 10100 different chimeras. SLR can be used to generate libraries comprised of over 101000 different progeny chimeras. Thus, aspects of the present invention include non-stochastic methods of producing a set of finalized chimeric nucleic acid molecule shaving an overall assembly order that is chosen by design. This method includes the steps of generating by design a plurality of specific nucleic acid building blocks having serviceable mutually compatible ligatable ends, and assembling these nucleic acid building blocks, such that a designed overall assembly order is achieved.

The mutually compatible ligatable ends of the nucleic acid building blocks to be assembled are considered to be "serviceable" for this type of ordered assembly if they enable the building blocks to be coupled in predetermined orders. Thus, the overall assembly order in which the nucleic acid building blocks can be coupled is specified by the design of the ligatable ends. If more than one assembly step is to be used, then the overall assembly order in which the nucleic acid building blocks can be coupled is also specified by the sequential order of the assembly step(s). In one aspect, the annealed building pieces are treated with an enzyme, such as a ligase (e.g. T4 DNA ligase), to achieve covalent bonding of the building pieces.

In one aspect, the design of the oligonucleotide building blocks is obtained by analyzing a set of progenitor nucleic acid sequence templates that serve as a basis for producing a progeny set of finalized chimeric polynucleotides. These parental oligonucleotide templates thus serve as a source of sequence information that aids in the design of the nucleic acid building blocks that are to be mutagenized, e.g., chimerized or shuffled. In one aspect of this method, the sequences of a plurality of parental nucleic acid templates are aligned in order to select one or more demarcation points. The demarcation points can be located at an area of homology, and are comprised of one or more nucleotides. These demarcation points are preferably shared by at least two of the progenitor templates. The demarcation points can thereby be used to delineate the boundaries of oligonucleotide building blocks to be generated in order to rearrange the parental polynucleotides. The demarcation points identified and selected in the progenitor molecules serve as potential chimerization points in the assembly of the final chimeric progeny molecules. A demarcation point can be an area of homology (comprised of at least one homologous nucleotide base) shared by at least two parental polynucleotide sequences. Alternatively, a demarcation point can be an area of homology that is shared by at least half of the parental polynucleotide sequences, or, it can be an area of homology that is shared by at least two thirds of the parental polynucleotide sequences. Even more preferably a serviceable demarcation points is an area of homology that is shared by at least three fourths of the parental polynucleotide sequences, or, it can be shared by at almost all of the parental polynucleotide sequences. In one aspect, a demarcation point is an area of homology that is shared by all of the parental polynucleotide sequences.

In one aspect, a ligation reassembly process is performed exhaustively in order to generate an exhaustive library of progeny chimeric polynucleotides. In other words, all possible ordered combinations of the nucleic acid building blocks are represented in the set of finalized chimeric nucleic acid molecules. At the same time, in another aspect, the assembly order (i.e. the order of assembly of each building block in the 5' to 3 sequence of each finalized chimeric nucleic acid) in each combination is by design (or non-stochastic) as described above. Because of the non-stochastic nature of this invention, the possibility of unwanted side products is greatly reduced.

In another aspect, the ligation reassembly method is performed systematically. For example, the method is performed in order to generate a systematically compartmentalized library of progeny molecules, with compartments that can be screened systematically, e.g. one by one. In other words this invention provides that, through the selective and judicious use of specific nucleic acid building blocks, coupled with the selective and judicious use of sequentially stepped assembly reactions, a design can be achieved where specific sets of progeny products are made in each of several reaction vessels. This allows a systematic examination and screening procedure to be performed. Thus, these methods allow a potentially very large number of progeny molecules to be examined systematically in smaller groups. Because of its ability to perform chimerizations in a manner that is highly flexible yet exhaustive and systematic as well, particularly when there is a low level of homology among the progenitor molecules, these methods provide for the generation of a library (or set) comprised of a large number of progeny molecules. Because of the non-stochastic nature of the instant ligation reassembly invention, the progeny molecules generated preferably comprise a library of finalized chimeric nucleic acid molecules having an overall assembly order that is chosen by design. The saturation mutagenesis and optimized directed evolution methods also can be used to generate different progeny molecular species. It is appreciated that the invention provides freedom of choice and control regarding the selection of demarcation points, the size and number of the nucleic acid building blocks, and the size and design of the couplings. It is appreciated, furthermore, that the requirement for intermolecular homology is highly relaxed for the operability of this invention. In fact, demarcation points can even be chosen in areas of little or no intermolecular homology. For example, because of codon wobble, i.e. the degeneracy of codons, nucleotide substitutions can be introduced into nucleic acid building blocks without altering the amino acid originally encoded in the corresponding progenitor template. Alternatively, a codon can be altered such that the coding for an originally amino acid is altered. This invention provides that such substitutions can be introduced into the nucleic acid building block in order to increase the incidence of intermolecular homologous demarcation points and thus to allow an increased number of couplings to be achieved among the building blocks, which in turn allows a greater number of progeny chimeric molecules to be generated.

In another aspect, the synthetic nature of the step in which the building blocks are generated allows the design and introduction of nucleotides (e.g., one or more nucleotides, which may be, for example, codons or introns or regulatory sequences) that can later be optionally removed in an in vitro process (e.g. by mutagenesis) or in an in vivo process (e.g. by utilizing the gene splicing ability of a host organism). It is appreciated that in many instances the introduction of these nucleotides may also be desirable for many other reasons in addition to the potential benefit of creating a serviceable demarcation point.

In one aspect, a nucleic acid building block is used to introduce an intron. Thus, functional introns are introduced into a man-made gene manufactured according to the methods described herein. The artificially introduced intron(s) can be functional in a host cells for gene splicing much in the way that naturally-occurring introns serve functionally in gene splicing.

*Optimized Directed Evolution System*

The invention provides a non-stochastic gene modification system termed "optimized directed evolution system" to generate hydrolases and antibodies with new or altered properties. Optimized directed evolution is directed to the use of repeated cycles of reductive reassortment, recombination and selection that allow for the directed molecular evolution of nucleic acids through recombination. Optimized directed evolution allows generation of a large population of evolved chimeric sequences, wherein the generated population is significantly enriched for sequences that have a predetermined number of crossover events.

A crossover event is a point in a chimeric sequence where a shift in sequence occurs from one parental variant to another parental variant. Such a point is normally at the juncture of where oligonucleotides from two parents are ligated together to form a single sequence. This method allows calculation of the correct concentrations of oligonucleotide sequences so that the final chimeric population of sequences is enriched for the chosen number of crossover events. This provides more control over choosing chimeric variants having a predetermined number of crossover events.

In addition, this method provides a convenient means for exploring a tremendous amount of the possible protein variant space in comparison to other systems. Previously, if one generated, for example, $10^{13}$ chimeric molecules during a reaction, it would be extremely difficult to test such a high number of chimeric variants for a particular activity. Moreover, a significant portion of the progeny population would have a very high number of crossover events which resulted in proteins that were less likely to have increased levels of a particular activity. By using these methods, the population of chimerics molecules can be enriched for those variants that have a particular number of crossover events. Thus, although one can still generate $10^{13}$ chimeric molecules during a reaction, each of the molecules chosen for further analysis most likely has, for example, only three crossover events. Because the resulting progeny population can be skewed to have a predetermined number of crossover events, the boundaries on the functional variety between the chimeric molecules is reduced. This provides a more manageable number of variables when calculating which oligonucleotide from the original parental polynucleotides might be responsible for affecting a particular trait.

One method for creating a chimeric progeny polynucleotide sequence is to create oligonucleotides corresponding to fragments or portions of each parental sequence. Each oligonucleotide in one aspect includes a unique region of overlap so that mixing the oligonucleotides together results in a new variant that has each oligonucleotide fragment assembled in the correct order. Alternatively protocols for practicing these methods of the invention can be found in U.S. Patent Nos. 6,773,900; 6,740,506; 6,713,282; 6,635,449; 6,605,449; 6,537,776; 6,361,974.

The number of oligonucleotides generated for each parental variant bears a relationship to the total number of resulting crossovers in the chimeric molecule that is ultimately created. For example, three parental nucleotide sequence variants might be provided to undergo a ligation reaction in order to find a chimeric variant having, for example, greater activity at high temperature. As one example, a set of 50 oligonucleotide sequences can be generated corresponding to each portions of each parental variant. Accordingly, during the ligation reassembly process there could be up to 50 crossover events within each of the chimeric sequences. The probability that each of the generated chimeric polynucleotides will contain oligonucleotides from each parental variant in alternating order is very low. If each oligonucleotide fragment is present in the ligation reaction in the same molar quantity it is likely that in some positions oligonucleotides from the same parental polynucleotide will ligate next to one another and thus not result in a crossover event. If the concentration of each oligonucleotide from each parent is kept constant during any ligation step in this example, there is a 1/3 chance (assuming 3 parents) that an oligonucleotide from the same parental variant will ligate within the chimeric sequence and produce no crossover.

Accordingly, a probability density function (PDF) can be determined to predict the population of crossover events that are likely to occur during each step in a ligation reaction given a set number of parental variants, a number of oligonucleotides corresponding to each variant, and the concentrations of each variant during each step in the ligation reaction. The statistics and mathematics behind determining the PDF is described below. By utilizing these methods; one can calculate such a probability density function, and thus enrich the chimeric progeny population for a predetermined number of crossover events resulting from a particular ligation reaction. Moreover, a target number of crossover events can be predetermined, and the system then programmed to calculate the starting quantities of each parental oligonucleotide during each step in the ligation reaction to result in a probability density function that centers on the predetermined number of crossover events. These methods are directed to the use of repeated cycles of reductive reassortment, recombination and selection that allow for the directed molecular evolution of a nucleic acid encoding a polypeptide through recombination. This system allows generation of a large population of evolved chimeric sequences, wherein the generated population is significantly enriched for sequences that have a predetermined number of crossover events. A crossover event is a point in a chimeric sequence where a shift in sequence occurs from one parental variant to another parental variant. Such a point is normally at the juncture of where oligonucleotides from two parents are ligated together to form a single sequence. The method allows calculation of the correct concentrations of oligonucleotide sequences so that the final chimeric population of sequences is enriched for the chosen number of crossover events. This provides more control over choosing chimeric variants having a predetermined number of crossover events.

*Determining Crossover Events*

Aspects of the invention include a system and software that receive a desired crossover probability density function (PDF), the number of parent genes to be reassembled, and the number of fragments in the reassembly as inputs. The output of this program is a "fragment PDF" that can be used to determine a recipe for producing reassembled genes, and the estimated crossover PDF of those genes. The processing described herein is preferably performed in MATLAB™ (The Mathworks, Natick, Massachusetts) a programming language and development environment for technical computing.

*Iterative Processes*

In practicing the invention, these processes can be iteratively repeated. For example a nucleic acid (or, the nucleic acid) responsible for an altered hydrolase or antibody phenotype is identified, re-isolated, again modified, retested for activity. This process can be iteratively repeated until a desired phenotype is engineered. For example, an entire biochemical anabolic or catabolic pathway can be engineered into a cell, including proteolytic activity.

Similarly, if it is determined that a particular oligonucleotide has no affect at all on the desired trait (e.g., a new hydrolase phenotype), it can be removed as a variable by synthesizing larger parental oligonucleotides that include the sequence to be removed. Since incorporating the sequence within a larger sequence prevents any crossover events, there will no longer be any variation of this sequence in the progeny polynucleotides. This iterative practice of determining which oligonucleotides are most related to the desired trait, and which are unrelated, allows more efficient exploration all of the possible protein variants that might be provide a particular trait or activity.

*In vivo shuffling*

*In vivo* shuffling of molecules is use in methods of the invention that provide variants ofpolypeptides of the invention, e.g., antibodies, hydrolases, and the like. *In vivo* shuffling can be performed utilizing the natural property of cells to recombine multimers. While recombination *in vivo* has provided the major natural route to molecular diversity, genetic recombination remains a relatively complex process that involves 1) the recognition of homologies; 2) strand cleavage, strand invasion, and metabolic steps leading to the production of recombinant chiasma; and finally 3) the resolution of chiasma into discrete recombined molecules. The formation of the chiasma requires the recognition of homologous sequences.

In one aspect, the invention provides a method for producing a hybrid polynucleotide from at least a first polynucleotide and a second polynucleotide. The invention can be used to produce a hybrid polynucleotide by introducing at least a first polynucleotide and a second polynucleotide which share at least one region of partial sequence homology into a suitable host cell. The regions of partial sequence homology promote processes which result in sequence reorganization producing a hybrid polynucleotide. The term "hybrid polynucleotide", as used herein, is any nucleotide sequence which results from the method of the present invention and contains sequence from at least two original polynucleotide sequences. Such hybrid polynucleotides can result from intermolecular recombination events which promote sequence integration between DNA molecules. In addition, such hybrid polynucleotides can result from intramolecular reductive reassortment processes which utilize repeated sequences to alter a nucleotide sequence within a DNA molecule.

*Producing sequence variants*

The invention also provides methods of making sequence variants of the nucleic acid and hydrolase and antibody sequences of the invention or isolating hydrolases using the nucleic acids and polypeptides of the invention. In one aspect, the invention provides for variants of a hydrolase gene of the invention, which can be altered by any means, including, e.g., random or stochastic methods, or, non-stochastic, or "directed evolution," methods, as described above.

The isolated variants may be naturally occurring. Variant can also be created in vitro. Variants may be created using genetic engineering techniques such as site directed mutagenesis, random chemical mutagenesis, Exonuclease III deletion procedures, and standard cloning techniques. Alternatively, such variants, fragments, analogs, or derivatives may be created using chemical synthesis or modification procedures. Other methods of making variants are also familiar to those skilled in the art. These include procedures in which nucleic acid sequences obtained from natural isolates are modified to generate nucleic acids which encode polypeptides having characteristics which enhance their value in industrial or laboratory applications. In such procedures, a large number of variant sequences having one or more nucleotide differences with respect to the sequence obtained from the natural isolate are generated and characterized. These nucleotide differences can result in amino acid changes with respect to the polypeptides encoded by the nucleic acids from the natural isolates.

For example, variants may be created using error prone PCR. In error prone PCR, PCR is performed under conditions where the copying fidelity of the DNA polymerase is low, such that a high rate of point mutations is obtained along the entire length of the PCR product. Error prone PCR is described, e.g., in Leung, D.W., et al., Technique, 1: 11-15, 1989) and Caldwell, R. C. & Joyce G.F., PCR Methods Applic., 2:28-33, 1992. Briefly, in such procedures, nucleic acids to be mutagenized are mixed with PCR primers, reaction buffer, $MgCl_2$, $MnCl_2$, Taq polymerase and an appropriate concentration of dNTPs for achieving a high rate of point mutation along the entire length of the PCR product. For example, the reaction may be performed using 20 fmoles of nucleic acid to be mutagenized, 30 pmole of each PCR primer, a reaction buffer comprising 50 mM KCl, 10 mM Tris HCl (pH 8.3) and 0.01% gelatin, 7 mM $MgCl_2$, 0.5 mM $MnCl_2$, 5 units of Taq polymerase, 0.2 mM dGTP, 0.2 mM dATP, 1 mM dCTP, and 1 mM dTTP. PCR may be performed for 30 cycles of 94°C for 1 min, 45°C for 1 min, and 72°C for 1 min. However, it will be appreciated that these parameters may be varied as appropriate. The mutagenized nucleic acids are cloned into an appropriate vector and the activities of the polypeptides encoded by the mutagenized nucleic acids is evaluated.

Variants may also be created using oligonucleotide directed mutagenesis to generate site-specific mutations in any cloned DNA of interest. Oligonucleotide mutagenesis is described, e.g., in Reidhaar-Olson (1988) Science 241: 53-57. Briefly, in such procedures a plurality of double stranded oligonucleotides bearing one or more mutations to be introduced into the cloned DNA are synthesized and inserted into the cloned DNA to be mutagenized. Clones containing the mutagenized DNA are recovered and the activities of the polypeptides they encode are assessed.

Another method for generating variants is assembly PCR. Assembly PCR involves the assembly of a PCR product from a mixture of small DNA fragments. A large number of different PCR reactions occur in parallel in the same vial, with the products of one reaction priming the products of another reaction. Assembly PCR is described in, e.g., U.S. Patent No. 5,965,408.

Still another method of generating variants is sexual PCR mutagenesis. In sexual PCR mutagenesis, forced homologous recombination occurs between DNA molecules of different but highly related DNA sequence in vitro, as a result of random fragmentation of the DNA molecule based on sequence homology, followed by fixation of the crossover by primer extension in a PCR reaction. Sexual PCR mutagenesis is described, e.g., in Stemmer (1994) Proc. Natl. Acad. Sci. USA 91:10747-10751. Briefly, in such procedures a plurality of nucleic acids to be recombined are digested with DNase to generate fragments having an average size of 50-200 nucleotides. Fragments of the desired average size are purified and resuspended in a PCR mixture. PCR is conducted under conditions which facilitate recombination between the nucleic acid fragments. For example, PCR may be performed by resuspending the purified fragments at a concentration of 10-30 ng/:l in a solution of 0.2 mM of each dNTP, 2.2 mM $MgCl_2$, 50 mM KCL, 10 mM Tris HCl, pH 9.0, and 0.1 % Triton X-100. 2.5 units ofTaq polymerase per 100:1 of reaction mixture is added and PCR is performed using the following regime: 94°C for 60 seconds, 94°C for 30 seconds, 50-55°C for 30 seconds, 72°C for 30 seconds (30-45 times) and 72°C for 5 minutes. However, it will be appreciated that these parameters may be varied as appropriate. In some aspects, oligonucleotides may be included in the PCR reactions. In other aspects, the Klenow fragment of DNA polymerase I may be used in a first set of PCR reactions and Taq polymerase may be used in a subsequent set of PCR reactions. Recombinant sequences are isolated and the activities of the polypeptides they encode are assessed.

Variants may also be created by *in vivo* mutagenesis. In some aspects, random mutations in a sequence of interest are generated by propagating the sequence of interest in a bacterial strain, such as an *E. coli* strain, which carries mutations in one or more of the DNA repair pathways. Such "mutator" strains have a higher random mutation rate than that of a wild-type parent. Propagating the DNA in one of these strains will eventually generate random mutations within the DNA. Mutator strains suitable for use for in vivo mutagenesis are described, e.g., in PCT Publication No. WO 91/16427.

Variants may also be generated using cassette mutagenesis. In cassette mutagenesis a small region of a double stranded DNA molecule is replaced with a synthetic oligonucleotide "cassette" that differs from the native sequence. The oligonucleotide often contains completely and/or partially randomized native sequence.

Recursive ensemble mutagenesis may also be used to generate variants. Recursive ensemble mutagenesis is an algorithm for protein engineering (protein mutagenesis) developed to produce diverse populations of phenotypically related mutants whose members differ in amino acid sequence. This method uses a feedback mechanism to control successive rounds of combinatorial cassette mutagenesis. Recursive ensemble mutagenesis is described, e.g., in Arkin (1992) Proc. Natl. Acad. Sci. USA 89:7811-7815.

In some aspects, variants are created using exponential ensemble mutagenesis. Exponential ensemble mutagenesis is a process for generating combinatorial libraries with a high percentage of unique and functional mutants, wherein small groups of residues are randomized in parallel to identify, at each altered position, amino acids which lead to functional proteins. Exponential ensemble mutagenesis is described, e.g., in Delegrave (1993) Biotechnology Res. 11:1548-1552. Random and site-directed mutagenesis are described, e.g., in Arnold (1993) Current Opinion in Biotechnology 4:450-455.

In some aspects, the variants are created using shuffling procedures wherein portions of a plurality of nucleic acids which encode distinct polypeptides are fused together to create chimeric nucleic acid sequences which encode chimeric polypeptides as described in, e.g., U.S. Patent Nos. 5,965,408; 5,939,250.

The invention also provides variants of polypeptides of the invention comprising sequences in which one or more of the amino acid residues (e.g., of an exemplary polypeptide, such as SEQ ID NO:2, SEQ ID NO:4, SEQ ID NO: 6, SEQ ID NO:8, etc.) are substituted with a conserved or non-conserved amino acid residue (e.g., a conserved amino acid residue) and such substituted amino acid residue may or may not be one encoded by the genetic code. Conservative substitutions are those that substitute a given amino acid in a polypeptide by another amino acid of like characteristics. Thus, polypeptides of the invention include those with conservative substitutions of sequences of the invention, e.g., the exemplary sequences of the invention, such as SEQ ID NO:2, SEQ ID NO:4, SEQ ID NO:6, SEQ ID NO:8, etc., including but not limited to the following replacements: replacements of an aliphatic amino acid such as Alanine, Valine, Leucine and Isoleucine with another aliphatic amino acid; replacement of a Serine with a Threonine or vice versa; replacement of an acidic residue such as Aspartic acid and Glutamic acid with another acidic residue; replacement of a residue bearing an amide group, such as Asparagine and Glutamine, with another residue bearing an amide group; exchange of a basic residue such as Lysine and Arginine with another basic residue; and replacement of an aromatic residue such as Phenylalanine, Tyrosine with another aromatic residue. Other variants are those in which one or more of the amino acid residues of the polypeptides of the invention includes a substituent group.

Other variants within the scope of the invention are those in which the polypeptide is associated with another compound, such as a compound to increase the half-life of the polypeptide, for example, polyethylene glycol. Additional variants within the scope of the invention are those in which additional amino acids are fused to the polypeptide, such as a leader sequence, a secretory sequence, a proprotein sequence or a sequence which facilitates purification, enrichment, or stabilization of the polypeptide. In some aspects, the variants, fragments, derivatives and analogs of the polypeptides of the invention retain the same biological function or activity as the exemplary polypeptides, e.g., a proteolytic activity, as described herein. In other aspects, the variant, fragment, derivative, or analog includes a proprotein, such that the variant, fragment, derivative, or analog can be activated by cleavage of the proprotein portion to produce an active polypeptide.

*Optimizing codons to achieve high levels ofprotein expression in host cells*

The invention provides methods for modifying hydrolase-encoding nucleic acids to modify codon usage. In one aspect, the invention provides methods for modifying codons in a nucleic acid encoding a hydrolase to increase or decrease its expression in a host cell, e.g., a bacterial, insect, mammalian, yeast or plant cell. The invention also provides nucleic acids encoding a hydrolase modified to increase its expression in a host cell, hydrolase so modified, and methods of making the modified hydrolases. The method, comprises identifying a "non-preferred" or a "less preferred" codon in hydrolase-encoding nucleic acid and replacing one or more of these non-preferred or less preferred codons with a "preferred codon" encoding the same amino acid as the replaced codon and at least one non-preferred or less preferred codon in the nucleic acid has been replaced by a preferred codon encoding the same amino acid. A preferred codon is a codon over-represented in coding sequences in genes in the host cell and a non-preferred or less preferred codon is a codon under-represented in coding sequences in genes in the host cell.

Host cells for expressing the nucleic acids, expression cassettes and vectors of the invention include bacteria, yeast, fungi, plant cells, insect cells and mammalian cells. Thus, the invention provides methods for optimizing codon usage in all of these cells, codon-altered nucleic acids and polypeptides made by the codon-altered nucleic acids. Exemplary host cells include gram negative bacteria, such as *Escherichia coli*; gram positive bacteria, such as any *Bacillus* (e.g., *B. cereus or B. subtilis*) or *Streptomyces, Lactobacillus gasseri, Lactococcus lactis, Lactococcus cremoris.* Exemplary host cells also include eukaryotic organisms, e.g., various yeast, such as Saccharomyces sp., including *Saccharomyces cerevisiae, Schizosaccharomyces pombe, Pichia pastoris*, and *Kluyveromyces lactis, Hansenula polymorpha, Aspergillus niger*, and mammalian cells and cell lines and insect cells and cell lines. Thus, the invention also includes nucleic acids and polypeptides optimized for expression in these organisms and species.

For example, the codons of a nucleic acid encoding a hydrolase isolated from a bacterial cell are modified such that the nucleic acid is optimally expressed in a bacterial cell different from the bacteria from which the hydrolase was derived, a yeast, a fungi, a plant cell, an insect cell or a mammalian cell. Methods for optimizing codons are well known in the art, see, e.g., U.S. Patent No. 5,795,737; Baca (2000) Int. J. Parasitol. 30:113-118; Hale (1998) Protein Expr. Purif. 12:185-188; Narum (2001) Infect. Immun. 69:7250-7253. See also Narum (2001) Infect. Immun. 69:7250-7253, describing optimizing codons in mouse systems; Outchkourov (2002) Protein Expr. Purif. 24:18-24, describing optimizing codons in yeast; Feng (2000) Biochemistry 39:15399-15409, describing optimizing codons in E. coli; Humphreys (2000) Protein Expr. Purif. 20:252-264, describing optimizing codon usage that affects secretion in *E. coli.*

Transgenic non-human animals

The invention provides transgenic non-human animals comprising a nucleic acid, a polypeptide (e.g., a hydrolase or an antibody of the invention), an expression cassette, a vector, a transfected or a transformed cell of the invention. The transgenic non-human animals can be, e.g., goats, rabbits, sheep, pigs, cows, rats and mice, comprising the nucleic acids of the invention. These animals can be used, e.g., as *in vivo* models to study hydrolase activity, or, as models to screen for agents that change the hydrolase activity *in vivo.* The coding sequences for the polypeptides to be expressed in the transgenic non-human animals can be designed to be constitutive, or, under the control of tissue-specific, developmental-specific or inducible transcriptional regulatory factors. Transgenic non-human animals can be designed and generated using any method known in the art; see, e.g., U.S. Patent Nos. 6,211,428; 6,187,992; 6,156,952; 6,118,044; 6,111,166; 6,107,541; 5,959,171; 5,922,854; 5,892,070; 5,880,327; 5,891,698; 5,639,940; 5,573,933; 5,387,742; 5,087,571, describing making and using transformed cells and eggs and transgenic mice, rats, rabbits, sheep, pigs and cows. See also, e.g., Pollock (1999) J. Immunol. Methods 231:147-157, describing the production of recombinant proteins in the milk of transgenic dairy animals; Baguisi (1999) Nat. Biotechnol. 17:456-461, demonstrating the production of transgenic goats. U.S. Patent No. 6,211,428, describes making and using transgenic non-human mammals which express in their brains a nucleic acid construct comprising a DNA sequence. U.S. Patent No. 5,387,742, describes injecting cloned recombinant or synthetic DNA sequences into fertilized mouse eggs, implanting the injected eggs in pseudo-pregnant females, and growing to term transgenic mice whose cells express proteins related to the pathology of Alzheimer's disease. U.S. Patent No. 6,187,992, describes making and using a transgenic mouse whose genome comprises a disruption of the gene encoding amyloid precursor protein (APP).

"Knockout animals" can also be used to practice the methods of the invention. For example, in one aspect, the transgenic or modified animals of the invention comprise a "knockout animal," e.g., a "knockout mouse," engineered not to express an endogenous gene, which is replaced with a gene expressing a hydrolase of the invention, or, a fusion protein comprising a hydrolase of the invention. As noted above, functional knockouts can also be generated using antisense sequences of the invention, e.g., double-stranded RNAi molecules.

Transgenic Plants and Seeds

The invention provides transgenic plants and seeds comprising a nucleic acid, a polypeptide (e.g., a hydrolase or an antibody of the invention), an expression cassette or vector or a transfected or transformed cell of the invention. The transgenic plant can be dicotyledonous (a dicot) or monocotyledonous (a monocot). The invention also provides methods of making and using these transgenic plants and seeds. The transgenic plant or plant cell expressing a polypeptide of the present invention may be constructed in accordance with any method known in the art. See, for example, U.S. Patent No. 6,309,872.

Nucleic acids and expression constructs of the invention can be introduced into a plant cell by any means. For example, nucleic acids or expression constructs can be introduced into the genome of a desired plant host, or, the nucleic acids or expression constructs can be episomes. Introduction into the genome of a desired plant can be such that the host's hydrolase production is regulated by endogenous transcriptional or translational control elements. The invention also provides "knockout plants" where insertion of gene sequence by, e.g., homologous recombination, has disrupted the expression of the endogenous gene. Means to generate "knockout" plants are well-known in the art, see, e.g., Strepp (1998) Proc Natl. Acad. Sci. USA 95:4368-4373; Miao (1995) Plant J 7:359-365. See discussion on transgenic plants, below.

The nucleic acids of the invention can be used to confer desired traits on essentially any plant, e.g., on oilseed producing plants, including rice bran, rapeseed (canola), sunflower, olive, palm or soy, and the like, or on glucose or starch-producing plants, such as corn, potato, wheat, rice, barley, and the like. Nucleic acids of the invention can be used to manipulate metabolic pathways of a plant in order to optimize or alter host's expression of a hydrolase or a substrate or product of a hydrolase, e.g., an oil, a lipid, such as a mono-, di- or tri-acylglyceride and the like. The can change the ratios of lipids, lipid conversion and turnover in a plant. This can facilitate industrial processing of a plant. Alternatively, hydrolases of the invention can be used in production of a transgenic plant to produce a compound not naturally produced by that plant. This can lower production costs or create a novel product.

In one aspect, the first step in production of a transgenic plant involves making an expression construct for expression in a plant cell. These techniques are well known in the art. They can include selecting and cloning a promoter, a coding sequence for facilitating efficient binding of ribosomes to mRNA and selecting the appropriate gene terminator sequences. One exemplary constitutive promoter is CaMV35S, from the cauliflower mosaic virus, which generally results in a high degree of expression in plants. Other promoters are more specific and respond to cues in the plant's internal or external environment. An exemplary light-inducible promoter is the promoter from the cab gene, encoding the major chlorophyll a/b binding protein.

In one aspect, the nucleic acid is modified to achieve greater expression in a plant cell. For example, a sequence of the invention is likely to have a higher percentage of A-T nucleotide pairs compared to that seen in a plant, some of which prefer G-C nucleotide pairs. Therefore, A-T nucleotides in the coding sequence can be substituted with G-C nucleotides without significantly changing the amino acid sequence to enhance production of the gene product in plant cells.

Selectable marker gene can be added to the gene construct in order to identify plant cells or tissues that have successfully integrated the transgene. This may be necessary because achieving incorporation and expression of genes in plant cells is a rare event, occurring in just a few percent of the targeted tissues or cells. Selectable marker genes encode proteins that provide resistance to agents that are normally toxic to plants, such as antibiotics or herbicides. Only plant cells that have integrated the selectable marker gene will survive when grown on a medium containing the appropriate antibiotic or herbicide. As for other inserted genes, marker genes also require promoter and termination sequences for proper function.

In one aspect, making transgenic plants or seeds comprises incorporating sequences of the invention and, optionally, marker genes into a target expression construct (e.g., a plasmid, a phage), along with positioning of the promoter and the terminator sequences. This can involve transferring the modified gene into the plant through a suitable method. For example, a construct may be introduced directly into the genomic DNA of the plant cell using techniques such as electroporation and microinjection of plant cell protoplasts, or the constructs can be introduced directly to plant tissue using ballistic methods, such as DNA particle bombardment. For example, see, e.g., Christou (1997) Plant Mol. Biol. 35:197-203; Pawlowski (1996) Mol. Biotechnol. 6:17-30; Klein (1987) Nature 327:70-73; Takumi (1997) Genes Genet. Syst. 72:63-69, discussing use of particle bombardment to introduce transgenes into wheat; and Adam (1997) supra, for use of particle bombardment to introduce YACs into plant cells. For example, Rinehart (1997) supra, used particle bombardment to generate transgenic cotton plants. Apparatus for accelerating particles is described U.S. Pat. No. 5,015,580; and, the commercially available BioRad (Biolistics) PDS-2000 particle acceleration instrument; see also, John, U.S. Patent No. 5,608,148; and Ellis, U.S. Patent No. 5, 681,730, describing particle-mediated transformation of gymnosperms.

In one aspect, protoplasts can be immobilized and injected with a nucleic acids, e.g., an expression construct. Although plant regeneration from protoplasts is not easy with cereals, plant regeneration is possible in legumes using somatic embryogenesis from protoplast derived callus. Organized tissues can be transformed with naked DNA using gene gun technique, where DNA is coated on tungsten microprojectiles, shot 1/100th the size of cells, which carry the DNA deep into cells and organelles. Transformed tissue is then induced to regenerate, usually by somatic embryogenesis. This technique has been successful in several cereal species including maize and rice.

Nucleic acids, e.g., expression constructs, can also be introduced in to plant cells using recombinant viruses. Plant cells can be transformed using viral vectors, such as, e.g., tobacco mosaic virus derived vectors (Rouwendal (1997) Plant Mol. Biol. 33:989-999), see Porta (1996) "Use of viral replicons for the expression of genes in plants," Mol. Biotechnol. 5:209-221.

Alternatively, nucleic acids, e.g., an expression construct, can be combined with suitable T-DNA flanking regions and introduced into a conventional *Agrobacterium tumefaciens* host vector. The virulence functions of the *Agrobacterium tumefaciens* host will direct the insertion of the construct and adjacent marker into the plant cell DNA when the cell is infected by the bacteria. *Agrobacterium tumefaciens-mediated* transformation techniques, including disarming and use of binary vectors, are well described in the scientific literature. See, e.g., Horsch (1984) Science 233:496-498; Fraley (1983) Proc. Natl. Acad. Sci. USA 80:4803 (1983); Gene Transfer to Plants, Potrykus, ed. (Springer-Verlag, Berlin 1995). The DNA in an A. *tumefaciens* cell is contained in the bacterial chromosome as well as in another structure known as a Ti (tumor-inducing) plasmid. The Ti plasmid contains a stretch of DNA termed T-DNA (~20 kb long) that is transferred to the plant cell in the infection process and a series of vir (virulence) genes that direct the infection process. *A. tumefaciens* can only infect a plant through wounds: when a plant root or stem is wounded it gives off certain chemical signals, in response to which, the vir genes ofA. *iumefaciens* become activated and direct a series of events necessary for the transfer of the T-DNA from the Ti plasmid to the plant's chromosome. The T-DNA then enters the plant cell through the wound. One speculation is that the T-DNA waits until the plant DNA is being replicated or transcribed, then inserts itself into the exposed plant DNA. In order to use *A. tumefaciens* as a transgene vector, the tumor-inducing section of T-DNA have to be removed, while retaining the T-DNA border regions and the vir genes. The transgene is then inserted between the T-DNA border regions, where it is transferred to the plant cell and becomes integrated into the plant's chromosomes.

The invention provides for the transformation of monocotyledonous plants using the nucleic acids of the invention, including important cereals, see Hiei (1997) Plant Mol. Biol. 35:205-218. See also, e.g., Horsch, Science (1984) 233:496; Fraley (1983) Proc. Natl. Acad. Sci USA 80:4803; Thykjaer (1997) supra; Park (1996) Plant Mol. Biol. 32: 1135-1148, discussing T-DNA integration into genomic DNA. See also D'Halluin, U.S. Patent No. 5,712,135, describing a process for the stable integration of a DNA comprising a gene that is functional in a cell of a cereal, or other monocotyledonous plant.

In one aspect, the third step can involve selection and regeneration of whole plants capable of transmitting the incorporated target gene to the next generation. Such regeneration techniques rely on manipulation of certain phytohormones in a tissue culture growth medium, typically relying on a biocide and/or herbicide marker that has been introduced together with the desired nucleotide sequences. Plant regeneration from cultured protoplasts is described in Evans et al., Protoplasts Isolation and Culture, Handbook of Plant Cell Culture, pp. 124-176, MacMillilan Publishing Company, New York, 1983; and Binding, Regeneration of Plants, Plant Protoplasts, pp. 21-73, CRC Press, Boca Raton, 1985. Regeneration can also be obtained from plant callus, explants, organs, or parts thereof. Such regeneration techniques are described generally in Klee (1987) Ann. Rev. of Plant Phys. 38:467-486. To obtain whole plants from transgenic tissues such as immature embryos, they can be grown under controlled environmental conditions in a series of media containing nutrients and hormones, a process known as tissue culture. Once whole plants are generated and produce seed, evaluation of the progeny begins.

After the expression cassette is stably incorporated in transgenic plants, it can be introduced into other plants by sexual crossing. Any of a number of standard breeding techniques can be used, depending upon the species to be crossed. Since transgenic expression of the nucleic acids of the invention leads to phenotypic changes, plants comprising the recombinant nucleic acids of the invention can be sexually crossed with a second plant to obtain a final product. Thus, the seed of the invention can be derived from a cross between two transgenic plants of the invention, or a cross between a plant of the invention and another plant. The desired effects (e.g., expression of the polypeptides of the invention to produce a plant with altered, increased and/or decreased lipid or oil content) can be enhanced when both parental plants express the polypeptides of the invention. The desired effects can be passed to future plant generations by standard propagation means.

The nucleic acids and polypeptides of the invention are expressed in or inserted in any plant or seed. Transgenic plants of the invention can be dicotyledonous or monocotyledonous. Examples of monocot transgenic plants of the invention are grasses, such as meadow grass (blue grass, *Poa*), forage grass such as festuca, lolium, temperate grass, such as *Agrostis,* and cereals, e.g., wheat, oats, rye, barley, rice, sorghum, and maize (corn). Examples of dicot transgenic plants of the invention are tobacco, legumes, such as lupins, potato, sugar beet, pea, bean and soybean, and cruciferous plants (family *Brassicaceae*), such as cauliflower, rape seed, and the closely related model organism *Arabidopsis thaliana.* Thus, the transgenic plants and seeds of the invention include a broad range of plants, including, but not limited to, species from the genera *Anacardium. Arachis, Asparagus, Atropa, Avena, Brassica, Citrus, Citrullus, Capsicum, Carthamus, Cocos, Coffea, Cucumis, Cucurbita, Daucus, Elaeis, Fragaria, Glycine, Gossypium, Helianthus, Heterocallis, Hordeum, Hyoscyamus, Lactuca, Linum, Lolium, Lupinus, Lycopersicon, Malus, Manihot, Majorana, Medicago, Nicotiana, Olea, Oryza, Panieum, Pannisetum, Persea, Phaseolus, Pistachia, Pisum, Pyrus, Prunus, Raphanus, Ricinus, Secale, Senecio, Sinapis, Solanum, Sorghum, Theobromus, Trigonella, Triticum, Vicia, Vitis, Vigna*, and *Zea.*

In alternative embodiments, the nucleic acids of the invention are expressed in plants which contain fiber cells, including, *e.g.*, cotton, silk *cotton* tree (Kapok, Ceiba pentandra), desert willow, creosote bush, winterfat, balsa, ramie, kenaf, hemp, roselle, jute, sisal abaca and flax. In alternative embodiments, the transgenic plants of the invention can be members of the genus *Gossypium,* including members of any *Gossypium* species, such as *G. arboreum*;. *G. her-*

*baceum, G. barbadense,* and *G. hirsutum.*

The invention also provides for transgenic plants to be used for producing large amounts of the polypeptides (e.g., antibodies, hydrolases) of the invention. For example, see Palmgren (1997) Trends Genet. 13:348; Chong (1997) Transgenic Res. 6:289-296 (producing human milk protein beta-casein in transgenic potato plants using an auxin-inducible, bidirectional mannopine synthase (mas1',2') promoter with *Agrobacterium tumefaciens*-mediated leaf disc transformation methods).

Using known procedures, one of skill can screen for plants of the invention by detecting the increase or decrease of transgene mRNA or protein in transgenic plants. Means for detecting and quantitation ofmRNAs or proteins are well known in the art.

In one aspect, the invention produces fatty acids or fatty acid derivatives from transgenic plants of the invention, e.g., transgenic oleaginous plants. In one aspect, transgenic oleaginous plants comprising at least one hydrolase of the invention are produced. In one aspect, the transgenic plant comprises a hydrolase gene operably linked to a promoter, permitting an expression of the gene either in cellular, extracellular or tissue compartments other than those in which the plant lipids accumulate, or permitting exogenous induction of the hydrolase. In one aspect, seeds and/or fruits containing the lipids of the plants are collected, the seeds and/or fruits are crushed (if necessary after hydrolase (e.g., lipase) gene-induction treatment) so as to bring into contact the lipids and hydrolase of the invention contained in the seeds and/or fruits. The mixture can be allowed to incubate to allow enzymatic hydrolysis of the lipids of the ground material by catalytic action of the lipase of the invention contained in the crushed material. In one aspect, the fatty acids formed by the hydrolysis are extracted and/or are converted in order to obtain the desired fatty acid derivatives.

This enzymatic hydrolysis process of the invention uses mild operating conditions and can be small-scale and use inexpensive installations. In this aspect the plant of the invention is induced to produce the hydrolase for transformation of plant lipids. Using this strategy, the enzyme is prevented from coming into contact with stored plant lipids so as to avoid any risk of premature hydrolysis ("self-degradation of the plant") before harvesting. The crushing and incubating units can be light and small-scale; many are known in the agricultural industry and can be carried out at the sites where the plants are harvested.

In one aspect, transgenic plants of the invention are produced by transformation of natural oleaginous plants. The genetically transformed plants of the invention are then reproduced sexually so as to produce transgenic seeds of the invention. These seeds can be used to obtain transgenic plant progeny.

In one aspect, the hydrolase gene is operably linked to an inducible promoter to prevent any premature contact of hydrolase and plant lipid. This promoter can direct the expression of the gene in compartments other than those where the lipids accumulate or the promoter can initiate the expression of the hydrolase at a desired time by an exogenous induction.

Polypeptides and peptides

The invention provides isolated, synthetic or recombinant polypeptides having a sequence identity (e.g., at least 50% sequence identity) to an exemplary sequence of the invention, e.g., SEQ ID NO:2, SEQ ID NO:4, SEQ ID NO:6, SEQ ID NO:8, SEQ ID NO:10, SEQ ID NO:12, SEQ ID NO:14, SEQ ID NO:16, SEQ ID NO:18, SEQ ID NO:20, SEQ ID NO:22, SEQ ID NO:24, SEQ ID NO:26, SEQ ID NO:28, SEQ ID NO:30, SEQ ID NO:32, SEQ ID NO:34, SEQ ID NO:36, SEQ ID NO:38, SEQ ID NO:40, SEQ ID NO:42, SEQ ID NO:44, SEQ ID NO:46, SEQ ID NO:48, SEQ ID NO: 50, SEQ ID NO:52, SEQ ID NO:54, SEQ ID NO:56, SEQ ID NO:58, SEQ ID NO:60, SEQ ID NO:62, SEQ ID NO:64, SEQ ID NO:66, SEQ ID NO:68, SEQ ID NO:70, SEQ ID NO:72, SEQ ID NO:74, SEQ ID NO:76, SEQ ID NO:78, SEQ ID NO:80, SEQ ID NO:82, SEQ ID NO:84, SEQ ID NO:86, SEQ ID NO:88, SEQ ID NO:90, SEQ ID NO:92, SEQ ID NO: 94, SEQ ID NO:96, SEQ ID NO:98, SEQ ID NO:100, SEQ ID NO:102, SEQ ID NO:104, SEQ ID NO:106, SEQ ID NO: 108, SEQ ID NO:110, SEQ ID NO:112, SEQ ID NO:114, SEQ ID NO:116, SEQ ID NO:118, SEQ ID NO:120, SEQ ID NO:122, SEQ ID NO:124, SEQ ID NO:126, SEQ ID NO:128, SEQ ID NO:130, SEQ ID NO:132, SEQ ID NO:134, SEQ ID NO:136, SEQ ID NO:138, SEQ ID NO:140, SEQ ID NO:142, SEQ ID NO:143, SEQ ID NO:146, SEQ ID NO:148, SEQ ID NO:150, SEQ ID NO:152, SEQ ID NO:154, SEQ ID NO:156, SEQ ID NO:158, SEQ ID NO:160, SEQ ID NO: 162, SEQ ID NO:164 and/or SEQ ID NO:166. As discussed above, the identity can be over the full length of the polypeptide, or, the identity can be over a region of at least about 50, 60, 70, 80, 90, 100, 150, 200, 250, 300, 350, 400, 450, 500, 550, 600, 650, 700 or more residues. Polypeptides of the invention can also be shorter than the full length of exemplary polypeptides. In one aspect, the invention provides a polypeptide comprising only a subsequence of a sequence of the invention, exemplary subsequences can be about 5, 10, 15, 20, 25, 30, 35, 40, 45, 50, 55, 60, 65, 70, 75, 80, 85, 90, 100, 125, 150, 175, 200, 250, 300, 350, 400, 450, 500, 550, 600, 650, 700, or more residues. In alternative aspects, the invention provides polypeptides (peptides, fragments) ranging in size between about 5 and the full length of a polypeptide, e.g., an enzyme, such as a hydrolase, including an esterase, an acylase, a lipase, a phospholipase or a protease; exemplary sizes being of about 5, 10, 15, 20, 25, 30, 35, 40, 45, 50, 55, 60, 65, 70, 75, 80, 85, 90, 100, 125, 150, 175, 200, 250, 300, 350, 400, 450, 500, 550, 600, 650, 700, or more residues, e.g., contiguous residues of an exemplary hydrolase of the invention. Peptides of the invention can be useful as, e.g., labeling probes, antigens, toleragens, motifs, hydrolase active sites. Polypeptides of the invention also include antibodies capable of binding to a hydrolase of the invention.

As used herein, the term "hydrolase" encompasses polypeptides (e.g., antibodies, enzymes) and peptides (e.g., "active sites") having any hydrolase activity, i.e., the polypeptides of the invention can have any hydrolase activity, including lipase, esterase, phospholipase and/or protease activity.

The term "lipase" includes all polypeptides having any lipase activity, including lipid synthesis or lipid hydrolysis activity, i.e., the polypeptides of the invention can have any lipase activity. Lipases of the invention include enzymes active in the bioconversion of lipids through catalysis of hydrolysis, alcoholysis, acidolysis, esterification and aminolysis reactions. In one aspect, lipases of the invention can hydrolyze lipid emulsions. In one aspect, enzymes of the invention can act preferentially on sn-1 and/or sn-3 bonds of triglycerides to release fatty acids from the glycerol backbone. For example, lipase activity of the polypeptides of the invention include synthesis of cocoa butter, poly-unsaturated fatty acids (PUFAs), 1,3-diacyl glycerides (DAGs), 2-monoglycerides (MAGs) and triacylglycerides (TAGs). The term also includes lipases capable of isomerizing bonds at high temperatures, low temperatures, alkaline pHs and at acidic pHs.

The term "phospholipase" encompasses enzymes having any phospholipase activity, i.e., the polypeptides of the invention can have any phospholipase activity. For example, a phospholipase activity of the invention can comprise cleaving a glycerolphosphate ester linkage (catalyzing hydrolysis of a glycerolphosphate ester linkage), e.g., in an oil, such as a vegetable oil. A phospholipase activity of the invention can generate a water extractable phosphorylated base and a diglyceride. A phospholipase activity of the invention also includes hydrolysis of glycerolphosphate ester linkages at high temperatures, low temperatures, alkaline pHs and at acidic pHs. The term "a phospholipase activity" also includes cleaving a glycerolphosphate ester to generate a water extractable phosphorylated base and a diglyceride. The term "a phospholipase activity" also includes cutting ester bonds of glycerin and phosphoric acid in phospholipids. The term "a phospholipase activity" also includes other activities, such as the ability to bind to a substrate, such as an oil, e.g. a vegetable oil, substrate also including plant and animal phosphatidylcholines, phosphatidyl-ethanolamines, phosphatidylserines and sphingomyelins. The phospholipase activity can comprise a phospholipase C (PLC) activity, a phospholipase A (PLA) activity, such as a phospholipase A1 or phospholipase A2 activity, a phospholipase B (PLB) activity, such as a phospholipase B1 or phospholipase B2 activity, a phospholipase D (PLD) activity, such as a phospholipase D1 or a phospholipase D2 activity. The phospholipase activity can comprise hydrolysis of a glycoprotein, e.g., as a glycoprotein found in a potato tuber or any plant of the genus *Solanum,* e.g., *Solanum tuberosum.* The phospholipase activity can comprise a patatin enzymatic activity, such as a patatin esterase activity (see, e.g., Jimenez (2002) Biotechnol. Prog. 18:635-640). The phospholipase activity can comprise a lipid acyl hydrolase (LAH) activity.

The term "protease" includes all polypeptides having a protease activity, including a peptidase and/or a proteinase activity; i.e., the polypeptides of the invention can have any protease activity. A protease activity of the invention can comprise catalysis of the hydrolysis of peptide bonds. The proteases of the invention can catalyze peptide hydrolysis reactions in both directions. The direction of the reaction can be determined, e.g., by manipulating substrate and/or product concentrations, temperature, selection of protease and the like. The protease activity can comprise an endoprotease activity and/or an exoprotease activity. The protease activity can comprise a protease activity, e.g., a carboxypeptidase activity, a dipeptidylpeptidase or an aminopeptidase activity, a serine protease activity, a metalloproteinase activity, a cysteine protease activity and/or an aspartic protease activity. In one aspect, protease activity can comprise activity the same or similar to a chymotrypsin, a trypsin, an elastase, a kallikrein and/or a subtilisin activity

The term esterase includes all polypeptides having an esterase activity, i.e., the polypeptides of the invention can have any esterase activity. For example, the invention provides polypeptides capable of hydrolyzing ester groups to organic acids and alcohols. The term "esterase" also encompasses polypeptides having lipase activity (in the hydrolysis of lipids), acidolysis reactions (to replace an esterified fatty acid with a free fatty acid), trans-esterification reactions (exchange of fatty acids between triglycerides), ester synthesis and ester interchange reactions. In one aspect, the hydrolases of the invention can hydrolyze a lactone ring or acylate an acyl lactone or a diol lactone. The polypeptides of the invention can be enantiospecific, e.g., as when used in chemoenzymatic reactions in the synthesis of medicaments and insecticides. The polynucleotides of the invention encode polypeptides having esterase activity.

A hydrolase variant (e.g., "lipase variant", "esterase variant", "protease variant" "phospholipase variant") can have an amino acid sequence which is derived from the amino acid sequence of a "precursor". The precursor can include naturally-occurring hydrolase and/or a recombinant hydrolase. The amino acid sequence of the hydrolase variant is "derived" from the precursor hydrolase amino acid sequence by the substitution, deletion or insertion of one or more amino acids of the precursor amino acid sequence. Such modification is of the "precursor DNA sequence" which encodes the amino acid sequence of the precursor lipase rather than manipulation of the precursor hydrolase enzyme per se. Suitable methods for such manipulation of the precursor DNA sequence include methods disclosed herein, as well as methods known to those skilled in the art.

The polypeptides of the invention include hydrolases in an active or inactive form. For example, the polypeptides of the invention include proproteins before "maturation" or processing of prepro sequences, e.g., by a proprotein-processing enzyme, such as a proprotein convertase to generate an "active" mature protein. The polypeptides of the invention include hydrolases inactive for other reasons, e.g., before "activation" by a post-translational processing event, e.g., an endo- or exo-peptidase or proteinase action, a phosphorylation event, an amidation, a glycosylation or a sulfation, a dimerization event, and the like. Methods for identifying "prepro" domain sequences and signal sequences are well known in the art, see, e.g., Van de Ven (1993) Crit. Rev. Oncog. 4(2):115-136. For example, to identify a prepro sequence, the protein is purified from the extracellular space and the N-terminal protein sequence is determined and compared to the unprocessed form.

The polypeptides of the invention include all active forms, including active subsequences, e.g., catalytic domains or active sites, of an enzyme of the invention. In one aspect, the invention provides catalytic domains or active sites as set forth below. In one aspect, the invention provides a peptide or polypeptide comprising or consisting of an active site domain as predicted through use of a database such as Pfam (which is a large collection of multiple sequence alignments and hidden Markov models covering many common protein families, The Pfam protein families database, A. Bateman, E. Birney, L. Cerruti, R. Durbin, L. Etwiller, S.R. Eddy, S. Griffiths-Jones, K.L. Howe, M. Marshall, and E.L.L. Sonnhammer, Nucleic Acids Research, 30(1):276-280, 2002) or equivalent.

The invention includes polypeptides with or without a signal sequence and/or a prepro sequence. The invention includes polypeptides with heterologous signal sequences and/or prepro sequences. The prepro sequence (including a sequence of the invention used as a heterologous prepro domain) can be located on the amino terminal or the carboxy terminal end of the protein. The invention also includes isolated, synthetic or recombinant signal sequences, prepro sequences and catalytic domains (e.g., "active sites") comprising sequences of the invention.

Polypeptides and peptides of the invention can be isolated from natural sources, be synthetic, or be recombinantly generated polypeptides. Peptides and proteins can be recombinantly expressed *in vitro* or *in vivo.* The peptides and polypeptides of the invention can be made and isolated using any method known in the art. Polypeptide and peptides of the invention can also be synthesized, whole or in part, using chemical methods well known in the art. See e.g., Caruthers (1980) Nucleic Acids Res. Symp. Ser. 215-223; Horn (1980) Nucleic Acids Res. Symp. Ser. 225-232; Banga, A.K., Therapeutic Peptides and Proteins, Formulation, Processing and Delivery Systems (1995) Technomic Publishing Co., Lancaster, PA. For example, peptide synthesis can be performed using various solid-phase techniques (see e.g., Roberge (1995) Science 269:202; Merrifield (1997) Methods Enzymol. 289:3-13) and automated synthesis maybe achieved, e.g., using the ABI 431A Peptide Synthesizer (Perkin Elmer) in accordance with the instructions provided by the manufacturer.

The invention provides "amino acids" or "amino acid sequences" comprising (including) oligopeptides, peptides, polypeptides, or protein sequences, or fragments, portions or subunits of any of these, including naturally occurring or synthetic molecular forms thereof. The terms "polypeptide" and "protein" can include amino acids joined to each other by peptide bonds or modified peptide bonds, i.e., peptide isosteres, and may contain modified amino acids other than the 20 gene-encoded amino acids. The term "polypeptide" also includes peptides and polypeptide fragments, motifs and the like. The term also includes glycosylated polypeptides. The peptides and polypeptides of the invention also include all "mimetic" and "peptidomimetic" forms, as described in further detail, below.

The term "isolated" can mean that the material is removed from its original environment (e.g., the natural environment if it is naturally occurring). For example, a naturally occurring polynucleotide or polypeptide present in a living animal is not isolated, but the same polynucleotide or polypeptide, separated from some or all of the coexisting materials in the natural system, is isolated. Such polynucleotides could be part of a vector and/or such polynucleotides or polypeptides could be part of a composition, and still be isolated in that such vector or composition is not part of its natural environment. As used herein, an isolated material or composition can also be a "purified" composition, i.e., it does not require absolute purity; rather, it is intended as a relative definition. Individual nucleic acids obtained from a library can be conventionally purified to electrophoretic homogeneity. In alternative aspects, the invention provides nucleic acids which have been purified from genomic DNA or from other sequences in a library or other environment by at least one, two, three, four, five or more orders of magnitude.

The peptides and polypeptides of the invention can also be glycosylated. The glycosylation can be added post-translationally either chemically or by cellular biosynthetic mechanisms, wherein the later incorporates the use of known glycosylation motifs, which can be native to the sequence or can be added as a peptide or added in the nucleic acid coding sequence. The glycosylation can be O-linked or N-linked.

The peptides and polypeptides of the invention, as defined above, include all "mimetic" and "peptidomimetic" forms. The terms "mimetic" and "peptidomimetic" refer to a synthetic chemical compound which has substantially the same structural and/or functional characteristics of the polypeptides of the invention. The mimetic can be either entirely composed of synthetic, non-natural analogues of amino acids, or, is a chimeric molecule of partly natural peptide amino acids and partly non-natural analogs of amino acids. The mimetic can also incorporate any amount of natural amino acid conservative substitutions as long as such substitutions also do not substantially alter the mimetic's structure and/or activity. As with polypeptides of the invention which are conservative variants, routine experimentation will determine whether a mimetic is within the scope of the invention, i.e., that its structure and/or function is not substantially altered.

Thus, in one aspect, a mimetic composition is within the scope of the invention if it has a hydrolase activity.

Polypeptide mimetic compositions of the invention can contain any combination of non-natural structural components. In alternative aspect, mimetic compositions of the invention include one or all of the following three structural groups: a) residue linkage groups other than the natural amide bond ("peptide bond") linkages; b) non-natural residues in place of naturally occurring amino acid residues; or c) residues which induce secondary structural mimicry, i.e., to induce or stabilize a secondary structure, e.g., a beta turn, gamma turn, beta sheet, alpha helix conformation, and the like. For example, a polypeptide of the invention can be characterized as a mimetic when all or some of its residues are joined by chemical means other than natural peptide bonds. Individual peptidomimetic residues can be joined by peptide bonds, other chemical bonds or coupling means, such as, e.g., glutaraldehyde, N-hydroxysuccinimide esters, bifunctional maleimides, N,N'-dicyclohexylcarbodiimide (DCC) or N,N'-diisopropylcarbodiimide (DIC). Linking groups that can be an alternative to the traditional amide bond ("peptide bond") linkages include, e.g., ketomethylene (e.g., $-C(=O)-CH_2-$ for -C(=O)-NH-), aminomethylene ($CH_2$-NH), ethylene, olefin (CH=CH), ether ($CH_2$-O), thioether ($CH_2$-S), tetrazole ($CN_4$-), thiazole, retroamide, thioamide, or ester (see, e.g., Spatola (1983) in Chemistry and Biochemistry of Amino Acids, Peptides and Proteins, Vol. 7, pp 267-357, "Peptide Backbone Modifications," Marcell Dekker, NY).

A polypeptide of the invention can also be characterized as a mimetic by containing all or some non-natural residues in place of naturally occurring amino acid residues. Non-natural residues are well described in the scientific and patent literature; a few exemplary non-natural compositions useful as mimetics of natural amino acid residues and guidelines are described below. Mimetics of aromatic amino acids can be generated by replacing by, e.g., D- or L- naphylalanine; D- or L- phenylglycine; D- or L-2 thieneylalanine; D- or L-1, -2, 3-, or 4- pyreneylalanine; D- or L-3 thieneylalanine; D-or L-(2-pyridinyl)-alanine; D- or L-(3-pyridinyl)-alanine; D- or L-(2-pyrazinyl)-alanine; D- or L-(4-isopropyl)-phenylglycine; D-(trifluoromethyl)-phenylglycine; D-(trifluoromethyl)-phenylalanine; D-p-fluoro-phenylalanine; D- or L-p-biphenylphenylalanine; D- or L-p-methoxy-biphenylphenylalanine; D- or L-2-indole(alkyl)alanines; and, D- or L-alkylainines, where alkyl can be substituted or unsubstituted methyl, ethyl, propyl, hexyl, butyl, pentyl, isopropyl, iso-butyl, sec-isotyl, iso-pentyl, or a non-acidic amino acids. Aromatic rings of a non-natural amino acid include, e.g., thiazolyl, thiophenyl, pyrazolyl, benzimidazolyl, naphthyl, furanyl, pyrrolyl, and pyridyl aromatic rings.

Mimetics of acidic amino acids can be generated by substitution by, e.g., non-carboxylate amino acids while maintaining a negative charge; (phosphono)alanine; sulfated threonine. Carboxyl side groups (e.g., aspartyl or glutamyl) can also be selectively modified by reaction with carbodiimides (R'-N-C-N-R') such as, e.g., 1-cyclohexyl-3(2-morpholinyl-(4-ethyl) carbodiimide or 1-ethyl-3(4-azonia- 4,4-dimetholpentyl) carbodiimide. Aspartyl or glutamyl can also be converted to asparaginyl and glutaminyl residues by reaction with ammonium ions. Mimetics of basic amino acids can be generated by substitution with, e.g., (in addition to lysine and arginine) the amino acids ornithine, citrulline, or (guanidino)-acetic acid, or (guanidino)alkyl-acetic acid, where alkyl is defined above. Nitrile derivative (e.g., containing the CN-moiety in place of COOH) can be substituted for asparagine or glutamine. Asparaginyl and glutaminyl resides can be deaminated to the corresponding aspartyl or glutamyl residues. Arginine residue mimetics can be generated by reacting arginyl with, e.g., one or more conventional reagents, including, e.g., phenylglyoxal, 2,3-butanedione, 1,2-cyclohexanedione, or ninhydrin, preferably under alkaline conditions. Tyrosine residue mimetics can be generated by reacting tyrosyl with, e.g., aromatic diazonium compounds or tetranitromethane. N-acetylimidizol and tetranitromethane can be used to form O-acetyl tyrosyl species and 3-nitro derivatives, respectively. Cysteine residue mimetics can be generated by reacting cysteinyl residues with, e.g., alpha-haloacetates such as 2-chloroacetic acid or chloroacetamide and corresponding amines; to give carboxymethyl or carboxyamidomethyl derivatives. Cysteine residue mimetics can also be generated by reacting cysteinyl residues with, e.g., bromo-trifluoroacetone, alpha-bromo-beta-(5-imidozoyl) propionic acid; chloroacetyl phosphate, N-alkylmaleimides, 3-nitro-2-pyridyl disulfide; methyl 2-pyridyl disulfide; p-chloromercuribenzoate; 2-chloromercuri-4 nitrophenol; or, chloro-7-nitrobenzo-oxa-1,3-diazole. Lysine mimetics can be generated (and amino terminal residues can be altered) by reacting lysinyl with, e.g., succinic or other carboxylic acid anhydrides. Lysine and other alpha-amino-containing residue mimetics can also be generated by reaction with imidoesters, such as methyl picolinimidate, pyridoxal phosphate, pyridoxal, chloroborohydride, trinitro-benzenesulfonic acid, O-methylisourea, 2,4, pentanedione, and transamidase-catalyzed reactions with glyoxylate. Mimetics of methionine can be generated by reaction with, e.g., methionine sulfoxide. Mimetics of proline include, e.g., pipecolic acid, thiazolidine carboxylic acid, 3- or 4- hydroxy proline, dehydroproline, 3- or 4-methylproline, or 3,3,-dimethylproline. Histidine residue mimetics can be generated by reacting histidyl with, e.g., diethylprocarbonate or para-bromophenacyl bromide. Other mimetics include, e.g., those generated by hydroxylation of proline and lysine; phosphorylation of the hydroxyl groups of seryl or threonyl residues; methylation of the alpha-amino groups of lysine, arginine and histidine; acetylation of the N-terminal amine; methylation of main chain amide residues or substitution with N-methyl amino acids; or amidation of C-terminal carboxyl groups.

A residue, e.g., an amino acid, of a polypeptide of the invention can also be replaced by an amino acid (or peptidomimetic residue) of the opposite chirality. Thus, any amino acid naturally occurring in the L-configuration (which can also be referred to as the R or S, depending upon the structure of the chemical entity) can be replaced with the amino acid of the same chemical structural type or a peptidomimetic, but of the opposite chirality, referred to as the D- amino acid, but also can be referred to as the R- or S- form.

The invention also provides methods for modifying the polypeptides of the invention by either natural processes, such as post-translational processing (e.g., phosphorylation, acylation, etc), or by chemical modification techniques, and the resulting modified polypeptides. Modifications can occur anywhere in the polypeptide, including the peptide backbone, the amino acid side-chains and the amino or carboxyl termini. It will be appreciated that the same type of modification may be present in the same or varying degrees at several sites in a given polypeptide. Also a given polypeptide may have many types of modifications. Modifications include acetylation, acylation, ADP-ribosylation, amidation, covalent attachment of flavin, covalent attachment of a heme moiety, covalent attachment of a nucleotide or nucleotide derivative, covalent attachment of a lipid or lipid derivative, covalent attachment of a phosphatidylinositol, cross-linking cyclization, disulfide bond formation, demethylation, formation of covalent cross-links, formation of cysteine, formation of pyroglutamate, formylation, gamma-carboxylation, glycosylation, GPI anchor formation, hydroxylation, iodination, methylation, myristolyation, oxidation, pegylation, proteolytic processing, phosphorylation, prenylation, racemization, selenoylation, sulfation, and transfer-RNA mediated addition of amino acids to protein such as arginylation. See, e.g., Creighton, T.E., Proteins - Structure and Molecular Properties 2nd Ed., W.H. Freeman and Company, New York (1993); Posttranslational Covalent Modification of Proteins, B.C. Johnson, Ed., Academic Press, New York, pp. 1-12 (1983).

Solid-phase chemical peptide synthesis methods can also be used to synthesize the polypeptides, or fragments thereof, of the invention. Such method have been known in the art since the early 1960's (Merrifield, R. B., J. Am. Chem. Soc., 85:2149-2154, 1963) (See also Stewart, J. M. and Young, J. D., Solid Phase Peptide Synthesis, 2nd Ed., Pierce Chemical Co., Rockford, Ill., pp. 11-12)) and have recently been employed in commercially available laboratory peptide design and synthesis kits (Cambridge Research Biochemicals). Such commercially available laboratory kits have generally utilized the teachings ofH. M. Geysen et al, Proc. Natl. Acad. Sci., USA, 81:3998 (1984) and provide for synthesizing peptides upon the tips of a multitude of "rods" or "pins" all of which are connected to a single plate. When such a system is utilized, a plate of rods or pins is inverted and inserted into a second plate of corresponding wells or reservoirs, which contain solutions for attaching or anchoring an appropriate amino acid to the pin's or rod's tips. By repeating such a process step, i.e., inverting and inserting the rod's and pin's tips into appropriate solutions, amino acids are built into desired peptides. In addition, a number of available FMOC peptide synthesis systems are available. For example, assembly of a polypeptide or fragment can be carried out on a solid support using an Applied Biosystems, Inc. Model 431A™ automated peptide synthesizer. Such equipment provides ready access to the peptides of the invention, either by direct synthesis or by synthesis of a series of fragments that can be coupled using other known techniques.

*Enzymes of the invention*

The invention provides novel hydrolases, including esterases, acylases, lipases, phospholipases or proteases, e.g., proteins comprising at least about 50% sequence identity to an exemplary polypeptide of the invention, e.g., a protein having a sequence as set forth in SEQ ID NO:2, SEQ ID NO:4, SEQ ID NO:6, SEQ ID NO:8, etc., through to SEQ ID NO:166, and antibodies that bind them, and methods for making and using them. The polypeptides of the invention can have any hydrolase activity, e.g., an esterase, acylase, lipase, phospholipase or protease activity. In alternative aspects, an activity of an enzyme of the invention comprises hydrolysis or synthesis of lipids or oils. The hydrolases of the invention can modify oils by hydrolysis, alcoholysis, esterification, transesterification and/or interesterification, including "forced migration" reactions.

In alternative aspects, the hydrolases of the invention can have modified or new activities as compared to the exemplary hydrolases or the activities described herein. For example, the invention includes hydrolases with and without signal sequences and the signal sequences themselves. The invention includes immobilized hydrolases, anti-hydrolase antibodies and fragments thereof. The invention provides proteins for inhibiting hydrolase activity, e.g., antibodies that bind to the hydrolase active site. The invention includes homodimers and heterocomplexes, e.g., fusion proteins, heterodimers, etc., comprising the hydrolases of the invention. The invention includes hydrolases having activity over a broad range of high and low temperatures and pH's (e.g., acidic and basic aqueous conditions).

In one aspect, one or more hydrolases (e.g., lipases) of the invention is used for the biocatalytic synthesis of structured lipids, i.e., lipids that contain a defined set of fatty acids distributed in a defined manner on the glycerol backbone, including cocoa butter alternatives, poly-unsaturated fatty acids (PUFAs), 1,3-diacyl glycerides (DAGs), 2-monoglycerides (MAGs) and triacylglycerides (TAGs).

In one aspect, the invention provides methods of generating enzymes having altered (higher or lower) $K_{cat}/K_m$. In one aspect, site-directed mutagenesis is used to create additional hydrolase enzymes with alternative substrate specificities. The can be done, for example, by redesigning the substrate binding region or the active site of the enzyme. In one aspect, hydrolases of the invention are more stable at high temperatures, such as 80°C to 85°C to 90°C to 95°C, as compared to hydrolases from conventional or moderate organisms.

Various proteins of the invention have a hydrolase activity, e.g., an esterase, acylase, lipase, phospholipase or protease activity, under various conditions. The invention provides methods of making hydrolases with different catalytic efficiency and stabilities towards temperature, oxidizing agents and pH conditions. These methods can use, e.g., the techniques of site-directed mutagenesis and/or random mutagenesis. In one aspect, directed evolution can be used to produce hydrolases with alternative specificities and stability.

The proteins of the invention are used in methods of the invention that can identify hydrolase modulators, e.g., activators or inhibitors. Briefly, test samples (e.g., compounds, such as members of peptide or combinatorial libraries, broths, extracts, and the like) are added to hydrolase assays to determine their ability to modulate, e.g., inhibit or activate, substrate cleavage. These inhibitors can be used in industry and research to reduce or prevent undesired isomerization. Modulators found using the methods of the invention can be used to alter (e.g., decrease or increase) the spectrum of activity of a hydrolase.

The invention also provides methods of discovering hydrolases using the nucleic acids, polypeptides and antibodies of the invention. In one aspect, lambda phage libraries are screened for expression-based discovery of hydrolases. In one aspect, the invention uses lambda phage libraries in screening to allow detection of toxic clones; improved access to substrate; reduced need for engineering a host, by-passing the potential for any bias resulting from mass excision of the library; and, faster growth at low clone densities. Screening of lambda phage libraries can be in liquid phase or in solid phase. In one aspect, the invention provides screening in liquid phase. This gives a greater flexibility in assay conditions; additional substrate flexibility; higher sensitivity for weak clones; and ease of automation over solid phase screening.

The invention provides screening methods using the proteins and nucleic acids of the invention involving robotic automation. This enables the execution of many thousands of biocatalytic reactions and screening assays in a short period of time, e.g., per day, as well as ensuring a high level of accuracy and reproducibility (see discussion of arrays, below). As a result, a library of derivative compounds can be produced in a matter of weeks.

The invention includes hydrolase enzymes which are non-naturally occurring hydrolases having a different hydrolase activity, stability, substrate specificity, pH profile and/or performance characteristic as compared to the non-naturally occurring hydrolase. These hydrolases have an amino acid sequence not found in nature. They can be derived by substitution of a plurality of amino acid residues of a precursor hydrolase with different amino acids. The precursor hydrolase may be a naturally-occurring hydrolase or a recombinant hydrolase. In one aspect, the hydrolase variants encompass the substitution of any of the naturally occurring L-amino acids at the designated amino acid residue positions.

The following chart describes selected characteristics of exemplary nucleic acids and polypeptides of the invention, including sequence identity comparison of the exemplary sequences to public databases. All sequences described in these Table are exemplary sequences of the invention, and all have been subject to a BLAST search (as described in detail, below) against two sets of databases. The first database set is available through NCBI (National Center for Biotechnology Information). All results from searches against these databases are found in the columns entitled "NR Description", "NR Accession Code", "NR Evalue" or "NR Organism". "NR" refers to the Non-Redundant nucleotide database maintained by NCBI. This database is a composite of GenBank, GenBank updates, and EMBL updates. The entries in the column "NR Description" refer to the definition line in any given NCBI record, which includes a description of the sequence, such as the source organism, gene name/protein name, or some description of the function of the sequence. The entries in the column "NR Accession Code" refer to the unique identifier given to a sequence record. The entries in the column "NR Evalue" refer to the Expect value (Evalue), which represents the probability that an alignment score as good as the one found between the query sequence (the sequences of the invention) and a database sequence would be found in the same number of comparisons between random sequences as was done in the present BLAST search. The entries in the column "NR Organism" refer to the source organism of the sequence identified as the closest BLAST hit, and also indicate an exemplary enzymatic activity of the designated sequence of the invention. For example, in the first column, second row, the chart reads that the polypeptide having the sequence of SEQ ID NO:4, encoded e.g., by SEQ ID NO:3, can have by homology and/or source, inter alia, a para-nitrobenzyl esterase activity.

The second set of databases is collectively known as the GENESEQ™ database, which is available through Thomson Derwent (Philadelphia, PA). All results from searches against this database are found in the columns entitled "GENESEQ™ Protein Description", "GENESEQ™ Protein Accession Code", "GENESEQ™ Protein Evalue", "GENESEQ™ DNA Description", "GENESEQ™ DNA Accession Code" or "GENESEQ™ DNA Evalue". The information found in these columns is comparable to the information found in the NR columns described above, except that it was derived from BLAST searches against the GENESEQ™ database instead of the NCBI databases.

For example, in the first column, 6th row, the chart reads that the polypeptide having the sequence of SEQ ID NO:2, encoded e.g., by SEQ ID NO:1, can have, as analyzed by homology and/or source, inter alia, a hydrolase activity.

In addition, this table includes the column "Predicted EC No.". An EC number is the number assigned to a type of enzyme according to a scheme of standardized enzyme nomenclature developed by the Enzyme Commission of the Nomenclature Committee of the International Union of Biochemistry and Molecular Biology (IUBMB). The results in the "Predicted EC No." column are determined by a BLAST search against the Kegg (Kyoto Encyclopedia of Genes and Genomes) database. If the top BLAST match has an Evalue equal to or less than $e^{-6}$, the EC number assigned to the top match is entered into the table. The EC number of the top hit is used as a guide to what the EC number of the sequence of the invention might be. The columns "Query DNA Length" and "Query Protein Length" refer to the number of nucleotides or the number amino acids, respectively, in the sequence of the invention that was searched or queried against either the NCBI or GENESEQ™ databases. The columns "GENESEQ™ or NR DNA Length" and "GENESEQ™ or NR Protein Length" refer to the number of nucleotides or the number amino acids, respectively, in the sequence of the top match from the BLAST search. The results provided in these columns are from the search that returned the lower Evalue, either from the NCBI databases or the Geneseq database. The columns "GENESEQ™ or NR %ID Protein" and "GENESES™ or NR %ID DNA" refer to the percent sequence identity between the sequence of the invention and the sequence of the top BLAST match. The results provided in these columns are from the search that returned the lower Evalue, either from the NCBI databases or the GENESEQ™ database.

| SEQ ID NO: | NR Description | NR Accession Code | NR Evalue | NR Organism | Geneseq Protein Description | Geneseq Protein Accession Code | Geneseq Protein Evalue | Geneseq DNA Description | Geneseq DNA Accession Code |
|---|---|---|---|---|---|---|---|---|---|
| 1, 2 | hypothetical protein MED134_02625 [Cellulophaga sp. MED134] gi\|85817680\|gb\|EAQ38854.1\| hypothetical protein MED134_02625 [Cellulophaga sp. MED134] | 86131858 | 2.00E-42 | Cellulophaga sp. MED134 | Environmental isolate hydrolase, SEQ ID NO:44. | AEH47778 | 1.00E-153 | Environmental isolate hydrolase, SEQ ID NO:44. | AEH47777 |
| 3, 4 | para-nitrobenzyl esterase [Candidatus Desulfococcus oleovorans Hxd3] gi\|121519426\|gb\|EAX56274.1\| para-nitrobenzyl esterase [Candidatus Desulfococcus oleovorans Hxd3] | 121540632 | 4.00E-76 | Candidatus Desulfococcus oleovorans Hxd3 | Thermus DNA encoding a thermostable esterase, TspA/E101. | AAU01849 | 3.00E-71 | Streptomyces tautomycetin polyketide synthase enzyme ORF4. | AEE75855 |
| 5, 6 | \|1QZ3\|A Chain A; Crystal Structure Of Mutant M211sR215L OF CARBOXYLESTERASE Est2 Complexed With Hexadecanesulfonate | 47168664 | 1.00E-178 | | Environmental isolate hydrolase, SEQ ID NO:44. | AEH47202 | 0 | Eukaryotic peptide chain release factor 3. | AEJ56197 |
| 7, 8 | Patatin [Thiomicrospira denitrificans ATCC 33889] | 78777519 | 2.00E-20 | Thiomicrospira denitrificans ATCC 33889 | Environmental isolate hydrolase, SEQ ID NO:44. | AEH46928 | 1.00E-145 | Environmental isolate hydrolase, SEQ ID NO:44. | AEH46927 |
| 9, 10 | carboxylesterase (estA) [Archaeoglobus fulgidus]. | 11499305 | 1.00E-176 | Archaeoglobus fulgidus | Environmental isolate hydrolase, SEQ ID NO:44. | AEH47868 | 1.00E-177 | Environmental isolate hydrolase, SEQ ID NO:44. | AEH47867 |

(continued)

| SEQ ID NO: | NR Description | NR Accession Code | NR Evalue | NR Organism | Geneseq Protein Description | Geneseq Protein Accession Code | Geneseq Protein Evalue | Geneseq DNA Description | Geneseq DNA Accession Code |
|---|---|---|---|---|---|---|---|---|---|
| 11, 12 | carboxylesterase [Oceanobacillus iheyensis]. | 23099884 | 1.00E-92 | Oceanobacillu s iheyensis | Environmental isolate hydrolase, SEQ ID NO:44. | AEH47646 | 1.00E-142 | Environmental isolate hydrolase, SEQ ID NO:44. | AEH47645 |
| 13, 14 | carboxylesterase [Oceanobacillus iheyensis]. | 23099884 | 7.00E-93 | Oceanobacillu s iheyensis | DNA encoding hydrolase BD423. | ABG31303 | 1.00E-142 | DNA encoding hydrolase BD423. | ABK89958 |
| 15, 16 | Esterase/lipase-like [Parvibaculum lavamentivorans DS-1] gi\|121298676\|gb\|EAX39865.1\| Esterase/lipase-like [Parvibaculum lavamentivorans DS-1] | 121525729 | 1.00E-50 | Parvibaculum lavamentivora ns DS-1 | Environmental isolate hydrolase, SEQ ID NO:44. | AEH47084 | 1.00E-178 | Environmental isolate hydrolase, SEQ ID NO:44. | AEH47083 |
| 17, 18 | Alpha/beta hydrolase fold-3 domain protein [Marinobacter aquaeolei VT8] gi\|120324929\|gb\|ABM19244.1\| Alpha/beta hydrolase fold-3 domain protein [Marinobacter aquaeolei VT8] | 120555080 | 0 | Marinobacter aquaeolei VT8 | Environmental isolate hydrolase, SEQ ID NO:44. | AEH47276 | 0 | Environmental isolate hydrolase, SEQ ID NO:44. | AEH47275 |
| 19, 20 | acylaminoacyl-peptidase [Geobacillus kaustophilus HTA426] | 56419496 | 0 | Geobacillus kaustophilus HTA426 | Environmental isolate hydrolase, SEQ ID NO:44. | AEH47848 | 0 | Environmental isolate hydrolase, SEQ ID NO:44. | AEH47847 |

(continued)

| SEQ ID NO: | NR Description | NR Accession Code | NR Evalue | NR Organism | Geneseq Protein Description | Geneseq Protein Accession Code | Geneseq Protein Evalue | Geneseq DNA Description | Geneseq DNA Accession Code |
|---|---|---|---|---|---|---|---|---|---|
| 21, 22 | Alpha/beta hydrolase fold-3 domain protein [Burkholderia multivorans ATCC 17616] gi\|118658329\|gb\|EAV65076.1\| Alpha/beta hydrolase fold-3 domain protein [Burkholderia multivorans ATCC 17616] | 118718703 | 5.00E-91 | Burkholderia multivorans ATCC 17616 | Environmental isolate hydrolase, SEQ ID NO:44. | AEH47802 | 1.00E-179 | Environmental isolate hydrolase, SEQ ID NO:44. | AEH47801 |
| 23, 24 | Beta-lactamase [Caulobacter sp. K31] gi\|13730265\|gb\|EAU11337.1\| Beta-lactamase [Caulobacter sp. K31] | 113935431 | 5.00E-98 | Caulobacter sp. K31 | Environmental isolate hydrolase, SEQ ID NO:44. | AEH47594 | 0 | Environmental isolate hydrolase, SEQ ID NO:44. | AEH47593 |
| 25, 26 | phospholipase/ carboxylesterase [Haloarcula marismortui ATCC 43049] | 55377970 | 6.00E-61 | Haloarcula marismortui ATCC 43049 | Bacterial polypeptide #10001. | ADS30648 | 5.00E-16 | Bacterial polypeptide #10001. | ADT44930 |
| 27, 28 | hypothetical protein AAur_4086 [Arthrobacter aurescens TC1] gi\|119949023\|gb\|ABM07934.1\| conserved hypothetical protein [Arthrobacter aurescens TC1] | 119962164 | 2.00E-11 | Arthrobacter aurescens TC1 | Environmental isolate hydrolase, SEQ ID NO:44. | AEH47748 | 2.00E-75 | Environmental isolate hydrolase, SEQ ID NO:44. | AEH47747 |
| 29, 30 | possible esterase/lipase [Rhodococcus sp. RHA1] | 111023814 | 6.00E-79 | Rhodococcus sp. RHA1 | Environmental isolate hydrolase, SEQ ID NO:44. | AEH47180 | 0 | Environmental isolate hydrolase, SEQ ID NO:44. | AEH47179 |

(continued)

| SEQ ID NO: | NR Description | NR Accession Code | NR Evalue | NR Organism | Geneseq Protein Description | Geneseq Protein Accession Code | Geneseq Protein Evalue | Geneseq DNA Description | Geneseq DNA Accession Code |
|---|---|---|---|---|---|---|---|---|---|
| 31, 32 | possible esterase/lipase [Rhodococcus sp. RHA1] | 111023814 | 2.00E-78 | Rhodococcus sp. RHA1 | Environmental isolate hydrolase, SEQ ID NO:44. | AEH47232 | 0 | Environmental isolate hydrolase, SEQ ID NO:44. | AEH47231 |
| 33, 34 | alpha/beta hydrolase fold [Sphingopyxis alaskensis RB2256] | 103487703 | 7.00E-67 | Sphingopyxis alaskensis RB2256 | Environmental isolate hydrolase, SEQ ID NO:44. | AEH47698 | 1.00E-170 | Environmental isolate hydrolase, SEQ ID NO:44. | AEH47697 |
| 35, 36 | Esterase/lipase/thioesterase [Ralstonia eutropha JMP134] | 73539284 | 4.00E-73 | Ralstonia eutropha JMP134 | Environmental isolate hydrolase, SEQ ID NO:44. | AEH47648 | 0 | Environmental isolate hydrolase, SEQ ID NO:44. | AEH47647 |
| 37, 38 | putative lipase; SCN_31 [Streptomyces cinnamonensis]. | 29123008 | 2.00E-48 | Streptomyces cinnamonensis | Environmental isolate hydrolase, SEQ ID NO:44. | AEH47456 | 0 | Environmental isolate hydrolase, SEQ ID NO:44. | AEH47455 |
| 39, 40 | esterase [uncultured bacterium] | 66356146 | 1.00E-112 | uncultured bacterium | Environmental isolate hydrolase, SEQ ID NO:44. | AEH47294 | 0 | Anti-blofilm polypeptide #7. | ADR51278 |
| 41, 42 | carboxylesterase family protein [uncultured bacterium 105] | 40062502 | 8.00E-39 | uncultured bacterium 105 | Environmental isolate hydrolase, SEQ ID NO:44. | AEH47196 | 0 | Anti-biofilm polypeptide #7. | ADR51264 |

(continued)

| SEQ ID NO: | NR Description | NR Accession Code | NR Evalue | NR Organism | Geneseq Protein Description | Geneseq Protein Accession Code | Geneseq Protein Evalue | Geneseq DNA Description | Geneseq DNA Accession Code |
|---|---|---|---|---|---|---|---|---|---|
| 43, 44 | Alpha/beta hydrolase fold-3 domain protein [Nocardioides sp. JS614] gi\|119537678\|gb\|ABL82295.1\| Alpha/beta hydrolase fold-3 domain protein [Nocardioides sp. JS614] | 119717017 | 2.00E-19 | Nocardioides sp. JS614 | Environmental isolate hydrolase, SEQ ID NO:44. | AEH47278 | 0 | Environmental isolate hydrolase, SEQ ID NO:44. | AEH47277 |
| 45, 46 | Esterase/lipase [Lactobacillus brevis ATCC 367] | 116333009 | 7.00E-46 | Lactobacillus brevis ATCC 367 | Environmental isolate hydrolase, SEQ ID NO:44. | AEH47660 | 1.00E-173 | Environmental isolate hydrolase, SEQ ID NO:44. | AEH47659 |
| 47, 48 | conserved hypothetical protein [Caulobacter sp. K31] gi\|113733419\|gb\|EAU14487.1\| conserved hypothetical protein [Caulobacter sp. K31] | 113933198 | 1.00E-139 | Caulobacter sp. K31 | Environmental isolate hydrolase, SEQ ID NO:44. | AEH47058 | 0 | Environmental isolate hydrolase, SEQ ID NO:44. | AEH47057 |
| 49, 50 | Esterase/lipase-like [Parvibaculum lavamentivorans DS-1] gi\|121298676\|gb\|EAX39865.1\| Esterase/lipase-like [Parvibaculum lavamentivorans DS-1] | 121525729 | 4.00E-57 | Parvibaculum lavamentivora ns DS-1 | Environmental isolate hydrolase, SEQ ID NO:44. | AEH47204 | 1.00E-148 | Environmental isolate hydrolase, SEQ ID NO:44. | AEH47203 |
| 51, 52 | esterase [Acinetobacter lwoffii]. | 21070428 | 1.00E-172 | Acinetobacter lwoffii | Environmental isolate hydrolase, SEQ ID NO:44. | AEH47096 | 0 | Environmental isolate hydrolase, SEQ ID NO:44. | AEH47095 |

(continued)

| SEQ ID NO: | NR Description | NR Accession Code | NR Evalue | NR Organism | Geneseq Protein Description | Geneseq Protein Accession Code | Geneseq Protein Evalue | Geneseq DNA Description | Geneseq DNA Accession Code |
|---|---|---|---|---|---|---|---|---|---|
| 53, 54 | hypothetical protein DSY1047 [Desulfitobacterium hafniense Y51] | 89893793 | 4.00E-58 | Desulfitobacterium hafniense Y51 | Environmental isolate hydrolase, SEQ ID NO:44, | AEH47828 | 0 | Environmental isolate hydrolase, SEQ ID NO:44. | AEH47827 |
| 55, 56 | esterase/tipase/thioesterase [Parvibaculum lavamentivorans DS-1] gi\|121297416\|gb\|EAX38605.1\| esterase/lipase/thioesterase [Parvibaculum lavamentivorans DS-1] | 121524469 | 2.00E-67 | Parvibaculum lavamentivora ns DS-1 | Environmental isolate hydrolase, SEQ ID NO:44. | AEH47628 | 0 | Environmental isolate hydrolase, SEQ ID NO:44. | AEH47627 |
| 57, 58 | Esterase/lipase/thioesterase [Ralstonia eutropha JMP134] | 73539284 | 9.00E-84 | Ralstonia eutropha JMP134 | Environmental isolate hydrolase, SEQ ID NO:44. | AEH47556 | 1.00E-177 | Environmental isolate hydrolase, SEQ ID NO:44. | AEH47555 |
| 59, 60 | esterase [Acinetobacter Iwoffi]. | 21070428 | 1.00E-137 | Acinetobacter Iwoffil | Environmental isolate hydrolase, SEQ ID NO:44. | AEH47512 | 0 | Environmental isolate hydrolase, SEQ ID NO:44. | AEH47511 |
| 61, 62 | esterase [Bacillus sp. NRRL B-14911] gi\|89088253\|gb\|EAR67363.1\| esterase [Bacillus sp. NRRL B-14911] | 89096873 | 1.00E-108 | Bacillus sp. NRRL B-14911 | Environmental isolate hydrolase, SEQ ID NO:44. | AEH47712 | 1.00E-140 | Environmental isolate hydrolase, SEQ ID NO:44. | AEH47711 |

(continued)

| SEQ ID NO: | NR Description | NR Accession Code | NR Evalue | NR Organism | Geneseq Protein Description | Geneseq Protein Accession Code | Geneseq Protein Evalue | Geneseq DNA Description | Geneseq DNA Accession Code |
|---|---|---|---|---|---|---|---|---|---|
| 63, 64 | putative lipase [Symbiobacterium thermophilum IAM 14863] | 51893263 | 2.00E-72 | Symbiobacterium thermophilum IAM 14863 | Environmental isolate hydrolase, SEQ ID NO:44. | AEH47200 | 1.00E-178 | Environmental isolate hydrolase, SEQ ID NO:44. | AEH47199 |
| 65, 66 | putative xylanase [Flavobacterium johnsoniae UW101] gi\|90434170\|gb\|EAS59531.1\| putative xylanase [Flavobacterium johnsoniae UW101] | 90590243 | 2.00E-75 | Flavobacterium johnsoniae UW101 | Environmental isolate hydrolase, SEQ ID NO:44. | AEH47418 | 1.00E-179 | Environmental isolate hydrolase, SEQ ID NO:44. | AEH47417 |
| 67, 68 | hypothetical protein Saro_0907 [Novosphingobium aromaticivorans DSM 12444] | 87198929 | 1.00E-166 | Novosphingobium aromaticivorans DSM 12444 | Environmental isolate hydrolase, SEQ ID NO:44. | AEH47236 | 0 | Environmental isolate hydrolase, SEQ ID NO:44. | AEH47235 |
| 69, 70 | hypothetical protein MGP2080_07112 [marine gamma proteobacterium HTCC2080] gi\|119461403\|gb\|EAW42493.1\| hypothetical protein MGP2080_07112 [marine gamma proteobacterium HTCC2080] | 119503057 | 2.00E-30 | marine gamma proteobacterium HTCC2080 | Mycobacterium tuberculosis mycobacterial antigen protein SEQ ID NO:5. | ABM15916 | 2.00E-22 | Plasmid pTJS75: dxS:dX r:Tn5Kn. | ABL53861 |
| 71, 72 | Alpha/beta hydrolase fold-3 [Rhodoferax ferrireducens T118] | 89901193 | 3.00E-94 | Rhodoferax ferrireducens T118 | Environmental isolate hydrolase, SEQ ID NO:44. | AEH47754 | 0 | Environmental Isolate hydrolase, SEQ ID NO:44. | AEH47753 |

(continued)

| SEQ ID NO: | NR Description | NR Accession Code | NR Evalue | NR Organism | Geneseq Protein Description | Geneseq Protein Accession Code | Geneseq Protein Evalue | Geneseq DNA Description | Geneseq DNA Accession Code |
|---|---|---|---|---|---|---|---|---|---|
| 73, 74 | Alpha/beta hydrolase fold [Ralstonia eutropha JMP134] | 73538759 | 4.00E-79 | Ralstonia eutropha JMP134 | Environmental isolate hydrolase, SEQ ID NO:44. | AEH47160 | 1.00E-174 | Environmental isolate hydrolase, SEQ ID NO:44. | AEH47159 |
| 75, 76 | hydrolase, alpha/beta hydrolase fold family [Caulobacter crescentus]. | 16126469 | 1.00E-74 | Caulobacter crescentus | Environmental isolate hydrolase, SEQ ID NO:44. | AEH47426 | 1.00E-147 | Environmental isolate hydrolase, SEQ ID NO:44. | AEH47425 |
| 77, 78 | hydrolase, alpha/beta hydrolase fold family [Caulobacter crescentus]. | 16124639 | 4.00E-66 | Caulobacter crescentus | Environmental isolate hydrolase, SEQ ID NO:44. | AEH47076 | 4.00E-57 | Environmental isolate hydrolase, SEQ ID NO:44. | AEH47425 |
| 79, 80 | alpha/beta hydrolase [Sulfitobacter sp. EE-36] gi\|83846434\|gb\|EAP84310.1\| alpha/beta hydrolase [Sulfitobacter sp. EE-36] | 83942341 | 1.00E-87 | Sulfitobacter sp. EE-36 | Environmental isolate hydrolase, SEQ ID NO:44. | AEH47466 | 0 | Environmental isolate hydrolase, SEQ ID NO:44. | AEH47465 |
| 81, 82 | probable endo-1,4-beta-xylanase B [Robiginitalea biformata HTCC2501] gi\|88783781\|gb\|EAR14952.1\| probable endo-1,4-beta-xylanase B [Robiginitalea biformata HTCC2501] | 88806172 | 4.00E-41 | Robiginitalea biformata HTCC2501 | Environmental isolate hydrolase, SEQ ID NO:44. | AEH47600 | 0 | Environmental isolate hydrolase, SEQ ID NO:44. | AEH47599 |

(continued)

| SEQ ID NO: | NR Description | NR Accession Code | NR Evalue | NR Organism | Geneseq Protein Description | Geneseq Protein Accession Code | Geneseq Protein Evalue | Geneseq DNA Description | Geneseq DNA Accession Code |
|---|---|---|---|---|---|---|---|---|---|
| 83, 84 | Lipase (lipP-1) [Sulfolobus solfataricus]. | 15899234 | 1.00E-160 | Sulfolobus solfataricus | Environmental isolate hydrolase, SEQ ID NO:44. | AEH47870 | 1.00E-173 | Environmental isolate hydrolase, SEQ ID NO:44. | AEH47869 |
| 85, 86 | alpha/beta hydrolase fold [Delftia acidovorans SPH-1] gi\|18668165\|gb\|EAV74760.1\| alpha/beta hydrolase fold [Delftia acidovorans SPH-1] | 118731306 | 3.00E-15 | Delftia acidovorans SPH-1 | Environmental isolate hydrolase, SEQ ID NO:44. | AEH47404 | 1.00E-169 | Environmental isolate hydrolase, SEQ ID NO:44. | AEH47403 |
| 87, 88 | carboxylesterase family protein [Caulobacter crescentus]. | 16126537 | 4.00E-57 | Caulobacter crescentus | Environmental isolate hydrolase, SEQ ID NO:44. | AEH47572 | 1.00E-174 | Environmental isolate hydrolase, SEQ ID NO:44. | AEH47571 |
| 89, 90 | lipase/esterase [uncultured bacterium] | 45775279 | 1.00E-110 | uncultured bacterium | Environmental isolate hydrolase, SEQ ID NO:44. | AEH47120 | 1.00E-167 | Environmental isolate hydrolase, SEQ ID NO:44. | AEH47119 |
| 91, 92 | Hypothetical protein XCC2094 | 51702175 | 3.00E-91 | | Environmental isolate hydrolase, SEQ ID NO:44. | AEH47074 | 0 | Environmental isolate hydrolase, SEQ ID NO:44. | AEH47073 |

(continued)

| SEQ ID NO: | NR Description | NR Accession Code | NR Evalue | NR Organism | Geneseq Protein Description | Geneseq Protein Accession Code | Geneseq Protein Evalue | Geneseq DNA Description | Geneseq DNA Accession Code |
|---|---|---|---|---|---|---|---|---|---|
| 93, 94 | possible esterase [marine gamma proteobacterium HTCC2143] gi\|119451010\|gb\|EAW32243.1\| possible esterase [marine gamma proteobacterium HTCC2143] | 119474807 | 1.00E-113 | marine gamma proteobacteriu m HTCC2143 | DNA encoding hydrolase BD423. | ABG31307 | 1.00E-171 | DNA encoding hydrolase BD423. | ABK89962 |
| 95, 96 | putative lipase [Symbiobacterium thermophilum IAM 14863] | 51893263 | 2.00E-72 | Symbiobacterium thermophilum IAM 14863 | Environmental isolate hydrolase, SEQ ID NO:44. | AEH47366 | 1.00E-154 | Environmental isolate hydrolase, SEQ ID NO:44. | AEH47365 |
| 97, 98 | putative lipase [Symbiobacterium thermophilum IAM 14863] | 51893263 | 1.00E-101 | Symbiobacteri um thermophilum IAM 14863 | Environmental isolate hydrolase, SEQ ID NO:44. | AEH47138 | 1.00E-180 | Anti-biofilm polypeptide #7. | ADR51212 |
| 99, 100 | Esterase/lipase-like [Parvibaculum lavamentivorans DS-1] gi\|121298676\|gb\|EAX39865.1\| Esterase/lipase-like [Parvibaculum lavamentivorans DS-1] | 121525729 | 2.00E-57 | Parvibaculum lavamentivora ns DS-1 | Environmental isolate hydrolase, SEQ ID NO:44. | AEH47204 | 1.00E-152 | Environmental isolate hydrolase, SEQ ID NO:44. | AEH47203 |
| 101, 102 | Carboxyl esterase, a/b hydrolase [Clostridium acetobutylicum]. | 15004800 | 2.00E-69 | Clostridium acetobutylicum | Anti-Kaslauskas lipase protein. | ADO26338 | 3.00E-55 | Mouse ischaemic condition related cDNA sequence SEQ ID NO:1012. | ABI99360 |

(continued)

| SEQ ID NO: | NR Description | NR Accession Code | NR Evalue | NR Organism | Geneseq Protein Description | Geneseq Protein Accession Code | Geneseq Protein Evalue | Geneseq DNA Description | Geneseq DNA Accession Code |
|---|---|---|---|---|---|---|---|---|---|
| 103, 104 | Alpha/beta hydrolase fold-3 domain protein [Marinobacter aquaeolei VT8] gi\|120324929\|gb\|ABM19244.1\| Alpha/beta hydrolase fold-3 domain protein [Marinobacter aquaeolei VT8] | 120555080 | 0 | Marinobacter aquaeolei VT8 | Environmental isolate hydrolase, SEQ ID NO:44. | AEH47276 | 0 | Environmental isolate hydrolase, SEQ ID NO:44. | AEH47275 |
| 105, 106 | carboxylesterase [Oceanobacillus iheyensis]. | 23099884 | 6.00E-93 | Oceanobacillu s iheyensis | DNA encoding hydrolase BD423. | ABG31303 | 1.00E-143 | DNA encoding hydrolase BD423. | ABK89958 |
| 107, 108 | carboxylesterase [Oceanobacillus iheyensis]. | 23099884 | 2.00E-92 | Oceanobacillu s iheyensis | DNA encoding hydrolase BD423. | ABG31303 | 1.00E-143 | DNA encoding hydrolase BD423. | ABK89958 |
| 109, 110 | carboxylesterase [Oceanobacillus iheyensis]. | 23099884 | 2.00E-96 | Oceanobacillu s iheyensis | Environmental isolate hydrolase, SEQ ID NO:44. | AEH47382 | 1.00E-143 | Environmental isolate hydrolase, SEQ ID NO:44. | AEH47381 |
| 111, 112 | carboxylesterase [Oceanobacillus iheyensis]. | 23099884 | 6.00E-93 | Oceanobacillu s iheyensis | DNA encoding hydrolase BD423. | ABG31303 | 1.00E-143 | DNA encoding hydrolase BD423. | ABK89958 |
| 113, 114 | carboxylesterase [Oceanobacillus iheyensis]. | 23099884 | 7.00E-93 | Oceanobacillu s iheyensis | DNA encoding hydrolase BD423. | ABG31303 | 1.00E-142 | DNA encoding hydrolase BD423. | ABK89958 |

(continued)

| SEQ ID NO: | NR Description | NR Accession Code | NR Evalue | NR Organism | Geneseq Protein Description | Geneseq Protein Accession Code | Geneseq Protein Evalue | Geneseq DNA Description | Geneseq DNA Accession Code |
|---|---|---|---|---|---|---|---|---|---|
| 115, 116 | Putative exported phospholipase [Burkholderia xenovorans LB400] | 91784160 | 4.00E-88 | Burkholderia xenovorans LB400 | Environmental isolate hydrolase, SEQ ID NO:44. | AEH47102 | 1.00E-67 | Environmental isolate hydrolase, SEQ ID NO:44. | AEH47101 |
| 117, 118 | EstB [Geobacillus thermoleovorans] | 82791236 | 1.00E-91 | Geobacillus thermoleovora ns | Thermus DNA encoding a thermostable esterase, TspA/E101. | AAU01849 | 3.00E-76 | Thermostable esterase E101. | AAT86701 |
| 119, 120 | lipase [Geobacillus thermoleovorans]. | 4835874 | 1.00E-136 | Geobacillus thermoleovora ns | Environmental isolate hydrolase, SEQ ID NO:44. | AEH47562 | 1.00E-138 | Environmental isolate hydrolase, SEQ ID NO:44. | AEH47561 |
| 121, 122 | Alpha/beta hydrolase fold-3 domain protein [Metallosphaera sedula DSM 5348] gi\|118690043\|gb\|EAV96366.1\| Alpha/beta hydrolase fold-3 domain protein [Metallosphaera sedula DSM 5348] | 118751693 | 1.00E-170 | Metallosphaer a sedula DSM 5348 | DNA encoding hydrolase BD423. | ABG31304 | 1.00E-172 | DNA encoding hydrolase BD423. | ABK89959 |
| 123, 124 | Alpha/beta hydrolase fold [Dechloromonas aromatica RCB] | 71908444 | 3.00E-89 | Dechloromona s aromatica RCB | L. pneumophila protein SEQ ID NO 3367. | AEB35768 | 3.00E-51 | | |

(continued)

| SEQ ID NO: | NR Description | NR Accession Code | NR Evalue | NR Organism | Geneseq Protein Description | Geneseq Protein Accession Code | Geneseq Protein Evalue | Geneseq DNA Description | Geneseq DNA Accession Code |
|---|---|---|---|---|---|---|---|---|---|
| 125, 126 | conserved hypothetical protein [Xanthobacter autotrophicus Py2] gi\|89348870\|gb\|EAS14162.1\| conserved hypothetical protein [Xanthobacter autotrophicus Py2] | 89362291 | 4.00E-98 | Xanthobacter autotrophicus Py2 | Environmental isolate hydrolase, SEQ ID NO:44. | AEH47244 | 6.00E-04 | N. meningitidis vaccine antigen #48. | ABX09893 |
| 127, 128 | dienelactone hydrolase [Sphingomonas witticli RW1] gi\|118706350\|gb\|EAW05500.1\| dienelactone hydrolase [Sphingomonas wittichii RW1] | 118758435 | 3.00E-46 | Sphingomonas wittichli RW1 | Environmental Isolate hydrolase, SEQ ID NO:44. | AEH47808 | 4.00E-18 | Human polynucleotide SEQ ID NO 13646. | AAI86664 |
| 129, 130 | lysophospholipase A [Legionella pneumophila subsp. pneumophila str. Philadelphia 1] | 52842553 | 1.00E-48 | Legionella pneumophila subsp. pneumophila str. Philadelphia 1 | L. pneumophila protein SEQ ID NO 3367. | AEB38417 | 4.00E-49 | L. pneumophila protein SEQ ID NO 3367. | AEB39166 |
| 31, 32 | hypothetical protein Maqu_3412 [Marinobacter aquaeolei VT8] gi\|120326168\|gb\|ABM20483.1\| conserved hypothetical protein [Marinobacter aquaeolei VT8] | 120556319 | 1.00E-49 | Marinobacter aquaeolei VT8 | Environmental isolate hydrolase, SEQ ID NO:44. | AEH47172 | 1.00E-144 | Environmental isolate hydrolase, SEQ ID NO:44. | AEH47171 |
| 33, 34 | Patatin [Delftia acidovorans SPH-1] gi\|118666914\|gb\|EAV73512.1\| Patatin [Delftia acidovorans SPH-1] | 118733332 | 5.00E-32 | Delftia acidovorans SPH-1 | Environmental isolate hydrolase, SEQ ID NO:44. | AEH47304 | 1.00E-171 | Environmental isolate hydrolase, SEQ ID NO:44. | AEH47303 |

(continued)

| SEQ ID NO: | NR Description | NR Accession Code | NR Evalue | NR Organism | Geneseq Protein Description | Geneseq Protein Accession Code | Geneseq Protein Evalue | Geneseq DNA Description | Geneseq DNA Accession Code |
|---|---|---|---|---|---|---|---|---|---|
| 35, 36 | lipase/esterase [uncultured bacterium] | 45775279 | 6.00E-61 | uncultured bacterium | Environmental isolate hydrolase, SEQ ID NO:44. | AEH47120 | 3.00E-64 | Bifidobacterium longum NCC2705 ORF amino acid sequence SEQ ID NO:408. | ABQ81847 |
| 37, 38 | putative carboxylesterase [Salmonella typhimurium LT2]. | 16764967 | 0 | Salmonella typhimurium LT2 | Klebsiella pneumoniae polypeptide seqid 7178. | ABO62949 | 0 | Klebsiella pneumoniae polypeptideseqid 7178. | ACH96500 |
| 139, 140 | Esterase/lipase-like [Parvibaculum lavamentivorans DS-1] gi\|121298676\|gb\|EAX39865.1\| Esterase/lipase-like [Parvibaculum lavamentivorans DS-1] | 121525729 | 2.00E-57 | Parvibaculum lavamentivora ns DS-1 | Environmental isolate hydrolase, SEQ ID NO:44. | AEH47204 | 1.00E-152 | Environmental isolate hydrolase, SEQ ID NO:44. | AEH47203 |
| 141, 142 | esterase [Xylella fastidiosa 9a5c]. | 15838344 | 1.00E-129 | Xylella fastidiosa 9a5c | Environmental isolate hydrolase, SEQ ID NO:44. | AEH47436 | 1.00E-142 | Anti-biofilm polypeptide #7. | ADR51258 |
| 143, 144 | Putative esterase [Burkholderia xenovorans LB400] | 91780665 | 6.00E-30 | Burkholderia xenovorans LB400 | Non-ribosomal peptide synthetase reductase domain #2. | ABJ37468 | 1.00E-26 | Primer RG277 for canarypox virus C3 open reading frame. | AAT47564 |

(continued)

| SEQ ID NO: | NR Description | NR Accession Code | NR Evalue | NR Organism | Geneseq Protein Description | Geneseq Protein Accession Code | Geneseq Protein Evalue | Geneseq DNA Description | Geneseq DNA Accession Code |
|---|---|---|---|---|---|---|---|---|---|
| 145, 146 | conserved hypothetical protein [Pyrobaculum aerophilum]. | 18314165 | 2.00E-86 | Pyrobaculum aerophilum | Environmental isolate hydrolase, SEQ ID NO:44. | AEH47814 | 0.006 | Staphylococcus aureus DNA for cellular proliferation protein #1219. | AAS51218 |
| 147, 148 | putative esterase [Thermus thermophilus HB27] | 46199208 | 1.00E-165 | Thermus thermophilus HB27 | Thermus DNA encoding a thermostable esterase, TspA/E101. | AAU01852 | 1.00E-147 | Thermostable esterase E101. | AAT86704 |
| 149, 150 | alpha/beta hydrolase [Parvibaculum lavamentivorans DS-1] gi\|121299709\|gb\|EAX40898.1\| alpha/beta hydrolase [Parvibaculum lavamentivorans DS-1] | 121526762 | 1.00E-140 | Parvibaculum lavamentivora ns DS-1 | Environmental isolate hydrolase, SEQ ID NO:44. | AEH47072 | 1.00E-140 | Environmental isolate hydrolase, SEQ ID NO:44. | AEH47071 |
| 151, 152 | hypothetical protein BT4075 [Bacteroides thetiotaomicron VPI-5482] | 29349483 | 2.00E-21 | Bacteroides thetaiotaomicr on VPI-5482 | Prokaryotic essential gene #34740. | ABU24763 | 1.00E-08 | Human AR gene SEQ ID NO:5. | ADS89487 |
| 153, 154 | Enoyl-CoA hydratase/ isomerase [Mesorhizobium sp. BNC1] | 110632842 | 3.00E-74 | Mesorhizobiu m sp. BNC1 | Bacterial polypeptide #10001. | ADN24902 | 3.00E-20 | Mycobacterium tuberculosis antigen TbRa13 encoding DNA. | AAT91405 |

(continued)

| SEQ ID NO: | NR Description | NR Accession Code | NR Evalue | NR Organism | Geneseq Protein Description | Geneseq Protein Accession Code | Geneseq Protein Evalue | Geneseq DNA Description | Geneseq DNA Accession Code |
|---|---|---|---|---|---|---|---|---|---|
| 155, 156 | Alpha/beta hydrolase fold-3 domain protein [Burkholderia cenocepacia H12424] | 116688189 | 9.00E-92 | Burkholderia cenocepacia H12424 | Environmental isolate hydrolase, SEQ ID NO:44. | AEH47416 | 2.00E-22 | Peptide, to probe the function of PKS genes in ansamitocin biosynthesis. | AAL61225 |
| 157, 158 | hypothetical protein CHGG_ 05597 [Chaetomium globosum CBS 148.51] | 116191759 | 7.00E-95 | Chaetomium globosum CBS 148.51 | Xylanase from an environmental sample seq id 14. | ADJ34868 | 4.00E-58 | Streptomyces hygroscopicus non-ribosomal peptide synthetase complex DNA. | ADE86070 |
| 159, 160 | probable lipase/esterase [Rhodopirellula baltica SH 1] | 32472501 | 6.00E-33 | Rhodopirellula baltica SH 1 | Environmental isolate hydrolase, SEQ ID NO:44. | AEH47492 | 1.00E-161 | Environmental isolate hydrolase, SEQ ID NO:44. | AEH47491 |
| 161, 162 | Patatin [Chlorobium limicola DSM 245] gi\|67784038\|gb\|EAM43418.1\| Patatin [Chlorobium limicola DSM 245] | 67918382 | 6.00E-44 | Chlorobium limicola DSM 245 | Environmental isolate hydrolase, SEQ ID NO:44. | AEH47216 | 6.00E-43 | Pseudomonas aeruginosa polypeptide #3. | ABD05045 |
| 163, 164 | carboxylesterase [Oceanobacillus iheyensis HTE831]. | 22778115 | | Oceanobacillu s iheyensis HTE831 | | | | | |
| 165, 166 | Outer membrane autotransporter barrel [Pseudomonas fluorescens PfO-1] | 77461338 | 0 | Pseudomonas fluorescens PfO-1 | Pseudomonas aeruginosa esterase, estA. | ADZ79322 | 0 | Pseudomonas aeruginosa polypeptide #3. | ABD17863 |

| SEQ ID NO: | NR Description | Geneseq DNA Accession Code | Geneseq DNA Evalue | Predicted EC Number | Query DNA Length | Query Protein Length | Geneseq/NR DNA Length | Geneseq/NR Protein Length | Geneseq/NR %ID Protein | Geneseq/NR %ID DNA |
|---|---|---|---|---|---|---|---|---|---|---|
| 1, 2 | hypothetical protein MED134_02625 [Cellulophaga sp. MED134] gi\|85817680\|gb\|EAQ38854.1\| hypothetical protein MED134_02625 [Cellulophaga sp. MED134] | AEH47777 | 0 | 3.4.21. | 828 | 275 | 828 | 275 | | |
| 3, 4 | para-nitrobenzyl esterase [Candidatus Desulfococcus oleovorans Hxd3] gi\|121519426\|gb\|EAX56274.1\| para-nitrobenzyl esterase [Candidatus Desulfococcus oleovorans Hxd3] | AEE75855 | 3.00E-10 | 3.1.1.1 | 1665 | 554 | 0 | 551 | | |
| 5, 6 | \|1QZ3\|A Chain A; Crystal Structure Of Mutant M211sR215L OF CARBOXYLESTERASE Est2 Complexed With Hexadecanesulfonate | AEJ56197 | 0 | 3.1.. | 933 | 310 | 933 | 310 | | |
| 7, 8 | Patatin [Thiomicrospira denitrificans ATCC 33889] | AEH46927 | 0 | 3.4.21. | 780 | 259 | 780 | 259 | | |
| 9, 10 | carboxylesterase (estA) [Archaeoglobus fulgidus]. | AEH47867 | 0 | 3.1.1.1 | 927 | 308 | 927 | 308 | | |
| 11, 12 | carboxylesterase [Oceanobacillus iheyensis]. | AEH47645 | 0 | 3.1.1.1 | 747 | 248 | 747 | 248 | | |

(continued)

| SEQ ID NO: | NR Description | Geneseq DNA Accession Code | Geneseq DNA Evalue | Predicted EC Number | Query DNA Length | Query Protein Length | Geneseq/NR DNA Length | Geneseq/NR Protein Length | Geneseq/NR %ID Protein | Geneseq/NR %ID DNA |
|---|---|---|---|---|---|---|---|---|---|---|
| 13, 14 | carboxylesterase [Oceanobacillus iheyensis]. | ABK89958 | 0 | 3.1.1.1 | 747 | 248 | 747 | 248 | | |
| 15, 16 | Esterase/lipase-like [Parvibaculum lavamentivorans DS-1] gi\|121298676\|gb\|EAX39865.1\| Esterase/lipase-like [Parvibaculum lavamentivorans DS-1] | AEH47083 | 0 | | 924 | 307 | 924 | 307 | | |
| 17, 18 | Alpha/beta hydrolase fold-3 domain protein [Marinobacter aquaeolei VT8] gi\|120324929\|gb\|ABM19244.1\| Alpha/beta hydrolase fold-3 domain protein [Marinobacter aquaeolei VT8] | AEH47275 | 0 | | 1050 | 349 | 0 | 349 | | |
| 19, 20 | acylaminoacyl-peptidase [Geobacillus kaustophilus HTA426] | AEH47847 | 0 | 3.4.21. | 2022 | 673 | 0 | 673 | 99 | |
| 21, 22 | Alpha/beta hydrolase fold-3 domain protein [Burkholderia multivorans ATCC 17616] gi\|118658329\|gb\|EAV65076.1\| Alpha/beta hydrolase fold-3 domain protein [Burkholderia multivorans ATCC 17616] | AEH47801 | 0 | 3.1.. | 930 | 309 | 1020 | 339 | | |

(continued)

| SEQ ID NO: | NR Description | Geneseq DNA Accession Code | Geneseq DNA Evalue | Predicted EC Number | Query DNA Length | Query Protein Length | Geneseq/NR DNA Length | Geneseq/NR Protein Length | Geneseq/NR %ID Protein | Geneseq/NR %ID DNA |
|---|---|---|---|---|---|---|---|---|---|---|
| 23, 24 | Beta-lactamase [Caulobacter sp. K31] gi\|113730265\|gb\|EAU11337.1\| Beta-lactamase [Caulobacter sp. K31] | AEH47593 | 0 | 3.5.2.6 | 1152 | 383 | 1152 | 383 | | |
| 25, 26 | phospholipase/ carboxylesterase [Haloarcula marismortui ATCC 43049] | ADT44930 | 0.002 | | 669 | 222 | 0 | 212 | 49 | |
| 27, 28 | hypothetical protein AAur_4086 [Arthrobacter aurescens TC1] gi\|119949023\|gb\|ABM07934.1\| conserved hypothetical protein [Arthrobacter aurescens TC1] | AEH47747 | 0 | | 408 | 135 | 408 | 135 | | |
| 29, 30 | possible esterase/lipase [Rhodococcus sp. RHA1] | AEH47179 | 0 | 3.1.1.1 | 1245 | 414 | 1245 | 414 | | |
| 31, 32 | possible esterase/lipase [Rhodococcus sp. RHA1] | AEH47231 | 0 | 3.1.1.1 | 1245 | 414 | 1245 | 414 | | |
| 33, 34 | alpha/beta hydrolase fold [Sphingopyxis alaskensis RB2256] | AEH47697 | 0 | | 900 | 299 | 900 | 299 | | |
| 35, 36 | Esterase/lipase/thioesterase [Ralstonia eutropha JMP134] | AEH47647 | 0 | 3.1.. | 942 | 313 | 942 | 313 | | |
| 37, 38 | putative lipase; SCN_31 [Streptomyces cinnamonensis]. | AEH47455 | 0 | 3.1.1.3 | 957 | 318 | 957 | 318 | | |
| 39, 40 | esterase [uncultured bacterium] | ADR51278 | 0 | 3.1.1. | 1092 | 363 | 1092 | 363 | | |

(continued)

| SEQ ID NO: | NR Description | Geneseq DNA Accession Code | Geneseq DNA Evalue | Predicted EC Number | Query DNA Length | Query Protein Length | Geneseq/NR DNA Length | Geneseq/NR Protein Length | Geneseq/NR %ID Protein | Geneseq/NR %ID DNA |
|---|---|---|---|---|---|---|---|---|---|---|
| 41, 42 | carboxylesterase family protein [uncultured bacterium 105] | ADR51264 | 0 | 3.1.1.1 | 1005 | 334 | 1050 | 349 | | |
| 43, 44 | Alpha/beta hydrolase fold-3 domain protein [Nocardioides sp. JS614] gi\|119537678\|gb\|ABL82295.1\| Alpha/beta hydrolase fold-3 domain protein [Nocardioides sp. JS614] | AEH47277 | 0 | 3.1.1.1 | 1044 | 347 | 1044 | 347 | | |
| 45, 46 | Esterase/lipase [Lactobacillus brevis ATCC 367] | AEH47659 | 0 | | 885 | 294 | 885 | 294 | | |
| 47, 48 | conserved hypothetical protein [Caulobacter sp. K31] gi\|113733419\|gb\|EAU14487.1\| conserved hypothetical protein [Caulobacter sp. K31] | AEH47057 | 0 | | 960 | 319 | 960 | 319 | | |
| 49, 50 | Esterase/lipase-like [Parvibaculum lavamentivorans DS-1] gi\|121298676\|gb\|EAX39865.1\| Esterase/lipase-like [Parvibaculum lavamentivorans DS-1] | AEH47203 | 0 | | 897 | 298 | 810 | 269 | | |
| 51, 52 | esterase [Acinetobacter lwoffii]. | AEH47095 | 0 | 3.1.. | 1071 | 356 | 1071 | 356 | | |

(continued)

| SEQ ID NO: | NR Description | Geneseq DNA Accession Code | Geneseq DNA Evalue | Predicted EC Number | Query DNA Length | Query Protein Length | Geneseq/NR DNA Length | Geneseq/NR Protein Length | Geneseq/NR %ID Protein | Geneseq/NR %ID DNA |
|---|---|---|---|---|---|---|---|---|---|---|
| 53, 54 | hypothetical protein DSY1047 [Desulfitobacterium hafniense Y51] | AEH47827 | 0 | | 1047 | 348 | 1047 | 348 | | |
| 55, 56 | esterase/lipase/thioesterase [Parvibaculum lavamentivorans DS-1] gi\|121297416\|gb\|EAX38605.1\| esterase/lipase/thioesterase [Parvibaculum lavamentivorans DS-1] | AEH47627 | 0 | 3.1.1. | 948 | 315 | 948 | 315 | | |
| 57, 58 | Esterase/lipase/thioesterase [Ralstonia eutropha JMP134] | AEH47555 | 0 | 3.1.. | 927 | 308 | 927 | 308 | | |
| 59, 60 | esterase [Acinetobacter lwoffii]. | AEH47511 | 0 | 3.1.1.1 | 1071 | 356 | 1071 | 356 | | |
| 61, 62 | esterase [Bacillus sp. NRRL B-14911] gi\|89088253\|gb\|EAR67363.1\| esterase [Bacillus sp. NRRL B-14911] | AEH41711 | 0 | 3.1.1.1 | 750 | 249 | 750 | 249 | | |
| 63, 64 | putative lipase [Symbiobacterium thermophilum IAM 14863] | AEH47199 | 0 | 3.1.. | 945 | 314 | 945 | 314 | | |

(continued)

| SEQ ID NO: | NR Description | Geneseq DNA Accession Code | Geneseq DNA Evalue | Predicted EC Number | Query DNA Length | Query Protein Length | Geneseq/NR DNA Length | Geneseq/NR Protein Length | Geneseq/NR %ID Protein | Geneseq/NR %ID DNA |
|---|---|---|---|---|---|---|---|---|---|---|
| 65, 66 | putauve xylatrase [Flavobacterium johnsoniae UW101] gi\|90434170\|gb\|EAS59531.1\| putative xylanase [Flavobacterium johnsonlae UW101] | AEH47417 | 0 | | 930 | 309 | 930 | 309 | | |
| 67, 68 | hypothetical protein Saro_0907 [Novosphingobium aromaticivorans DSM 12444] | AEH47235 | 0 | | 1137 | 378 | 1137 | 378 | | |
| 69, 70 | hypothetical protein MGP2080_07112 [marine gamma proteobacterium HTCC2080] gi\|119461403\|gb\|EAW42493.1\| hypothetical protein MGP2080_07112 [marine gamma proteobacterium HTCC2080] | ABL53861 | 8.4 | | 807 | 268 | 0 | 233 | | |
| 71, 72 | Alpha/beta hydrolase fold-3 [Rhodoferax ferrireducens T118] | AEH47753 | 0 | | 1098 | 365 | 1098 | 365 | | |
| 73, 74 | Alpha/beta hydrolase fold [Ralstonia eutropha JMP134] | AEH47159 | 0 | 3.1.1.24 | 930 | 309 | 930 | 309 | | |
| 75, 76 | hydrolase, alpha/beta hydrolase fold family [Caulobacter crescentus]. | AEH47425 | 0 | | 906 | 301 | 795 | 264 | | |

(continued)

| SEQ ID NO: | NR Description | Geneseq DNA Accession Code | Geneseq DNA Evalue | Predicted EC Number | Query DNA Length | Query Protein Length | Geneseq/NR DNA Length | Geneseq/NR Protein Length | Geneseq/NR %ID Protein | Geneseq/NR %ID DNA |
|---|---|---|---|---|---|---|---|---|---|---|
| 77, 78 | hydrolase, alpha/beta hydrolase fold family [Caulobacter crescentus]. | AEH47425 | 4.00E-05 | | 936 | 311 | 831 | 276 | 45 | 56 |
| 79, 80 | alpha/beta hydrolase [Sulfitobacter sp. EE-36] gi\|83846434\|gb\|EAP84310.1\| alpha/beta hydrolase [Sulfitobacter sp. EE-36] | AEH47465 | 0 | 3... | 960 | 319 | 969 | 322 | | |
| 81, 82 | probable endo-1,4-beta-xylanase B [Robiginitalea biformata HTCC2501] gi\|88783781\|gb\|EAR14952.1\| probable endo-1,4-beta-xylanase B [Robiginitalea biformata HTCC2501] | AEH47599 | 0 | | 969 | 322 | 969 | 322 | | |
| 83, 84 | Lipase (lipP-1) [Sulfolobus solfataricus]. | AEH47869 | 0 | 3.1.1.1 | 918 | 305 | 918 | 305 | | |
| 85, 86 | alpha/beta hydrolase fold [Delftia acidovorans SPH-1] gi\|118668165\|gb\|EAV74760.1\| alpha/beta hydrolase fold [Delftia acidovorans SPH-1] | AEH47403 | 0 | 3.1.1.1 | 885 | 294 | 885 | 294 | | |
| 87, 88 | carboxylesterase family protein [Caulobacter crescentus]. | AEH47571 | 0 | 3.1.1.1 | 891 | 296 | 891 | 296 | | |
| 89, 90 | lipase/esterase [uncultured bacterium] | AEH47119 | 0 | | 891 | 296 | 891 | 296 | | |

(continued)

| SEQ ID NO: | NR Description | Geneseq DNA Accession Code | Geneseq DNA Evalue | Predicted EC Number | Query DNA Length | Query Protein Length | Geneseq/NR DNA Length | Geneseq/NR Protein Length | Geneseq/NR %ID Protein | Geneseq/NR %ID DNA |
|---|---|---|---|---|---|---|---|---|---|---|
| 91, 92 | Hypothetical protein XCC2094 | AEH47073 | 0 | | 1578 | 525 | 1578 | 525 | | |
| 93, 94 | possible esterase [marine gamma proteobacterium HTCC2143] gi\|119451010\|gb\|EAW32243.1\| possible esterase [marine gamma proteobacterium HTCC2143] | ABK89962 | 0 | 3.1.1. | 990 | 329 | 1065 | 354 | | |
| 95, 96 | putative lipase [Symbiobacterium thermophilum IAM 14863] | AEH47365 | ####### | 3.1.. | 924 | 307 | 1038 | 345 | | |
| 97, 98 | putative lipase [Symbiobacterium thermophilum IAM 14863] | ADR51212 | 0 | 3.1.1. | 939 | 312 | 1095 | 364 | | |
| 99, 100 | Esterase/lipase-like [Parvibaculum lavamentivorans DS-1] gi\|121298676\|gb\|EAX39865.1\| Esterase/lipase-like [Parvibaculum lavamentivorans DS-1] | AEH47203 | 0 | | 1038 | 345 | 810 | 269 | | |
| 101, 102 | Carboxyl esterase, a/b hydrolase [Clostridium acetobutylicum]. | ABI99360 | 2.4 | | 906 | 301 | 900 | 299 | 44 | 52 |

(continued)

| SEQ ID NO: | NR Description | Geneseq DNA Accession Code | Geneseq DNA Evalue | Predicted EC Number | Query DNA Length | Query Protein Length | Geneseq/NR DNA Length | Geneseq/NR Protein Length | Geneseq/NR %ID Protein | Geneseq/NR %ID DNA |
|---|---|---|---|---|---|---|---|---|---|---|
| 103, 104 | Alpha/beta hydrolase fold-3 domain protein [Marinobacter aquaeolei VT8] gi\|120324929\|gb\|ABM19244.1\| Alpha/beta hydrolase fold-3 domain protein [Marinobacter aquaeolei VT8] | AEH47275 | 0 | | 948 | 315 | 0 | 349 | | |
| 105, 106 | carboxylesterase [Oceanobacillus iheyensis]. | ABK89958 | 0 | 3.1.1.1 | 747 | 248 | 747 | 248 | | |
| 107, 108 | carboxylesterase [Oceanobacillus iheyensis]. | ABK89958 | 0 | 3.1.1.1 | 747 | 248 | 747 | 248 | | |
| 109, 110 | carboxylesterase [Oceanobacillus iheyensis]. | AEH47381 | 0 | 3.1.1.1 | 750 | 249 | 750 | 249 | | |
| 111, 112 | carboxylesterase [Oceanobacillus iheyensis]. | ABK89958 | 0 | 3.1.1.1 | 747 | 248 | 747 | 248 | | |
| 113, 114 | carboxylesterase [Oceanobacillus iheyensis]. | ABK89958 | 0 | 3.1.1.1 | 747 | 248 | 747 | 248 | | |
| 115, 116 | Putative exported phospholipase [Burkholderia xenovorans LB400] | AEH47101 | 2.00E-04 | 3.4.21. | 957 | 318 | 0 | 315 | 55 | |
| 117, 118 | EstB [Geobacillus thermoleovorans] | AAT86701 | 1.00E-80 | 3.1.1.1 | 483 | 160 | 0 | 175 | 98 | |
| 119, 120 | lipase [Geobacillus thermoleovorans]. | AEH47561 | 0 | | 714 | 237 | 1251 | 416 | | |

(continued)

| SEQ ID NO: | NR Description | Geneseq DNA Accession Code | Geneseq DNA Evalue | Predicted EC Number | Query DNA Length | Query Protein Length | Geneseq/NR DNA Length | Geneseq/NR Protein Length | Geneseq/NR %ID Protein | Geneseq/NR %ID DNA |
|---|---|---|---|---|---|---|---|---|---|---|
| 121, 122 | Alpha/beta hydrolase fold-3 domain protein [Metallosphaera sedula DSM 5348] gi\|118690043\|gb\|EAV96366.1\| Alpha/beta hydrolase fold-3 domain protein [Metallosphaera sedula DSM 5348] | ABK89959 | 0 | 3.1.1.1 | 906 | 301 | 906 | 301 | | |
| 123, 124 | Alpha/beta hydrolase fold [Dechloromonas aromatic RCB] | | 0 | 3... | 990 | 329 | 0 | 284 | 50 | |
| 125, 126 | conserved hypothetical protein [Xanthobacter autotrophicus Py2] gi\|89348870\|gb\|EAS14162.1\| conserved hypothetical protein [Xanthobacter autotrophicus Py2] | ABX09893 | 0.8 | | 1158 | 385 | 0 | 387 | 51 | |
| 127, 128 | dienelactone hydrolase [Sphingomonas wittichii RW1] gi\|118706350\|gb\|EAW05500.1\| dienelactone hydrolase [Sphingomonas wittichii RW1] | AAI86664 | 2.3 | | 852 | 283 | 0 | 283 | | |
| 129, 130 | lysophospholipase A [Legionella pneumophila subsp. pneumophila str. Philadelphia 1] | AEB39166 | 0.64 | | 942 | 313 | 123957 | 325 | | |

(continued)

| SEQ ID NO: | NR Description | Geneseq DNA Accession Code | Geneseq DNA Evalue | Predicted EC Number | Query DNA Length | Query Protein Length | Geneseq/NR DNA Length | Geneseq/NR Protein Length | Geneseq/NR %ID Protein | Geneseq/NR %ID DNA |
|---|---|---|---|---|---|---|---|---|---|---|
| 131, 132 | hypothetical protein Maqu_3412 [Marinobacter aquaeolei VT8] gi\|120326168\|gb\|ABM20483.1\| conserved hypothetical protein [Marinobacter aquaeolei VT8] | AEH47171 | 0 | | 930 | 309 | 756 | 251 | | |
| 133, 134 | Patatin [Delftia acidovorans SPH-1] gi\|118666914\|gb\|EAV73512.1\| Patatin [Delftia acidovorans SPH-1] | AEH47303 | 0 | 3.4.21. | 921 | 306 | 921 | 306 | | |
| 135, 136 | lipase/esterase [uncultured bacterium] | ABQ81847 | 2.5 | | 921 | 306 | 349980 | 296 | | |
| 137, 138 | putative carboxylesterase [Salmonella typhimurlum LT2]. | ACH96500 | ####### | 3.1.1.1 | 1509 | 502 | 1509 | 502 | 76 | 73 |
| 139, 140 | Esterase/lipase-like [Parvibaculum lavamentivorans DS-1] gi\|121298676\|gb\|EAX39865.1\| Esterase/lipase-like [Parvibaculum lavamentivorans DS-1] | AEH47203 | 0 | | 900 | 299 | 810 | 269 | | |
| 141, 142 | esterase [Xylella fastidiosa 9a5c]. | ADR51258 | 3.00E-12 | | 936 | 311 | 774 | 341 | | |
| 143, 144 | Putative esterase [Burkholderia xenovorans LB400] | AAT47564 | 0.67 | 3.1.1. | 987 | 328 | 0 | 296 | 28 | |
| 145, 146 | conserved hypothetical protein [Pyrobaculum aerophilum]. | AAS51218 | 0.31 | | 498 | 165 | 591 | 196 | 98 | 99 |

(continued)

| SEQ ID NO: | NR Description | Geneseq DNA Accession Code | Geneseq DNA Evalue | Predicted EC Number | Query DNA Length | Query Protein Length | Geneseq/NR DNA Length | Geneseq/NR Protein Length | Geneseq/NR %ID Protein | Geneseq/NR %ID DNA |
|---|---|---|---|---|---|---|---|---|---|---|
| 147, 148 | putative esterase [Thermus thermophilus HB27] | AAT86704 | 1.00E-82 | | 987 | 328 | 0 | 329 | 84 | |
| 149, 150 | alpha/beta hydrolase [Parvibaculum lavamentivorans DS-1] gi\|121299709\|gb\|EAX40898.1\| alpha/beta hydrolase [Parvibaculum lavamentivorans DS-1] | AEH47071 | 0 | 3.1.1.1 | 777 | 258 | 0 | 258 | | |
| 151, 152 | hypothetical protein BT4075 [Bacteroides thetaiotaomicron VPI-5482] | ADS89487 | 2.4 | | 894. | 297 | 0 | 467 | 27 | |
| 153, 154 | Enoyl-CoA hydratase/ isomerase [Mesorhizobium sp. BNC1] | AAT91405 | 0.51 | 4.2.1.17 | 768 | 255 | 0 | 254 | 55 | |
| 155, 156 | Alpha/beta hydrolase fold-3 domain protein [Burkholderia cenocepacia HI2424] | AAL61225 | 0.039 | 3.1.. | 906 | 301 | 0 | 300 | 58 | |
| 157, 158 | hypothetical protein CHGG_ 05597 [Chaetomium globosum CBS 148.51] | ADE86070 | 2.2 | | 843 | 280 | 0 | 286 | 58 | |
| 159, 160 | probable lipase/esterase [Rhodopirellula baltica SH 1] | AEH47491 | 0 | 3.1.. | 999 | 332 | 981 | 326 | | |

(continued)

| SEQ ID NO: | NR Description | Geneseq DNA Accession Code | Geneseq DNA Evalue | Predicted EC Number | Query DNA Length | Query Protein Length | Geneseq/NR DNA Length | Geneseq/NR Protein Length | Geneseq/NR %ID Protein | Geneseq/NR %ID DNA |
|---|---|---|---|---|---|---|---|---|---|---|
| 161, 162 | Patatin [Chlorobium limicola DSM 245] gi\|67784038\|gb\|EAM43418.1\| Patatin [Chlorobium limicola DSM 245] | ABD05045 | 0.49 | 3.4.21. | 747 | 248 | 0 | 250 | 41 | |
| 163, 164 | carboxylesterase [Oceanobacillus iheyensis HTE831]. | | | | | | | | 63 | |
| 165, 166 | Outer membrane autotransporter barrel [Pseudomonas fluorescens PfO-1] | ABD17863 | 1.00E-31 | | 1911 | 636 | 0 | 636 | 86 | |

*Hydrolase signal sequences, prepro and catalytic domains*

The invention provides signal sequences (e.g., signal peptides (SPs)), prepro domains and catalytic domains (CDs). The SPs, prepro domains and/or CDs of the invention can be isolated, synthetic or recombinant peptides or can be part of a fusion protein, e.g., as a heterologous domain in a chimeric protein. The invention provides nucleic acids encoding these catalytic domains (CDs), prepro domains and signal sequences (SPs, e.g., a peptide having a sequence comprising/ consisting of amino terminal residues of a polypeptide of the invention). In one aspect, the invention provides a signal sequence comprising a peptide comprising/ consisting of a sequence as set forth in residues 1 to 12, 1 to 13, 1 to 14, 1 to 15, 1 to 16, 1 to 17, 1 to 18, 1 to 19, 1 to 20, 1 to 21, 1 to 22, 1 to 23, 1 to 24, 1 to 25, 1 to 26, 1 to 27, 1 to 28, 1 to 28, 1 to 30, 1 to 31, 1 to 32, 1 to 33, 1 to 34, 1 to 35, 1 to 36, 1 to 37, 1 to 38, 1 to 39, 1 to 40, 1 to 41, 1 to 42, 1 to 43, 1 to 44 (or a longer peptide) of a polypeptide of the invention.

The following table describes exemplary signal sequences (signal peptides) of the invention; to help reading the table, for example, "SEQ ID NO:1,2" means the polypeptide having the sequence of SEQ ID NO:2, encoded e.g., by SEQ ID NO:1; the "class" means the enzyme activity class of hydrolase, for example, "esterase"; "source" is the source from which the sequence was initially derived; "Signal sequence position (AA = Amino Acid)" means the amino terminal signal sequence position. For example, one exemplary signal sequence of the invention comprises, or consists of, the subsequence of SEQ ID NO:126 "MSVQSSVGRLSSLFDRRLGSLLLVLLFISGCAS."

| SEQ ID NO: in D1180-8WO | Class | Source | Signal sequence position (AA = Amino Acid) | Signal sequence |
|---|---|---|---|---|
| 1, 2 | Esterase | Unknown | | |
| 101, 102 | Esterase | Unknown | | |
| 103, 104 | Esterase | Unknown | | |
| 105, 106 | Esterase | Unknown | | |
| 107, 108 | Esterase | Unknown | | |
| 109, 110 | Esterase | Unknown | | |
| 11, 12 | Esterase | Unknown | | |
| 111,112 | Esterase | Unknown | | |
| 113, 114 | Esterase | Unknown | | |
| 115, 116 | Esterase | Bacteria | | |
| 117, 118 | Esterase | Unknown | | |
| 119, 120 | Esterase | Unknown | | |
| 121, 122 | Esterase | Archaea | | |
| 123, 124 | Esterase | Unknown | | |
| 125, 126 | Esterase | Unknown | AA1-33 | MSVQSSVGRLSSLFDRRLGSLLLVLLFISGCAS |
| 127, 128 | Esterase | Unknown | | |
| 129, 130 | Esterase | Unknown | AA1-24 | MDMQFRAKIITFLCLLTLSFAASA |
| 13, 14 | Esterase | Unknown | | |
| 131, 132 | Esterase | Unknown | | |
| 133, 134 | Esterase | Unknown | | |
| 135,136 | Esterase | Unknown | | |
| 137, 138 | Esterase | Unknown | | |
| 139, 140 | Esterase | Unknown | | |
| 141, 142 | Esterase | Unknown | | |
| 143, 144 | Esterase | Unknown | | |
| 145,146 | Esterase | Archaea | | |
| 147, 148 | Esterase | Unknown | AA1-16 | MKRLLALLSLLGLALA |
| 149, 150 | Esterase | Unknown | | |
| 15, 16 | Esterase | Unknown | | |
| 151,152 | Esterase | Unknown | AA1-21 | MKIGYLKFLILIFFCSIQTWS |
| 153,154 | Esterase | Unknown | | |

(continued)

| SEQ ID NO: in D1180-8WO | Class | Source | Signal sequence position (AA = Amino Acid) | Signal sequence |
|---|---|---|---|---|
| 155,156 | Esterase | Unknown | | |
| 157, 158 | Esterase | Cochliobolus heterostrophus ATCC 48331 | AA1-26 | MLRPASLFALGALLFLSLLDSVSAAT |
| 159, 160 | Esterase | Unknown | AA1-29 | MAPLTRRGFVKASGALAAAPALAALAAHA |
| 161, 162 | Esterase | Unknown | | |
| 163, 164 | Esterase | Unknown | | |
| 165, 166 | Esterase | Unknown | AA1-24 | MIKQSLFVPLAGCLLAIACAQANA |
| 17,18 | Esterase | Unknown | | |
| 19, 20 | Esterase | Unknown | | |
| 21, 22 | Esterase | Unknown | | |
| 23, 24 | Esterase | Unknown | | |
| 25, 26 | Esterase | Unknown | | |
| 27,28 | Esterase | Unknown | | |
| 29, 30 | Esterase | Unknown | | |
| 3, 4 | Esterase | Unknown | AA1-20 | MKKKIFTIAGVLILVLVAWQ |
| 31, 32 | Esterase | Unknown | | |
| 33, 34 | Esterase | Unknown | | |
| 35, 36 | Esterase | Unknown | | |
| 37, 38 | Esterase | Unknown | AA1 - 25 | MRIRALTTCFALLAAGLLLSPPAMA |
| 39,40 | Esterase | Unknown | | |
| 41,42 | Esterase | Unknown | AA1-23 | MRISLIHVALLLGPLFPVSALSA |
| 43, 44 | Esterase | Unknown | | |
| 45, 46 | Esterase | Unknown | | |
| 47, 48 | Esterase | Unknown | AA1 - 29 | MRGFARSITFRLVFAATAFWALATLAQA |
| 49, 50 | Esterase | Unknown | | |
| 5, 6 | Esterase | Alicyclobacillus acidocaldarius ATCC 27009 | | |
| 51, 52 | Esterase | Unknown | | |
| 53, 54 | Esterase | Unknown | | |
| 55, 56 | Esterase | Unknown | | |
| 57, 58 | Esterase | Unknown | | |
| 59,60 | Esterase | Unknown | | |
| 61, 62 | Esterase | Unknown | | |
| 63, 64 | Esterase | Unknown | | |
| 65, 66 | Esterase | Unknown | AA1 - 22 | MKKTARILTVLSLLALSVPSMA |
| 67, 68 | Esterase | Unknown | AA1-18 | MARKFLYLIALLAVMVIA |
| 69, 70 | Esterase | Unknown | | |
| 7, 8 | Esterase (Lipase) | Unknown | | |
| 71,72 | Esterase | Unknown | AA1 - 22 | MTFVLTILGILAAWGVFLLLA |
| 73,74 | Esterase | Unknown | AA1 - 17 | MRNWAIGGLALGAGALG |
| 75, 76 | Esterase | Unknown | AA1-29 | MRLIVKRRAHAALLMFAAALLNWGVAQA |

(continued)

| SEQ ID NO: in D1180-8WO | Class | Source | Signal sequence position (AA = Amino Acid) | Signal sequence |
|---|---|---|---|---|
| 77, 78 | Esterase | Unknown | AA1-20 | MLKSLAAAAVLLTAVGCATA |
| 79, 80 | Esterase | Unknown | AA1-21 | MQSAASILALALALLAGGVWL |
| 81, 82 | Esterase | Unknown | AA1 - 37 | MTLPHRRSHLRPIDERLFARWLAAGAVAMLLSVTACA |
| 83, 84 | Esterase | Sulfolobus solfataricus P1 | | |
| 85, 86 | Esterase | Unknown | | |
| 87,88 | Esterase | Unknown | | |
| 89, 90 | Esterase | Unknown | | |
| 9, 10 | Esterase | Archaeoglobus fulgidus-VC16 | | |
| 91, 92 | Esterase | Unknown | AA1 - 25 | MRSAARISVAAVAFLCLLLTTRVSA |
| 93, 94 | Esterase | Unknown | | |
| 95, 96 | Esterase | Unknown | | |
| 97, 98 | Esterase | Unknown | | |
| 99, 100 | Esterase | Unknown | AA1-21 | MKRIATAVFLLHAMTSVAVCA |

The hydrolase signal sequences (signal peptides, or SPs), CDs, and/or prepro sequences of the invention can be isolated peptides, or, sequences joined to another hydrolase or a non- hydrolase polypeptide, e.g., as a fusion (chimeric) protein. In one aspect, the invention provides polypeptides comprising hydrolase signal sequences of the invention. In one aspect, polypeptides comprising hydrolase signal sequences SPs, CDs, and/or prepro of the invention comprise sequences heterologous to hydrolases of the invention (e.g., a fusion protein comprising an SP, CD, and/or prepro of the invention and sequences from another hydrolase or a non-hydrolase protein). In one aspect, the invention provides hydrolases of the invention with heterologous SPs, CDs, and/or prepro sequences, e.g., sequences with a yeast signal sequence. A hydrolase of the invention can comprise a heterologous SP and/or prepro in a vector, e.g., a pPIC series vector (Invitrogen, Carlsbad, CA).

In one aspect, SPs, CDs, and/or prepro sequences of the invention are identified following identification of novel hydrolase polypeptides. The pathways by which proteins are sorted and transported to their proper cellular location are often referred to as protein targeting pathways. One of the most important elements in all of these targeting systems is a short amino acid sequence at the amino terminus of a newly synthesized polypeptide called the signal sequence. This signal sequence directs a protein to its appropriate location in the cell and is removed during transport or when the protein reaches its final destination. Most lysosomal, membrane, or secreted proteins have an amino-terminal signal sequence that marks them for translocation into the lumen of the endoplasmic reticulum. The signal sequences can vary in length from 13 to 45 or more amino acid residues. Various methods of recognition of signal sequences are known to those of skill in the art. For example, in one aspect, novel hydrolase signal peptides are identified by a method referred to as SignalP. SignalP uses a combined neural network which recognizes both signal peptides and their cleavage sites. (Nielsen, et al., "Identification of prokaryotic and eukaryotic signal peptides and prediction of their cleavage sites." Protein Engineering, vol. 10, no. 1, p. 1-6 (1997).

It should be understood that in some aspects hydrolases of the invention may not have SPs and/or prepro sequences, and/or catalytic domains (CDs). In one aspect, the invention provides polypeptides (e.g., hydrolases) lacking all or part of an SP, a CD and/or a prepro domain. In one aspect, the invention provides a nucleic acid sequence encoding a signal sequence (SP), a CD, and/or prepro from one hydrolase operably linked to a nucleic acid sequence of a different hydrolase or, optionally, a signal sequence (SPs) and/or prepro domain from a non-hydrolase protein may be desired.

The invention also provides isolated, synthetic or recombinant polypeptides comprising signal sequences (SPs), prepro domain and/or catalytic domains (CDs) of the invention and heterologous sequences. The heterologous sequences are sequences not naturally associated (e.g., to a hydrolase) with an SP, prepro domain and/or CD. The sequence to which the SP, prepro domain and/or CD are not naturally associated can be on the SP's, prepro domain and/or CD's amino terminal end, carboxy terminal end, and/or on both ends of the SP and/or CD. In one aspect, the invention provides an isolated, synthetic or recombinant polypeptide comprising (or consisting of) a polypeptide comprising a signal sequence (SP), prepro domain and/or catalytic domain (CD) of the invention with the proviso that it is not associated with any sequence to which it is naturally associated (e.g., hydrolase sequence). Similarly in one aspect, the invention provides isolated, synthetic or recombinant nucleic acids encoding these polypeptides. Thus, in one aspect, the isolated, synthetic or recombinant nucleic acid of the invention comprises coding sequence for a signal sequence (SP), prepro domain and/or catalytic domain (CD) of the invention and a heterologous sequence (i.e., a sequence not naturally associated with the a signal sequence (SP), prepro domain and/or catalytic domain (CD) of the invention). The heterologous sequence can be on the 3' terminal end, 5' terminal end, and/or on both ends of the SP, prepro domain and/or CD coding sequence.

The invention provides fusion of N-terminal or C-terminal subsequences of enzymes of the invention (e.g., signal sequences, prepro sequences) with other polypeptides, active proteins or protein fragments. The production of an enzyme of the invention (e.g., a hydrolase, e.g., a lipase such as a phospholipase) may also be accomplished by expressing the enzyme as an inactive fusion protein that is later activated by a proteolytic cleavage event (using either an endogenous or exogenous protease activity, e.g. trypsin) that results in the separation of the fusion protein partner and the mature enzyme, e.g., hydrolase of the invention. In one aspect, the fusion protein of the invention is expressed from a hybrid nucleotide construct that encodes a single open reading frame containing the following elements: the nucleotide sequence for the fusion protein, a linker sequence (defined as a nucleotide sequence that encodes a flexible amino acid sequence that joins two less flexible protein domains), protease cleavage recognition site, and the mature enzyme (e.g., any enzyme of the invention, e.g., a hydrolase) sequence. In alternative aspects, the fusion protein can comprise a pectate lyase sequence, a xylanase sequence, a phosphatidic acid phosphatase sequence, or another sequence, e.g., a sequence that has previously been shown to be over-expressed in a host system of interest. Any host system can be used (see discussion, above), for example, *E. coli* or *Pichia pastoris*. The arrangement of the nucleotide sequences in the chimeric nucleotide construction can be determined based on the protein expression levels achieved with each fusion construct. Proceeding from the 5' end of the nucleotide construct to the 3' prime end of the construct, in one aspect, the nucleotide sequences is assembled as follows: Signal sequence/fusion protein/linker sequence/ protease cleavage recognition site/ mature enzyme (e.g., any enzyme of the invention, e.g., a hydrolase) or Signal sequence/pro sequence/mature enzyme/linker sequence/fusion protein. The expression of enzyme (e.g., any enzyme of the invention, e.g., a hydrolase) as an inactive fusion protein may improve the overall expression of the enzyme's sequence, may reduce any potential toxicity associated with the overproduction of active enzyme and/or may increase the shelf life of enzyme prior to use because enzyme would be inactive until the fusion protein e.g. pectate lyase is separated from the enzyme, e.g., hydrolase of the invention.

In various aspects, the invention provides specific formulations for the activation of a hydrolase of the invention expressed as a fusion protein. In one aspect, the activation of the hydrolase activity initially expressed as an inactive fusion protein is accomplished using a proteolytic activity or potentially a proteolytic activity in combination with an amino-terminal or carboxyl-terminal peptidase (the peptidase can be an enzyme of the invention, or, another enzyme). This activation event may be accomplished in a variety of ways and at variety of points in the manufacturing/storage process prior to application in oil degumming. Exemplary processes of the invention include: Cleavage by an endogenous activity expressed by the manufacturing host upon secretion of the fusion construct into the fermentation media; Cleavage by an endogenous protease activity (which can be a protease of the invention) that is activated or comes in contact with intracellularly expressed fusion construct upon rupture of the host cells; Passage of the crude or purified fusion construct over a column of immobilized protease (which can be a protease of the invention) activity to accomplish cleavage and enzyme (e.g., hydrolase of the invention, e.g., a protease, lipase, esterase or phospholipase) activation prior to enzyme formulation; Treatment of the crude or purified fusion construct with a soluble source of proteolytic activity; Activation of a hydrolase (e.g., a hydrolase of the invention) at the oil refinery using either a soluble or insoluble source of proteolytic activity immediately prior to use in the process; and/or, Activation of the hydrolase (e.g., a hydrolase of the invention) activity by continuously circulating the fusion construct formulation through a column of immobilized protease activity at reduced temperature (for example, any between about 4°C and 20°C). This activation event may be accomplished prior to delivery to the site of use or it may occur on-site at the oil refinery.

*Glycosylation*

The peptides and polypeptides of the invention (e.g., hydrolases, antibodies) can also be glycosylated, for example, in one aspect, comprising at least one glycosylation site, e.g., an N-linked or O-linked glycosylation. In one aspect, the polypeptide can be glycosylated after being expressed in a *P. pastoris* or a *S. pombe.* The glycosylation can be added post-translationally either chemically or by cellular biosynthetic mechanisms, wherein the later incorporates the use of known glycosylation motifs, which can be native to the sequence or can be added as a peptide or added in the nucleic acid coding sequence.

*Assays for phospholipases activity*

The invention provides isolated, synthetic or recombinant polypeptides having a phospholipase activity and nucleic acids encoding them. Any of the many phospholipase activity assays known in the art can be used to determine if a polypeptide has a phospholipase activity and is within the scope of the invention. Routine protocols for determining phospholipase A, B, D and C, patatin and lipid acyl hydrolase activities are well known in the art.
Exemplary activity assays include turbidity assays, methylumbelliferyl phosphocholine (fluorescent) assays, Amplex red (fluorescent) phospholipase assays, thin layer chromatography assays (TLC), cytolytic assays and p-nitrophenylphosphorylcholine assays. Using these assays polypeptides can be quickly screened for phospholipase activity.
The phospholipase activity can comprise a lipid acyl hydrolase (LAH) activity. See, e.g., Jimenez (2001) Lipids 36:1169-1174, describing an octaethylene glycol monododecyl ether-based mixed micellar assay for determining the lipid acyl hydrolase activity of a patatin. Pinsirodom (2000) J. Agric. Food Chem. 48:155-160, describes an exemplary lipid acyl hydrolase (LAH) patatin activity.
Turbidity assays to determine phospholipase activity are described, e.g., in Kauffmann (2001) "Conversion of Bacillus thermocatenulatus lipase into an efficient phospholipase with increased activity towards long-chain fatty acyl substrates by directed evolution and rational design," Protein Engineering 14:919-928; Ibrahim (1995) "Evidence implicating phospholipase as a virulence factor of Candida albicans," Infect. Immun. 63:1993-1998.
Methylumbelliferyl (fluorescent) phosphocholine assays to determine phospholipase activity are described, e.g., in Goode (1997) "Evidence for cell surface and internal phospholipase activity in ascidian eggs," Develop. Growth Differ. 39:655-660; Diaz (1999) "Direct fluorescence-based lipase activity assay," BioTechniques 27:696-700.
Amplex Red (fluorescent) Phospholipase Assays to determine phospholipase activity are available as kits, e.g., the detection of phosphatidylcholine-specific phospholipase using an Amplex Red phosphatidylcholine-specific phospholipase assay kit from Molecular Probes Inc. (Eugene, OR), according to manufacturer's instructions. Fluorescence is measured in a fluorescence microplate reader using excitation at 560 $\pm$ 10 nm and fluorescence detection at 590 $\pm$ 10 nm. The assay is sensitive at very low enzyme concentrations.
Thin layer chromatography assays (TLC) to determine phospholipase activity are described, e.g., in Reynolds (1991) Methods in Enzymol. 197:3-13; Taguchi (1975) "Phospholipase from Clostridium novyi type A.I," Biochim. Biophys. Acta 409:75-85. Thin layer chromatography (TLC) is a widely used technique for detection of phospholipase activity. Various modifications of this method have been used to extract the phospholipids from the aqueous assay mixtures. In some PLC assays the hydrolysis is stopped by addition of chloroform/methanol (2:1) to the reaction mixture. The unreacted starting material and the diacylglycerol are extracted into the organic phase and may be fractionated by TLC, while the head group product remains in the aqueous phase. For more precise measurement of the phospholipid digestion, radiolabeled substrates can be used (see, e.g., Reynolds (1991) Methods in Enzymol. 197:3-13). The ratios of products and reactants can be used to calculate the actual number of moles of substrate hydrolyzed per unit time. If all the components are extracted equally, any losses in the extraction will affect all components equally. Separation of phospholipid digestion products can be achieved by silica gel TLC with chloroform/methanol/water (65:25:4) used as a solvent system (see, e.g., Taguchi (1975) Biochim. Biophys. Acta 409:75-85).
p-Nitrophenylphosphorylcholine assays to determine phospholipase activity are described, e.g., in Korbsrisate (1999) J. Clin. Microbiol. 37:3742-3745; Berka (1981) Infect. Immun. 34:1071-1074. This assay is based on enzymatic hydrolysis of the substrate analog p-nitrophenylphosphorylcholine to liberate a yellow chromogenic compound p-nitrophenol, detectable at 405 nm. This substrate is convenient for high-throughput screening.
A cytolytic assay can detect phospholipases with cytolytic activity based on lysis of erythrocytes. Toxic phospholipases can interact with eukaryotic cell membranes and hydrolyze phosphatidylcholine and sphingomyelin, leading to cell lysis. See, e.g., Titball (1993) Microbiol. Rev. 57:347-366.

<u>Hybrid hydrolases and peptide libraries</u>

In one aspect, the invention provides hybrid hydrolases and fusion proteins, including peptide libraries, comprising sequences of the invention. The peptide libraries of the invention can be used to isolate peptide modulators (e.g., activators or inhibitors) of targets. The peptide libraries of the invention can be used to identify formal binding partners of targets, such as ligands, e.g., cytokines, hormones and the like.
In one aspect, the fusion proteins of the invention (e.g., the peptide moiety) are conformationally stabilized (relative to linear peptides) to allow a higher binding affinity for targets. The invention provides fusions of hydrolases of the invention and other peptides, including known and random peptides. They can be fused in such a manner that the structure of the hydrolases are not significantly perturbed and the peptide is metabolically or structurally conformationally stabilized. This allows the creation of a peptide library that is easily monitored both for its presence within cells and its quantity.
Amino acid sequence variants of the invention can be characterized by a predetermined nature of the variation, a feature that sets them apart from a naturally occurring form, e.g, an allelic or interspecies variation of a hydrolase sequence. In one aspect, the variants of the invention exhibit the same qualitative biological activity as the naturally occurring analogue. Alternatively, the variants can be selected for having modified characteristics. In one aspect, while the site or region for introducing an amino acid sequence variation is predetermined, the mutation per se need not be predetermined. For example, in order to optimize the performance of a mutation at a given site, random mutagenesis may be conducted at the target codon or region and the expressed hydrolase variants screened for the optimal combination of desired activity. Techniques for making substitution mutations at predetermined sites in DNA having a known sequence are well known, as discussed herein for example, M13 primer mutagenesis and PCR mutagenesis. Screening of the mutants can be done using assays of proteolytic activities. In alternative aspects, amino acid substitutions can be single residues; insertions can be on the order of from about 1 to 20 amino acids, although considerably larger insertions can be done. Deletions can range from about 1 to about 20, 30, 40, 50, 60, 70 residues or more. To obtain a final derivative with the optimal properties, substitutions, deletions, insertions or any combination thereof may be used. Generally, these changes are done on a few amino acids to minimize the alteration of the molecule. However, larger changes may be tolerated in certain circumstances.

The invention provides hydrolases where the structure of the polypeptide backbone, the secondary or the tertiary structure, e.g., an alpha-helical or beta-sheet structure, has been modified. In one aspect, the charge or hydrophobicity has been modified. In one aspect, the bulk of a side chain has been modified. Substantial changes in function or immunological identity are made by selecting substitutions that are less conservative. For example, substitutions can be made which more significantly affect: the structure of the polypeptide backbone in the area of the alteration, for example an alpha-helical or a beta-sheet structure; a charge or a hydrophobic site of the molecule, which can be at an active site; or a side chain. The invention provides substitutions in polypeptide of the invention where (a) a hydrophilic residues, e.g. seryl or threonyl, is substituted for (or by) a hydrophobic residue, e.g. leucyl, isoleucyl, phenylalanyl, valyl or alanyl; (b) a cysteine or proline is substituted for (or by) any other residue; (c) a residue having an electropositive side chain, e.g. lysyl, arginyl, or histidyl, is substituted for (or by) an electronegative residue, e.g. glutamyl or aspartyl; or (d) a residue having a bulky side chain, e.g. phenylalanine, is substituted for (or by) one not having a side chain, e.g. glycine. The variants can exhibit the same qualitative biological activity (i.e. hydrolase activity) although variants can be selected to modify the characteristics of the hydrolases as needed.

Polypeptide and peptides of the invention also comprise sequences that are "substantially identical" to an amino acid sequence of the invention, i.e., wherein "substantially identical" means a sequence that differs from a reference sequence (a sequence of the invention) by one or more conservative or non-conservative amino acid substitutions, deletions, or insertions, particularly when such a substitution occurs at a site that is not the active site of the molecule, and provided that the polypeptide essentially retains its functional properties. In one aspect, conservative amino acid substitution, for example, substitutes one amino acid for another of the same class (e.g., substitution of one hydrophobic amino acid, such as isoleucine, valine, leucine, or methionine, for another, or substitution of one polar amino acid for another, such as substitution of arginine for lysine, glutamic acid for aspartic acid or glutamine for asparagine). One or more amino acids can be deleted, for example, from a hydrolase, resulting in modification of the structure of the polypeptide, without significantly altering its biological activity. For example, amino- or carboxyl-terminal amino acids that are not required for hydrolase activity can be removed.

In one aspect, hydrolases of the invention comprise epitopes or purification tags, signal sequences or other fusion sequences, etc. In one aspect, the hydrolases of the invention can be fused to a random peptide to form a fusion polypeptide. By "fused" or "operably linked" herein is meant that the random peptide and the hydrolase are linked together, in such a manner as to minimize the disruption to the stability of the hydrolase structure, e.g., it retains hydrolase activity. The fusion polypeptide (or fusion polynucleotide encoding the fusion polypeptide) can comprise further components as well, including multiple peptides at multiple loops.

In one aspect, the peptides (e.g., hydrolase subsequences) and nucleic acids encoding them are randomized, either fully randomized or they are biased in their randomization, e.g. in nucleotide/residue frequency generally or per position. "Randomized" means that each nucleic acid and peptide consists of essentially random nucleotides and amino acids, respectively. In one aspect, the nucleic acids which give rise to the peptides can be chemically synthesized, and thus may incorporate any nucleotide at any position. Thus, when the nucleic acids are expressed to form peptides, any amino acid residue may be incorporated at any position. The synthetic process can be designed to generate randomized nucleic acids, to allow the formation of all or most of the possible combinations over the length of the nucleic acid, thus forming a library of randomized nucleic acids. The library can provide a sufficiently structurally diverse population of randomized expression products to affect a probabilistically sufficient range of cellular responses to provide one or more cells exhibiting a desired response. Thus, the invention provides an interaction library large enough so that at least one of its members will have a structure that gives it affinity for some molecule, protein, or other factor.

Screening Methodologies and "On-line" Monitoring Devices

In practicing the methods of the invention, a variety of apparatus and methodologies can be used to in conjunction with the polypeptides and nucleic acids of the invention, e.g., to screen polypeptides for hydrolase activity, to screen compounds as potential activators or inhibitors of a hydrolase activity (e.g., for potential drug screening), for antibodies that bind to a polypeptide of the invention, for nucleic acids that hybridize to a nucleic acid of the invention, to screen for cells expressing a polypeptide of the invention and the like. See, e.g., U.S. Patent No. 6,337,187.

*Capillary Arrays*

Capillary arrays, such as the GIGAMATRIX™, Diversa Corporation, San Diego, CA, can be used to in the methods of the invention. Nucleic acids or polypeptides of the invention can be immobilized to or applied to an array, including capillary arrays. Arrays can be used to screen for or monitor libraries of compositions (e.g., small molecules, antibodies, nucleic acids, etc.) for their ability to bind to or modulate the activity of a nucleic acid or a polypeptide of the invention. Capillary arrays provide another system for holding and screening samples. For example, a sample screening apparatus can include a plurality of capillaries formed into an array of adjacent capillaries, wherein each capillary comprises at least one wall defining a lumen for retaining a sample. The apparatus can further include interstitial material disposed between adjacent capillaries in the array, and one or more reference indicia formed within of the interstitial material. A capillary for screening a sample, wherein the capillary is adapted for being bound in an array of capillaries, can include a first wall defining a lumen for retaining the sample, and a second wall formed of a filtering material, for filtering excitation energy provided to the lumen to excite the sample.

A polypeptide or nucleic acid, e.g., a ligand or a substrate, can be introduced into a first component into at least a portion of a capillary of a capillary array. Each capillary of the capillary array can comprise at least one wall defining a lumen for retaining the first component. An air bubble can be introduced into the capillary behind the first component. A second component can be introduced into the capillary, wherein the second component is separated from the first component by the air bubble. A sample of interest can be introduced as a first liquid labeled with a detectable particle into a capillary of a capillary array, wherein each capillary of the capillary array comprises at least one wall defining a lumen for retaining the first liquid and the detectable particle, and wherein the at least one wall is coated with a binding material for binding the detectable particle to the at least one wall. The method can further include removing the first liquid from the capillary tube, wherein the bound detectable particle is maintained within the capillary, and introducing a second liquid into the capillary tube.

The capillary array can include a plurality of individual capillaries comprising at least one outer wall defining a lumen. The outer wall of the capillary can be one or more walls fused together. Similarly, the wall can define a lumen that is cylindrical, square, hexagonal or any other geometric shape so long as the walls form a lumen for retention of a liquid or sample. The capillaries of the capillary array can be held together in close proximity to form a planar structure. The capillaries can be bound together, by being fused (e.g., where the capillaries are made of glass), glued, bonded, or clamped side-by-side. The capillary array can be formed of any number of individual capillaries, for example, a range from 100 to 4,000,000 capillaries. A capillary array can form a micro titer plate having about 100,000 or more individual capillaries bound together.

*Arrays, or "Biochips*"

Nucleic acids and/or polypeptides of the invention can be immobilized to or applied to an array of the invention, which can include any "array" or "microarray" or "biochip" or "chip", including any product of manufacture, e.g., a device, comprising a plurality of target elements, each target element comprising a defined amount of one or more polypeptides (including antibodies) or nucleic acids immobilized onto a defined area of a substrate surface, as discussed in further detail, below.

Arrays can be used to screen for or monitor libraries of compositions (e.g., small molecules, antibodies, nucleic acids, etc.) for their ability to bind to or modulate the activity of a nucleic acid or a polypeptide of the invention. For example, in one aspect of the invention, a monitored parameter is transcript expression of a hydrolase gene. One or more, or, all the transcripts of a cell can be measured by hybridization of a sample comprising transcripts of the cell, or, nucleic acids representative of or complementary to transcripts of a cell, by hybridization to immobilized nucleic acids on an array, or "biochip." By using an "array" of nucleic acids on a microchip, some or all of the transcripts of a cell can be simultaneously quantified. Alternatively, arrays comprising genomic nucleic acid can also be used to determine the genotype of a newly engineered strain made by the methods of the invention. Polypeptide arrays" can also be used to simultaneously quantify a plurality of proteins. The present invention can be practiced with any known "array," also referred to as a "microarray" or "nucleic acid array" or "polypeptide array" or "antibody array" or "biochip," or variation thereof. Arrays are generically a plurality of "spots" or "target elements," each target element comprising a defined amount of one or more biological molecules, e.g., oligonucleotides, immobilized onto a defined area of a substrate surface for specific binding to a sample molecule, e.g., mRNA transcripts.

In one aspect, the hydrolases are used as immobilized forms. Any immobilization method can be used, e.g., immobilization upon an inert support such as diethylaminoethyl-cellulose, porous glass, chitin or cells. Cells that express hydrolases of the invention can be immobilized by cross-linking, e.g. with glutaraldehyde to a substrate surface.

In practicing the methods of the invention, any known array and/or method of making and using arrays can be incorporated in whole or in part, or variations thereof, as described, for example, in U.S. Patent Nos. 6,277,628; 6,277,489; 6,261,776; 6,258,606; 6,054,270; 6,048,695; 6,045,996; 6,022,963; 6,013,440; 5,965,452; 5,959,098; 5,856,174; 5,830,645; 5,770,456; 5,632,957; 5,556,752; 5,143,854; 5,807,522; 5,800,992; 5,744,305; 5,700,637; 5,556,752; 5,434,049; see also, e.g., WO 99/51773; WO 99/09217; WO 97/46313; WO 96/17958; see also, e.g., Johnston (1998) Curr. Biol. 8:R171-R174; Schummer (1997) Biotechniques 23:1087-1092; Kern (1997) Biotechniques 23:120-124; Solinas-Toldo (1997) Genes, Chromosomes & Cancer 20:399-407; Bowtell (1999) Nature Genetics Supp. 21:25-32. See also published U.S. patent applications Nos. 20010018642;20010019827;20010016322;20010014449;20010014448; 20010012537; 20010008765.

*Antibodies and Antibody-based screening methods*

The invention provides isolated, synthetic or recombinant antibodies that specifically bind to a hydrolase of the invention. These antibodies can be used to isolate, identify or quantify the hydrolase of the invention or related polypeptides. These antibodies can be used to isolate other polypeptides within the scope the invention or other related hydrolases.

Antibodies of the invention include a peptide or polypeptide derived from, modeled after or substantially encoded by an immunoglobulin gene or immunoglobulin genes, or fragments thereof, capable of specifically binding an antigen or epitope, see, e.g. Fundamental Immunology, Third Edition, W.E. Paul, ed., Raven Press, N.Y. (1993); Wilson (1994) J. Immunol. Methods 175:267-273; Yarmush (1992) J. Biochem. Biophys. Methods 25:85-97. The term antibody includes antigen-binding portions, i.e., "antigen binding sites," (e.g., fragments, subsequences, complementarity determining regions (CDRs)) that retain capacity to bind antigen, including (i) a Fab fragment, a monovalent fragment consisting of the VL, VH, CL and CH1 domains; (ii) a F(ab')2 fragment, a bivalent fragment comprising two Fab fragments linked by a disulfide bridge at the hinge region; (iii) a Fd fragment consisting of the VH and CH1 domains; (iv) a Fv fragment consisting of the VL and VH domains of a single arm of an antibody, (v) a dAb fragment (Ward et al., (1989) Nature 341: 544-546), which consists of a VH domain; and (vi) an isolated complementarity determining region (CDR). Single chain antibodies are also included by reference in the term "antibody." Thus, the invention provides antibodies, including antigen binding sites and single chain antibodies that specifically bind to a hydrolase of the invention. In practicing the methods of the invention, polypeptides having a hydrolase activity can also be used.

The antibodies can be used in immunoprecipitation, staining, immunoaffinity columns, and the like. If desired, nucleic acid sequences encoding for specific antigens can be generated by immunization followed by isolation of polypeptide or nucleic acid, amplification or cloning and immobilization of polypeptide onto an array of the invention. Alternatively, the methods of the invention can be used to modify the structure of an antibody produced by a cell to be modified, e.g., an antibody's affinity can be increased or decreased. Furthermore, the ability to make or modify antibodies can be a phenotype engineered into a cell by the methods of the invention.

Methods of immunization, producing and isolating antibodies (polyclonal and monoclonal) are known to those of skill in the art and described in the scientific and patent literature, see, e.g., Coligan, CURRENT PROTOCOLS IN IMMUNOLOGY, Wiley/Greene, NY (1991); Stites (eds.) BASIC AND CLINICAL IMMUNOLOGY (7th ed.) Lange Medical Publications, Los Altos, CA ("Stites"); Goding, MONOCLONAL ANTIBODIES: PRINCIPLES AND PRACTICE (2d ed.) Academic Press, New York, NY (1986); Kohler (1975) Nature 256:495; Harlow (1988) ANTIBODIES, A LABORATORY MANUAL, Cold Spring Harbor Publications, New York. Antibodies also can be generated in vitro, e.g., using recombinant antibody binding site expressing phage display libraries, in addition to the traditional in vivo methods using animals. See, e.g., Hoogenboom (1997) Trends Biotechnol. 15:62-70; Katz (1997) Annu. Rev. Biophys. Biomol. Struct. 26:27-45.

Polypeptides or peptides of the invention can be used to generate antibodies, which bind specifically to the polypeptides of the invention, e.g., specifically bind to the exemplary the polypeptides of the invention, including SEQ ID NO:2, SEQ ID NO:4, etc.. The resulting antibodies may be used in immunoaffinity chromatography procedures to isolate or purify the polypeptide or to determine whether the polypeptide is present in a biological sample. In such procedures, a protein preparation, such as an extract, or a biological sample is contacted with an antibody capable of specifically binding to one of the polypeptides of the invention.

In immunoaffinity procedures, the antibody is attached to a solid support, such as a bead or other column matrix. The protein preparation is placed in contact with the antibody under conditions in which the antibody specifically binds to one of the polypeptides of the invention. After a wash to remove non-specifically bound proteins, the specifically bound polypeptides are eluted.

The ability of proteins in a biological sample to bind to the antibody may be determined using any of a variety of procedures familiar to those skilled in the art. For example, binding may be determined by labeling the antibody with a detectable label such as a fluorescent agent, an enzymatic label, or a radioisotope. Alternatively, binding of the antibody to the sample may be detected using a secondary antibody having such a detectable label thereon. Particular assays include ELISA assays, sandwich assays, radioimmunoassays, and Western Blots.

Polyclonal antibodies generated against the polypeptides of the invention can be obtained by direct injection of the polypeptides into an animal or by administering the polypeptides to a non-human animal. The antibody so obtained will then bind the polypeptide itself. In this manner, even a sequence encoding only a fragment of the polypeptide can be used to generate antibodies which may bind to the whole native polypeptide. Such antibodies can then be used to isolate the polypeptide from cells expressing that polypeptide.

For preparation of monoclonal antibodies, any technique which provides antibodies produced by continuous cell line cultures can be used. Examples include the hybridoma technique, the trioma technique, the human B-cell hybridoma technique, and the EBV-hybridoma technique (see, e.g., Cole (1985) in Monoclonal Antibodies and Cancer Therapy, Alan R. Liss, Inc., pp. 77-96).

Techniques described for the production of single chain antibodies (see, e.g., U.S. Patent No. 4,946,778) can be adapted to produce single chain antibodies to the polypeptides of the invention. Alternatively, transgenic mice may be used to express humanized antibodies to these polypeptides or fragments thereof.

Antibodies generated against the polypeptides of the invention (including anti-idiotype antibodies) may be used in screening for similar polypeptides from other organisms and samples. In such techniques, polypeptides from the organism are contacted with the antibody and those polypeptides which specifically bind the antibody are detected. Any of the procedures described above may be used to detect antibody binding.

Immobilized hydrolases

In one aspect, the hydrolase of the invention, e.g., esterases, acylases, lipases, phospholipases or proteases, are used as immobilized forms, e.g., to process lipids, in the structured synthesis of lipids, to digest proteins and the like. The immobilized lipases of the invention can be used, e.g., for hydrolysis of triglycerides, diglycerides or esters or for the esterification or transesterification of fatty acids, diglycerides or triglycerides, or in the interesterification of fats. In one aspect, the lipase is specific for esterification of fatty acids with alcohol, 1,3-specific or randomizing transesterification lipase or lipase specific for the hydrolysis of partial glycerides, esters or triglycerides. Immobilized lipase of the invention can be used in a packed bed for continuous transesterification of solvent free fats. See, e.g., U.S. Patent No. 4,818,695; 5,569,594.

Any immobilization method or form of support can be used, e.g., arrays, beads, capillary supports and the like, as described above. In one aspect, hydrolase immobilization can occur upon an inert support such as diethylaminoethyl-cellulose, porous glass, chitin or cells. Cells that express hydrolases of the invention can be immobilized by cross-linking, e.g. with glutaraldehyde to a substrate surface. Immobilized hydrolases of the invention can be prepared containing hydrolase bound to a dry, porous particulate hydrophobic support, with a surfactant, such as a polyoxyethylene sorbitan fatty acid ester or a polyglycerol fatty acid ester. The support can be an aliphatic olefinic polymer, such as a polyethylene or a polypropylene, a homo- or copolymer of styrene or a blend thereof or a pre-treated inorganic support. These supports can be selected from aliphatic olefinic polymers, oxidation polymers, blends of these polymers or pre-treated inorganic supports in order to make these supports hydrophobic. This pre-treatment can comprise silanization with an organic silicon compound. The inorganic material can be a silica, an alumina, a glass or a ceramic. Supports can be made from polystyrene, copolymers of styrene, polyethylene, polypropylene or from co-polymers derived from (meth)acrylates. See, e.g., U.S. Patent No. 5,773,266.

Kits

The invention provides kits comprising the compositions, e.g., nucleic acids, expression cassettes, vectors, cells, transgenic seeds or plants or plant parts, polypeptides (e.g., hydrolases) and/or antibodies of the invention. The kits also can contain instructional material teaching the methodologies and industrial uses of the invention, as described herein.

Measuring Metabolic Parameters

The methods of the invention provide whole cell evolution, or whole cell engineering, of a cell to develop a new cell strain having a new phenotype by modifying the genetic composition of the cell, where the genetic composition is modified by addition to the cell of a nucleic acid, e.g., a hydrolase-encoding nucleic acid of the invention. To detect the new phenotype, at least one metabolic parameter of a modified cell is monitored in the cell in a "real time" or "on-line" time frame. In one aspect, a plurality of cells, such as a cell culture, is monitored in "real time" or "on-line." In one aspect, a plurality of metabolic parameters is monitored in "real time" or "on-line." Metabolic parameters can be monitored using the fluorescent polypeptides of the invention (e.g., hydrolases of the invention comprising a fluorescent moiety).

Metabolic flux analysis (MFA) is based on a known biochemistry framework. A linearly independent metabolic matrix is constructed based on the law of mass conservation and on the pseudo-steady state hypothesis (PSSH) on the intracellular metabolites. In practicing the methods of the invention, metabolic networks are established, including the:

- identity of all pathway substrates, products and intermediary metabolites
- identity of all the chemical reactions interconverting the pathway metabolites, the stoichiometry of the pathway reactions,
- identity of all the enzymes catalyzing the reactions, the enzyme reaction kinetics,
- the regulatory interactions between pathway components, e.g. allosteric interactions, enzyme-enzyme interactions etc,
- intracellular compartmentalization of enzymes or any other supramolecular organization of the enzymes, and,
- the presence of any concentration gradients of metabolites, enzymes or effector molecules or diffusion barriers to their movement.

Once the metabolic network for a given strain is built, mathematic presentation by matrix notion can be introduced to estimate the intracellular metabolic fluxes if the on-line metabolome data is available. Metabolic phenotype relies on the changes of the whole metabolic network within a cell. Metabolic phenotype relies on the change of pathway utilization with respect to environmental conditions, genetic regulation, developmental state and the genotype, etc. In one aspect of the methods of the invention, after the on-line MFA calculation, the dynamic behavior of the cells, their phenotype and other properties are analyzed by investigating the pathway utilization. For example, if the glucose supply is increased and the oxygen decreased during the yeast fermentation, the utilization of respiratory pathways will be reduced and/or stopped, and the utilization of the fermentative pathways will dominate. Control of physiological state of cell cultures will become possible after the pathway analysis. The methods of the invention can help determine how to manipulate the fermentation by determining how to change the substrate supply, temperature, use of inducers, etc. to control the physiological state of cells to move along desirable direction. In practicing the methods of the invention, the MFA results can also be compared with transcriptome and proteome data to design experiments and protocols for metabolic engineering or gene shuffling, etc.

In practicing the methods of the invention, any modified or new phenotype can be conferred and detected, including new or improved characteristics in the cell. Any aspect of metabolism or growth can be monitored.

*Monitoring expression of an mRNA transcript*

In one aspect of the invention, the engineered phenotype comprises increasing or decreasing the expression of an mRNA transcript or generating new transcripts in a cell. This increased or decreased expression can be traced by use of a hydrolase-encoding nucleic acid of the invention. mRNA transcripts, or messages, also can be detected and quantified by any method known in the art, including, e.g., Northern blots, quantitative amplification reactions, hybridization to arrays, and the like. Quantitative amplification reactions include, e.g., quantitative PCR, including, e.g., quantitative reverse transcription polymerase chain reaction, or RT-PCR; quantitative real time RT-PCR, or "real-time kinetic RT-PCR" (see, e.g., Kreuzer (2001) Br. J. Haematol. 114:313-318; Xia (2001) Transplantation 72:907-914).

In one aspect of the invention, the engineered phenotype is generated by knocking out expression of a homologous gene. The gene's coding sequence or one or more transcriptional control elements can be knocked out, e.g., promoters or enhancers. Thus, the expression of a transcript can be completely ablated or only decreased.

In one aspect of the invention, the engineered phenotype comprises increasing the expression of a homologous gene. This can be effected by knocking out of a negative control element, including a transcriptional regulatory element acting in cis- or trans-, or, mutagenizing a positive control element. One or more, or, all the transcripts of a cell can be measured by hybridization of a sample comprising transcripts of the cell, or, nucleic acids representative of or complementary to transcripts of a cell, by hybridization to immobilized nucleic acids on an array.

*Monitoring expression of a polypeptides, peptides and amino acids*

In one aspect of the invention, the engineered phenotype comprises increasing or decreasing the expression of a polypeptide or generating new polypeptides in a cell. This increased or decreased expression can be traced by use of a hydrolase or an antibody of the invention. Polypeptides, peptides and amino acids also can be detected and quantified by any method known in the art, including, e.g., nuclear magnetic resonance (NMR), spectrophotometry, radiography (protein radiolabeling), electrophoresis, capillary electrophoresis, high performance liquid chromatography (HPLC), thin layer chromatography (TLC), hyperdiffusion chromatography, various immunological methods, e.g. immunoprecipitation, immunodiffusion, immuno-electrophoresis, radioimmunoassays (RIAs), enzyme-linked immunosorbent assays (ELISAs), immuno-fluorescent assays, gel electrophoresis (e.g., SDS-PAGE), staining with antibodies, fluorescent activated cell sorter (FACS), pyrolysis mass spectrometry, Fourier-Transform Infrared Spectrometry, Raman spectrometry, GC-MS, and LC-Electrospray and cap-LC-tandem-electrospray mass spectrometries, and the like. Novel bioactivities can also be screened using methods, or variations thereof, described in U.S. Patent No. 6,057,103. Furthermore, as discussed below in detail, one or more, or, all the polypeptides of a cell can be measured using a protein array.

Industrial and Medical Applications

The invention provides many industrial uses and medical applications for the hydrolases (e.g., lipases, phospholipases, esterases, proteases) of the invention, and a few exemplary uses and compositions of the invention are described below. The processes of the invention comprise converting a non-hydratable phospholipid to a hydratable form, oil degumming, food processing, processing of oils from plants, fish, algae and the like, to name just a few applications.

*Phospholipases*

The invention provides many industrial uses and medical applications for the hydrolases, e.g., lipases and phospholipases of the invention, e.g., phospholipases A, B, C and D. Methods of using phospholipase enzymes in industrial applications are well known in the art. For example, the phospholipases and methods of the invention can be used for the processing of fats and oils as described, e.g., in JP Patent Application Publication H6-306386, describing converting phospholipids present in the oils and fats into water-soluble substances containing phosphoric acid groups.

Phospholipases of the invention can be used to process plant oils and phospholipids such as those derived from or isolated from rice bran, soy, canola, palm, cottonseed, corn, palm kernel, coconut, peanut, sesame, sunflower. Phospholipases of the invention can be used to process essential oils, e.g., those from fruit seed oils, e.g., grapeseed, apricot, borage, etc. Phospholipases of the invention can be used to process oils and phospholipids in different forms, including crude forms, degummed, gums, wash water, clay, silica, soapstock, and the like. The phospholipids of the invention can be used to process high phosphorous oils, fish oils, animal oils, plant oils, algae oils and the like. In any aspect of the invention, any time a phospholipase C can be used, an alternative comprises use of a phospholipase D of the invention and a phosphatase (e.g., using a PLD/ phosphatase combination to improve yield in a high phosphorus oil, such as a soy bean oil).

In one aspect, the invention provides compositions and methods (which can comprise use of phospholipases of the invention) for oil degumming comprising using varying amounts of acid and base without making soapstock. Using this aspect of the invention for oil degumming, acid (including phosphoric and/or citric) can be used to hydrate non-hydratable phospholipids in high phosphorous oils (including, e.g., rice bran, soybean, canola, and sunflower). Once the phospholipids are hydrated, the pH of the aqueous phase can be raised using caustic addition: the amount of caustic added can create a favorable pH for enzyme activity but will not result in the formation of a significant soapstock fraction in the oil. Because a soapstock is not formed, the free fatty acids in the oil can be removed downstream, following the degumming step, during bleaching and deodorization.

Phospholipases of the invention can be used to process and make edible oils, biodiesel oils, liposomes for pharmaceuticals and cosmetics, structured phospholipids and structured lipids. Phospholipases of the invention can be used in oil extraction. Phospholipases of the invention can be used to process and make various soaps.

The phospholipases of the invention can also be used to study the phosphoinositide (PI) signaling system; in the diagnosis, prognosis and development of treatments for bipolar disorders (see, e.g., Pandey (2002) Neuropsychopharmacology 26:216-228); as antioxidants; as modified phospholipids; as foaming and gelation agents; to generate angiogenic lipids for vascularizing tissues; to identify phospholipase, e.g., PLA, PLB, PLC, PLD and/or patatin modulators (agonists or antagonists), e.g., inhibitors for use as anti-neoplastics, anti-inflammatory and as analgesic agents. They can be used to generate acidic phospholipids for controlling the bitter taste in food and pharmaceuticals. They can be used in fat purification. They can be used to identify peptides inhibitors for the treatment of viral, inflammatory, allergic and cardiovascular diseases. They can be used to make vaccines. They can be used to make polyunsaturated fatty acid glycerides and phosphatidylglycerols.

The phospholipases of the invention, for example PLA and PLC enzymes, are used to generate immunotoxins and various therapeutics for anti-cancer treatments.

The phospholipases of the invention can be used in conjunction with other enzymes for decoloring (i.e. chlorophyll removal) and in detergents (see above), e.g., in conjunction with other enzymes (e.g., lipases, proteases, esterases, phosphatases). For example, in any instance where a PLC is used, a PLD and a phosphatase may be used in combination, to produce the same result as a PLC alone.

*Detoxification*

The hydrolases of the invention (e.g., lipases, esterases, proteases and/or phospholipases of the invention) can be used in detoxification processes, e.g., for the detoxification of endotoxins, e.g., compositions comprising lipopolysaccharides (LPS). In one aspect, a lipase and/or an esterase of the invention is used to detoxify a lipopolysaccharide (LPS). In one aspect, this detoxification is by deacylation of 2' and/or 3' fatty acid chains from lipid A. In one aspect, a hydrolase (e.g., a lipase and/or an esterase) of the invention is used to hydrolyze a 2'-lauroyl and/or a 3'-myristoyl chain from a lipid, e.g., a lipid A (e.g., from a bacterial endotoxin). In one aspect, the process of the invention is used to destroy an endotoxin, e.g., a toxin from a gram negative bacteria, as from *E. coli.* In one aspect, a hydrolase (e.g., a lipase and/or an esterase) of the invention is used to ameliorate the effects of toxin poisoning (e.g., from an on-going gram negative infection), or, to prophylactically to prevent the effects of endotoxin during an infection (e.g., an infection in an animal or a human). Accordingly, the invention provides a pharmaceutical composition comprising a hydrolase (e.g., a lipase and/or an esterase) of the invention, and method using a hydrolase of the invention, for the amelioration or prevention of lipopolysaccharide (LPS) toxic effects, e.g., during sepsis.

*Processing foods*

The hydrolases, e.g., lipases, esterases, proteases and/or phospholipases of the invention, or a combination thereof, can be used to process foods, e.g., to change their stability, shelf-life, flavor, texture and the like. For example, in one aspect, phospholipases of the invention are used to generate acidic phospholipids for controlling bitter taste in foods.

In one aspect, the invention provides cheese-making processes using hydrolases (e.g., lipases, esterases, proteases, phospholipases) of the invention (and, thus, the invention also provides cheeses comprising hydrolases of the invention). In one aspect, the enzymes of the invention (e.g., lipases, esterases, proteases, phospholipases, e.g., phospholipase A, lysophospholipase or a combination thereof) are used to process cheeses for flavor enhancement, to increase yield and/ or for "stabilizing" cheeses, e.g., by reducing the tendency for "oil-off," or, in one aspect, the enzymes of the invention are used to produce cheese from cheese milk. These processes of the invention can incorporate any method or protocol, e.g., as described, e.g., in U.S. Patent Nos. 6,551,635, and 6,399,121, WO 03/070013, WO 00/054601. For example, in one aspect, hydrolases (e.g., lipases, esterases, proteases and/or phospholipases) of the invention are used to stabilize fat emulsion in milk or milk-comprising compositions, e.g. cream, and are used to stabilize milk compositions, e.g. for the manufacturing of creams or cream liquors. In one aspect, the invention provides a process for enhancing the favor of a cheese using at least one enzyme of the invention, the process comprising incubating a protein, a fat and a protease (e.g., of the invention) and a lipase (e.g., of the invention) in an aqueous medium under conditions that produce an enhanced cheese flavor (e.g., reduced bitterness), e.g., as described in WO 99/66805. In one aspect, phospholipases of the invention are used to enhance flavor in a cheese (e.g., a curd) by mixing with water, a protease (e.g., of the invention), and a lipase (e.g., of the invention) at an elevated temperature, e.g., between about 75°C to 95°C, as described, e.g., in U.S. Patent No. 4,752,483. In one aspect, phospholipases of the invention are used to accelerate cheese aging by adding an enzyme of the invention to a cheese (e.g., a cheese milk) before adding a coagulant to the milk, or, adding an enzyme (e.g., a lipase or a phospholipase) of the invention to a curd with salt before pressing, e.g., as described, e.g., in U.S. Patent No. 4,707,364. In one aspect, a lipase of the invention is used degrade a triglyceride in milk fat to liberate free fatty acids, resulting in flavor enhancement. A protease of the invention also can be used in any of these processes of the invention, see, e.g., Brindisi (2001) J. of Food Sci. 66:1100-1107.

In one aspect, a hydrolase (e.g., lipases, esterase, protease and/or phospholipase of the invention) is used to reduce the content of phosphorus components in a food, e.g., an oil, such as a vegetable oil having a high non-hydratable phosphorus content, e.g., as described in WO 98/26057.

*Caustic refining*

In one aspect, enzymes of the invention, e.g., phospholipases, lipases, esterases, proteases, are used as caustic refining aids. In one aspect, a PLC or PLD of the invention and a phosphatase are used in the processes as a drop-in, either before, during, or after a caustic neutralization refining process (either continuous or batch refining. The amount of enzyme added may vary according to the process. The water level used in the process should be low, e.g., about 0.5 to 5%. Alternatively, caustic is be added to the process multiple times. In addition, the process may be performed at different temperatures (25°C to 70°C), with different acids or caustics, and at varying pH (4-12). Acids that may be used in a caustic refining process include, but are not limited to, phosphoric, citric, ascorbic, sulfuric, fumaric, maleic, hydrochloric and/or acetic acids. Acids are used to hydrate non-hydratable phospholipids. Caustics that may be used include, but are not limited to, KOH- and NaOH. Caustics are used to neutralize free fatty acids. Alternatively, phospholipases of the invention, or more particularly a PLC or a PLD of the invention and a phosphatase, are used for purification of phytosterols from the gum/soapstock.

An alternate embodiment of the invention to add a phospholipase of the invention before caustic refining, e.g., by expressing the phospholipase in a plant. In another embodiment, the phospholipase of the invention is added during crushing of the plant, seeds or other plant part. Alternatively, the phospholipase of the invention is added following crushing, but prior to refining (i.e. in holding vessels). In addition, phospholipase is added as a refining pre-treatment, either with or without acid.

Another embodiment of the invention comprises adding a phospholipase of the invention during a caustic refining process. Levels of acid and caustic can be varied depending on the level of phosphorous and the level of free fatty acids. Broad temperature and pH ranges can be used in the process dependent upon the type of enzyme used.

In another embodiment of the invention, the phospholipase of the invention is added after caustic refining. In one aspect, the phospholipase is added in an intense mixer or in a retention mixer, prior to separation. Alternatively, the phospholipase is added following the heat step. In another embodiment, the phospholipase of the invention is added in the centrifugation step. In an additional embodiment, the phospholipase is added to the soapstock. Alternatively, the phospholipase is added to the washwater. In another instance, the phospholipase of the invention is added during the bleaching and/or deodorizing steps.

*Structured synthesis and processing of oils*

The invention provides methods for the structured synthesis of oils, lipids and the like using hydrolases (e.g., lipases, phospholipases, esterases, proteases) of the invention. The methods of the invention comprise a biocatalytic synthesis of structured lipids, i.e., lipids that contain a defined set of fatty acids distributed in a defined manner on a backbone, e.g., a glycerol backbone. Products generated using the hydrolases of the invention and practicing the methods of the invention include cocoa butter alternatives, lipids containing poly-unsaturated fatty acids (PUFAs), 1,3-diacyl glycerides (DAGs), 2-monoacylglycerides (MAGs) and triacylglycerides (TAGs). The methods of the invention enable synthesis of lipids or fatty acids with defined regiospecificities and stereoselectivities.

The invention provides methods for processing (modifying) oils, lipids and the like using hydrolases of the invention. The methods of the invention can be used to process oils from plants, animals, microorganisms. The methods of the invention can be used in the structured synthesis of oils similar to those found in plants, animals, microorganisms. Lipids and oils can be processed to have a desired characteristic. Lipids and oils that can be processed by the methods of the invention (using the hydrolases of the invention) include cocoa butter alternatives, lipids containing poly-unsaturated fatty acids (PUFAs), 1,3-diacyl glycerides (DAGs), 2-monoacylglycerides (MAGs) and triacylglycerides (TAGs). In one aspect, the processed and synthetic oils and fats of the invention (e.g., cocoa butters alternatives and vegetable oils) can be used in a variety of applications, e.g., in the production of foods (e.g., confectionaries, pastries) and in the formulation of pharmaceuticals, nutraceuticals and cosmetics. In one aspect, the invention provides methods of processing fats and oils, e.g., oilseeds, from plants, including, e.g., rice bran, canola, sunflower, olive, palm, soy or lauric type oils using a hydrolase, e.g., a lipase, esterase or phospholipase, of the invention.

In one aspect, the invention provides methods of processing oils from animals, e.g., fish and mammals, using the hydrolases of the invention. In one aspect, the invention provides methods for the structured synthesis of oils similar to those found in animals, e.g., fish and mammals and microorganisms, using the hydrolases of the invention. In one aspect, these synthetic or processes oils are used as feed additives, foods, as ingredients in pharmaceutical formulations, nutraceuticals or in cosmetics. For example, in one aspect the hydrolases of the invention are used to make fish oil fatty acids as a feed additive. In one aspect, the hydrolases of the invention can be used to process oil from restaurant waste and rendered animal fats.

In one aspect, the hydrolases of the invention are versatile biocatalysts in organic synthesis, e.g., in the structured synthesis of oils, lipids and the like. Enzymes of the invention (including, e.g., esterases such as carboxyl esterases and lipases) can accept a broad range of substrates, including secondary and tertiary alcohols, e.g., from a natural product such as alpha-terpineol, linalool and the like. In some aspects, the hydrolases of the invention have good to excellent enantiospecificity (e.g., stereospecificity).

In one aspect, the hydrolase of the invention comprises a GGGX motif. As discussed above, in one aspect, the invention provides a fragment or subsequence of an enzyme of the invention comprising a catalytic domain ("CD") or "active site." In one aspect, a catalytic domain ("CD") or "active site" comprising peptide, catalytic antibody or polypeptide of the invention comprises a GGGX motif. In one aspect, this motif is located on a protein loop near the binding site of the substrate ester's carboxylic group. In one aspect, the GGGX motif is involved in the formation of an "oxyanion hole" which stabilizes the anionic carbonyl oxygen of a tetrahedral intermediate during the catalytic cycle of ester hydrolysis. In one aspect, the invention provides an esterase or a lipase comprising a GGGX motif for the hydrolysis of a tertiary alcohol ester. In one aspect, the invention provides an esterase or a lipase for the hydrolysis of a terpinyl-, linalyl, 2-phenyl-3-butin-2-yl acetate and/or a 3-methyl-1-pentin-3-yl-acetate, wherein the enzyme of the invention comprises a GGGX motif.

In one aspect, the invention provides an oil (e.g., vegetable oils, cocoa butters, and the like) conversion process comprising at least one enzyme (e.g., a lipase) of the invention. In one aspect, an oil conversion process comprises a controlled hydrolysis and acylation, e.g., a glycerol acylation, which can result in high purity and a broad end of products. In one aspect, hydrolases (e.g., lipases) of the invention are used to produce diacylglycerol oils and structured nutritional oils. In one aspect, the invention provides processes for the esterification of propylene glycol using an enzyme of the invention, e.g., a regio- and/or chemo- selective lipase for mono-substituted esterification at the sn-1 position. In one aspect, the invention provides processes for the structured synthesis of oils with targeted saturated or unsaturated fatty acid profiles using an enzyme of the invention, e.g., a regio- and/or chemo- selective lipase for the removal of a saturated fatty acid, or, for the targeted addition of a fatty acid to a glycerol backbone. In one aspect, the invention provides processes for modifying saturated fatty acids using an enzyme of the invention, e.g., by adding double bonds using an enzyme with desaturase activity (in one aspect, this process is done in whole cell systems). In one aspect, the invention provides processes for modifying saturated fatty acids using an enzyme of the invention, e.g., by the removal double bonds using enzymes with hydrogenation and/or dehydrogenation activity (in one aspect, this process is done in whole cell systems). In one aspect, the invention provides processes for the total hydrolysis of triglycerides without trans-isomer formation using an enzyme of the invention, e.g., a non-selective lipase of the invention for total hydrolysis without formation of trans-isomers. In one aspect, the invention provides processes for enzyme catalyzed monoesterification of a glycol, e.g., a propylene glycol, using a hydrolase (e.g., a lipase, an esterase) of the invention. In one aspect, oleic, linoleic or alpha-linolenic acids are used in the enzyme catalyzed monoesterification. Any oil, e.g., a vegetable oil such as soy, cotton, corn, rice bran or sunflower can be used in this process. The enzyme can be chemoselective and/or enantioselective. For example, in one aspect, a chemoselective enzyme of the invention can be selective for a single acid, e.g., oleic, linoleic or alpha-linolenic acid individually, or, can be selective for two acids only, e.g., oleic or linoleic acids only, or, linoleic or alpha-linolenic only, etc. Alternatively, an enzyme of the invention can be enantioselective (in esterification or hydrolysis). For example, an enzyme can be selective for only a single position, or, selective for only two positions, e.g., only 1,2 esterification, or, only 1,3 esterification, or, only 2,3 esterification (or, in the reverse reaction, hydrolysis).

In one aspect, the invention provides processes for the selective removal of fatty acids (e.g., undesirable fatty acids) from oils, e.g., separating saturated and/or unsaturated fatty acids from oils, using a hydrolase (e.g., a lipase, an esterase) of the invention. The process of the invention can separate saturated and/or unsaturated fatty acids from any oil, e.g., a soy oil. The enzyme can be chemoselective and/or enantioselective. The process can comprise selective acylation with *cis* isomers, Sn-2 esterification, enzymatic hydrogenation. In one aspect, these processes generate high stability fats and oils, e.g., "healthy" frying oils. The process of the invention can be used to generate oils with less sulfur, e.g., using a process comprising sulfur removal from crude oil. The enzymes of the invention can also be used in interesterification processes for these and other purposes.

In one aspect, an enzyme of the invention is used to generate a "no-trans" fat oil. In one aspect, a "no-trans" oil is generated from a partially hydrogenated oil to produce a cis-only oil. The enzyme can be chemoselective and/or enantioselective.

In one aspect, the invention provides processes for modifying cocoa butters using an enzyme of the invention. About 80% of cocoa butters comprise POP, SOS and POS triglycerides (P is palmitic fatty acid, O is oleic fatty acid, S is stearic fatty acid). The saturated-unsaturated-saturated fatty acid structure of cocoa butters imparts their characteristic melting profiles, e.g., in chocolates. In one aspect, the structured and direct synthetic processes of the invention are used on cocoa butters to reduce cocoa butter variations or to produce synthetic cocoa butters ("cocoa butter alternatives"). In one aspect, a chemoselective and/or enantioselective (e.g., a regio-selective) hydrolase (e.g., lipase or esterase) of the invention is used to make a cocoa butter alternative, e.g., a cocoa butter substitute, a cocoa butter replacer and/or a cocoa butter equivalent. Thus, the invention also provides cocoa butter alternatives, including cocoa butter substitutes, cocoa butter replacers and cocoa butter equivalents and their manufacturing intermediates comprising an enzyme of the invention. A process of the invention (using an enzyme of the invention) for making cocoa butter alternatives can comprise blending a vegetable oil, e.g., a palm oil, with shea or equivalent, illipe or equivalent and Sal sterins or equivalent. In one aspect, the process of the invention comprises use of interesterification. The process of the invention can generate compositional or crystalline forms that mimic "natural" cocoa butter.

In one aspect, the invention provides processes (using an enzyme of the invention) for producing a diacylglycerol (DAG), e.g., 1, 3 diacylglycerol, using a vegetable oil, e.g., a low cost oil. The enzyme can be chemoselective and/or enantioselective. The process of the invention can result in a DAG-comprising composition having good stability, long shelf life and high temperature performance.

*Enzymatic processing of oilseeds*

The invention provides compositions (e.g., hydrolase enzymes of the invention, such as lipases, phospholipases, esterases, proteases) and methods for enzymatic processing of oilseeds, including soybean, canola, coconut, avocado and olive paste. In one aspect, these processes of the invention can increase the oil yield and to improve the nutritional quality of the obtained meals. In some aspects, enzymatic processing of oilseeds using compositions and methods of the invention will provide economical and environmental benefits, as well as alternative technologies for oil extraction and processing food for human and animal consumption. In alternative aspects, the processes of the invention comprise use of any hydrolase of the invention, e.g., a phospholipases of the invention (or another phospholipase), a protease of the invention (or another protease), phosphatases, phytases, xylanases, an amylase, e.g., α-amylases, a glucanase, e.g., β-glucanases, a polygalacturonase, galactolipases, a cellulase, a hemicellulase, a pectinases and/or other plant cell wall degrading enzymes, as well as mixed enzyme preparations and cell lysates, or enzyme preparations from recombinant sources, e.g., host cells or transgenic plants.

In alternative aspects, the processes of the invention can be practiced in conjunction with other processes, e.g., enzymatic treatments, e.g., with carbohydrases, including cellulase, hemicellulase and other side degrading activities, or, chemical processes, e.g., hexane extraction of soybean oil. The enzymatic treatment can increase the oil extractability by 8-10% when the enzymatic treatment is carried out prior to the solvent extraction.

In alternative aspects, the processes of the invention can be practiced with aqueous extraction processes. The aqueous extraction methods can be environmentally cleaner alternative technologies for oil extraction. Low extraction yields of aqueous process can be overcome by using enzymes that hydrolyze the structural polysaccharides forming the cell wall of oilseeds, or that hydrolyze the proteins which form the cell and lipid body membranes, e.g., utilizing digestions comprising cellulase, hemicellulase, and/or protopectinase for extraction of oil from soybean cells. In one aspect, methods are practiced with an enzyme of the invention as described by Kasai (2003) J. Agric. Food Chem. 51: 6217-6222, who reported that the most effective enzyme to digest the cell wall was cellulase.

In one aspect, proteases of the invention or other proteases are used in combination with the methods of the invention. The combined effect of operational variables and enzyme activity of a protease and cellulase on oil and protein extraction yields combined with other process parameters, such as enzyme concentration, time of hydrolysis, particle size and solid-to-liquid ratio has been evaluated. In one aspect, methods are practiced with an enzyme of the invention as described by Rosenthal (2001) Enzyme and Microb. Tech. 28:499-509, who reported that use of protease can result in significantly higher yields of oil and protein over the control when heat treated flour is used.

In one aspect, complete protein, pectin, and hemicellulose extraction are used in combination with the methods of the invention. The plant cell consists of a series of polysaccharides often associated with or replaced by proteins or phenolic compounds. Most of these carbohydrates are only partially digested or poorly utilized by the digestive enzymes. The disruption of these structures through processing or degrading enzymes can improve their nutrient availability. In one aspect, methods are practiced with an enzyme of the invention as described by Ouhida (2002) J. Agric. Food Chem. 50:1933-1938, who reported that a significant degradation of the soybean cell wall cellulose (up to 20%) has been achieved after complete protein, pectin, and hemicellulose extraction.

In one aspect, the methods of the invention further comprise incorporation of various enzymatic treatments in the treatment of seeds, e.g., canola seeds, these treatments comprising use of proteases of the invention (or other proteases), cellulases, and hemicellulases (in various combinations with each other and with one or more enzymes of the invention). For example, the methods can comprise enzymatic treatments of canola seeds at 20 to 40 moisture during the incubation with enzymes prior to a conventional process; as described, e.g., by Sosulski (1990) Proc. Can. Inst. Food Sci. Technol. 3:656. The methods of the invention can further comprise incorporation of proteases of the invention (or other proteases), α-amylases, polygalacturonases (in various combinations with each other and with one or more enzymes of the invention) to hydrolyze cellular material in coconut meal and release the coconut oil, which can be recovered by centrifugation, as described, e.g., by McGlone (1986) J. of Food Sci. 51:695-697. The methods of the invention can further comprise incorporation of pectinases, α-amylases, proteases of the invention (or other proteases), cellulases in different combinations (with each other and with one or more enzymes of the invention) to result in significant yield improvement (~70% in the best case) during enzymatic extraction of avocado oil, as described, e.g., by Buenrostro (1986) Biotech. Letters 8(7):505-506. In processes of the invention for olive oil extraction, olive paste is treated with cellulase, hemicellulase, poligalacturonase, pectin-methyltransferase, protease of the invention (or other proteases) and their combinations (with each other and with one or more enzymes of the invention), as described, e.g., by Montedoro (1976) Acta Vitamin. Enzymol. (Milano) 30:13.

*Oil degumming and vegetable oil processing*

The enzymes of the invention (e.g., lipases, phospholipases, esterases, proteases of the invention) can be used in various vegetable oil processing steps, such as in vegetable oil extraction, particularly, in the removal of "phospholipid gums" in a process called "oil degumming,".

In one aspect, the invention provides oil degumming processes comprising use of a hydrolase of the invention having a phospholipase C (PLC) activity. In one aspect, the process further comprises addition of a PLA of the invention and/or a patatin-like phospholipase of the invention. In one aspect, all enzymes are added together, or, alternatively, the PLC addition is followed by PLA and/or patatin addition. In one aspect, this process provides a yield improvement as a result of the PLC treatment. In one aspect, this process provides an additional decrease of the phosphorus content of the oil as a result of the PLA treatment.

The invention provides methods for processing vegetable oils from various sources, such as rice bran, soybeans, rapeseed, peanuts and other nuts, sesame, sunflower, palm and corn. The methods can used in conjunction with processes based on extraction with as hexane, with subsequent refining of the crude extracts to edible oils, including use of the methods and enzymes of the invention. The first step in the refining sequence is the so-called "degumming" process, which serves to separate phosphatides by the addition of water. The material precipitated by degumming is separated and further processed to mixtures of lecithins. The commercial lecithins, such as soybean lecithin and sunflower lecithin, are semi-solid or very viscous materials. They consist of a mixture of polar lipids, mainly phospholipids, and oil, mainly triglycerides.

The enzymes (e.g., phospholipases) of the invention can be used in any "degumming" procedure, including water degumming, ALCON oil degumming (e.g., for soybeans), safinco degumming, "super degumming," UF degumming, TOP degumming, uni-degumming, dry degumming and ENZYMAX™ degumming. See, e.g., U.S. Patent Nos. 6,355,693; 6,162,623; 6,103,505; 6,001,640; 5,558,781; 5,264,367. Various "degumming" procedures incorporated by the methods of the invention are described in Bockisch, M. (1998) In Fats and Oils Handbook, The extraction of Vegetable Oils (Chapter 5), 345-445, AOCS Press, Champaign, Illinois. The enzymes (e.g., phospholipases) of the invention can be used in the industrial application of enzymatic degumming of triglyceride oils as described, e.g., in EP 513 709.

In one aspect, hydrolases (e.g., phospholipases) of the invention are used to treat vegetable oils, e.g., crude oils, such as rice bran, soy, canola, flower and the like. In one aspect, this improves the efficiency of the degumming process. In one aspect, the invention provides methods for enzymatic degumming under conditions of low water, e.g., in the range of between about 0.1 % to 20 % water, or, 0.5% to 10% water. In one aspect, this results in the improved separation of a heavy phase from the oil phase during centrifugation. The improved separation of these phases can result in more efficient removal of phospholipids from the oil, including both hydratable and nonhydratable oils. In one aspect, this can produce a gum fraction that contains less entrained neutral oil, thereby improving the overall yield of oil during the degumming process.

The hydrolases (e.g., phospholipases) of the invention can be used in the industrial application of enzymatic degumming as described, e.g., in CA 1102795, which describes a method of isolating polar lipids from cereal lipids by the addition of at least 50% by weight of water. This method is a modified degumming in the sense that it utilizes the principle of adding water to a crude oil mixture.

In one aspect, the invention provides enzymatic processes comprising use of phospholipases of the invention (e.g., a PLC) comprising hydrolysis of hydrated phospholipids in oil at a temperature of about 20°C to 40°C, at an alkaline pH, e.g., a pH of about pH 8 to pH 10, using a reaction time of about 3 to 10 minutes. This can result in less than 10 ppm final oil phosphorus levels. The invention also provides enzymatic processes comprising use of phospholipases of the invention (e.g., a PLC) comprising hydrolysis of hydratable and non-hydratable phospholipids in oil at a temperature of about 50°C to 60°C, at a pH slightly below neutral, e.g., of about pH 5 to pH 6.5, using a reaction time of about 30 to 60 minutes. This can result in less than 10 ppm final oil phosphorus levels.

In one aspect, the invention provides enzymatic processes that utilize a phospholipase C enzyme to hydrolyze a glyceryl phosphoester bond and thereby enable the return of the diacylglyceride portion of phospholipids back to the oil, e.g., a vegetable, fish or algae oil (a "phospholipase C (PLC) caustic refining aid"); and, reduce the phospholipid content in a degumming step to levels low enough for high phosphorous oils to be physically refined ( a "phospholipase C (PLC) degumming aid"). The two approaches can generate different values and have different target applications.

In various exemplary processes of the invention, a number of distinct steps compose the degumming process preceding the core bleaching and deodorization refining processes. These steps include heating, mixing, holding, separating and drying. Following the heating step, water and often acid are added and mixed to allow the insoluble phospholipid "gum" to agglomerate into particles which may be separated. While water separates many of the phosphatides in degumming, portions of the phospholipids are non-hydratable phosphatides (NHPs) present as calcium or magnesium salts. Degumming processes address these NHPs by the addition of acid. Following the hydration of phospholipids, the oil is mixed, held and separated by centrifugation. Finally, the oil is dried and stored, shipped or refined. The resulting gums are either processed further for lecithin products or added back into the meal.

In various exemplary processes of the invention phosphorous levels are reduced low enough for physical refining. The separation process can result in potentially higher yield losses than caustic refining. Additionally, degumming processes may generate waste products that may not be sold as commercial lecithin. Therefore, these processes have not achieved a significant share of the market and caustic refining processes continue to dominate the industry for rice bran, soy, canola and sunflower. Note however, that a phospholipase C enzyme employed in a special degumming process would decrease gum formation and return the diglyceride portion of the phospholipid back to the oil.

In one aspect, a phospholipase C enzyme of the invention hydrolyzes a phosphatide at a glyceryl phosphoester bond to generate a diglyceride and water-soluble phosphate compound. The hydrolyzed phosphatide moves to the aqueous phase, leaving the diglyceride in the oil phase. One objective of the PLC "Caustic Refining Aid" is to convert the phospholipid gums formed during neutralization into a diacylglyceride that will migrate back into the oil phase. In contrast, one objective of the "PLC Degumming Aid" is to reduce the phospholipids in crude oil to a phosphorous equivalent of less than 10 parts per million.

In one aspect, a phospholipase C enzyme of the invention will hydrolyze the phosphatide from both hydratable and non-hydratable phospholipids in neutralized crude and degummed oils before bleaching and deodorizing. The target enzyme can be applied as a drop-in product in the existing caustic neutralization process. In this aspect, the enzyme will not be required to withstand extreme pH levels if it is added after the addition of caustic.

In one aspect, a phospholipase of the invention enables phosphorous to be removed to the low levels acceptable in physical refining. In one aspect, a PLC of the invention will hydrolyze the phosphatide from both hydratable and non-hydratable phospholipids in crude oils before bleaching and deodorizing. The target enzyme can be applied as a drop-in product in the existing degumming operation. Given sub-optimal mixing in commercial equipment, it is likely that acid will be required to bring the non-hydratable phospholipids in contact with the enzyme at the oil/water interface. Therefore, in one aspect, an acid-stable PLC of the invention is used.

In one aspect, a PLC Degumming Aid process of the invention can eliminate losses in one, or all three, areas: 1) Oil lost in gum formation & separation; 2) Saponified oil in caustic addition; 3) Oil trapped in clay in bleaching. Losses associated in a PLC process can be estimated to be about 0.8% versus 5.2% on a mass basis due to removal of the phosphatide. Additional potential benefits of this process of the invention include the following:

- ♦ Reduced adsorbents - less adsorbents required with lower (< 5ppm) phosphorous
- ♦ Lower chemical usage - less chemical and processing costs associated with hydration of non-hydratable phospholipids
- ♦ Lower waste generation - less water required to remove phosphorous from oil

Oils processed (e.g., "degummed") by the methods of the invention include plant oilseeds, e.g., rice bran, soybean oil, rapeseed oil and sunflower oil. In one aspect, the "PLC Caustic Refining Aid" of the invention can save 1.2% over existing caustic refining processes. The refining aid application addresses soy oil that has been degummed for lecithin and these are also excluded from the value/load calculations.

Other processes that can be used with a phospholipase of the invention, e.g., a phospholipase $A_1$ of the invention can convert non-hydratable native phospholipids to a hydratable form. In one aspect, the enzyme is sensitive to heat. This may be desirable, since heating the oil can destroy the enzyme. However, the degumming reaction must be adjusted to pH 4-5 and 60°C to accommodate this enzyme. At 300 Units/kg oil saturation dosage, this exemplary process is successful at taking previously water-degummed oil phosphorous content down to ≤10 ppm P. Advantages can be decreased $H_20$ content and resultant savings in usage, handling and waste.

In addition to these various "degumming" processes, the enzymes of the invention can be used in any vegetable oil processing step. For example, phospholipase enzymes of the invention can be used in place of PLA, e.g., phospholipase A2, in any vegetable oil processing step. Oils that are "processed" or "degummed" in the methods of the invention include soybean oils, rapeseed oils, corn oils, oil from rice bran oils, palm kernels, canola oils, sunflower oils, sesame oils, peanut oils, and the like. The main products from this process include triglycerides.

In one exemplary process, when the enzyme is added to and reacted with a crude oil, the amount of phospholipase employed is about 10-10,000 units, or, alternatively, about, 100-2,000 units, per 1 kg of crude oil. The enzyme treatment is conducted for 5 min to 10 hours at a temperature of 30°C to 90°C, or, alternatively, about, 40°C to 70°C. The conditions may vary depending on the optimum temperature of the enzyme. The amount of water added to dissolve the enzyme is 5-1,000 wt. parts per 100 wt. parts of crude oil, or, alternatively, about, 10 to 200 wt. parts per 100 wt. parts of crude oil.

Upon completion of such enzyme treatment, the enzyme liquid is separated with an appropriate means such as a centrifugal separator and the processed oil is obtained. Phosphorus-containing compounds produced by enzyme decomposition of gummy substances in such a process are practically all transferred into the aqueous phase and removed from the oil phase. Upon completion of the enzyme treatment, if necessary, the processed oil can be additionally washed with water or organic or inorganic acid such as, e.g., acetic acid, phosphoric acid, succinic acid, and the like, or with salt solutions.

In one exemplary process for ultra-filtration degumming, the enzyme is bound to a filter or the enzyme is added to an oil prior to filtration or the enzyme is used to periodically clean filters.

In one aspect, the invention provides processes using a hydrolase of the invention, e.g., a phospholipase of the invention, such as a phospholipase-specific phosphohydrolase of the invention, or another phospholipase, in a modified "organic refining process," which can comprise addition of the enzyme (e.g., a hydrolase, such as a PLC) in a citric acid holding tank.

Enzymes of the invention are used to improve oil extraction and oil degumming (e.g., vegetable oils). In one aspect, a hydrolase (e.g., phospholipase, such as a PLC) of the invention and at least one plant cell wall degrader (e.g.,

a cellulase, a hemicellulase or the like, to soften walls and increase yield at extraction) is used in a process of the invention. In this exemplary approach to using enzymes of the invention to improve oil extraction and oil degumming, a hydrolase (e.g., phospholipase C) of the invention as well as other hydrolases (e.g., a cellulase, a hemicellulase, an esterase of the invention or another esterase, a protease of the invention of the invention or another protease and/or a phosphatase) are used during the crushing steps associated with oil production (including but not limited to soybean, canola, rice bran and sunflower oil). By using enzymes prior to or in place of solvent extraction, it is possible to increase oil yield and reduce the amount of hydratable and non-hydratable phospholipids in the crude oil. The reduction in non-hydratable phospholipids may result from conversion of potentially non-hydratable phospholipids to diacylglycerol and corresponding phosphate-ester prior to complexation with calcium or magnesium. The overall reduction of phospholipids in the crude oil will result in improved yields during refining with the potential for eliminating the requirement for a separate degumming step prior to bleaching and deodorization.

In one exemplary process for a phospholipase-mediated physical refining aid, water and enzyme are added to crude oil. In one aspect, a PLC or a PLD and a phosphatase are used in the process. In phospholipase-mediated physical refining, the water level can be low, i.e. 0.5 - 5% and the process time should be short (less than 2 hours, or, less than 60 minutes, or, less than 30 minutes, or, less than 15 minutes, or, less than 5 minutes). The process can be run at different temperatures (25°C to 70°C), using different acids and/or caustics, at different pHs (e.g., 3-10).

In alternate aspects, water degumming is performed first to collect lecithin by centrifugation and then PLC or PLC and PLA is added to remove non-hydratable phospholipids (the process should be performed under low water concentration). In another aspect, water degumming of crude oil to less than 10 ppm (edible oils) and subsequent physical refining (less than 50 ppm for biodiesel) is performed. In one aspect, an emulsifier is added and/or the crude oil is subjected to an intense mixer to promote mixing. Alternatively, an emulsion-breaker is added and/or the crude oil is heated to promote separation of the aqueous phase. In another aspect, an acid is added to promote hydration of non-hydratable phospholipids. Additionally, phospholipases can be used to mediate purification of phytosterols from the gum/ soapstock.

The enzymes of the invention can be used in any oil processing method, e.g., degumming or equivalent processes. For example, the enzymes of the invention can be used in processes as described in U.S. Patent Nos. 5,558,781; 5,264,367; 6,001,640. The process described in USPN 5,558,781 uses either phospholipase A1, A2 or B, essentially breaking down lecithin in the oil that behaves as an emulsifier.

The enzymes and methods of the invention can be used in processes for the reduction of phosphorus-containing components in edible oils comprising a high amount of non-hydratable phosphorus by using of a phospholipase of the invention, e.g., a polypeptide having a phospholipase A and/or B activity, as described, e.g., in EP Patent Number: EP 0869167. In one aspect, the edible oil is a crude oil, a so-called "non-degummed oil." In one aspect, the method treat a non-degummed oil, including pressed oils or extracted oils, or a mixture thereof, from, e.g., rice bran, rapeseed, soybean, sesame, peanut, corn or sunflower. The phosphatide content in a crude oil can vary from 0.5 to 3% w/w corresponding to a phosphorus content in the range of 200 to 1200 ppm, or, in the range of 250 to 1200 ppm. Apart from the phosphatides, the crude oil can also contains small concentrations of carbohydrates, sugar compounds and metal/phosphatide acid complexes of Ca, Mg and Fe. In one aspect, the process comprises treatment of a phospholipid or lysophospholipid with the phospholipase of the invention so as to hydrolyze fatty acyl groups. In one aspect, the phospholipid or lysophospholipid comprises lecithin or lysolecithin. In one aspect of the process the edible oil has a phosphorus content from between about 50 to 250 ppm, and the process comprises treating the oil with a phospholipase of the invention so as to hydrolyze a major part of the phospholipid and separating an aqueous phase containing the hydrolyzed phospholipid from the oil. In one aspect, prior to the enzymatic degumming process the oil is water-degummed. In one aspect, the methods provide for the production of an animal feed comprising mixing the phospholipase of the invention with feed substances and at least one phospholipid.

The enzymes and methods of the invention can be used in processes of oil degumming as described, e.g., in WO 98/18912. The phospholipases of the invention can be used to reduce the content of phospholipid in an edible oil. The process can comprise treating the oil with a phospholipase of the invention to hydrolyze a major part of the phospholipid and separating an aqueous phase containing the hydrolyzed phospholipid from the oil. This process is applicable to the purification of any edible oil, which contains a phospholipid, e.g. vegetable oils, such as rice bran, soybean oil, rapeseed oil and sunflower oil, fish oils, algae and animal oils and the like. Prior to the enzymatic treatment, the vegetable oil is preferably pretreated to remove slime (mucilage), e.g. by wet refining. The oil can contain 50-250 ppm of phosphorus as phospholipid at the start of the treatment with phospholipase, and the process of the invention can reduce this value to below 5-10 ppm.

The enzymes of the invention can be used in processes as described in JP Application No.: H5-132283, filed April 25, 1993, which comprises a process for the purification of oils and fats comprising a step of converting phospholipids present in the oils and fats into water-soluble substances containing phosphoric acid groups and removing them as water-soluble substances. An enzyme action is used for the conversion into water-soluble substances. An enzyme having a phospholipase C activity is preferably used as the enzyme.

The enzymes of the invention can be used in processes as described as the "Organic Refining Process," (ORP) (IPH, Omaha, NE) which is a method of refining seed oils. ORP may have advantages over traditional chemical refining, including improved refined oil yield, value added co-products, reduced capital costs and lower environmental costs.

The enzymes of the invention can be used in processes for the treatment of an oil or fat, animal or vegetal, raw, semi-processed or refined, comprising adding to such oil or fat at least one enzyme of the invention that allows hydrolyzing and/or depolymerizing the non-glyceridic compounds contained in the oil, as described, e.g., in EP Application number: 82870032.8. Exemplary methods of the invention for hydrolysis and/or depolymerization of non-glyceridic compounds in oils are:

1) The addition and mixture in oils and fats of an enzyme of the invention or enzyme complexes previously dissolved in a small quantity of appropriate solvent (for example water). A certain number of solvents are possible, but a non-toxic and suitable solvent for the enzyme is chosen. This addition may be done in processes with successive loads, as well as in continuous processes. The quantity of enzyme(s) necessary to be added to oils and fats, according to this process, may range, depending on the enzymes and the products to be processed, from 20 to 400 ppm, i.e., from 0.02 kg to 0.4 kg of enzyme for 1000 kg of oil or fat, and preferably from 20 to 100 ppm, i.e., from 0.02 to 0.1 kg of enzyme for 1000 kg of oil, these values being understood to be for concentrated enzymes, i.e., without diluent or solvent.

2) Passage of the oil or fat through a fixed or insoluble filtering bed of enzyme(s) of the invention on solid or semi-solid supports, preferably presenting a porous or fibrous structure. In this technique, the enzymes are trapped in the micro-cavities of the porous or fibrous structure of the supports. These consist, for example, of resins or synthetic polymers, cellulose carbonates, gels such as agarose, filaments of polymers or copolymers with porous structure, trapping small droplets of enzyme in solution in their cavities. Concerning the enzyme concentration, it is possible to go up to the saturation of the supports.

3) Dispersion of the oils and fats in the form of fine droplets, in a diluted enzymatic solution, preferably containing 0.2 to 4% in volume of an enzyme of the invention. This technique is described, e.g., in Belgian patent No. 595,219. A cylindrical column with a height of several meters, with conical lid, is filled with a diluted enzymatic solution. For this purpose, a solvent that is non-toxic and non-miscible in the oil or fat to be processed, preferably water, is chosen. The bottom of the column is equipped with a distribution system in which the oil or fat is continuously injected in an extremely divided form (approximately 10,000 flux per $m^2$). Thus an infinite number of droplets of oil or fat are formed, which slowly rise in the solution of enzymes and meet at the surface, to be evacuated continuously at the top of the conical lid of the reactor.

Palm oil can be pre-treated before treatment with an enzyme of the invention. For example, about 30 kg of raw palm oil is heated to +50°C. 1% solutions were prepared in distilled water with cellulases and pectinases. 600 g of each of these was added to aqueous solutions of the oil under strong agitation for a few minutes. The oil is then kept at +50°C under moderate agitation, for a total reaction time of two hours. Then, temperature is raised to +90°C to deactivate the enzymes and prepare the mixture for filtration and further processing. The oil is dried under vacuum and filtered with a filtering aid.

The enzymes of the invention can be used in processes as described in EP patent EP 0 513 709 B2. For example, the invention provides a process for the reduction of the content process for the reduction of the content of phosphorus-containing components in animal and vegetable oils by enzymatic decomposition using a phospholipase of the invention. A predemucilaginated animal and vegetable oil with a phosphorus content of 50 to 250 ppm is agitated with an organic carboxylic acid and the pH value of the resulting mixture set to pH 4 to pH 6, an enzyme solution which contains phospholipase $A_1$, $A_2$, or B of the invention is added to the mixture in a mixing vessel under turbulent stirring and with the formation of fine droplets, where an emulsion with 0.5 to 5 % by weight relative to the oil is formed, said emulsion being conducted through at least one subsequent reaction vessel under turbulent motion during a reaction time of 0.1 to 10 hours at temperatures in the range of 20 to 80° C and where the treated oil, after separation of the aqueous solution, has a phosphorus content under 5 ppm.

The organic refining process is applicable to both crude and degummed oil. The process uses inline addition of an organic acid under controlled process conditions, in conjunction with conventional centrifugal separation. The water separated naturally from the vegetable oil phospholipids ("VOP") is recycled and reused. The total water usage can be substantially reduced as a result of the Organic Refining Process.

The phospholipases and methods of the invention can also be used in the enzymatic treatment of edible oils, as described, e.g., in U.S. Patent No. 6,162,623. In this exemplary methods, the invention provides an amphiphilic enzyme. It can be immobilized, e.g., by preparing an emulsion containing a continuous hydrophobic phase and a dispersed aqueous phase containing the enzyme and a carrier for the enzyme and removing water from the dispersed phase until this phase turns into solid enzyme coated particles. The enzyme can be a lipase. The immobilized lipase can be used for reactions catalyzed by lipase such as interesterification of mono-, di- or triglycerides, de-acidification of a triglyceride oil, or removal of phospholipids from a triglyceride oil when the lipase is a phospholipase. The aqueous phase may contain a fermentation liquid, an edible triglyceride oil may be the hydrophobic phase, and carriers include sugars, starch, dextran, water soluble cellulose derivatives and fermentation residues. This exemplary method can be used to process triglycerides, diglycerides, monoglycerides, glycerol, phospholipids or fatty acids, which may be in the hydrophobic phase. In one aspect, the process for the removal of phospholipids from triglyceride oil comprising mixing a triglyceride oil containing phospholipids with a preparation containing a phospholipase of the invention; hydrolyzing the phospholipids to lysophospholipid; separating the hydrolyzed phospholipids from the oil, wherein the phospholipase is an immobilized phospholipase.

The enzymes (e.g., lipases, phospholipases, esterases, proteases) of the invention and methods of the invention can also be used in the enzymatic treatment of edible oils, as described, e.g., in U.S. Patent No. 6,127,137. One exemplary method hydrolyzes both fatty acyl groups in intact phospholipid. A phospholipase of the invention used in this method can have no lipase activity and can be active at very low pH. These properties make it very suitable for use in oil degumming, as enzymatic and alkaline hydrolysis (saponification) of the oil can both be suppressed.

In one aspect, the invention provides a process for hydrolyzing fatty acyl groups in a phospholipid or lysophospholipid comprising treating the phospholipid or lysophospholipid with the phospholipase that hydrolyzes both fatty acyl groups in a phospholipid and is essentially free of lipase activity. In one aspect, the phospholipase of the invention has a temperature optimum at about 50°C, measured at pH 3 to pH 4 for 10 minutes, and a pH optimum of about pH 3, measured at 40°C for about 10 minutes. In one aspect, the phospholipid or lysophospholipid comprises lecithin or lysolecithin. In one aspect, after hydrolyzing a major part of the phospholipid, an aqueous phase containing the hydrolyzed phospholipid is separated from the oil. In one aspect, the invention provides a process for removing phospholipid from an edible oil, comprising treating the oil at pH 1.5 to 3 with a dispersion of an aqueous solution of the phospholipase of the invention, and separating an aqueous phase containing the hydrolyzed phospholipid from the oil. In one aspect, the oil is treated to remove mucilage prior to the treatment with the phospholipase. In one aspect, the oil prior to the treatment with the phospholipase contains the phospholipid in an amount corresponding to 50 to 250 ppm of phosphorus. In one aspect, the treatment with phospholipase is done at 30°C to 45°C for 1 to 12 hours at a phospholipase dosage of 0.1 to 10 mg/l in the presence of 0.5 to 5% of water.

The enzymes (e.g., lipases, phospholipases, esterases, proteases) of the invention and methods of the invention can also be used in the enzymatic treatment of edible oils, as described, e.g., in U.S. Patent No. 6,025,171. In this exemplary method, enzymes of the invention are immobilized by preparing an emulsion containing a continuous hydrophobic phase, such as a triglyceride oil, and a dispersed aqueous phase containing an amphiphilic enzyme, such as lipase or a phospholipase of the invention, and carrier material that is partly dissolved and partly undissolved in the aqueous phase, and removing water from the aqueous phase until the phase turns into solid enzyme coated carrier particles. The undissolved part of the carrier material may be a material that is insoluble in water and oil, or a water soluble material in undissolved form because the aqueous phase is already saturated with the water soluble material. The aqueous phase may be formed with a crude lipase fermentation liquid containing fermentation residues and biomass that can serve as carrier materials. Immobilized lipase is useful for ester rearrangement and de-acidification in oils. After a reaction, the immobilized enzyme can be regenerated for a subsequent reaction by adding water to obtain partial dissolution of the carrier, and with the resultant enzyme and carrier-containing aqueous phase dispersed in a hydrophobic phase evaporating water to again form enzyme coated carrier particles.

The enzymes (e.g., lipases, phospholipases, esterases, proteases) of the invention and methods of the invention can also be used in the enzymatic treatment of edible oils, as described, e.g., in U.S. Patent No. 6,143,545. This exemplary method is used for reducing the content of phosphorous containing components in an edible oil comprising a high amount of non-hydratable phosphorus content using a phospholipase of the invention. In one aspect, the method is used to reduce the content of phosphorus containing components in an edible oil having a non-hydratable phosphorus content of at least 50 ppm measured by pre-treating the edible oil, at 60°C, by addition of a solution comprising citric acid monohydrate in water (added water vs. oil equals 4.8% w/w; (citric acid) in water phase = 106 mM, in water/oil emulsion = 4.6 mM) for 30 minutes; transferring 10 ml of the pre-treated water in oil emulsion to a tube; heating the emulsion in a boiling water bath for 30 minutes; centrifuging at 5000 rpm for 10 minutes, transferring about 8 ml of the upper (oil) phase to a new tube and leaving it to settle for 24 hours; and drawing 2 g from the upper clear phase for measurement of the non-hydratable phosphorus content (ppm) in the edible oil. The method also can comprise contacting an oil at a pH from about pH 5 to 8 with an aqueous solution of a phospholipase A or B of the invention (e.g., PLA1, PLA2, or a PLB), which solution is emulsified in the oil until the phosphorus content of the oil is reduced to less than 11 ppm, and then separating the aqueous phase from the treated oil.

The enzymes (e.g., lipases, phospholipases, esterases, proteases) of the invention and methods of the invention can also be used in the enzymatic treatment of edible oils, as described, e.g., in U.S. Patent No. 5,532,163. The invention provides processes for the refining of oil and fat by which phospholipids in the oil and fat to be treated can be decomposed and removed efficiently. In one aspect, the invention provides a process for the refining of oil and fat which comprises reacting, in an emulsion, the oil and fat with an enzyme of the invention, e.g., an enzyme having an activity to decompose glycerol-fatty acid ester bonds in glycerophospholipids (e.g., a PLA2 of the invention); and another process in which the enzyme-treated oil and fat is washed with water or an acidic aqueous solution. In one aspect, the acidic aqueous solution to be used in the washing step is a solution of at least one acid, e.g., citric acid, acetic acid, phosphoric acid and salts thereof. In one aspect, the emulsified condition is formed using 30 weight parts or more of water per 100 weight parts of the oil and fat. Since oil and fat can be purified without employing the conventional alkali refining step, generation of washing waste water and industrial waste can be reduced. In addition, the recovery yield of oil is improved because loss of neutral oil and fat due to their inclusion in these wastes does not occur in the inventive process. In one aspect, the invention provides a process for refining oil and fat containing about 100 to 10,000 ppm of phospholipids which comprises: reacting, in an emulsified condition, said oil and fat with an enzyme of the invention having activity to decompose glycerol-fatty acid ester bonds in glycerophospholipids. In one aspect, the invention provides processes for refining oil and fat containing about 100 to 10,000 ppm of phospholipids which comprises reacting, in an emulsified condition, oil and fat with an enzyme of the invention having activity to decompose glycerol-fatty acid ester bonds in glycerophospholipids; and subsequently washing the treated oil and fat with a washing water.

The enzymes (e.g., lipases, phospholipases, esterases, proteases) of the invention and methods of the invention can also be used in the enzymatic treatment of edible oils, as described, e.g., in U.S. Patent No. 5,264,367. The content of phosphorus-containing components and the iron content of an edible vegetable or animal oil, such as an oil, e.g., soybean oil, which has been wet-refined to remove mucilage, are reduced by enzymatic decomposition by contacting the oil with an aqueous solution of an enzyme of the invention, e.g., a phospholipase A1, A2, or B, and then separating the aqueous phase from the treated oil. In one aspect, the invention provides an enzymatic method for decreasing the content of phosphorus- and iron-containing components in oils, which have been refined to remove mucilage. An oil, which has been refined to remove mucilage, can be treated with an enzyme of the invention, e.g., phospholipase C, A1, A2, or B. Phosphorus contents below 5 ppm and iron contents below 1 ppm can be achieved. The low iron content can be advantageous for the stability of the oil.

The enzymes (e.g., lipases, phospholipases, esterases, proteases) of the invention and methods of the invention can also be used for preparing transesterified oils, as described, e.g., in U.S. Patent No. 5,288,619. The invention provides methods for enzymatic transesterification for preparing a margarine oil having both low trans- acid and low intermediate chain fatty acid content. The method includes the steps of providing a transesterification reaction mixture containing a stearic acid source material and an edible liquid vegetable oil, transesterifying the stearic acid source material and the vegetable oil using a 1-, 3- positionally specific lipase, and then finally hydrogenating the fatty acid mixture to provide a recycle stearic acid source material for a recyclic reaction with the vegetable oil. The invention also provides a counter- current method for preparing a transesterified oil. The method includes the steps of providing a transesterification reaction zone containing a 1-, 3-positionally specific lipase, introducing a vegetable oil into the trans-esterification zone, introducing a stearic acid source material, conducting a supercritical gas or subcritical liquefied gas counter- current fluid, carrying out a transesterification reaction of the triglyceride stream with the stearic acid or stearic acid monoester stream in the reaction zone, withdrawing a transesterified triglyceride margarine oil stream, withdrawing a counter-current fluid phase, hydrogenating the transesterified stearic acid or stearic acid monoester to provide a hydrogenated recycle stearic acid source material, and introducing the hydrogenated recycle stearic acid source material into the reaction zone.

In one aspect, the highly unsaturated phospholipid compound may be converted into a triglyceride by appropriate use of a phospholipase C of the invention to remove the phosphate group in the sn-3 position, followed by 1,3 lipase acyl ester synthesis. The 2-substituted phospholipid may be used as a functional food ingredient directly, or may be subsequently selectively hydrolyzed in reactor 160 using an immobilized phospholipase C of the invention to produce a 1- diglyceride, followed by enzymatic esterification as described herein to produce a triglyceride product having a 2-substituted polyunsaturated fatty acid component.

The enzymes (e.g., lipases, phospholipases, esterases, proteases) of the invention and methods of the invention can also be used in a vegetable oil enzymatic degumming process as described, e.g., in U.S. Patent No. 6,001,640. This method of the invention comprises a degumming step in the production of edible oils. Vegetable oils from which hydratable phosphatides have been eliminated by a previous aqueous degumming process are freed from non- hydratable phosphatides by enzymatic treatment using a phospholipase of the invention. The process can be gentle, economical and environment-friendly. Phospholipases that only hydrolyze lysolecithin, but not lecithin, are used in this degumming process.

In one aspect, to allow the enzyme of the invention to act, both phases, the oil phase and the aqueous phase that contain the enzyme, must be intimately mixed. It may not be sufficient to merely stir them. Good dispersion of the enzyme in the oil is aided if it is dissolved in a small amount of water, e.g., 0.5-5 weight-% (relative to the oil), and emulsified in the oil in this form, to form droplets of less than 10 micrometers in diameter (weight average). The droplets can be smaller than 1 micrometer. Turbulent stirring can be done with radial velocities above 100 cm/sec. The oil also can be circulated in the reactor using an external rotary pump. The aqueous phase containing the enzyme can also be finely dispersed by means of ultrasound action. A dispersion apparatus can be used.

The enzymatic reaction probably takes place at the border surface between the oil phase and the aqueous phase. It is the goal of all these measures for mixing to create the greatest possible surface for the aqueous phase which contains the enzyme. The addition of surfactants increases the microdispersion of the aqueous phase. In some cases, therefore, surfactants with HLB values above 9, such as Na-dodecyl sulfate, are added to the enzyme solution, as described, e.g., in EP-A 0 513 709. A similar effective method for improving emulsification is the addition of lysolecithin. The amounts added can lie in the range of 0.001 % to 1%, with reference to the oil. The temperature during enzyme treatment is not critical. Temperatures between 20°C and 80°C can be used, but the latter can only be applied for a short time. In this aspect, a phospholipase of the invention having a good temperature and/or low pH tolerance is used. Application temperatures of between 30°C and 50°C are optimal. The treatment period depends on the temperature and can be kept shorter with an increasing temperature. Times of 0.1 to 10 hours, or, 1 to 5 hours are generally sufficient. The reaction takes place in a degumming reactor, which can be divided into stages, as described, e.g., in DE-A 43 39 556. Therefore continuous operation is possible, along with batch operation. The reaction can be carried out in different temperature stages. For example, incubation can take place for 3 hours at 40°C, then for 1 hour at 60°C. If the reaction proceeds in stages, this also opens up the possibility of adjusting different pH values in the individual stages. For example, in the first stage the pH of the solution can be adjusted to 7, for example, and in a second stage to 2.5, by adding citric acid. In at least one stage, however, the pH of the enzyme solution must be below 4, or, below 3. If the pH was subsequently adjusted below this level, a deterioration of effect may be found. Therefore the citric acid can be added to the enzyme solution before the latter is mixed into the oil.

After completion of the enzyme treatment, the enzyme solution, together with the decomposition products of the NHP contained in it, can be separated from the oil phase, in batches or continuously, e.g., by means of centrifugation. Since the enzymes are characterized by a high level of stability and the amount of the decomposition products contained in the solution is slight (they may precipitate as sludge) the same aqueous enzyme phase can be used several times. There is also the possibility of freeing the enzyme of the sludge, see, e.g., DE-A 43 39 556, so that an enzyme solution which is essentially free of sludge can be used again. In one aspect of this degumming process, oils which contain less than 15 ppm phosphorus are obtained. One goal is phosphorus contents of less than 10 ppm; or, less than 5 ppm. With phosphorus contents below 10 ppm, further processing of the oil according to the process of distillative de-acidification is easily possible. A number of other ions, such as magnesium, calcium, zinc, as well as iron, can be removed from the oil, e.g., below 0.1 ppm. Thus, this product possesses ideal prerequisites for good oxidation resistance during further processing and storage.

The enzymes (e.g., lipases, phospholipases, esterases, proteases) of the invention and methods of the invention also can also be used for reducing the amount of phosphorous-containing components in vegetable and animal oils as described, e.g., in EP patent EP 0513709. In this method, the content of phosphorus-containing components, especially phosphatides, such as lecithin, and the iron content in vegetable and animal oils, which have previously been deslimed, e.g. soya oil, are reduced by enzymatic breakdown using a phospholipase A1, A2 or B of the invention.

The enzymes (e.g., lipases, phospholipases, esterases, proteases) of the invention and methods of the invention can also be used for refining fat or oils as described, e.g., in JP 06306386. The invention provides processes for refining a fat or oil comprising a step of converting a phospholipid in a fat or an oil into a water-soluble phosphoric-group-containing substance and removing this substance. The action of an enzyme of the invention (e.g., a PLC) is utilized to convert the phospholipid into the substance. Thus, it is possible to refine a fat or oil without carrying out an alkali refining step from which industrial wastes containing alkaline waste water and a large amount of oil are produced. Improvement of yields can be accomplished because the loss of neutral fat or oil from escape with the wastes can be reduced to zero. In one aspect, gummy substances are converted into water-soluble substances and removed as water-soluble substances by adding an enzyme of the invention having a phospholipase C activity in the stage of degumming the crude oil and conducting enzymatic treatment. In one aspect, the phospholipase C of the invention has an activity that cuts ester bonds of glycerin and phosphoric acid in phospholipids. If necessary, the method can comprise washing the enzyme-treated oil with water or an acidic aqueous solution. In one aspect, the enzyme of the invention is added to and reacted with the crude oil. The amount of phospholipase C employed can be 10 to 10,000 units, or, about 100 to 2,000 units, per 1 kg of crude oil.

The enzymes (e.g., lipases, phospholipases, esterases, proteases) of the invention and methods of the invention can also be used for water-degumming processes as described, e.g., in Dijkstra, Albert J., et al., Oleagineux, Corps Gras, Lipides (1998), 5(5), 367-370. In this exemplary method, the water-degumming process is used for the production of lecithin and for dry degumming processes using a degumming acid and bleaching earth. This method may be economically feasible only for oils with a low phosphatide content, e.g., palm oil, lauric oils, etc. For seed oils having a high NHP-content, the acid refining process is used, whereby this process is carried out at the oil mill to allow gum disposal via the meal. In one aspect, this acid refined oil is a possible "polishing" operation to be carried out prior to physical refining.

The enzymes (e.g., lipases, phospholipases, esterases, proteases) of the invention and methods of the invention can also be used for degumming processes as described, e.g., in Dijkstra, et al., Res. Dev. Dep., N.V. Vandemoortele Coord. Cent., Izegem, Belg. JAOCS, J. Am. Oil Chem. Soc. (1989), 66:1002-1009. In this exemplary method, the total degumming process involves dispersing an acid such as $H_3PO_4$ or citric acid into soybean oil, allowing a contact time, and then mixing a base such as caustic soda or Na silicate into the acid-in-oil emulsion. This keeps the degree of neutralization low enough to avoid forming soaps, because that would lead to increased oil loss. Subsequently, the oil passed to a centrifugal separator where most of the gums are removed from the oil stream to yield a gum phase with minimal oil content. The oil stream is then passed to a second centrifugal separator to remove all remaining gums to yield a dilute gum phase, which is recycled. Washing and drying or in-line alkali refining complete the process. After the adoption of the total degumming process, in comparison with the classical alkali refining process, an overall yield improvement of about 0.5% is realized. The totally degummed oil can be subsequently alkali refined, bleached and deodorized, or bleached and physically refined.

The enzymes (e.g., lipases, phospholipases, esterases, proteases) of the invention and methods of the invention can also be used for the removal of nonhydratable phospholipids from a plant oil, e.g., soybean oil, as described, e.g., in Hvolby, et al., Sojakagefabr., Copenhagen, Den., J. Amer. Oil Chem. Soc. (1971) 48:503-509. In this exemplary method, water-degummed oil is mixed at different fixed pH values with buffer solutions with and without $Ca^{++}$, Mg/Ca-binding reagents, and surfactants. The nonhydratable phospholipids can be removed in a nonconverted state as a component of micelles or of mixed emulsifiers. Furthermore, the nonhydratable phospholipids are removable by conversion into dissociated forms, e.g., by removal of Mg and Ca from the phosphatidates, which can be accomplished by acidulation or by treatment with Mg/Ca-complexing or Mg/Ca-precipitating reagents. Removal or chemical conversion of the nonhydratable phospholipids can result in reduced emulsion formation and in improved separation of the deacidified oil from the emulsion layer and the soapstock.

The enzymes (e.g., lipases, phospholipases, esterases, proteases) of the invention and methods of the invention can also be used for the degumming of vegetable oils as described, e.g., Buchold, et al., Frankfurt/Main, Germany. Fett Wissenschaft Technologie (1993), 95(8), 300-304. In this exemplary process of the invention for the degumming of edible vegetable oils, aqueous suspensions of an enzyme of the invention, e.g., phospholipase A2, is used to hydrolyze the fatty acid bound at the sn2 position of the phospholipid, resulting in 1-acyl-lysophospholipids which are insoluble in oil and thus more amenable to physical separation. Even the addition of small amounts corresponding to about 700 lecitase units/kg oil results in a residual P concentration of less than 10 ppm, so that chemical refining is replaceable by physical refining, eliminating the necessity for neutralization, soapstock splitting, and wastewater treatment.

The enzymes (e.g., lipases, phospholipases, esterases, proteases) of the invention and methods of the invention can also be used for the degumming of vegetable oils as described, e.g., by EnzyMax, Dahlke, Klaus. Dept. G-PDO, Lurgi Ol-Gas, Chemie, GmbH, Frankfurt, Germany; Oleagineux, Corps Gras, Lipides (1997), 4(1), 55-57. This exemplary process is a degumming process for the physical refining of almost any kind of oil. By an enzymatic-catalyzed hydrolysis, phosphatides are converted to water-soluble lysophosphatides which are separated from the oil by centrifugation. The residual phosphorus content in the enzymatically degummed oil can be as low as 2 ppm P.

The enzymes (e.g., lipases, phospholipases, esterases, proteases) of the invention and methods of the invention can also be used for the degumming of vegetable oils as described, e.g., by Cleenewerck, et al., N.V. Vamo Mills, Izegem, Belg. Fett Wissenschaft Technologie (1992), 94:317-22; and, Clausen, Kim; Nielsen, M., Novozymes A/S, Den. Dansk Kemi (2002) 83(2):24-27. The phospholipases and methods of the invention can incorporate the pre-refining of vegetable oils with acids as described, e.g., by Nilsson-Johansson, et al., Fats Oils Div., Alfa-Laval Food Eng. AB, Tumba, Swed. Fett Wissenschaft Technologie (1988), 90(11), 447-51; and, Munch, Ernst W. Cereol Deutschland GmbH, Mannheim, Germany. Editor(s): Wilson, Richard F., Proceedings of the World Conference on Oilseed Processing Utilization, Cancun, Mexico, Nov. 12-17, 2000 (2001), Meeting Date 2000, 17-20.

The enzymes (e.g., lipases, phospholipases, esterases, proteases) of the invention and methods of the invention can also be used for the degumming of vegetable oils as described, e.g., by Jerzewska, et al., Inst. Przemyslu Miesnego i Tluszczowego, Warsaw, Pol., Tluszcze Jadalne (2001), 36(3/4), 97-110. In this process of the invention, enzymatic degumming of hydrated low-erucic acid rapeseed oil is by use of a phospholipase A2 of the invention. The enzyme can catalyze the hydrolysis of fatty acid ester linkages to the central carbon atom of the glycerol moiety in phospholipids. It can hydrolyze non-hydratable phospholipids to their corresponding hydratable lyso-compounds. With a nonpurified enzyme preparation, better results can be achieved with the addition of 2% preparation for 4 hours (87% P removal).

In another exemplary process of the invention for oil degumming (or an oil degumming process using an enzyme of the invention), an acidic polymer, e.g., an alginate or pectin, is added. In this oil degumming process of the invention, an acidic polymer (e.g. alginic acid or pectin or a more soluble salt form) is added to the crude oil with a low amount of water (e.g., in a range of between about 0.5 to 5%). In this aspect, the acidic polymers can reduce and/or disrupt phospholipid-metal complexes by binding calcium and/or magnesium in the crude oil, thereby improving the solubility of nonhydratable phospholipids. In one aspect, these phospholipids will enter the aqueous phase and either be converted to diacylglycerol and the corresponding side chain or the intact phospholipid will be removed by subsequent centrifugation as a component of the heavy phase. The presence of the acidic polymer in the aqueous phase can also increase the density of the aqueous phase and result in an improved separation of the heavy phase from the oil (light) phase.

One exemplary process of the invention for oil degumming (or an oil degumming process using an enzyme of the invention) alters the deodorization procedure to get a diacylglycerol (DAG) fraction. In alternative aspect, if necessary or desired, following enzyme-assisted degumming, the deodorization conditions (temperature, pressure, configuration of the distillation apparatus) can be modified with the goal of improving the separation of the free fatty acids (FFA) from the diacylglycerol/triacylglycerol fraction or further modified to separate the diacylglycerol from the triacylglycerol fraction. As a result of these modifications, using this method of the invention, it is possible to obtain food grade FFA and diacylglycerol if a hydrolase of the invention (e.g., a phosphatase, or, a PLC or a combination of PLC and phosphatases) are used to degum edible oil in a physical refining process.

In various aspects, practicing the methods of the invention as described herein (or using the enzymes of the invention), have advantages such as: decrease or eliminate solvent and solvent recovery; lower capital costs; decrease downstream refining costs, decrease chemical usage, equipment, process time, energy (heat) and water usage/wastewater generation; produce higher quality oil; expeller pressed oil may be used without refining in some cooking and sautéing applications (this pressed oil may have superior stability, color and odor characteristics and high tocopherol content); produce higher quality meal; produce a lower fat content in meal (currently, meal coming out of mechanical press causes digestion problems in ruminants); produce improved nutritional attributes - reduced levels of glucosinolates, tannins, sinapine, phytic acid (as described, e.g., in Technology and Solvents for Extracting Oilseeds and Nonpetroleum Oils, AOCS 1997).

In one aspect, the invention provides methods for refining vegetable oils (e.g., soybean oil, corn oil, cottonseed oil, palm oil, peanut oil, rapeseed oil, safflower oil, sunflower seed oil, sesame seed oil, rice bran oil, coconut oil or canola oil) and their byproducts, and processes for deodorizing lecithin, for example, as described in U.S. Patent No. 6,172,248, or 6,172,247, wherein the methods comprise use of at least one hydrolase of the invention, e.g., a phospholipase, such as a phospholipase C of the invention. Thus, the invention provides lecithin and vegetable oils comprising at least one enzyme of the invention. In an exemplary organic acid refining process, vegetable oil is combined with a dilute aqueous organic acid solution and subjected to high shear to finely disperse the acid solution in the oil. The resulting acid-and-oil mixture is mixed at low shear for a time sufficient to sequester contaminants into a hydrated impurities phase, producing a purified vegetable oil phase. In this exemplary process, a mixer or recycle system (e.g., recycle water tank) and/or a phosphatide or lecithin storage tank can be used, e.g., as described in U.S. Patent Nos. 4,240,972, 4,049,686, 6,172,247 or 6,172,248. These processes can be conducted as a batch or continuous process. Crude or degummed vegetable oil can be supplied from a storage tank (e.g., through a pump) and can be heated. The vegetable oil to be purified can be either crude or "degummed" oil.

In one aspect, hydrolase enzymes such as the phosphatidylinositol-PLC (PI-PLC) enzymes of the invention are used for vegetable oil degumming. Hydrolase enzymes of the invention having PI-PLC activity can be used alone or in combination with other enzymes (for instance PLC, PLD, phosphatase enzymes of the invention) to improve oil yield during the degumming of vegetable oils (including soybean, canola, and sunflower). The PI-PLC enzymes of the invention may preferentially convert phosphatidylinositol to 1, 2-diacylglycerol (DAG) and phosphoinositol but it may also demonstrate activity on other phospholipids including phosphatidylcholine, phosphatidylethanolamine, phosphatidylserine, or phosphatidic acid. The improvement in yield will be realized as an increase in the amount of DAG in the enzyme-treated vegetable oil and an increase in neutral oil, due to a decrease in the amount of oil entrained in the smaller gum fraction that results from enzyme treatment of the vegetable oil.

*Purification of phytosterols from vegetable oils*

The invention provides methods for purification of phytosterols and triterpenes, or plant sterols, from vegetable oils using the enzymes of the invention. Phytosterols that can be purified using enzymes (e.g., phospholipases) and methods of the invention include β-sitosterol, campesterol, stigmasterol, stigmastanol, β-sitostanol, sitostanol, desmosterol, chalinasterol, poriferasterol, clionasterol and brassicasterol. Plant sterols are important agricultural products for health and nutritional industries. Thus, enzymes and methods of the invention are used to make emulsifiers for cosmetic manufacturers and steroidal intermediates and precursors for the production of hormone pharmaceuticals. Enzymes and methods of the invention are used to make (e.g., purify) analogs of phytosterols and their esters for use as cholesterol-lowering agents with cardiologic health benefits. Enzymes and methods of the invention are used to purify plant sterols to reduce serum cholesterol levels by inhibiting cholesterol absorption in the intestinal lumen. Enzymes and methods of the invention are used to purify plant sterols that have immunomodulating properties at extremely low concentrations, including enhanced cellular response of T lymphocytes and cytotoxic ability of natural killer cells against a cancer cell line. Enzymes and methods of the invention are used to purify plant sterols for the treatment of pulmonary tuberculosis, rheumatoid arthritis, management of HIV-infested patients and inhibition of immune stress, e.g., in marathon runners.

Enzymes and methods of the invention are used to purify sterol components present in the sterol fractions of commodity vegetable oils (e.g., coconut, canola, cocoa butter, corn, cottonseed, linseed, olive, palm, peanut, rice bran, safflower, sesame, soybean, sunflower oils), such as sitosterol (40.2-92.3 %), campesterol (2.6-38.6 %), stigmasterol (0-31 %) and 5-avenasterol (1.5 -29 %).

Methods of the invention can incorporate isolation of plant-derived sterols in oil seeds by solvent extraction with chloroform-methanol, hexane, methylene chloride, or acetone, followed by saponification and chromatographic purification for obtaining enriched total sterols. Alternatively, the plant samples can be extracted by supercritical fluid extraction with supercritical carbon dioxide to obtain total lipid extracts from which sterols can be enriched and isolated. For subsequent characterization and quantification of sterol compounds, the crude isolate can be purified and separated by a wide variety of chromatographic techniques including column chromatography (CC), gas chromatography, thin-layer chromatography (TLC), normal phase high-performance liquid chromatography (HPLC), reversed-phase HPLC and capillary electrochromatography. Of all chromatographic isolation and separation techniques, CC and TLC procedures employ the most accessible, affordable and suitable for sample clean up, purification, qualitative assays and preliminary estimates of the sterols in test samples.

Phytosterols are lost in the vegetable oils lost as byproducts during edible oil refining processes. Phospholipases and methods of the invention use phytosterols isolated from such byproducts to make phytosterol-enriched products isolated from such byproducts. Phytosterol isolation and purification methods of the invention can incorporate oil processing industry byproducts and can comprise operations such as molecular distillation, liquid-liquid extraction and crystallization.

Methods of the invention can incorporate processes for the extraction of lipids to extract phytosterols. For example, methods of the invention can use nonpolar solvents as hexane (commonly used to extract most types of vegetable oils) quantitatively to extract free phytosterols and phytosteryl fatty-acid esters. Steryl glycosides and fatty-acylated steryl glycosides are only partially extracted with hexane, and increasing polarity of the solvent gave higher percentage of extraction. One procedure that can be used is the Bligh and Dyer chloroform-methanol method for extraction of all sterol lipid classes, including phospholipids. One exemplary method to both qualitatively separate and quantitatively analyze phytosterol lipid classes comprises injection of the lipid extract into HPLC system.

Enzymes and methods of the invention can be used to remove sterols from fats and oils, as described, e.g., in U.S. Patent No. 6,303,803. This is a method for reducing sterol content of sterol-containing fats and oils. It is an efficient and cost effective process based on the affinity of cholesterol and other sterols for amphipathic molecules that form hydrophobic, fluid bilayers, such as phospholipid bilayers. Aggregates of phospholipids are contacted with, for example, a sterol-containing fat or oil in an aqueous environment and then mixed. The molecular structure of this aggregated phospholipid mixture has a high affinity for cholesterol and other sterols, and can selectively remove such molecules from fats and oils. The aqueous separation mixture is mixed for a time sufficient to selectively reduce the sterol content of the fat/oil product through partitioning of the sterol into the portion of phospholipid aggregates. The sterol-reduced fat or oil is separated from the aqueous separation mixture. Alternatively, the correspondingly sterol-enriched fraction also may be isolated from the aqueous separation mixture. These steps can be performed at ambient temperatures, costs involved in heating are minimized, as is the possibility of thermal degradation of the product. Additionally, a minimal amount of equipment is required, and since all required materials are food grade, the methods require no special precautions regarding handling, waste disposal, or contamination of the final product(s).

Enzymes and methods of the invention can be used to remove sterols from fats and oils, as described, e.g., in U.S. Patent No. 5,880,300. Phospholipid aggregates are contacted with, for example, a sterol-containing fat or oil in an aqueous environment and then mixed. Following adequate mixing, the sterol-reduced fat or oil is separated from the aqueous separation mixture. Alternatively, the correspondingly sterol-enriched phospholipid also may be isolated from the aqueous separation mixture. Plant (e.g., vegetable) oils contain plant sterols (phytosterols) that also may be removed using the methods of the present invention. This method is applicable to a fat/oil product at any stage of a commercial processing cycle. For example, the process of the invention may be applied to refined, bleached and deodorized oils ("RBD oils"), or to any stage of processing prior to attainment of RBD status. Although RBD oil may have an altered density compared to pre-RBD oil, the processes of the are readily adapted to either RBD or pre-RBD oils, or to various other fat/oil products, by variation of phospholipid content, phospholipid composition, phospholipid:water ratios, temperature, pressure, mixing conditions, and separation conditions as described below.

Alternatively, the enzymes and methods of the invention can be used to isolate phytosterols or other sterols at intermediate steps in oil processing. For example, it is known that phytosterols are lost during deodorization of plant oils. A sterol-containing distillate fraction from, for example, an intermediate stage of processing can be subjected to the sterol-extraction procedures described above. This provides a sterol-enriched lecithin or other phospholipid material that can be further processed in order to recover the extracted sterols.

*Nutraceuticals*

In one aspect, the compositions and methods of the invention can be used to make nutraceuticals by processing or synthesizing lipids and oils using the enzymes of the invention, e.g., esterases, acylases, lipases, phospholipases or proteases of the invention. In one aspect, the processed or synthesized lipids or oils include poly-unsaturated fatty acids (PUFAs), diacylglycerides, e.g., 1,3-diacyl glycerides (DAGs), monoacylglycerides, e.g., 2-monoacylglycerides (MAGs) and triacylglycerides (TAGs). In one aspect, the nutraceuticals is made by processing diacylglycerides, e.g., 1,3-diacyl glycerides (DAGs), monoacylglycerides, e.g., 2-monoacylglycerides (MAGs) and/or triacylglycerides (TAGs) from plant (e.g., oilseed) sources or from animal (e.g., fish oil) sources.

In one aspect, the compositions and methods of the invention can be used to fortify dietary compositions, especially cow's milk based products, e.g., cow's milk-based infant formulas, with bile salt-activated hydrolases. The compositions made by the methods and compositions of the invention can be used to feed newborn and premature infants, including administration of a bile salt-activated hydrolase of the invention to increase fat digestion and therefore growth rate. Similarly, the invention provides compositions and methods for treating subjects for inadequate pancreatic enzyme production by administration of bile salt-activated hydrolase in conjunction with ingestion of fats; see also discussion, below.

In one aspect, the invention provides a dietary composition comprising a hydrolase of the invention, e.g., bile salt-activated hydrolase of the invention. In one aspect, the invention provides a dietary composition comprising a nutritional base comprising a fat and an effective amount of bile salt-activated hydrolase of the invention. In one aspect, the invention provides a cow's milk-based infant formula comprising a hydrolase of the invention, e.g., bile salt-activated hydrolase of the invention. In one aspect, the hydrolase of the invention is active in the digestion of long chain fatty acids, e.g., $C_{12}$ to $C_{22}$, which make up a very high percentage of most milks, e.g., 99% of human breast milk. See, e.g., U.S. Patent No. 5,000,975.

In one aspect, the invention provides a dietary composition comprising a vegetable oil fat and a hydrolase of the invention. The invention provides methods of processing milk based products and/or vegetable oil-comprising compositions to make dietary compositions. In one aspect, the processed compositions comprise a lauric acid oil, an oleic acid oil, a palmitic acid oil and/or a linoleic acid oil. In one aspect, a rice bran oil, sunflower oleic oil and/or canola oil may be used as oleic acids oils. In one aspect, fats and oils, e.g., oilseeds, from plants, including, e.g., rice, canola, sunflower, olive, palm, soy or lauric type oils for use in the nutraceuticals and dietary compositions are processed or made using a hydrolase of the invention. See, e.g., U.S. Patent No. 4,944,944.

In one aspect, the enzymes of the invention are provided in a form that is stable to storage in the formula and/or the stomach, but active when the formulation reaches the portion of the gastrointestinal tract where the formula would normally be digested. Formulations (e.g., microcapsules) for release in the intestine are well known in the art, e.g., biodegradable polymers such as polylactide and polyglycolide, as described, e.g., in U.S. Patent. Nos. 4,767,628; 4,897,268; 4,925,673; 5,902,617.

*Confectionaries, cacao butter and foods*

In one aspect, the compositions and methods of the invention can be used to make and process hard butters, such as cacao butter (cocao butter). The compositions and methods of the invention can be used to make cocoa butter alternatives by "structured" synthetic techniques using the enzymes of the invention, e.g., esterases, acylases, lipases, phospholipases or proteases of the invention. For example, in one aspect, the methods of the invention process or synthesize triacylglycerides, diacylglycerides and/or monoacylglycerides for use as, e.g., cocoa butter alternatives. In one aspect, the methods of the invention generate a hard butter with a defined "plastic region" to maintain sufficient hardness below or at room temperature. In one aspect, the processed or synthesized lipid is designed to have a very narrow "plastic region," e.g., in one aspect, where it rapidly melts at about body temperature. Natural cacao butter begins to soften at approximately $30^0$C to $32^0$C, and completely melts at approximately $36^0$C. Natural cacao butter can contain 70 wt % or more of three 1,3-disaturated -2-oleoyl glycerols, which are 1,3-dipalmitoyl-2-oleoyl glycerol (POP), 1-palmitoyl-2-oleoyl glycerol (POSt) and 1,3-distearoyl-2-oleoyl glycerol (StOSt). These three glycerols show a similar melting behavior to each other and are responsible for melting properties of the cacao butter, exhibiting a very narrow plastic region. The invention provides synthetic cacao butters or processed cacao butters (synthesized or processed using a hydrolase of the invention, all possible composition are referred to as cocoa-butter alternatives) with varying percentages of ,3-dipalmitoyl-2-oleoyl glycerol (POP), 1-palmitoyl-2-oleoyl glycerol (POSt) and 1,3-distearoyl-2-oleoyl glycerol (StOSt), depending on the desired properties of the synthetic cacao butter, and, synthetic cacao butters with more or less than 70 wt % of the three 1,3-disaturated -2-oleoyl glycerols. The synthetic cacao butters of the invention can partially or completely replace natural or unprocessed cacao butters and can maintain or improve essential hard butter properties.

The invention provides synthetic cacao butters or processed cacao butters (synthesized or processed using a hydrolase of the invention) with desired properties for use in confectionary, bakery and pharmaceutical products. In one aspect, the invention provides confectionary, bakery and pharmaceutical products comprising a hydrolase of the invention. In one aspect, the methods of the invention make or process a lipid (a fat) from a confection (e.g., a chocolate) or to be used in a confection. In one aspect, a lipid is made or processed such that the chocolate shows less finger-imprinting than chocolate made from natural cocoa butter, while still having sharp melting characteristics in the mouth. In one aspect, a lipid is made or processed such that a confection (e.g., chocolate) can be made at a comparatively high ambient temperature, or, be made using a cooling water at a comparatively high temperature. In one aspect, the lipid is made or processed such that a confection (e.g., chocolate) can be stored under relatively warmer conditions, e.g., tropical or semi-tropical conditions or in centrally heated buildings. In one aspect, the lipids are made or processed such that a confection (e.g., chocolate) will have a lipid (fat) content of consistent composition and quality. The enzymes of the invention can be used to provide a substitute composition for cacao butter which can significantly improve its thermal stability and replace it in a wide range of applications.

*Margarine and shorteningproduction*

The invention provides synthetic or processed fats, e.g., margarine and shortening synthesized or processed using a hydrolase of the invention. In one aspect, the invention provides processed fats comprising a vegetable oil, such as soybean oil, corn oil, rapeseed oil, palm oil or lauric type oils synthesized or processed using a hydrolase of the invention. The synthetic or processed fats, e.g., margarine and shortening, are designed to have a desired "plasticity." Many of the plastic fat products, such as margarine and shortening, are produced from hard stocks and liquid oils as raw materials. For example, liquid oils such as soybean oil, corn oil, palm oil and rapeseed oil, are blended with their hardened oils (hard stocks), and the blend is adjusted to have an appropriate consistency (plasticity). The plastic fat products such as margarine and shortening so produced tend to cause the formation of relatively coarse crystallines because fats and oils used as the raw materials are composed of fatty acids having almost the same carbon chain length. In other words, they have a highly-unified composition of fatty acids. For this reason, the plasticity of these products can be maintained at an appropriate degree only within a narrow temperature range, so that the liquid oils contained therein have a tendency to exude. In one aspect, the invention provides methods of making or processing fats designed such that they have a varied (and defined) composition of fatty acids. The resultant oil, e.g., margarine or shortening, can have a broader range of plasticity.

In one aspect, the methods and compositions of the invention are used to make or process vegetable oils, such as soybean oil, corn oil, rapeseed oil, palm oil or lauric type oils using the hydrolases of the invention, including inter-esterification and enzymatic transesterification, see e.g., U.S. Patent No. 5,288,619. The methods and compositions of the invention can be used in place of random inter-esterification as described in, e.g., U.S. Patent No. 3,949,105. In one aspect, the methods and compositions of the invention are used to in enzymatic transesterification for preparing an oil, e.g., a margarine oil, having both low trans- acid and low intermediate chain fatty acid content.

In one aspect, the symmetric structure of an oil, e.g., a palm or lauric type oils is modified, e.g., into a random structure. Thus, the methods of the invention can be used to modify the properties of plastic fat products. In one aspect, the modification of oils by the methods of the invention can be designed to prevent or slow gradually hardening of the oil with time, particularly when the products are being stored.

In one aspect, the methods and compositions of the invention in a trans-esterification reaction mixture comprising a stearic acid source material and an edible liquid vegetable oil, trans-esterifying the stearic acid source material and the vegetable oil using a 1-, 3-positionally specific lipase of the invention, and then hydrogenating the fatty acid mixture to provide a recycle stearic acid source material for a recyclic reaction with the vegetable oil. See e.g., U.S. Patent No. 5,288,619.

In one aspect, an inter-esterification reaction is conducted with a lipase of the invention. In one aspect, the lipase of the invention has a selectivity for the 1- and 3-positions of triglyceride to slow or inhibit an increase in the amount of tri-saturated triglycerides in the oil. In this reaction of the invention, deficiencies of conventional random inter- esterification and the difficulty of inter-esterification with a non-specific lipase can be overcome because the inter-esterification is conducted by an enzyme of the invention having a specificity for the 1- and 3- positions of triglycerides. In one aspect, the exudation of liquid oils contained in the products is slowed or prevented with a temperature increase in the reaction to inhibit a rise in the melting point caused by an increase in the amount of tri-saturated triglycerides. This addresses the problem of hardening of products during long-term storage.

*Latex processing*

The methods and compositions (e.g., enzymes of the invention, e.g., esterases, acylases, lipases, phospholipases or proteases of the invention) of the invention can be used to selectively hydrolyze saturated esters over unsaturated esters into acids or alcohols. In one aspect, the invention provides for the selective hydrolysis of ethyl propionate over ethyl acrylate. In one aspect, these methods are used to remove undesired esters from monomer feeds used in latex polymerization and from the latexes after polymerization. The methods and compositions (hydrolases) of the invention can be used to treat latexes for a variety of purposes, e.g., to treat latexes used in hair fixative compositions to remove unpleasant odors. Latexes treated by the methods and compositions of the invention include, e.g., polymers containing acrylic, vinyl and unsaturated acid monomers, including alkyl acrylate monomers such as methyl acrylate, ethyl acrylate, propyl acrylate and butyl acrylate, and acrylate acids such as acrylic acid, methacrylic acid, crotonic acid, itaconic acid and mixtures thereof. See, e.g., U.S. Patent No. 5,856,150.

*Treating hydrolase deficiencies*

The methods and compositions (enzymes of the invention, e.g., esterases, acylases, lipases, phospholipases or proteases of the invention) of the invention can be used in the treatment of a hydrolase deficiency in an animal, e.g., a mammal, such as a human. For example, in one aspect, the methods and compositions of the invention are used to treat patients suffering from a deficiency of a pancreatic lipase. In one aspect, the lipase is administered orally. An enzyme of the invention can be delivered in place of or with a preparation of pig pancreas enzyme.

In one aspect, the compositions of the invention used for these treatments are active under acidic conditions. In one aspect, the compositions of the invention are administered orally in formulations (e.g., tablets) that pass through the acid regions of the stomach and discharge the enzyme only in the relatively alkaline environment of the jejunum. In one aspect, a hydrolase of the invention is formulated with a carrier such as lactose, saccharose, sorbitol, mannitol, starch, cellulose derivatives or gelatine or any other such excipient. A lubricant such as magnesium stearate, calcium stearate or polyethylene glycol wax also can be added. A concentrated sugar solution, which may contain additives such as talc, titanium dioxide, gelatine or gum Arabic, can be added as a coating. Soft or hard capsules can be used to encapsulate a hydrolase as a liquid or as a solid preparation. See, e.g., U.S. Patent No. 5,691,181; 5,858,755.

*Detergents*

The methods and compositions (enzymes of the invention, e.g., esterases, acylases, lipases, phospholipases or proteases of the invention) of the invention can be used in making and using detergents. A hydrolase of the invention can be added to, e.g., be blended with, any known detergent composition, solid or liquid, with or without changing the composition of the detergent composition. For examples, a hydrolase of the invention can be added to any soap, e.g., aliphatic sulfates such as straight or branched chain alkyl or alkenyl sulfates, amide sulfates, alkyl or alkenyl ether sulfates having a straight or branched chain alkyl or alkenyl group to which one or more of ethylene oxide, propylene oxide and butylene oxide added, aliphatic sulfonates such as alkyl sulfonates, amide sulfonates, dialkyl sulfosuccinates, sulfonates of alpha-olefins, of vinylidene-type olefins and of internal olefins, aromatic sulfonates such as straight or branched chain alkylbenzenesulfonates, alkyl or alkenyl ether carbonates or amides having a straight or branched chain alkyl or alkenyl group to which one or more of ethylene oxide, propylene oxide and butylene oxide added, or amides, alpha-sulfo-fatty acid salts or esters, amino acid type surfactants, phosphate surfactants such as alkyl or alkenyl acidic phosphates, and alkyl or alkenyl phosphates, sulfonic acid type amphoteric surfactants, betaine type amphoteric surfactants, alkyl or alkenyl ethers or alcohols having a straight or branched chain alkyl or alkenyl group to which one or more of ethylene oxide, propylene oxide and butylene oxide added, polyoxy-ethylenealkyl phenyl ethers having a straight or branched chain alkyl group to which one or more of ethylene oxide, propylene oxide and butylene oxide added, higher fatty acid alkanolamides or alkylene oxide adducts thereof, sucrose fatty acid esters, fatty acid glycerol monoesters, alkyl- or alkenyl-amine oxides, tetraalkyl-ammonium salt type cationic surfactants, or a combination thereof. See, e.g., U.S. Patent No. 5,827,718.

The invention provides detergent compositions comprising one or more polypeptides (hydrolases) of the invention. Surface-active and/or non-surface-active forms can be used. In one aspect, the amount of total hydrolase, surface-active and/or non-surface-active, used in the invention can be from about 0.0001% to about 1.0%, or from about 0.0002% to about 0.5%, by weight, of the detergent composition. In one aspect, of the detergent composition, the surface-active hydrolase is from about 5% to about 67% and the non-surface-active hydrolase is from about 33% to about 95% of the total hydrolase activity in the enzymatic mixture. In one aspect, the optimum pH of the total enzymatic mixture is between about 5 to about 10.5.

In one aspect, the detergent compositions of the invention include alkaline hydrolases of the invention which function at alkaline pH values, since the pH of a washing solution can be in an alkaline pH range under ordinary washing conditions. See, e.g., U.S. Patent No. 5,454,971

The polypeptides of the invention (enzymes of the invention, e.g., esterases, acylases, lipases, phospholipases or proteases of the invention) can be used in any detergent composition, which are well known in the art, see, e.g., U.S. Patent No. 5,069,810; 6,322,595; 6,313,081. For example, in one aspect, a laundry detergent composition is provided. It can comprise 0.8 ppm to 80 ppm of a lipase of the invention.

The invention incorporates all methods of making and using detergent compositions, see, e.g., U.S. Patent No. 6,413,928; 6,399,561; 6,365,561; 6,380,147.

The invention incorporates all methods of making and using detergent compositions, see, e.g., U.S. Patent No. 6,413,928; 6,399,561; 6,365,561; 6,380,147. The detergent compositions can be a one and two part aqueous composition, a non-aqueous liquid composition, a cast solid, a granular form, a particulate form, a compressed tablet, a gel and/or a paste and a slurry form. The hydrolases of the invention can also be used as a detergent additive product in a solid or a liquid form. Such additive products are intended to supplement or boost the performance of conventional detergent compositions and can be added at any stage of the cleaning process.

The invention also provides methods capable of removing gross food soils, films of food residue and other minor food compositions using these detergent compositions. Hydrolases of the invention can facilitate the removal of stains by means of catalytic hydrolysis of proteins. Hydrolases of the invention can be used in dishwashing detergents in textile laundering detergents.

The actual active enzyme content depends upon the method of manufacture of a detergent composition and is not critical, assuming the detergent solution has the desired enzymatic activity. In one aspect, the amount of hydrolases present in the final solution ranges from about 0.001 mg to 0.5 mg per gram of the detergent composition. The particular enzyme chosen for use in the process and products of this invention depends upon the conditions of final utility, including the physical product form, use pH, use temperature, and soil types to be degraded or altered. The enzyme can be chosen to provide optimum activity and stability for any given set of utility conditions. In one aspect, the hydrolases of the present invention are active in the pH ranges of from about 4 to about 12 and in the temperature range of from about 20°C to about 95°C. The detergents of the invention can comprise cationic, semi-polar nonionic or zwitterionic surfactants; or, mixtures thereof.

Enzymes of the invention can be formulated into powdered and liquid detergents having pH between 4.0 and 12.0 at levels of about 0.01 to about 5% (preferably 0.1 % to 0.5%) by weight. These detergent compositions can also include other enzymes such as proteases, cellulases, lipases or endoglycosidases, endo-beta.-1,4-glucanases, beta-glucanases, endo-beta-1,3(4)-glucanases, cutinases, peroxidases, laccases, amylases, glucoamylases, pectinases, reductases, oxidases, phenoloxidases, ligninases, pullulanases, arabinanases, hemicellulases, mannanases, xyloglucanases, xylanases, pectin acetyl esterases, rhamnogalacturonan acetyl esterases, polygalacturonases, rhamnogalacturonases, galactanases, pectin lyases, pectin methylesterases, cellobiohydrolases and/or transglutaminases. These detergent compositions can also include builders and stabilizers.

The addition of hydrolases of the invention to conventional cleaning compositions does not create any special use limitation. In other words, any temperature and pH suitable for the detergent is also suitable for the compositions of the invention as long as the enzyme is active at or tolerant of the pH and/or temperature of the intended use. In addition, the proteases of the invention can be used in a cleaning composition without detergents, again either alone or in combination with builders and stabilizers.

The present invention provides cleaning compositions including detergent compositions for cleaning hard surfaces, detergent compositions for cleaning fabrics, dishwashing compositions, oral cleaning compositions, denture cleaning compositions, and contact lens cleaning solutions.

In one aspect, the invention provides a method for washing an object comprising contacting the object with a polypeptide of the invention under conditions sufficient for washing. A hydrolase of the invention may be included as a detergent additive. The detergent composition of the invention may, for example, be formulated as a hand or machine laundry detergent composition comprising a polypeptide of the invention. A laundry additive suitable for pre-treatment of stained fabrics can comprise a polypeptide of the invention. A fabric softener composition can comprise a hydrolase of the invention. Alternatively, a hydrolases of the invention can be formulated as a detergent composition for use in general household hard surface cleaning operations. In alternative aspects, detergent additives and detergent compositions of the invention may comprise one or more other enzymes such as a protease, a lipase, a cutinase, another protease, a carbohydrase, a cellulase, a pectinase, a mannanase, an arabinase, a galactanase, a xylanase, an oxidase, e.g., a lactase, and/or a peroxidase (see also, above). The properties of the enzyme(s) of the invention are chosen to be compatible with the selected detergent (i.e. pH-optimum, compatibility with other enzymatic and non-enzymatic ingredients, etc.) and the enzyme(s) is present in effective amounts. In one aspect, enzymes of the invention are used to remove malodorous materials from fabrics. Various detergent compositions and methods for making them that can be used in practicing the invention are described in, e.g., U.S. Patent Nos. 6,333,301; 6,329,333; 6,326,341; 6,297,038; 6,309,871; 6,204,232; 6,197,070; 5,856,164.

When formulated as compositions suitable for use in a laundry machine washing method, the hydrolases of the invention can comprise both a surfactant and a builder compound. They can additionally comprise one or more detergent components, e.g., organic polymeric compounds, bleaching agents, additional enzymes, suds suppressors, dispersants, lime-soap dispersants, soil suspension and anti-redeposition agents and corrosion inhibitors. Laundry compositions of the invention can also contain softening agents, as additional detergent components. Compositions containing hydrolases of the invention can provide fabric cleaning, stain removal, whiteness maintenance, softening, color appearance, dye transfer inhibition and sanitization when formulated as laundry detergent compositions.

The density of.the laundry detergent compositions of the invention can range from about 200 to 1500 g/liter, or, about 400 to 1200 g/liter, or, about 500 to 950 g/liter, or, 600 to 800 g/liter, of composition; this can be measured at about 20°C.

The "compact" form of laundry detergent compositions of the invention is best reflected by density and, in terms of composition, by the amount of inorganic filler salt. Inorganic filler salts are conventional ingredients of detergent compositions in powder form. In conventional detergent compositions, the filler salts are present in substantial amounts, typically 17% to 35% by weight of the total composition. In one aspect of the compact compositions, the filler salt is present in amounts not exceeding 15% of the total composition, or, not exceeding 10%, or, not exceeding 5% by weight of the composition. The inorganic filler salts can be selected from the alkali and alkaline-earth-metal salts of sulphates and chlorides, e.g., sodium sulphate.

Liquid detergent compositions of the invention can also be in a "concentrated form." In one aspect, the liquid detergent compositions can contain a lower amount of water, compared to conventional liquid detergents. In alternative aspects, the water content of the concentrated liquid detergent is less than 40%, or, less than 30%, or, less than 20% by weight of the detergent composition. Detergent compounds of the invention can comprise formulations as described in WO 97/01629.

Hydrolases of the invention can be useful in formulating various cleaning compositions. A number of known compounds are suitable surfactants including nonionic, anionic, cationic, or zwitterionic detergents, can be used, e.g., as disclosed in U.S. Patent Nos. 4,404,128; 4,261,868; 5,204,015. In addition, enzymes of the invention can be used, for example, in bar or liquid soap applications, dish care formulations, contact lens cleaning solutions or products, peptide hydrolysis, waste treatment, textile applications, as fusion-cleavage enzymes in protein production, and the like. Hydrolases of the invention may provide enhanced performance in a detergent composition as compared to another detergent protease, that is, the enzyme group may increase cleaning of certain enzyme sensitive stains such as grass or blood, as determined by usual evaluation after a standard wash cycle. Hydrolases of the invention can be formulated into known powdered and liquid detergents having pH between 6.5 and 12.0 at levels of about 0.01 to about 5% (for example, about 0.1% to 0.5%) by weight. These detergent cleaning compositions can also include other enzymes such as other known esterases, phospholipases, proteases, amylases, cellulases, lipases or endoglycosidases, as well as builders and stabilizers.

*Processes for coating and finishing fabrics*

The methods and compositions (enzymes of the invention, e.g., esterases, acylases, lipases, phospholipases or proteases of the invention) of the invention can be used in processes for coating and finishing fabrics, fibers or yarns. In one aspect, insoluble cellulosic polymers are reacted with carboxylic acids or esters thereof in the presence of a hydrolase of the invention. The cellulosic polymer may be cotton, viscose, rayon, lyocell, flax, linen, ramie, and all blends thereof; and blends thereof with polyesters, wool, polyamides, acrylics and polyacrylics. In alternative aspects, the methods and compositions (hydrolases) of the invention can be used in a softening finish process, i.e. improvement of the hand and drape of the final fabric, for dyeing a polymeric material, for obtaining flame retardancy, for obtaining water repellency, for obtaining brightness, e.g, optical brightness, of a polymeric material, and for obtaining resin finishing ("permanent press"). In one aspect, the methods and compositions (hydrolases) of the invention can be used for obtaining flame retardancy in a fabric using, e.g., a halogen-substituted carboxylic acid or an ester thereof, i.e. a fluorinated, chlorinated or bromated carboxylic acid or an ester thereof. In one aspect, the processes are carried out under conditions (e.g. temperature, pH, solvent) that favors the esterification process over hydrolytic cleavage of an ester bend. In one aspect, the esterification process is carried out using water as a solvent, or, the process may be carried out without a solvent, or, the reaction may take place in a microemulsion formed by adding an carboxylic acid or an ester thereof to a mixture of water and a suitable surfactant. See, e.g., U.S. Patent No. 5,733,750.

In one aspect, a hydrolase of the invention is absorbed or adsorbed or otherwise immobilized on a surface, such as a fabric, fiber or yam. In one aspect, a fabric-hydrolase complex is formed for, e.g., lipid removal, e.g., food or oil stain removal. The hydrolase may be sorbed on the fabric, fiber or yarn before or after staining. The active hydrolase can hydrolyze the stain on dry fabric, fiber or yarn, or fabric, fiber or yarn in laundering solutions. In one aspect, a hydrolase of the invention has enhanced stability to denaturation by surfactants and to heat deactivation. In the way, the hydrolase can be resistant to removal from the fabric, fiber or yarn during laundering, can retain substantial activity after drying at an elevated temperature, and can retain activity during fabric storage or wear. Redeposition of food, oil and oil hydrolysis by-products during laundering of fabric also can be retarded by a hydrolase of the invention. Oil hydrolysis by-products can be removed during laundering of the fabric, e.g., at a basic pH or in the presence of a surfactant. In one aspect, a hydrolase of the invention is absorbed or adsorbed or otherwise immobilized on a gel, glass, plastic or metal solid as well as a fabric, fiber or yam.

In alternative aspects, hydrolases of the invention are useful to treat a wide variety of natural, synthetic or metallic fabrics, including textiles or woven or non-woven cloths, including nylon, polycotton, polyester, woven polyester, double knit polyester, silk, vinyl, cotton flannel, rayon velvet, acrylic felt, wool blend (polyester/wool), synthetic blend (polyester/polyurethane), latexes. Other surfaces that can be treated with a hydrolase of the invention include kitchen or cooking devices and utensils, e.g., pot cleaner materials such as cellulose sponge, nylon and stainless steel scrubbers and copper cloth, dishwashers, food storage devices, e.g., refrigerators, freezers and the like.

The surfaces that have been treated in accordance with the invention can already be stained by (or carrying) oil before an enzyme-fabric complex is formed or the complex can be formed before such exposure. Examples of embodiments useful for the former applications include pre-wash liquid or gelled compositions that can be sprayed or directly applied to specific areas of oily stains. The garments or linens can then be stored in a laundry hamper, for example, and laundered in the normal course of a household's routine because degradation of the oily stain into hydrolysis by-products will be occurring during storage. Alternatively, fabric may be pretreated before use to convey improved oil stain removal properties. In one aspect, a hydrolase is immobilized on surfaces to facilitate oil removal from the surfaces and to alter wettability of the surfaces. In one aspect, a hydrolase is adsorbed on a fabric before or after an oil stain, and the hydrolase is active to hydrolyze an oil stain on dry fabric or fabric in laundering solutions. In one aspect, the sorbed hydrolase has enhanced stability to denaturation by surfactants and to heat deactivation, is resistant to removal from fabric during laundering, retains substantial activity after drying fabric at an elevated temperature, and/or retains activity during fabric storage or wear. In one aspect, redeposition of oil and oil hydrolysis by-products during laundering of fabric is retarded by the hydrolase. In one aspect, oil hydrolysis by-products are removable during laundering of fabric at a basic pH or in the presence of a surfactant. See, e.g., U.S. Patent No. 6,265,191.

*Treating fibers and textiles*

The invention provides methods of treating fibers and fabrics using one or more hydrolases of the invention. The hydrolases can be used in any fiber- or fabric-treating method, which are well known in the art, see, e.g., U.S. Patent No. 6,261,828; 6,077,316; 6,024,766; 6,021,536; 6,017,751; 5,980,581; US Patent Publication No. 20020142438 A1. For example, hydrolases of the invention can be used in fiber and/or fabric desizing. In one aspect, the feel and appearance of a fabric is improved by a method comprising contacting the fabric with a hydrolase of the invention in a solution. In one aspect, the fabric is treated with the solution under pressure. For example, hydrolases of the invention can be used in the removal of stains.

In one aspect, hydrolases of the invention are applied during or after the weaving of textiles, or during the desizing stage, or one or more additional fabric processing steps. During the weaving of textiles, the threads are exposed to considerable mechanical strain. Prior to weaving on mechanical looms, warp yarns are often coated with sizing starch or starch derivatives in order to increase their tensile strength and to prevent breaking. The hydrolases of the invention can be applied to remove these sizing starch or starch derivatives. After the textiles have been woven, a fabric can proceed to a desizing stage. This can be followed by one or more additional fabric processing steps. Desizing is the act of removing "size" from textiles. After weaving, the size coating must be removed before further processing the fabric in order to ensure a homogeneous and wash-proof result. The invention provides a method of desizing comprising enzymatic treatment of the "size" by the action of hydrolases of the invention.

The enzymes of the invention can be used to desize fabrics, including cotton-containing fabrics, as detergent additives, e.g., in aqueous compositions. The invention provides methods for producing a stonewashed look on indigo-dyed denim fabric and garments. For the manufacture of clothes, the fabric can be cut and sewn into clothes or garments. These can be finished before or after the treatment. In particular, for the manufacture of denim jeans, different enzymatic finishing methods have been developed. The finishing of denim garment normally is initiated with an enzymatic desizing step, during which garments are subjected to the action of amylolytic enzymes in order to provide softness to the fabric and make the cotton more accessible to the subsequent enzymatic finishing steps. The invention provides methods of finishing denim garments (e.g., a "bio-stoning process"), enzymatic desizing and providing softness to fabrics using the hydrolases of the invention. The invention provides methods for quickly softening denim garments in a desizing and/or finishing process.

Other enzymes can be also be used in these desizing processes. For example, an alkaline and thermostable amylase and hydrolase can be combined in a single bath for desizing and bioscouring. Among advantages of combining desizing and scouring in one step are cost reduction and lower environmental impact due to savings in energy and water usage and lower waste production. Exemplary application conditions for desizing and bioscouring are about pH 8.5 to 10.0 and temperatures of about 40°C and up. Using a hydrolase of the invention, low enzyme dosages, e.g., about 100 grams (g) per a ton of cotton, and short reaction times, e.g., about 15 minutes, can be used to obtain efficient desizing and scouring with out added calcium.

In one aspect, an alkaline and thermostable amylase and hydrolase are combined in a single bath desizing and bioscouring. Among advantages of combining desizing and scouring in one step are cost reduction and lower environmental impact due to savings in energy and water usage and lower waste production. Application conditions for desizing and bioscouring can be between about pH 8.5 to pH 10.0 and temperatures at about 40°C and up. Low enzyme dosages (e.g., about 100 g per a ton of cotton) and short reaction times (e.g., about 15 minutes) can be used to obtain efficient desizing and scouring with out added calcium.

The hydrolases of the invention can be used in combination with other carbohydrate degrading enzymes, e.g., cellulase, arabinanase, xyloglucanase, pectinase, and the like, for the preparation of fibers or for cleaning of fibers. These can be used in combination with detergents. In one aspect, hydrolases of the invention can be used in treatments to prevent the graying of a textile.

The hydrolases of the invention can be used to treat any cellulosic material, including fibers (e.g., fibers from cotton, hemp, flax or linen), sewn and unsewn fabrics, e.g., knits, wovens, denims, yarns, and toweling, made from cotton, cotton blends or natural or manmade cellulosics (e.g. originating from xylan-containing cellulose fibers such as from wood pulp) or blends thereof. Examples of blends are blends of cotton or rayon/viscose with one or more companion material such as wool, synthetic fibers (e.g. polyamide fibers, acrylic fibers, polyester fibers, polyvinyl alcohol fibers, polyvinyl chloride fibers, polyvinylidene chloride fibers, polyurethane fibers, polyurea fibers, aramid fibers), and cellulose-containing fibers (e.g. rayon/viscose, ramie, hemp, flax/linen, jute, cellulose acetate fibers, lyocell).

The textile treating processes of the invention (using hydrolases of the invention) can be used in conjunction with other textile treatments, e.g., scouring and bleaching. Scouring is the removal of non-cellulosic material from the cotton fiber, e.g., the cuticle (mainly consisting of waxes) and primary cell wall (mainly consisting of pectin, protein and xyloglucan). A proper wax removal is necessary for obtaining a high wettability. This is needed for dyeing. Removal of the primary cell walls by the processes of the invention improves wax removal and ensures a more even dyeing. Treating textiles with the processes of the invention can improve whiteness in the bleaching process. The main chemical used in scouring is sodium, hydroxide in high concentrations and at high temperatures. Bleaching comprises oxidizing the textile. Bleaching typically involves use of hydrogen peroxide as the oxidizing agent in order to obtain either a fully bleached (white) fabric or to ensure a clean shade of the dye.

The invention also provides alkaline hydrolases (hydrolases active under alkaline conditions). These have wide-ranging applications in textile processing, degumming of plant fibers (e.g., plant bast fibers), treatment of pectic wastewaters, paper-making, and coffee and tea fermentations. See, e.g., Hoondal (2002) Applied Microbiology and Biotechnology 59:409-418.

*Treating foods and food processing*

The hydrolases of the invention have numerous applications in food processing industry. For example, in one aspect, the hydrolases of the invention are used to improve the extraction of oil from oil-rich plant material, e.g., oil-rich seeds, for example, soybean oil from soybeans, olive oil from olives, rapeseed oil from rapeseed and/or sunflower oil from sunflower seeds or rice bran oil.

The hydrolases of the invention can be used for separation of components of plant cell materials. For example, hydrolases of the invention can be used in the separation of protein-rich material (e.g., plant cells) into components, e.g., sucrose from sugar beet or starch or sugars from potato, pulp or hull fractions. In one aspect, hydrolases of the invention can be used to separate protein-rich or oil-rich crops into valuable protein and oil and hull fractions. The separation process may be performed by use of methods known in the art.

The hydrolases of the invention can be used in the preparation of fruit or vegetable juices, syrups, extracts and the like to increase yield. The hydrolases of the invention can be used in the enzymatic treatment (e.g., hydrolysis of proteins) of various plant cell wall-derived materials or waste materials, e.g. from wine or juice production, or agricultural residues such as vegetable hulls, bean hulls, sugar beet pulp, olive pulp, potato pulp, and the like. The hydrolases of the invention can be used to modify the consistency and appearance of processed fruit or vegetables. The hydrolases of the invention can be used to treat plant material to facilitate processing of plant material, including foods, facilitate purification or extraction of plant components. The hydrolases of the invention can be used to improve feed value, decrease the water binding capacity, improve the degradability in waste water plants and/or improve the conversion of plant material to ensilage, and the like. Syrups of the invention or made by methods of this invention (using polypeptide of this invention) can be an aqueous solution or slurry comprising carbohydrates such as mono-, oligo- or polysaccharides.

*Animal feeds and food or feed additives*

The invention provides methods for treating animal feeds and foods and food or feed additives, including nutritional supplements or additives, using hydrolases of the invention, animals including mammals (e.g., humans), birds, fish and the like. The invention provides animal feeds, foods, and additives comprising hydrolases of the invention. In one aspect, treating animal feeds, foods and additives using hydrolases of the invention can help in the availability of nutrients, e.g., starch, in the animal feed or additive. By breaking down difficult to digest proteins or indirectly or directly unmasking starch (or other nutrients), the hydrolase makes nutrients more accessible to other endogenous or exogenous enzymes. The hydrolase can also simply cause the release of readily digestible and easily absorbed nutrients and sugars.

Hydrolases of the present invention, in the modification of animal feed or a food, can process the food or feed either *in vitro* (by modifying components of the feed or food) or *in vivo.* Hydrolases can be added to animal feed or food compositions containing high amounts of arabinogalactans or galactans, e.g. feed or food containing plant material from soy bean, rape seed, lupin and the like. When added to the feed or food the hydrolase significantly improves the *in vivo* break-down of plant cell wall material, whereby a better utilization of the plant nutrients by the animal (e.g., human) is achieved. In one aspect, the growth rate and/or feed conversion ratio (i.e. the weight of ingested feed relative to weight gain) of the animal is improved. For example a partially or indigestible galactan-comprising protein is fully or partially degraded by a hydrolase of the invention, e.g. in combination with another enzyme, e.g., beta-galactosidase, to peptides and galactose and/or galacto-oligomers. These enzyme digestion products are more digestible by the animal. Thus, hydrolases of the invention can contribute to the available energy of the feed or food. Also, by contributing to the degradation of galactan-comprising proteins, a hydrolase of the invention can improve the digestibility and uptake of carbohydrate and non-carbohydrate feed or food constituents such as protein, fat and minerals.

In another aspect, hydrolase of the invention can be supplied by expressing the enzymes directly in transgenic feed crops (as, e.g., transgenic plants, seeds and the like), such as corn, soy bean, rape seed, lupin and the like. As discussed above, the invention provides transgenic plants, plant parts and plant cells comprising a nucleic acid sequence encoding a polypeptide of the invention. In one aspect, the nucleic acid is expressed such that the hydrolase of the invention is produced in recoverable quantities. The hydrolase can be recovered from any plant or plant part. Alternatively, the plant or plant part containing the recombinant polypeptide can be used as such for improving the quality of a food or feed, e.g., improving nutritional value, palatability, and rheological properties, or to destroy an antinutritive factor.

*Paper or pulp treatment*

The hydrolases of the invention can be in paper or pulp treatment or paper deinking. For example, in one aspect, the invention provides a paper treatment process using hydrolases of the invention. In another aspect, paper components of recycled photocopied paper during chemical and enzymatic deinking processes. In one aspect, hydrolases of the invention can be used in combination with cellulases, pectate lyases or other enzymes. The paper can be treated by the following three processes: 1) disintegration in the presence of hydrolases of the invention, 2) disintegration with a deinking chemical and hydrolases of the invention, and/or 3) disintegration after soaking with hydrolases of the invention. The recycled paper treated with hydrolases can have a higher brightness due to removal of toner particles as compared to the paper treated with just cellulase. While the invention is not limited by any particular mechanism, the effect of hydrolases of the invention may be due to its behavior as surface-active agents in pulp suspension.

The invention provides methods of treating paper and paper pulp using one or more hydrolases of the invention. The hydrolases of the invention can be used in any paper- or pulp-treating method, which are well known in the art, see, e.g., U.S. Patent No. 6,241,849; 6,066,233; 5,582,681. For example, in one aspect, the invention provides a method for deinking and decolorizing a printed paper containing a dye, comprising pulping a printed paper to obtain a pulp slurry, and dislodging an ink from the pulp slurry in the presence of hydrolases of the invention (other enzymes can also be added). In another aspect, the invention provides a method for enhancing the freeness of pulp, e.g., pulp made from secondary fiber, by adding an enzymatic mixture comprising hydrolases of the invention (can also include other enzymes, e.g., pectate lyase, cellulase, amylase or glucoamylase enzymes) to the pulp and treating under conditions to cause a reaction to produce an enzymatically treated pulp. The freeness of the enzymatically treated pulp is increased from the initial freeness of the secondary fiber pulp without a loss in brightness.

*Waste treatment*

The hydrolases of the invention can be used in a variety of other industrial applications, e.g., in waste treatment. For example, in one aspect, the invention provides a solid waste digestion process using hydrolases of the invention. The methods can comprise reducing the mass and volume of substantially untreated solid waste. Solid waste can be treated with an enzymatic digestive process in the presence of an enzymatic solution (including hydrolases of the invention) at a controlled temperature. This results in a reaction without appreciable bacterial fermentation from added microorganisms. The solid waste is converted into a liquefied waste and any residual solid waste. The resulting liquefied waste can be separated from said any residual solidified waste. See e.g., U.S. Patent No. 5,709,796.

In addition, the hydrolases (e.g., proteases) of the invention can be used in the animal rendering industry, to e.g., get rid of feathers, e.g., as described by Yamamura (2002) Biochem. Biophys. Res. Com. 294:1138-1143. Alkaline proteases of the invention can also be used in the production of proteinaceous fodder from waste feathers or keratin-containing materials, e.g., as described by Gupta (2002) Appl. Microbiol. Biotechnol. 59:15-32.

*Lubricants and hydraulic oils*

The methods and compositions (enzymes of the invention, e.g., esterases, acylases, lipases, phospholipases or hydrolases of the invention) of the invention can be used to prepare lubricants, such as hydraulic oils. Thus, the invention also provides lubricants and hydraulic oils comprising a hydrolase of the invention. The purpose of a lubricant of the invention is to minimize friction and wear of metals. Lubricants can further comprise base fluids and additives improving the lubricative properties. See, e.g., U.S. Patent No. 5,747,434.

*Interesterification*

In one aspect, the methods and compositions of the present invention can be used to modify the properties of triglyceride mixtures, and, in one aspect, their consistency. In one aspect, an enzyme of the invention can be used in the presence of a catalyst such as sodium metal or sodium methoxide to promote acyl migration between glyceride molecules such that the products consist of glyceride mixtures in which the fatty acyl residues are randomly distributed among the glyceride molecules.
In one aspect, the enzymes of the invention can be used to produce interesterification products in the reaction where hydrolysis of fat is minimized so that lipase-catalyzed interesterification becomes the dominant reaction. These conditions may include, for example, restricting the amount of water in the system.
In one aspect, enzymes of the invention can be used to catalyze interesterification reaction using mixtures of triglycerides and free fatty acids, as described, e.g., in EP 0 093 602 B2. In these cases, free fatty acid can be exchanged with the acyl groups of the triglycerides to produce new triglycerides enriched in the added fatty acid. In one aspect, 1,3-specific lipases of the invention can be used to confine the reaction to the 1- and 3-positions of the glycerides, which allow to obtain a mixture of triglycerides unobtainable by chemical interesterification or reaction with a non-specific lipase. In one aspect, non-specific lipases are used to attain results similar to chemical interesterification.
The ability to produce novel triglyceride mixtures using positionally specific lipases of the invention is useful to the oils and fats industry because some of these mixtures have valuable properties. For example, 1,3-specific lipase-catalyzed interesterification of 1,3-dipalmitoyl-2-monoleine (POP), which is the major triglyceride of the mid-fraction of palm oil, with either stearic acid or tristearin gives products enriched in the valuable 1-palmitoyl-3-stearoyl-2-monoleine (POSt) and 1,3-distearoyl-2-monoleine (StOSt). POSt and StOSt are the important components of cocoa butter. Thus, one aspect of the invention provides an interesterification reaction to produce cocoa butter equivalents from cheap starting materials.
In one aspect, the invention provides a method of production of a hard fat replacer using the 1,3-specific lipases of the invention. In one aspect, a hard fat replacer comprises a mixture of palm mid-fraction and StOSt, POSt or StOSt/POSt of at least 85% purity.

*Oral care products*

The invention provides oral care product comprising hydrolases of the invention. Exemplary oral care products include toothpastes, dental creams, gels or tooth powders, odontics, mouth washes, pre- or post brushing rinse formulations, chewing gums, lozenges, or candy. See, e.g., U.S. Patent No. 6,264,925.

*Brewing and fermenting*

The invention provides methods of brewing (e.g., fermenting) beer comprising hydrolases of the invention. In one exemplary process, starch-containing raw materials are disintegrated and processed to form a malt. A hydrolase of the invention is used at any point in the fermentation process. For example, hydrolases (e.g., proteases) of the invention can be used in the processing of barley malt. The major raw material of beer brewing is barley malt. This can be a three stage process. First, the barley grain can be steeped to increase water content, e.g., to around about 40%. Second, the grain can be germinated by incubation at 15 to 25°C for 3 to 6 days when enzyme synthesis is stimulated under the control of gibberellins. In one aspect, hydrolases of the invention are added at this (or any other) stage of the process. The action of hydrolases results in an increase in fermentable reducing sugars. This can be expressed as the diastatic power, DP, which can rise from around 80 to 190 in 5 days at 12°C. Hydrolases (e.g., proteases) of the invention can be used in any beer or alcoholic beverage producing process, as described, e.g., in U.S. Patent No. 5,762,991; 5,536,650; 5,405,624; 5,021,246; 4,788,066.

*Medical and research applications*

Hydrolases, (e.g., proteases) of the invention can be used for cell isolation from tissue for cellular therapies in the same manner that collagenases. For example, metallo-endoproteinases and other enzymes of the invention that can cleave collagen into smaller peptide fragments, can be used as "liberase enzymes" for tissue dissociation and to improve the health of isolated cells. "Liberase enzymes" can replace traditional collagenase. Proteases of the invention having collagenase I, collagenase II, clostripain and/or neutral protease activity can be used for tissue dissociation. In one aspect, for tissue dissociation, collagenase isoforms of the invention are blended with each other, and, optionally, with a neutral protease. In one aspect, the neutral protease is a neutral protease dispase and/or the neutral protease thermolysin.
Additionally, proteases of the invention can be used as antimicrobial agents, due to their bacteriolytic properties, as described, e.g., in Li, S. et. al. Bacteriolytic Activity and Specificity ofAchromobacter b-Lytic Protease, J. Biochem. 124, 332-339 (1998).

Proteases of the invention can also be used therapeutically to cleave and destroy specific proteins. Potential targets include toxin proteins, such as Anthrax, *Clostridium botulinum*, Ricin, and essential viral or cancer cell proteins.

Proteases of the invention can also be used in disinfectants, as described, e.g., in J. Gen Microbiol (1991) 137 (5): 1145-1153; Science (2001) 249:2170-2172.

Additional medical uses of the proteases of the invention include lipoma removal, wound debraidment and scar prevention (collagenases), debriding chronic dermal ulcers and severely burned areas.

Enzymes of the invention (e.g., esterases, proteases, etc., of the invention) can be used to in sterile enzymatic debriding compositions, e.g., ointments, in one aspect, containing about 250 collagenase units per gram. White petrolatum USP can be a carrier. In one aspect, enzymes (e.g., proteases) of the invention can be used in indications similar to Santyl® Ointment (BTC, Lynbrook, NY). Proteases of the invention can also be used in alginate dressings, antimicrobial barrier dressings, burn dressings, compression bandages, diagnostic tools, gel dressings, hydro-selective dressings, hydrocellular (foam) dressings, hydrocolloid Dressings, I.V dressings, incise drapes, low adherent dressings, odor absorbing dressings, paste bandages, post operative dressings, scar management, skin care, transparent film dressings and/or wound closure. Proteases of the invention can be used in wound cleansing, wound bed preparation, to treat pressure ulcers, leg ulcers, burns, diabetic foot ulcers, scars, IV fixation, surgical wounds and minor wounds.

Additionally, enzymes of the invention can be used in proteomics and lab work in general. For instance, proteases can be used in the same manner as DNA restriction enzymes.

*Biomass conversion and production of clean bio fuels*

The invention provides enzymes of the invention, including the exemplary hydrolase enzymes of the invention, and the polypeptides of the invention having hydrolase activity, as described herein (including mixtures, or "cocktails" of different enzymes) and methods for the conversion of a biomass or any lignocellulosic material (e.g., any composition comprising cellulose, hemicellulose and lignin), to fuels (e.g., bioethanol, biodiesel), in addition to feeds, foods and chemicals. Thus, the compositions and methods of the invention provide effective and sustainable alternatives or adjuncts to use of petroleum-based products, e.g., as a mixture of bioethanol and gasoline. The invention provides organisms expressing enzymes of the invention for participation in chemical cycles involving natural biomass conversion. In one aspect, enzymes and methods for the conversion are used in enzyme ensembles for the efficient depolymerization of cellulosic and hemicellulosic polymers to metabolizeable carbon moieties. The invention provides methods for discovering and implementing the most effective of enzymes to enable these important new "biomass conversion" and alternative energy industrial processes.

The methods of the invention also include taking the converted lignocellulosic material (processed by enzymes of the invention) and making it into a fuel (e.g. a bioethanol, a biodiesel) by fermentation and/or by chemical synthesis. In one aspect, the produced sugars are fermented and/or the non-fermentable products are gasified.

The enzymes of the invention (including, for example, organisms, such as microorganisms, e.g., fungi, yeast or bacteria, making and in some aspects secreting recombinant enzymes of the invention) can be used in or included/integrated at any stage of any biomass conversion process, e.g., at any one step, several steps, or included in all of the steps, or all of the following methods of biomass conversion processes, or all of these biofuel alternatives:

☐ Direct combustion: the burning of material by direct heat and is the simplest biomass technology; can be very economical if a biomass source is nearby.

☐ Pyrolysis: is the thermal degradation of biomass by heat in the absence of oxygen. In one aspect, biomass is heated to a temperature between about 800 and 1400 degrees Fahrenheit, but no oxygen is introduced to support combustion resulting in the creation of gas, fuel oil and charcoal.

☐ Gasification: biomass can be used to produce methane through heating or anaerobic digestion. Syngas, a mixture of carbon monoxide and hydrogen, can be derived from biomass.

☐ Landfill Gas: is generated by the decay (anaerobic digestion) of buried garbage in landfills. When the organic waste decomposes, it generates gas consisting of approximately 50% methane, the major component of natural gas.

☐ Anaerobic digestion: converts organic matter to a mixture of methane, the major component of natural gas, and carbon dioxide. In one aspect, biomass such as waterwaste (sewage), manure, or food processing waste, is mixed with water and fed into a digester tank without air.

☐ Fermentation

☐ Alcohol Fermentation: fuel alcohol is produced by converting starch to sugar, fermenting the sugar to alcohol, then separating the alcohol water mixture by distillation. Feedstocks such as wheat, barley, potatoes, and waste paper, sawdust, and straw containing sugar, starch, or cellulose can be converted to alcohol by fermentation with yeast.

■ Transesterification: An exemplary reaction for converting oil to biodiesel is called transesterification. The transesterification process reacts an alcohol (like methanol) with the triglyceride oils contained in vegetable oils, animal fats, or recycled greases, forming fatty acid alkyl esters (biodiesel) and glycerin. The reaction requires heat and a strong base catalyst, such as sodium hydroxide or potassium hydroxide.

☐ Biodiesel: Biodiesel is a mixture of fatty acid alkyl esters made from vegetable oils, animal fats or recycled greases. Biodiesel can be used as a fuel for vehicles in its pure form, but it is usually used as a petroleum diesel additive to reduce levels of particulates, carbon monoxide, hydrocarbons and air toxics from diesel-powered vehicles.

☐ Hydrolysis: includes hydrolysis of a compound, e.g., a biomass, such as a lignocellulosic material, catalyzed using an enzyme of the instant invention.

☐ Congeneration: is the simultaneous production of more than one form of energy using a single fuel and facility. In one aspect, biomass cogeneration has more potential growth than biomass generation alone because cogeneration produces both heat and electricity.

In one aspect, the polypeptides of the invention have a polysaccharide hydrolyzing enzymatic activity, for example, a lignocellulosic or cellulolytic activity, including e.g., polypeptides of the invention having hydrolase activity, such as esterases, endoglucanases, cellobiohydrolases and/or β-glucosidases (beta-glucosidases), or other enzymatic activity for generating biodiesel or bioethanol from an organic material, e.g., a biomass, such as compositions derived from plants and animals, including any agricultural crop or other renewable feedstock, an agricultural residue or an animal waste, or the organic components of municipal and industrial wastes or byproducts, or microorganisms such as algae or yeast.

In one aspect, polypeptides of the invention are used in processes for converting lignocellulosic biomass to ethanol, or otherwise are used in processes for hydrolyzing or digesting biomaterials such that they can be used as a biofuel (including biodiesel or bioethanol), or for making it easier for the biomass to be processed into a fuel. In an alternative aspect, polypeptides of the invention are used in processes for a transesterification process reacting an alcohol (like methanol) with a triglyceride oil contained in a vegetable oil, animal fat or recycled greases, forming fatty acid alkyl esters (biodiesel) and glycerin. In one aspect, biodiesel is made from soybean oil or recycled cooking oils. Animal's fats, other vegetable oils, and other recycled oils can also be used to produce biodiesel, depending on their costs and availability. In another aspect, blends of all kinds of fats and oils are used to produce a biodiesel fuel of the invention.

Enzymes of the invention can also be used in glycerin refining. The glycerin byproduct contains unreacted catalyst and soaps that are neutralized with an acid. Water and alcohol are removed to produce 50% to 80% crude glycerin. The remaining contaminants include unreacted fats and oils, which can be processes using the polypeptides of the invention. In a large biodiesel plants of the invention, the glycerin can be further purified, e.g., to 99% or higher purity, for the pharmaceutical and cosmetic industries.

Both bioethanol and biodiesel made using the polypeptides of the invention can be used with fuel oxygenates to improve combustion characteristics. Adding oxygen results in more complete combustion, which reduces carbon monoxide emissions. This is another environmental benefit of replacing petroleum fuels with biofuels (e.g., a fuel of the invention). A bioethanol made using the compositions and/or methods of this invention can be blended with gasoline to form an E10 blend (about 5% to 10% ethanol and about 90% to 95% gasoline), but it can be used in higher concentrations such as E85 or in its pure form. A bioethanol made using the compositions and/or methods of this invention can be blended with petroleum diesel to form a B20 blend (20% biodiesel and 80% petroleum diesel), although other blend levels can be used up to B100 (pure biodiesel).

The invention also provides processes for making ethanol ("bioethanol") from compositions comprising a biomass, e.g., a lignocellulosic biomass, including industrial processing or waste products or byproducts. The lignocellulose biomass material can be obtained from agricultural crops, as a byproduct of food or feed production, or as lignocellulosic waste products, such as plant residues and waste paper. Examples of suitable plant sources or plant residues for treatment with polypeptides of the invention include kelp, algae, grains, seeds, stems, leaves, hulls, husks, corn cobs, corn stover, straw, grasses (e.g., Indian grass, such as *Sorghastrum nutans*; or, switch grass, e.g., *Panicum* species, such as *Panicum virgatum*), and the like, as well as wood, wood chips, wood pulp, and sawdust. Examples of paper waste suitable for treatment with polypeptides of the invention include discard photocopy paper, computer printer paper, notebook paper, notepad paper, typewriter paper, and the like, as well as newspapers, magazines, cardboard, and paper-based packaging materials.

In one aspect, the enzymes and methods of the invention can be used in conjunction with more "traditional" means of making ethanol from biomass, e.g., as methods comprising hydrolyzing lignocellulosic materials by subjecting dried lignocellulosic material in a reactor to a catalyst comprised of a dilute solution of a strong acid and a metal salt; this can lower the activation energy, or the temperature, of cellulose hydrolysis to obtain higher sugar yields; see, e.g., U.S. Patent Nos. 6,660,506; 6,423,145.

Another exemplary method that incorporated use of enzymes of the invention comprises hydrolyzing lignocellulosic material containing hemicellulose, cellulose and lignin by subjecting the material to a first stage hydrolysis step in an aqueous medium at a temperature and a pressure chosen to effect primarily depolymerization of hemicellulose without major depolymerization of cellulose to glucose. This step results in a slurry in which the liquid aqueous phase contains dissolved monosaccharides resulting from depolymerization of hemicellulose and a solid phase containing cellulose and lignin. A second stage hydrolysis step can comprise conditions such that at least a major portion of the cellulose is depolymerized, such step resulting in a liquid aqueous phase containing dissolved/ soluble depolymerization products of cellulose. See, e.g., U.S. Patent No. 5,536,325. Enzymes of the invention can be added at any stage of this exemplary process.

Another exemplary method that incorporated use of enzymes of the invention comprises processing a lignocellulose-containing biomass material by one or more stages of dilute acid hydrolysis with about 0.4% to 2% strong acid; and treating an unreacted solid lignocellulosic component of the acid hydrolyzed biomass material by alkaline delignification to produce precursors for biodegradable thermoplastics and derivatives. See, e.g., U.S. Patent No. 6,409,841. Enzymes of the invention can be added at any stage of this exemplary process.

Another exemplary method that incorporates use of enzymes of the invention comprises prehydrolyzing lignocellulosic material in a prehydrolysis reactor; adding an acidic liquid to the solid lignocellulosic material to make a mixture; heating the mixture to reaction temperature; maintaining reaction temperature for time sufficient to fractionate the lignocellulosic material into a solubilized portion containing at least about 20% of the lignin from the lignocellulosic material and a solid fraction containing cellulose; removing a solubilized portion from the solid fraction while at or near reaction temperature wherein the cellulose in the solid fraction is rendered more amenable to enzymatic digestion; and recovering a solubilized portion. See, e.g., U.S. Patent No. 5,705,369. Enzymes of the invention can be added at any stage of this exemplary process.

The invention provides methods for making motor fuel compositions (e.g., for spark ignition motors) based on liquid hydrocarbons blended with a fuel grade alcohol made by using an enzyme or a method of the invention. In one aspect, the fuels made by use of an enzyme of the invention comprise, e.g., coal gas liquid- or natural gas liquid-ethanol blends. In one aspect, a co-solvent is biomass-derived 2-methyltetrahydrofuran (MTHF). See, e.g., U.S. Patent No. 6,712,866.

In one aspect, methods of the invention for the enzymatic degradation of lignocellulose, e.g., for production of ethanol from lignocellulosic material, can also comprise use of ultrasonic treatment of the biomass material; see, e.g., U.S. Patent No. 6,333,181.

In another aspect, methods of the invention for producing bioethanol from a cellulosic substrate comprise providing a reaction mixture in the form of a slurry comprising cellulosic substrate, an enzyme of this invention and a fermentation agent (e.g., within a reaction vessel, such as a semi-continuously solids-fed bioreactor), and the reaction mixture is reacted under conditions sufficient to initiate and maintain a fermentation reaction (as described, e.g., in U.S. Pat. App. No. 20060014260). In one aspect, experiment or theoretical calculations can determine an optimum feeding frequency. In one aspect, additional quantities of the cellulosic substrate and the enzyme are provided into the reaction vessel at an interval(s) according to the optimized feeding frequency.

One exemplary process for making a biofuels and biodiesels of the invention is described in U.S. Pat. App. Pub. Nos. 20050069998; 20020164730; and in one aspect comprises stages of grinding the lignocellulosic biomass (e.g., to a size of 15-30 mm), subjecting the product obtained to steam explosion pre-treatment (e.g., at a temperature of 190-230°C) for between 1 and 10 minutes in a reactor; collecting the pre-treated material in a cyclone or related product of manufacture; and separating the liquid and solid fractions by filtration in a filter press, introducing the solid fraction in a fermentation deposit and adding one or more enzymes of the invention, e.g., a hydrolase of this invention, including esterases, beta-glucosidase enzymes (e.g., dissolved in citrate buffer pH 4.8) and the like.

Another exemplary process for making a biofuels and biodiesels of the invention comprising ethanol using enzymes of the invention comprises pretreating a starting material comprising a lignocellulosic feedstock comprising at least hemicellulose and cellulose. In one aspect, the starting material comprises potatoes, soybean (rapeseed), barley, rye, corn, oats, wheat, beets or sugar cane or a component or waste or food or feed production byproduct. The starting material ("feedstock") is reacted at conditions which disrupt the plant's fiber structure to effect at least a partial hydrolysis of the hemicellulose and cellulose. Disruptive conditions can comprise, e.g., subjecting the starting material to an average temperature of 180°C to 270°C at pH 0.5 to 2.5 for a period of about 5 seconds to 60 minutes; or, temperature of 220°C to 270°C, at pH 0.5 to 2.5 for a period of 5 seconds to 120 seconds, or equivalent. This generates a feedstock with increased accessibility to being digested by an enzyme, e.g., a hydrolase of this invention or other enzymes of the invention. U.S. Patent No. 6,090,595.

Exemplary conditions for hydrolysis of lignocellulosic material using an enzyme of this invention include reactions at temperatures between about 30°C and 48°C, and/or a pH between about 4.0 and 6.0. Other exemplary conditions include a temperature between about 30°C and 60°C and a pH between about 4.0 and 8.0.

*Other industrial applications*

The invention also includes a method of increasing the flow of production fluids from a subterranean formation by removing a viscous, protein-containing, damaging fluid formed during production operations and found within the subterranean formation which surrounds a completed well bore comprising allowing production fluids to flow from the well bore; reducing the flow of production fluids from the formation below expected flow rates; formulating an enzyme treatment (comprising an enzyme of the invention) by blending together an aqueous fluid and a polypeptide of the invention; pumping the enzyme treatment to a desired location within the well bore; allowing the enzyme treatment to degrade the viscous, protein-containing, damaging fluid, whereby the fluid can be removed from the subterranean formation to the well surface; and wherein the enzyme treatment is effective to attack protein in cell walls.

Hydrolases of the invention can be used for peptide synthesis, in the leather industry, e.g., for hide processing, e.g., in hair removal and/or bating, for waste management, e.g., removal of hair from drains, in the photography industry, e.g., for silver recovery from film, in the medical industry, e.g., as discussed above, e.g., for treatment of bums, wounds, carbuncles, furuncles and deep abscesses or to dissolve blood clots by dissolving fibrin, for silk degumming.

In other aspects, enzymes of the invention can be used as flavor enhancers in, for example, cheese and pet food, as described, e.g., in Pommer, K., Investigating the impact of enzymes on pet food palatability, Petfood Industry, May 2002, 10-11.

In yet another embodiment of the invention, enzymes of the invention can be used to increase starch yield from corn wet milling, as described, e.g., in Johnston, D.B., and Singh, V. Use of proteases to Reduce Steep Time and $SO_2$ requirements in a corn wet-milling process, Cereal Chem. 78(4):405-411.

In other aspects, enzymes of the invention can be used in biodefense (e.g., destruction of spores or bacteria). Use of enzymes in biodefense applications offer a significant benefit, in that they can be very rapidly developed against any currently unknown biological warfare agents of the future. In addition, proteases of the invention can be used for decontamination of affected environments.

Additionally, enzymes of the invention can be used in biofilm degradation, in biomass conversion to ethanol, and/or in the personal care and cosmetics industry.

Enzymes of the invention can also be used to enhance enantioselectivity, as described, e.g., in Arisawa, A. et. al. Streptomyces Serine Protease (DHP-A) as a New Biocatalyst Capable of Forming Chiral Intermediates of 1,4-Diohydropyridine Calcium Antagonists. Appl Environ Mircrobiol 2002 Jun; 68(6):2716-2725; Haring, D. et. al. Semisynthetic Enzymes in Asymmetric Synthesis:Enantioselective Reduction of Racemic Hydroperoxides Catalyzed by Seleno-Subtilisin. J. Org. Chem. 1999, 64:832-835.

## EXAMPLES

<u>Example 1: Exemplary lipase assays</u>

The following example describes exemplary assays to screen for lipase activity. In one aspect, these exemplary assays can be used as routine screens to determine if a polypeptide is within the scope of the invention. Such assays include use of pH indicator compounds to detect cleavage of fatty acids from triglycerides, spectrophotometric methods, HPLC, GC, MS, TLC and others. Jaeger (1994) FEMS Microbiol. Rev. 15:29-63; Ader (1997) Methods Enzymol. 286: 351-386; Vorderwülbecke (1992) Enzyme Microb. Technol. 14:631-639; Renard (1987) Lipids 22: 539- 541.

In one aspect, the methods of the invention screen for regio-selective lipases, e.g., Sn-1, Sn-3 and/or Sn-3 regio-selective lipases. In one aspect, the substrates 1,3-diamide and 1,3-diether TAG analogues are used to target, or select for, Sn-2 selective lipases. In one aspect, the methods of the invention screen for lipases that exhibit regioselectivity for the 2-position of lipids, e.g., TAGs. Structured synthesis of lipids using Sn-2 selective lipases can be useful for the synthesis of a variety of TAGs, including 1,3-diacylglycerides (1,3-DAGs) and components of cocoa butter.

In one aspect, regio-selective lipases, including Sn-2 selective lipases, are characterized for regioselectivity using rigorous analytical methods. This can eliminate false results due to acyl migration. In one aspect, lipases are tested for Sn2 specificity using analytical methods such as NMR spectroscopy. Also, a structured triacylglyceride of the ABA-type (where A and B denote fatty acids distributed along the glycerol backbone) can be subjected to hydrolysis or alcoholysis using lipases (e.g. of the invention) followed by analysis of the partial glycerides and fatty acid (esters) formed. Alcoholysis conditions at controlled water activity, e.g. using primary alcohols such as methanol or ethanol are preferred as undesired acyl migration can be avoided need different references. Sn-2 selectivity of lipases was reported, however, the extent of sn-2 selectivity was very low (Briand (1995) Eur. J. Biochem. 228: 169-175; Rogalska (1993) Chirality 5, 24-30.

In one aspect, regio-selective lipases are assayed for their regio-specificity (Sn2 versus Sn1/Sn3 versus Sn1,3) on appropriate lipids, such as tripalmitin, tristearin, triolein, tricaprylin, and trilaurin, and also for their fatty acid specificity.

*Screening for Lipase/Esterase Activity*

Colonies are picked with sterile toothpicks and used to singly inoculate each of the wells of 96-well microtiter plates. The wells contained 250 μL of LB media with 100 μg/mL ampicillin, 80 μg/mL methicillin, and 10% v/v glycerol (LB Amp/Meth, glycerol). The cells were grown overnight at 37°C without shaking. This constituted generation of the "Source GenBank." Each well of the Source GenBank thus contained a stock culture of *E. coli* cells, each of which contained a pBluescript with a unique DNA insert.

Plates of the source GenBank were used to multiply inoculate a single plate (the "condensed plate") containing in each well 200 μL of LB Amp/Meth, glycerol. This step was performed using the High Density Replicating Tool (HDRT) of the Beckman Biomek with a 1% bleach, water, isopropanol, air-dry sterilization cycle in between each inoculation. Each well of the condensed plate thus contained 10 to 12 different pBluescript clones from each of the source library plates. The condensed plate was grown for 16 hours at 37° C. and then used to inoculate two white 96-well Polyfiltronics microtiter daughter plates containing in each well 250 μL of LB Amp/Meth (no glycerol). The original condensed plate was put in storage -80°C. The two condensed daughter plates were incubated at 37°C for 18 hours.

The short chain esterase '600 μM substrate stock solution' was prepared as follows: 25 mg of each of the following compounds was dissolved in the appropriate volume of DMSO to yield a 25.2 mM solution. The compounds used were 4-methylumbelliferyl proprionoate, 4-methylumbelliferyl butyrate, and 4-methylumbelliferyl heptanoate. Two hundred fifty microliters of each DMSO solution was added to ca 9 mL of 50 mM, pH 7.5 HEPES buffer which contained 0.6% of Triton X-100 and 0.6 mg per mL of dodecyl maltoside (Anatrace, Maumee, OH). The volume was taken to 10.5 mL with the above HEPES buffer to yield a slightly cloudy suspension.

The long chain '600 μM substrate stock solution' was prepared as follows: 25 mg of each of the following compounds was dissolved in DMSO to 25.2 mM as above. The compounds used were 4-methylumbelliferyl elaidate, 4-methylumbelliferyl palmitate, 4-methylumbelliferyl oleate, and 4-methylumbelliferyl stearate. All required brief warming in a 70° C. bath to achieve dissolution. Two hundred fifty microliters of each DMSO solution was added to the HEPES buffer and diluted to 10.5 mL as above. All seven umbelliferones were obtained from Sigma Chemical Co. (St. Louis, MO).

Fifty μL of the long chain esterase or short chain esterase '600 μM substrate stock solution' was added to each of the wells of a white condensed plate using the Biomek to yield a final concentration of substrate of about 100 μM. The fluorescence values were recorded (excitation=326 nm, emission=450 nm) on a plate-reading fluorometer immediately after addition of the substrate. The plate was incubated at 70°C for 60 minutes in the case of the long chain substrates, and 30 minutes at RT in the case of the short chain substrates. The fluorescence values were recorded again. The initial and final fluorescence values were compared to determine if an active clone was present.

To isolate the individual clone which carried the activity, the Source GenBank plates were thawed and the individual wells used to singly inoculate a new plate containing LB Amp/Meth. As above, the plate was incubated at 37°C to grow the cells, 50 μL of 600 μM substrate stock solution was added using the Biomek and the fluorescence was determined. Once the active well from the source plate was identified, cells from this active well were streaked on agar with LB/Amp/Meth and grown overnight at 37° C. to obtain single colonies. Eight single colonies were picked with a sterile toothpick and used to singly inoculate the wells of a 96-well microtiter plate. The wells contained 250 μL of LB Amp/Meth. The cells were grown overnight at 37°C without shaking. A 200 μL aliquot was removed from each well and assayed with the appropriate long or short chain substrates as above. The most active clone was identified and the remaining 50 μL of culture was used to streak an agar plate with LB/Amp/Meth. Eight single colonies were picked, grown and assayed as above. The most active clone was used to inoculate 3 mL cultures of LB/Amp/Meth, which were grown overnight. The plasmid DNA was isolated from the cultures and utilized for sequencing.

Example 2: Exemplary structured lipid synthesis methods

The following example describes exemplary structured lipid synthesis methods of the invention using lipases of the invention.

In one aspect, the invention provides a "Forced Migration Methodology" for the structured synthesis of lipids using the lipases of the invention. This method provides for the efficient synthesis of a variety of structured lipids, including 1,3-DAGs and components of cocoa butter, as illustrated in Figure 7. In one aspect, the method for producing structured lipids, in this example a structured triacylglyceride (sTAG), comprises three major steps:

1. Regiospecific hydrolysis or alcoholysis (e.g. ethanolysis) of a TAG using a Sn1-specific or Sn3-specific lipases to yield a 2,3 or 1,2-DAG, respectively;
2. Promotion of acyl migration in a purified or unpurified DAG under kinetically-controlled conditions using ion-exchange resins or other method(s), resulting in the structured 1,3-DAG; and
3. Fatty acid-specific lipase catalyzed addition of a fatty acid or a fatty acid derivative, such as a fatty acid ethyl ester or vinyl ester, at the Sn2 position, yielding the sTAG.

This route can provide access to two target groups of lipids, 1,3-DAGs and sTAGs with the same set of enzymes and methodology. The method can use lipases that have Sn1 and/or Sn3 regiospecificity; such enzymes are commercially available (Rhizopus delemar (Amano, Japan) and *Rhizomucor miehei* (Novozymes, Denmark

In one aspect, *Rhizopus* sp. lipases and *Rhizomucor miehei* lipases are used. These lipases are known to exhibit higher specificity for hydrolysis of fatty acids in the Sn1 position compared with the Sn3 position. In one aspect, the methods further comprise use of these *Rhizopus* and *Rhizomucor meihei* lipases to confirm Step 1, of Figure 7, i.e., the regiospecific hydrolysis or alcoholysis (e.g. ethanolysis) of a TAG using an Sn1-specific or Sn3-specific lipase to yield a 2,3 or 1,2-DAG, respectively.

In one aspect, the invention provides a forced migration method supplemented with glycerol. Addition of glycerol to the enzyme reaction prior to the treatment with anion exchange resin (or other migration catalysts) can be a way to increase yields of 1,3 DAGs in forced migration reactions, as illustrated in Figure 27.

In one aspect, the methods further comprise confirmation of Step 2, Figure 7, by treatment of purified or unpurified 1,2-DAG or 2,3-DAG with an ion-exchange resin, for example, an ion-exchange column, under a variety of conditions. Major variables include the nature of the ion-exchange resin, pH, flow rates, buffer type and ionic strengths. Alternative methods of promoting acyl migration can be used. In one aspect, the acyl migration can be performed under non-equilibrium conditions, e.g., such that the end product contains greater ratios of one product over another, for example, such that the end product contains a 2:1 ratio of 1,3-DAG to 2,3-DAG. Substrates for the Step 2 validation studies are available commercially.

In one aspect, acyl migration in 2,3-DAGs (or 1,2-DAGs) is promoted under kinetic conditions such that the final product is a purified 1,3-DAG in greater than about a 70% yield.

In one aspect, Step 3, Figure 7, uses a lipase that is fatty acid specific, but there are no regiospecific requirements given a pure 1,3-DAG as substrate. The lipase from *Geotrichium candidum* exhibits a very high specificity for fatty acids that have Δ9 unsaturation. This enzyme is readily available and can be utilized to confirm Step 3.

Example 3: Exemplary structured synthesis of cocoa butter alternatives (CBAs)

The following example describes exemplary structured lipid synthesis methods of the invention using the lipases of the invention. This example describes the structured synthesis of triacylglycerides (sTAGs) as cocoa butter alternatives (CBAs).

Natural cocoa butter consists mostly of three TAG: 1,3-dipalmitoyl-2-oleoylglycerol (POP), 1,3-distearoyl-2-oleoylglycerol (SOS) and 1-palmitoyl-2-oleoyl-3-stearoylglycerol (POS). The relative proportions of these three TAGs differ somewhat depending upon the source of cocoa butter, but are approximately 21:31:48 (POP:SOS:POS). The methods of the invention provide for the structured synthesis of a cocoa butter alternatives having any proportion of POP:SOS:POS, including the natural 21:31:48 POP:SOS:POS. The methods of the invention provide for the structured synthesis related TAGs, e.g., 1-oleoyl-2,3-dimyristoylglycerol (OMM). The methods of the invention also provide for the selective processing of natural cocoa butter using the lipases of the invention.

In one aspect, both the Sn2 lipase and the methods outlined above (including Example 2) are used in the synthesis of key structured lipids of CBAs. Lipases can be assayed for their regiospecificity (Sn2 versus Sn1/Sn3 versus Sn1,3) on appropriate lipids, such as tripalmitin, tristearin, triolein, tricaprolein, and trilaurein. Lipases also can be assayed for their fatty acid specificity.

Example 4: Exemplary structured synthesis of nutraceuticals

The following example describes exemplary structured lipid synthesis methods of the invention for making nutraceuticals using the lipases of the invention.

In one aspect, 1,3-DAGs are synthesized for use in nutraceuticals. 1,3-DAGs are the products of the first step of the Sn2 lipase synthesis of sTAGs shown in Figure 6 and first two steps of the synthesis of structured lipids as shown in Figure 7 and Figure 8. Routine assaying of lipases for Sn2 versus Sn1 or Sn3 specificity can provide the data to determine the optimum lipases and methodology for different 1,3-DAG and nutraceutical synthesis applications.

In one aspect, poly-unsaturated fatty acids (PUFAs) are made using the methods and lipases of the invention, e.g., using a protocol as set forth in Figure 9, bottom. PUFAs are themselves valuable commodities and can be extracted from PUFA-containing fat sources, such as fish oil, using PUFA-specific lipases of the invention. In one aspect, the invention uses enzymes that can distinguish between esters of different PUFAs, e.g. docosahexaenoic acid (DHA) versus eicosapentaenoic acid (EPA), facilitating the development of highly purified products (S. Wongsakul et al., Eur. J. Lipid Sci. Technol. 105 (2003) 68-73). Lipases can be tested for their specificities on a variety of PUFA esters and glycerol esters.

Example 5: Exemplary structured synthesis of lipids containing poly-unsaturated fatty acids

The following example describes exemplary structured lipid synthesis methods of the invention for making lipids containing poly-unsaturated fatty acids (PUFAs) using the lipases of the invention. These PUFA-containing lipids can be used in foods, feeds, cosmetics, pharmaceuticals and drug delivery agents, nutraceuticals and the like.

In one aspect, fish oil is a starting material, since in fish oil the majority of fatty acids at the 2 position are PUFAs. In one aspect, the methods comprise a 1,3-lipase-catalyzed interesterification of fish oil with medium-chain fatty acid esters to form MLM-type lipids (triacylglycerols (TAG) can be of types MML, MLM, MLL, and LML (M, medium-chain fatty acid; L, long-chain fatty acid, see, e.g., Kurvinen (2001) Lipids 36:1377-1382)).

In one aspect, the invention provides methods for making PUFA-containing sTAGs, as illustrated at the top of Figure 9 (Figure 9A), and 2-PUFA sMAGs and purified PUFAs, as illustrated at the bottom of Figure 9 (Figure 9B). Any appropriate starting oil can be used. In one aspect, if it is more economic to use fish-oil fatty acids instead of purified PUFAs, lipases with specificity for fatty acid B (in Figure 9A, top) and PUFAs can be used. Lipases can be screened for fatty acid specificity on simple esters and glycerol esters. In one aspect, 2-PUFA MAGs are synthesized from fish oil using either a Sn1,3-lipase (see Figure 9B) or a non-regiospecific lipase that does not cleave PUFA esters.

Example 6: Exemplary Growth-Kill assay

The following example describes an exemplary Growth-Kill assay for testing the activity of the lipases of the invention. See, e.g., Chem. Communication (2002) 1428-1429.

The Growth-Kill assay provides a method for *in vivo* selection of enzymes, e.g. lipases, or mutants thereof, with desirable properties. The assay combines two components, a growth component and a kill component. The first of these is a substrate from which the enzyme, e.g. lipase, liberates an element which allows the host organisms to grow, e.g. a carbon source. The second of these is a substrate from which the enzyme, e.g. lipase, liberates an element which prevents the host organism from growing or kills the host organism, e.g. an antibiotic.

The invention provides methods of modifying a nucleic acid encoding a lipase to generate an enzyme with modified properties. A Growth-Kill assay can be used to discriminate between two fatty acids, to determine if a lipase has altered enzyme specificity, to determine if a lipase is within the scope of the invention, and the like. An exemplary Growth-Kill assay is outlined in Figure 11, where R1 is a growth substrate for the screening host and R2 is a substance that is toxic to the cell and kills the host if released from the ester. In one aspect, growth substrates (1-acyl glycerol esters) and kill substrates (3-acyl chloramphenicol esters) are used

Example 7: Protocol for the synthesis of 1,3-diglyceride

The following example describes an exemplary protocol used to practice the compositions (the lipases) and the methods of the invention. These exemplary protocols can also be used to determine if a lipase is within the scope of the invention. In one aspect, an exemplary protocol for the synthesis of 1,3-diglyceride (1,3-DG) and structured lipids using lipases of the invention is described.

In one aspect, glycerol and free fatty acid (FFA) or fatty acid vinyl ester (FAVE) are esterified immobilized glycerol (see, e.g., J. Am. Oil Chem. Soc., 1992, 69:955-960). The immobilized glycerol can be on a silica gel. In one exemplary assay, Lipozyme RM IM™ (an immobilized 1 ,3-specific lipase, Novozymes, Denmark), with MTBE, at room temperature was used. This has the advantage of high yield and purity of 1,3-DG, and fast reaction. However, MTBE is not allowed in food use and there is difficulty in separation of immobilized enzyme and silica gel

In one aspect, the esterification is done using non-immobilized glycerol. In one exemplary assay, esterification of glycerol and fatty acid (FFA) or fatty acid vinyl ester (FAVE) is in a solvent-free/organic solvent with an immobilized lipase from *Candida antarctica* type B (CAL-B), a lipase from *Rhizopus delemar* immobilized on EP 100 (D-EP100), and Lipozyme RM IM™ (Novozymes, Denmark), at 0°C or room temperature (RT). One advantage of this exemplary protocol is the solvent-free condition; it allows ease of separation of immobilized enzyme and with a further purification step, a moderate-high yield.

In one aspect, alcoholysis and hydrolysis of triglycerides (TG) is accomplished using 1,3-regiospecific lipases, D-EP100 and Lipozyme RM IM™ (Novozymes, Denmark ), organic solvents, preferentially at controlled water activity. In this reaction, the substrates (natural oil) are cheap and the reaction provides an acceptable yield. Most DAG formed is 1,2(2,3)-DAG.

In one aspect, an exemplary reaction involved induction of acyl migration of 1,2(2,3)-DAG. Most DAG obtained from alcoholysis and hydrolysis of TAG is 1,2 (2,3)-DaG. It is thus necessary to induce the acyl migration of 1,2(2,3)-DAG to 1,3-DAG. Several factors were studied, including ion-exchangers, acid or base, heat, carrier, water activity. One exemplary reaction involved the esterification between 1,3-DAG and FFA or FAVE using an sn2-specific enzyme or a non-regiospecific lipase but specific to fatty acid change length or specific fatty acid.

*Esterification using immobilized glycerol)*

1,3-DAG was synthesized from glycerol, 1 mmol, immobilized on 4 g silica gel) and vinyl laurate (2 mmol) in 8 ml methyl-tert-butyl ether (MTBE) at room temperature using Lipozyme RM IM™ (Novozymes, Denmark) (10% based on glycerol weight) as catalyst (Matthias et al. 1992???). The reaction was carried out in a 10-ml vial and the reaction mixture was mixed by magnetic stirrer (500 rpm). After 24 hours (h), enzyme was separated from the reaction mixture by filtration to stop the reaction. The filtrate was evaporated under vacuum. 1,3-DAG in oily residue was recovered and purified by crystallization in dry methanol at 4°C followed by filtration. See, e.g., J. Am. Oil Chem. Soc., 1992, 69:955-960).

*Esterification using non-immobilized glycerol*

1,3-DAG was synthesized by esterification of glycerol (1mmol) and FFA or FAVE (2mmol) in a solvent-free condition or in organic solvent at 0°C using CAL-B (10% based on glycerol weight) as catalyst. The reaction was carried out in a 4-ml vial and the reaction mixture was mixed by magnetic stirrer (400 rpm). Activiated molecular sieve was added in a reaction with FFA to remove produced water from the reaction mixture. In some reactions organic solvent (2 ml) was added to the reaction mixture to dissolve a solid FFA. The reaction was stopped by dissolving the reaction mixture in n-hexane (in case of a solvent-free reaction condition) and centrifuged to separate immobilized enzyme from the reaction mixture. The 1,3-DAG was recovered and purified by crystallization at -20°C. If high contents of MAG were present, a recrystallization in dry methanol at -20°C afforded pure 1,3-DAG. Samples (10μl) were periodically withdrawn during the reaction to determine the acylglycerol composition. Samples were pretreated before analysis by adding 0.3 ml Folsh's solution (chloroform:methanol, 2:1 by vol) and 0.3 ml distilled water, mixed for 30 sec, followed by centrifuging (10000 rpm, 2 min). Organic layer was used for analysis by IATROSCAN™ (Shell-usa, Fredericksburg VA).

*Alcoholysis and hydrolysis of TAG*

TAG (3 mmol) was dissolved in organic solvent (2 ml) and pre-equilibrated over a saturated-salt solution at $a_W$ 0.11 for 48 h (only for alcoholysis reaction). Dry ethanol or water (3 mmol) was added and the reaction mixture was incubated at 40°C for 15 min. Immobilized lipase (10% based on TAG weight) was added to start the reaction. The reaction was carried out in a 4-ml screw-capped vial and the reaction mixture was mixed by magnetic stirrer (400 rpm). An aliquot of the reaction mixture was periodically withdrawn and diluted with chloroform to appropriate dilution, followed by analysis with IATROSCAN™ (Shell-usa, Fredericksburg VA) to determine acylglycerol composition. Immobilized lipase was separated from the reaction mixture after 48h by centrifugation to stop the reaction.

*Induction of acyl migration*

Effect of temperature, FFA (oleic acid), carrier (celite) and ion-exchanger on acyl migration of 1,2-dipalmitin (1,2-DP, this also includes the stereoisomer 2,3-DP) were studied. 1,2-DP was dissolved in n-hexane (8 mg/ml). Oleic acid (2-4 mmol) or celite (8 mg) or ion-exchanger (10-100 mg) was added directly to the reaction mixture. All reactions were carried out in a 1.5-ml Eppendorf reaction vial with shaking (1400 rpm) at room temperature (25°C), except when testing effect of temperature, then the reaction was carried out at 40 or 60°C.

*Synthesis ofstructured triglycerides (ST) from 1,3-diglycerides (1,3-DAG) and free fatty acid or fatty acid vinyl este*

Structured triglycerides (ST) was synthesized by esterification of 1,3-DAG and oleic acid (OA) or oleic acid vinyl ester (OAVE) in n-hexane at 60°C using immobilized lipase from *Pseudomonas* sp. (Amano PS-D, Amano Enzyme USA, Elgin, IL) as biocatalyst. 0.1mmol of 1,3-DG (45.7 mg of 1,3-dilaurin or 34.4mg of 1,3-dicaprylin) and 0.2mmol of OA (28.2mg) or OAVE (60.2mg) was dissolved in 1 ml n-hexane in a 2-ml screw-capped vial. Activated molecular sieve was added when OA was used as acyl donor. The reaction was started by addition of PS-D (10% weight of 1,3-DG). The vials were shaken at 1400 rpm at 60°C. An aliquot of reaction mixtures was withdrawn for analysis with IATROSCAN™ (Shell-usa, Fredericksburg VA). ST thus obtained was purified on TLC plate and the TAG band was scrapped of and methylated followed by GC analysis.

Acylglycerol composition was determined by IATROSCAN™ (Shell-usa, Fredericksburg VA) analysis (TLC-FID). Total fatty acid composition of ABA-ST was determined by GC analysis of corresponding methylesters. Purity of 1,3-DAG was confirmed by $^1$H-NMR spectroscopy.

*Determination of fatty acid composition by GC analysis*

10 mg of 1,3-DG was methylated with 0.5 % NaOH in methanol (500 μl) and then incubated for 10 min at 60°C. The methylesters were extracted with n-hexane (400 μl) for 1 min. The n-hexane layer was washed with 200 μl distilled water and dried over anhydrous sodium sulfate. Analysis was carried out with a Hewlett-Packard 5890 (series II) gas chromatograph (GS) (Hewlett-Packard, USA) on a FFAP column (Permabond FFAP-DF-0.25, 25 m x 0.25 mm i.d., Macherey-Nagel GmbH, Düren, Germany). Hydrogen was used as the carrier gas. The temperature program used was 150°C (4°C/min, 0.50 min), 170°C (5°C/min), 195°C (10°C/min) and 215°C (9.50 min). Injector and detector temperatures were 250°C. Response factors were determined using a standard mixture of fatty acid methylesters

*Determination ofglyceride composition by TLC/FID analysis*

Changes in glyceride composition during reaction were quantitatively determined using Iatroscan analytical method. Before analysis, a blank of the chromarod was scanned. After treating chromarod with boric acid (3%) and drying for 5 min, 1 μl of the reaction medium (diluted in chloroform at appropriate dilution) is spotted onto the chromarod and the spotted sample was developed for 10 cm in a mixture of benzene:chloroform:acetic acid (50:30:0.5, by vol). After drying, the chromarod in an oven at 110°C for 5 min, scanning is performed at a hydrogen flow rate of 160 ml/min and an air flow rate of 2.0 l/min to produce a chromatogram.

*HPLCseparation of triacylglycerols*

The composition of the triacylglycerols formed during the enzymatic esterification was characterized by HPLC using a nucleosil $C_{18}$ column, (5μm, 250 x 4 mm, Sykam, Gilching, Germany) and an evaporative light scattering detector (ELSD) (Polymer labs) at a flow rate of 1.5 ml/min. The purpose of ELSD is to complement ultraviolet (UV) detection of solutes, and to detect solutes, which do not absorb UV light such as medium-chain triglycerides. The principle of ELSD applies to all solutes having a lower volatility than the mobile phase. Elution was performed using a gradient elution system of acetonitrile and dichloromethane (70% to 55% acetonitrile over 10 min, followed by 55% to 70% acetonitrile over 8 minute).

*Regiospecific analysis of triglycerides*

The regiospecific analysis of oil was conducted by Grignard degradation with allylmagnesium bromide followed by gas chromatograph (GS) analysis. 20 mg of TG was dissolved in dry diethyl ether (2 ml). 800 μl of allyl magnesium bromide solution (1M) was added, and the mixture was shaken for 30 second, then 300 μl glacial acetic acid was added, followed by 5 ml of 0.4-M boric acid to stop the reaction. A mixture of deacylated products was extracted with diethyl ether. This extract was washed with 5 ml solution of aqueous boric acid (0.4 M)/aqueous $NaHCO_3$ (2%), 50:50 (vol/vol). The ether layer was directly subjected to TLC plate, which was impregnated with boric acid, to isolate each fraction of deacylated products. The plate was developed with a chloroform/acetone/acetic acid solution (85:15:1, by vol) as developing system. The 1-MG bands were scraped off and methylated to determine their fatty acid composition using the same method described above. The molar percentage of fatty acid composition at sn 1(3)- and sn 2-positions of the produced TG were calculated. Equation for % FA in sn2-position is shown below:

$$[\%\ FA_{sn2\text{-}position}] = 3\ [\%\ FA_{TG}] - 2\ [\%\ FA_{1\text{-}MG}]$$

where [% $FA_{1\text{-}MG}$] and [% $FA_{TG}$] indicated for each fatty acid, its percentage found in 1-monoglyceride and in triglycerides, respectively.

*Dicaprylin (1,3-DCy)*

1,3DCy was purified by crystallization from n-hexane at -20°C several times. When high amount of MG was presented, the second crystallization in dry methanol has to be done to obtain 1,3-DCy in high purity (>98%). Highest yield of 1,3-DCy (93%) obtained from esterification between vinyl caprylate (CyVE) and glycerol at 0°C in a solvent-free condition catalyzed by CAL-B. The yield thus obtained was higher than the yield obtained (75%)in literature (see, e.g., J. Am. Oil Chem. Soc., 1992, 69:955-960), see Figure 12. Figure 12 illustrates data of various esterification reactions in the synthesis of 1,3-DCy. Method 1 is the esterification of glycerol and caprylic acid in a solvent-free condition at 0°C catalyzed by CAL-B. Method 2 is the esterification of glycerol and caprylic acid vinyl ester in a solvent-free condition at 0°C catalyzed by CAL-B. Method 3 see, e.g., J. Am. Oil Chem. Soc., 1992, 69:955-960) is the esterification of glycerol immobilized on silica gel and caprylic acid vinyl ester in MTBE at room temperature catalyzed by Lipozyme RM IM™ (Novozymes, Denmark). DG = 1,3-dicaprylin, MG = 1-monocaprylin.

Esterification between caprylic acid (Cy) and glycerol gave moderate yield (55%) at lower reaction rates and high amount of MAG was produced during the reaction. The yield could be increased up to 65% by increasing the reaction temperature from 0°C to room temperature. CAL-B gave higher yield and less MAG and allowed faster reaction rate than Lipozyme RM IM™ (Novozymes, Denmark) in esterification of glycerol and Cy or CyVE, both in organic solvent and a solvent-free condition.

Studies on effect of ratio of glycerol:caprylic acid on esterification reaction showed that initial reaction rate decreased and the yield of 1,3-DCy slightly increased with increasing ratio from 1:2 to 1:6, as illustrated in (Figure 13). Figure 13 summarizes data showing the effect of substrate ratio on esterification between glycerol and caprylic acid in n-hexane at 0°C catalyzed by CAL-B (DG = 1,3-dicaprylin, MG = 1-monocaprylin).

*1,3-Dilaurin (1,3-DLa)*

1,3DLa was easily purified and recovered by crystallization in dry methanol at room temperature (RT) or in hexane at -20°C (purity >98%). When lauric acid (La) was used as an acyl-donor, solvent was added to dissolve the FFA. Though the method as described in J. Am. Oil Chem. Soc., 1992, 69:955-960,allowed faster reaction rate with high yield of 1,3-DLa (65%), the highest yield (78%) of 1,3-DLa obtained from esterification between glycerol and lauric acid vinyl ester (LaVE) at 0°C in a solvent-free condition catalyzed by CAL-B after 24h, as illustrated in Figure 14. Figure 14 summarizes data of various synthesis of 1,3-dilaurin. Method1 = esterification of glycerol and lauric acid in n-hexane at 0°C catalyzed by CAL-B; Method2 = esterification of glycerol and lauric acid vinyl ester in a solvent-free condition at 0°C catalyzed by CAL-B; Method3 (Schneider's method) = esterification of glycerol (immobilized on silica gel) and lauric acid vinyl ester in MTBE at room temperature catalyzed by Lipozyme RM IM™ (Eurzyme, Dublin, Ireland). (DG = 1,3-dilaurin, MG = 1-monolaurin). LaVE, as acyl donor, allowed higher yield and faster reaction with less amount of MG than La.

CAL-B gave highest yield and fastest reaction rate of esterification of LaVE and glycerol at 0°C, while Lipozyme RM IM™ gave moderate yield of 1,3-DLa with high amount ofMG and Lipozyme TL™ showed very low activity in the same reaction condition. When increasing reaction temperature to 25°C, Lipozyme RM IM™ showed higher activity, while CAL-B was less active. In the esterification reaction between glycerol and lauric acid in n-hexane catalyzed by CAL-B, reaction rate and the yield of 1,3-DLa decreased with increasing amount of La, as shown in Figure 15. Figure 15 summarizes the effect of substrate ration o esterification of glycerol and lauric acid in n-hexane at room temperature catalyzed by CAL-B (DG = 1,3-dilaurin, MG = 1-monolaurin).

*1,3-Dipalmitin (1,3-DP) and 1,3-distearin (1,3-DS)*

Reactions were carried out in organic solvent and at higher temperature (25°C to 40°C) due to a low solubility of palmitic acid (PA) and stearic acid (SA). The DG yield was lower than the yield of the reaction with La or Cy. Highest yield (80%) and fastest reaction rate was obtained from esterification of glycerol and palmitic acid vinyl ester (PAVE) in MTBE at 40°C catalyzed by D-EP100. Reaction reached the equilibrium within 6-8 h with low amount of MG. Esterification of PA and immobilized glycerol gave higher yield and faster reaction than esterification with free glycerol, as shown in Figure 16. Figure 16 summarizes the synthesis of 1,3-dipalmitin. Method1 = esterification of glycerol and palmitic acid in MTBE at 40°C catalyzed by D-EP100; Method2 = esterification of glycerol and palmitic acid vinyl ester in MTBE at 40°C catalyzed by D-EP 100; Method 3 (Schneider's method) = esterification of glycerol (immobilized on silica gel) and palmitic acid vinyl ester in MTBE at room temperature catalyzed by Lipozyme RM IM™. (DG = 1,3-dipalmitin, MG = 1-monopalmitin).

D-EP100 gave higher yield and activity than Lipozyme RM IM™ in all cases of 1,3-DP synthesis. Moreover, Lipozyme RM IM™ showed no activity in esterification of PA or PAVE when the reaction was performed in MTBE and low activity in n-hexane. D-EP100 preferred the esterification of PA than SA, while not much different activity on PA and SA was observed with Lipozyme RM IM™, as illustrated in Figure 17. Figure 17 summarizes data for the esterification of glycerol and palmitic (C16:O) or stearic (C18:0) acid in n-hexane at 40°C (RM = Lipozyme RM IM™, DEP = D-EP 100, DG = 1,3-diglycerides, MG = 1-monoglycerides).

*Alcoholysis of triglycerides*

Alcoholysis of pure triglycerides (TGs), including trilaurin, tripalmitin and tristearin, was carried out. Most diglyceride (DG) obtained from alcoholysis reaction were 1,2-DG. A high amount of unreacted TG remained in the reaction mixture. Acyl migration was observed during the reaction, especially in hydrolysis reaction, as illustrated in Figure 18. Figure 18 shows data from alcoholysis reaction showing the 1,3-DS/1,2-DS ratio during alcoholysis and hydrolysis of tristearin. DEP = D-EP100, RM = Lipozyme RM IM, Hx = n-hexane, HYD = hydrolysis, ALC = alcoholysis.

The reaction catalyzed by Lipozyme RM IM™, though gave lower yield, showed higher acyl migration than the reaction catalyzed by D-EP100. Low acyl migration was observed in alcoholysis using CAL-B after 6h. This could be because 1,2-DG and 1,3-DG were produced at the same time according to the non-specificity of CAL-B.

D-EP100 showed higher activity than CAL-B and Lipozyme RM, respectively, in alcoholysis of tripalmitin and tristearin. *Effect of $a_W$:* Higher yield and less MG was obtained from alcoholysis at aW 0.11 than 0.43. It was found that MG was increased with increasing $a_W$. Effect of solvents (on yield and acyl migration): Highest yield was obtained with MTBE. Alcoholysis in n-hexane and isooctane gave moderate yield, while acetone was a poor solvent for Lipozyme RM. The reaction performed in n-hexane showed faster acyl migration than in MTBE, as illustrated in Figures 18 and 19. Figure 19 illustrates data from the hydrolysis of trilaurin at 60°C by Lipozyme RM IM™ (DG = dilaurin).

*Hydrolysis of triglycerides*

Hydrolysis of pure triglycerides (TGs), including trilaurin, tripalmitin and tristearin, was carried out. A high amount of FFA was produced during the reaction and high amount ofunreacted TG remained in the reaction mixture. Most DG was 1,2-DG. Hydrolysis reaction showed higher acyl migration than alcoholysis reaction.

Effect of amount of water: highest yield was obtained using TG:water ratio of 1:1, as illustrated in Figure 20. Figure 20 shows the effect of trilaurin:water ratio on hydrolysis of trilaurin in MTBE at 60°C by Lipozyme RM IM™ (DG = 1,2-dilaurin + 1,3-dilaurin, MG = monolaurin). The amount of MG was increased with increasing TG:water ratio, especially in MTBE.

Effect of solvents: the result was corresponding to the result obtained from alcoholysis reaction. Hydrolysis in MTBE allowed higher yield than in n-hexane, isooctane and acetone, respectively, as illustrated in Figure 21. Figure 21 summarizes data showing the effect of organic solvents on hydrolysis of trilaurin at 60°C using Lipozyme RM IM™ (DG = 1,2-dilaurin + 1,3-dilaurin, MG = 1-monolaurin + 2-monolaurin). Though reaction in MTBE gave higher yield, high amount of FFA was produced and lower acyl migration was found than in n-hexane. The separation of TG, DG, MG and FFA can be a problem.

*Alcoholysis and Hydrolysis of natural oils*

Alcoholysis and hydrolysis of natural oils, including coconut and palm kernel oils, was carried out. The 1,3-DG yield of reaction with natural oils was slightly less than the yield of alcoholysis and hydrolysis of pure TG. The highest DG yield (45-50%) and fastest reaction rate was obtained from alcoholysis in MTBE at 40°C by D-EP 100, as illustrated in Figure 22. Figure 22 shows the results of alcoholysis and hydrolysis of coconut oil in organic solvent at 40°C (TG: ethanol = 1:1 mol/mol, TG:water = 1:2 mol/mol). Lipozyme RM IM™ gave higher yield and less MG than Lipozyme TL™. Reaction performed in MTBE gave higher yield and faster reaction rate than in n-hexane and acetone, respectively.

*Induction of Acyl migration*

Acyl migration was carried out on natural oils, including coconut and palm kernel oils. The effect of temperature and carrier was not clear. Almost no acyl migration was observed after 72 h. Addition of oleic acid to the reaction mixture slightly induced acyl migration, as illustrated in Figure 23. Figure 23 shows the effect of oleic acid on acyl migration of 1,2-dipalmitin in n-hexane at room temperature. The acyl migration rate was increased with increasing oleic acid:1,2-DP ratio.

Anion exchangers showed high induction of acyl migration, while cation exchange showed no effect. The acyl migration rate was increased with increasing amount of anion exchanger, as illustrated in Figure 24. Figure 24 shows the effect of the amount of anion exchanger on acyl migration of 1,2-dipalmitin (5 mg/ml) in n-hexane at room temperature. A large amount of anion-exchanger was required to induce a fast acyl migration.

*Esterification of 1,3-DG and FFA/FAVE*

Esterification of 1,3-DLa and OA in n-hexane was carried out with immobilized lipase from *Pseudomonas* sp. (Amano PS-D), *Candida antarctica* type A (CAL-A), and *Penicillium cyclopium* (Lipase G). Molecular sieve was added to the reaction mixtures to remove the produced water. It was found that only PS-D was capable of catalyzing the esterification reaction of 1,3-DG and oleic acid (OA) or vinyl oleate (OAVE). The reaction was fast. Almost all 1,3-DG was consumed after 2 h for 1,3-DCy and 8 h for 1,3-DLa.

Table 1 shows the fatty acid compositions of the ST products thus obtained. By-products of CyOO and OOO were present due to the non-specificity of PS-D, as illustrated in Figure 25.

Table 1 Fatty acid composition of structured triglycerides products.

| Structured triglycerides | Fatty acids (%)* | | |
|---|---|---|---|
| | C8:0 | C12:0 | C18:1 |
| CyOCy | 61.0 | - | 39.0 |
| LaOLa | - | 63.3 | 36.7 |
| CyOCy (larger scale) | 66.9 | - | 33.1 |

* determined by GC analysis

Figure 25 shows data from the esterification in larger scale of 1,3-dicaprylin and oleic acid vinyl ester in n-hexane at 60°C by the immobilized lipase from a *Pseudomonas* sp. (PS-D). Acylglycerol composition was analyzed by HPLC.

Vinyl oleate (OAVE) allowed much faster reaction than OA and 1,3-DCy allowed faster reaction than 1,3-DLa, as illustrated in Figure 26. Figure 26 shows data from the esterification of 1,3-DG and oleic acid (C18:1) or oleic acid vinyl ester (OAVE) in n-hexane at 60°C using PS-D. Acylglycerol composition was determined by TLC/FID (Cy = reaction with 1,3-dicaprylin, La = reaction with 1,3-dilaurin, TG = triglycerides, DG = 1,3-diglycerides, VE = vinyl ester).

EXAMPLE 8: Protease activity assays

The following example describes exemplary protease activity assays to determine the catalytic activity of a protease (e.g., an enzyme of the invention). These exemplary assays can be used to determine if a polypeptide (e.g., a protease) is within the scope of the invention.

The activity assays used for proteinases (active on proteins) include zymograms and liquid substrate enzyme assays. Three different types of zymograms were used to measure activity: casein, gelatin and zein. For the liquid substrate enzyme assays, three main types were used: gel electrophoresis, O-pthaldialdehyde (OPA), and fluorescent end point assays. For both the gel electrophoresis and OPA assays, four different substrates were used: zein, Soybean Trypsin Inhibitor (SBTI, SIGMA-Aldrich, T6522), wheat germ lectin and soybean lectin. The substrate for the fluorescent end point assay was gelatin.

The activity assays used for proteinases and peptidases (active on peptides) used pNA linked small peptide substrates. The assays included specificity end point assays, unit definition kinetic assays and pH assays.

The following example describes the above-mentioned exemplary protease activity assays. These exemplary assays can be used to determine if a polypeptide is within the scope of the invention.

Protein (proteinase activity)

*Casein zymogram gel assays*

Casein zymogram gels were used to assess proteinase activity. The protease activity assays were assessed using 4-16% gradient gels (Invitrogen Corp., Carlsbad, CA) containing casein conjugated to a blue dye and embedded within the gel matrix. All zymogram gels were processed according to the manufacturer's instructions. Briefly, each sample was mixed with an equal volume of 2x loading dye and incubated without heating for ten minutes before loading. After electrophoresis, gels were incubated in a renaturing buffer to remove the SDS and allow the proteins to regain their native form. Gels were then transferred to a developing solution and incubated at 37°C for 4 to 24 hours. If a protease digests the casein in the gel, a clear zone is produced against the otherwise blue background that corresponds to the location of the protease in the gel. Negative controls (indicated with NC on gel images) were processed along with the experimental samples in each experiment and electrophoresed on the casein zymograms next to their corresponding protease(s).

Unlike traditional SDS-PAGE, samples are not heat denatured prior to electrophoresis of casein zymograms. As a result, it is sometimes difficult to accurately assess the molecular weight of the proteases. For example, Subtilisin A (Sigma, P5380, indicated with Subt.A on the gel images), which was used as a positive control in these experiments, is predicted to be approximately 27 kDa in size. However, when electrophoresed through casein zymograms using the conditions described, Subtilisin A barely migrates into the gel and is visible only above 183kDa. Therefore, the zymograms do not define the MW of the proteases indicated, but rather used as an indicator of activity.

*Gelatin zymogram assays*

Gelatin zymograms, Novex® Zymogram Gels, were performed according to manufacturer's instructions (Invitrogen Corp., Carlsbad, CA). Unlike the casein zymograms, gelatin zymograms were post-stained following development using either a Colloidal Blue Staining Kit or the SIMPLYBLUE™ Safestain, (both from Invitrogen). Areas of protease activity appeared as clear bands against a dark background.

*Corn Zein assays*

Corn zein was used as substrate for protease activity assays, using powder, Z-3625 (Sigma Chemical Co. St. Louis, MO), and Aquazein, 10% solution (Freeman Industries, Tuckahoe, NY). When fractionated through a SDS-PAGE gel, zein from both suppliers produced bands of 24 and 22 kDa. The two zein bands correspond in molecular weight to those previously described for alpha-zein, the most abundant subclass of zeins, which are estimated to comprise 71-84% of total zein in corn (see, e.g., Consoli (2001) Electrophoresis 22:2983-2989).

Lyophilized culture supernatants containing active protease were resuspended, dialyzed, and incubated with zein in 50 mM $KPO_4$, pH 7.5. Reactions were run in a 96-well microtiter format. "Substrate only" and "enzyme preparation only" controls were processed as well as experimental samples. After 24 hours at 30°C, aliquots were removed and subjected to OPA, SDS-PAGE, or Zymogram analysis. In some cases, fresh aliquots were removed and analyzed after 48 or 72 hours at 30°C.

Zein Zymogram: Aquazein was added to a final concentration of 0.075% in a 10% polyacrylamide gel. Aliquots of dialyzed protease samples were electrophoresed through the zein zymogram using standard conditions. Following electrophoresis, the zymogram gel was washed, incubated in a renaturing buffer, incubated overnight in a developing buffer optimized for protease activity (contains NaCl, $CaCl_2$, and Brij 35, in Tris buffer pH 8), and stained with Coomassie blue stain.

SDS-PAGE: Aliquots of equal volume were removed from each sample and subjected to SDS-PAGE analysis. Following electrophoresis, proteins in the gels were stained with SYPRO Orange (Molecular Probes) and visualized using UV transillumination.

OPA: In the presence of Beta-mercaptoethanol (BME), OPA reacts with free amino ends to produce a fluorescent imidazole that can be detected using a standard fluorescence plate reader. In this assay, aliquots of equal volume were removed from each sample and placed in a black fluorescence plate. Samples were then diluted 1:10 in OPA reagents. Fluorescence (Ex = 340 nm, Em = 450 nm) was determined after a 5-minute incubation.

*Soybean Trypsin Inhibitor assays*

Soybean Trypsin Inhibitor (SBTI, SIGMA-Aldrich, T6522) was used as a substrate for protease activity. Lyophilized culture supernatants containing active protease were resuspended, dialyzed, and incubated with SBTI (1 mg/ml final conc.) at 37°C in 50 mM $KPO_4$, pH 7.5. Substrate alone and enzyme preparation alone controls were processed along with experimental samples. After 24 hours, aliquots were removed and subjected to OPA and SDS-PAGE analysis. SDS-PAGE: for SBTI, following electrophoresis, proteins in the gels were stained with Coomassie blue.

*Wheat Germ Lectin assays*

Wheat germ lectin (WGA, EY Laboratories, L-2101, Pure) was used as a substrate for protease activity. Lyophilized culture supernatants containing active protease were resuspended, dialyzed, and incubated with WGA (1 mg/ml final concentration) at 37°C in 50 mM $KPO_4$, pH 7.5. Substrate alone and enzyme preparation alone controls were processed along with experimental samples. After 24 hours, aliquots were removed and subjected to OPA and SDS-PAGE analysis as. SDS-PAGE: for WGA, following electrophoresis, proteins in the gels were stained with Coomassie blue.

*Soybean lectin assays*

Soybean lectin (SBA, EY Laboratories, L-1300, Crude) was used as a substrate for protease activity. Lyophilized culture supernatants containing active protease were resuspended, dialyzed, and incubated with SBA (1 mg/ml final concentration) at 37°C in 50 mM $KPO_4$, pH 7.5. Substrate alone and enzyme preparation alone controls were processed along with experimental samples. After 24 hours, aliquots were removed and subjected to OPA and SDS-PAGE analysis. SDS-PAGE: for SBA, following electrophoresis, proteins in the gels were stained with Coomassie blue.

*Gelatin in fluorescent liquid end point assay*

DQ Gelatin (Molecular Probes, fluorescein conjugate, D-12054) was used to assess the proteolytic activity of the proteases of the invention. DQ gelatin is a protein that is so heavily labeled with a fluorophore that its fluorescence is quenched when the molecule is intact. Proteases that cleave the substrate will release the fluorophores from internal quenching and fluorescence will increase in proportion to the protease activity. DQ Gelatin was diluted to a final concentration of 25 ug/ml in 100 ul reactions containing a suitable buffer such as zymogram developing buffer (Invitrogen) and varying amounts of protease preparations. Reactions were incubated in a 384 well, clear, flat-bottom microtiter plate at 37°C for various time periods from 1 hr to overnight. Fluorescence was monitored using a fluorescence plate reader after incubation at 37°C for various times.

EXAMPLE 9: Simulation of PLC-Mediated Degumming

This example describes an exemplary use of a hydrolase of the invention, a phospholipase of the invention, comprising the simulation of phospholipase C (PLC)-mediated degumming.

Due to its poor solubility in water phosphatidylcholine (PC) was originally dissolved in ethanol (100 mg/ml). For initial testing, a stock solution of PC in 50 mM 3-morpholinopropanesulpholic acid or 60 mM citric acid/NaOH at pH 6 was prepared. The PC stock solution (10μl, 1μg/μl) was added to 500 μl of refined soybean oil (2% water) in an Eppendorf tube. To generate an emulsion the content of the tube was mixed for 3 min by vortexing. The oil and the water phase were separated by centrifugation for 1 min at 13,000 rpm. The reaction tubes were pre-incubated at the desired temperature ($37^0$C, $50^0$C, or $60^0$C) and 3 μl of PLC from *Bacillus cereus* (0.9 U/μl) were added to the water phase. The disappearance of PC was analyzed by TLC using chloroform/ methanol/water (65:25:4) as a solvent system (see, e.g., Taguchi (1975) supra) and was visualized after exposure to $I_2$ vapor. The oil and water phases are separated after centrifugation and PLC is added to the water phase, which contains the precipitated phosphatides ("gums"). The PLC hydrolysis takes place in the water phase. The time course of the reaction is monitored by withdrawing aliquots from the water phase and analyzing them by TLC.

EXAMPLE 10: Expression of Hydrolases (e.g., Phospholipases) of the Invention

This example describes the construction of a commercial production strain of the invention that can express multiple hydrolases of the invention, e.g., phospholipase enzymes of the invention. In order to produce a multi-enzyme formulation suitable for use in the degumming of food-grade vegetable oils (including soybean, canola, and sunflower), a recombinant expression strain can be generated that expresses two different hydrolases of the invention, e.g., phospholipase enzymes of the invention, in the same expression host. For example, this strain may be constructed to contain one or more copies of a hydrolase (e.g., a PLC) gene and one or more copies of another hydrolase gene (e.g., a phosphatidylinositol-PLC gene). These genes may exist on one plasmid, multiple plasmids, or the genes may be inserted into the genome of the expression host by homologous recombination. When the genes are introduced by homologous recombination, the genes may be introduced into a single site in the host genome as a DNA expression cassette that contains one or more copies of both genes. Alternatively, one or more copies of each gene may be introduced into distinct sites in the host chromosome. The expression of these two gene sequences could be driven by one type of promoter or each gene sequence may be driven by an independent promoter. Depending on the number of copies of each gene and the type of promoter, the final strain will express varying ratios of each active enzyme type. The expression strains can be constructed using any *Streptomyces* or *Bacillus, Bacillus cereus, E. coli, S. pombe, P. pastoris,* or other gram-negative, gram-positive, or yeast expression systems.

In one aspect, the invention provides a two-enzyme system for degumming of soybean oil, wherein at least one enzyme is a hydrolase enzyme of the invention. PLC plus PI-PLC produces more DAG than either enzyme alone. However both enzymes produce more DAG than a no enzyme control sample. In one aspect, reaction conditions comprise 1 milliliter soybean oil, ~0.4% initial moisture, 50°C, 0.2% Citric acid neutralized with 2.75M NaOH, 10U PLC, 15μL PI-PLC (0.45mg total protein), 1 hour total reaction time. Figure 30 illustrates a table summarizing data from this two-enzyme degumming system of the invention.

In another aspect, a PI-PLC enzyme of the invention can be used under the same conditions described for PLC. These include chemical refining of vegetable oils and water degumming of vegetable oils.

EXEMPLARY SEQUENCES OF THE INVENTION

**1) SEQ ID NO:1**

ATGCGCGGTCTGGCGCGTTCAATCACTTTTCGTTTGGTGTTCGCTGCGACTGCGTTCGTCGTT
GCGCTGGCAACGTTGGCCCAGGCGCAAAACGACAAGATGACACCCATCGCCACACCGGCGCAGCCCAAT
GCGATCGAGATCGGAACCGGTCCGCTGCCCGATGCGAAGAACCCCGAGTCCTGGCATAGCCAGTACGGC
AGCAAGTTTGCCCGTAACGTAACCATAGCGACGCTGACACCATTTCTGCCGGATGCTGCCAGGGCAAGC
GGCGCCGCAGTCATCGTCGCGCCCGGCGGCGGTTTCCGCACCTTGTCGATGGAAAATGAAGGCTGGAAC
GTTGCGCGCGCCCTGGCTGAAAAGGGCGTCGCTGCGTTCGTCCTGAAGTATCGATTGAATCAGACCCCG
GCGGACATGCCGGGCTTCGAGCAGTCCATGCGAGAAATGTTTTCGGGAACAGCGCGACCGCCGCGCCCC
GACCCGGCGAAGGCTATTGCCGGTCTTGCTCCGCAAATCGCCGACGCCCGCGCGGCATTTGCATTAATC
CGCAAGCGTTCTTCCGAATGGCACGTCGATCCTAACCGGATCGGCATGGTTGGTTTTTCCGCCGGCGCG
ATGTTGACCATGGCGACCGCGCTCGCGGGCGAGGATGCGAAGCCGGCCTTCATCGGCAACATCTACGGT
CCGCTCGCGTCAGTCACCGTGCCGGCTGACGCGCCCCCATTGTTCATCGCGCTTGCTGCCGACGATCCT
TTCTTCGCGAACGGCGGCTATGGGCTCATCGATAGCTGGAAGACGGCGAAGCGGCCCGTTGAATTCCAT
CTTTATGAACAGGGTGGGCATGGCTTCGGGATGTATCCGAAGGAAACGACGAGCACCGGCTGGTTCGAA
GCCTTCGTGCGGTGGATAGGAATGCACGGCATGCTGAAGCCGGCAGCGTCAGGAAGCGGCGCGAAGGGA
TCTCATCACCATCACCATCACTAA

**2) SEQ ID NO:3**

ATGACCACATCTACACAACACATCAGCGAGCTACCTCTCTTACCAGGCCGTCTCGGCGACCCC
GATCGCGTCTTGAAGACTGATCCCCGTGCCGATCCTCGGTTGGTCGCCGCCTGCGCCCCCTTTGCTTTA
GACGTTGCGCCCCCACCCGCACCGGTGAACGCGCACTCGCCCTTGCAGGACAAGCTCGACTACAGTGCG
GCCAACGAGGCGGGCATGGAAACCGTGTTTGCCGCCCTGTTCGCCGACCTCCCGCCGATGACGAACGTG
GAGCGGCGGACCGAGGTCATCAAGGGCGTGGACGGTAACGACATCAAGCTGTATATCCATACGCCCCAG
CACGTTTCTGGCCCGCTGCCCTGCGTGTATCATATACACGGCGGCGGCATGGTCATTCTGACGGCCGCT

GGCCCTACCTATGTACGCTGGCGGGACGAGCTTGCCGCCCTGGGCATGGTCGTGGTGGGTGTGGAATTC
CGTAATGGCGCCGGCAAGCTTGGCAACCATCCCTTTCCAGCCGGGCTCAACGACTGCATGAGTGGCTTA
CAGTGGACGTTTGACCACAAAACCACCCTGGGGATCTCGAAGATAATCGTGTCCGGTGAGTCTGGTGGA
GGCAATCTCTCCCTGGCCGTGTGCCTCAAGGCCAAGAAGGACAAACGCCTTGAGCAGATTCATGGTGTC
TACGCCTTGTGTCCGTACATTTATGGCGCATGGGCGCAGAAGAGCAAAGATCTCCCTTCCCTATACGAA
AACGACGGCTACTTGATCAACTGTAGCCTGATGGAGGTGCTGGCAAGCGTCTATGACCCTGAAGGCAAA
AACGCCACCAATCCGCTGTGCTGGCCATACTGGGCCACACGTGAGGACCTGCAAGGTCTGCCTCCGCAT
GTGATCTCGGTCAACGAGTTAGACCCCCTGCGAGACGAGGGGCTGAAATACTATCAGAGGCTCCTGGCG
GCTGGGGTGCGTGTATACAGCCGGACCGTCAACGGCACGTGCCATGCGGGCGACGTCCTGTTCCGCAAA
GCGCTCCCTGACGTGTACGCAGCCACCCTCCGCGATATCAAGGGGTTCGCAGACTCGCTGGGATCTCAT
CACCATCACCATCACTAA

**3) SEQ ID NO:5**

ATGTCACTTGATCCACAAACGAAATTTGTATTAGCCCAATTGGCTGCTGCCGATGCTCCTCCA
ATGGAGACTTTGTCACCGGAAATGGCTAGACAGGCATTCATATTGCCGCAAGGTGCAGTGGAGGAAGTA
GGGAAGGTAGAAAATCGAACGATTCCTGGTCCTGCAACGGACATCCCTGTCCGTGTTTATTACCCTAAA
GAACTCCAGCCTGAAAATCCCGCGCTAGTTTTCTATCATGGCGGCGGCTGGGTAATTGGCAATTTGGAC
TCTCATGACGATATTTGCCGTGCTTTAACCAACCTCGCCAACTGCGTGACCATTTCCGTCGACTACCGC
CTGGCTCCAGAGAATAAATTTCCTGCTGCGGTTGAGGATGCTTATGCTGCTGCACAATATGTGTATGAC
CATGCAGAAGATTTCAAAGTCGACAAGACCCGTATTGCCGTTGGCGGTGACAGTGCCGGTGGAAATCTT
GCCGCTGTCGTGACGAATCTGGCAAAAGACAAAAACTCACCTTCTATTTGTTTCCAACTTCTGATTTAT
CCAAGCACCAATGCAGGTGGTGAGCCAACAGCATCAATGGTTGAGAATGCCCATGGCTATTTCTTAGAA
AAAGGCACGATGGATTGGTTCCGTGACTGCTACCTGAACAGTGAAGAAGACAAACAGAATCCGCTAGTC
TCACCGATGCTTTATGATGATTTCCAAGGACTGCCTCCAGCAATTGTGATTACTGCCGAGTACGATCCA
TTGCGAGATGAAGGCGAAGCATATGCCAAAAAGCTGGGCGAAGCAGGGGTTGCTGTCCAAACCATCCGC
TTTGACGGTACGATTCATGGCTTTGTCAGCATGTCGGCTGTCATTAGCCAAGGGAAGGCGGCATTGGAA
AAAGCAGGAGAGGCTTTAACTAAAGCCTTTCAAGGATCTCATCACCATCACCATCACTAA

4) SEQ ID NO:7

ATGCCGCTCGATCCCGTCATTCAGCAGGTGCTCGATCAACTCAACCGCATGCCTGCCCCG
GACTACAAACATCTCTCCGCCCAGCAATTTCGTTCCCAACAGTCGCTGTTTCCTCCTGTC
AAGAAGGAGCCCGTGGCCGAGGTCCGAGAGTTTGACATGGATCTGCCTGGCCGCACGCTC
AAGGTGCGCATGTACCGCCCGGAGGGCGTCGAACCGCCCTACCCCGCGCTCGTGTATTAT
CACGGCGGCGGTTGGGTCGTCGGAGACCTCGAGACGCACGATCCCGTCTGCCGCGTCCTC
GCGAAAGACGGCCGCGCGGTCGTGTTCTCCGTCGACTACCGCCTGGCGCCGGAGCACAAG
TTCCCTGCCGCCGTGGAAGACGCCTACGACGCGCTTCAGTGGATCGCGGAGCGCGCAGCG
GACTTTCATCTCGATCCAGCCCGCATCGCGGTCGGCGGAGACAGCGCCGGAGGGAATCTT

GCCGCTGTGACGAGCATCCTTGCCAAAGAGCGCGGCGGGCCGGCCATCGCGTTCCAGCTG
CTCATCTACCCTTCCACGGGGTACGATCCGGCTCATCCTCCCGCATCTATCGAAGAAAAT
GCGGAAGGCTATCTCCTGACCGGCGGCATGATGCTCTGGTTCCGGGATCAATACTTGAAC
AGCCTGGAGGAACTCACGCATCCGTGGTTTTCACCCGTCCTCTACCCGGACTTGAGCGGC
TTGCCTCCGGCGTACATCGCGACGGCGCAGTACGATCCGCTGCGCGACGTCGGCAAGCTT
TACGCGGAAGCGCTGAACAAGGCGGGCGTCAAGGTCGAGATCGAGAACTTCGAAGATCTG
ATCCACGGATTCGCACAGTTTTACAGCCTTTCGCCTGGCGCGACGAAGGCGCTCGTCCGC
ATTGCGGAGAAACTTCGAGACGCGCTGGCCTGA

5) SEQ ID NO:9

ATGCTCTGGTTTCTGCTCGCTGTCGTTACTCTTGTTGTCCTGATCACACTGCTGATCTTCCGG
GTAAAGGATCTTTCCGAGTACGACGGCCAGGACTGGGTTATCAAGGAAGTTGCTCCAAACCCGGCTCAT
GATGAGGTCATCGAACGCATCAAGGACATGGGGCGCGCCTCCCGCGGACTGAAGGGCAAAGCCCGGCTG
ATCGCCCTCCGCAACCATCTGGACAGCCTGGGTGAAGGCGTGGATATCGAATCGGACATCCGGCATCAG
GATCATCCACGTGGCGAATGGATATTGGCGCCCGGGGCCGATTCCCGCCGACGTGTGCTCTATATTCAC
GGCGGTGCCTGGGCTGCTGGCAGCCCTCGCAGTCACCGGGCGATTACCGACCGTTTTTCCCGGATTGCC
AATGCTGCCGTTTTTGCGGTTGATTACCGGCTCATGCCCGAACACCGGTTCATGGATGGCATTCGGGAC
TGCCGCCAGGCCTATGCATGGCTGCTGAATCATGGCCCGGACGGCGAAGCGCCCGTCGACTTTATGGTG
GTTGCCGGAGACTCGGCGGGCGGCAGCCATACCCTGTCACTGCTGGCGTGGATTCGCGACAATGGCCTG
CGGCAGGCCGATGCCGCCGTTGCCCTGTCACCCTCCACGGATCTCACGCTGACGGCGCCTAGCAATCGC
GAGAATATTCGCACGGACCCACTCCTGGGCCCGGTGTTTGGTGGTTTGTCCAAAATCCCGCTGCCCGTG
TTGTGGTGGGGAACACTTGCCGCGTTCAGACTCTCTCCCACCAACCCGGTTGCATCACCTCTGCGGGGT
GAGCTTAACAAGCTGCCACCCGTGCTTATCCACGCAAGCACCACGGAAATGCTGCTGGACAACGCCACG
CGTTACGCCGCCAAGGCAAAAGCCCAGGGTTCACCGGTGGAGCTTCACACCTGGCAGAACATGGTGCAT
GTATGGCACATCTTCACGCCACTGCTGCCTGAGGCAGACGAGGCATTCGACGACATCGCGGCTTTTCTG
AAGAAAGTGGAACAACCTGGATCTCATCACCATCACCATCACTAA

**6) SEQ ID NO:11**

ATGACCTTGATTACGCCAAGCTCGAAATTAACCCTCACTAAAGGGAACAAAAGCTGGAGCTCG
CGCGCCTGCAGGTCGACACTAGTGGATCCGAGGATCGACCCGCGCATCAAGGCGGTGATGGGCGCGTTC
GAGGCCACCAGCGCGCAGGGTGACGTCGCCAGCCGCGACGAACTGCTGGCCGAGGCGGCCAGCGACGAA
GCGGTGGCCCAGCGGGAGATGCTCACCGCCTTCCTCGGCATGTGCGACACCGAGGAGATCGCGCCGTCG
GCGGGGCTCACGGTCTCCGAGCACCGCGTCGCCTCTCAGCCCGACGGCAACAAGATCAACATCCGGTTC
ATCCGGCCCGAGGGCGACGACGTGCTGCCGTGCGTGTACTACATCCACGGCGGTGGCATGGCGTCGATG
TCGTGCTATGACGGCAACTACCGGGCGTGGGGCCGGATCATCGCCGCCCAGGGCGTGGCGGTTGCCATG
GTCGACTTCCGCAACGCGCTCACACCGTCGTCGGCCGAGGAGGTGGCCCCGTTCCCCGCTGGGCTCAAC
GACTGCGTGTCGGGGCTCAAGTGGGTCGCGGCCAACGCCGCCGATCTGCGCATCGACCCGGCCCGCATC
GTCGTGGCCGGCGAGAGCGGCGGCGGGAACCTCACCCTCGCCACCGGCCTGAAGCTCAAGCGCGACGGC

GACCTCGGGCTGATCAAGGGTCTCTATGCCCTCTGCCCCTACATTGCCGGCGAGTGGCCGCTGCCGCAG
AACCCGTCGTCGACCGAGAACGAGGGCATCCTCCTGCACCTGCACAACAACCGCGGCCGCATGACGTAC
GGCATCGAGGAGTTCGAGGCGCGGAACCCGCTGGCGTGGCCCGGCTTCGCCACTGAAGACGACGTCGCC
GGCCTCGTGCCCACCATCATCAGCGTGAACGAGTGCGACCCGCTGCGCGACGAGGGCGTCGGCTTCTAC
CGCCTCCTCCTCCGCGCGGGTGTGCCCACCCGGTGCCGCCAGGTGATGGGCACCATCCACGGCACCGAG
ATCTTCCCGATGCTGTGCCCCGACGTGAGCCGGGACACCGCGGCCAGCATCGCCGACTTCGCCCGCAAC
GCCGGGTCCAGATCTCATCACCATCACCATCACTAA

**7) SEQ ID NO:3**

GTGGATCCGAAGGCCCAGATCGTCGGCGAGTTCGTCAAGTCCATCCGCCAGCCGGGTTAC
TTTCCCCCGCTACCCGAGCTGCGCCAGCAGCTCCGGACGATGGTGGCGCTGATGGACGAG
CCGGCACCCGCGCTGGCGCGCATCGAGGACCGCCGCATCCCGGGCCCGGCCGGCGACATA
CCGGTGCGCGTGTACGCCTCGGACGTCGGCGCGTCGCGTCCCGTCGTGGCGTACTTTCAC
GGCGGCGGCTGGGTGCAGGGCGACCTCGAGACTCACCACGGCCTCTGCGCGAGGCTGGCG
AAGCACGCCGGGGCCCTCGTCGTGGCCGTCGACTACCGGCTGGCGCCCGAGCACAAGTTC
CCGGCCGCCGTCGAGGACTGCCTGGCGGCCTATCGCTGGCTCCGAGCCCACGGAGGCGAC
CTCGGCGCCGACCCGGGGCGCGTGGCGGTGGCCGGGGACTCGGCGGGCGGCAATCTCTCG
GCCGTCGTCTCGCAGGTCGCAGCCGCGGAGGGCGCTCCGGTGCCCACGTGCCAGGTCCTG
ATCTATCCGGCGGTGGACTTCTCGCTCGACAGCGACTCCCACCGAGAGCTGGCGGAGGGC
CACATCATCCCGCGCGACCGTGTCCTCTGGTACTCGGAGCAGTACCTGAGGGGCGAGGCC
GACAAGCGGGATCTGCGCGCCTCGCCCCTGCGGGCGCCGAAGCTCGCCGGCCAGCCGCCG
ACGCTCGTCGTCACCGCCGGGTTCGATCCTCTCCGCGACGAGGGCCGGGCGTATGCCGAT
CGGCTCCGCGAGGCCGGGGTGGACGTCGTCTACCGCGAGTACCCGGGCCAGATCCATGCC
TTCGTGTCGCTGACGAAGGCGATTCCGGAGGGCATGGCCTGCACGCTGGAGATTGCCGAG
TACCTGCGCCAGCGCCTCGGCTGA

8) SEQ ID NO:15

ATGGATGCCGGTTTTTCCCGCTCAAACAGGATCGTTATGAGAATCAGCCTTATTCACGTTGCT
TTGCTGCTCGGCCCGCTTTTCCCGGTTAGCGCACTTTCCGCCGCCGACCAGCTGCGCCGCGGCGAACGC
AAGGAAACAACCAAGGCCGAAGCCCCGCGCAAGGAGGAGAAGAAGGACGAGCCGGCGCAATCGCCCGTC
GAGATCATCAAGAACATTTCCTACCGGGACGATGAGGCCGCCGACCCGACGCGCCACAAGCTCGACCTG
TACCTGCCGCGCGGCGCCAAGGACTTCCCGATTGTCTTTTTCGTCCACGGCGGCGCCTGGCGAGCCGGC
AACAAGGATGAGTATCCCAGACTTGGCGAATTCTTTGCCACCGAGGGCATTGGCTGCGTCGTCATCAAT
TACCGCCTCTCCCCCAAGGTGCAGCATCCCGCCCATATTGAAGACGTCGCCCGTGCCTTCGCCTGGACC
GTCGCCAACATCGAAAAATACGGCGGCCGTAAAGATCGCATTTTCGCCGTGGGCCACTCGGCCGGCGGC
CACCTGGTCGCCCTGCTGGCCACCGATGCCACGTACCTCAAGAACGAAGGACTGGCGCTGACCGACATT

CGCGGCGTGATCCCCATTAGCGGCGTGCATCAGATCAACTCGGCCCTGCCGATGTTCCGTAACATTTTC
AGCAGGGACAAGGAGGAGTGCCGGCTTGCCTCGCCCATCACCCATGTCAGCAATGGCCACCCGCCTTTC
CTGCTCCTTTACGCTGAGAACGACCTGCCGCTCTTGGGCAGGATGGCCGAGGACATGAGCGAGGTGCTG
AAGAAGAATCAGTGCGATGTCGAATGCCTGAAGATTGACCAGCGCAACCACATGACGATCATCACCCAG
CTTTCCCAGGAAAATGACCCGACGCGGCAAGCGGTCTTCGACTTCATCGGCCGGCATAGCGACTGGCAG
CCCGCGGCCAGGGCCGAGGCTGCCAACGGCAATAAGCGCGAAGCCGGCACGACGGACGCCGCTATGAAG
GACGACAAAGGATCTCATCACCATCACCATCACTAA

9) SEQ ID NO:17

ATGAAAATTAGACAACCACAACCATTTACATTTGAAGCAGGAAAAAGGGCCGTTTTATTATTG
CATGGATTTACAGGTCATTCGGCAGATGTACGCATGCTTGGTCGCTATTTAGAGGAAAAAGGTTATACC
TCTCATGCGCCAATTTATCGTGGCCATGGTCTTCCACCTGAGCAGCTCATGGAAACAAATCCAAACATG
TGGTGGGAGGATGTTGTACAAGCATATGAACATTTACGTCAACTCGGTTATGAAGAAATAGCAGTGGCC
GGCCTATCGTTAGGTGGAATGATGGCATTAAAGTTAGCGACAGAGACAAAAATAAAAGCTGTTATATCG
ATGTGTGTTCCTATGATATTTGATGATAAAAATAATTTAACAGAGTCTTTTGAGCGTTTTGTACGACAA
TATAAACAGTTTGAAAAAAAGGATAAAGCGACGATTGATCATGAAACAGAACAAATAATGGAACAATCC
GTTGATTTGTTTGAGGAAATTAAAGCATTTAATTTATCGGTTAAAGAAGTCATTGATTTGATTTATGCA
CCAACACTTGTTATACAAGCACGTAACGATGAGGTTATTAATCCTGAAAGTGCACAATATATATATGAT
TCAGTTGAAACAGATGAAAAAGAAATAAAGTGGTATGAAGAATCAGGCCATGTTATCACATTAGGTGAA
GAACGCGACCAATTACATGAAGATATTTATATGTTTCTCGAACAGTTAGAATGGGAACAATAA

**10) SEO ID NO:19**

ATGGCTTCCATTCAATCTCGTATTTTCACTGGGTTATTGCGTATTGCGTCAGGCTACCGCTCT
CGAAGCGATCGCACCTCCGCGCAAACAGCCGTGAACAAGTCCCGTGCCTTTACGCGGCTGATGACCTTG
CCCCAGCTTCCGGGAACGCGATCCATCCACGCCGACATTCCGAACCTTCGCGCAACGTGGCATGTGGGG
AAGCACGTACGGGACGATGTGCGCATCGTCTATTTCCACGGCGGAGCCTATACCGCAGGGTCTGTCGCG
ACGCATCGACACTTCACCTCGATGCTCGCAAAACTCAGCCATATCCCCGTGCTTTCTGTCGACTATCGC
CTCGCACCAGAACATCCTTTTCCTGCGGGCTTTGATGATGCAATTGCCGCCTATCAATGGGCCAAAGAG
CACGGGCCAGTAGGAAAGAGCGAAGCGCAACATATCGTAATTGCAGGTGATTCTGCGGGCGGAGGCCTC
AGTGCGGCGATCGCGATGCAACTGCGCGATGCAGGACAGCTCCTAGACGGCGGCCTCATGCTGCTTTCG
CCGTGGATGGATCTGACCTGTAGCTCCGGCGCAGAACATCGCATCGGACATCTTGATGCCATGCTCAAT
GCGGACCACGCGCGTATCATGGCGAGTCTTTACGCAGGAACCCATGACTTGACCGACCCACGACTTTCG
CCGCTTTATGGAGACTTTGCGGGTCTTCCGCCCATGTTCGTCGCCATTGGCGGACGAGAAATCGTGCTC
GATGAGGTGGTTCATGCCCTTGAAAAAGCGCGCGCAGCGGGCGTGGAAGTTACCCTCGAACGTAATGAA
GAAATGTTCCACGTTTGGCCAGTGATGTTCCATCTCGTCCCCGAAGGACGCGCAAGCTTAGAGCGCATG
GTCGATTGGCTGAATTGGAAGTTTCCTGCACATCACCATCACCATCACTAATGA

**11) SEQ ID NO:21**

ATGACAACAGTTCCAGAAAAGCTTGACGCCAAAGTCGCTGACGCGCTTGACCCGATCATTGAT
GCAGCACTCACCGAAGGTCGATTGGCAGGCGGCGTCGCACTGGTGGCAAAGCGTGGAAAGTTGGTCTAT
GCACGCGCTGCTGGTTTTGCCGATATTGAGAGCAAGCGTCCCATGACCCGAGACGCCATCTTCCTTGCG
GCCTCGCTCACCAAGCCGATTGTGACAGCCGCGTTCCTCTCATTGGTCGAGGACGGCGTGATGAGCATG
GATGAGCCGATCACAAAATACCTCCCCGACTTCACACCGGCGTTTGAGGGTAAGACACAGCAGATCACG
TTGCGCCAACTACTCACTCATACGTCTGGTCTTTCCTATGGTTTCCTGCAACCGGCAGACGGTCCCTAT
CTGCGCCTCGGCGTTTCGGATGGGTTGAACGAACCGGGTCGGTCCTTTGCTGACAACTTGGCGCGGATT
GTCGAAGCCGGTCTGTTCTTTCCGCCGGGCGGGGCTTGGCTCTACTCGGTCAGCATGGATGTGTTGGGT
GCGGCGATGGAAGTGGCGACCGGCAAGAGCTTGCCGCAAGTCGTAAGCGAGCGTGTGTCCAGCAAGCTC
GGCATGGCAGATAGCGGGTTTGCAATCACGGATCGCGCGAGGCTCGCAACACCTTATGCAGATGGTCCG
CCGCCGGTGCCCATGGGAAACGATTTCGTTTTCCCCTTCGCCGAACTTTCAGGCATTCGCTTCACGCCC
GGCCGCATTTTCGATCAGACGTCGTTTCCATCGGGCGGCGGCGGCATGGCGTGCAGCGCGCCTGATCTG
CTGACCTTTCTGGAAGCCATCCGCACTGGTGGCGCACCGATTGTGAAGGCTGAGACAGCAAAGGCGATG
ATGACCAACCAGACTGGCTCGCACGCCTTGCTACTGAACGGCCCCGGTTGGGGCTTTGGGTATGGTGGC
GCGGTGTTGCTGGACCCGACGCCAACCGCTTCGCGCCTGCCAGTGGGCACATGGACCTGGGGTGGTGTT
TACGGTCACAGCTGGTCCGTCGATCCAACAGGAGAGACGAGCTGTGTCTTGATGACCAACACCACGGTC
GAAGGGATGGCGGGTGCATTGTCTGTCGATTTGATGGCTGCAGCATTGGCAGGATCTCATCACCATCAC
CATCACTAA

12) SEQ ID NO:23

ATGAAAAGATATTTCTATTTGTTCTTATATCACCTCCTTACCATACAGTTAGTTTTCAGTATA
TCACTGGAAGCAAAACCGCTGCTTCTTAAAAATGAATATAAAGTTGCTAGGGATGTAGAATGGGCAAAA
CCTGATGGATTCAGTCTGACAATGGATATCTATACGCCTGAAGGTGATAAAGATAATTATCCGGTGCTG
CTTATATTTCACGGCGGTGGTTGGCTGGCAAACGATAAATCTATAATGAATGATATGAGTCAGTATGTG
GTCCGACATGGAGACTATGTAGTGTGTAATATTAATTATAGATTGCTCAAAGACCAGAACAATACCGTT
ACCATGGACCAGATAGTGGAAGATGCCTTCGGAGCACTGCTATGGGTCAGAGAGCATGTCAGTCAATAT
GGAGGTGATCCTGACCGGATAGCCGTATCCGGTGATAGTGCCGGGGGGCACCTGGCGGCCATGATCGTA
AATGCCGGTCCTAATTTAAGTGAGTCGGGCCTTAAAGATGGAGAGAAGGGATTCACGCCCAGCTACATT
CCTGAAGGGATGGGTCTGGATGACATAAGGGATAAGGGCTGGATGGAGGTTCAGGGGGCAGTATTGAAT
TACGCCGCTTTAGATATATATACCTTGGCGAGGAAGGGATATGAAGAACAAAACAATAGATTTTGGCAG
GGAGCAGGAGTAGAACCCCGTGGCATTTTCGGCGATGATTATAATGTAGAGGATAATCCTGAGCTGTAT
CAGGCGGTGTCTCCCGTTCATACTCTCCCAAGCGCTACAGAGAGAAGATTGCCTCCCCAACTACTGACT
GTTGGAACAGAAGATGAGCTTATACCACCGACACAAATCAGAGAGTATAAAAGGCTTTTGGAAGAGGCA
GGGCATCCGGTAGAGTACTGGGAGCACGAAGGTAGACCTCATGCTTTTCTGGATTCAGGAGAAAATCCT
CGTTTGGGTATCAGCTTTGAAGAGGATGCCCCCGAGGCTTTGGATAGGATTATAGCTTTTCTAGATGCT
ATTTTTTACCAAGGATCTCATCACCATCACCATCACTAA

13) SEQ ID NO:25

ATGAAAAGGAGGCAATTGATTATGAGTCTTCATCCTCAAGCAAAAGCTTTGCTTGCGGTGCTT
GAATCGAGCCCGCCTATCGAACAGCAAACCGTTGAAGAAGCCAGGCAAGCATATGCGAAAATGTGCAGT
TATTTTAATAAACGTGAAGATGTTTATAGAGTAGAAGACAGGCAAATAAAAGGATTCCAGCAGAATATT
TCGATACGCATCTATACCCCAGACGATAAAGCCATTCATCCGGCGCTCGTTTATTTTCATGGCGGCGGC
TGGGTTATCGGCGACTTGGAAACCCATGATGCCCTTTGCCGTTCTTTGGCAAATAAATCTGGAGCGGCT
GTTGTTTCAGTGGATTATAGCCTTTCACCCGAATATAGATTTCCAGTTGCCATCGAAGATTCGTATCTG
GCAGTTAAATGGGTGGCAGATCATGCAGAAGAGCTTGGAATCGACAAAACCATGATTGCCGTTGGCGGT
GACAGTGCAGGCGGTAATCTCGCGACTGTCGTTTGCCATTTAGCGAATCAAAGAAAAGCCCCTGCCATC
AGCTATCAAATGCTGTTTTACCCTTCAACCGGATATGAGCGAACACCGTCTATCGATAAATATGGTGAA
GGTTATCATCTTACGAATTCAACAATGAAGTGGTTTCAACAACAATACTTATCCCAACCTGAAGACCTG
CGTAACCCCTTGGCCGCCCCTATGTTACTGTCAGAATCTGAAATTGCCTTATTACCGCCAGCTTATGTC
TTAACTGCCGAATATGACCCCCTATGTGATGGCGGAGAAATGTACGCCAAAAAATTGGAACATGCGGGA
GTAGAAGTAACATACGTCTGCTACCCGGGAATGATCCATGGCTTCCTGACGATGTCGGAGCCGTTGGAT
GATTGCAAGCGAGCGATTAATGAAGCGGCACAAAACTTGAAAGCCCATTTTACAAAGACAGTTGCCGTA
AACGAGGGATCTCATCACCATCACCATCACTAA

**14) SEQ ID NO:27**

ATGGGAGAAATCGTGAAACGAATCGCAACCGCCGTGTTTTTGCTGCATGCCATGACGTCGGTG
GCCGTGTGCGCCGATGTCGCGAGCGACATCCAGAAACAGGTATGGGGACGCTACCTGGAAAAGAGCGAA
GAAGCCGCGCTCACGGCAATGATGCCGCAACAAAACCCGCCGCCGGAAACCCCCATTGACAAATCGGCC
CGGGAACGCATCGGCGAAACCAGGAAAGCATTCCTGAATATGACGGCGGAGCTTGCGACTTCGCTGGCG
GACGTTCCGACGGCGGAGTATGACCAGGAGGGTATCCGGGGAAGCTGGATCATGCCCGATAAAGCGGAT
TCCGAACGCGTGCTTTTGTATCTTCACGGCGGCGGTTACATAGTTGGAAGCGATGAAACGCCTCGCGCG
ATAACCGCATTTCTGGCGCGCGAGGCAAAAGTGCGGTGTTTTTCCCTGGATTATCCGCTTGCTCCCGAG
CATCCATTTCCGGCTGCGCTCGATAGCGCGGTGCGATCGTATGAAATGCTGTTGGAAAAGGGGTTCAGC
CCCGGAAACATCGTGCTGGGCGGCGACTCGGCCGGCGGCGGCCTGGCGCTTGCCCTGTTGCTCGCCATC
CGGGAACACGGGCTGCCGATGCCGGCGGGAGCGTATCTCCTTTCCCCGTGGACTGATTTGACGCAGAGT
TTTCCCACGCATTTACGCAAGGCCGATGTCGAAATCAGTATTGCCCCCGAGCTGCTCGAGGAAGCGGCA
GCCAGCTATGCGGGCGATTCCGATAGAACCAATCCGCTTATCTCCCCCGCATTCGGCGAGTTCCGGGGA
TTCCCGCCGCTCCTGATACAAGTTGGTTCCCATGAACGTCTGTTGGACGATTCCCTGACGGTGGCGCGC
AATGCCGCCCTCGCCGATGTGCCGACGACATTGAAGGTATGGCCCGGCTATCCCCATGTGTTTCAACTC
TATCATCAGAATCTCGACGGCGCCAAAAAGGCGTTGAGGGAAGCCGCGGAATTCATCACCGGAGCGATG
GATAAAGAGTTGCTTCAAGGATCTCATCACCATCACCATCACTAA

**15) SEQ ID NO:29**

ATGGATGACCGGTGGATTGGCATTCCCGCCAATACAAGCAAGTGCATTGCGACGGAAAAACGT
TGTCGGAATTCGATGGGAGAAATCATGAAACGAATCGCAACCGCCGTGTTTTTGCTGCATGCCATGACG
TCGGTGGCCGTGTGCGCCGACGTCGCGGGCGATACCCAGAAACAGATATGGGGACGCTACCTGGAAAGG
AGCGAAGAAGCCGCGCTCACGGCAATGATACCGCAACAAACCCCGCCGCCGGAAACCCCCGTTGACAAA
TCGGCCCGGGAACGCATCGGCGAAACCAGGAAAGCGTTCCTGAATATGACGGCGGAGTTCGCGACTTCG
CTGGCGGACATTCCGACGACGGAGTATGACCAGGAGGGTATCCGGGGAAGCTGGATCATGCCCAATGAT
GCGGATTCCGAACGCGTGCTTTTGTATCTTCACGGCGGCGGCTACATAGTCGGAAGCGATGAAACGCCC
CGCGCGATAACCGCATTTCTGGCGCGCGAGGCAAAAGTGCGGTGTTTTTCCCTGGATTATCCGCTTGCC
CCCGAGCATCCATTTCCGGCTGCGCTCGATAGCGCGGTGCGATCGTATGAAATGCTGTTGGAAAAAGGG
TTCAGCCCTGGAAACATCGTGCTGGGCGGCGATTCGGCCGGCGGCGGCCTGGCGCTTGCCCTGTTGCTC
GCCATCCGGGAACACGGGCTGCCGATGCCGGCGGGAGCGTATCTCCTTTCCCCGTGGACTGATTTGACG
CAGAGTTTTCCCACGCATTCACGCAAGGCCGATGTCGAAATCAGCATTACCCCCGAGCTGCTTGAGGAA
GCGGCAGCCAGCTATGCGGGTGATTCCGACAGAACCAATCCGCTTATCTCCCCCGCATTCGGCGAGTTC
CGGGGATTCCCGCCGCTCCTGATACAAGTTGGTTCCCATGAACGTCTGTTGGACGACTCCCTGACGGTG
GCGCGCAATGCCGCCCTCGCCGATGTGCCGACGACATTGAAGGTATGGCCCGGCTATCCCCATGTGTTT
CAAGTCTATCACCAGAATCTCGACGGCGCCAAAAAGGCGTTGAGGGAAGCCGCGGAATTCATCACCGGA
GCGATGGATAAAGAGTTGCTTCAAGGATCTCATCACCATCACCATCACTAA

**16) SEQ ID NO:31**

ATGACGTTTGTTTTGACGATACTGGGCATCCTGGCTGCGGTCGTTGGCGTGTTTCTGCTTCTC
GCCTGGCTTTTTCGTCGCGGCGAGAATCTGTCCCGTTACGACACGCCCGTCGATCCGGCCGCGCTCGAA
TCCTGCGGTGATCCGGACGGCCCCAGCGCGGGCCATGCCGGCGCCGTCGCGGCGATCGAAAAGCTGCGC
CCGCAGGCCGAAGGGCTCTCCCGCGGCGGAATGATCCGGTTCGCCCCGGCAATTCATGGAGGATATACCG
ACCGGCCGTCGGTTCGATTGCGAGTTTCGGCCGGTCGAAGCCGCTGGCGTTCGCTGCGAATGGGTGCTG
GCGCCCGGCGCGGATCCGGAACGCCGTGTACTTTACCTGCACGGCGGCGCATTCATCGCCGGCAGCCCT
CACAGCCACCGAACGGCGACCTGCCGGTTTTCGACCGTCGCCCGAGCGGCGGTGCTGGCGGTCGACTAC
CGACTGATGCCGGAGCATTCGCGGCAGGACCTCATTCAGGACTGCCAGGCGGCATACCGCTGGCTGCTC
GACCACGGGCCGCACGGGCCGGCTGCGGCTCAACGGGTGTATTTCGGCGGCGACTCCGCCGGCGGCAAT
CTCGCGCTGACGCTGTCCAACTGGACGCGGGACGCCGGGCTGCGCCAGCCCGACGCCGTCGTGGCCCTG
TCGCCGTTGACGGATTCGACTTACAGCAGCCCGAGCATCCGCGGCAACCTGGCCACCGATACCCTGATC
TCGACCCTGTTCGGGCGGCTGGCGCGCGTGCCGCAAAGCGTGCTGACCTGGATGTTCGTGCTGGATAAC
CGCATCCGCCCGGTCGATCCCCTGGTCTCGCCGTCGCGGGGCGACCTGTCGGGCCTGCCACCCACGCTG
CTTCAGGTGAGCGAATCCGAGATGCTGTACGACGACGCCCGTCGCTACGTGAACAAGGCCGTGGCGGCT
GGTTCACCGGCCCGTCTGCAGAGCTGGCCGGGACTGCTGCACGTCTGGCAGCTGTTCTACCCCGAGATC
CCCGAGGCCGGGCAGGCCTGGCGCCGGGTCGGCGAGTTCATCGAGGCGGTCGACGCGGAAAGCGCGGAT
CTCATCACCATCACCATCACTAA

**17) SEQ ID NO:33**

ATGCCCCTAGATCCTAGAATTAAAAAGTTACTAGAATCAGCTCTTACTATACCAATTGGT
AAAGCCCCAGTAGAAGAGGTAAGAAAGATATTTAGGCAATTAGCGTCGGCAGCTCCCAAA
GTCGAAGTTGGAAAAGTAGAAGATATAAAAATACCAGGCAGTGAAACCGTTATAAACGCT
AGAGTGTATTTTCCGAAGAGTAGCGGTCCTTATGGTGTTCTAGTGTATCTTCATGGAGGC
GGTTTTGTAATAGGCGATGTGGAATCTTATGACCCATTATGTAGAGCAATTACAAATGCG
TGCAATTGCGTTGTAGTATCAGTGGACTATAGGTTAGCTCCAGAATACAAGTTTCCTTCT
GCAGTTATCGATTCATTTGACGCTACTAATTGGGTTTATAACAATTTAGATAAATTTGAT
GGAAAGATGGGAGTTGCTATTGCGGGAGATAGTGCTGGAGGAAATTTGGCAGCGGTTGTA
GCTCTTCTTTCAAAGGGTAAAATTAATTTGAAGTATCAAATACTGGTTTACCCAGCGGTA
AGTTTAGATAACGTTTCAAGATCCATGATAGAGTACTCTGATGGGTTCTTCCTTACCAGA
GAGCATATAGAGTGGTTCGGTTCTCAATACTTACGAAGCCCTGCAGATTTGCTAGACTTT
AGGTTCTCTCCAATTCTGGCGCAAGATTTCAACGGATTACCTCCAGCCTTGATAATAACA
GCAGAATACGATCCACTAAGGGATCAAGGAGAAGCGTATGCAAATAAACTACTACAAGCT
GGAGTCTCAGTTACTAGTGTGAGATTTAACAACGTTATACACGGATTCCTCTCATTCTTT
CCGTTGATGGAGCAAGGAAGAGATGCTATAGGTCTGATAGGGTCTGTGTTAAGACGAGTA
TTTTATGATAAAATTTAA

**18) SEQ ID NO:35**

ATGAGAGGATCGCATCACCATCACCATCACGGATCCATGATAAAAAACTTCGACAGAGAAAAT
TTTAGCTTAGTACTTTCCGGTGGTGGTGCTTTGGGTATTGCTCACTTGGGTGTACTGCATGACCTTGAA
AAACAAAATATTGTACCAAATGAAATTGTTGGTACAAGTATGGGTGGTATCATTGGTGCATCTATGGCT
ATTGGGATGAAAGAGAAAGAAATACTCGAAGAAATCAAAAACTTTTCCAATGTCTTCAACTGGATAAAA
TTCTCTTTTTCCGGTAATTCTGTTGTCGATAACGAGAAGATCGCTAAGATATTTGATACTCTTTTTAAA
GACAGAAAGATGAAAGATACGGTGATCCCTCTTAAACTCATCGCTACAAACTTACATAATGGGCATAAA
AAAGTATTTACTGCTTCGGATGATGTACTGATCAAAGATGCAATACTCTCAACAATGGCAATACCCGGT
GTATTTGAAGAACATATTATTGATGGTGAAACCTATGGCGACGGTTTTCTTTGTGAAAACCTTGGTGTG
AATGAGGCAACATTCAATGATGTTTTAGCTGTAGATGTCATGGGTGAGAACTCTTTTGAAAAAGCAATG
CCGGACAACTTCTTTAAAACATCAAATGTTTTAGAAATGTTTGAAAAATCAATGCGACTTCTTATTTAC
AACCAGACACAGACACATATTAAAAATGCAAATAAAAATATTTATCTTATTGAACCCGTTACAAAAGAG
TATAAAACATTTCAATTTCATAAACATAAAGAGATACGTGCTTTAGGCTTGGGTTTACTGTGA

**19) SEQ ID NO:37**

ATGAAGCTCGACGACGAAGCCGCCATGGTGCTGGACCTGATGAAGAAATCGGGCCGGCCG
GCGCTGGAGACGCTCGACGCGGCGGCGGCGCGCGCCCAGGCCGACGGGCTGTCGGAGAAA
AGCGAGATCGATCCGCCGGAGGTGGCGAAGGTCGTCGACGGCACGCTGCCCGGACCCGGC

GGCCCGATCCGGTTCCGCCGCTACTGGCCGATCGGGGCCGGCGGCGCGACCCTGCCGACG
CTGATCTACTACCACGGCGGCGGCTTCGTGATCGGCAATCTGGAGACCCACGACTCGACC
TGCAGGCGGATCGCCAATGTCAGCCAGTGCCAGGTCGTCGCCATCGACTATCGCCTCGCG
CCCGAGCATCCCTTCCCCGCGCCGGTCGACGACGCCATCGCCGCTTTCCGCCACATCGCC
GCCAATGCCGCGCAGTTCGACACGGAAGCCGATGCGCTCGCGGTCGGCGGCGATTCGGCC
GGCGGCAATCTCGCTGCGGTGATCTGCCAGGCGCAGAGGGAAACGGGCGGCCAGATGCCC
TCGTTCCAGCTGCTGATCTACCCGGCCACCGACAACCGCAACGAAAGCCCGTCGCGACGC
ATGCTGGGCGACGGCTATTTCCTCACCACGGCGCTGATGAAATGGTTCTTCGACCACTAC
GCCCCCGCAGGCGTCGATCGCACCGATCCGCGCATCTCGCCGCTGTTCGCCAAGGACTAT
TCCGGCCTGCCGCCGGCCTTCGTGCTGACCGCCGGTTTCGATCCGCTGCGCGACGAGGGC
CGCGACTACGCCAACCGGCTGATCGACGCGGGCGTCAAGGTCACCTATTCCAACTATCCC
GGCACCATCCACGGTTTCTTCTCGATGACGCGTTTCCTCAAGCAGGGCTTGCGCGCCAAC
GACGAGGCCGCCTGCGCGCTGAAGGTGCATTTCGGCATCTGA

**20) SEQ ID NO:39**

ATGGCAAGTGAAGCACTGACGATGATCGTGAACTTGTTGCGCAGCCAGCGCCCGTTGCAG
GAACCGACGGTGGAACAAATGCGTGCGGGGTTGGAGGCGATGGCGCAAATGTCGCCGCTA
CCTGCGGACGTGGAATTGACCACCGTGGACGCAGGAGGTGTGCCTGGTGCCTGGGTGCGC
GTGCCCGAGAGCGACCCCGACCGCGTCGTGCTGTACCTTCATGGAGGAGGGTACGTCATT
GGGTCGATTCGGACACATCGCGACCTTGCTCAGCGAATTGCCCGCGCTGCCCGTTGCCGT
GTGTTGTTGATCGACTACCGGCTCGCACCCGAACACCCGCATCCGGCCGCGGTGGAAGAC
TCGACGCGAGCGTACCGATGGCTCCTCGAGACCGGAAGCGCCCCCAAACGCATGGCCATT
GCCGGCGATTCGGCCGGCGGCGGGCTGACGGTGGCGACACTGGTCGCACTGCGAGACGCG
GGAGTGCCCCTGCCCGCTGCCGCCGTATGCTTATCGCCTTGGGTCGACTTGGAGGGAATC
GGCGAGTCGATGACCACGAAGGCCGCAGTCGATCCGATGGTCCAACGAGAGCCGTTGCTC
CGCATGGCGTCGATGTACCTGGCGGGTCAGGACCCGCGCACGCCTCTGGCGGCTCCGTTG
TACGCCGACTTGCGAGAGCTGCCGCCCTTGTTGATTCAGGTGGGTACTGCGGAAACCCTT
CTCGACGACTCGATGCGCCTGGCTGAGCGCGCACGCGCTGCGGGCGTACAGGTGACGCTC
GAACCTTGGGAAGACATGATTCACGTATGGCAGGCGTTCGCGGCAATGCTTCCCGAGGGC
CAGCAGGCGATCGAGCGGATTGGCGAGTTTTTACGCCAGCATTGGCAGTAA

**21) SEQ ID NO:41**

ATGAAGGCAAGCTGCCGCGACATCGAAATCGAATACGAAACATTCGGCCATCCGGACGACCCG
GCCATCGTGCTGATCATGGGACTCGGTGGCCAGCTCATCCTGTGGCCGGAAGCGTTCTGCCGCATGCTG
GCCGACGCTGGTCACTACGTGGTGCGCTTCGACAACCGCGACATCGGCCTGTCGACGCATCTCGATCAC
CTTCCCCGCCCCAACCTGCCGCTCGCCGCGCTCCGCCAGGCGCTGCGCCTGCCAGTTCGCGCCAGTTAC

ACGCTCGACGACATGGCGGACGACGTCGCGGGCCTGCTTGATGCACTGAACATCAAGCAGGCGCACGTC
GTCGGCGTGTCGATGGGCGGCATGATCGCCCAGCTGCTGGCCGCACGGCACGCGACCCGCGTGCGCAGC
CTGACCTTGCTGATGACCACCAGCGGCGCGCGCAACGTTCCGGGCCCCTCACTCGGCATGCGCATGGAA
ATGATCCGTCGACCGCGCGACACCTCGCGCGAGGGGCTGATCCGCCATGGTATGCGTACCTGGCGGATC
ATCGGCAGCCCGACGTACCCGAAGCCGGAAGCCGAACTGCGCCGCATCGTCGCCGAGGGCTTTGACCGC
GCGTTTCACCCGGCCGGTTTCATGCGCCAGCTGCACGCCGTTCTCGCGGCACCGAGCCGCGCACCCCTG
CTGCCGCGCATCAAACAGCCGGCCGACGTCATTCACGGCGACGCCGACCTGCTGGTACCAGTGGCAGCG
GCACGTGATCTGGTGCGCCGCCTGCCGAACGCCACGCTCGACATCGTGCCGGGCATGGGGCATGACTTC
CCGACCGAGATCATGCCGCGTATCGCGCGCCGCATTGTCGAAACCGCGGCACGCGACCCGCAGCGGCTC
GCCGCCCATCACCATCACCATCACTAA

22) SEQ ID NO:43

ATGAGGATCCGTGCGCTGACCACCTGCTTCGCCCTGCTCGCCGCAGGGCTGCTCCTGTCGCCA
CCCGCGATGGCGGAGGGTGACCCCGAGTACAGCGTCCCCCTGGAGCTGCTGCGCGGCTCGGTGGACTGC
AATGCCGAGTTCGTCCATCTCGACCGGGAGCCGGTCCTGCTCGTGCACGGGACGTCCGGCACGCCCGAG
GAAGCCTGGGCCTGGAACTACATCGAGAACCTCCCCCGCCAGGGGTTCGACGTGTGCTGGGTCCGCCTC
CCCAACCGGGCGCTCAGCGACCAGCAGGAGTCGGCAGAGTACGTGGTGTACGCGCTGCGACTCATGCAC
GAGCGGGCGGAGGGACGCAAGATCGACGTGATAGGCCACAGCCAGGGTGGGCTGCTGCCCCGCTGGGCG
CTGCGTTGGTGGCCGAGCCTGCGCGACATCGTGGACGACTTCGTCCCGCTCGCCGCGCCCGCCCACGGC
GCCAGCGGCGCAGAGATGTTCTGTCCGACGAGCTGCGCGCCCGCGGTCCAGCAGATGAAGCCCGACTCC
CGCTACCTCGCCGCCCTCAACTCAGTGGACGAAACCCCCGGCGACGTGGACTACACGAGCATCTACAGC
ATGACCGACGAGCTCGTGCAGCCCTACACCAGCCCTCCGCTGGAAGGCGGGACCAACATCGCGATCCAG
GACGTCTGTCCCGGCCGGGTCGTGCATCACTACGGGATCGTCTACGACGCGGTCGCCTACGCCCTGGTG
GTCGATGCGCTCGTCAACCCGGGCCCGGCCGACCCGGCCCGGCTCCCCGCCGACATCTGCCTACAGACG
TGGATGCCGGGTGTGAACGAGATCGACGTCGTGGCCGGGAACATGATGGCTTACGGGAACGCCTACCTG
GCCTTCGGTGCCTACCCACCCAGCGATTCCGAGCCCGAGCCGAAGCCCTACGTGGACGAGGACCATCAC
CATCACCATCACTAA

23) SEQ ID NO:45

ATGAGAAAAGAAAAACCGGAATATGGCATTGTGCTGAGTGGTGGAGGCGCCCGCGGAATTGCC
CACATTGGTGTGCTAGCTGCACTGGAAAAGCACGGGATCAGTCCGGGTGTCGTTTCCGGCTCTAGCATG
GGCGCCATCGTAGGCGCATTATACGCCGGGGGGGTGAGCACTGAAGAAATGCTTGAAATCGCCCGGAAC
CGGAAGCTTAACGACCTCTTCGAATGGTCGTTCAAAAAGCATGGCGGTATGCTTTCATTAAAGATGCTC
ATGCAGGTTCTTGAAACACATATCCCTGAAAATTCTTTCGAATCGTTAAAGAAAAAGTTATTCATTACC
GTATCCAATATTTCATCCGGCCAGGAGGAGGTCATTTCAGAAGGTAAGTTGTACCAGGCAGTGGTAGCA
TCGGCTTCTATCCCGATCATTTTTGAGCCTCAGCTGATCAATGGCCAGACTTATGTCGATGGCGGCCTG
TTCAACGATCTGCCGGTTGATCCACTGATAGGAAAGTGCAGGAGTATCATCGCTTCACATGTGAACTAC

AATGGTCCGAATCCTGATCTGACCAGTATTCGCGCCATCGCAGAACGGGTTTACCGCCTGGCTATTTAT
CAACATGTACATAAGAATTTCAGCAAATGTGATTATATTATTGACCCGCCCGCGTTGAGGGATCATAGC
ATTTTTGATTTCAAGCATATCGACCGGTTGTTTGAGATCGGGTTTGAAGCGGCTGAGATCCTTATTACC
CAGATGCAGGAACCATCAGTTGAAAAACCGCTTATAATCCGGGAAATTAAGTCCACTAAAAAGAGATTA
AAACATCACCATCACCATCACTAA

24) SEQ ID NO:47

ATGAAGGAAACATATCCTGAGTATAGGGTTGAACCTTTTGAATTGGGTGATTCTGAAAGCAAA
GTAGGTTGTCTTCTTTTTCACGGCTACACGGGGTCTCCTCTGGACTTGTTCCCAGTGGCCAAGATATGC
GAAAAGTATGGGTGGCATGTTAGGGCTGTTAGGCTTCCGGGCCATGGAAGACCCATTGAAGAACTAATC
AAGGTCAAGCACACTGATTGGTTGCGGGCTGCTGAAAAAGAATACGAGGAATTCGCCAAAAACAAGGAA
AGAGTTATTTTAGTTGGTCATTCCTTGGGTAACGTCTTGATTTTTCATCTCGATGTAAAGTACCGGGAA
GAAAAGAAGATAACCAATCTTGTTTCTATTTCGCCCCCGGTTGTTATTAGGAAAGGCTGGATGCTTCCT
TTTGTTGGTTTGGCAAAGATATTTGTTAGGCACATAGATTATTCTGAGATTGCGTTTAAAGACAAGAAA
ATAATGCAACACCCGTTGTATGACTACTTGAGGTTCACGTACAAGAAATTTCCTTTAGGTATTATTAAG
GAGTTAGGGAAGGCGGTTGAGTACGGTGTCAAGCTTGTGCCTTTGCTCGAGACTGATGTCATTGTTCTT
TTTGGTAAGGAAGATGATGTCGTGGATCCATTGTCGGGATACGAAATCTTGATGAAAGCAAAGAGCAGG
CACAAGTCCTTGGTGATGCTTGAGGGAATGCATGTGCCAATGCTTGACATTGACTATAAAGCGTTTCAA
GAAAGCCTGATCTCGTATTTCAATGGCATAAGCGAGAAAAAACTTGGCTTTGAAGATACTTTCGACATT
TTTGATTTGTTTGATGAGGCCGCCCGGATAACTAATATTGCTCTTGGTAAGCTAAAAGGTCATCACCAT
CACCATCACTAA

25) SEQ ID NO:49

ATGTTCTGGTTGGCACTTATCTGGGCCCTGCTGGCCCTGAATCTGTATTACCCCTGGAAGGGC
AAACCCGAGGTCGGTGGCGTGATCAGCTTCGCCTTCGGGCTGTTGGTGGGCGAACTCGCCTTGCATGTG
ATTGCACTGCAAGTCCTCACTACCCTGCTGTTTATCACCTTCTGCGGCCTCTCGGGCTTCGGCGATGCG
TTGGGCCTGATGCTCGCCATGGCCACCTGGCTGGCGATGGCCTGGTTCTACGCACGCAGCGAGCGCGCG
GGGCCGGCGATGGAGAACGGCATTCGCCAGGCCCTGGGCGAAGACTATTCAAAACTGATTTCGGCCGAG
CGCAGCGACTGCCTGCACCGCGACATAGATTACCGGCAGCTGCTTAACCCCTTTGCCTTCAGCAAACCC
GAAGTGACCCGACACCGCAATCTGCCCTACGCGGAAGTGGACGGCAAAAACCTTCATCTGGATATCTTC
CACCACCGCGACAAGCCACAAAACGCGCCAGTACTGCTGCAAATTCACGGCGGCGCCTGGCTGCAGAAT
CTGGGCAACAAGGATCAGCAGGCACTGCCGCTGATGAATGAACTGGCCAGCCGCGGCTGGGTTTGCGTA
ACAGTGCAATACCGGCTGAGCCCCGGCGTCGCCATGCCCACCCATATTATTGACTGCAAGCGCGCACTG
GTCTGGGCGAAGGAGCATATCGCCGATTACGGTGGCGATCCGAACTTCATTATCGTCACCGGCGGTTCC
GCTGGCGGCCATCTGTCAGCGATGGTGGCGCTCACCGCCAACCAACCCGAATTCCAGCCGGGTTTCGAG
GACACGGATACCCGCGTACAAGGTGCCGTACCTTTCTACGGTATCCACGACTTTACCAACAGCCGTGGT
CAGCGCGAGCATAAGGGGCTGGAGAACTTCATCGCGCAACGCGTAATGAAAGTGACCCGCTACGATAAC

CCCAAACTGTGGGAGCAATGCTCTCCCCTGTTTCAGGTAAAAGACGACGCGCCTCCAATGCTGTTGGTA
CACGGCGAGGCCGATACCCTGGCACCGATCAGCGAATCGCAGGCCTTGCTGGAGGTTTTGCGGGAACAC
GACGTCAAAGCGGGTCTGGCGGCACTCCCCGATGCGCAGCACGCCTTCGAACTGATGTACTCGCTGCGC
ACCCTGTATATGATTAACGCCGTGGAGCGCTTCGGCAACGCGCTGCACGGCAAGTATCTACAGGATGGC
GCAGCTCATCACCATCACCATCACTAA

26) SEQ ID NO:51

ATGACTGAGGCCACGCAGGCTGTGCCCGAAGGCAAAGGCGTGCCGATCCCGCACGCTGATCTG
GGGGCTCGAGGAGTGCTCGACCCCCAGGTGGCTGAAGTTCTGCGCCTGCATGCCAAGGCGGGACAAGCC
CCGTACGAGACCATCGGAGCCAATGCCGCGCGCAAGGCTTTTGACAAGGCCAGCCGTGTGCTCGAGATT
GGCTCACGCCCCGTGCATCGGATTGATCATGTCGAGATTCCCGGGCGTGCCGGGCCGGTGCATGGCCGG
CTGATCTGGCCCCATGAACCGAGCTGGGTCGATCGACTTCCTGTGCTGCTGCTGTTGCACGGCGGCGGT
TTCACGGTGGGCTCTTCCGCGACCATCGAACACATTGCGCGCATGCTCGCCATGGAGGTGCATTGCGCC
ATACTGGCGCTGGACTACCGTCTGGCGCCCGAGCACCGGTTTCCAGCCGCCTTCGAGGATGCCTGGGAT
GCGCTGGTCTGGCTGCATGAGGAAGCGGGCGCACTGGGGCTCGACGCTTCGCGCATTGCCGTTGGAGGC
GACTCGGCCGGGGGGACCCTGGCGGCCGCCTGTGCATTGCAGGCCCGCGACGCAGGCTTGCCATTGCGT
CTGCAGTGGCTGGTCTACCCGGGGACCGGCGCGCACCAGGACACCGCCTCTCACCTGCGTCTGGCTGAA
GGCTACCTCATCACACGCAAGACCATTCTCTGGTTCTTTGAGCAATACCTCGCCCACCCGGCACAACGT
GAAGACTGGCGCTTCGCCCCCTTGCTCGCAACTGACCTCCGCGGCCTGGCGCCGGCCTGGATAGCGGTG
GCCGAATTCGATCCGCTGGTCGATGAAGGCGTGGCATATGCACGGCGCATGCAACAGTCGGGGGTGGAA
GTCCACCTCGATTTCTATCCGGGCATGGTGCATGCGTTCTTCAACATGGGGGGCTATGTCACCATGGCC
AGGCGCGCACATGCCCAGGGTGTGGCAGCATTGCGCGCAGCATTCACGATTCAGGGCGGATCTCATCAC
CATCACCATCACTAA

27) SEQ ID NO:53

ATGAATCATGACGGCCCGGCGTGGCCGGTTGCAATAATCCCCGGGCTTTGCAGTTCTTGCGAC
TTGAAAGGGTGCAACCGAATAGATTCCATTCCAAATATTGCTATTATGAAAAAACTTGGACTTGCATTG
GGCGGCGGCGCTGTGTTGGGGGCTGCCCATATCGGTGTTCTGGAAGCCCTGGAGGAAAGGGGTATCAGG
CCCGAATATATCAGCGGAACCAGCATTGGCGCTTTTGTCGGTGCTCTCTATGCTTTCGGCAAGACGGCA
GCCGAGCTCAAAGAAATCGCCCTGGAATTGGACTGGCTCGATGTCACCAATTTTAAATTGTCGAAAATG
GGGTTGTTGGGGAATGACAGGATCGGTAAACTGATTCTTGATAACCTGGGAAAACAACAGATTGAAGAT
TCTCAAATCAAACTCGCTATTGTAACAACGGATATCTCAAATGGGGAAAAAGTGGTGCTGACCGAGGGT
CCGCTCTTCAAGGCTGTTATGGCCAGTTCTTGCCTGCCGGGCATATTTATTCCGGTTGAATGGGGGGAT
CGCCTCCTGGTTGACGGGGTCTTAAGCGAAAATGTGCCGGTATCACCTCTCCGGGATATGGGGGCAGAA
GATATTATTGCCGTTGACCTTACAACAAACAGGGACTTTAAACGTCCCAATGATATCATTGATGTACTG
TCCAATACGTTTGCTATTGGCCTCAATAAAATTGTTATCGAACAACTCGACGATACGCGAACCATCATG
ATTCAACCGAAACTGAGCGCATACAACAAAGCGGATACCCGAAAAATGAAGGCGTTGATCAAAGAAGGG

TATGAGGAGACATGCCGGATTTTGGATCAGCAGCAGCTGAACTCCCTGAAACACGGATCTCATCACCAT
CACCATCACTAA

Note: There is an internal site that is an alternative start site, this sequence is shown below

**ATG**AAAAAACTTGGACTTGCATTGGCGGCGGCGCTGTGTTGGGGGCTGCCCATATCGGTGTT
CTGGAAGCCCTGGAGGAAAGGGGTATCAGGCCCGAATATATCAGCGGAACCAGCATTGGCGCTTTTGTC
GGTGCTCTCTATGCTTTCGGCAAGACGGCAGCCGAGCTCAAAGAAATCGCCCTGGAATTGGACTGGCTC
GATGTCACCAATTTTAAATTGTCGAAAATGGGGTTGTTGGGGAATGACAGGATCGGTAAACTGATTCTT
GATAACCTGGGAAAACAACAGATTGAAGATTCTCAAATCAAACTCGCTATTGTAACAACGGATATCTCA
AATGGGGAAAAAGTGGTGCTGACCGAGGGTCCGCTCTTCAAGGCTGTTATGGCCAGTTCTTGCCTGCCG
GGCATATTTATTCCGGTTGAATGGGGGGATCGCCTCCTGGTTGACGGGGTCTTAAGCGAAAATGTGCCG
GTATCACCTCTCCGGGATATGGGGGCAGAAGATATTATTGCCGTTGACCTTACAACAAACAGGGACTTT
AAACGTCCCAATGATATCATTGATGTACTGTCCAATACGTTTGCTATTGGCCTCAATAAAATTGTTATC
GAACAACTCGACGATACGCGAACCATCATGATTCAACCGAAACTGAGCGCATACAACAAAGCGGATACC
CGAAAAATGAAGGCGTTGATCAAAGAAGGGTATGAGGAGACATGCCGGATTTTGGATCAGCAGCAGCTG
AACTCCCTGAAACACGGATCTCATCACCATCACCATCACTAA

**28) SEQ ID NO:55**

ATGAGAAAGAAAGAAGTGAGTATCAGGGGAAATCTGATTCGGGAAATGATGCGGGATGTGATG
GGAACGGCTCTCGGCAAGCCGATTCAGTCCGGGGAGATTAGAAGGCATCCGGTTGAGCCGGCCTGGATC
TGTCCGCCGGGATTTATCTATGAACTGATCGATATGGAACATTTCAAGATGGAATATCTGCAGCCTCAG
GATGTTTATACCGGGCGGGTGGTGCTTCAGCTGCATGGTGGCGGATATATTGGCCCGATGAAGAATATT
TACCGGAGCTTTGCTGTGAAATATTCAAGATTAAGTATGTATGGAGATGTTTTGACGATTGATTACCGG
GTGGCGCCGGAACATCCATATCCGGCGGCGCTGGAGGATGCGATTGCTGCGTATCGGTGGCTTTTGGAT
GAAAAAAACTATCGGCCGGAGCAAATCGTGGTAGCAGGAGATTCCGCCGGTGGCGGCCTGGCACTGGCG
TTGTGTCTGTATCTCCGGGATCATCAGATTCCGCTGCCGGCCGGTATCATAGCCATGTCGCCCTGGACG
GATCTGACACTAAGTGGGGAGTCTTATACATCTAATTATGACCACGATCCGCTGTTTGGTAATACCAGA
GAAAGTATGTTGTATAATTCAACTTATATTGGGGACAGCGATCCGACGGATCCGTATATGTCTCCGTTG
TTTGGAGAGTATTTTGGATTCCCGCCGGTGTTATTGCAGGTGGGGGATATTGAGGTGCTTCTCAGCGAT
ACACTTCGGGTTTCTGAAAAATTGCGGGAGGCTGGGGTAAAGCAGCGGCTTTCGGTTTATGAAGGCATG
TTTCATTCGTTTCAGATGGGACTGGATCTGATTCCGGAGAGCCGTGAAGCCTGGGAAGAAGTGGAACGA
TTTATCGGGATTGTCTGCCAGATTGAGCGGAAAGCTGAAGGGAAAATTGTTCGCCGGGTGAAGGGGAAA
ACAGAGAATAAAGATGAAAATCACATTGGTTACGGTTGGAAAAATAAAAGAAAAATTTTACCGGGAGGC
AATTTCGGAATACAGGGATCTCATCACCATCACCATCACTAA

**29) SEQ ID NO:57**

ATGTGGTGGGATTTTGTCCCATAGTCCTTACTTGTATTTTCCGTTTTACTCTTTACGCTTTA
CTCTTTACGTCTCAGGTTCTCAGTCATCAACGATCACAACTCTCTCACTCAATAAGGAGGATCATT**ATG**
CCACTCGACCCAAAAGCCAAAGCGTTTTTAGACCAGGCCGCTGCGGCTGGCGCGCCAGCACTGGACGCG
CTTCCCGTTCGAGAAGCTCGCGAGGCGATTCGCGGCCTGTTTGCGGCCATAGGAGAAAAAGAACCCGTC
AAACAAATAGAAGATCGCATGATCCCTGGCCCGGGCGGACAGATGCCTGTGCGTATGTATACGCCGGCA
GGTACCACGCCTTTTCCCGTCCTCGTTTATTTCCATGGCGGAGGATGGGTCCTGGGTGATCTAGAAACT
CACGATGCGACCTGCCGGGCGCTGGCAAATGCTGCTGGCTGCATGGTGATCTCGGTTGACTATCGTTTG
GCGCCGGAGCACAAATTCCCTACCGCTCCCGAGGATTGCTATGCGGCCACCAAATGGGCAGCGCTGAAT
GCGGCGGCTCTTGGCGGAGATCCAACCAAGATTGCCGTTGGTGGCGATAGTGCCGGAGGGAATCTTGCT
GCCGCAGTGGCACAAATGGCCAGTGACCGTGGGGCTCCTTCGCTCGGGTTTCAGTTGCTCATCTATCCA
GTGACCAATTACGCGTTCGATACCGCCTCATACCGGGCAAATGCGGAGGGCTACCTGCTCACAAAAAGC
GCGATGGGGTGGTTCTGGAACCATTATCTGCAGAACGAAACCGATGGCCAGAATCCCTATGCTTCGCCT
TTACGTGGACAATATCTGCGCAATCTACCCCCAGCACTTGTCATCACCGCGGAGTATGACCCGCTCCGC
GACGAGGGAGAAGCGTATGCAGCGCGGCTGCGCGAAGCAGGTGTTGCTGTCACTCACACTGACTACAAA
GGCATGATTCATGGCTTCTTCAGCATGACCAACGTAGTCGATCAGGCGAAGCAAGCCATCAATGAAGCT
GGTGCCGCACTGAAGGCAGCGTTTGCGGCGAAACGAGGATCTCATCACCATCACCATCACTAA

**Note:** There is an internal site that is an alternative start site, this sequence is shown below

**ATG**CCACTCGACCCAAAAGCCAAAGCGTTTTTAGACCAGGCCGCTGCGGCTGGCGCGCCAGCA
CTGGACGCGCTTCCCGTTCGAGAAGCTCGCGAGGCGATTCGCGGCCTGTTTGCGGCCATAGGAGAAAAA
GAACCCGTCAAACAAATAGAAGATCGCATGATCCCTGGCCCGGGCGGACAGATGCCTGTGCGTATGTAT
ACGCCGGCAGGTACCACGCCTTTTCCCGTCCTCGTTTATTTCCATGGCGGAGGATGGGTCCTGGGTGAT
CTAGAAACTCACGATGCGACCTGCCGGGCGCTGGCAAATGCTGCTGGCTGCATGGTGATCTCGGTTGAC
TATCGTTTGGCGCCGGAGCACAAATTCCCTACCGCTCCCGAGGATTGCTATGCGGCCACCAAATGGGCA
GCGCTGAATGCGGCGGCTCTTGGCGGAGATCCAACCAAGATTGCCGTTGGTGGCGATAGTGCCGGAGGG
AATCTTGCTGCCGCAGTGGCACAAATGGCCAGTGACCGTGGGGCTCCTTCGCTCGGGTTTCAGTTGCTC
ATCTATCCAGTGACCAATTACGCGTTCGATACCGCCTCATACCGGGCAAATGCGGAGGGCTACCTGCTC
ACAAAAAGCGCGATGGGGTGGTTCTGGAACCATTATCTGCAGAACGAAACCGATGGCCAGAATCCCTAT
GCTTCGCCTTTACGTGGACAATATCTGCGCAATCTACCCCCAGCACTTGTCATCACCGCGGAGTATGAC
CCGCTCCGCGACGAGGGAGAAGCGTATGCAGCGCGGCTGCGCGAAGCAGGTGTTGCTGTCACTCACACT
GACTACAAAGGCATGATTCATGGCTTCTTCAGCATGACCAACGTAGTCGATCAGGCGAAGCAAGCCATC

AATGAAGCTGGTGCCGCACTGAAGGCAGCGTTTGCGGCGAAACGAGGATCTCATCACCATCACCATCAC
TAA

**30) SEQ ID NO:59**

ATGGAGATAGGCATGACAGCATTTAATCAAAAAATTCAAACATTGCTAGAAAAGGGCCAAGGG
CCTGCTGCTCGAGCTCTTGATAAGTTACCTCGATTAGCTCAAGAATCACTTGCTAAAATTCTTGGATAT
TCTTACCAATATCCCGATCTCGACTCATTTACTAAATGTATGATGGCTGTACAAATTAAACAGGGTCAT
ATCGGTTTCATTGGTTCAGACCCAATTGAATCTAGAAGGCAGTTCGATACGCAAATGCTGGCTATACGC
CACAAGTCAACAGAGATCGACTTAGTAGAAGATATCCGTTTACCTCTCCAAAGTGGAACCGTTTTTGCG
AGACACTATCACCCAGCACCGAATAAGAAATTACCTCTTATTGTTTTCTATCATGGAGGAGGATTTGTT
GTTGGTGGTTTAGATACTCATGATGAGGTTTGTCGTATATTGGCAAAATATGCAAAAGTACAAGTCCTC
AGTATCGATTACCCTCTAGCACCTGAGGTTTCTCCTCAGCATTTAATCCAATCTTGTGAAGATGCTTTA
GCTTGGGTATATCAAAATCGTAGACAGTTAAAAATATTAAAAGGTCGAATTGCGGTAGCGGGGGATAGT
GCTGGTGGAAACATTAGTACAGTCGTGGCTCAAAGATCAGTAAATAAACCTTATGCTCCTCAGGCCCAA
CTTCTTATTTATCCTGCCGTCGATTTCAAGAGTCGACATCCTTCCTTCTATGCATATAACGAAGGATTG
GTTTTAACGGATAACGATATCAATAATGTCACTCAATATTATGCAACCCAGCATAACGTAGAACTAGAT
GATCCAATTATTTCTCCAACTTATGGCAACTTGAAGAAGAATCCTCCGGCTTTTGTGATTACTGCTGGG
CATGATGTATTACATGATGAAGGCAAAATTTATAGCTATAAGCTACGCCAAAATGGTGTCAAAATGCAT
TATGAAGAATATTCAGATCAAACTCATGGTTTTATAAATTTGACGCCTATTTCCAAAAAAGCCAAACGG
TACACCATTGAGTTCAGTAAAAACTTTAGAAAATTTTGGGATAAAAATAGCGGATCTCATCACCATCAC
CATCACTAA

31) SEQ ID NO:61

ATGTCAGGCAAGTACACTGTTTTGGAAGGCGCTGAACCGTTTTATTTTGAAGGAAGCAGAGTT
GGGATTCTTGTGTCGCATGGGTTTACCGGCAGCACCCAAAGCATGAGGCCGCTTGGGGAAGCCTATGCA
AAGGCAGGTTATACGGTTTGCGGCCCGCGTCTGAAGGGGCATGGCACCCATTACGAGGAGATGGAACAG
ACTACATATAACGATTGGATTGATTCAGTCGAGGAAGGCTACCAGTGGCTGAAGGAACGCTGCGATACA
ATATTTGTAACTGGTTTGTCCATGGGCGGAACCCTTACCCTGTATATGGCTGAAAATCATCCTGAAATT
AAGGCAATCATACCAATTAATGCAGCTATTCAAATTTCCGACATGGCCGCTGCAGCCAACCTTGATGGA
GTGCGATTCCTTGATGCGATTGGTTCCGACATTAAGAAGCAGGGTGAAAAAGAGCTCGCTTACGAGAAA
ACTCCTGTAAAGTCCCTTGGTGAGATCGTAGCGTTAATGGAAAAGGTTAAAGCTGACCTTTCCGATATC
ACTTGTCCAGCACTTATTTTTGTTTCAAAGGAAGATCACGTTGTGCCGCCAGATAACTCAAGGATTATC
AAGGAACAAATCCGTTCTGCCGATAAGAGCCTGATTGAGCTGTCAGAAAGCTATCATGTTGCCACACTG
GACAATGACCAGGAGATCATTATCAAGGAAACATTAAAATTCATTGAGGAACAGCTTGGGAAAGGATCT
CATCACCATCACCATCACTAA

32) SEQ ID NO:63

ATGAAATTTTTCCTGGAGAATCTTTTGCCAATCCAGGAAGGAGCTGTACCTACCCTTGAGGAA
ATTCGCGCAAGAAGTGCTGCAATGCCTTCGGGTACGGTGGAGGAGGTCCAGGAAGTGTCAGACAGACTT
ATCCCTGGCTCTGAATCCGACATCCCAGTTCGCATTTATACTCCTGAAGGAGAGGGACCATTTCCGGTA
ACCGTATTTTTCCACGGCGGAGGATTTGTATACGGGGGACTTGATACCCATGATGCGGTCTGCAGAAGC
ATCGTGAAGGCTTCTGGCCAGATGGTTGTTGCGGTAGAATACCGGCTTGCTCCGGAAAATCCGTTCCCT
GCAGCACCTGAAGACTGCTTTGCAGCCGCAAATTGGGTAAATGAGAATGCAGAAAAGCTAAACGCTGAC
CGAACTAAGCTAAGCGTCGCGGGAGACAGTGCCGGAGGGAATTTGGCAACTGTAGTCTGCCTGCTTGCG
AGAGAGAGGGGAGGCCTGTCTATCAGCAAGCAGGTTCTGATCTACCCTGTTACAGATAAATACCAGCCT
GGAAAATATCCGTCCTATGAAGAGAATGGAAGCGGCTATTTCCTCACAACTGACTCGATGGCATTATTT
GCAGCCCTTTACATTCAATCTGAAGAGGGACGTGACAATCCGCTTTCGGCTCCGATACTGGCCAAGAAC
TTAAGCGGGCTGCCGCCTGCACTGGTCATTACGGCCGAGTATGACCCACTAAGGGATGAAGGCGAAGCC
TATGCTGAAAAACTCCGTGACGCGGGAGTTGAAGTGATTTTAAAAAGGGAAGCAGGCCAGATTCACGGC
TTTTTCAATCTCTTCAGCATTATGGATTCCAAGGATGATTTAAAGGACGTATATGCCTATATCGGCGAG
TTCCTAAATAGTGAAATCGCCGCAGTTCTAGGATCTCATCACCATCACCATCACTAA

33) SEQ ID NO:65

ATGGTGCCAAAGCTAGAACCACGGAGGAAACAAAGAATGAAAACAACCATACACCCAAAGCTG
GAGCGAACGGATATCGTCCTGATGGATGACGTCGTCTATTCTCAGCCGCTGGCGTATTTTGGTAGAAGA
CCACACCCGCTTAAGATGATGCTCCTCCGCCCGATTGAGTGGTGGGGCGAGACGCAGCTCCTGCCGCTT
CCGACGATCCTCTGGGTCGTGGGCGGCGCGTGGCAGCAGACGTTTCCCTCCTGGCGCATGGCAGAGTTT
AGTTATTTGGCCTATGCGGGGTATCAGGTAGCGGCAATCGACTACCGAACGATCAATGAAGCGGCGTTT
CCCGCGCAAGTGGAGGACTGCAAAGCTGCGGTTCGCTTCCTCCGCGCAAACGCAAAACGCTATGGCGTA
GATCCGGAGCGCATCGGCATCATGGGCGACTCGGCGGGCGGCTATCTCGCTGCTATGATCGGTGCAACG
GGAGATGATCCCGCGTTTGAAACCGACGAATGGGCGGGATATTCCAGCGCGGTGCAGGCTGTTGCGGAC
TGGTATGGCCCTATCGATATGCTGCGTATGGGGCAGTATGCCAAGGAGCAAAACCCGGATGCGGGAGAA
GAGGACACGCTGCTCTTCCGCCTCTTTGCGGGCTGCCCGATCACGGAGGAGAACCATAAGTTTCTTGAG
AGCATGAGTCCGGAGCGCTATGTTTCGGCAAATACGCCGCCGCATCTGATTTTGCACGGCACGGCGGAC
GAGATGGTTGATGTACGCGAGAGCGAGCGTTATTACGAGGCGCTGACGAAGGCGGGCGTGCCTGCCGAT
TTGGTTCTGCTCGAAGGGGCAGGGCATTGCGACCAAGCATTTTCACAGCCGGAAGTGCAGAATATCATA
CTTGAGTTTTTCACCAAACATTTAAAGGGATCTCATCACCATCACCATCACTAA

34) SEQ ID NO:67

ATGAAGAAAAAGATTTTTACCATCGCGGGGGTTTTGATTCTCGTGCTAGTGGCATGGCAATTG
TGGCCGGAGATAAAGACATTACCAGCGGGCAGCGCTCCCACAGAAGCGATTCAAACGACCACCGGCGCG
CTGCGCGGCTTCACCCAAAACGGATTGGATACCTACCTGGGGATTCCCTACGCGGAGCCGCCGGTCGGC

AGTCTGCGTTTCAAATCGCCGGTGGCGCGCGAACCCTGGCAGGGCACGTTCGAGGCCTATGAATTTGGC
GCGTTCTGCCCACAGGCCTATGACCCGGTGGTGATTGATGATCCGCACGAAGACTTGAACAACGAAGAC
TGCCTGTACCTGAACATCTGGAAGCCCTCCGCCGCGCAGGAGAAACGCGCCGTCATGGTCTACATCCAC
GGCGGCGGATTCGTCGGCGGCTCGAGCAAGGAAGACCTCTACAACGGCGGCGTCATCGCCAGGGATGGC
GACGTGGTCGTGGTCACCGTGAACTATCGCGTTGGCATTCTGGGCTTCTTCGACTTTTCCGTCATCGGC
GGGTCGGAGTACACCGGCAGCGCCGACGCGGGGATCGAGGATCAACTCCTGGCGTTGCGCTGGGTCAAG
GACAACATCGCGGCGTTTGGCGGCGACCCCGAGAACATCACCGTGTTCGGCGAGTCGGCTGGAGGTGCG
AGCGTCCTGGCCCTGTTGGGCATAGACCATCCCCAGGAATTGTTCAAGCGCGCCATCGTCATGAGCGGG
TCACCTTTGCATACCGCCGAAAACAGCCGGGATATTGCCAACCTGCTCAAAACCGAAACCGGGATAACT
TCGTCGACCATCTGGCTCTGGGCGCCAACCCAGGCTGTGATGTACATTCAGGATCAGGTGTTGACAGCC
GTCGGCTCGCCTCTTTCGGATCTCATTTTTGCTCCCACCTACGGAAGCGGCTACGTGGTCAAGCAAAGT
CCGCTCGAAGCCATCGCCTCCGGCAACACCAAAGGCATTGACCTGATGATCGGCAACATGGCCGACGAG
TTGAGTTATTGGTCGTTCTATGATACTCCGGATAGTCACATCTGCGAACAGACTCTCAAGGATAACCTC
TTCACCATGATCAATCCGGACATCGAGCCGGAACTGAAAAAATTGTACGATCTGTATGTCAACGACCCG
GAGCGGGTCTGGAAAGAAGAAGGCGATATCATCTTGGCCATGGGCGATGACTATGCCTTCCGCGTCGAC
GCGTTGGACATAGCGTCAGCACAAGCTACCGTTGCCAAAACCTACTACTACCGCTTCAACTATCCGGTC
AACCTGCCGGAGCAGCCCTGTCAGGATCACCGCTCGCCTCATGGAGCGGAACTGCCTTTCGTGTTCGGC
AGTGTCAACACACAGACCGGGTTCGACTTCATCGGCAAGCCGCGCGACGAGCAGGATAATGCCGCTCGT
AACCGCCTCATGCAACAGGCGATCCTTGCATGGACCAACTTCGCCAAGACCGGCGATCCCAATGGCGGC
GACCTGCCCGTCTGGCCGCTGTTCGACGCCGGGACCCAGCCGACCATGGTCTTCGCCGCGGACGTCCAC
GTTGAGGACGCGCCCTTCCTGGCCGAGTACAAAGCCATGTCCGACTTCCTGAAGATCTTCAACGTCTTC
GACGCGTTGAAAGGATCTCATCACCATCACCATCACTAA

35) SEQ ID NO:69

ATGACACATACAACAGCTAACGGAATCGACATTGAATATGATACCTTCGGAGATAGAAACCGA
TCCCCGCTGCTTCTTATCATGGGACTCAGCAGCCAGATGGTTGCCTGGCCCGAGTCGTTTTGTCGTAAA
CTCGCGCGAAGCGGCCATTGGGTTCTGCGTTTTGATAATCGCGACGTCGGACTTTCATCCAAATTCGAT
GGCGTCGGCCTACCCGATCTGATGGCGGCCACGGCGGCCCATCTGCAGGGACAGCCGGTGGCAGCGCCG
TATACCCTTTCAGACATGGCGGCTGATGCCGTGCGGCTGATGGATGGCCTTAAGCTGGAAAACGCCCAT
GTGTGCGGATTATCCATGGGTGGCATGATCGCTCAGGTTATGGCGCTAGAGTATCCCCAGCGCGTAACG
AGTCTTATTTCCATGGAATCCTCCACCGGCGATCCGACGCTGCCGCCTGCAGACCCCCAGACCATGGAA
GCCATGTTATCCCCGCCACCACAGGATCGCGCCGGGTACATTCAGCATGCGGTCGAAGTGTTTCGCGCA
TTTTCCGGCGGATCCGATAAATTTGATGAAACCCTGGAAAAGGAACTCTCGGCAAACTCATATGACCGC
TCGTTTTATCCGGCCGGCTTTGTTCGTCAACTGGCGGCCATCCTGGCCTCCGGCGACCGCACCGAGAGT
CTGGCATCCGTGACGGCTCCCACGCTGGTGATTCACGGCGCTAATGACCCTTTGGTGCCGCTGGCCCAT
GGTCGTGCCACAGCCAGGGCCATACCGGGAGCAAAACTGGTGGTGATCGAGGGCCTGGGTCACGGGATC

GCCTATCCCACCCTGTGGGATGAGATCGCTGATGCTATCACGCAGCATACGAGCGCCGCTTCCGGCCGC
GAGCGTCCAGTCGGATCTCATCACCATCACCATCACTAA

36) SEQ ID NO:71

ATGGGTGCGAATAATCCGGTCACAAAATTTGTAATGGATAAGGGGCAGGGCACTGCAGCACGT
TTGCTGGATTGCCTGCCGAGCTTTGCACAAGAACGTCTGGTAAAAGCATTGGATTATCCTTATGACTAT
CCTGATTTGGATCCTTATATAAAATGCTTGATGGCGATTCAGATTAAACAGGGAGAGCACAGTTTTATT
AGTGCAGATGCGGTGCAGTCACGTCAACTGTTTGATGAACGCATGAAAGCTATTCAGGCGAAACCGACG
CCGGTCAAGGCAGTCGAGGATCTGCGTTTGCCATTGCAAAATGGCACTATCTTTGCCCGACATTATCAT
CCGGCACCACAGAAAAAATTGCCGATGGTCATTTTCTATCATGGTGGTGCATTTATAGTGGGTGGTCTG
GATACGCATGATGAGTTCTGCCGTTTATTGGCGGTGCATGCCAAGGTGCAGGTACTCAGCGTGGCTTAT
CCGCTAACGCCCGAATACAGTCCTTTGCAGATGGTACAGGTCTGTGAAGATGCTCTGGCTTGGGTACAT
CAAAACATCAAGCAGTTGAAAATCTATAAAAACCAGATTGTAGTGGCAGGGGATAGTGCAGGTGGAAAT
CTGGCGGCAGTGGTTGCGCAGCGGAGTGCCGATAAAATCTATGCACCTCGCGCACAGTTATTGCTTTAC
CCGGCTGTCGATTTTAAAAGCCGCCATCCTTCTTTTTATGCATACAATCAGGGCCTGGTACTTTCAGCT
CAGGATATTGATCTGGTGACCAAGCTATATGCTGAAACACATCAGGTCGAACTGGATGATCCGCTGATT
TCACCGACCTATGGTGAACTGAAGAATCTGCCGCCTGCCTATGTGATTACCGCCCGTCATGATGTGTTG
CATGATGAGGGTTCAATCTATGCGCTGAAGTTACGTGAGAATGGGGTGCGAGTGTATTATCAGGAGTAT
ACGGATCAGGCCCACGGTTTTATCAATCTGACCCCAATTCATAAACGTTCGAAAAAGCAGGTCATTGAA
CTCAGCAAGAATTTCCGTAAATTCCTGGATAAAAAGATCGGATCTCATCACCATCACCATCACTAA

**37) SEQ ID NO:73**

ATGAAAATTAAGTTGCCAGAACCATTCACATTCGAAGCAGGAAATCGAGCAGTTTTATTATTA
CATGGATTTACTGGCCATTCAGCAGATGTAAGAATGCTTGGTCGATTTCTAGAAAAGAAAGGTTATACA
ACTCATGCACCAATTTATAGAGGGCATGGTCAAGAACCAGAAGCATTATTAAAATCTTCACCTGATGAA
TGGTGGGAGGATATTTTATCAGCTTATAATCATTTGAAAAATCTAGGATATAATGAAATTGCTGTTGCT
GGTCTTTCAATGGGTGGTGCTTTAGCAATTAAACTAGCTACTAAGCATGAAATAAAAGGTGTCATTCCA
ATGTGTACACCAATGTACTTTGATAATCAAAAACAATTAACTCAAGCATTTTTAAATTTTGCACGACAA
TTTAAAAAATTTGAGAAAAAAGATGAAGAAACAATTAAAAAAGAAATAGATTTACTACAACAAAATTCA
GCAGAATTATTTAATGAGATCGGCACTTTTGTAGAACAAGTCAATGGTATAATAGATAGAGTTGACGTA
CCTACATTCGTAGTTCAAGCAAGTAAAGATGAAATAATCAATCCTGAAAGTGCGACATTTATTTATGAA
AACATTAAAAATGAAAAAAAAGATTTAAAATGGTATAAAAATTCAACTCACTTGATAACATTTGGCGAT
GAAAAAGATGTCTTACATGAAGATATATATCATTTTTTAGAAACATTAGATTGGAACAACCATCACCAT
CACCATCACTAA

**38) SEQ ID NO:75**

ATGTTCTGGTTGGCACTTATCTGGGCCCTGCTGGCCCTGAATCTGTATTACCCCTGGAAGGGC
AAACCCGAGGTCGGTGGCGTGATCAGCTTCGCCTTCGGGCTGTTGGTGGGCGAACTCGCCTTGCATGTG
ATTGCGCTGCAAGTTCTCACTACCCTGCTGTTTATCACCTTCTGCGGCCTGTCAGGCTTCGGCGATGCG
CTGGGGCTGATGCTCGCCATGGCCACCTGGCTGGCGATGGCCTGGTTCTACGCACGCAGCGAGCGCGCG
GGGCCCGCGATGGAGAGCGGCATTCGCCAGGCCCTGGGCGAAGACTATTCAAAACTGATTTCGGCCGAG
CGCAGCGACTGCCTGCACCGCGACATAGATTATCGGCAGCTGCTTAACCCCTTTGCCTTCAGCAAACCC
GAAGTGACCCGACACCGCAATCTGCCCTACGCGGAAGTGGACGGCAAAAACCTTTATTTGGATATCTTC
CACCACCGCGACAAGCCACAAAACGCGCCAGTACTGCTGCAAATTCACGGCGGCGCCTGGCTGCAGAAT
CTGGGCAACAAGGATCAGCAGGCGCTGCCGCTGATGAATGAACTGGCCAGCCGCGGCTGGGTGTGCGTG
ACGGTGCAATATCGGCTGAGTCCCGGCGTCACCATGCCCACCCATATTATTGACTGCAAGCGCGCGCTG
GCCTGGGCGAAGGAGCATATCGCTGATTACGGTGGCGATCCGAACTTCATTATCGTCACCGGCGGTTCC
GCTGGCGGGCATCTGTCAGCGATGGTGGCGCTCACCGCCAACCAGCCCGAATTCCAGCCGGGTTTCGAG
GACACGGATACCCGCGTACAAGGTGCCGTACCTTTCTACGGTATCCACGACTTTACCAACAGCCGTGGT
CAGCGCGAGCATAAGGGGCTGGAGAACTTCATCGCGCAACGCGTAATGAAAGTGACCCGCTACGATAAC
CCCAAACTGTGGGAGCAATGCTCTCCCCTGTTTCAGGTAAAAGACGACGCGCCTCCGATGCTGTTGGTA
CACGGCGAGGCCGATACCCTGGCACCGATCAGCGAATCGCAGGCCTTGCTGGAGGTTTTGCGGGAACAC
GACGTCAAAGCGGGGCTGGCGGCACTGCCCGATGCGCAGCACGCCTTCGAACTGATGTACTCGCTGCGC
ACCCTGTATATGATTAATGCCGTGGAGCGCTTCGGCAACGCACTGCACGGCAAGTATCTACAGGATGGC
GCAGCTGGATCTCATCACCATCACCATCACTAA

39) SEQ ID NO:77

ATGGCACTGTTCGTTCTCGTCCACGGAGCTTGGGGCACGCCCACCGAGCTCGCCCCGGTCGAA
CCGGCGCTCACGGCGGCGGGGCACGAGGTCCGAATCGTCGACCTGCCCTGCACCGACCCCGACGCCACC
TTCCACGACTACGCCGAGGCGGTGATCGCCCAGTTGGGCCCGCCCGAGCGCGACCCCGAGACCCTGCTC
GTCGGGCACTCGTTCGGCGGCGCGACCATCGGTCTGGTCCGCGAACGCCGGCCCGACGTGGCGCTGGTG
TACGTCACGGCAGTGGTGCTCGAACCCGGCCAGTCGCTGCTCGAGTTGTTCTTCGGCTCCGATCCGTTC
GACGACCCCGACGTGGACGACCCGTGGGAGGGCTTCGAAGGGCTGGTGCTCGACGCCGGGCCGGGCCTC
ACCCGGGTCGACCTCGACGTGTTGGCCGCCGGGGTACCCGAGGACCAGCGCGCCGAGTTGCTCGCTGCC
CTCGAAGGCACCCAGCGCGAGCAGGGCATCGCGGTCATGCGGGAGGGCTGGCCGGGCGCGTCGGTGCCG
AGCGGTTGGGTCAGCTACATCGTGGCGACCGCCGACACGCTGCTCGAGCCCGACCTGCAGCGCCCGATG
GCCGACATGCTCGGGGCCGACGTGTACGAGGTGCACTCCGACCACGAGGTGTTCATCGAGCAGCCCGAA
GAGTTGGCGAAGATCCTCGACGACCTCGCCGCCGGCCTCACCAGCCGAGGATCTCATCACCATCACCAT
CACTAA

40) SEQ ID NO:79

ATGGGTAAGGCCTGCCGCCTTGCCACTGGCGATCTGTTCGGGCGTGGCCTGGGAACTGCCGCG
CAGCACCAGGTCGGCCCCGAGGAACTCGGTGGCGCGCAACAGCATCGCCCCATTGAGGCGGGCGCCGAA

GTAGCCGATGGCGGTGCTGGCCGCCACCGCCACCAGCAGGGCGAAGAACAGCACGCGCAGCTCGCCGGC
ACGGGCATCGCGCAACAGCTGGCGCAGGGCCAGGACCAGCAGGCGGATAAACGGCAGGCGGGCCATCAG
GCCTCCAGCGGCGCGACCAGGTGGCCGGCGTCCAGGCGGATCAGACGACGGCAGCGCTGGGCCAGGCGT
TCGTCATGGGTGACCAGCACCAGGGTGGTGCCGCGCTCCTTGTTCAGTTCGAACAGCAGGTCGCTGATG
CGCTCGCCGGTATGGCTGTCGAGGTTGCCGGTGGGTTCGTCGGCGAACAGCACCGCAGGCTCGGCGGCG
AAGGCCCGGGCAATGGCCACCCGTTGCTGCTCGCCGCCGGACAGCTGGCGCGGCGAGTGGCTCAGGCGC
TGGCTCAGGCCGACCCGTTCCAGCAGGCTGCGGGCGTGCTCGCGGGCGTCGCGGCGACCGTCCAGCTCC
AGCGGCAGCATGACGTTCTCCAGGGCGTTGAGGCTGTCCAGCAGTTGGAACGACTGGAACACGAAGCCC
ACGTGCTCGGCGCGCACCCGGGCGCGCTGGTCTTCATCCAGCGGCCCGAGGTCGTGGCCGGCGAGGGTC
ACGGAGCCGGCACTGGGCAGGTCGAGGCCGGCCAGCAGGCCGAGCAGGGTCGACTTGCCCGAGCCCGAG
GCGCCGACGATGGCCAGGCTATCGCCCTGTTTCAATTCCAGCGACAGCGCGTGCAGGATGGTCAGGTCG
CCTTCCGCGCTGGGAACCACTTTGCTAAGGTTGCGCGCAGTGAGAATGCTGGGGCCCATGGAGAATCCG
ATGCGAATGTGGTTGTTGAGTGCCGGCCTGGCCTTGATGTGCATGACCCAGGGGGCAGCCGCAGGTACT
GTACTGGTCGTCGGCGATAGTATCAGTGCCGCTTTCGGCCTGGATACCCGCCAGGGCTGGGTCGCCTTG
CTGGAACAGCGCCTGAAAAGCGAGGGTTTCGACGCCAGGGTGGTCAACGCCTCGATCAGCGGCGACACC
AGTGCCGGAGGCCAGGCGCGGCTGCCGGCGCTGCTTGCAGAGCACAAGCCGGAGCTGGTGGTGCTGGAG
CTGGGAGGCAATGACGGCCTGCGTGGGCAGCCGCCAAAGCAATTGCAACAAAATCTTGCCTCGATGATC
GACCGCTCCCGTGAGGCCGGGGCCAAGGTGCTGTTGCTGGGCATGCGCTTGCCGCCCAACTACGGCAAG
CGTTACACCGACGCCTTCGCAAAGGTCTATGCCGACCTGCAGGCCGAGAAACAGGTGGCGCTGGTGCCG
TTTTTCCTCGAAGGGGTAGGGGGTGTGCCCGAGCTGATGCAGGCCGATGGCATTCATCCGGGGGCGGGC
GCCCAGCAAAGGTTGCTGGAAAATGCCTGGCCGCAACTAAAACCGCTGCTTGGATCTCATCACCATCAC
CATCACTAA

**41) SEQ ID NO:81**

ATGGCCCGCAAGTTCCTCTATCTTATCGCCCTGCTTGCCGTGATGGTCATTGCAGCGATCTTC
GCGCTGCGCATCTGGCAGAACGAGCTCACCCGCTTCGCATTCGTACCGCGCGTGGCTTACGCCCGCCTC
GATCCGCTGCCTGCAGATGCCTATGCCGGCAACCGGATGTGGCTCGCCCGCCCGGGGCTCGGTGATAGC
GATCCCGCACAGTGGCTCCCTGCAGGAGTAAGGAAGAGTGCCGGTCCGGGCACATCTGCCGCAGTGTTC
TTCATTCATCCGACCAGCTATCTCGCGCGCGAAAGCTGGAACGGTCCGCTCGACGATACCGATACGAAT
CGCCGGGCCACCTATTTCATCCAAGGTATGGCCTCTGCCTTCAACGGGCAGGCACAGGTCTGGGCACCG
CGCTATCGGCAAGCCGCCTTCGGCGCATTCCTCACCGACAAGGCCGAGGGCGAGCTGGCCCTGGACTCT
GCCTACCTCGATGTCCGGCAGGCCTTCGACGCCTTCCTTGCGGCGACACCGCCCGATGCACCGATCGTG
CTGGCAGGGCACAGCCAGGGCGCGCTCCACCTGATGCGCCTCTTGAAGGACCGGATTGCCGGAACACCA
CTCGCTGGCCGGGTGGTAGCGGCCTACCCGATCGGATGGCCGATCTCGGTCGAGCATGATCTTCCCCCG
CTTGGCCTTCCGGCTTGCGACAAGAGCGATGCTACTGGCTGCATCATGACGTGGTCGAGTTTCGCTGAG
CCCGCCGAACCGCAACTTGTGTTGGAAGCGTACCGCACGCGCCCCGCGCTTGATGGGAAAGCCAAGGAT
GCAAAGCCGGTTCTGTGCACCAACCCGCTTACTGGAAGCATCGGCGGACAGGCACCCGCGTCGGCCAAC

CTTGGCACGCTAAAGCCCAGCAAGGACCTGAAGAGTGGTGAGTTGATCGCCAAGGCTGTTCCGGCCCGC
TGCGATACCAGCACCGGCTTGCTGATGATCGGGGACCCACCGGACCTTGGGCCGTTTGTCCTGCCAGGC
AACAACTACCACGTCTATGACATCCCCCTGTTCTGGGCGAACCTGCGCGCAGATGTCGGCCGGCGGACC
GCAACGTGGGAGAAGGCACGCGGAGCGGCCGCCCGGGGATCTCATCACCATCACCATCACTAA

42) SEQ ID NO:83

ATGGATGGCGGCACGGCCAAGGTGGGCGACGGCATCGCCTATGCCGACGGGCCGCGGCACAAG
CTCGACATCTATGCGCCCGAGCAACGCGGCGCGCCCGCCCCGGTCGTGTTTTTCATCTATGGCGGCGGC
TGGAACCGGGGCGAGCGCAGCGACTATCAGTTCGTCGGGCGCGCCCTGGCCTCGCGCGGCTTCGTCACT
GTCATTGCCGACTATCGGCTGGTGCCGGAAGTACGGTTTCCCGGCTTCCTGACCGACAGCGCCAATGCG
TTGCGCTGGGTGCAGGACAATATTGCCAATTACGGCGGCGACCCCAATCGGCTGTTTCTCGCCGGGCAT
TCGGCCGGTGCCTATAATGCGGTCATGCTGGCGCTCGACCCTGCCTATCGGCAGGAATTCGGCGTCACC
ATGCCGATCCTGGCGGTGGCGGCCCTGTCGGGCCCCTATGATTTCTATCCGTTCGAATATTTCGAGGTG
CAGGAGGCTTTCGGCCAGGCGCCCAATCCGGAAGGGACGCAGCCGATCAACCTGATCACCGCCGAGGCG
CCACCGATGTATCTGGCGACCGGCACTACCGATCCGATCGTGCGGGTGCAGAATACCGAACGCTTTGCC
GATCGGCTGCGGGCCCAGGGCGTGTGGGTGACGACCAGATATTACGAGGGGTTCGGGCATATGGAACCG
GTGATCGCCATGGGCGCGCTGTGGCGTTGGCGCATGCCGGTACTCGATGAGATGGTCGGCTTTTTCCAG
CGTTTCGGCGCCTTTCCCAGCGGCGTGCCCTATCTCGCCGTGACGCCGGAAGCGCCACAAATGCTGCCC
GATTCGGTGGCGCCGACCGATGAAATCGTCCGCCAGCTCAACGAGATGTTCCAGCCGATCGAGGAACAT
CACCATCACCATCACTAA

43) SEQ ID NO:85

ATGAATACAGCCGACCTATTGAAGCCACCACCCGCAAGCATGACAGTTCTCGAGGCGAGAGCG
CTGCTGGACATATGCAAGATGAGCGCCCCATTGGCGCGCTTGCTATTCAAAAAGAACTCGCCCTGGCGC
AAACAACGGGTTCTCGTAATACCTGGCTTTGGCGCTGATGATCGCTACACCTGGCCGTTGCGCAATTTC
GTCCAGGCACAGGGCTATGCCACGACTGGCTGGGGCCTGGGCACCAACAAGGCAGGTCTCAATATGCCG
CATCAACTATCCGACGTCCACCCCAGATGGAAGCTAAAACCCAAGACGCCGTACCGTGGTGAGGCGGGC
GTACCTTACGTGATTGACCGCTTGATCGAACGGTTTGACGAATTGGCATCGACGGATCCGCAACCCATC
GCACTTATAGGTTGGAGTCTGGGTGGTTTCATGGCCCGTGAAGTTGCCCGAGAGCGCCCAAACCAGGTG
AGTCAGGTTATTACCCTCGGTTCTCCTGTCATCGGAGGCCCAAAATACACCCTCGCTGCATCGGCTTTC
ATCCGGCGCAAATACGATTTGGACTGGGTGGAGCAAGTGATCGCGGAGCGGGAAGATCGCCCCATTACT
GTTCCTATTACAGCAATAGTCAGCCAGTCTGATGGCATCGTCGGATATTCAGCGGCAATCGATCACCAC
AGTCCCGCTGTGCAGCATTTACATATGGATGTTGCCCATTTGGGCTTTCCTTACAACACGAGGGTTTGG
TCAGAAATCGCCAATGCGCTCAACTCTTTAGAGGTGGAGAAGGAGCGTGTTGGATCTCATCACCATCAC
CATCACTAA

44) SEQ ID NO:87

ATGAGACTGATCGTGAAAAGACGCGCTCACGCCGCCCTGCTGATGTTCGCAGCGGCGCTGTTG
AATGTTGTTGGCGTGGCTCAGGCCGCTGTCCCGTTCCGCTCCGACCGGATGAGCGTCGAGGTCCGCGGG
AGCGGCCCCGACGTGGTGCTGATCCCCGGGCTCGCCTCCTCGCCGGAGGTCTGGCAGGCGACCGCGGAC
CGGCTCGACGACACCCATCGCGTACATCTGGTGCAGGTGAACGGCTTCGCCGGCGCGCCCGCCGGGGCG
AACGCCGAAGGCGCGGTGGTCGCGCCGCTGGTCGGGGAGCTGAACCGCTACATCGCCGAGACCGGGCTG
AAGCGGCCGGCCCTGATCGGCCACTCGCTGGGCGGCTTCGCGGCGCTGCGGTTGGCGGCCGAGCATCCG
CAGGCGGTGGGCCGCGTGCTGGTGGTGGACTCGCTGCCCTTCTTCCCGCTGCTGTTCAGCCCGGCGGCG
ACTGTGGAGGCGGTGACCCCGCAGGCGAACGCCATGCGGGACGGCATGCTGGCCATGCCGGCCGAGCGC
TTCCCGGCGGTGCAGGCGGCCGGGATGGGACGGCTGGTCAAGAACCCCGAAGCACGCAGCAAGCACGTC
CGCATCGGCGGCGCCTCCGACCCTGCGGTGGTCGCGCGCGCCATGCACGAGATCATGACCACCGACCTG
CGGCCCCAGCTGCGCGGGATCGTTGCGCCCGTCACCGTTCTCTACGCCTGGGACCCCGCCATGGGACTC
ACGGTCGAGGCCTACGACCAGCTCTGGCGCGGCGCCTACGCCGGACTGAAGGGGGCGAGCCTGACGCGG
GTGGACGGCAGCTTCCACTTCATCATGGACGACCAGCCGGAGCGCTTCTCCGCGGAGGTCGAGCGGTTC
CTGGCGGCCGAGGGATCTCATCACCATCACCATCACTAA

**45) SEQ ID NO:89**

ATGAGCTGGATGCAATCGGCCGCTAGCATCCTTGCCCTGGCGCTGGCCCTACTGGCCGGCGGG
GTGTGGCTCGTGGACTGGCGCGCCGACCGGCGCGAGGCGGAAGCGCTCGCGCAGTGGCCGCCGGAGGGC
ATTTTTGTCCAGGTTGAGGACCGCCGAGTCCACGCCGTCACGCGGGGGCAAGGCCCCGACCTCGTCCTG
ATCCACGGCGCCTCGGGCAATGCGCGGGATATGACCTTCCGCTTTATGGGCCTGCTAACGGACCGCTAC
CGTGTCACCGCGTTCGACCGTCCGGGCCTGGGCTACACCGACCCCGCGCCGGCCGATTGGGGCGAGAGC
CCGAAGGAGCAGGCCGCCTTCCTACAGGCCGCAGCGCGGGAGCTGCATATCGAGAGCCCCATCGTCCTG
GGCCACTCCTTCGGCGGGGCTGTGGCGCTGGCCTGGGGATTGAGCGACCCGGAGGGGACCGCCGCCGTC
GTGGACGTTTCCGGTGTGGCGATGCCCTGGCCCGGCCGCCTGGGCTGGCTCTACCGCATCAATGGCCCG
TGGCTCGGTACGGCCCTTGTGCCGCCTCTCGTTGCTGCCTTTGCGCCCCAACCGCTCCTAGACCGGGCG
CTCGCGGCGACGTTCGCGCCCGATCCCGTCCCCGAAGGCTATGCCGAGCACTTCGGAGCCAGCCTCTCG
ATCCGACGCGCCTCGTTTCGGACCAACGCCCGTCAGGTGAACGCGCTGCGGGCCTCGGTCATGGAGATG
TCCCGGGACTATTCAACCTTCTCCGTGCCCCTTGAAATCGTCCACGGCGAAGCCGACACCATTGTCCCC
CTCGAGGTCCATTCCTGCCCTCTGTCGCGAATCATTCCTGGCGCGAACCTTACGGTGCTGCCCGGCGTC
GGCCATATGCCGCACCAGACGCACCCCGAGGCGGTGGTCGCCGCTGTCGATCGGGCGCGAGCCCGCGCT
GGGCTCGGATCTCATCACCATCACCATCACTAA

**46) SEQ ID NO:91**

ATGAGGAATTGGGCAATCGGCGGGCTGGCGCTGGGCGCGGGTGCGCTGGGAGCGGGCCTGTAC
ACCAGCCGCAAGGCACGGGAGGCCGAGGCCGCGGTGCCGATGGACGGGCGCATGGTAGAGGCCGGCGGG
CACCGCTTCCATGTCGCGGAGCAGGGGCAGGGCCGGCCGCTGCTGCTGATCCATGGGCTCGCCGGGCAG
ATGCGCAACTTCGGGCAGCCGATGCTCGACGACCTGGCGCGCGACTATCGGGTCATCCGGATCGACCGG

CCGGGGTCCGGCTACTCGCCGCGGCTGGCGAGCGGGCGCGGGCATCTGTCGGGACAGGCCGACGCCATC
GCCGCGCTGATCGACGCGCTTGGCCTGGAGAAACCGGTGCTGGTGGGGCATTCGCTGGGTGGTGCGGTG
AGCCTCGCGACGGCCATGCGCCATCCCGACAAGGTCGGCGCGCTGGCACTGATCGCTCCGGCGACCCAG
CCGATCCAGAGCATTCCCGAGGTGTTCAAGGGACTGGTCGTCCCGCCCGCTTTGCTGCCCCTGGTCGCC
TGGACCATCGCCGTTCCCGTGGGCGTCGCGACCAAGGACAAGGTGCTGCAGCAGGTGTTCGCGCCCGAG
CCGGTGCCGGCGAACTTTCTGGTGGATGGCGGCGGCATGCTCGGGTTCCGGCCCAAGAACTTCGAGGGC
GCGGCAACCGACCTCGCCGACGCCAGCAGCGAGGCCGAGACCCTGCTGCCGGGCTACGCCGGGCTGAAG
CTGCCGGTCGGCATCTTCTACGGGCGCGGGGACAATCTGCTCGACTATCGACTGCATGGCGAGAAGACC
GCATCCGAGATCCCGGGCGCCCGGCTGACGCTGACCGAGGGCGGCCACATGCTGCCCTTCACCCAACCG
GAGGCGACCGCCCGCTTCGTCCGGAGCGTGATCGAGGGATCTCATCACCATCACCATCACTAA

**47) SEQ ID NO:93**

ATGACGCTTCCGCACCGACGTTCGCACCTGCGCCCGATTGATGAGAGGCTCTTTGCCCGCTGG
CTAGCGGCCGGCGCCGTCGCAATGCTGCTCAGTGTCACTGCTTGCGCCGCGCAACCGGCGAAAAGCGAG
CGACCAGCGGCCGCTGACGTGGTTCGGGTCTGGCCGGGGGCGGCTCCCGGCACGGAGAGTTGGACCGGA
GCGGAGGTCGAGCTGGACGCCGATTTGCCGGCCGGCAAAGTCCGGGTCGTCACCAATGTCACCGCGCCG
GCGCTGACGATCGTGCGCCCCAAGCCGGGCACCGCCAACGGACCGCGATGCTGGTTCTACCCGGCGGT
GCATTCCGGGCGCTCGCCTGGGACCTCGAAGGAACCGAAGCCGCGCAACGGCTGGCCGGCCGCGGCATC
ACGGCCTTTGTCCTCAAATATCGCGTCCGGCCCCCGGTCGGAGCGCCCGCCGGGCCGGAATCTTTCGAC
GCCTTCACCGTTCGAACGCAGCCAGCGAGAGACATTGCGGTCGCCGACGCCCGGCAGGCGCTTCGCCTG
ATCCGGGCACGGGCGAAAGACTATGGCATCTCGGCCGATCGGATCGGAATGATCGGCTTTTCAGCTGGA
GCCATGGCGGTAATGGAGCTGGCGCTGGCGCAAGAGGTGACGGATCGCCCGAACTTCGCAGCGTCCATC
TATGGAGCGGCGCCCACCGAGCGGCAACCGGCCGCCGGCGCCCCGCCCTTGTTCCTTGTGGCTGCTCAG
GACGATCCCCAGGTCCCCTCCGACCGAAGCGTCGATCTGTATCGCCGCTGGACCCAAGCGGGCCTGCCC
GCCGAGCTGCACCTCTACGAAAAGGGCGGGCACGGCTTCGGCACGCGCGCCCGCAACCTCCCCGCGGAC
CAGTGGCTTGAAGCGCTCGAGGCCTGGCTCATCTCCCGCCGCCTGGCGCCACCACCCGCAGCGGCCAAG
CACAAAGGATCTCATCACCATCACCATCACTAA

**48) SEQ ID NO:95**

ATGAACAACAACGAACAACCGACAAAACGCCGCGGCATAACGGTGGAAGATTTGCTTCGC
CTCCGCTCGGTGCGCGACCCGCACTACGCGCCGGACGGGACGCGCGCCGTGTTTGTGGAA
AAATCGATTGATGAGGAAAAACAATACCGCTCCCATTTATGGATATGGGCGGCAGACGGC
TCCGTCCGTCAATGGACGTTCGGCCGCTGGCGCGACATGAAGCCGCGTTTTTCCCCAAGA
GGTGAAATGATCGCCTTTTTATCTGACCGCTCCGGACGCACACAGCTTTGGCTTTTGCCC
GCCAATGGCGGTGAGGCGCGCCAACTGACGTTTTTCAAAAACGGTGTGCGCGATTACGTT
TGGTCGCCGGACGGGACGTTTCTCATCGCCCCTAACGACGCTTGGCGACGAAGAAACGATC
GAAGACCGAGAAGAGCAGAAAACGGCGGAACAAAAACCGGCCGATTCGAAGCCGCGCGTT

GTGGAACGGCTCTATTACAAATCAGACGCGTCGGGTTTTCTTGACGGCAAGCAGGACGTG
CTGGTGCGCATCGATGCGGCGTCAGGAAAAAGCGAAGCGTTGACGGGCCGCGAGGAAGAA
ATCGGTTCGTTTGCCGTTTCCCCAACCGGACGGACGCTCGCCTTTGTCGCCAACCGAAAC
GATGATCCAGACACAACCTTTACGCGCGACATTGTTTTGCTTGATCTCGAATCGAAAGCG
GAAACGAACTTGACGAACGGATGCGGCACATTCGCTTCGCTCGCCTGGTCGCCGGACGGA
ACGAAGCTCGCCGCCATCGGCCATGATCTTGCTTATCTCGGGGCTACGCTTCACCGGCTT
TACGTCTTTGAGCCGGAGCGTGGAACGAAGCGGGTGTTGACCGCCGATTGGGACGTCCAT
CTCGGCGATGCCATGGTCGGCGACACACACGCTGATGCGAAAGGGCCGGGACCGATTTGG
GCGAGCGACGGAAGCGGCCTGTATGTCACCGCCTCGGAGCGCGGACGCGTCAACTTGTAT
TTCGTCCCGCTCGACGGCCCGATCGTTCCGGTGATCGAAGGCAACTTCCATTTATACGGT
TTAGCCATCCACCCAAGCGAACAGCAAGCCATCGCCGCCATGAGCGAGCCGACGGTCATT
GGCGATTTGTATATAATCTCGCTTGAAGACGGAACGAAAACACGGCTTACGCGTGCCAAT
GAAGCGCTCGAAAACGAAGTGGTGTTCGCCGATGCCGAACCGTTTACGTATCGATCGGCG
GATGGTTGGGAGATTCAAGGCTGGATCATGAAACCGCCCGGGCTTGGCGAAGGGGAAAAG
GCGCCGCTTGTCGTCGAAATTCACGGCGGGCCGCATGCGATGTACGGGTTTACCTTTTTC
CATGAATTTCAACTGCTCGTTTCGCGTGGTTATGCGGTCTTGTTCACGAACCCGCGCGGC
AGCCACGGATATGGACAGGCATTCGTCAATGCCGTGCGCGGCGACTATGGCGGCATGGAT
TATGAAGACATTATGGCCGGCGTCGACGCCGCGATCAGCAAGTTCGACTTCATTGACGAG
ACATGGCTTGGCGTCACTGGCGGCAGCTACGGCGGGTTTATGACCAATTGGATCGTCGGG
CATACCGACCGGTTCAAAGCGGCTGTCACCCAGCGTTCCATTTCCAACTGGCTCAGTTTC
TCCGGAGTGAGTGACATCGGCTACTTTTTCACAAAATGGGAGGTCGGCTGCGACATCTGG
GAAGACGCGGAGCGGCTTTGGCATCATTCACCGCTCAAATACGTCCAAAACATGCGCACA
CCGCTCTTAATCTTGCATAGCGAGCGCGATTACCGCTGCCCGATCGAGCAGGCGGAACAG
CTGTTTATCGCATTGAAACAACTCGGGCGGGAAACGAAACTTGTCCGCTTCCCGGACGCC
AACCACGATTTGTCGCGCACCGGCAACCCGGCGCTTCGCCTCGAACGTCTTCGCCAAATC
GTCGGTTGGTTTGATCATTATTTGAAAGGACCACTGGATTAA

**49) SEQ ID NO:97**

ATGCTTGATATGCCAATCGACCCTGTTTACTACCAGCTTGCTGAGTATTTCGACAGTCTGCCG
AAGTTCGACCAGTTTTCCTCGGCCAGAGAGTACAGGGAGGCGATAAATCGAATATACGAGGAGAGAAAC
CGGCAGCTGAGCCAGCATGAGAGGGTTGAAAGAGTTGAGGACAGGACGATTAAGGGGAGGAACGGAGAC
ATCAGAGTCAGAGTTTACCAGCAGAAGCCCGATTCCCCGGTTCTGGTTTACTATCACGGTGGTGGATTT
GTGATTTGCAGCATCGAGTCGCACGACGCCTTATGCAGGAGAATTGCGAGACTTTCAAACTCTACCGTA
GTCTCCGTGGATTACAGGCTCGCTCCTGAGCACAAGTTTCCCGCCGCAGTTTATGATTGCTACGATGCG
ACCAAGTGGGTTGCTGAGAACGCCGAGGAGCTGAGGATTGACCCGTCAAAAATCTTCGTTGGGGGGGAC
AGTGCGGGAGGGAATCTTGCCGCGGCGGTTTCAATAATGGCGAGAGACAGCGGAGAAGATTTCATAAAG
CATCAAATTCTAATTTACCCCGTTGTGAACTTTGTAGCCCCCACACCATCGCTTCTGGAGTTTGGAGAG

GGGCTGTGGATTCTCGACCAGAAGATAATGAGTTGGTTCTCGGAGCAGTACTTCTCCAGAGAGGAAGAT
AAGTTCAACCCCCTCGCCTCCGCAATCTTTGCGGACCTTGAGAACCTACCTCCTGCGCTGATCATAACC
GCCGAATACGACCCGCTGAGAGATGAAGGAGAAGTTTTCGGGCAGATGCTGAGAAGAGCCGGTGTTGAG
GCGAGCATCGTCAGATACAGAGGCGTGCTTCACGGATTCATCAATTACTATCCCGTGCTGAAGGCTGCG
AGGGATGCGATAAACCAGATTGCCGCTCTTCTTGTGTTCGACTAG

**50) SEQ ID NO:99**

ATGAGATCAGGAGCTCGCATCAGCGTGGCGGCAGTTGCCTTTCTTTGCCTGCTCTTGACGACT
CGGGTTTCCGCCCAGATCGTGCCGGCGATGGAATGTGCGGATCTGGCGAATCAGCAGCTTCCCAACACG
ACGATCACCTCGGCCCAGACCGTCACCACCGGATCGTTAACGCCCCCGGGCTCGACGAATCCGATCACC
GACCTGCCTCCTTTCTGCCGTGTCACAGGCGCCATCGCCCCGACGAGCGAGTCGCACATCCTCTTCGAG
GTCTGGCTGCCGCTGGATAAATGGAACGGCAAGTTCGCCGGCGTGGGCAACGGCGGCTGGGCCGGCATC
ATCTCCTTCGGCGCCCTCGGAAGCCAGCTCAGGCGCGGCTACGCGACCGCCTCCACGAATACGGGTCAC
GAAGCGGCGCCGGGGATGAACGCAGCCAGGTTTGCGTTCGAGAAGCCGGAGCAGCTTATCGACTTCGCC
TATCGCTCCCAGCACGAGACGGCCCTGAAAGCGAAGGCGCTGGTTCAGGCTTTCTACGGGAAGCCGCCG
GAACACTCCTATTTCATCGGGTGCTCATCGGGTGGGTACCAGGGCCTGATGGAGGCCCAACGATTTCCG
GCCGACTACGACGGGATCGTCGCCGGTATGCCGGCGAACAACTGGACACGGCTGATGGCCGGCGACTTG
GACGCGATCCTTGCCGTCTCCGTAGATCCTGCCAGCCACCTTCCCGTCTCCGCATTGGGTCTGTTGTAT
CGCTCGGTGCTCGCTGCCTGCGACGGCATCGACGGTGTTGTAGACGGTGTTCTGGAGGATCCGCGCCGA
TGCCGGTTCGACCCGGCCGTGTTGATGTGCAAGGCGGATCAGAATCCCGATGGCTGCCTTACGCCGGCT
CAGGTGGAAGCGGCACGGCGCATATACGGCGGTCTGAAGGATCCCAAGACCGGCGCTCAGCTCTATCCG
GGGCTGGCGCCGGGAAGCGAGCCGTTCTGGCCGCACCGCAATCCGGCGAATCCGTTCCCTATTCCGATC
GCGCACTACAAGTGGCTCGTCTTTGCCGATCCAAACTGGGATTGGAGAACATTCAAGTTCACGGATCCG
GCGGACTACCAGGCTTTCCTCAATGCGGAAGCCACGTATGCCCCTACTCTCAATGCGACCAATCCGGAC
CTCCGGGAGTTCAGCCGGCGCGGCGGCAGGTTGATTCAGTACCATGGCTGGAACGATCAGCTGATTGCC
CCGCAAAACAGCATCGACTATTACGAGAGCGTCCTTTCGTTCTTCGGGTCCGGCAAACAGGATCGAGCG
CAGACCGTGCGCGAGGTTCAGAGCTTCTACCGGCTGTTCATGGCGCCGGGTATGGCTCACTGTGGAGGC
GGTACAGGTCCGAACTCATTTGACATGCTGGATGCCCTCGAGAAGTGGGTGGAAGGCGGGATAGCGCCG
GAACGAGTCCTTGCGACGCGTTCCATAAACGGCGTAGTCGACCGGCTGCGCCCGCTCTGTCCATATCCG
CAGGTCGCCGTGTACAAGGGTCATGGGGATACAAACGACGCCGCGAACTTCGTCTGTCGCGATGGATCT
CATCACCATCACCATCACTAA

A number of embodiments of the invention have been described. Nevertheless, it will be understood that various modifications may be made without departing from the spirit and scope of the invention. Accordingly, other embodiments are within the scope of the following claims.
The following numbered paragraphs assist further in describing the invention and its embodiments.

1. An isolated, synthetic or recombinant nucleic acid comprising

(a) a nucleic acid sequence having at least 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%,, or more sequence identity to SEQ ID NO:1, SEQ ID NO:3, SEQ ID NO:5, SEQ ID NO:7, SEQ ID NO:9, SEQ ID NO:11, SEQ ID NO:13, SEQ ID NO:15, SEQ ID NO:17, SEQ ID NO:19, SEQ ID NO:21, SEQ ID NO: 23, SEQ ID NO:25, SEQ ID NO:27, SEQ ID NO:29, SEQ ID NO:31, SEQ ID NO:33, SEQ ID NO:35, SEQ ID NO:37, SEQ ID NO:39, SEQ ID NO:41, SEQ ID NO:43, SEQ ID NO:45, SEQ ID NO:47, SEQ ID NO:49, SEQ ID NO:51, SEQ ID NO: 53, SEQ ID NO:55, SEQ ID NO:57, SEQ ID NO:59, SEQ ID NO:61, SEQ ID NO:63, SEQ ID NO:65, SEQ ID NO:67, SEQ ID NO:69, SEQ ID NO:71, SEQ ID NO:73, SEQ ID NO:75, SEQ ID NO: 77, SEQ ID NO:79, SEQ ID NO:81, SEQ ID NO:83, SEQ ID NO:85, SEQ ID NO:87, SEQ ID NO:89, SEQ ID NO:91, SEQ ID NO:93, SEQ ID NO:95, SEQ ID NO:97, SEQ ID NO:99, SEQ ID NO:101, SEQ ID NO:103, SEQ ID NO:105, SEQ ID NO:107, SEQ ID NO:109, SEQ ID NO. 111, SEQ ID NO:113, SEQ ID NO:115, SEQ ID NO:117, SEQ ID NO:119, SEQ ID NO:121, SEQ ID NO:123, SEQ ID NO:125, SEQ ID NO:127, SEQ ID NO: 129, SEQ ID NO:131, SEQ ID NO:133, SEQ ID NO:135, SEQ ID NO:137, SEQ ID NO:139, SEQ ID NO:141, SEQ ID NO:143, SEQ ID NO:145, SEQ ID NO:147, SEQ ID NO:149, SEQ ID NO:151, SEQ ID NO:153, SEQ ID NO:155, SEQ ID NO:157, SEQ ID NO:159, SEQ ID NO:161, SEQ ID NO:163 and/or SEQ ID NO:165, over a region of at least about 50, 75, 100, 150, 200, 250, 300, 350, 400, 450, 500, 550, 600, 650, 700, 750, 800, 850, 900, 950, 1000, 1050, 1100, 1150, 1200, 1250, 1300, 1350, 1400, 1450, 1500, 1550 or more residues, wherein the nucleic acid encodes at least one polypeptide having a hydrolase activity, or the nucleic acid encodes a polypeptide that can generate antibody that specifically binds to SEQ ID NO:2, SEQ ID NO:4, SEQ ID NO:6, SEQ ID NO:8, SEQ ID NO: 10, SEQ ID NO: 12, SEQ ID NO: 14, SEQ ID NO: 16, SEQ ID NO: 18, SEQ ID NO: 20, SEQ ID NO:22, SEQ ID NO:24, SEQ ID NO:26, SEQ ID NO:28, SEQ ID NO:30, SEQ ID NO:32, SEQ ID NO:34, SEQ ID NO:36, SEQ ID NO:38, SEQ ID NO:40, SEQ ID NO:42, SEQ ID NO:44, SEQ ID NO:46, SEQ ID NO:48, SEQ ID NO:50, SEQ ID NO:52, SEQ ID NO:54, SEQ ID NO:56, SEQ ID NO:58, SEQ ID NO:60, SEQ ID NO:62, SEQ ID NO:64, SEQ ID NO:66, SEQ ID NO:68, SEQ ID NO:70, SEQ ID NO:72, SEQ ID NO: 74, SEQ ID NO:76, SEQ ID NO:78, SEQ ID NO: 80, SEQ ID NO: 82, SEQ ID NO:84, SEQ ID NO:86, SEQ ID NO:88, SEQ ID NO:90, SEQ ID NO:92, SEQ ID NO:94, SEQ ID NO:96, SEQ ID NO:98, SEQ ID NO.100, SEQ ID NO:102, SEQ ID NO:104, SEQ ID NO:106, SEQ ID NO:108, SEQ ID NO:110, SEQ ID NO:112, SEQ ID NO: 114, SEQ ID NO:116, SEQ ID NO:118, SEQ ID NO:120, SEQ ID NO:122, SEQ ID NO:124, SEQ ID NO:126, SEQ ID NO:128, SEQ ID NO:130, SEQ ID NO:132, SEQ ID NO:134, SEQ ID NO:136, SEQ ID NO:138, SEQ ID NO:140, SEQ ID NO:142, SEQ ID NO:143, SEQ ID NO:146, SEQ ID NO:148, SEQ ID NO: 150, SEQ ID NO: 152, SEQ ID NO: 154, SEQ ID NO: 156, SEQ ID NO: 158, SEQ ID NO:160, SEQ ID NO:162, SEQ ID NO: 164 and/or SEQ ID NO:166, and optionally the sequence identities are determined by analysis with a sequence comparison algorithm or by a visual inspection;

(b) a nucleic acid sequence encoding SEQ ID NO:2, SEQ ID NO:4, SEQ ID NO:6, SEQ ID NO:8, SEQ ID NO: 10, SEQ ID NO:12, SEQ ID NO:14, SEQ ID NO:16, SEQ ID NO: 18, SEQ ID NO:20, SEQ ID NO:22, SEQ ID NO:24, SEQ ID NO:26, SEQ ID NO:28, SEQ ID NO:30, SEQ ID NO:32, SEQ ID NO:34, SEQ ID NO:36, SEQ ID NO:38, SEQ ID NO:40, SEQ ID NO:42, SEQ ID NO:44, SEQ ID NO:46, SEQ ID NO:48, SEQ ID NO:50, SEQ ID NO:52, SEQ ID NO:54, SEQ ID NO:56, SEQ ID NO:58, SEQ ID NO:60, SEQ ID NO:62, SEQ ID NO: 64, SEQ ID NO:66, SEQ ID NO:68, SEQ ID NO:70, SEQ ID NO:72, SEQ ID NO:74, SEQ ID NO:76, SEQ ID NO:78, SEQ ID NO:80, SEQ ID NO:82, SEQ ID NO:84, SEQ ID NO:86, SEQ ID NO:88, SEQ ID NO:90, SEQ ID NO:92, SEQ ID NO:94, SEQ ID NO:96, SEQ ID NO:98, SEQ ID NO:100, SEQ ID NO:102, SEQ ID NO:104, SEQ ID NO:106, SEQ ID NO:108, SEQ ID NO. 110, SEQ ID NO:112, SEQ ID NO:114, SEQ ID NO:116, SEQ ID NO:118, SEQ ID NO: 120, SEQ ID NO: 122, SEQ ID NO: 124, SEQ ID NO: 126, SEQ ID NO:128, SEQ ID NO:130, SEQ ID NO:132, SEQ ID NO:134, SEQ ID NO:136, SEQ ID NO:138, SEQ ID NO:140, SEQ ID NO: 142, SEQ ID NO:143, SEQ ID NO:146, SEQ ID NO:148, SEQ ID NO:150, SEQ ID NO:152, SEQ ID NO:154, SEQ ID NO:156, SEQ ID NO: 158, SEQ ID NO: 160, SEQ ID NO: 162, SEQ ID NO: 164 and/or SEQ ID NO: 166, or enzymatically active fragments thereof;

(c) a nucleic acid sequence that hybridizes under stringent hybridization conditions to SEQ ID NO:1, SEQ ID NO:3, SEQ ID NO:5, SEQ ID NO:7, SEQ ID NO:9, SEQ ID NO:11, SEQ ID NO:13, SEQ ID NO:15, SEQ ID NO:17, SEQ ID NO:19, SEQ ID NO:21, SEQ ID NO:23, SEQ ID NO:25, SEQ ID NO:27, SEQ ID NO:29, SEQ ID NO:31, SEQ ID NO:33, SEQ ID NO:35, SEQ ID NO:37, SEQ ID NO:39, SEQ ID NO:41 , SEQ ID NO:43, SEQ ID NO:45, SEQ ID NO:47, SEQ ID NO:49, SEQ ID NO:51, SEQ ID NO:53, SEQ ID NO:55, SEQ ID NO: 57, SEQ ID NO:59, SEQ ID NO:61, SEQ ID NO:63, SEQ ID NO:65, SEQ ID NO:67, SEQ ID NO:69, SEQ ID NO:71, SEQ ID NO:73, SEQ ID NO:75, SEQ ID NO:77, SEQ ID NO:79, SEQ ID NO:81, SEQ ID NO:83, SEQ ID NO:85, SEQ ID NO:87, SEQ ID NO:89, SEQ ID NO:91, SEQ ID NO:93, SEQ ID NO:95, SEQ ID NO:97, SEQ ID NO:99, SEQ ID NO:101, SEQ ID NO:103, SEQ ID NO:105, SEQ ID NO:107, SEQ ID NO:109, SEQ ID NO.111, SEQ ID NO:113, SEQ ID NO:115, SEQ ID NO:117, SEQ ID NO:119, SEQ ID N0:121, SEQ ID NO: 123, SEQ ID NO:125, SEQ ID NO:127, SEQ ID NO:129, SEQ ID NO:131, SEQ ID NO:133, SEQ ID NO:135, SEQ ID NO:137, SEQ ID NO:139, SEQ ID NO:141, SEQ ID NO:143, SEQ ID NO: 145, SEQ ID NO:147, SEQ ID NO:149, SEQ ID NO:151, SEQ ID NO:153, SEQ ID NO:155, SEQ ID NO:157, SEQ ID NO:159, SEQ ID NO: 161, SEQ ID NO: 163 and/or SEQ ID NO: 165, wherein the stringent hybridization conditions comprise a wash step comprising a wash in 0.2X SSC at a temperature of about 65°C for about 15 minutes, wherein the polypeptide has a laccase activity or the polypeptide can generate a humoral immune response to make an anti-laccase antibody;

(d) a laccase-encoding nucleic acid generated by amplification of a polynucleotide using an amplification primer pair, wherein the primer pair is capable of amplifying the nucleic acid sequence of SEQ ID NO:1, SEQ ID NO: 3, SEQ ID NO:5, SEQ ID NO:7, SEQ ID NO:9, SEQ ID NO:11, SEQ ID NO: 13, SEQ ID NO: 15, SEQ ID NO: 17, SEQ ID NO: 19, SEQ ID NO:21, SEQ ID NO:23, SEQ ID NO:25, SEQ ID NO:27, SEQ ID NO:29, SEQ ID NO:31, SEQ ID NO:33, SEQ ID NO:35, SEQ ID NO:37, SEQ ID NO:39, SEQ ID NO:41, SEQ ID NO:43, SEQ ID NO:45, SEQ ID NO:47, SEQ ID NO:49, SEQ ID NO:51, SEQ ID NO.53, SEQ ID NO:55, SEQ ID NO:57, SEQ ID NO:59, SEQ ID NO:61, SEQ ID NO:63, SEQ ID NO:65, SEQ ID NO:67, SEQ ID NO:69, SEQ ID NO: 71, SEQ ID NO:73, SEQ ID NO:75, SEQ ID NO.77, SEQ ID NO:79, SEQ ID NO:81, SEQ ID NO:83, SEQ ID NO:85, SEQ ID NO:87, SEQ ID NO:89, SEQ ID NO:91, SEQ ID NO:93, SEQ ID NO:95, SEQ ID NO.97, SEQ ID NO:99, SEQ ID NO: 101, SEQ ID NO:103, SEQ ID NO:105, SEQ ID NO:107, SEQ ID NO:109, SEQ ID NO: 111, SEQ ID NO: 113, SEQ ID NO: 115, SEQ ID NO:117, SEQ ID NO:119, SEQ ID NO: 121, SEQ ID NO:123, SEQ ID NO: 125, SEQ ID N: 127, SEQ ID NO: 129, SEQ ID NO: 131, SEQ ID NO: 133, SEQ ID NO: 135, SEQ ID NO: 137, SEQ ID NO:139, SEQ ID NO:141, SEQ ID NO: 143, SEQ ID NO:145, SEQ ID NO:147, SEQ ID NO:149, SEQ ID NO:151, SEQ ID NO:153, SEQ ID NO:155, SEQ ID NO:157, SEQ ID NO: 159, SEQ ID NO: 161, SEQ ID NO: 163 and/or SEQ ID NO: 165, wherein a first member of the amplification primer pair comprises at least the first (the 5') 12 bases of the nucleic acid sequence of (a), (b) or (c), and a second member of the amplification primer comprises at least the first (the 5') 12 bases of the complementary strand of the nucleic acid sequence of (a), (b) or (c); (e) the nucleic acid sequence of (a), (b), (c) or (d), wherein the sequence does not encode a polypeptide containing a signal sequence (signal peptide); (f) the nucleic acid sequence of (a), (b), (c), (d) or (e), further comprising a heterologous sequence; (g) the nucleic acid sequence of (f), wherein the heterologous sequence comprises a signal sequence (signal peptide), and optionally the signal sequence (signal peptide) is derived from another hydrolase or a non-hydrolase (a heterologous) enzyme; (h) the nucleic acid sequence of (f), wherein the heterologous sequence comprises a prepro sequence and/ or catalytic domain (CD); (i) the nucleic acid sequence of (f), wherein the heterologous sequence comprises an N-terminal identification peptide, and optionally the N-terminal identification peptide imparts a desired characteristic, and optionally the desired characteristic is increased stability or simplified purification of the polypeptide; or (j) a nucleic acid sequence completely complementary to (a), (b), (c), (d), (e), (f), (g), (h) or (i).

2. The isolated, synthetic or recombinant nucleic acid of paragraph 1, wherein the sequence identity is over a region of at least about at least about 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%,, or more, or has 100% sequence identity (completely identical).

3. The isolated, synthetic or recombinant nucleic acid of paragraph 1 , wherein the sequence identity is over a region of at least about 50, 75, 100, 150, 200, 250, 300, 350, 400, 450, 500, 550, 600, 650, 700, 750, 800, 850, 900, 950, 1000, 1050, 1100, 1 150, 1200, 1250, 1300, 1350, 1400, 1450, 1500, 1550 or more residues, or the full length of a gene or transcript.

4. The isolated, synthetic or recombinant nucleic acid of paragraph 1, wherein the nucleic acid sequence comprises a sequence as set forth in SEQ ID NO: 1 , SEQ ID NO:3, SEQ ID NO:5, SEQ ID NO:7, SEQ ID NO:9, SEQ ID NO: 11, SEQ ID NO: 13, SEQ ID NO:15, SEQ ID NO:17, SEQ ID NO:19, SEQ ID NO:21, SEQ ID NO:23, SEQ ID NO: 25, SEQ ID NO:27, SEQ ID NO:29, SEQ ID NO:31, SEQ ID NO:33, SEQ ID NO:35, SEQ ID NO:37, SEQ ID NO: 39, SEQ ID NO:41, SEQ ID NO:43, SEQ ID NO:45, SEQ ID NO:47, SEQ ID NO:49, SEQ ID NO:51, SEQ ID NO: 53, SEQ ID NO:55, SEQ ID NO:57, SEQ ID NO:59, SEQ ID NO:61, SEQ ID NO:63, SEQ ID NO:65, SEQ ID NO: 67, SEQ ID NO:69, SEQ ID NO:71, SEQ ID NO:73, SEQ ID NO:75, SEQ ID NO:77, SEQ ID NO:79, SEQ ID NO: 81, SEQ ID NO:83, SEQ ID NO:85, SEQ ID NO:87, SEQ ID NO:89, SEQ ID NO:91, SEQ ID NO:93, SEQ ID NO: 95, SEQ ID NO:97, SEQ ID NO:99, SEQ ID NO: 101, SEQ ID NO: 103, SEQ ID NO:105, SEQ ID NO:107, SEQ ID NO:109, SEQ ID NO:111, SEQ ID NO:113, SEQ ID NO: 115, SEQ ID NO:117, SEQ ID NO:119, SEQ ID NO:121, SEQ ID NO:123, SEQ ID NO:125, SEQ ID NO:127, SEQ ID NO.129, SEQ ID NO:131, SEQ ID NO:133, SEQ ID NO:135, SEQ ID NO:137, SEQ ID NO.139, SEQ ID NO:141, SEQ ID NO:143, SEQ ID NO:145, SEQ ID NO:147, SEQ ID NO:149, SEQ ID NO:151, SEQ ID NO:153, SEQ ID NO: 155, SEQ ID NO: 157, SEQ ID NO: 159, SEQ ID NO: 161, SEQ ID NO: 163 and/or SEQ ID NO: 165.

5. The isolated, synthetic or recombinant nucleic acid of paragraph 1, wherein the nucleic acid sequence encodes a polypeptide having a sequence as set forth in SEQ ID NO:2, SEQ ID NO:4, SEQ ID NO:6, SEQ ID NO:8, SEQ ID NO:10, SEQ ID NO:12, SEQ ID NO:14, SEQ ID NO:16, SEQ ID NO:18, SEQ ID NO:20, SEQ ID NO:22, SEQ ID NO:24, SEQ ID NO:26, SEQ ID NO:28, SEQ ID NO:30, SEQ ID NO:32, SEQ ID NO:34, SEQ ID NO:36, SEQ ID NO:38, SEQ ID NO:40, SEQ ID N0:42, SEQ ID NO:44, SEQ ID NO:46, SEQ ID NO:48, SEQ ID NO:50, SEQ ID NO:52, SEQ ID NO:54, SEQ ID NO:56, SEQ ID NO:58, SEQ ID NO:60, SEQ ID NO:62, SEQ ID NO:64, SEQ ID NO:66, SEQ ID NO:68, SEQ ID NO:70, SEQ ID NO:72, SEQ ID NO:74, SEQ ID NO:76, SEQ ID NO:78, SEQ ID NO:80, SEQ ID NO:82, SEQ ID NO:84, SEQ ID NO:86, SEQ ID NO:88, SEQ ID NO:90, SEQ ID NO:92, SEQ ID NO:94, SEQ ID NO:96, SEQ ID NO:98, SEQ ID NO: 100, SEQ ID NO: 102, SEQ ID NO: 104, SEQ ID NO: 106, SEQ ID NO: 108, SEQ ID NO: 110, SEQ ID NO: 112, SEQ ID NO: 114, SEQ ID NO:116, SEQ ID NO: 118, SEQ ID NO: 120, SEQ ID NO: 122, SEQ ID NO: 124, SEQ ID NO: 126, SEQ ID NO: 128, SEQ ID NO: 130, SEQ ID NO: 132, SEQ ID NO:134, SEQ ID NO:136, SEQ ID NO:138, SEQ ID NO:140, SEQ ID NO:142, SEQ ID NO:143, SEQ ID NO:146, SEQ ID NO:148, SEQ ID NO:150, SEQ ID NO:152, SEQ ID NO: 154, SEQ ID NO: 156, SEQ ID NO: 158, SEQ ID NO: 160, SEQ ID NO: 162, SEQ ID NO: 164 and/or SEQ ID NO: 166.

6. The isolated, synthetic or recombinant nucleic acid of paragraph 1, wherein the sequence comparison algorithm is a BLAST version 2.2.2 algorithm where a filtering setting is set to blastall -p blastp -d "nr pataa" -F F, and all other options are set to default.

7. The isolated, synthetic or recombinant nucleic acid of paragraph 1, wherein the hydrolase activity comprises a lipase activity, a protease activity, an esterase activity or a phospholipase activity.

8. The isolated, synthetic or recombinant nucleic acid of paragraph 7, wherein the lipase activity comprises hydrolyzing a triacylglycerol to a diacylglycerol and a free fatty acid, or, hydrolyzing a triacylglycerol to a monoacylglycerol and free fatty acids, or, hydrolyzing a diacylglycerol to a monoacylglycerol and free fatty acids, or, hydrolyzing a monoacylglycerol to a free fatty acid and a glycerol, or, comprises hydrolyzing a triacylglycerol (TAG), a diacylglycerol (DAG) or a monoacylglycerol (MAG).

9. The isolated, synthetic or recombinant nucleic acid of paragraph 7, wherein the lipase activity comprises synthesizing a tryacylglycerol from a diacylglycerol or a monoacylglycerol and free fatty acids.

10. The isolated, synthetic or recombinant nucleic acid of paragraph 7, wherein the lipase activity comprises synthesizing 1,3-dipalmitoyl-2-oleoylglycerol (POP), 1,3- distearoyl-2-oleoylglycerol (SOS), 1-palmitoyl-2-oleoyl-3-stearoylglycerol (POS) or 1-oleoyl-2,3-dimyristoylglycerol (OMM), long chain polyunsaturated fatty acids, arachidonic acid, docosahexaenoic acid (DHA) or eicosapentaenoic acid (EPA).

11. The isolated, synthetic or recombinant nucleic acid of paragraph 7, wherein the lipase activity is triacylglycerol (TAG), diacylglycerol (DAG) or monoacylglycerol (MAG) position - specific.

12. The isolated, synthetic or recombinant nucleic acid of paragraph 11, wherein the lipase activity is Sn2-specific, Sn1- or Sn3-specific.

13. The isolated, synthetic or recombinant nucleic acid of paragraph 7, wherein the lipase activity is fatty acid specific.

14. The isolated, synthetic or recombinant nucleic acid of paragraph 7, wherein the lipase activity comprises modifying oils by hydrolysis, alcoholysis, esterification, transesterification or interesterification.

15. The isolated, synthetic or recombinant nucleic acid of paragraph 7, wherein the lipase activity is regio-specifc or chemoselective.

16. The isolated, synthetic or recombinant nucleic acid of paragraph 15, wherein the lipase activity comprises synthesis of enantiomerically pure chiral products.

17. The isolated, synthetic or recombinant nucleic acid of paragraph 7, wherein the lipase activity comprises synthesis of umbelliferyl fatty acid (FA) esters.

18. The isolated, synthetic or recombinant nucleic acid of paragraph 7, wherein the lipase activity is thermostable.

19. The isolated, synthetic or recombinant nucleic acid of paragraph 18, wherein the polypeptide retains a lipase activity under conditions comprising a temperature range of between about 37°C to about 95°C, or between about 55°C to about 85°C, or between about 70°C to about 95°C, or between about 90°C to about 95°C.

20. The isolated, synthetic or recombinant nucleic acid of paragraph 1, wherein the hydrolase activity is thermotolerant.

21. The isolated, synthetic or recombinant nucleic acid of paragraph 20, wherein the polypeptide retains a hydrolase activity after exposure to a temperature in the range from greater than 37°C to about 95°C, from greater than 55°C to about 85°C, or from greater than 90°C to about 95°C.

22. An isolated, synthetic or recombinant nucleic acid, wherein the nucleic acid comprises a sequence that hybridizes under stringent conditions to a nucleic acid comprising SEQ ID NO:1, SEQ ID NO:3, SEQ ID NO:5, SEQ ID NO:7, SEQ ID NO:9, SEQ ID NO:11, SEQ ID NO:13, SEQ ID NO:15, SEQ ID NO:17, SEQ ID NO:19, SEQ ID NO:21, SEQ ID NO:23, SEQ ID NO:25, SEQ ID NO:27, SEQ ID NO:29, SEQ ID NO:31, SEQ ID NO:33, SEQ ID NO:35, SEQ ID NO:37, SEQ ID NO:39, SEQ ID NO:41 , SEQ ID NO:43, SEQ ID NO:45, SEQ ID NO:47, SEQ ID NO:49, SEQ ID NO:51, SEQ ID NO:53, SEQ ID NO:55, SEQ ID NO:57, SEQ ID NO:59, SEQ ID NO:61, SEQ ID NO:63, SEQ ID NO:65, SEQ ID NO:67, SEQ ID NO:69, SEQ ID NO:71, SEQ ID NO:73, SEQ ID NO:75, SEQ ID NO:77, SEQ ID NO:79, SEQ ID NO:81, SEQ ID NO:83, SEQ ID NO:85, SEQ ID NO:87, SEQ ID NO:89, SEQ ID NO:91 , SEQ ID NO:93, SEQ ID NO:95, SEQ ID NO:97, SEQ ID NO:99, SEQ ID NOrlOl, SEQ ID NO:103, SEQ ID NO:105, SEQ ID NO:107, SEQ ID NO:109, SEQ ID NO:111, SEQ ID NO:113, SEQ ID NO:115, SEQ ID NO:117, SEQ ID NO:119, SEQ ID NO:121, SEQ ID NO:123, SEQ ID NO:125, SEQ ID NO:127, SEQ ID NO:129, SEQ ID NO:131, SEQ ID NO:133, SEQ ID NO:135, SEQ ID NO:137, SEQ ID NO.139, SEQ ID NO:141, SEQ ID NO:143, SEQ ID NO:145, SEQ ID NO:147, SEQ ID NO:149, SEQ ID NO:151, SEQ ID NO:153, SEQ ID NO:155, SEQ IDNO:157, SEQ ID NO:159, SEQ ID NO: 161 , SEQ ID NO: 163 and/or SEQ ID NO: 165, wherein the nucleic acid encodes a polypeptide having a hydrolase activity.

23. The isolated, synthetic or recombinant nucleic acid of paragraph 23, wherein the nucleic acid is at least about 50, 75, 100, 150, 200, 300, 400, 500, 600, 700, 800, 900, 1000 or more residues in length or the full length of the gene or transcript.

24. The isolated, synthetic or recombinant nucleic acid of paragraph 22, wherein the stringent conditions include a wash step comprising a wash in 0.2X SSC at a temperature of about 65°C for about 15 minutes.

25. A nucleic acid probe for identifying a nucleic acid encoding a polypeptide with a hydrolase activity, wherein the probe comprises at least 10 consecutive bases of a sequence as set forth in paragraph 1, wherein the probe identifies the nucleic acid by binding or hybridization.

26. The nucleic acid probe of paragraph 25, wherein the probe comprises an oligonucleotide comprising at least about 10 to 50, about 20 to 60, about 30 to 70, about 40 to 80, about 60 to 100, or about 50 to 150 consecutive bases.

27. A nucleic acid probe for identifying a nucleic acid encoding a polypeptide having a hydrolase activity, wherein the probe comprises a nucleic acid comprising at least about 10 consecutive residues of SEQ ID NO:1, SEQ ID NO:3, SEQ ID NO:5, SEQ ID NO:7, SEQ ID NO:9, SEQ ID NO:11, SEQ ID NO:13, SEQ ID NO:15, SEQ ID NO: 17, SEQ ID NO: 19, SEQ ID NO:21, SEQ ID NO:23, SEQ ID NO:25, SEQ ID NO:27, SEQ ID NO:29, SEQ ID NO: 31, SEQ ID NO:33, SEQ ID NO:35, SEQ ID NO:37, SEQ ID NO:39, SEQ ID NO:41 , SEQ ID NO:43, SEQ ID NO: 45, SEQ ID NO:47, SEQ ID NO:49, SEQ ID NO:51, SEQ ID NO:53, SEQ ID NO:55, SEQ ID NO:57, SEQ ID NO: 59, SEQ ID NO:61, SEQ ID NO:63, SEQ ID NO:65, SEQ ID NO:67, SEQ ID NO:69, SEQ ID NO:71, SEQ ID NO: 73, SEQ ID NO:75, SEQ ID NO:77, SEQ ID NO:79, SEQ ID NO:81, SEQ ID NO:83, SEQ ID NO:85, SEQ ID NO: 87, SEQ ID NO:89, SEQ ID NO:91, SEQ ID NO:93, SEQ ID NO:95, SEQ ID NO:97, SEQ ID NO:99, SEQ ID NO: 101, SEQ ID NO:103, SEQ ID NO:105, SEQ ID NO:107, SEQ ID NO:109, SEQ ID NO:111, SEQ ID NO:113, SEQ ID NO:115, SEQ ID NO:117, SEQ ID NO:119, SEQ ID NO:121, SEQ ID NO:123, SEQ ID NO:125, SEQ ID NO:127, SEQ ID NO:129, SEQ ID NO:131, SEQ ID NO: 133, SEQ ID NO:135, SEQ ID NO: 137, SEQ ID NO:139, SEQ ID NO:141, SEQ ID NO: 143, SEQ ID NO:145, SEQ ID NO:147, SEQ ID NO:149, SEQ ID NO:151, SEQ ID NO:153, SEQ ID NO:155, SEQ ID NO:157, SEQ ID NO:159, SEQ ID NO:161, SEQ ID NO:163 and/or SEQ ID NO:165, wherein the sequence identities are determined by analysis with a sequence comparison algorithm or by visual inspection.

28. The nucleic acid probe of paragraph 27, wherein the probe comprises an oligonucleotide comprising at least about 10 to 50, about 20 to 60, about 30 to 70, about 40 to 80, about 60 to 100, or about 50 to 150 consecutive bases.

29. An amplification primer sequence pair for amplifying a nucleic acid encoding a polypeptide having a hydrolase activity, wherein the primer pair is capable of amplifying a nucleic acid comprising a sequence as set forth in paragraph 1 or paragraph 22, or a subsequence thereof.

30. The amplification primer pair of paragraph 29, wherein each member of the amplification primer sequence pair comprises an oligonucleotide comprising at least about 10 to 50 consecutive bases of the sequence.

31. A method of amplifying a nucleic acid encoding a polypeptide having a hydrolase activity comprising amplification of a template nucleic acid with an amplification primer sequence pair capable of amplifying a nucleic acid sequence as set forth in paragraph 1 or paragraph 22, or a subsequence thereof.

32. An expression cassette comprising a nucleic acid comprising a sequence as set forth in paragraph 1 or paragraph 22.

33. A vector comprising a nucleic acid comprising a sequence as set forth in paragraph 1 or paragraph 22.

34. A cloning vehicle comprising a nucleic acid comprising a sequence as set forth in paragraph 1 or paragraph 22, wherein the cloning vehicle comprises a viral vector, a plasmid, a phage, a phagemid, a cosmid, a fosmid, a bacteriophage or an artificial chromosome.

35. The cloning vehicle of paragraph 34, wherein the viral vector comprises an adenovirus vector, a retroviral vector or an adeno-associated viral vector.

36. The cloning vehicle of paragraph 34, comprising a bacterial artificial chromosome (BAC), a plasmid, a bacteriophage P1 -derived vector (PAC), a yeast artificial chromosome (YAC), or a mammalian artificial chromosome (MAC).

37. A transformed cell comprising a nucleic acid comprising a sequence as set forth in paragraph 1 or paragraph 22.

38. A transformed cell comprising an expression cassette as set forth in paragraph 32.

39. The transformed cell of paragraph 37 or paragraph 38, wherein the cell is a bacterial cell, a mammalian cell, a fungal cell, a yeast cell, an insect cell or a plant cell.

40. A transgenic non-human animal comprising a sequence as set forth in paragraph 1 or paragraph 22.

41. The transgenic non-human animal of paragraph 40, wherein the animal is a mouse.

42. A transgenic plant comprising a sequence as set forth in paragraph 1 or paragraph 22.

43. The transgenic plant of paragraph 42, wherein the plant is a corn plant, a sorghum plant, a potato plant, a tomato plant, a wheat plant, an oilseed plant, a rapeseed plant, a soybean plant, a rice plant, a barley plant, a grass, or a tobacco plant.

44. A transgenic seed comprising a sequence as set forth in paragraph 1 or paragraph 22.

45. The transgenic seed of paragraph 44, wherein the seed is rice, a corn seed, a wheat kernel, an oilseed, a rapeseed, a soybean seed, a palm kernel, a sunflower seed, a sesame seed, a rice, a barley, a peanut or a tobacco plant seed.

46. An antisense oligonucleotide comprising a nucleic acid sequence complementary to or capable of hybridizing under stringent conditions to a sequence as set forth in paragraph 1 or paragraph 22, or a subsequence thereof.

47. The antisense oligonucleotide of paragraph 46, wherein the antisense oligonucleotide is between about 10 to 50, about 20 to 60, about 30 to 70, about 40 to 80, or about 60 to 100 bases in length.

48. A method of inhibiting the translation of a hydrolase message in a cell comprising administering to the cell or expressing in the cell an antisense oligonucleotide comprising a nucleic acid sequence complementary to or capable of hybridizing under stringent conditions to a sequence as set forth in paragraph 1 or paragraph 22.

49. A double-stranded inhibitory RNA (RNAi) molecule comprising a subsequence of a sequence as set forth in paragraph 1 or paragraph 22.

50. The double-stranded inhibitory RNA (RNAi) molecule of paragraph 49, wherein the RNAi is about 15, 16, 17, 18, 19, 20, 21, 22, 23, 24, 25 or more duplex nucleotides in length.

51. A method of inhibiting the expression of a hydrolase in a cell comprising administering to the cell or expressing in the cell a double-stranded inhibitory RNA (iRNA), wherein the RNA comprises a subsequence of a sequence as set forth in paragraph 1 or paragraph 22.

52. An isolated, synthetic or recombinant polypeptide

(a) having at least 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99% or more sequence identity to SEQ ID NO:2, SEQ ID NO:4, SEQ ID NO:6, SEQ ID NO:8, SEQ ID NO:10, SEQ ID NO: 12, SEQ ID NO:14, SEQ ID NO:16, SEQ ID NO:18, SEQ ID NO.20, SEQ ID NO:22, SEQ ID NO:24, SEQ ID NO:26, SEQ ID NO:28, SEQ ID NO:30, SEQ ID NO:32, SEQ ID NO:34, SEQ ID NO:36, SEQ ID NO:38, SEQ ID NO:40, SEQ ID NO.42, SEQ ID NO:44, SEQ ID NO:46, SEQ ID NO:48, SEQ ID NO:50, SEQ ID NO:52, SEQ ID NO: 54, SEQ ID NO:56, SEQ ID NO:58, SEQ ID NO:60, SEQ ID NO:62, SEQ ID NO:64, SEQ ID NO:66, SEQ ID NO:68, SEQ ID NO:70, SEQ ID NO:72, SEQ ID NO:74, SEQ ID NO:76, SEQ ID NO:78, SEQ ID NO:80, SEQ ID NO:82, SEQ ID NO:84, SEQ ID NO:86, SEQ ID NO:88, SEQ ID NO:90, SEQ ID NO:92, SEQ ID NO:94, SEQ ID NO:96, SEQ ID NO:98, SEQ ID NO:100, SEQ ID NO:102, SEQ ID NO: 104, SEQ ID NO: 106, SEQ ID NO: 108, SEQ ID NO: 110, SEQ ID NO: 112, SEQ ID NO:114, SEQ ID NO:116, SEQ ID NO:118, SEQ ID NO: 120, SEQ ID NO:122, SEQ ID NO:124, SEQ ID NO:126, SEQ ID NO:128, SEQ ID NO:130, SEQ ID NO:132, SEQ ID NO:134, SEQ ID NO:136, SEQ ID NO:138, SEQ ID NO:140, SEQ ID NO:142, SEQ ID NO:143, SEQ ID NO:146, SEQ ID NO:148, SEQ ID NO:150, SEQ ID NO:152, SEQ ID NO: 154, SEQ ID NO: 156, SEQ ID NO: 158, SEQ ID NO: 160, SEQ ID NO: 162, SEQ ID NO:164 and/or SEQ ID NO:166, over a region of at least about 10, 15, 20, 25, 30, 35, 40, 45, 50, 75, 100, 150, 200, 250, 300, 350 or 400 or more residues, wherein optionally the sequence identities are determined by analysis with a sequence comparison algorithm or by a visual inspection;
(b) encoded by a nucleic acid as set forth in paragraph 1 or paragraph 22;
(c) the amino acid sequence of (a) or (b), having at least one conservative amino acid substitution, wherein the conservative amino acid substitution substitutes one amino acid for another of the same class, and optionally the at least one conservative amino acid substitution is: substitution of one hydrophobic amino acid for another; substitution of isoleucine, valine, leucine or methionine for an isoleucine, valine, leucine or methionine; substitution of one polar amino acid for another; substitution of arginine for lysine, glutamic acid for aspartic acid or glutamine for asparagine; wherein the polypeptide having the at least one conservative amino acid substitution has a hydrolase activity or can generate a humoral immune response to make an anti-hydrolase antibody;
(d) a hydrolase encoded by a nucleic acid generated by amplification of a polynucleotide using an amplification primer pair, wherein the primer pair is capable of amplifying the nucleic acid sequence of paragraph 1, wherein a first member of the amplification primer pair comprises at least the first (the 5') 12 bases of the nucleic acid sequence of paragraph 1, and a second member of the amplification primer comprises at least the first (the 5') 12 bases of the complementary strand of the nucleic acid sequence of paragraph 1;
(e) the amino acid sequence of (a), (b), (c) or (d), wherein the polypeptide does not contain a signal sequence (signal peptide);
(f) the amino acid sequence of (a), (b), (c), (d) or (e), further comprising a heterologous amino acid sequence;
(g) the amino acid sequence of (f), wherein the heterologous amino acid sequence comprises a signal sequence (signal peptide), and optionally the signal sequence (signal peptide) is derived from another hydrolase or a non-hydrolase (a heterologous) enzyme;
(h) the amino acid sequence of (f), wherein the heterologous amino acid sequence comprises a prepro sequence and/ or catalytic domain (CD), or a binding domain, an epitope or a tag; (i) the amino acid sequence of (f), wherein the heterologous amino acid sequence comprises an N-terminal identification peptide, and optionally the N-terminal identification peptide imparts a desired characteristic, and optionally the desired characteristic is increased stability or simplified purification of the polypeptide.

53. The isolated, synthetic or recombinant polypeptide of paragraph 52, wherein the sequence identity is over a region of at least about at least about 51%, 52%, 53%, 54%, 55%, 56%, 57%, 58%, 59%, 60%, 61%, 62%, 63%, 64%, 65%, 66%, 67%, 68%, 69%, 70%, 71%. 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%,, or more, or has 100% sequence identity (is completely identical).

54. The isolated, synthetic or recombinant polypeptide of paragraph 52, wherein the sequence identity is over a region of at least about 10, 15, 20, 25, 30, 35, 40, 45, 50, 75, 100, 150, 200, 250, 300, 350, 400, 450, 500, 550, 600 or more residues, or the full length of an enzyme.

55. The isolated, synthetic or recombinant polypeptide of paragraph 52, wherein the polypeptide has a sequence as set forth in SEQ ID NO:2, SEQ ID NO:4, SEQ ID NO:6, SEQ ID NO:8, SEQ ID NO: 10, SEQ ID NO: 12, SEQ ID NO: 14, SEQ ID NO: 16, SEQ ID NO: 18, SEQ ID NO:20, SEQ ID NO:22, SEQ ID NO:24, SEQ ID NO:26, SEQ ID NO:28, SEQ ID NO:30, SEQ ID NO:32, SEQ ID NO:34, SEQ ID NO:36, SEQ ID NO:38, SEQ ID NO:40, SEQ ID NO:42, SEQ ID NO:44, SEQ ID NO:46, SEQ ID NO:48, SEQ ID NO:50, SEQ ID NO:52, SEQ ID NO:54, SEQ ID NO:56, SEQ ID NO:58, SEQ ID NO:60, SEQ ID NO:62, SEQ ID NO:64, SEQ ID NO:66, SEQ ID NO:68, SEQ ID NO:70, SEQ ID NO:72, SEQ ID NO:74, SEQ ID NO:76, SEQ ID NO:78, SEQ ID NO:80, SEQ ID NO:82, SEQ ID NO:84, SEQ ID NO:86, SEQ ID NO:88, SEQ ID NO:90, SEQ ID NO:92, SEQ ID NO:94, SEQ ID NO:96, SEQ ID NO:98, SEQ ID NO:100, SEQ ID NO:102, SEQ ID NO:104, SEQ ID NO:106, SEQ ID NO: 108, SEQ ID NO:110, SEQ ID NO:112, SEQ ID NO:114, SEQ ID NO:116, SEQ ID NO:118, SEQ ID NO: 120, SEQ ID NO: 122, SEQ ID NO: 124, SEQ ID NO: 126, SEQ ID NO:128, SEQ ID NO:130, SEQ ID NO:132, SEQ ID NO:134, SEQ ID NO:136, SEQ ID NO:138, SEQ ID NO:140, SEQ ID NO:142, SEQ ID NO:143, SEQ ID NO:146, SEQ ID NO:148, SEQ ID NO:150, SEQ ID NO:152, SEQ ID NO:154, SEQ ID NO:156, SEQ ID NO: 158, SEQ ID NO: 160, SEQ ID NO: 162, SEQ ID NO: 164 and/or SEQ ID NO: 166.

56. The isolated, synthetic or recombinant polypeptide of paragraph 52, wherein the polypeptide has a hydrolase activity.

57. The isolated, synthetic or recombinant polypeptide of paragraph 52, wherein the hydrolase activity comprises an esterase activity, a lipase activity, a phospholipase activity or a protease activity.

58. The isolated, synthetic or recombinant polypeptide of paragraph 57, wherein the lipase activity comprises hydrolyzing a triacyl glycerol to a diacyl glycerol and a free fatty acid, or, hydrolyzing a triacylglycerol to a monoacylglycerol and free fatty acids, or, hydrolyzing a diacylglycerol to a monoacylglycerol and free fatty acids, or, hydrolyzing a monoacylglycerol to a free fatty acid and a glycerol, or, comprises hydrolyzing a triacylglycerol (TAG), a diacylglycerol (DAG) or a monoacylglycerol (MAG).

59. The isolated, synthetic or recombinant polypeptide of paragraph 57, wherein the lipase activity comprises synthesizing a tryacylglycerol from a diacylglycerol or a monoacylglycerol and free fatty acids.

60. The isolated, synthetic or recombinant polypeptide of paragraph 57, wherein the lipase activity comprises synthesizing1,3-dipalmitoyl-2-oleoylglycerol (POP), 1,3- distearoyl-2-oleoylglycerol (SOS), 1-palmitoyl-2-oleoyl-3-stearoyl glycerol (POS) or 1-oleoyl-2,3-dimyristoyl glycerol (OMM), long chain polyunsaturated fatty acids, arachidonic acid, docosahexaenoic acid (DHA) or eicosapentaenoic acid (EPA).

61. The isolated, synthetic or recombinant polypeptide of paragraph 57, wherein the lipase activity is triacylglycerol (TAG), diacylglycerol (DAG) or monoacylglycerol (MAG) position - specific.

62. The isolated, synthetic or recombinant polypeptide of paragraph 61, wherein the lipase activity is Sn2-specific, Sn1- or Sn3 -specific.

63. The isolated, synthetic or recombinant polypeptide of paragraph 57, wherein the lipase activity is fatty acid specific.

64. The isolated, synthetic or recombinant polypeptide of paragraph 57, wherein the lipase activity comprises modifying oils by hydrolysis, alcoholysis, esterification, transesterification or interesterification.

65. The isolated, synthetic or recombinant polypeptide of paragraph 57, wherein the hydrolase activity is regio-specific or chemoselective.

66. The isolated, synthetic or recombinant polypeptide of paragraph 65, wherein the hydrolase activity comprises synthesis of enantiomerically pure chiral products.

67. The isolated, synthetic or recombinant polypeptide of paragraph 57, wherein the hydrolase activity comprises synthesis of umbelliferyl fatty acid (FA) esters.

68. The isolated, synthetic or recombinant polypeptide of paragraph 57, wherein the hydrolase activity is thermostable.

69. The isolated, synthetic or recombinant polypeptide of paragraph 68, wherein the polypeptide retains a hydrolase activity under conditions comprising a temperature range of between about 37°C to about 95°C, between about 55°C to about 85°C, between about 70°C to about 95°C, or between about 90°C to about 95°C.

70. The isolated, synthetic or recombinant polypeptide of paragraph 56, wherein the hydrolase activity is thermotolerant.

71. The isolated, synthetic or recombinant polypeptide of paragraph 70, wherein the polypeptide retains a hydrolase activity after exposure to a temperature in the range from greater than 37°C to about 95°C, from greater than 55°C to about 85°C, or from greater than 90°C to about 95°C.

72. An isolated, synthetic or recombinant polypeptide comprising a polypeptide as set forth in paragraph 52 and lacking a signal sequence.

73. An isolated, synthetic or recombinant polypeptide comprising a polypeptide as set forth in paragraph 52 and having a heterologous signal sequence.

74. The isolated, synthetic or recombinant polypeptide of paragraph 56, wherein the hydrolase activity comprises a specific activity at about 37°C in the range from about 100 to about 1000 units per milligram of protein, from about 500 to about 750 units per milligram of protein, from about 500 to about 1200 units per milligram of protein, or from about 750 to about 1000 units per milligram of protein.

75. The isolated, synthetic or recombinant polypeptide of paragraph 70, wherein the thermotolerance comprises retention of at least half of the specific activity of the hydrolase at 37°C after being heated to an elevated temperature.

76. The isolated, synthetic or recombinant polypeptide of paragraph 70, wherein the thermotolerance comprises retention of specific activity at 37°C in the range from about 500 to about 1200 units per milligram of protein after being heated to an elevated temperature.

77. The isolated, synthetic or recombinant polypeptide of paragraph 52, wherein the polypeptide comprises at least one glycosylation site.

78. The isolated, synthetic or recombinant polypeptide of paragraph 77, wherein the glycosylation is an N-linked glycosylation.

79. The isolated, synthetic or recombinant polypeptide of paragraph 78, wherein the polypeptide is glycosylated after being expressed in a *P. pastoris* or a *S. pombe.*

80. The isolated, synthetic or recombinant polypeptide of paragraph 56, wherein the polypeptide retains a hydrolase activity under conditions comprising about pH 6.5, pH 6.0, pH 5.5, 5.0, pH 4.5 or 4.0.

81. The isolated, synthetic or recombinant polypeptide of paragraph 56, wherein the polypeptide retains a hydrolase activity under conditions comprising about pH 8.0, pH 8.5, pH 9, pH 9.5, pH 10 or pH 10.5.

82. A protein preparation comprising a polypeptide as set forth in paragraph 52, wherein the protein preparation comprises a liquid, a solid or a gel.

83. A heterodimer comprising a polypeptide as set forth in paragraph 52 and a second domain.

84. The heterodimer of paragraph 83, wherein the second domain is a polypeptide and the heterodimer is a fusion protein.

85. The heterodimer of paragraph 84, wherein the second domain is an epitope or a tag.

86. A homodimer comprising a polypeptide as set forth in paragraph 52.

87. An immobilized polypeptide, wherein the polypeptide comprises a sequence as set forth in paragraph 52, or a subsequence thereof.

88. The immobilized polypeptide of paragraph 87, wherein the polypeptide is immobilized on a cell, a metal, a resin, a polymer, a ceramic, a glass, a microelectrode, a graphitic particle, a bead, a gel, a plate, an array or a capillary tube.

89. An array comprising an immobilized polypeptide as set forth in paragraph 52.

90. An array comprising an immobilized nucleic acid as set forth in paragraph 1 or paragraph 22.

91. An isolated, synthetic or recombinant antibody that specifically binds to a polypeptide as set forth in paragraph 52.

92. The isolated, synthetic or recombinant antibody of paragraph 91, wherein the antibody is a monoclonal or a polyclonal antibody.

93. A hybridoma comprising an antibody that specifically binds to a polypeptide as set forth in paragraph 52.

94. A food supplement for an animal comprising a polypeptide as set forth in paragraph 52, or a subsequence thereof.

95. The food supplement of paragraph 94, wherein the polypeptide is glycosylated.

96. An edible enzyme delivery matrix comprising a polypeptide as set forth in paragraph 52.

97. The edible enzyme delivery matrix of paragraph 96, wherein the delivery matrix comprises a pellet.

98. The edible enzyme delivery matrix of paragraph 97, wherein the polypeptide is glycosylated.

99. The edible enzyme delivery matrix of paragraph 96, wherein the polypeptide has a thermotolerant or a thermostable hydrolase activity.

100. A method of isolating or identifying a polypeptide with a hydrolase activity comprising the steps of:

(a) providing an antibody as set forth in paragraph 91;
(b) providing a sample comprising polypeptides; and
(c) contacting the sample of step (b) with the antibody of step (a) under conditions wherein the antibody can specifically bind to the polypeptide, thereby isolating or identifying a polypeptide having a hydrolase activity.

101. A method of making an anti-hydrolase antibody comprising administering to a non-human animal a nucleic acid as set forth in paragraph 1 or paragraph 22 or a subsequence thereof in an amount sufficient to generate a humoral immune response, thereby making an anti-hydrolase antibody.

102. A method of making an anti-hydrolase antibody comprising administering to a non-human animal a polypeptide as set forth in paragraph 52 or a subsequence thereof in an amount sufficient to generate a humoral immune response, thereby making an anti- hydrolase antibody.

103. A method of producing a recombinant polypeptide comprising the steps of: (a) providing a nucleic acid operably linked to a promoter, wherein the nucleic acid comprises a sequence as set forth in paragraph 1 or paragraph 22; and (b) expressing the nucleic acid of step (a) under conditions that allow expression of the polypeptide, thereby producing a recombinant polypeptide.

104. The method of paragraph 103, further comprising transforming a host cell with the nucleic acid of step (a) followed by expressing the nucleic acid of step (a), thereby producing a recombinant polypeptide in a transformed cell.

105. A method for identifying a polypeptide having a hydrolase activity comprising the following steps:

(a) providing a polypeptide as set forth in paragraph 56;
(b) providing a hydrolase substrate; and
(c) contacting the polypeptide with the substrate of step (b) and detecting a decrease in the amount of substrate or an increase in the amount of a reaction product, wherein a decrease in the amount of the substrate or an increase in the amount of the reaction product detects a polypeptide having a hydrolase activity.

106. The method of paragraph 105, wherein the substrate is a fatty acid, a triacyl glycerol (TAG), a diacylglycerol (DAG) or a monoacylglycerol (MAG).

107. A method for identifying a hydrolase substrate comprising the following steps:

(a) providing a polypeptide as set forth in paragraph 56;
(b) providing a test substrate; and
(c) contacting the polypeptide of step (a) with the test substrate of step (b) and detecting a decrease in the amount of substrate or an increase in the amount of reaction product, wherein a decrease in the amount of the substrate or an increase in the amount of a reaction product identifies the test substrate as a hydrolase substrate.

108. A method of determining whether a test compound specifically binds to a polypeptide comprising the following steps:

(a) expressing a nucleic acid or a vector comprising the nucleic acid under conditions permissive for translation of the nucleic acid to a polypeptide, wherein the nucleic acid has a sequence as set forth in paragraph 1 or paragraph 22;
(b) providing a test compound;
(c) contacting the polypeptide with the test compound; and
(d) determining whether the test compound of step (b) specifically binds to the polypeptide.

109. A method of determining whether a test compound specifically binds to a polypeptide comprising the following steps:

(a) providing a polypeptide having a sequence as set forth in paragraph 52;
(b) providing a test compound;
(c) contacting the polypeptide with the test compound; and
(d) determining whether the test compound of step (b) specifically binds to the polypeptide.

110. A method for identifying a modulator of a hydrolase activity comprising the following steps:

(a) providing a polypeptide as set forth in paragraph 56;
(b) providing a test compound;
(c) contacting the polypeptide of step (a) with the test compound of step (b) and measuring an activity of the hydrolase, wherein a change in the hydrolase activity measured in the presence of the test compound compared to the activity in the absence of the test compound provides a determination that the test compound modulates the hydrolase activity.

111. The method of paragraph 110, wherein the hydrolase activity is measured by providing a hydrolase substrate and detecting a decrease in the amount of the substrate or an increase in the amount of a reaction product, or, an increase in the amount of the substrate or a decrease in the amount of a reaction product.

112. The method of paragraph 111, wherein a decrease in the amount of the substrate or an increase in the amount of the reaction product with the test compound as compared to the amount of substrate or reaction product without the test compound identifies the test compound as an activator of hydrolase activity.

113. The method of paragraph 111, wherein an increase in the amount of the substrate or a decrease in the amount of the reaction product with the test compound as compared to the amount of substrate or reaction product without the test compound identifies the test compound as an inhibitor of hydrolase activity.

114. A computer system comprising a processor and a data storage device wherein said data storage device has stored thereon a polypeptide sequence or a nucleic acid sequence, wherein the polypeptide sequence comprises sequence as set forth in paragraph 52, a polypeptide encoded by a nucleic acid as set forth in paragraph 1 or paragraph 22.

115. The computer system of paragraph 114, further comprising a sequence comparison algorithm and a data storage device having at least one reference sequence stored thereon.

116. The computer system of paragraph 115, wherein the sequence comparison algorithm comprises a computer program that indicates polymorphisms.

117. The computer system of paragraph 114, further comprising an identifier that identifies one or more features in said sequence.

118. A computer readable medium having stored thereon a polypeptide sequence or a nucleic acid sequence, wherein the polypeptide sequence comprises a polypeptide as set forth in paragraph 52; a polypeptide encoded by a nucleic acid as set forth in paragraph 1 or paragraph 22.

119. A method for identifying a feature in a sequence comprising the steps of: (a) reading the sequence using a computer program which identifies one or more features in a sequence, wherein the sequence comprises a polypeptide sequence or a nucleic acid sequence, wherein the polypeptide sequence comprises a polypeptide as set forth in paragraph 52; a polypeptide encoded by a nucleic acid as set forth in paragraph 1 or paragraph 22; and (b) identifying one or more features in the sequence with the computer program.

120. A method for comparing a first sequence to a second sequence comprising the steps of: (a) reading the first sequence and the second sequence through use of a computer program which compares sequences, wherein the first sequence comprises a polypeptide sequence or a nucleic acid sequence, wherein the polypeptide sequence comprises a polypeptide as set forth in paragraph 52 or a polypeptide encoded by a nucleic acid as set forth in paragraph 1 or paragraph 22; and (b) determining differences between the first sequence and the second sequence with the computer program.

121. The method of paragraph 120, wherein the step of determining differences between the first sequence and the second sequence further comprises the step of identifying polymorphisms.

122. The method of paragraph 120, further comprising an identifier that identifies one or more features in a sequence.

123. The method of paragraph 122, comprising reading the first sequence using a computer program and identifying one or more features in the sequence.

124. A method for isolating or recovering a nucleic acid encoding a polypeptide with a hydrolase activity from an environmental sample comprising the steps of:

(a) providing an amplification primer sequence pair as set forth in paragraph 29; (b) isolating a nucleic acid from the environmental sample or treating the environmental sample such that nucleic acid in the sample is accessible for hybridization to the amplification primer pair; and, (c) combining the nucleic acid of step (b) with the amplification primer pair of step (a) and amplifying nucleic acid from the environmental sample, thereby isolating or recovering a nucleic acid encoding a polypeptide with a hydrolase activity from an environmental sample.

125. The method of paragraph 124, wherein each member of the amplification primer sequence pair comprises an oligonucleotide comprising at least about 10 to 50 consecutive bases of a sequence as set forth in SEQ ID NO:1, SEQ ID NO: 3, SEQ ID NO:5, SEQ ID NO:7, SEQ ID NO:9, SEQ ID NO:11, SEQ ID NO: 13, SEQ ID NO: 15, SEQ ID NO: 17, SEQ ID NO: 19, SEQ ID NO:21, SEQ ID NO:23, SEQ ID NO:25, SEQ ID NO:27, SEQ ID NO:29, SEQ ID NO:31, SEQ ID NO:33, SEQ ID NO:35, SEQ ID NO:37, SEQ ID NO:39, SEQ ID NO:41, SEQ ID NO:43, SEQ ID NO:45, SEQ ID NO:47, SEQ ID NO:49, SEQ ID NO:51, SEQ ID NO:53, SEQ ID NO:55, SEQ ID NO:57, SEQ ID NO:59, SEQ ID NO:61, SEQ ID NO:63, SEQ ID NO:65, SEQ ID NO:67, SEQ ID NO:69, SEQ ID NO:71, SEQ ID NO:73, SEQ ID NO:75, SEQ ID NO:77, SEQ ID NO:79, SEQ ID NO:81, SEQ ID NO:83, SEQ ID NO:85, SEQ ID NO:87, SEQ ID NO:89, SEQ ID NO:91, SEQ ID NO:93, SEQ ID NO:95, SEQ ID NO:97, SEQ ID NO:99, SEQ ID NO: 101, SEQ ID NO: 103, SEQ ID NO: 105, SEQ ID NO:107, SEQ ID NO:109, SEQ ID NO:111, SEQ ID NO:

113, SEQ ID NO:115, SEQ ID NO:117, SEQ ID NO:119, SEQ ID NO:121, SEQ ID NO:123, SEQ ID NO:125, SEQ ID NO:127, SEQ ID NO:129, SEQ ID NO:131, SEQ ID NO:133, SEQ ID NO:135, SEQ ID NO: 137, SEQ ID NO: 139, SEQ ID NO:141, SEQ ID NO:143, SEQ ID NO:145, SEQ ID NO:147, SEQ ID NO:149, SEQ ID NO: 151, SEQ ID NO:153, SEQ ID NO:155, SEQ ID NO: 157, SEQ ID NO: 159, SEQ ID NO:161, SEQ ID NO: 163 and/or SEQ ID NO: 165, or a subsequence thereof.

126. A method for isolating or recovering a nucleic acid encoding a polypeptide with a hydrolase activity from an environmental sample comprising the steps of:

(a) providing a polynucleotide probe comprising a sequence as set forth in paragraph 1 or paragraph 22, or a subsequence thereof;
(b) isolating a nucleic acid from the environmental sample or treating the environmental sample such that nucleic acid in the sample is accessible for hybridization to a polynucleotide probe of step (a);
(c) combining the isolated nucleic acid or the treated environmental sample of step (b) with the polynucleotide probe of step (a); and (d) isolating a nucleic acid that specifically hybridizes with the polynucleotide probe of step (a), thereby isolating or recovering a nucleic acid encoding a polypeptide with a hydrolase activity from an environmental sample.

127. The method of paragraph 124 or paragraph 126, wherein the environmental sample comprises a water sample, a liquid sample, a soil sample, an air sample or a biological sample.

128. The method of paragraph 127, wherein the biological sample is derived from a bacterial cell, a protozoan cell, an insect cell, a yeast cell, a plant cell, a fungal cell or a mammalian cell.

129. A method of generating a variant of a nucleic acid encoding a polypeptide with a hydrolase activity comprising the steps of:

(a) providing a template nucleic acid comprising a sequence as set forth in paragraph 1 or paragraph 22; and
(b) modifying, deleting or adding one or more nucleotides in the template sequence, or a combination thereof, to generate a variant of the template nucleic acid.

130. The method of paragraph 129, further comprising expressing the variant nucleic acid to generate a variant hydrolase polypeptide.

131. The method of paragraph 129, wherein the modifications, additions or deletions are introduced by a method comprising error-prone PCR, shuffling, oligonucleotide-directed mutagenesis, assembly PCR, sexual PCR mutagenesis, *in vivo* mutagenesis, cassette mutagenesis, recursive ensemble mutagenesis, exponential ensemble mutagenesis, site-specific mutagenesis, gene reassembly, gene site saturated mutagenesis (GSSM), synthetic ligation reassembly (SLR) and a combination thereof.

132. The method of paragraph 129, wherein the modifications, additions or deletions are introduced by a method comprising recombination, recursive sequence recombination, phosphothioate-modified DNA mutagenesis, uracil-containing template mutagenesis, gapped duplex mutagenesis, point mismatch repair mutagenesis, repair- deficient host strain mutagenesis, chemical mutagenesis, radiogenic mutagenesis, deletion mutagenesis, restriction-selection mutagenesis, restriction-purification mutagenesis, artificial gene synthesis, ensemble mutagenesis, chimeric nucleic acid multimer creation and a combination thereof.

133. The method of paragraph 129, wherein the method is iteratively repeated until a hydrolase having an altered or different activity or an altered or different stability from that of a polypeptide encoded by the template nucleic acid is produced.

134. The method of paragraph 133, wherein the variant hydrolase polypeptide is thermotolerant, and retains some activity after being exposed to an elevated temperature.

135. The method of paragraph 133, wherein the variant hydrolase polypeptide has increased glycosylation as compared to the hydrolase encoded by a template nucleic acid.

136. The method of paragraph 133, wherein the variant hydrolase polypeptide has a hydrolase activity under a high temperature, wherein the hydrolase encoded by the template nucleic acid is not active under the high temperature.

137. The method of paragraph 129, wherein the method is iteratively repeated until a hydrolase coding sequence having an altered codon usage from that of the template nucleic acid is produced.

138. The method of paragraph 129, wherein the method is iteratively repeated until a hydrolase gene having higher or lower level of message expression or stability from that of the template nucleic acid is produced.

139. A method for modifying codons in a nucleic acid encoding a polypeptide with a hydrolase activity to increase its expression in a host cell, the method comprising the following steps:

(a) providing a nucleic acid encoding a polypeptide with a hydrolase activity comprising a sequence as set forth in paragraph 1 or paragraph 22; and, (b) identifying a non-preferred or a less preferred codon in the nucleic acid of step (a) and replacing it with a preferred or neutrally used codon encoding the same amino acid as the replaced codon, wherein a preferred codon is a codon over-represented in coding sequences in genes in the host cell and a non-preferred or less preferred codon is a codon under-represented in coding sequences in genes in the host cell, thereby modifying the nucleic acid to increase its expression in a host cell.

140. A method for modifying codons in a nucleic acid encoding a hydrolase polypeptide, the method comprising the following steps:

(a) providing a nucleic acid encoding a polypeptide with a hydrolase activity comprising a sequence as set forth in paragraph 1 or paragraph 22; and,
(b) identifying a codon in the nucleic acid of step (a) and replacing it with a different codon encoding the same amino acid as the replaced codon, thereby modifying codons in a nucleic acid encoding a hydrolase.

141. A method for modifying codons in a nucleic acid encoding a hydrolase polypeptide to increase its expression in a host cell, the method comprising the following steps:

(a) providing a nucleic acid encoding a hydrolase polypeptide comprising a sequence as set forth in paragraph 1 or paragraph 22; and,
(b) identifying a non-preferred or a less preferred codon in the nucleic acid of step (a) and replacing it with a preferred or neutrally used codon encoding the same amino acid as the replaced codon, wherein a preferred codon is a codon over-represented in coding sequences in genes in the host cell and a non-preferred or less preferred codon is a codon under-represented in coding sequences in genes in the host cell, thereby modifying the nucleic acid to increase its expression in a host cell.

142. A method for modifying a codon in a nucleic acid encoding a polypeptide having a hydrolase activity to decrease its expression in a host cell, the method comprising the following steps:

(a) providing a nucleic acid encoding a hydrolase polypeptide comprising a sequence as set forth in paragraph 1 or paragraph 22; and
(b) identifying at least one preferred codon in the nucleic acid of step (a) and replacing it with a non-preferred or less preferred codon encoding the same amino acid as the replaced codon, wherein a preferred codon is a codon over-represented in coding sequences in genes in a host cell and a non-preferred or less preferred codon is a codon under-represented in coding sequences in genes in the host cell, thereby modifying the nucleic acid to decrease its expression in a host cell.

143. The method of paragraph 141 or 142, wherein the host cell is a bacterial cell, a fungal cell, an insect cell, a yeast cell, a plant cell or a mammalian cell.

144. A method for producing a library of nucleic acids encoding a plurality of modified hydrolase active sites or substrate binding sites, wherein the modified active sites or substrate binding sites are derived from a first nucleic acid comprising a sequence encoding a first active site or a first substrate binding site the method comprising the following steps:

(a) providing a first nucleic acid encoding a first active site or first substrate binding site, wherein the first nucleic acid sequence comprises a sequence that hybridizes under stringent conditions to a sequence as set forth in paragraph 1 or paragraph 22, or a subsequence thereof, and the nucleic acid encodes a hydrolase active site or a hydrolase substrate binding site; (b) providing a set of mutagenic oligonucleotides that encode naturally-occurring amino acid variants at a plurality of targeted codons in the first nucleic acid; and, (c) using the set of mutagenic oligonucleotides to generate a set of active site- encoding or substrate binding site-encoding variant nucleic acids encoding a range of amino acid variations at each amino acid codon that was mutagenized, thereby producing a library of nucleic acids encoding a plurality of modified hydrolase active sites or substrate binding sites.

145. The method of paragraph 144, comprising mutagenizing the first nucleic acid of step (a) by a method comprising an optimized directed evolution system, gene site- saturation mutagenesis (GSSM), or a synthetic ligation reassembly (SLR).

146. The method of paragraph 144, comprising mutagenizing the first nucleic acid of step (a) or variants by a method comprising error-prone PCR, shuffling, oligonucleotide-directed mutagenesis, assembly PCR, sexual PCR mutagenesis, *in vivo* mutagenesis, cassette mutagenesis, recursive ensemble mutagenesis, exponential ensemble mutagenesis, site-specific mutagenesis, gene reassembly, gene site saturated mutagenesis (GSSM), synthetic ligation reassembly (SLR) and a combination thereof.

147. The method of paragraph 144, comprising mutagenizing the first nucleic acid of step (a) or variants by a method comprising recombination, recursive sequence recombination, phosphothioate-modified DNA mutagenesis, uracil-containing template mutagenesis, gapped duplex mutagenesis, point mismatch repair mutagenesis, repair-deficient host strain mutagenesis, chemical mutagenesis, radiogenic mutagenesis, deletion mutagenesis, restriction-selection mutagenesis, restriction-purification mutagenesis, artificial gene synthesis, ensemble mutagenesis, chimeric nucleic acid multimer creation and a combination thereof.

148. A method for making a small molecule comprising the following steps: (a) providing a plurality of biosynthetic enzymes capable of synthesizing or modifying a small molecule, wherein one of the enzymes comprises a hydrolase enzyme encoded by a nucleic acid comprising a sequence as set forth in paragraph 1 or paragraph 22; (b) providing a substrate for at least one of the enzymes of step (a); and (c) reacting the substrate of step (b) with the enzymes under conditions that facilitate a plurality of biocatalytic reactions to generate a small molecule by a series of biocatalytic reactions.

149. A method for modifying a small molecule comprising the following steps:

(a) providing a hydrolase enzyme, wherein the enzyme comprises a polypeptide as set forth in paragraph 56, or a polypeptide encoded by a nucleic acid comprising a nucleic acid sequence as set forth in paragraph 1 or paragraph 22; (b) providing a small molecule; and (c) reacting the enzyme of step (a) with the small molecule of step (b) under conditions that facilitate an enzymatic reaction catalyzed by the hydrolase enzyme, thereby modifying a small molecule by a hydrolase enzymatic reaction.

150. The method of paragraph 149, comprising a plurality of small molecule substrates for the enzyme of step (a), thereby generating a library of modified small molecules produced by at least one enzymatic reaction catalyzed by the hydrolase enzyme.

151. The method of paragraph 149, further comprising a plurality of additional enzymes under conditions that facilitate a plurality of biocatalytic reactions by the enzymes to form a library of modified small molecules produced by the plurality of enzymatic reactions.

152. The method of paragraph 151, further comprising the step of testing the library to determine if a particular modified small molecule which exhibits a desired activity is present within the library.

153. The method of paragraph 152, wherein the step of testing the library further comprises the steps of systematically eliminating all but one of the biocatalytic reactions used to produce a portion of the plurality of the modified small molecules within the library by testing the portion of the modified small molecule for the presence or absence of the particular modified small molecule with a desired activity, and identifying at least one specific biocatalytic reaction that produces the particular modified small molecule of desired activity.

154. A method for determining a functional fragment of a hydrolase enzyme comprising the steps of:

(a) providing a hydrolase enzyme, wherein the enzyme comprises a polypeptide as set forth in paragraph 56, or a polypeptide encoded by a nucleic acid as set forth in paragraph 1 or paragraph 22; and
(b) deleting a plurality of amino acid residues from the sequence of step (a) and testing the remaining subsequence for a hydrolase activity, thereby determining a functional fragment of a hydrolase enzyme.

155. The method of paragraph 154, wherein the hydrolase activity is measured by providing a hydrolase substrate and detecting a decrease in the amount of the substrate or an increase in the amount of a reaction product.

156. A method for whole cell engineering of new or modified phenotypes by using real-time metabolic flux analysis, the method comprising the following steps:

(a) making a modified cell by modifying the genetic composition of a cell, wherein the genetic composition is modified by addition to the cell of a nucleic acid comprising a sequence as set forth in paragraph 1 or paragraph 22;
(b) culturing the modified cell to generate a plurality of modified cells;
(c) measuring at least one metabolic parameter of the cell by monitoring the cell culture of step (b) in real time; and,
(d) analyzing the data of step (c) to determine if the measured parameter differs from a comparable measurement in an unmodified cell under similar conditions, thereby identifying an engineered phenotype in the cell using real-time metabolic flux analysis.

157. The method of paragraph 156, wherein the genetic composition of the cell is modified by a method comprising deletion of a sequence or modification of a sequence in the cell, or, knocking out the expression of a gene.

158. The method of paragraph 157, further comprising selecting a cell comprising a newly engineered phenotype.

159. The method of paragraph 158, further comprising culturing the selected cell, thereby generating a new cell strain comprising a newly engineered phenotype.

160. A method for hydrolyzing a triacyl glycerol (TAG), a diacylglycerol (DAG) or a monoacylglycerol (MAG) comprising the following steps:

(a) providing a polypeptide having a hydrolase activity, wherein the polypeptide comprises a polypeptide as set forth in paragraph 56, or a polypeptide encoded by a nucleic acid as set forth in paragraph 1 or paragraph 22;
(b) providing a composition comprising a triacylglycerol (TAG), a diacylglycerol (DAG) or a monoacylglycerol (MAG); and
(c) contacting the polypeptide of step (a) with the composition of step (b) under conditions wherein the polypeptide hydrolyzes the triacylglycerol (TAG), diacylglycerol (DAG) or monoacylglycerol (MAG).

161. A method for removing or decreasing the amount of a triacylglycerol (TAG), a diacylglycerol (DAG) or a monoacylglycerol (MAG) from a composition comprising the following steps:

(a) providing a polypeptide having a hydrolase activity, wherein the polypeptide comprises a polypeptide as set forth in paragraph 56, or a polypeptide encoded by a nucleic acid as set forth in paragraph 1 or paragraph 22;
(b) providing a composition comprising a triacylglycerol (TAG), a diacylglycerol (DAG) or a monoacylglycerol (MAG); and
(c) contacting the polypeptide of step (a) with the composition of step (b) under conditions wherein the polypeptide removes or decreases the amount of the triacylglycerol (TAG), diacylglycerol (DAG) or monoacylglycerol (MAG).

162. A method of increasing thermotolerance or thermostability of a hydrolase polypeptide, the method comprising glycosylating a hydrolase polypeptide, wherein the polypeptide comprises at least thirty contiguous amino acids of a polypeptide as set forth in paragraph 52, or a polypeptide encoded by a nucleic acid as set forth in paragraph 1 or paragraph 22, thereby increasing the thermotolerance or thermostability of the hydrolase polypeptide.

163. The method of paragraph 163, wherein the hydrolase specific activity is thermostable or thermotolerant at a temperature in the range from greater than about 37°C to about 95°C.

164. A method for overexpressing a recombinant hydrolase polypeptide in a cell comprising expressing a vector comprising a nucleic acid sequence as set forth in paragraph 1 or paragraph 22, wherein overexpression is effected by use of a high activity promoter, a dicistronic vector or by gene amplification of the vector.

165. A detergent composition comprising a polypeptide as set forth in paragraph 52, or a polypeptide encoded by a nucleic acid as set forth in paragraph 1 or paragraph 22, wherein the polypeptide comprises a hydrolase activity.

166. The detergent composition of paragraph 166, wherein the hydrolase is a nonsurface-active hydrolase or a surface-active hydrolase.

167. The detergent composition of paragraph 165, wherein the hydrolase is formulated in a non-aqueous liquid composition, a cast solid, a granular form, a particulate form, a compressed tablet, a gel form, a paste or a slurry form.

168. A method for washing an object comprising the following steps:

(a) providing a composition comprising a polypeptide having a hydrolase activity, wherein the polypeptide comprises a polypeptide as set forth in paragraph 56, or a polypeptide encoded by a nucleic acid as set forth in paragraph 1 or paragraph 22;
(b) providing an object; and
(c) contacting the polypeptide of step (a) and the object of step (b) under conditions wherein the composition can wash the object.

169. A method for hydrolyzing an oil in a feed or a food prior to consumption by an animal comprising the following steps:

(a) obtaining a feed material comprising an oil, wherein the oil can be hydrolyzed by a polypeptide having a hydrolase activity, wherein the polypeptide comprises a polypeptide as set forth in paragraph 56, or a polypeptide encoded by a nucleic acid as set forth in paragraph 1 or paragraph 22; and
(b) adding the polypeptide of step (a) to the feed or food material in an amount sufficient for a sufficient time period to cause hydrolysis of the oil and formation of a treated food or feed, thereby hydrolyzing the oil in the food or the feed prior to consumption by the animal.

170. The method as set forth in paragraph 170, wherein the food or feed comprises rice, corn, barley, wheat, legumes, or potato.

171. A feed or a food comprising a polypeptide as set forth in paragraph 52, or a polypeptide encoded by a nucleic acid as set forth in paragraph 1 or paragraph 22.

172. A composition comprising an oil and a polypeptide as set forth in paragraph 56, or a polypeptide encoded by a nucleic acid as set forth in paragraph 1 or paragraph 22.

173. A pharmaceutical composition comprising a polypeptide as set forth in paragraph 56, or a polypeptide encoded by a nucleic acid as set forth in paragraph 1 or paragraph 22.

174. The expression cassette of paragraph 32, wherein the nucleic acid is operably linked to a plant promoter.

175. The expression cassette of paragraph 174, further comprising a plant expression vector.

176. The expression cassette of paragraph 175, wherein the plant expression vector comprises a plant virus.

177. The expression cassette of paragraph 174, wherein the plant promoter comprises a potato promoter, a rice promoter, a corn promoter, a wheat or a barley promoter.

178. The expression cassette of paragraph 174, wherein the promoter comprises a promoter derived from T-DNA of *Agrobacterium tumefaciens.*

179. The expression cassette of paragraph 174, wherein the promoter is a constitutive promoter.

180. The expression cassette of paragraph 179, wherein the constitutive promoter is CaMV35S.

181. The expression cassette of paragraph 174, wherein the promoter is an inducible promoter or a tissue-specific promoter.

182. The expression cassette of paragraph 181, wherein the tissue-specific promoter is a seed-specific, a leaf-specific, a root-specific, a stem-specific or an abscission-induced promoter.

183. The transformed cell of paragraph 39, wherein the plant cell is a potato, rice, corn, wheat, tobacco or barley cell.

184. A method of making a transgenic plant comprising the following steps:

(a) introducing a heterologous nucleic acid sequence into the cell, wherein the heterologous nucleic sequence comprises a sequence as set forth in paragraph 1 or paragraph 22, thereby producing a transformed plant cell;
(b) producing a transgenic plant from the transformed cell.

185. The method as set forth in paragraph 184, wherein the step (a) further comprises introducing the heterologous nucleic acid sequence by electroporation or microinjection of plant cell protoplasts.

186. The method as set forth in paragraph 184, wherein the step (a) comprises introducing the heterologous nucleic acid sequence directly to plant tissue by DNA particle bombardment.

187. The method as set forth in paragraph 184, wherein the step (a) comprises introducing the heterologous nucleic acid sequence into the plant cell DNA using an *Agrobacterium tumefaciens* host.

188. A method of expressing a heterologous nucleic acid sequence in a plant cell comprising the following steps:

(a) transforming the plant cell with a heterologous nucleic acid sequence operably linked to a promoter, wherein the heterologous nucleic sequence comprises a sequence as set forth in paragraph 1 or paragraph 22; (b) growing the plant under conditions wherein the heterologous nucleic acids sequence is expressed in the plant cell.

189. A signal sequence comprising a peptide having a subsequence of a polypeptide as set forth in paragraph 52.

190. A signal sequence consisting of a peptide having a subsequence of a polypeptide as set forth in paragraph 52.

191. A chimeric protein comprising a first domain comprising a signal sequence as set forth in paragraph 189 or paragraph 190 and at least a second domain.

192. The chimeric protein of paragraph 191, wherein the protein is a fusion protein.

193. The chimeric protein of paragraph 191, wherein the second domain comprises an enzyme.

194. The chimeric protein of paragraph 193, wherein the enzyme is a hydrolase.

195. A method for biocatalytic synthesis of a structured lipid comprising the following steps:

(a) providing a hydrolase as set forth in paragraph 56;
(b) providing a composition comprising a triacylglyceride (TAG);
(c) contacting the polypeptide of step (a) with the composition of step (b) under conditions wherein the polypeptide hydrolyzes an acyl residue at the Sn2 position of the triacylglyceride (TAG), thereby producing a 1,3-diacylglyceride (DAG);
(d) providing an R1 ester;
(e) providing an R1 -specific hydrolase, and
(f) contacting the 1,3-DAG of step (c) with the R1 ester of step (d) and the R1- specific hydrolase of step (e) under conditions wherein the R1 -specific hydrolase catalyzes esterification of the Sn2 position, thereby producing the structured lipid.

196. The method of paragraph 195, wherein the hydrolase is an Sn2-specific lipase.

197. The method of paragraph 195, wherein the structured lipid comprises a cocoa butter alternative (CBA), a synthetic cocoa butter, a natural cocoa butter, 1,3-dipalmitoyl-2-oleoyl glycerol (POP), 1,3-distearoyl-2-oleoylglycerol (SOS), 1-palmitoyl-2-oleoyl-3-stearoylglycerol (POS) or 1-oleoyl-2,3-dimyristoylglycerol (OMM).

198. A method for biocatalytic synthesis of a structured lipid comprising the following steps:

(a) providing a hydrolase as set forth in paragraph 56;
(b) providing a composition comprising a triacylglyceride (TAG);
(c) contacting the polypeptide of step (a) with the composition of step (b) under conditions wherein the polypeptide hydrolyzes an acyi residue at the Sn1 or Sn3 position of the triacylglyceride (TAG), thereby producing a 1,2-DAG or 2,3-DAG; and
(d) promoting of acyl migration in the 1,2-DAG or 2,3-DAG of the step (c) under kinetically controlled conditions, thereby producing a 1,3-DAG.

199. The method of paragraph 198, further comprising providing an R1 ester and an R1-specific lipase, and contacting the 1,3-DAG of step (d) with the R1 ester and the R1-specific lipase under conditions wherein the R1-specific lipase catalyzes esterification of the Sn2 position, thereby producing a structured lipid.

200. The method of paragraph 198, wherein the lipase is an Sn1 or an Sn3-specific lipase.

201. The method of paragraph 198, wherein the structured lipid comprises a cocoa butter alternative (CBA), a synthetic cocoa butter, a natural cocoa butter, 1,3-dipalmitoyl- 2-oleoylglycerol (POP), I,3-distearoyl-2-oleoylglycerol (SOS), I-paimitoyl-2-oleoyl-3-stearoylglycerol (POS) or I-oleoyl-2,3-dimyristoylglycerol (OMM).

202. The method of paragraph 198, wherein step (d) further comprises using ion exchange resins.

203. The method of paragraph 198, wherein the kinetically controlled conditions comprise non-equilibrium conditions resulting in production of an end product having greater than a 2:1 ratio of 1, 3-DAG to 2,3-DAG.

204. The method of paragraph 195 or paragraph 198, wherein the composition of step (b) comprises a fluorogenic fatty acid (FA).

205. The method of paragraph 195 or paragraph 198, wherein the composition of step (b) comprises an umbelliferyl FA ester.

206. The method of paragraph 195 or paragraph 198, wherein the end product is enantiomerically pure.

207. A method for preparation of an optical isomer of a propionic acid from a racemic ester of the propionic acid comprising the following steps:

(a) providing a hydrolase as set forth in paragraph 56, wherein the hydrolase is stereoselective for an optical isomer of the propionic acid;
(b) providing racemic esters;
(c) contacting the polypeptide of step (a) with the racemic esters of step (b) wherein the polypeptide can selectively catalyze the hydrolysis of the esters of step (b), thereby producing the optical isomer of the propionic acid.

208. The method of paragraph 207, wherein the optical isomer of the propionic acid comprises S(+) of 2-(6-methoxy-2-naphthyl) propionic acid and the racemic esters comprises racemic (R,S) esters of 2-(6-methoxy-2-naphthyl) propionic acid.

209. A method for stereoselectively hydrolyzing racemic mixtures of esters of 2-substituted acids comprising the following steps:

(a) providing a hydrolase as set forth in paragraph 56, wherein the hydrolase is stereoselective;
(b) providing a composition comprising a racemic mixture of esters of 2-substituted acids; and
(c) contacting the polypeptide of step (a) with the composition of step (b) under conditions wherein the polypeptide of step (b) can selectively hydrolyze the esters.

210. The method of paragraph 195, paragraph 198, or paragraph 209 wherein the hydrolase is immobilized.

211. The method of paragraph 209, wherein the 2-substituted acid comprises a 2- aryloxy substituted acid, an R-2-(4-hydroxyphenoxy)propionic acid or a 2-arylpropionic acid.

212. The method of paragraph 209, wherein the 2-substituted acid comprises a ketoprofen.

213. A method for oil or fat modification comprising the following steps: (a) providing a hydrolase as set forth in paragraph 56;
(h) providing an oil or fat, and
(c) contacting the hydrolase of step (a) with the oil or fat of step (b) under conditions wherein the hydrolase can modify the oil or fat.

214. The method of paragraph 213, wherein the modification comprises a hydrolase-catalyzed hydrolysis of the fat or oil.

215. The method of paragraph 214, wherein the hydrolysis is a complete or a partial hydrolysis of the fat or oil.

216. The method of paragraph 214, wherein the oil comprises a glycerol ester of a polyunsaturated fatty acid, or a fish, animal, or vegetable oil.

217. The method of paragraph 216, wherein the vegetable oil comprises an olive, canola, sunflower, palm, soy or lauric oil or rice bran oil.

218. A method for hydrolysis of polyunsaturated fatty acid (PUFA) esters comprising the following steps:

(a) providing a hydrolase as set forth in paragraph 56;
(b) providing composition comprising a polyunsaturated fatty acid ester, and
(c) contacting the hydrolase with the composition of step (b) under conditions wherein the hydrolase can hydrolyze the polyunsaturated fatty acid (PUFA) ester.

219. A method of selective hydrolysis of polyunsaturated fatty acids esters over saturated fatty acid esters comprising the following steps:

(a) providing a hydrolase as set forth in paragraph 56, wherein the hydrolase has a lipase activity and selectively hydrolyzes polyunsaturated fatty acid (PUFA) esters;
(b) providing a composition comprising a mixture of polyunsaturated and saturated esters; and
(c) contacting the polypeptide of step (a) with the composition of step (b) under conditions wherein the polypeptide can selectively catalyze the hydrolysis of polyunsaturated fatty acids esters.

220. A method for preparing a food or a feed additive comprising polyunsaturated fatty acids (PUFA) comprising the following steps:

(a) providing a hydrolase as set forth in paragraph 56, wherein the hydrolase selectively hydrolyzes polyunsaturated fatty acid (PUFA) esters;
(b) providing a composition comprising a PUFA ester; and
(c) contacting the polypeptide of step (a) with the composition of step (b) under conditions wherein the polypeptide can selectively catalyze the hydrolysis of polyunsaturated fatty acid esters thereby producing the PUFA-containing food or feed additive.

221. A method for treatment of latex comprising the following steps:

(a) providing a hydrolase as set forth in paragraph 56, wherein the polypeptide has selectivity for a saturated ester over an unsaturated ester, thereby converting the saturated ester to its corresponding acid and alcohol;
(b) providing a latex composition comprising saturated and unsaturated esters;
(c) contacting the hydrolase of step (a) with the composition of step (b) under conditions wherein the polypeptide can selectively hydrolyze saturated esters, thereby treating the latex.

222. The method of paragraph 221 , wherein ethyl propionate is selectively hydrolyzed over ethyl acrylate.

223. The method of paragraph 221 , wherein the latex composition of step (b) comprises polymers containing acrylic, vinyl and unsaturated acid monomers, alkyl acrylate monomers, methyl acrylate, ethyl acrylate, propyl acrylate and butyl acrylate, acrylate acids, acrylic acid, methacrylic acid, crotonic acid, itaconic acid and mixtures thereof.

224. The method of paragraph 221, wherein the latex composition is a hair fixative.

225. The method of paragraph 221 , wherein the conditions of step (c) comprise a pH in the range from about pH 4 to pH 8 and a temperature in the range from about 20° to about 50°C.

226. A method for refining a lubricant comprising the following steps: (a) providing a composition comprising a hydrolase as set forth in paragraph 56; (b) providing a lubricant; and (c) treating the lubricant with the hydrolase under conditions wherein the hydrolase can selective hydrolyze oils in the lubricant, thereby refining it.

227. The method of paragraph 226, wherein the lubricant is a hydraulic oil.

228. A method of treating a fabric comprising the following steps: (a) providing a composition comprising a hydrolase as set forth in paragraph 56, wherein the hydrolase can selectively hydrolyze carboxylic esters; (b) providing a fabric; and (c) treating the fabric with the hydrolase under condition wherein the hydrolase can selectively hydrolyze carboxylic esters thereby treating the fabric..

229. The method of paragraph 228, wherein treatment of the fabric comprises improvement of the hand and drape of the final fabric, dyeing, obtaining flame retardancy, obtaining water repellency, obtaining optical brightness, or obtaining resin finishing.

230. The method of paragraph 228, wherein the fabric comprises cotton, viscose, rayon, lyocell, flax, linen, ramie, all blends thereof, or blends thereof with polyesters, wool, polyamides acrylics or polyacrylics.

231. A fabric, yarn or fiber comprising a hydrolase as set forth in paragraph 56.

232. The fabric, yarn or fiber of paragraph 231 , wherein the hydrolase is adsorbed, absorbed or immobilized on the surface of the fabric, yarn or fiber.

233. A method for removing or decreasing the amount of a food or oil stain comprising contacting a hydrolase as set forth in paragraph 56 with the food or oil stain under conditions wherein the hydrolase can hydrolyze oil or fat in the stain.

234. The method of paragraph 233, wherein the hydrolase has an enhanced stability to denaturation by surfactants and to heat deactivation.

235. The method of paragraph 233, wherein the hydrolase is a detergent or a laundry solution.

236. A dietary composition comprising a hydrolase as set forth in paragraph 56.

237. The dietary composition of paragraph 236, further comprising a nutritional base comprising a fat.

238. The dietary composition of paragraph 236, wherein the hydrolase is activated by a bile salt.

239. The dietary composition of paragraph 236, further comprising a cow's milk-based infant formula.

240. The dietary composition of paragraph 239, wherein the hydrolase can hydrolyze long chain fatty acids.

241. A method of reducing fat content in milk or vegetable-based dietary compositions comprising the following steps: (a) providing a composition comprising a hydrolase as set forth in paragraph 56; (b) providing a composition comprising a milk or a vegetable oil, and (c) treating the composition of step (b) with the hydrolase under conditions wherein the hydrolase can hydrolyze the oil or fat in the composition, thereby reducing its fat content.

242. A dietary composition for a human or non-ruminant animals comprising a nutritional base, wherein the base comprises a fat and no or little hydrolase, and an effective amount of a hydrolase as set forth in paragraph 56 to increase fat absorption and growth of human or non-ruminant animal.

243. A method of catalyzing an interesterification reaction to produce new triglycerides comprising the following steps: (a) providing a composition comprising a hydrolase as set forth in paragraph 56, wherein the hydrolase can catalyze an interesterification reaction; (b) providing a mixture of triglycerides and free fatty acids; (c) treating the composition of step (b) with the hydrolase under conditions wherein the hydrolase can catalyze exchange of free fatty acids with the acyl groups of triglycerides, thereby producing new triglycerides enriched in the added fatty acids.

244. The method of paragraph 243, wherein the hydrolase is an Sn1,3-specific lipase.

245. A transesterification method for preparing a margarine oil having a low trans-acid and a low intermediate chain fatty acid content, comprising the following steps:

(a) providing a transesterification reaction mixture comprising a stearic acid source material selected from the group consisting of stearic acid, stearic acid monoesters of low molecular weight monohydric alcohols and mixtures thereof,
(b) providing a liquid vegetable oil;
(c) providing a hydrolase as set forth in paragraph 56, wherein the polypeptide comprises a 1,3-specific lipase activity;
(d) transesterifying the stearic acid source material and the vegetable oil triglyceride, to substantially equilibrate the ester groups in the 1-, 3- positions of the glyceride component with non-glyceride fatty acid components of the reaction mixture,
(e) separating transesterified free fatty acid components from glyceride components of the transesterification mixture to provide a transesterified margarine oil product and a fatty acid mixture comprising fatty acids, fatty acid monoesters or mixtures thereof released from the vegetable oil, and
(f) hydrogenating the fatty acid mixture.

246. A method for making a composition comprising 1-palmitoyl-3-stearoyl-2-monoleine (POSt) and 1,3-distearoyl-2-monoleine (StOSt) comprising providing a lipase as set forth in paragraph 56, wherein the lipase is capable of 1,3 -specific lipase-catalyzed interesterification of 1,3-dipalmitoyl-2-monoleine (POP) with stearic acid or tristearin, to make a product enriched in the 1-palmitoyl-3-stearoyl-2-monoleine (POSt) or 1,3- distearoyl-2-monoleine (StOSt).

247. A method for ameliorating or preventing lipopolysaccharide (LPS)- mediated toxicity comprising administering to a patient a pharmaceutical composition comprising a polypeptide as set forth in paragraph 56, or a polypeptide encoded by a nucleic acid as set forth in paragraph 1 or paragraph 22.

248. A method for detoxifying an endotoxin comprising contacting the endotoxin with a polypeptide as set forth in paragraph 56, or a polypeptide encoded by a nucleic acid as set forth in paragraph 1 or paragraph 22.

249. A method for deacylating a 2' or a 3' fatty acid chain from a lipid A comprising contacting the lipid A with a polypeptide as set forth in paragraph 56, or a polypeptide encoded by a nucleic acid as set forth in paragraph 1 or paragraph 22.

## Claims

**1.** An isolated, synthetic or recombinant nucleic acid comprising:

(a) a nucleic acid sequence having at least 80%, 81%, 82%, 83%, 84%, 85%, 86%, 87%, 88%, 89%, 90%, 91%, 92%, 93%, 94%, 95%, 96%, 97%, 98%, 99% or has 100% (complete) sequence identity to SEQ ID NO: 157, SEQ ID NO:1, SEQ ID NO:3, SEQ ID NO:5, SEQ ID NO:7, SEQ ID NO:9, SEQ ID NO:11, SEQ ID NO: 13, SEQ ID NO:15, SEQ ID NO:17, SEQ ID NO:19, SEQ ID NO:21, SEQ ID NO:23, SEQ ID NO:25, SEQ ID NO:27, SEQ ID NO:29, SEQ ID NO:31, SEQ ID NO:33, SEQ ID NO:35, SEQ ID NO:37, SEQ ID NO:39, SEQ ID NO:41, SEQ ID NO:43, SEQ ID NO:45, SEQ ID NO:47, SEQ ID NO:49, SEQ ID NO:51, SEQ ID NO: 53, SEQ ID NO:55, SEQ ID NO:57, SEQ ID NO:59, SEQ ID NO:61, SEQ ID NO:63, SEQ ID NO:65, SEQ ID NO: 67, SEQ ID NO:69, SEQ ID NO:71, SEQ ID NO:73, SEQ ID NO:75, SEQ ID NO:77, SEQ ID NO:79, SEQ ID NO:81, SEQ ID NO:83, SEQ ID NO:85, SEQ ID NO:87, SEQ ID NO:89, SEQ ID NO:91, SEQ ID NO:93, SEQ ID NO:95, SEQ ID NO:97, SEQ ID NO:99, SEQ ID NO:101, SEQ ID NO:103, SEQ ID NO:105, SEQ ID NO: 107, SEQ ID NO:109, SEQ ID NO. 111, SEQ ID NO:113, SEQ ID NO:115, SEQ ID NO:117, SEQ ID NO:119, SEQ ID NO:121, SEQ ID NO:123, SEQ ID NO:125, SEQ ID NO:127, SEQ ID NO:129, SEQ ID NO:131, SEQ ID NO:133, SEQ ID NO:135, SEQ ID NO:137, SEQ ID NO:139, SEQ ID NO:141, SEQ ID NO:143, SEQ ID NO: 145, SEQ ID NO:147, SEQ ID NO:149, SEQ ID NO:151, SEQ ID NO:153, SEQ ID NO:155, SEQ ID NO:159, SEQ ID NO:161, SEQ ID NO:163 and/or SEQ ID NO:165,
wherein the nucleic acid encodes at least one polypeptide having an esterase activity, or an enzymatically active fragment thereof, or the nucleic acid encodes a polypeptide or a peptide that can generate an antibody that specifically binds to SEQ ID NO:158, SEQ ID NO:2, SEQ ID NO:4, SEQ ID NO:6, SEQ ID NO:8, SEQ ID NO: 10, SEQ ID NO: 12, SEQ ID NO: 14, SEQ ID NO: 16, SEQ ID NO: 18, SEQ ID NO:20, SEQ ID NO:22, SEQ ID NO:24, SEQ ID NO:26, SEQ ID NO:28, SEQ ID NO:305 SEQ ID NO:32, SEQ ID NO:34, SEQ ID NO:36, SEQ ID NO:38, SEQ ID NO:40, SEQ ID NO:42, SEQ ID NO:44, SEQ ID NO:46, SEQ ID NO:48, SEQ ID NO:50, SEQ ID NO:52, SEQ ID NO:54, SEQ ID NO:56, SEQ ID NO:58, SEQ ID NO:60, SEQ ID NO:62, SEQ ID NO: 64, SEQ ID NO:66, SEQ ID NO:68, SEQ ID NO:70, SEQ ID NO:72, SEQ ID NO:74, SEQ ID NO:76, SEQ ID NO:78, SEQ ID NO: 80, SEQ ID NO: 82, SEQ ID NO:84, SEQ ID NO:86, SEQ ID NO:88, SEQ ID NO:90, SEQ ID NO:92, SEQ ID NO:94, SEQ ID NO:96, SEQ ID NO:98, SEQ ID NO:100, SEQ ID NO:102, SEQ ID NO:104, SEQ ID NO:106, SEQ ID NO:108, SEQ ID NO:110, SEQ ID NO:112, SEQ ID NO:114, SEQ ID NO:116, SEQ ID NO:118, SEQ ID NO:120, SEQ ID NO:122, SEQ ID NO:124, SEQ ID NO:126, SEQ ID NO:128, SEQ ID NO: 130, SEQ ID NO:132, SEQ ID NO:134, SEQ ID NO:136, SEQ ID NO:138, SEQ ID NO:140, SEQ ID NO:142, SEQ ID NO:143, SEQ ID NO:146, SEQ ID NO:148, SEQ ID NO: 150, SEQ ID NO:152, SEQ ID NO:154, SEQ ID NO: 156, SEQ ID NO:160, SEQ ID NO:162, SEQ ID NO:164 and/or SEQ ID NO:166, and optionally the sequence identities are determined by analysis with a sequence comparison algorithm or by a visual inspection;
(b) a nucleic acid sequence encoding SEQ ID NO:158, SEQ ID NO:2, SEQ ID NO:4, SEQ ID NO:6, SEQ ID NO:8, SEQ ID NO:10, SEQ ID NO:12, SEQ ID NO:14, SEQ ID NO:16, SEQ ID NO: 18, SEQ ID NO:20, SEQ ID NO:22, SEQ ID NO:24, SEQ ID NO:26, SEQ ID NO:28, SEQ ID NO:30, SEQ ID NO:32, SEQ ID NO:34, SEQ ID NO:36, SEQ ID NO:38, SEQ ID NO:40, SEQ ID NO:42, SEQ ID NO:44, SEQ ID NO:46, SEQ ID NO: 48, SEQ ID NO:50, SEQ ID NO:52, SEQ ID NO:54, SEQ ID NO:56, SEQ ID NO:58, SEQ ID NO:60, SEQ ID NO:62, SEQ ID NO:64, SEQ ID NO:66, SEQ ID NO:68, SEQ ID NO:70, SEQ ID NO:72, SEQ ID NO:74, SEQ ID NO:76, SEQ ID NO:78, SEQ ID NO:80, SEQ ID NO:82, SEQ ID NO:84, SEQ ID NO:86, SEQ ID NO:88, SEQ ID NO:90, SEQ ID NO:92, SEQ ID NO:94, SEQ ID NO:96, SEQ ID NO:98, SEQ ID NO:100, SEQ ID NO: 102, SEQ ID NO:104, SEQ ID NO:106, SEQ ID NO:108, SEQ ID NO:110, SEQ ID NO:112, SEQ ID NO:114, SEQ ID NO:116, SEQ ID NO:118, SEQ ID NO: 120, SEQ ID NO: 122, SEQ ID NO: 124, SEQ ID NO: 126, SEQ ID NO:128, SEQ ID NO:130, SEQ ID NO:132, SEQ ID NO:134, SEQ ID NO:136, SEQ ID NO:138, SEQ ID NO: 140, SEQ ID NO:142, SEQ ID NO:143, SEQ ID NO:146, SEQ ID NO:148, SEQ ID NO:150 SEQ ID NO:152, SEQ ID NO:154, SEQ ID NO: 156, SEQ ID NO: 160, SEQ ID NO: 162, SEQ ID NO: 164 and/or SEQ ID NO: 166, or enzymatically active fragments thereof;

(c) a nucleic acid sequence that hybridizes under stringent hybridization conditions to the complement of SEQ ID NO:157, SEQ ID NO:1, SEQ ID NO:3, SEQ ID NO:5, SEQ ID NO:7, SEQ ID NO:9, SEQ ID N0:11, SEQ ID NO:13, SEQ ID NO:15, SEQ ID NO:17, SEQ ID NO:19, SEQ ID NO:21, SEQ ID NO:23, SEQ ID NO:25, SEQ ID NO:27, SEQ ID NO:29, SEQ ID NO:31, SEQ ID NO:33, SEQ ID NO:35, SEQ ID NO:37, SEQ ID NO:39, SEQ ID NO:41 , SEQ ID NO:43, SEQ ID NO:45, SEQ ID NO:47, SEQ ID NO:49, SEQ ID NO:51, SEQ ID NO: 53, SEQ ID NO:55, SEQ ID NO:57, SEQ ID NO:59, SEQ ID NO:61, SEQ ID NO:63, SEQ ID NO:65, SEQ ID NO:67, SEQ ID NO:69, SEQ ID NO:71, SEQ ID NO:73, SEQ ID NO:75, SEQ ID NO:77, SEQ ID NO:79, SEQ ID NO:81, SEQ ID NO:83, SEQ ID NO:85, SEQ ID NO:87, SEQ ID NO:89, SEQ ID NO:91, SEQ ID NO:93, SEQ ID NO:955 SEQ ID NO:97, SEQ ID NO:99, SEQ ID NO:101, SEQ ID NO:103, SEQ ID NO:105, SEQ ID NO:107, SEQ ID NO:109, SEQ ID NO:111, SEQ ID NO:113, SEQ ID NO:115, SEQ ID NO:117, SEQ ID NO: 119, SEQ ID NO:121, SEQ ID NO:123, SEQ ID NO:125, SEQ ID NO:127, SEQ ID NO:129, SEQ ID NO:131, SEQ ID NO:133, SEQ ID NO:135, SEQ ID NO:137, SEQ ID NO:139, SEQ ID NO:141, SEQ ID NO:143, SEQ ID NO: 145, SEQ ID NO:147, SEQ ID NO:149, SEQ ID NO:151, SEQ ID NO:153, SEQ ID NO:155, SEQ ID NO:159, SEQ ID NO: 161, SEQ ID NO: 163 and/or SEQ ID NO: 165, wherein the stringent hybridization conditions comprise a wash step comprising a wash in 0.2X SSC at a temperature of about 65°C for about 15 minutes, wherein the nucleic acid encodes a polypeptide having an esterase activity, or an enzymatically active fragment thereof, or a polypeptide which can generate a humoral immune response to make an anti-esterase antibody
(d) a nucleic acid generated by amplification of a polynucleotide using an amplification primer pair, wherein the primer pair is capable of amplifying the nucleic acid sequence of SEQ ID NO:157, SEQ ID NO:1, SEQ ID NO: 3, SEQ ID NO:5, SEQ ID NO:7, SEQ ID NO:9, SEQ ID NO:11, SEQ ID NO: 13, SEQ ID NO: 15, SEQ ID NO: 17, SEQ ID NO: 19, SEQ ID NO:21, SEQ ID NO:23, SEQ ID NO:25, SEQ ID NO:27, SEQ ID NO:29, SEQ ID NO:31, SEQ ID NO:33, SEQ ID NO:35, SEQ ID NO:37, SEQ ID NO:39, SEQ ID NO:41, SEQ ID NO:43, SEQ ID NO:45, SEQ ID NO:47, SEQ ID NO:49, SEQ ID NO:51, SEQ ID NO.53, SEQ ID NO:55, SEQ ID NO:57, SEQ ID NO:59, SEQ ID NO:61, SEQ ID NO:63, SEQ ID NO:65, SEQ ID NO:67, SEQ ID NO:69, SEQ ID NO: 71, SEQ ID NO:73, SEQ ID NO:75, SEQ ID NO.77, SEQ ID NO:79, SEQ ID NO:81, SEQ ID NO:83, SEQ ID NO:85, SEQ ID NO:87, SEQ ID NO:89, SEQ ID NO:91, SEQ ID NO:93, SEQ ID NO:95, SEQ ID NO.97, SEQ ID NO:99, SEQ ID NO:101, SEQ ID NO:103, SEQ ID NO:105, SEQ ID NO:107, SEQ ID NO:109, SEQ ID NO: 111, SEQ ID NO: 113, SEQ ID NO: 115, SEQ ID NO:117, SEQ ID NO:119, SEQ ID NO: 121, SEQ ID NO:123, SEQ ID NO: I25, SEQ ID NO: I27, SEQ ID NO: 129, SEQ ID NO: 131, SEQ ID NO: 133, SEQ ID NO: 135, SEQ ID NO: 137, SEQ ID NO:139, SEQ ID NO:141, SEQ ID NO: I43, SEQ ID NO:145, SEQ ID NO:147, SEQ ID NO:149, SEQ ID NO:151, SEQ ID NO:153, SEQ ID NO:155, SEQ ID NO: 159, SEQ ID NO:161, SEQ ID NO: 163 and/or SEQ ID NO: 165, wherein a first member of the amplification primer pair comprises at least the first (the 5') 12 bases of the nucleic acid sequence of (a), (b) or (c), and a second member of the amplification primer comprises at least the first (the 5') 12 bases of the complementary strand of the nucleic acid sequence of (a), (b) or (c), wherein the nucleic acid encodes a polypeptide having an esterase activity, or an enzymatically active fragment thereof;
(e) the nucleic acid sequence of (a), (b), (c) or (d), wherein the sequence does not encode a polypeptide containing a signal sequence (signal peptide);
(f) the nucleic acid sequence of (a), (b), (c), (d) or (e), further comprising a heterologous sequence;
(g) the nucleic acid sequence of (f), wherein the heterologous sequence comprises a signal sequence (signal peptide), and optionally the signal sequence (signal peptide) is derived from another esterase or a non-esterase (a heterologous) enzyme;
(h) the nucleic acid sequence of (f), wherein the heterologous sequence comprises a prepro sequence and/ or catalytic domain (CD);
(i) the nucleic acid sequence of (f), wherein the heterologous sequence comprises an N-terminal identification peptide, and optionally the N-terminal identification peptide imparts a desired characteristic, and optionally the desired characteristic is increased stability or simplified purification of the polypeptide;
(j) a nucleic acid sequence completely complementary to (a), (b), (c), (d), (e), (f), (g), (h) or (i); or
(k) the nucleic acid of (a), (b), (c), (d), (e), (f), (g), (h) or (i), wherein the esterase activity comprises a lipase activity or a phospholipase activity.

2. A nucleic acid probe for identifying a nucleic acid encoding a polypeptide with an esterase activity, wherein the probe comprises at least 10 consecutive bases of a sequence of claim 1, wherein the probe identifies the nucleic acid by binding or hybridization
wherein optionally the probe comprises an oligonucleotide comprising at least about 10 to 50, about 20 to 60, about 30 to 70, about 40 to 80, about 60 to 100, or about 50 to 150 consecutive bases.

3. An expression cassette, a vector or a cloning vehicle comprising a nucleic acid of claim 1 wherein optionally the cloning vehicle comprises a viral vector, a plasmid, a phage, a phagemid, a cosmid, a fosmid, a bacteriophage, or a bacterial artificial chromosome, wherein optionally the viral vector comprises an adenovirus vector, a retroviral vector, or an adeno-associated viral vector, or wherein optionally the cloning vehicle comprises a bacterial artificial chromosome (BAC), a plasmid, a bacteriophage P 1 -derived vector (PAC), a yeast artificial chromosome (YAC), or a mammalian artificial chromosome (MAC).

**4.** A transformed cell comprising a nucleic acid of claim 1, or an expression cassette, a vector or a cloning vehicle of claim 3.

**5.** A transgenic non-human animal, plant, plant part, or seed comprising a nucleic acid of claim 1, or an expression cassette, a vector or a cloning vehicle of claim 3, or a transformed cell of claim 4.

**6.** An isolated, synthetic, or recombinant polypeptide or peptide having an esterase activity comprising:

(a) an amino acid sequence having at least 80%, 81%, 82%, 83%, 84%, 85%, 86%, 87%, 88%, 89%, 90%, 91%, 92%, 93%, 94%, 95%, 96%, 97%, 98% or 99%, or 100% (complete) sequence identity to SEQ ID NO: 158, SEQ ID NO:2, SEQ ID NO:4, SEQ ID NO:6, SEQ ID NO:8, SEQ ID NO:10, SEQ ID NO: 12, SEQ ID NO: 14, SEQ ID NO:16, SEQ ID NO:18, SEQ ID NO.20, SEQ ID NO:22, SEQ ID NO:24, SEQ ID NO:26, SEQ ID NO:28, SEQ ID NO:30, SEQ ID NO:32, SEQ ID NO:34, SEQ ID NO:36, SEQ ID NO:38, SEQ ID NO:40, SEQ ID NO.42, SEQ ID NO:44, SEQ ID NO:46, SEQ ID NO:48, SEQ ID NO:50, SEQ ID NO:52, SEQ ID NO:54, SEQ ID NO:56, SEQ ID NO:58, SEQ ID NO:60, SEQ ID NO:62, SEQ ID NO:64, SEQ ID NO:66, SEQ ID NO: 68, SEQ ID NO:70, SEQ ID NO:72, SEQ ID NO:74, SEQ ID NO:76, SEQ ID NO:78, SEQ ID NO:80, SEQ ID NO:82, SEQ ID NO:84, SEQ ID NO:86, SEQ ID NO:88, SEQ ID NO:90, SEQ ID NO:92, SEQ ID NO:94, SEQ ID NO:96, SEQ ID NO:98, SEQ ID NO:100, SEQ ID NO:102, SEQ ID NO:104, SEQ ID NO:106, SEQ ID NO: 108, SEQ ID NO:110, SEQ ID NO:112, SEQ ID NO:114, SEQ ID NO:116, SEQ ID NO:118, SEQ ID NO:120, SEQ ID NO:122, SEQ ID NO:124, SEQ ID NO:126, SEQ ID NO:128, SEQ ID NO:130, SEQ ID NO:132, SEQ ID NO:134, SEQ ID NO:136, SEQ ID NO:138, SEQ ID NO:140, SEQ ID NO:142, SEQ ID NO:143, SEQ ID NO: 146, SEQ ID NO:148, SEQ ID NO:150, SEQ ID NO:152, SEQ ID NO:154, SEQ ID NO:156, SEQ ID NO:160, SEQ ID NO:162, SEQ ID NO:164 and/or SEQ ID NO:166, over a region of at least about 10, 15, 20, 25, 30, 35, 40, 45, 50, 75, 100, 150, 200, 250, 300, 350 or 400 or more residues, or over the full length of the polypeptide, and/or enzymatically active fragments thereof,
wherein optionally the sequence identities are determined by analysis with a sequence comparison algorithm or by a visual inspection;
(b) an amino acid sequence encoded by a nucleic acid of claim 1;
(c) the amino acid sequence of (a) or (b), and having at least one conservative amino acid substitution, wherein the conservative amino acid substitution substitutes one amino acid for another of the same class, and optionally the at least one conservative amino acid substitution is: substitution of one hydrophobic amino acid for another; substitution of isoleucine, valine, leucine or methionine for an isoleucine, valine, leucine or methionine; substitution of one polar amino acid for another; substitution of arginine for lysine, glutamic acid for aspartic acid or glutamine for asparagine; wherein the polypeptide having the at least one conservative amino acid substitution has an esterase activity or can generate a humoral immune response to make an anti-esterase antibody;
(d) an amino acid sequence encoded by a nucleic acid generated by amplification of a polynucleotide using an amplification primer pair, wherein the primer pair is capable of amplifying the nucleic acid sequence of claim 1, wherein a first member of the amplification primer pair comprises at least the first (the 5') 12 bases of the nucleic acid sequence of claim 1, and a second member of the amplification primer comprises at least the first (the 5') 12 bases of the complementary strand of the nucleic acid sequence of claim 1;
(e) the amino acid sequence of (a), (b), (c), or (d), wherein the polypeptide does not contain a signal sequence (signal peptide);
(f) the amino acid sequence of (a), (b), (c), (d), or (e), further comprising a heterologous amino acid sequence;
(g) the amino acid sequence of (f), wherein the heterologous amino acid sequence comprises a signal sequence (signal peptide), and optionally the signal sequence (signal peptide) is derived from another esterase or a non-esterase (a heterologous) enzyme;
(h) the amino acid sequence of (f), wherein the heterologous amino acid sequence comprises a prepro sequence and/ or catalytic domain (CD), or a binding domain, an epitope or a tag;
(i) the amino acid sequence of (f), wherein the heterologous amino acid sequence comprises an N-terminal identification peptide, and optionally the N-terminal identification peptide imparts a desired characteristic, and optionally the desired characteristic is increased stability or simplified purification of the polypeptide;
(j) the amino acid sequence of (a), (b), (c), (d), (e), (f), (g), (h) or (i), wherein the esterase activity comprises a lipase activity or a phospholipase activity; or
(k) the amino acid sequence of (a), (b), (c), (d), (e), (f), (g), (h), (i), or (j), wherein the polypeptide comprises at least one glycosylation site.

**7.** A method of producing a recombinant polypeptide comprising:

(I)

(a) providing a nucleic acid of claim 1; and
(b) expressing the nucleic acid of (a) under conditions that allow expression of the polypeptide, thereby producing a recombinant polypeptide, wherein optionally the method further comprising transforming a host cell with the nucleic acid of (a) followed by expressing the nucleic acid of (a), thereby producing a recombinant polypeptide in a transformed cell; or

(II)

(a) providing a vector comprising the nucleic acid of claim 1; and
(b) expressing the vector of (a), wherein optionally expression is effected by use of a high activity promoter, a dicistronic vector or by gene amplification of the vector.

**8.** A method of generating a variant of a nucleic acid encoding a polypeptide with an esterase activity comprising:

(a) providing a template nucleic acid comprising a nucleic acid of claim 1; and
(b) modifying, deleting, or adding one or more nucleotides in the template sequence, or a combination thereof, to generate a variant of the template nucleic acid; or
(c) the method of (a) and (b), further comprising expressing the variant, wherein optionally the variant:

(i) is thermotolerant, and retains some activity after being exposed to an elevated temperature;
(ii) has increased glycosylation as compared to the esterase encoded by a the template nucleic acid,
(iii) has an altered or different activity or an altered or different stability from that of a polypeptide encoded by the template nucleic acid, or
(iv) has an altered codon usage from that of the template nucleic acid.

**9.** A method for modifying codons in a nucleic acid encoding an esterase polypeptide, the method comprising:

(a) providing a nucleic acid encoding a polypeptide with an esterase activity comprising a sequence of claim 1; and
(b) identifying a codon in the nucleic acid of (a) and replacing it with a different codon encoding the same amino acid as the replaced codon, thereby modifying codons in a nucleic acid encoding an esterase.

**10.** A method for hydrolyzing an ester bond comprising:

(a) providing a polypeptide having an esterase activity, wherein the polypeptide comprises a polypeptide of claim 6 or a polypeptide encoded by a nucleic acid of claim 1;
(b) providing a composition comprising an ester bond; and
(c) contacting the polypeptide of (a) with the composition of (b) under conditions wherein the polypeptide hydrolyzes the ester bond.

**11.** A composition comprising a polypeptide of claim 6 or a polypeptide encoded by a nucleic acid of claim 1,
wherein optionally the composition is a beverage, food, feed, dietary composition, infant formula, detergent, pharmaceutical, fabric, yarn, fiber, or biomass,
wherein optionally the composition comprises an oil or a fat,
wherein optionally the composition comprises a milk,
wherein optionally, the pharmaceutical composition is for ameliorating or preventing lipopolysaccharide (LPS)- mediated toxicity,
wherein optionally, the polypeptide is glycosylated.

**12.** A method for stereoselectively hydrolyzing racemic mixtures of esters comprising:

(a) providing a polypeptide of claim 6 or a polypeptide encoded by a nucleic acid of claim 1, wherein the polypeptide is stereoselective;
(b) providing a composition comprising a racemic mixture of esters; and
(c) contacting the polypeptide of (a) with the composition of (b) under conditions wherein the polypeptide of (b) can selectively hydrolyze the esters.

**13.** A method for converting biomass or any lignocellulosic material into a fuel comprising contacting the biomass or lignocellulosic material with polypeptide of claim 6 or a polypeptide encoded by a nucleic acid of claim 1, wherein optionally the biomass or lignocellulosic material has been pre-treated prior to contact with the polypeptide, wherein optionally the pretreatment comprises contacting the biomass or lignocellulosic material with a cellulose and/or hemicellulose degrading enzyme, and
wherein optionally the fuel is an ethanol, methanol, propanol, butanol and/or diesel.

**14.** A method for treating a composition comprising contacting the composition with polypeptide of claim 6 or a polypeptide encoded by a nucleic acid of claim 1,
wherein optionally the composition comprises a paper, paper pulp, wood, wood pulp, wood product, wood chips, sawdust, paper waste, solid waste, waste water, textile, latex, fabric or cloth,
wherein optionally the composition comprises a beverage, food, feed, dietary composition, infant formula, oil, fat, biomass, detergent, lubricant, or pharmaceutical.

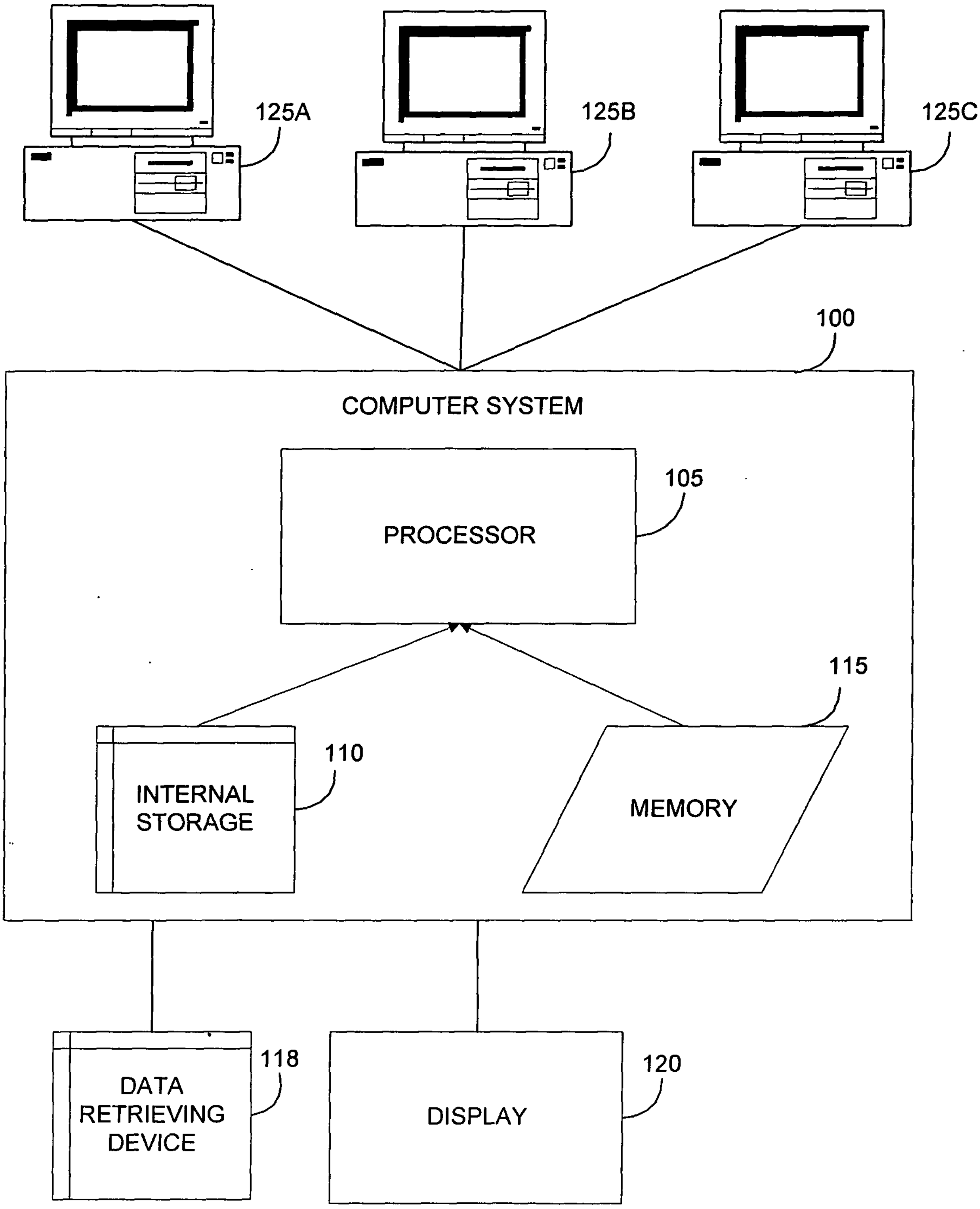
125A
125B
125C
100
COMPUTER SYSTEM
PROCESSOR
105
115
110
INTERNAL STORAGE
MEMORY
118
DATA RETRIEVING DEVICE
DISPLAY
120

FIGURE 1

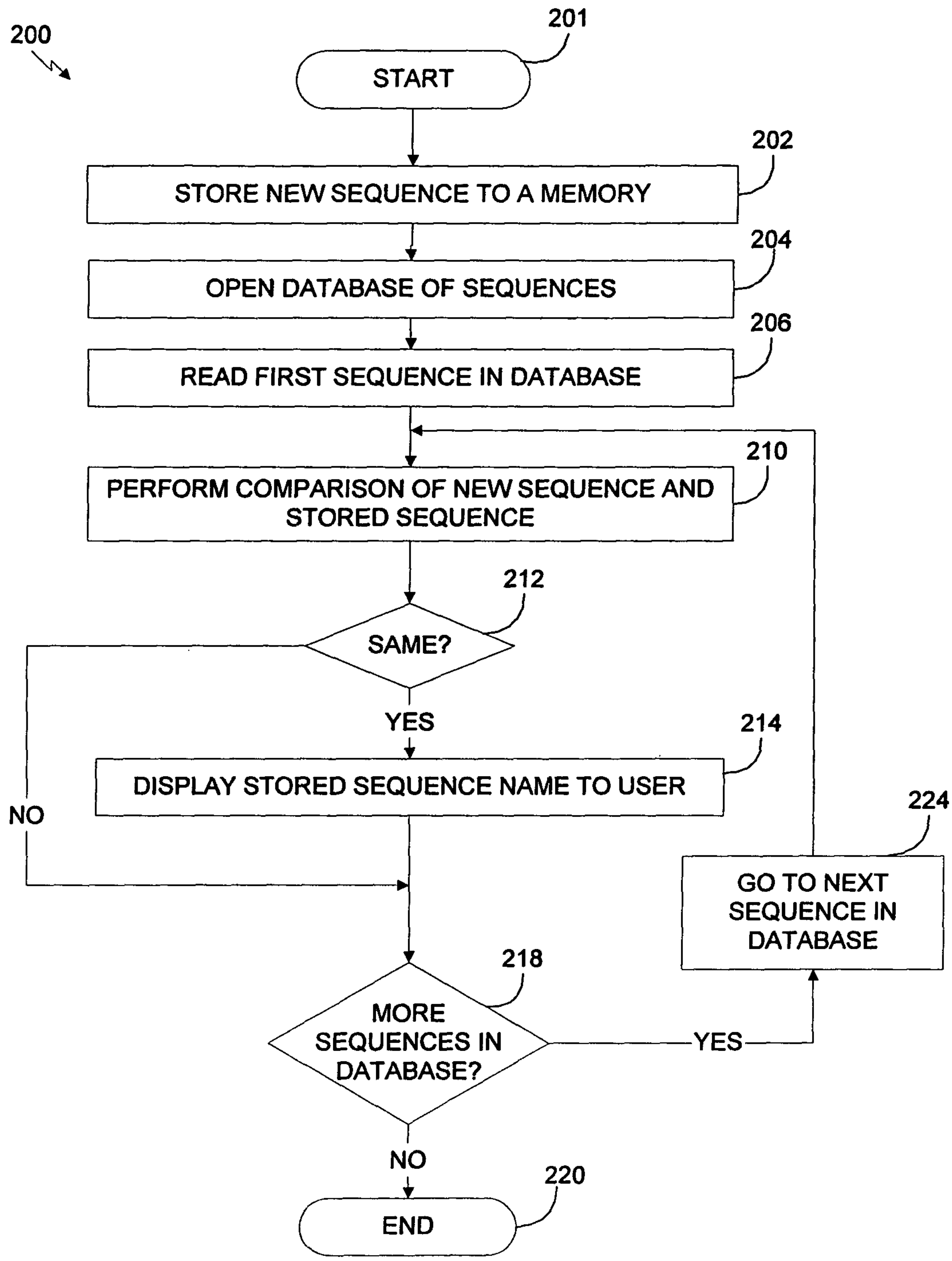
200
201
START
202
STORE NEW SEQUENCE TO A MEMORY
204
OPEN DATABASE OF SEQUENCES
206
READ FIRST SEQUENCE IN DATABASE
210
PERFORM COMPARISON OF NEW SEQUENCE AND STORED SEQUENCE
212
SAME?
YES
214
DISPLAY STORED SEQUENCE NAME TO USER
NO
224
GO TO NEXT SEQUENCE IN DATABASE
218
MORE SEQUENCES IN DATABASE?
YES
NO
220
END

FIGURE 2

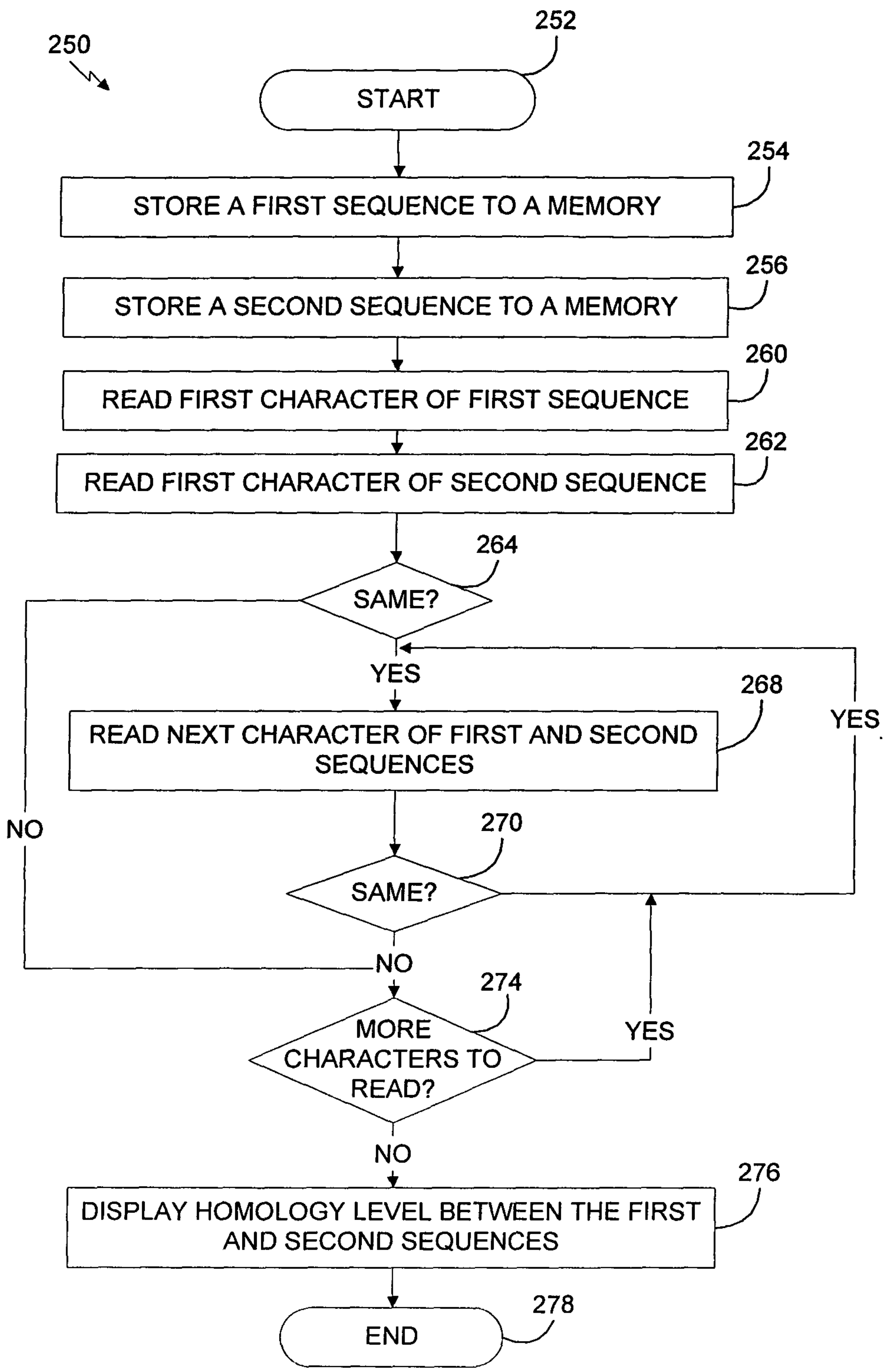
250
252
START
254
STORE A FIRST SEQUENCE TO A MEMORY
256
STORE A SECOND SEQUENCE TO A MEMORY
260
READ FIRST CHARACTER OF FIRST SEQUENCE
262
READ FIRST CHARACTER OF SECOND SEQUENCE
264
SAME?
YES
268
READ NEXT CHARACTER OF FIRST AND SECOND SEQUENCES
YES
NO
270
SAME?
NO
274
MORE CHARACTERS TO READ?
YES
NO
276
DISPLAY HOMOLOGY LEVEL BETWEEN THE FIRST AND SECOND SEQUENCES
278
END

FIGURE 3

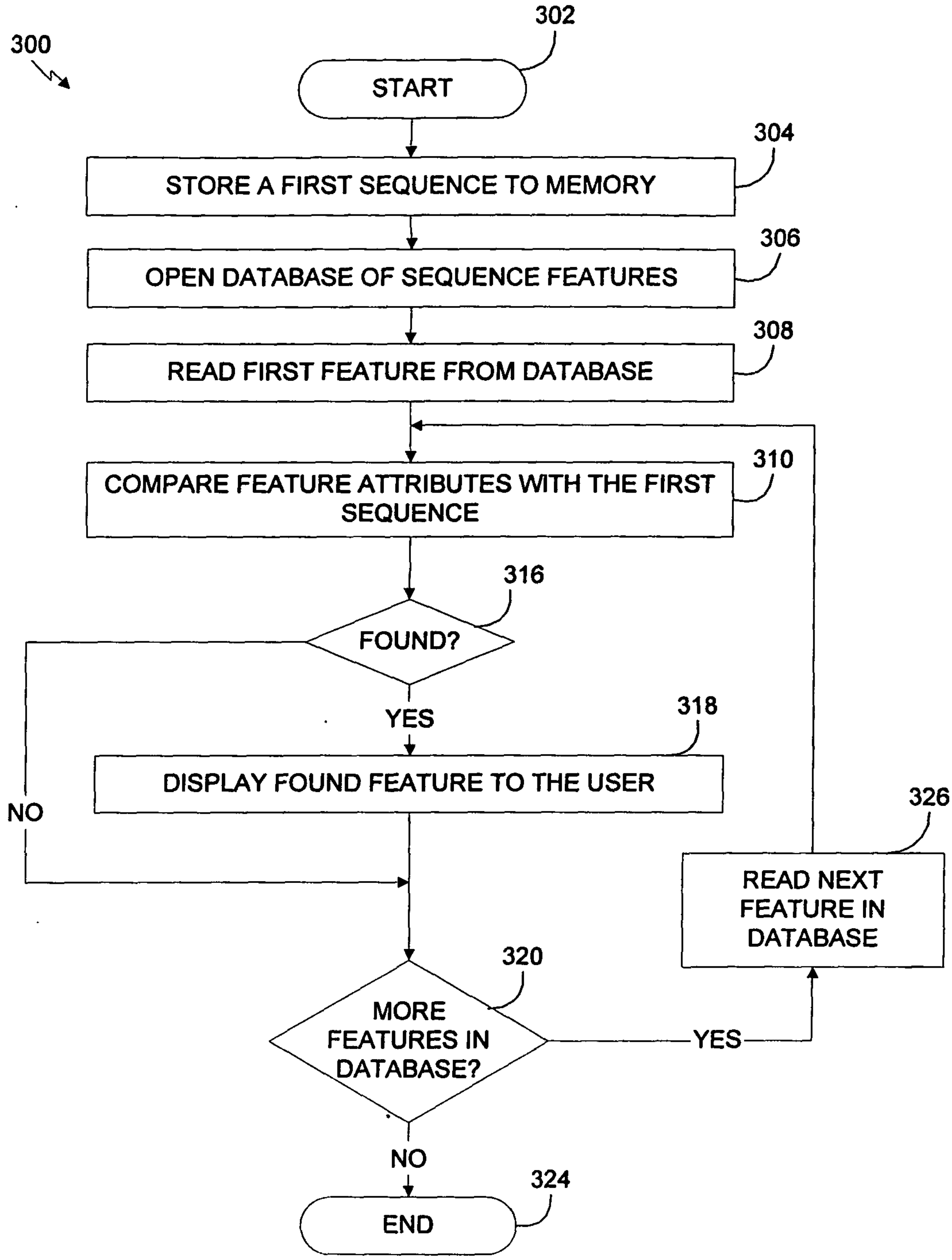
300
302
START
304
STORE A FIRST SEQUENCE TO MEMORY
306
OPEN DATABASE OF SEQUENCE FEATURES
308
READ FIRST FEATURE FROM DATABASE
310
COMPARE FEATURE ATTRIBUTES WITH THE FIRST SEQUENCE
316
FOUND?
YES
318
DISPLAY FOUND FEATURE TO THE USER
NO
326
READ NEXT FEATURE IN DATABASE
320
MORE FEATURES IN DATABASE?
YES
NO
324
END

FIGURE 4

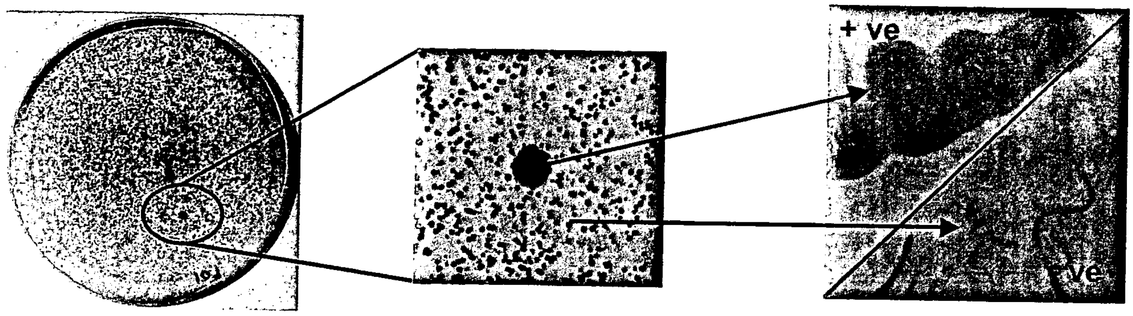


FIGURE 5

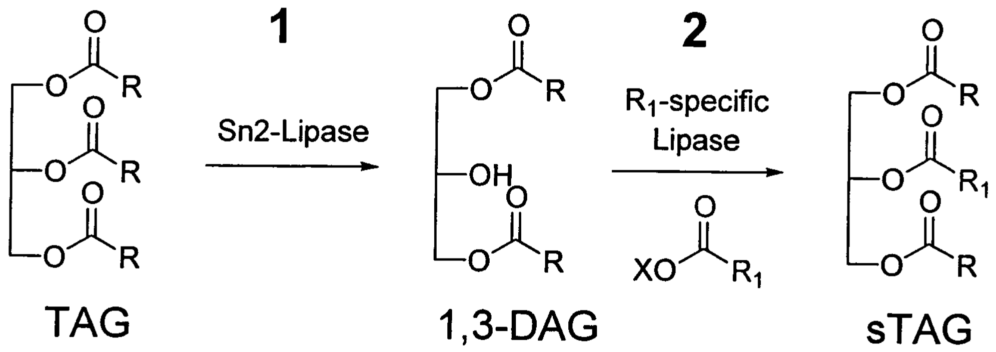


**FIGURE 6**

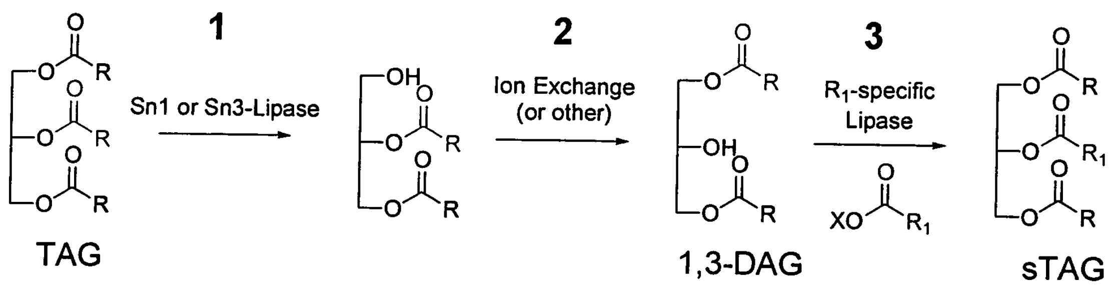


TAG

1,3-DAG

sTAG

**FIGURE 7**

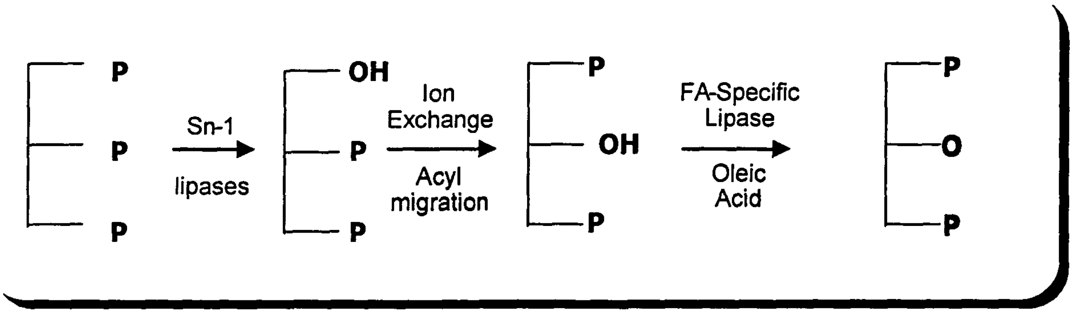
Sn-1
lipases
Ion
Exchange
Acyl
migration
FA-Specific
Lipase
Oleic
Acid

FIGURE 8

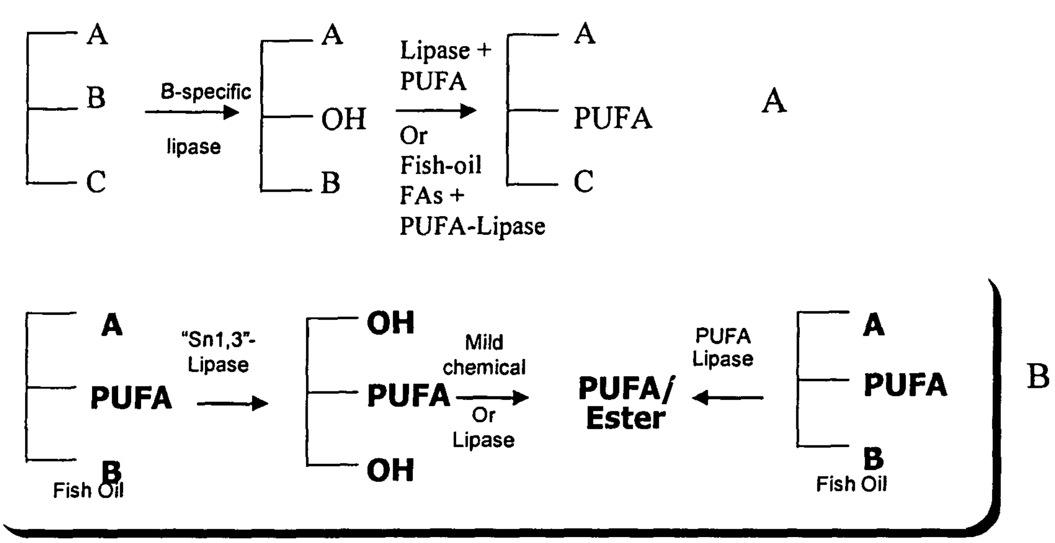
B-specific
lipase
Lipase +
PUFA
Or
Fish-oil
FAs +
PUFA-Lipase
A
"Sn1,3"-
Lipase
Fish Oil
Mild
chemical
Or
Lipase
PUFA
Lipase
Fish Oil
B

**FIGURE 9**

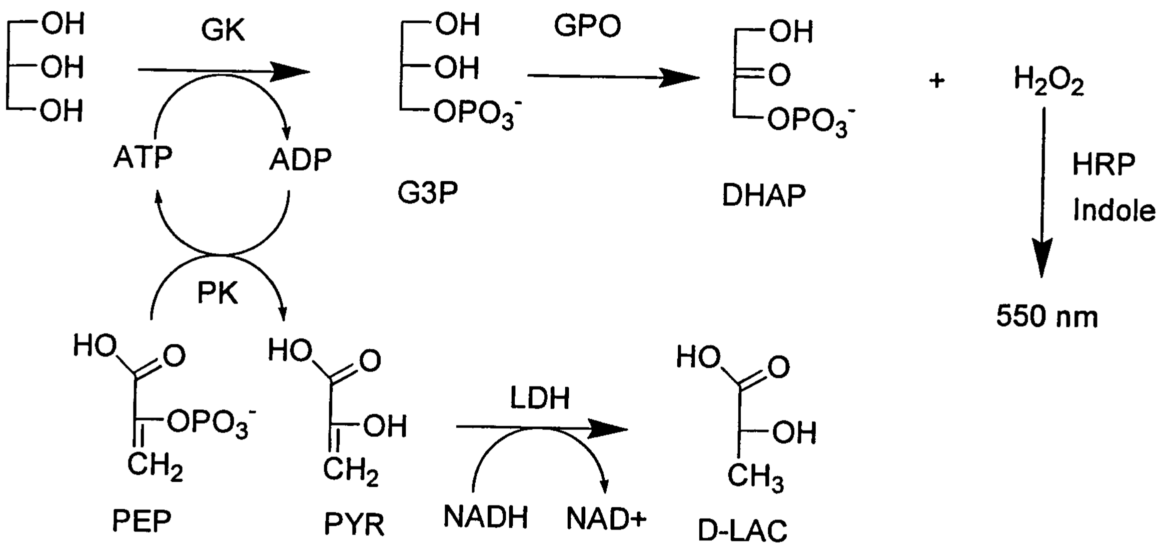
GK
ATP
ADP
PK
G3P
GPO
DHAP
+
HRP
Indole
550 nm
PEP
PYR
LDH
NADH
NAD+
D-LAC

**FIGURE 10**

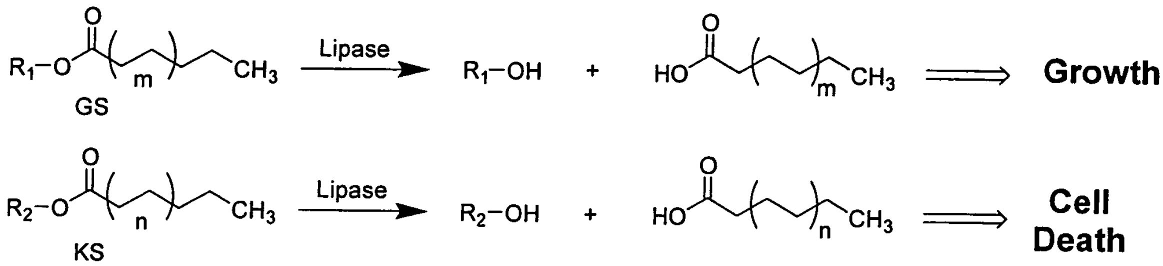


**FIGURE 11**

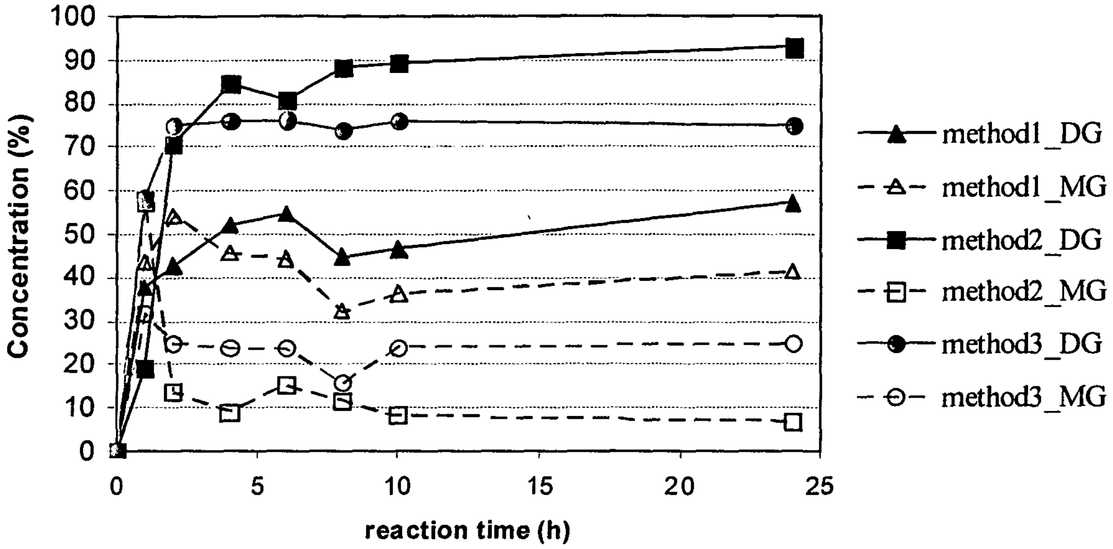
100
90
80
70
60
50
40
30
20
10
0
Concentration (%)
0
5
10
15
20
25
reaction time (h)
method1_DG
method1_MG
method2_DG
method2_MG
method3_DG
method3_MG

FIGURE 12

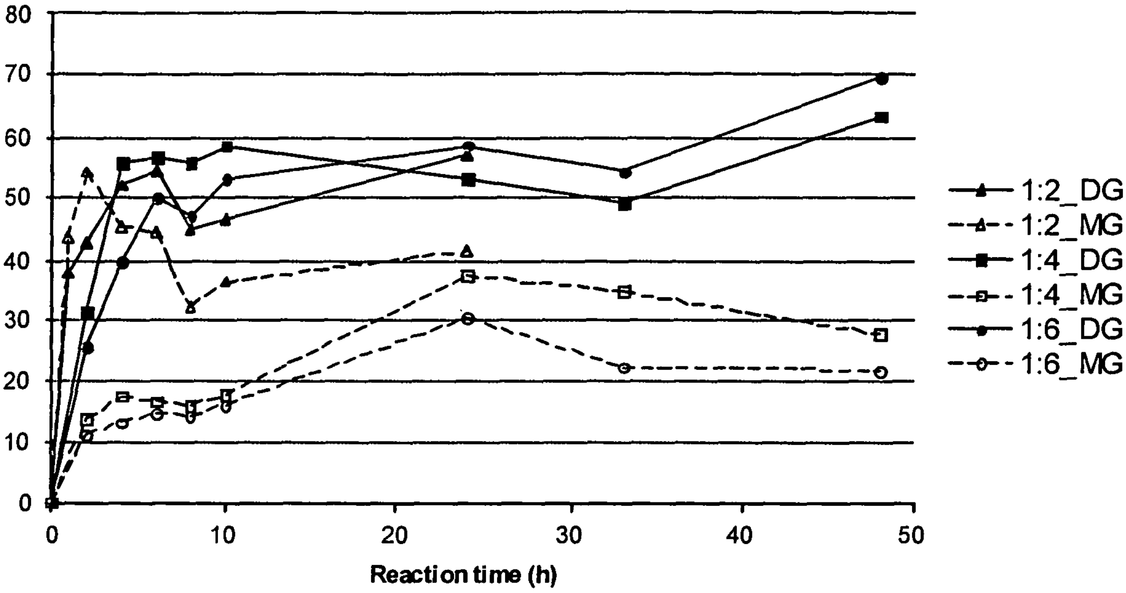
80
70
60
50
40
30
20
10
0
0
10
20
30
40
50
Reaction time (h)
1:2_DG
1:2_MG
1:4_DG
1:4_MG
1:6_DG
1:6_MG

**FIGURE 13**

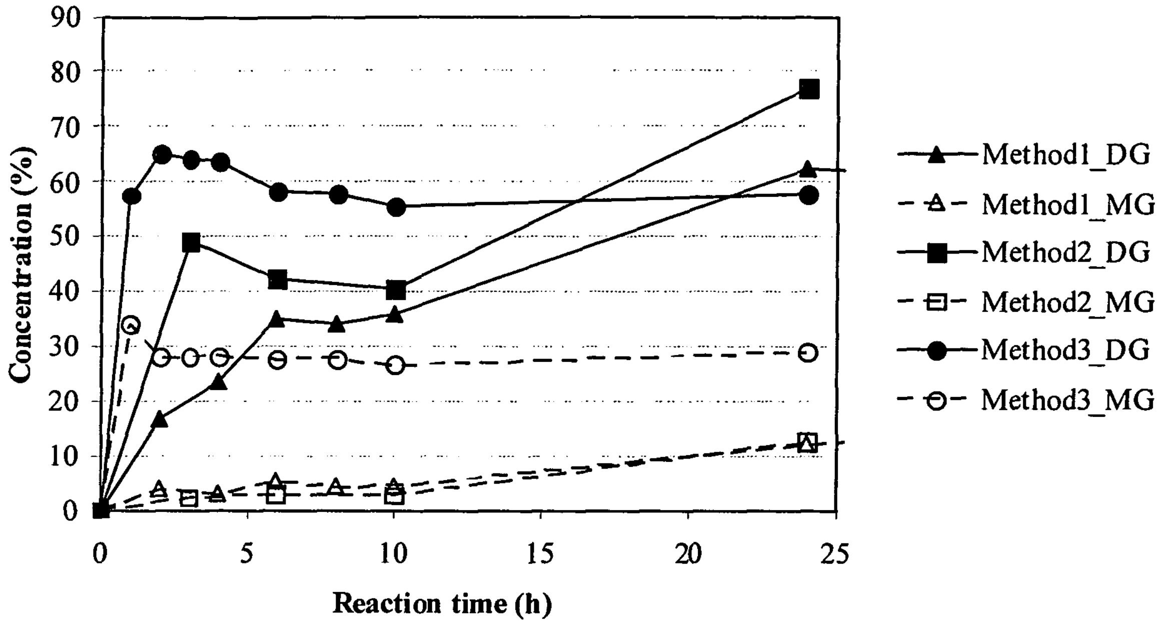
90
80
70
60
50
40
30
20
10
0
Concentration (%)
0
5
10
15
20
25
Reaction time (h)
Method1_DG
Method1_MG
Method2_DG
Method2_MG
Method3_DG
Method3_MG

FIGURE 14

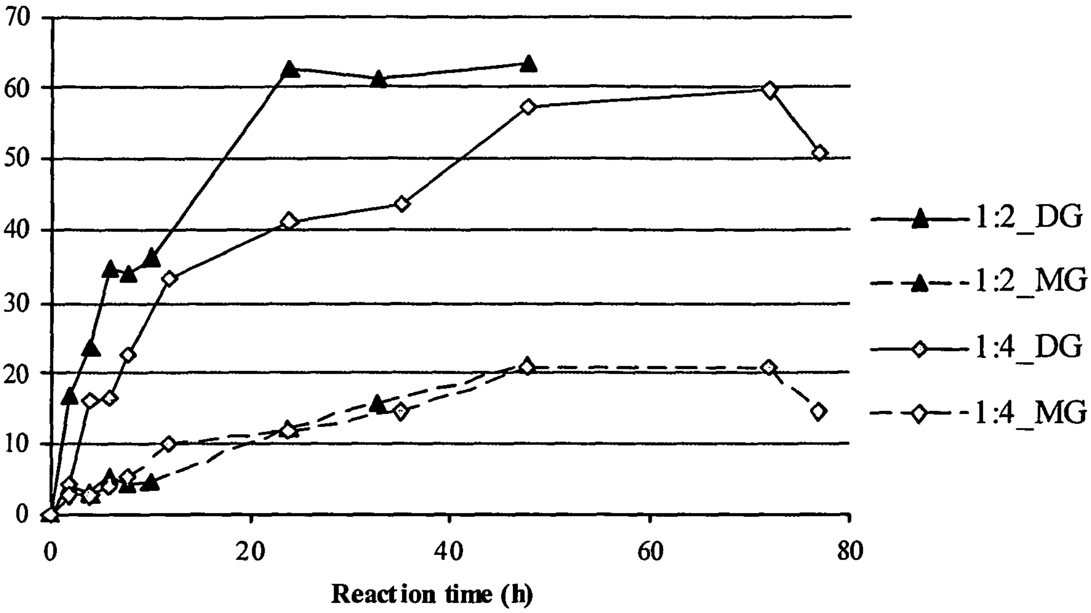
70
60
50
40
30
20
10
0
0
20
40
60
80
Reaction time (h)
1:2_DG
1:2_MG
1:4_DG
1:4_MG

FIGURE 15

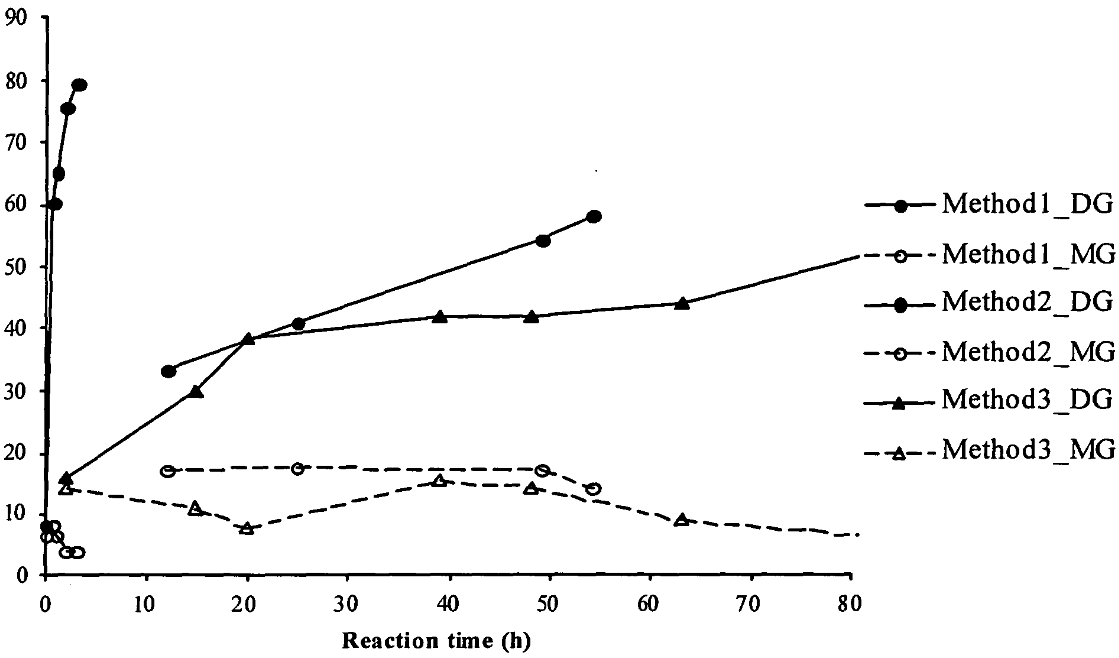
90
80
70
60
50
40
30
20
10
0
0
10
20
30
40
50
60
70
80
Reaction time (h)
Method1_DG
Method1_MG
Method2_DG
Method2_MG
Method3_DG
Method3_MG

FIGURE 16

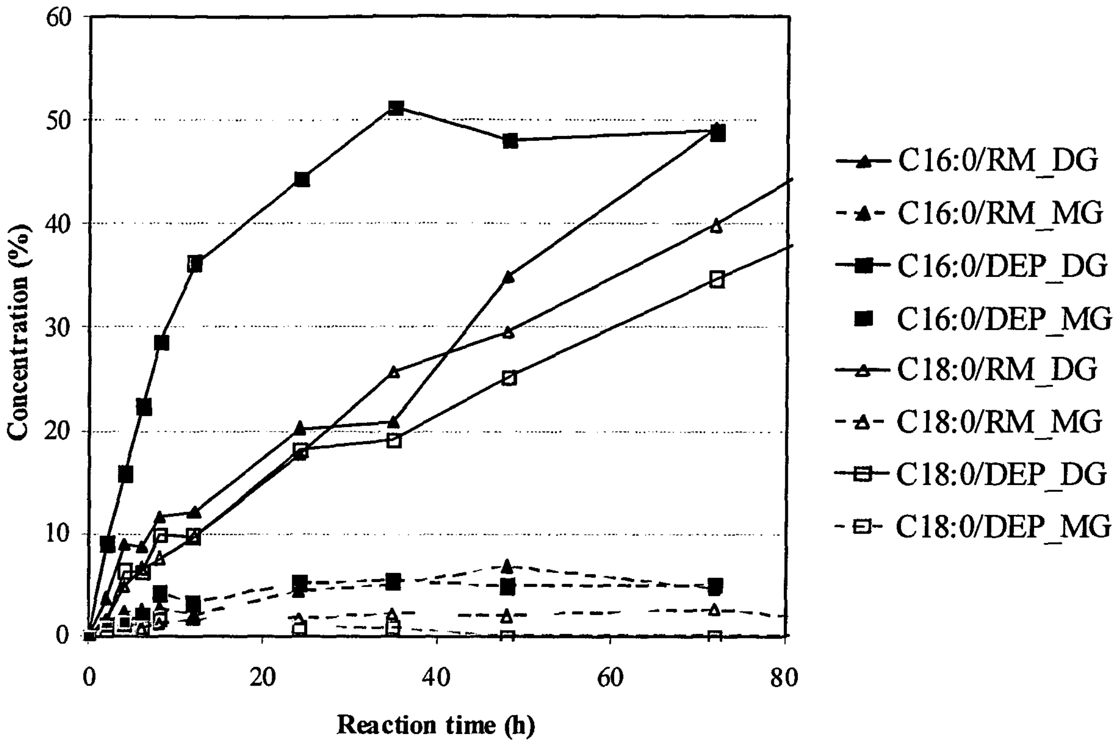

60
50
40
30
20
10
0
Concentration (%)
0
20
40
60
80
Reaction time (h)
C16:0/RM_DG
C16:0/RM_MG
C16:0/DEP_DG
C16:0/DEP_MG
C18:0/RM_DG
C18:0/RM_MG
C18:0/DEP_DG
C18:0/DEP_MG


**FIGURE 17**

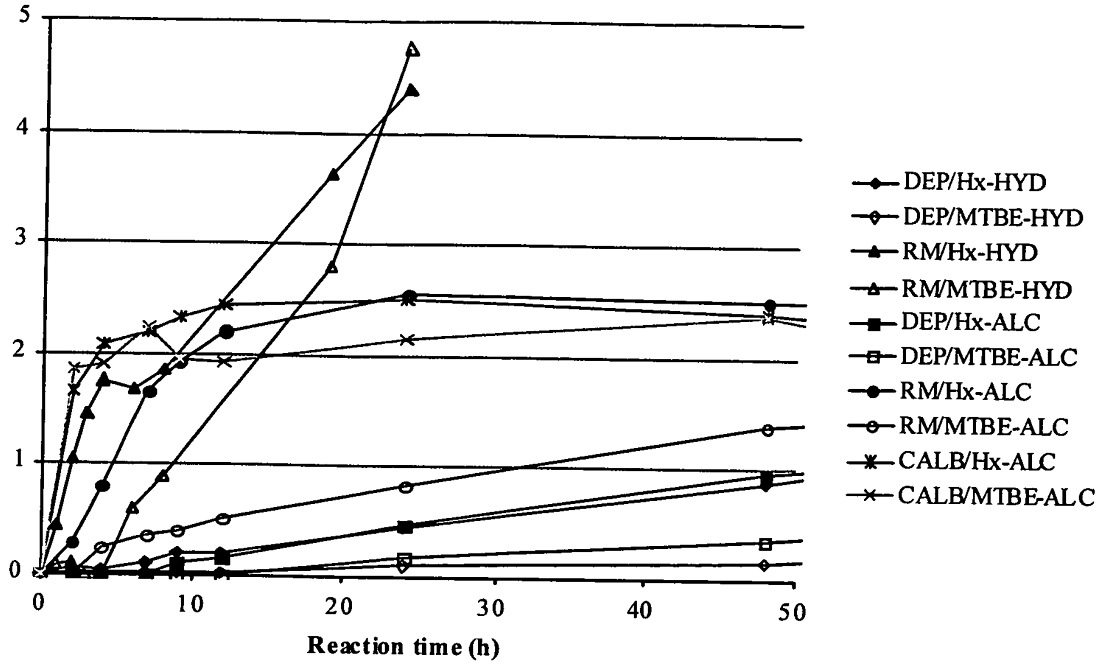
5
4
3
2
1
0
0
10
20
30
40
50
Reaction time (h)
DEP/Hx-HYD
DEP/MTBE-HYD
RM/Hx-HYD
RM/MTBE-HYD
DEP/Hx-ALC
DEP/MTBE-ALC
RM/Hx-ALC
RM/MTBE-ALC
CALB/Hx-ALC
CALB/MTBE-ALC

**FIGURE 18**

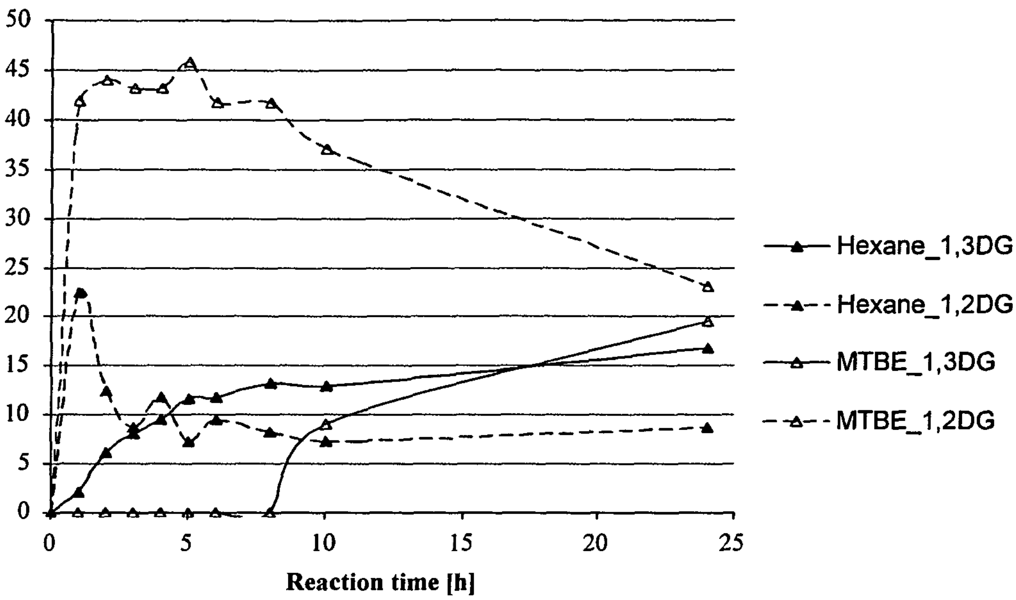
50
45
40
35
30
25
20
15
10
5
0
0
5
10
15
20
25
Reaction time [h]
Hexane_1,3DG
Hexane_1,2DG
MTBE_1,3DG
MTBE_1,2DG

FIGURE 19

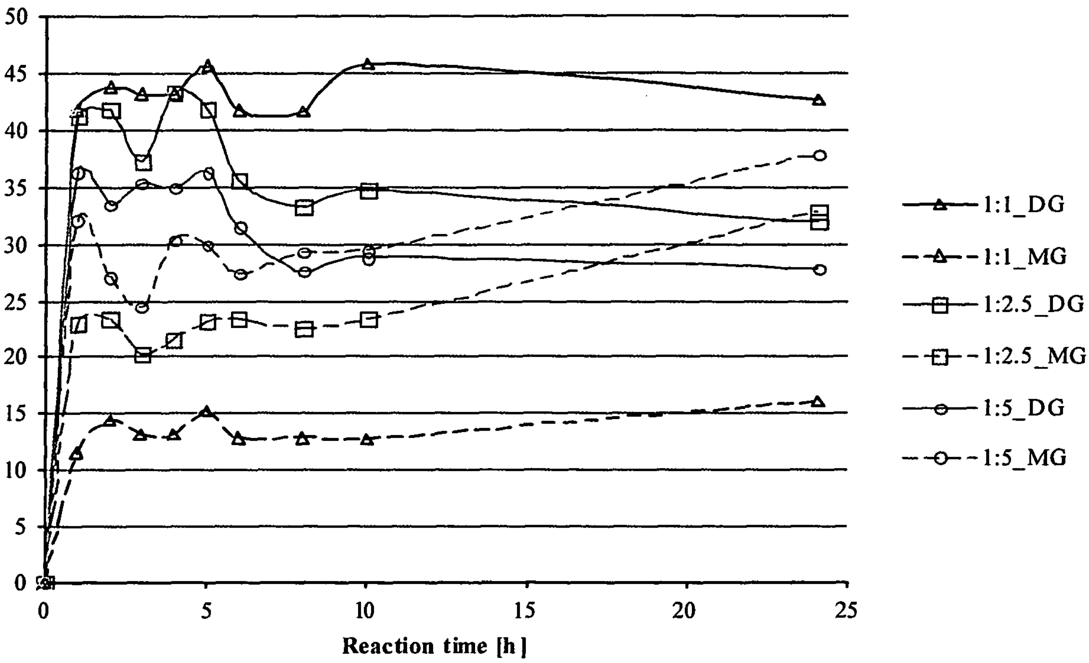
50
45
40
35
30
25
20
15
10
5
0
0
5
10
15
20
25
Reaction time [h]
1:1_DG
1:1_MG
1:2.5_DG
1:2.5_MG
1:5_DG
1:5_MG

FIGURE 20

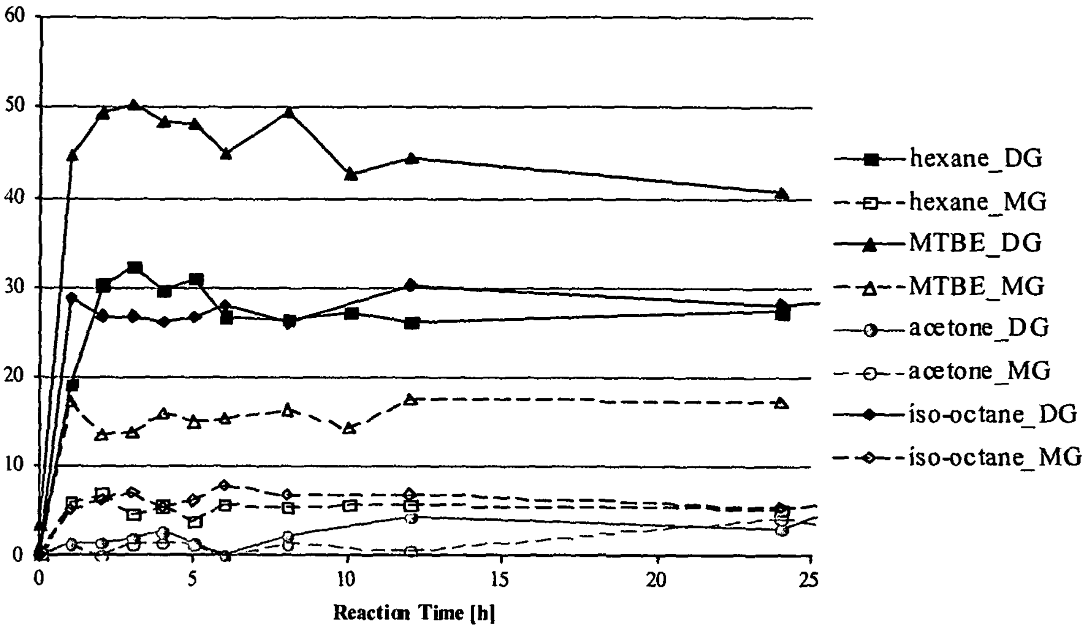
60
50
40
30
20
10
0
0
5
10
15
20
25
Reaction Time [h]
hexane_DG
hexane_MG
MTBE_DG
MTBE_MG
acetone_DG
acetone_MG
iso-octane_DG
iso-octane_MG

FIGURE 21

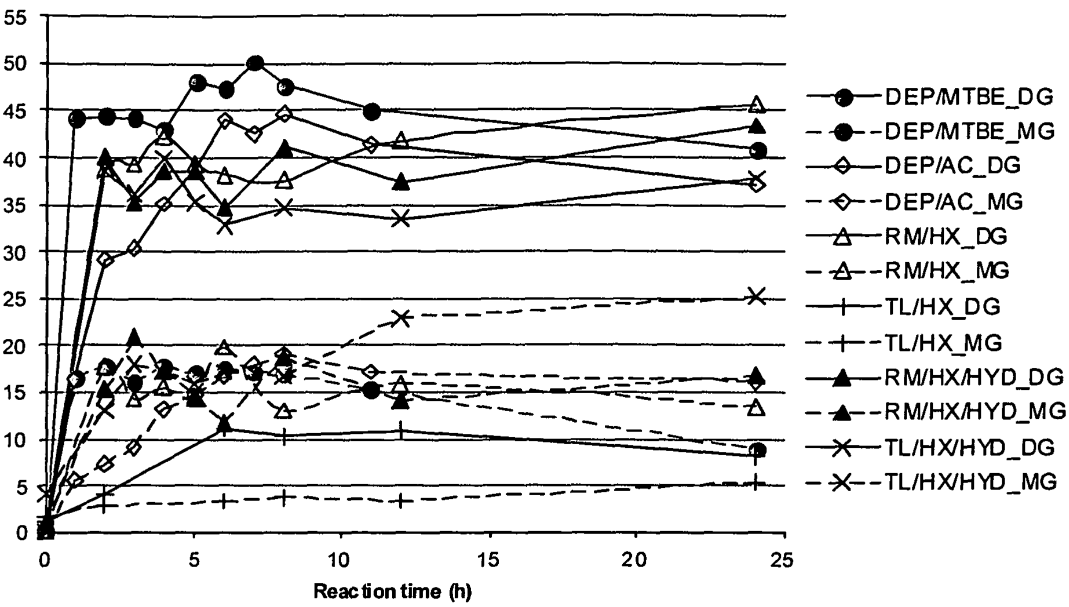
55
50
45
40
35
30
25
20
15
10
5
0
0
5
10
15
20
25
Reaction time (h)
DEP/MTBE_DG
DEP/MTBE_MG
DEP/AC_DG
DEP/AC_MG
RM/HX_DG
RM/HX_MG
TL/HX_DG
TL/HX_MG
RM/HX/HYD_DG
RM/HX/HYD_MG
TL/HX/HYD_DG
TL/HX/HYD_MG

FIGURE 22

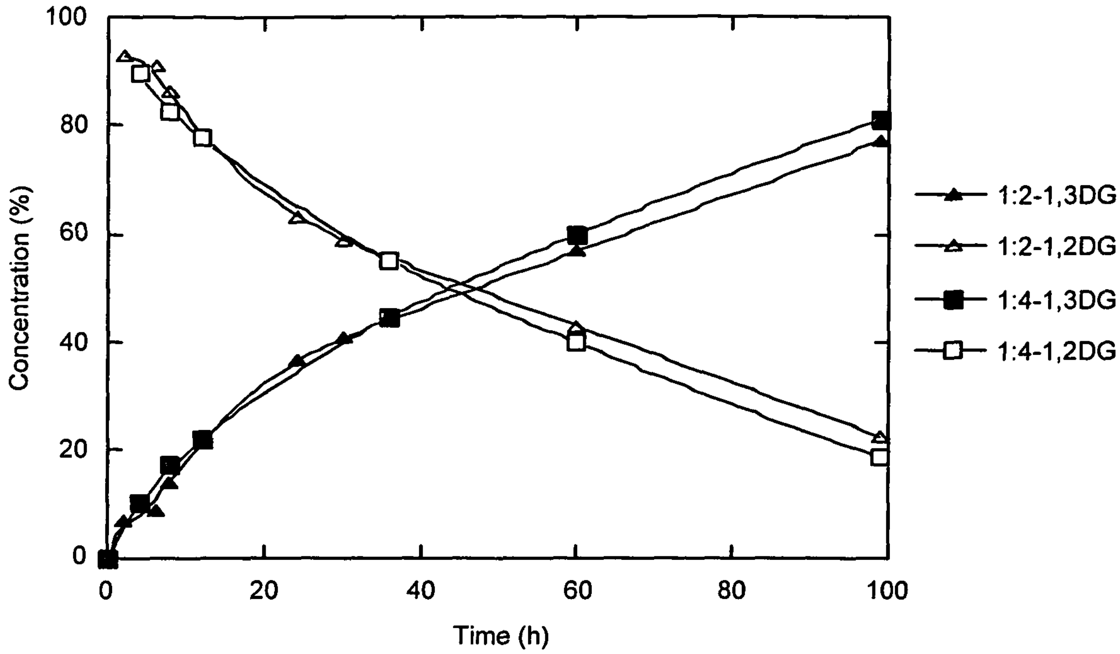
100
80
60
40
20
0
Concentration (%)
0
20
40
60
80
100
Time (h)
1:2-1,3DG
1:2-1,2DG
1:4-1,3DG
1:4-1,2DG

**FIGURE 23**

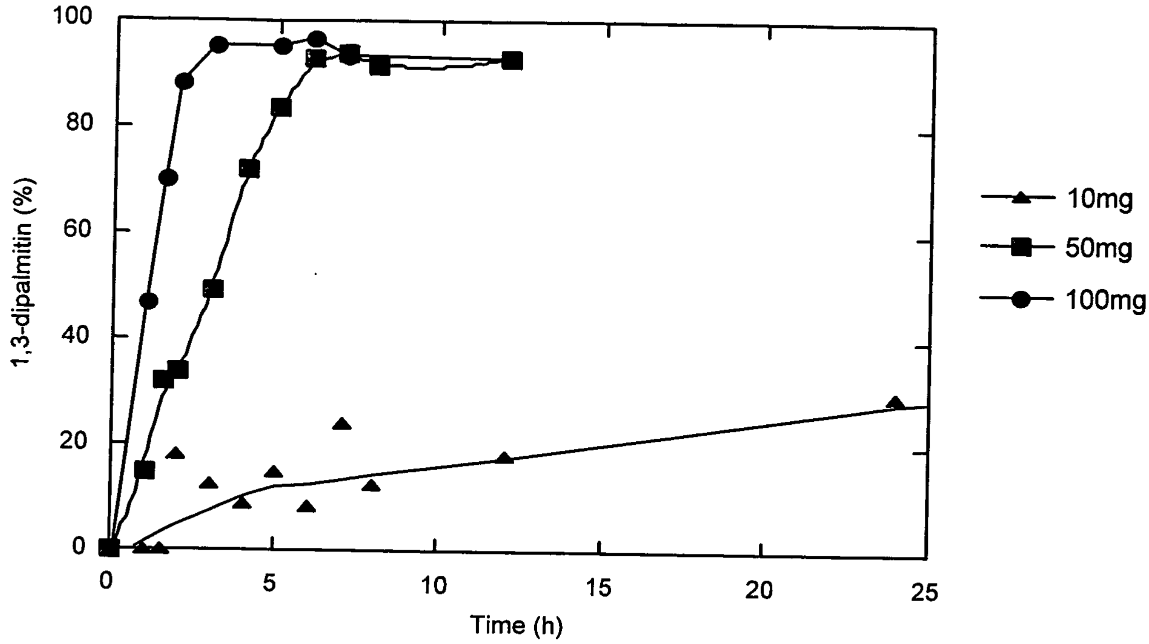
100
80
60
40
20
0
1,3-dipalmitin (%)
0
5
10
15
20
25
Time (h)
10mg
50mg
100mg

FIGURE 24

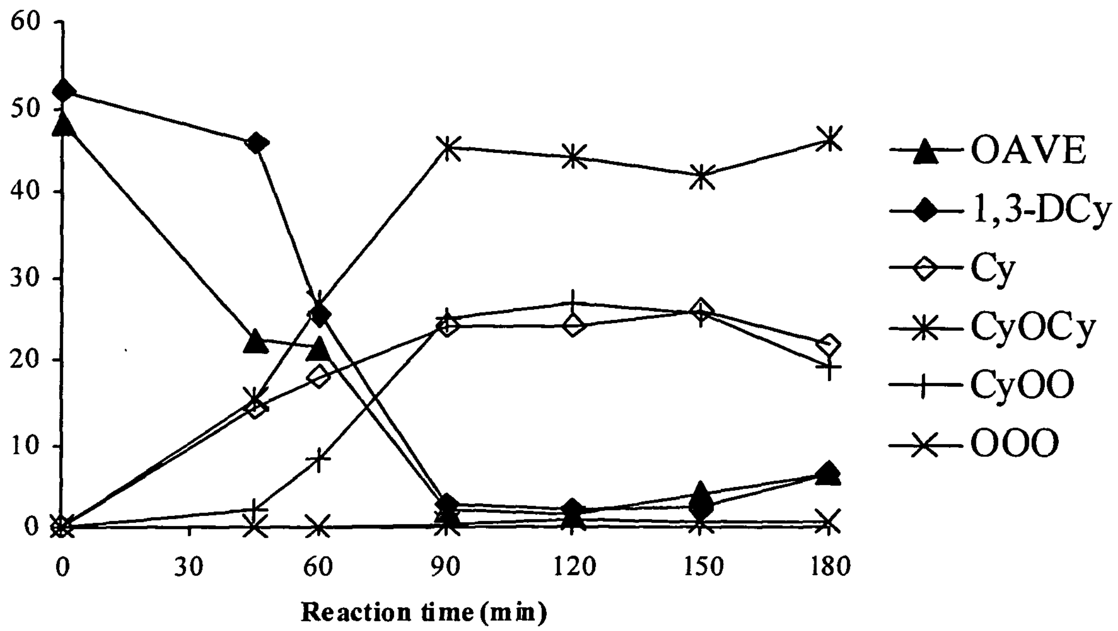
60
50
40
30
20
10
0
0
30
60
90
120
150
180
Reaction time (min)
OAVE
1,3-DCy
Cy
CyOCy
CyOO
OOO

**FIGURE 25**

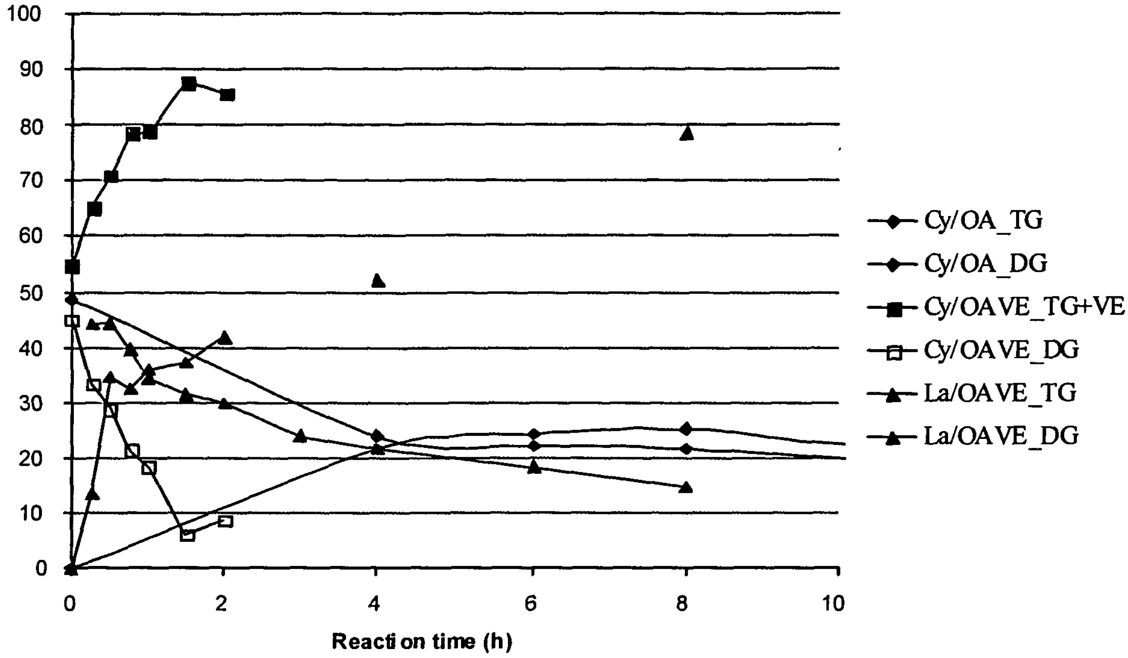
100
90
80
70
60
50
40
30
20
10
0
0
2
4
6
8
10
Reaction time (h)
Cy/OA_TG
Cy/OA_DG
Cy/OAVE_TG+VE
Cy/OAVE_DG
La/OAVE_TG
La/OAVE_DG

FIGURE 26

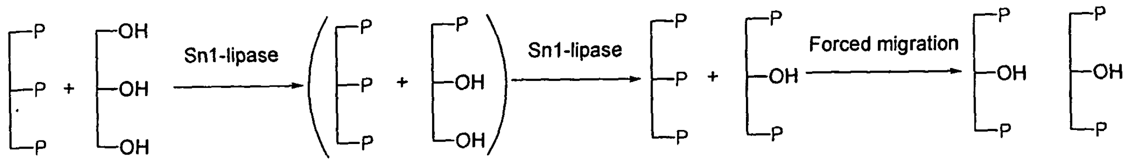


2 : 1

1 : 1

FIGURE 27

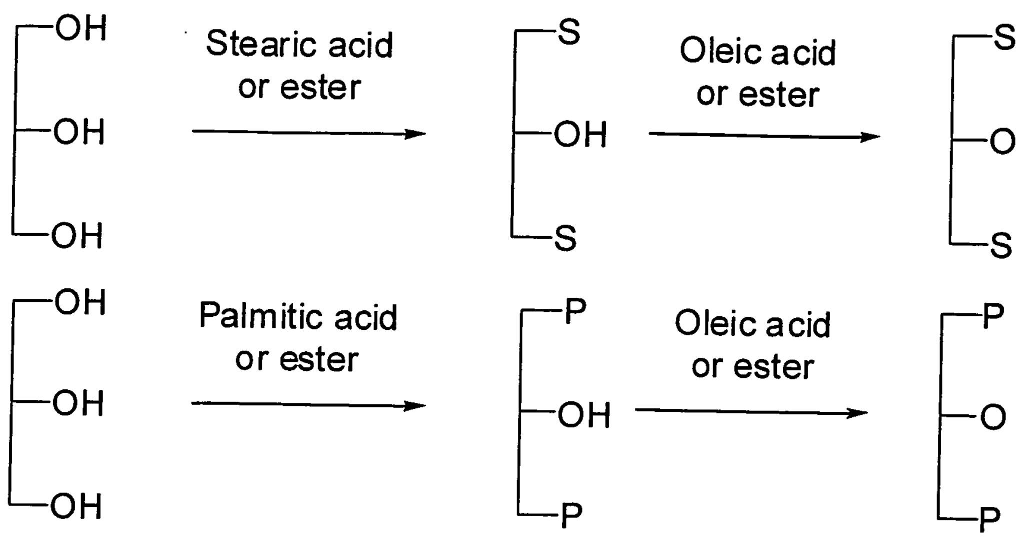
Stearic acid
or ester
Oleic acid
or ester
Palmitic acid
or ester
Oleic acid
or ester

**FIGURE 28**

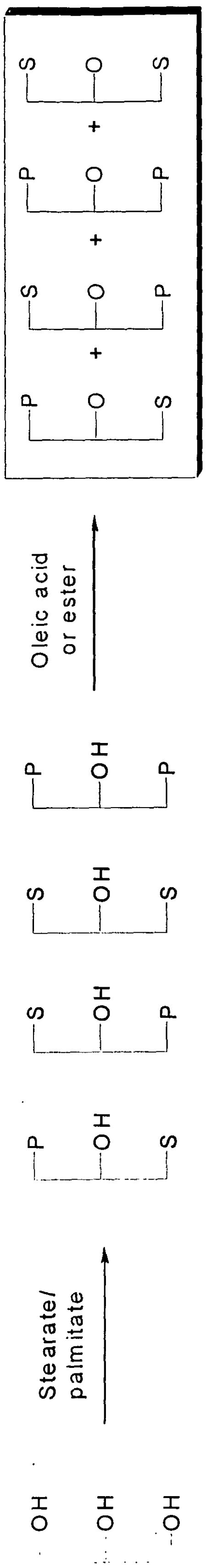


FIGURE 29

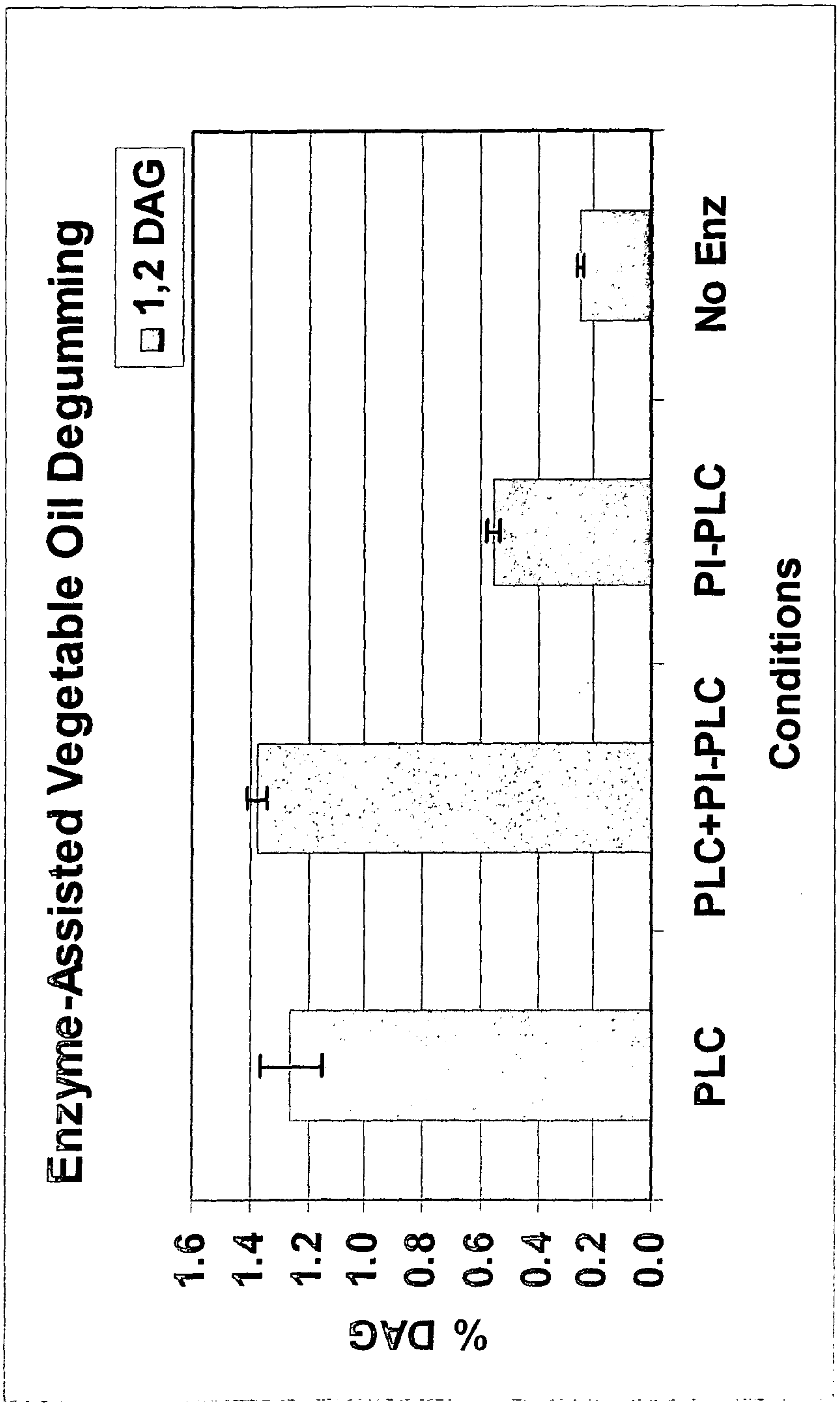
Enzyme-Assisted Vegetable Oil Degumming
1,2 DAG
% DAG
1.6
1.4
1.2
1.0
0.8
0.6
0.4
0.2
0.0
PLC
PLC+PI-PLC
PI-PLC
No Enz
Conditions

FIGURE 30

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5229280 A
- US 5108916 A
- US 5552317 A
- US 5834259 A
- US 4458066 A
- US 5721118 A
- US 6025155 A
- US 4962028 A
- US 5633440 A
- US 5608148 A, John
- US 5602321 A
- US 5589583 A, Klee
- US 5217879 A
- US 5750870 A
- US 4683195 A
- US 4683202 A
- WO 9703211 A
- WO 9639154 A
- US 4987071 A, Cech
- US 6506559 B
- US 6511824 B
- US 6515109 B
- US 6489127 B
- US 6361974 B
- US 5830696 A
- US 6291242 B
- US 6287862 B
- US 6287861 B
- US 5955358 A
- US 5830721 A
- US 5824514 A
- US 5811238 A
- US 5605793 A
- US 5834252 A, Stemmer
- US 5837458 A, Minshull
- WO 9522625 A, Stemmer and Crameri
- WO 9633207 A, Stemmer
- WO 9720078 A, Stemmer and Crameri
- WO 9735966 A, Minshull and Stemmer
- WO 9941402 A, Punnonen
- WO 9941383 A, Punnonen
- WO 9941369 A, Punnonen
- WO 9941368 A, Punnonen
- EP 752008 A, Stemmer and Crameri
- EP 0932670 A, Stemmer
- WO 9923107 A, Stemmer
- WO 9921979 A, Apt
- WO 9831837 A, del Cardayre
- WO 9827230 A, Patten and Stemmer
- WO 0000632 A
- WO 0009679 A
- WO 9842832 A, Arnold
- WO 9929902 A, Arnold
- WO 9841653 A
- WO 9841622 A, Borchert
- WO 9842727 A, Pati and Zarling
- US 09407800 B
- US 6379964 B
- US 6319714 B
- US 6368861 B
- US 6376246 B
- US 6423542 B
- US 6426224 B
- US 0001203 W
- US 6436675 B
- US 0001202 W, Selifonov
- US 09618579 B, Selifonov
- US 0001138 W, Selifonov and Stemmer
- US 09656549 B, Affholter
- US 6177263 B
- US 6153410 A
- US 6280926 B
- US 5939250 A
- US 6171820 B
- US 6579258 B
- US 6238884 B
- US 6773900 B
- US 6740506 B
- US 6713282 B
- US 6635449 B
- US 6605449 B
- US 6537776 B
- US 5965408 A
- WO 9116427 A
- US 5795737 A
- US 6211428 B
- US 6187992 B
- US 6156952 A
- US 6118044 A
- US 6111166 A
- US 6107541 A
- US 5959171 A
- US 5922854 A
- US 5892070 A
- US 5880327 A
- US 5891698 A
- US 5639940 A
- US 5573933 A

- US 5387742 A
- US 5087571 A
- US 6309872 B
- US 5015580 A
- US 5681730 A, Ellis
- US 5712135 A, D'Halluin
- US 6337187 B
- US 6277628 B
- US 6277489 B
- US 6261776 B
- US 6258606 B
- US 6054270 A
- US 6048695 A
- US 6045996 A
- US 6022963 A
- US 6013440 A
- US 5965452 A
- US 5959098 A
- US 5856174 A
- US 5830645 A
- US 5770456 A
- US 5632957 A
- US 5556752 A
- US 5143854 A
- US 5807522 A
- US 5800992 A
- US 5744305 A
- US 5700637 A
- US 5434049 A
- WO 9951773 A
- WO 9909217 A
- WO 9746313 A
- WO 9617958 A
- US 20010018642 A
- US 20010019827 A
- US 20010016322 A
- US 20010014449 A
- US 20010014448 A
- US 20010012537 A
- US 20010008765 A
- US 4946778 A
- US 4818695 A
- US 5569594 A
- US 5773266 A
- US 6057103 A
- JP H6306386 B
- US 6551635 B
- US 6399121 B
- WO 03070013 A
- WO 00054601 A
- WO 9966805 A
- US 4752483 A
- US 4707364 A
- WO 9826057 A
- US 6355693 B
- US 6162623 A
- US 6103505 A
- US 6001640 A
- US 5558781 A
- US 5264367 A
- EP 513709 A
- CA 1102795
- US PN5558781 A
- EP 0869167 A
- WO 9818912 A
- JP H5132283 B
- EP 82870032 A
- BE 595219
- EP 0513709 B2
- US 6127137 A
- US 6025171 A
- US 6143545 A
- US 5532163 A
- US 5288619 A
- EP 0513709 A
- DE 4339556 A
- JP 06306386 B
- US 6172248 B
- US 6172247 B
- US 4240972 A
- US 4049686 A
- US 6303803 B
- US 5880300 A
- US 5000975 A
- US 4944944 A
- US 4767628 A
- US 4897268 A
- US 4925673 A
- US 5902617 A
- US 3949105 A
- US 5856150 A
- US 5691181 A
- US 5858755 A
- US 5827718 A
- US 5454971 A
- US 5069810 A
- US 6322595 B
- US 6313081 B
- US 6413928 B
- US 6399561 B
- US 6365561 B
- US 6380147 B
- US 6333301 B
- US 6329333 B
- US 6326341 B
- US 6297038 B
- US 6309871 B
- US 6204232 B
- US 6197070 B
- US 5856164 A
- WO 9701629 A
- US 4404128 A
- US 4261868 A
- US 5204015 A
- US 5733750 A
- US 6265191 B

- US 6261828 B
- US 6077316 A
- US 6024766 A
- US 6021536 A
- US 6017751 A
- US 5980581 A
- US 20020142438 A
- US 6241849 B
- US 6066233 A
- US 5582681 A
- US 5709796 A
- US 5747434 A
- EP 0093602 B2
- US 6264925 B
- US 5762991 A
- US 5536650 A
- US 5405624 A
- US 5021246 A
- US 4788066 A
- US 6660506 B
- US 6423145 B
- US 5536325 A
- US 6409841 B
- US 5705369 A
- US 6712866 B
- US 6333181 B
- US 20060014260 A
- US 20050069998 A
- US 20020164730 A
- US 6090595 A


**Non-patent literature cited in the description**


- **Adams.** *J. Am. Chem. Soc.,* 1983, vol. 105, 661
- **Belousov.** *Nucleic Acids Res.,* 1997, vol. 25, 3440-3444
- **Frenkel.** *Free Radic. Biol. Med.,* 1995, vol. 19, 373-380
- **Blommers.** *Biochemistry,* 1994, vol. 33, 7886-7896
- **Narang.** *Meth. Enzymol.,* 1979, vol. 68, 90
- **Brown.** *Meth. Enzymol.,* 1979, vol. 68, 109
- **Beaucage.** *Tetra. Lett.,* 1981, vol. 22, 1859
- MOLECULAR CLONING: A LABORATORY MANUAL. Cold Spring Harbor Laboratory, 1989, vol. 1-3
- MOLECULAR BIOLOGY. John Wiley & Sons, Inc, 1997
- LABORATORY TECHNIQUES IN BIOCHEMISTRY AND MOLECULAR BIOLOGY: HYBRIDIZATION WITH NUCLEIC ACID PROBES, Part I. Theory and Nucleic Acid Preparation. Elsevier, 1993
- **Rosenfeld.** *Nat. Genet,* 1997, vol. 15, 333-335
- **Woon.** *Genomics,* 1998, vol. 50, 306-316
- **Kern.** *Biotechniques,* 1997, vol. 23, 120-124
- **Williams.** *Biochemistry,* 1995, vol. 34, 1787-1797
- **Dobeli.** *Protein Expr. Purif.,* 1998, vol. 12, 404-414
- **Kroll.** *DNA Cell. Biol.,* 1993, vol. 12, 441-53
- **Mata.** *Toxicol. Appl. Pharmacol.,* 1997, vol. 144, 189-197
- **Strauss-Soukup.** *Biochemistry,* 1997, vol. 36, 8692-8698
- **Samstag.** *Antisense Nucleic Acid Drug Dev,* 1996, vol. 6, 153-156
- **Huang.** *Plant Mol. Biol.,* 1996, vol. 33, 125-139
- **Zhong.** *Mol. Gen. Genet,* 1996, vol. 251, 196-203
- **Solocombe.** *Plant Physiol.,* 1994, vol. 104, 1167-1176
- **Martinez.** *J. Mol. Biol,* 1989, vol. 208, 551-565
- **Manjunath.** *Plant Mol. Biol.,* 1997, vol. 33, 97-112
- **Kumagai.** *Proc. Natl. Acad. Sci. USA,* 1995, vol. 92, 1679-1683
- **Verdaguer.** *Plant Mol. Biol.,* 1996, vol. 31, 1129-1139
- **Kirch.** *Plant Mol. Biol.,* 1997, vol. 33, 897 909
- **Blazquez.** *Plant Cell,* 1998, vol. 10, 791-800
- **Cardon.** *Plant J,* 1997, vol. 12, 367-77
- **Mandel.** *Plant Molecular Biology,* 1995, vol. 29, 995-1004
- **John.** *Proc. Natl. Acad. Sci. USA,* 1997, vol. 89, 5769-5773
- **DeLisle.** *Int. Rev. Cytol.,* 1990, vol. 123, 39-60
- **Busk.** *Plant J.,* 1997, vol. 11, 1285 1295
- **Guerrero.** *Mol. Gen. Genet.,* 1990, vol. 224, 161 168
- **Blume.** *Plant J.,* 1997, vol. 12, 731 746
- **Ficker.** *Plant Mol. Biol.,* 1997, vol. 35, 425 431
- **Reiser.** *Cell,* 1995, vol. 83, 735-742
- **Liu.** *Plant Physiol.,* 1997, vol. 115, 397-407
- **Chen.** *Plant J.,* 1996, vol. 10, 955-966
- **Streit.** *Mol. Plant Microbe Interact.,* 1997, vol. 10, 933-937
- **Sheen.** *Science,* 1996, vol. 274, 1900-1902
- **De Veylder.** *Plant Cell Physiol.,* 1997, vol. 38, 568-577
- **Masgrau.** *Plant J.,* 1997, vol. 11, 465-473
- **Stange.** *Plant J.,* 1997, vol. 11, 1315-1324
- **Covey.** *Proc. Natl. Acad. Sci. USA,* 1990, vol. 87, 1633-1637
- **Angell.** *EMBO J.,* 1997, vol. 16, 3675-3684

- **Casper.** *Gene,* 1996, vol. 173, 69-73
- **Hillman.** *Virology,* 1989, vol. 169, 42-50
- **Dolja.** *Virology,* 1997, vol. 234, 243-252
- **Morinaga.** *Microbiol Immunol.,* 1993, vol. 37, 471-476
- **Cecchini.** *Mol. Plant Microbe Interact.,* 1997, vol. 10, 1094-1101
- **Rubin.** *Mol. Cell. Biol.,* 1997, vol. 17, 6294-6302
- **Kunze.** *Curr. Top. Microbiol. Immunol.,* 1996, vol. 204, 161-194
- **Schlappi.** *Plant Mol. Biol.,* 1996, vol. 32, 717-725
- **Weising.** *Ann. Rev. Genet,* 1988, vol. 22, 421-477
- **Davis, L. ; Dibner, M. ; Battey, I.** *Basic Methods in Molecular Biology,* 1986
- PCR PROTOCOLS, A GUIDE TO METHODS AND APPLICATIONS. Academic Press, 1990
- PCR STRATEGIES. Academic Press, Inc, 1995
- **Wu.** *Genomics,* 1989, vol. 4, 560
- **Landegren.** *Science,* 1988, vol. 241, 1077
- **Barringer.** *Gene,* 1990, vol. 89, 117
- **Kwoh.** *Proc. Natl. Acad. Sci. USA,* 1989, vol. 86, 1173
- **Guatelli.** *Proc. Natl. Acad. Sci. USA,* 1990, vol. 87, 1874
- **Smith.** *J. Clin. Microbiol.,* 1997, vol. 35, 1477-1491
- **Burg.** *Mol. Cell. Probes,* 1996, vol. 10, 257-271
- **Berger.** *Methods Enzymol.,* 1987, vol. 152, 307-316
- **Sooknanan.** *Biotechnology,* 1995, vol. 13, 563-564
- **Stryer, Lubert.** Biochemistry. W. H Freeman & Co,
- **Pearson ; Lipman.** *Proc. Natl. Acad. Sci. USA,* 1988, vol. 85 (8), 2444-2448
- **Altschul et al.** *J. Mol. Biol.,* 1990, vol. 215 (3), 403-410
- **Thompson et al.** *Nucleic Acids Res.,* 1994, vol. 22 (2), 4673-4680
- **Higgins et al.** *Methods Enzymol.,* 1996, vol. 266, 383-402
- **Altschul et al.** *Nature Genetics,* 1993, vol. 3, 266-272
- **Smith ; Waterman.** *Adv. Appl. Math.,* 1981, vol. 2, 482
- **Needleman ; Wunsch.** *J. Mol. Biol.,* 1970, vol. 48, 443
- **Lipman.** *Proc. Nat'l. Acad. Sci. USA,* 1988, vol. 85, 2444
- **Altschul.** *Nuc. Acids Res.,* 1977, vol. 25, 3389-3402
- **Altschul.** *J. Mol. Biol.,* 1990, vol. 215, 403-410
- **Henikoff ; Henikoff.** *Proc. Natl. Acad. Sci. USA,* 1989, vol. 89, 10915
- **Karlin ; Altschul.** *Proc. Natl. Acad. Sci. USA,* 1993, vol. 90, 5873
- **Gonnet et al.** *Science,* 1992, vol. 256, 1443-1445
- **Henikoffand Henikoff.** *Proteins,* 1993, vol. 17, 49-61
- Matrices for Detecting Distance Relationships: Atlas of Protein Sequence and Structure. National Biomedical Research Foundation, 1978
- **Altschul et al.** *J. Mol. Biol.,* 1990, vol. 215, 403
- **Pearson ; Lipman.** *Proc. Natl. Acad. Sci. USA,* 1988, vol. 85, 2444
- **Brutlag et al.** *Comp. App. Biosci.,* 1990, vol. 6, 237-245
- **Ho.** *Methods Enzymol.,* 2000, vol. 314, 168-183
- **Smith.** *Eur. J. Pharm. Sci.,* vol. 11, 191-198
- **Mata.** *Toxicol Appl Pharmacol,* 1997, vol. 144, 189-197
- Antisense Therapeutics. Humana Press, 1996
- **Gold.** *J. of Biol. Chem.,* 1995, vol. 270, 13581-13584
- **Rossi.** *Aids Research and Human Retroviruses,* 1992, vol. 8, 183
- **Hampel.** *Biochemistry,* 1989, vol. 28, 4929
- **Hampel.** *Nuc. Acids Res.,* 1990, vol. 18, 299
- **Perrotta.** *Biochemistry,* 1992, vol. 31, 16
- **Guerrier-Takada.** *Cell,* 1983, vol. 35, 849
- **Shuey.** *Drug Discov. Today,* 2002, vol. 7, 1040-1046
- **Rice.** *Proc. Natl. Acad. Sci. USA,* 1992, vol. 89, 5467-5471
- **Crameri.** *Biotechniques,* 1995, vol. 18, 194-196
- **Stemmer.** Molecular breeding of viruses for targeting and other clinical properties. *Tumor Targeting,* 1999, vol. 4, 1-4
- **Ness.** *Nature Biotechnology,* 1999, vol. 17, 893-896
- **Chang.** Evolution of a cytokine using DNA family shuffling. *Nature Biotechnology,* 1999, vol. 17, 793-797
- **Minshull.** Protein evolution by molecular breeding. *Current Opinion in Chemical Biology,* 1999, vol. 3, 284-290
- **Christians.** Directed evolution of thymidine kinase for AZT phosphorylation using DNA family shuffling. *Nature Biotechnology,* 1999, vol. 17, 259-264
- **Crameri.** DNA shuffling of a family of genes from diverse species accelerates directed evolution. *Nature,* 1998, vol. 391, 288-291
- **Crameri.** Molecular evolution of an arsenate detoxification pathway by DNA shuffling. *Nature Biotechnology,* 1997, vol. 15, 436-438

- **Zhang.** Directed evolution of an effective fucosidase from a galactosidase by DNA shuffling and screening. *Proc. Natl. Acad. Sci. USA,* 1997, vol. 94, 4504-4509
- **Patten et al.** Applications of DNA Shuffling to Pharmaceuticals and Vaccines. *Current Opinion in Biotechnology,* 1997, vol. 8, 724-733
- **Crameri et al.** Construction and evolution of antibody-phage libraries by DNA shuffling. *Nature Medicine,* 1996, vol. 2, 100-103
- **Gates et al.** Affinity selective isolation of ligands from peptide libraries through display on a lac repressor 'headpiece dimer. *Journal of Molecular Biology,* 1996, vol. 255, 373-386
- Sexual PCR and Assembly PCR. **Stemmer.** The Encyclopedia of Molecular Biology. VCH Publishers, 1996, 447-457
- **Crameri ; Stemmer.** Combinatorial multiple cassette mutagenesis creates all the permutations of mutant and wildtype cassettes. *BioTechniques,* 1995, vol. 18, 194-195
- **Stemmer et al.** Single-step assembly of a gene and entire plasmid form large numbers of oligodeoxyribonucleotides. *Gene,* 1995, vol. 164, 49-53
- **Stemmer.** The Evolution of Molecular Computation. *Science,* 1995, vol. 270, 1510
- **Stemmer.** Searching Sequence Space. *Bio/Technology,* 1995, vol. 13, 549-553
- **Stemmer.** Rapid evolution of a protein in vitro by DNA shuffling. *Nature,* 1994, vol. 370, 389-391
- **Stemmer.** DNA shuffling by random fragmentation and reassembly: In vitro recombination for molecular evolution. *Proc. Natl. Acad. Sci. USA,* 1994, vol. 91, 10747-10751
- **Ling et al.** Approaches to DNA mutagenesis: an overview. *Anal Biochem.,* 1997, vol. 254 (2), 157-178
- **Dale et al.** Oligonucleotide-directed random mutagenesis using the phosphorothioate method. *Methods Mol. Biol.,* 1996, vol. 57, 369-374
- **Smith.** In vitro mutagenesis. *Ann. Rev. Genet,* 1985, vol. 19, 423-462
- **Botstein ; Shortle.** Strategies and applications of in vitro mutagenesis. *Science,* 1985, vol. 229, 1193-1201
- **Carter.** Site-directed mutagenesis. *Biochem. J.,* 1986, vol. 237, 1-7
- The efficiency of oligonucleotide directed mutagenesis. **Kunkel.** Nucleic Acids & Molecular Biology. Springer Verlag, 1987
- **Kunkel.** Rapid and efficient site-specific mutagenesis without phenotypic selection. *Proc. Natl. Acad. Sci. USA,* 1985, vol. 82, 488-492
- **Kunkel et al.** Rapid and efficient site-specific mutagenesis without phenotypic selection. *Methods in Enzymol.,* 1987, vol. 154, 367-382
- **Bass et al.** Mutant Trp repressors with new DNA-binding specificities. *Science,* 1988, vol. 242, 240-245
- *Methods in Enzymol.,* 1983, vol. 100, 468-500
- *Methods in Enzymol.,* 1987, vol. 154, 329-350
- **Zoller ; Smith.** Oligonucleotide-directed mutagenesis using M13-derived vectors: an efficient and general procedure for the production of point mutations in any DNA fragment. *Nucleic Acids Res.,* 1982, vol. 10, 6487-6500
- **Zoller ; Smith.** Oligonucleotide-directed mutagenesis of DNA fragments cloned into M13 vectors. *Methods in Enzymol.,* 1983, vol. 100, 468-500
- **Zoller ; Smith.** Oligonucleotide-directed mutagenesis: a simple method using two oligonucleotide primers and a single-stranded DNA template. *Methods in Enzymol.,* 1987, vol. 154, 329-350
- **Taylor et al.** The use of phosphorothioate-modified DNA in restriction enzyme reactions to prepare nicked DNA. *Nucl. Acids Res.,* 1985, vol. 13, 8749-8764
- **Taylor et al.** The rapid generation of oligonucleotide-directed mutations at high frequency using phosphorothioate-modified DNA. *Nucl. Acids Res.,* 1985, vol. 13, 8765-8787
- **Nakamaye.** Inhibition of restriction endonuclease Nci I cleavage by phosphorothioate groups and its application to oligonucleotide-directed mutagenesis. *Nucl. Acids Res.,* 1986, vol. 14, 9679-9698
- **Sayers et al.** Y-T Exonucleases in phosphorothioate-based oligonucleotide-directed mutagenesis. *Nucl. Acids Res.,* 1988, vol. 16, 791-802
- **Sayers et al.** Strand specific cleavage ofphosphorothioate-containing DNA by reaction with restriction endonucleases in the presence of ethidium bromide. *Nucl. Acids Res.,* 1988, vol. 16, 803-814
- **Kramer et al.** The gapped duplex DNA approach to oligonucleotide-directed mutation construction. *Nucl. Acids Res.,* 1984, vol. 12, 9441-9456
- **Kramer ; Fritz.** Oligonucleotide-directed construction of mutations via gapped duplex DNA. *Methods in Enzymol.,* 1987, vol. 154, 350-367
- **Kramer et al.** Improved enzymatic in vitro reactions in the gapped duplex DNA approach to oligonucleotide-directed construction of mutations. *Nucl. Acids Res.,* 1988, vol. 16, 7207
- **Fritz et al.** Oligonucleotide-directed construction of mutations: a gapped duplex DNA procedure without enzymatic reactions in vitro. *Nucl. Acids Res.,* 1988, vol. 16, 6987-6999
- **Kramer.** Point Mismatch Repair. *Cell,* 1984, vol. 38, 879-887
- **Carter et al.** Improved oligonucleotide site-directed mutagenesis using M13 vectors. *Nucl. Acids Res.,* 1985, vol. 13, 4431-4443
- **Carter.** Improved oligonucleotide-directed mutagenesis using M13 vectors. *Methods in Enzymol.,* 1987, vol. 154, 382-403

- **Eghtedarzadeh.** Use of oligonucleotides to generate large deletions. *Nucl. Acids Res.,* 1986, vol. 14, 5115
- **Wells et al.** Importance of hydrogen-bond formation in stabilizing the transition state of subtilisin. *Phil. Trans. R. Soc. Lond. A,* 1986, vol. 317, 415-423
- **Nambiar et al.** Total synthesis and cloning of a gene coding for the ribonuclease S protein. *Science,* 1984, vol. 223, 1299-1301
- **Sakamar ; Khorana.** Total synthesis and expression of a gene for the a-subunit of bovine rod outer segment guanine nucleotide-binding protein (transducin). *Nucl. Acids Res.,* 1988, vol. 14, 6361-6372
- **Wells et al.** Cassette mutagenesis: an efficient method for generation of multiple mutations at defined sites. *Gene,* 1985, 315-323
- **Grundstrom et al.** Oligonucleotide-directed mutagenesis by microscale 'shot-gun' gene synthesis. *Nucl. Acids Res.,* 1985, vol. 13, 3305-3316
- **Arnold.** Protein engineering for unusual environments. *Current Opinion in Biotechnology,* 1993, vol. 4, 450-455
- Oligonucleotide-directed double-strand break repair in plasmids of Escherichia coli: a method for site-specific mutagenesis. *Proc. Natl. Acad. Sci. USA,* vol. 83, 7177-7181
- *Methods in Enzymology,* vol. 154
- **Leung, D.W. et al.** *Technique,* 1989, vol. 1, 11-15
- **Caldwell, R. C. ; Joyce G.F.** *PCR Methods Applic.,* 1992, vol. 2, 28-33
- **Reidhaar-Olson.** *Science,* 1988, vol. 241, 53-57
- **Stemmer.** *Proc. Natl. Acad. Sci. USA,* 1994, vol. 91, 10747-10751
- **Arkin.** *Proc. Natl. Acad. Sci. USA,* 1992, vol. 89, 7811-7815
- **Delegrave.** *Biotechnology Res.,* 1993, vol. 11, 1548-1552
- **Arnold.** *Current Opinion in Biotechnology,* 1993, vol. 4, 450-455
- **Baca.** *Int. J. Parasitol.,* 2000, vol. 30, 113-118
- **Hale.** *Protein Expr. Purif.,* 1998, vol. 12, 185-188
- **Narum.** *Infect. Immun.,* 2001, vol. 69, 7250-7253
- **Outchkourov.** *Protein Expr. Purif.,* 2002, vol. 24, 18-24
- **Feng.** *Biochemistry,* 2000, vol. 39, 15399-15409
- **Humphreys.** *Protein Expr. Purif.,* 2000, vol. 20, 252-264
- **Pollock.** *J. Immunol. Methods,* 1999, vol. 231, 147-157
- **Baguisi.** *Nat. Biotechnol.,* 1999, vol. 17, 456-461
- **Strepp.** *Proc Natl. Acad. Sci. USA,* 1998, vol. 95, 4368-4373
- **Miao.** *Plant J,* 1995, vol. 7, 359-365
- **Christou.** *Plant Mol. Biol.,* 1997, vol. 35, 197-203
- **Pawlowski.** *Mol. Biotechnol.,* 1996, vol. 6, 17-30
- **Klein.** *Nature,* 1987, vol. 327, 70-73
- **Takumi.** *Genes Genet. Syst.,* 1997, vol. 72, 63-69
- **Rouwendal.** *Plant Mol. Biol.,* 1997, vol. 33, 989-999
- **Porta.** Use of viral replicons for the expression of genes in plants. *Mol. Biotechnol.,* 1996, vol. 5, 209-221
- **Horsch.** *Science,* 1984, vol. 233, 496-498
- **Fraley.** *Proc. Natl. Acad. Sci. USA,* 1983, vol. 80, 4803
- Gene Transfer to Plants,. Springer-Verlag, 1995
- **Hiei.** *Plant Mol. Biol.,* 1997, vol. 35, 205-218
- **Horsch.** *Science,* 1984, vol. 233, 496
- **Fraley.** *Proc. Natl. Acad. Sci USA,* 1983, vol. 80, 4803
- **Park.** *Plant Mol. Biol.,* 1996, vol. 32, 1135-1148
- Protoplasts Isolation and Culture. **Evans et al.** Handbook of Plant Cell Culture. MacMillilan Publishing Company, 1983, 124-176
- **Binding.** Regeneration of Plants, Plant Protoplasts. CRC Press, 1985, 21-73
- **Klee.** *Ann. Rev. of Plant Phys.,* 1987, vol. 38, 467-486
- **Palmgren.** *Trends Genet.,* 1997, vol. 13, 348
- **Chong.** *Transgenic Res.,* 1997, vol. 6, 289-296
- **Jimenez.** *Biotechnol. Prog.,* 2002, vol. 18, 635-640
- **Van de Ven.** *Crit. Rev. Oncog.,* 1993, vol. 4 (2), 115-136
- **A. Bateman ; E. Birney ; L. Cerruti ; R. Durbin ; L. Etwiller ; S.R. Eddy ; S. Griffiths-Jones ; K.L. Howe ; M. Marshall ; E.L.L. Sonnhammer.** *Nucleic Acids Research,* 2002, vol. 30 (1), 276-280
- **Caruthers.** *Nucleic Acids Res. Symp. Ser.,* 1980, 215-223
- **Horn.** *Nucleic Acids Res. Symp. Ser,* 1980, 225-232
- **Banga, A.K.** Therapeutic Peptides and Proteins, Formulation, Processing and Delivery Systems. Technomic Publishing Co, 1995
- **Roberge.** *Science,* 1995, vol. 269, 202
- **Merrifield.** *Methods Enzymol.,* 1997, vol. 289, 3-13
- **Spatola.** *Chemistry and Biochemistry of Amino Acids, Peptides and Proteins,* 1983, vol. 7, 267-357

- Peptide Backbone Modifications. Marcell Dekker
- **Creighton, T.E.** Proteins - Structure and Molecular Properties. W.H. Freeman and Company, 1993
- Posttranslational Covalent Modification of Proteins. Academic Press, 1983, 1-12
- **Merrifield, R. B.** *J. Am. Chem. Soc.,* 1963, vol. 85, 2149-2154
- **Stewart, J. M. ; Young, J. D.** Solid Phase Peptide Synthesis. Pierce Chemical Co, 11-12
- **H. M. Geysen et al.** *Proc. Natl. Acad. Sci., USA,* 1984, vol. 81, 3998
- **Nielsen et al.** Identification of prokaryotic and eukaryotic signal peptides and prediction of their cleavage sites. *Protein Engineering,* 1997, vol. 10 (1), 1-6
- **Jimenez.** *Lipids,* 2001, vol. 36, 1169-1174
- **Pinsirodom.** *J. Agric. Food Chem.,* 2000, vol. 48, 155-160
- **Kauffmann.** Conversion of Bacillus thermocatenulatus lipase into an efficient phospholipase with increased activity towards long-chain fatty acyl substrates by directed evolution and rational design. *Protein Engineering,* 2001, vol. 14, 919-928
- **Ibrahim.** Evidence implicating phospholipase as a virulence factor of Candida albicans. *Infect. Immun.,* 1995, vol. 63, 1993-1998
- **Goode.** Evidence for cell surface and internal phospholipase activity in ascidian eggs. *Develop. Growth Differ.,* 1997, vol. 39, 655-660
- **Diaz.** Direct fluorescence-based lipase activity assay. *BioTechniques,* 1999, vol. 27, 696-700
- **Reynolds.** *Methods in Enzymol.,* 1991, vol. 197, 3-13
- **Taguchi.** Phospholipase from Clostridium novyi type A.I. *Biochim. Biophys. Acta,* 1975, vol. 409, 75-85
- **Taguchi.** *Biochim. Biophys. Acta,* 1975, vol. 409, 75-85
- **Korbsrisate.** *J. Clin. Microbiol,* 1999, vol. 37, 3742-3745
- **Berka.** *Infect. Immun.,* 1981, vol. 34, 1071-1074
- **Titball.** *Microbiol. Rev.,* 1993, vol. 57, 347-366
- **Johnston.** *Curr. Biol.,* 1998, vol. 8, R171-R174
- **Schummer.** *Biotechniques,* 1997, vol. 23, 1087-1092
- **Solinas-Toldo.** *Genes, Chromosomes & Cancer,* 1997, vol. 20, 399-407
- **Bowtell.** *Nature Genetics,* 1999, vol. 21, 25-32
- Fundamental Immunology. Raven Press, 1993
- **Wilson.** *J. Immunol. Methods,* 1994, vol. 175, 267-273
- **Yarmush.** *J. Biochem. Biophys. Methods,* 1992, vol. 25, 85-97
- **Ward et al.** *Nature,* 1989, vol. 341, 544-546
- **Coligan.** CURRENT PROTOCOLS IN IMMUNOLOGY. Wiley/Greene, 1991
- BASIC AND CLINICAL IMMUNOLOGY. Lange Medical Publications
- **Goding.** MONOCLONAL ANTIBODIES: PRINCIPLES AND PRACTICE. Academic Press, 1986
- **Kohler.** *Nature,* 1975, vol. 256, 495
- **Harlow.** ANTIBODIES, A LABORATORY MANUAL. Cold Spring Harbor Publications, 1988
- **Hoogenboom.** *Trends Biotechnol.,* 1997, vol. 15, 62-70
- **Katz.** *Annu. Rev. Biophys. Biomol. Struct.,* 1997, vol. 26, 27-45
- **Cole.** Monoclonal Antibodies and Cancer Therapy. Alan R. Liss, Inc, 1985, 77-96
- **Kreuzer.** *Br. J. Haematol.,* 2001, vol. 114, 313-318
- **Xia.** *Transplantation,* 2001, vol. 72, 907-914
- **Pandey.** *Neuropsychopharmacology,* 2002, vol. 26, 216-228
- **Brindisi.** *J. of Food Sci.,* 2001, vol. 66, 1100-1107
- **Kasai.** *J. Agric. Food Chem.,* 2003, vol. 51, 6217-6222
- **Rosenthal.** *Enzyme and Microb. Tech.,* 2001, vol. 28, 499-509
- **Ouhida.** *J. Agric. Food Chem.,* 2002, vol. 50, 1933-1938
- **Sosulski.** *Proc. Can. Inst. Food Sci. Technol.,* 1990, vol. 3, 656
- **McGlone.** *J. of Food Sci.,* 1986, vol. 51, 695-697
- **Buenrostro.** *Biotech. Letters,* 1986, vol. 8 (7), 505-506
- **Montedoro.** *Acta Vitamin. Enzymol.,* 1976, vol. 30, 13
- Fats and Oils Handbook. **Bockisch, M.** The extraction of Vegetable Oils. AOCS Press, 345-445
- **Dijkstra, Albert J. et al.** *Oleagineux, Corps Gras, Lipides,* 1998, vol. 5 (5), 367-370
- **Dijkstra et al.** *Res. Dev. Dep., N.V. Vandemoortele Coord. Cent., Izegem, Belg. JAOCS, J. Am. Oil Chem. Soc.,* 1989, vol. 66, 1002-1009
- **Hvolby et al.** *J. Amer. Oil Chem. Soc.,* 1971, vol. 48, 503-509
- **Buchold et al.** *Fett Wissenschaft Technologie,* 1993, vol. 95 (8), 300-304
- *Oleagineux, Corps Gras, Lipides,* 1997, vol. 4 (1), 55-57
- **Cleenewerck et al.** *Belg. Fett Wissenschaft Technologie,* 1992, vol. 94, 317-22
- **Clausen, Kim ; Nielsen, M.** *Novozymes A/S, Den. Dansk Kemi,* 2002, vol. 83 (2), 24-27

- **Nilsson-Johansson et al.** *Fats Oils Div., Alfa-Laval Food Eng. AB, Tumba, Swed. Fett Wissenschaft Technologie,* 1988, vol. 90 (11), 447-51
- Proceedings of the World Conference on Oilseed Processing Utilization. 12 November 2000
- **Jerzewska et al.** *Inst. Przemyslu Miesnego i Tluszczowego, Warsaw, Pol.,* 2001, vol. 36 (3/4), 97-110
- Technology and Solvents for Extracting Oilseeds and Nonpetroleum Oils. AOCS, 1997
- **Hoondal.** *Applied Microbiology and Biotechnology,* 2002, vol. 59, 409-418
- **Yamamura.** *Biochem. Biophys. Res. Com.,* 2002, vol. 294, 1138-1143
- **Gupta.** *Appl. Microbiol. Biotechnol.,* 2002, vol. 59, 15-32
- **Li, S.** Bacteriolytic Activity and Specificity ofAchromobacter b-Lytic Protease. *J. Biochem.,* 1998, vol. 124, 332-339
- *J. Gen Microbiol,* 1991, vol. 137 (5), 1145-1153
- *Science,* 2001, vol. 249, 2170-2172
- **Pommer, K.** Investigating the impact of enzymes on pet food palatability. *Petfood Industry,* May 2002, 10-11
- *Cereal Chem.,* vol. 78 (4), 405-411
- **Arisawa, A.** Streptomyces Serine Protease (DHP-A) as a New Biocatalyst Capable of Forming Chiral Intermediates of 1,4-Diohydropyridine Calcium Antagonists. *Appl Environ Mircrobiol,* June 2002, vol. 68 (6), 2716-2725
- **Haring, D.** Semisynthetic Enzymes in Asymmetric Synthesis:Enantioselective Reduction of Racemic Hydroperoxides Catalyzed by Seleno-Subtilisin. *J. Org. Chem.,* 1999, vol. 64, 832-835
- **Jaeger.** *FEMS Microbiol. Rev.,* 1994, vol. 15, 29-63
- **Ader.** *Methods Enzymol.,* 1997, vol. 286, 351-386
- **Vorderwülbecke.** *Enzyme Microb. Technol.,* 1992, vol. 14, 631-639
- **Renard.** *Lipids,* 1987, vol. 22, 539-541
- **Briand.** *Eur. J. Biochem.,* 1995, vol. 228, 169-175
- **Rogalska.** *Chirality,* 1993, vol. 5, 24-30
- **S. Wongsakul et al.** *Eur. J. Lipid Sci. Technol.,* 2003, vol. 105, 68-73
- **Kurvinen.** *Lipids,* 2001, vol. 36, 1377-1382
- *Chem. Communication,* 2002, 1428-1429
- *J. Am. Oil Chem. Soc.,* 1992, vol. 69, 955-960
- **Consoli.** *Electrophoresis,* 2001, vol. 22, 2983-2989

SEQUENCE LISTING

<110> Verenium Corporation

<120> Esterases and related nucelic acids and methods

<130> P/61091.EP02

<140> Divisional of EP07763087.9

<141> 2007-02-02

<150> 60/764,486

<151> 2006-02-02

<160> 166

<170> PatentIn 3.4

<210> 1
<211> 828
<212> DNA
<213> Unknown

<220>
<223> Obtained from environmental sample

<400> 1
atgagaaaag aaaaaccgga atatggcatt gtgctgagtg gtggaggcgc ccgcggaatt 60

gcccacattg gtgtgctagc tgcactggaa aagcacggga tcagtccggg tgtcgtttcc 120

ggctctagca tgggcgccat cgtaggcgca ttatacgccg ggggggtgag cactgaagaa 180

atgcttgaaa tcgcccggaa ccggaagctt aacgacctct tcgaatggtc gttcaaaaag 240

catggcggta tgctttcatt aaagatgctc atgcaggttc ttgaaacaca tatccctgaa 300

aattctttcg aatcgttaaa gaaaaagtta ttcattaccg tatccaatat ttcatccggc 360

caggaggagg tcatttcaga aggtaagttg taccaggcag tggtagcatc ggcttctatc 420

ccgatcattt ttgagcctca gctgatcaat ggccagactt atgtcgatgg cggcctgttc 480

aacgatctgc cggttgatcc actgatagga aagtgcagga gtatcatcgc ttcacatgtg 540

aactacaatg gtccgaatcc tgatctgacc agtattcgcg ccatcgcaga acgggtttac 600

cgcctggcta tttatcaaca tgtacataag aatttcagca aatgtgatta tattattgac 660

ccgcccgcgt tgagggatca tagcattttt gatttcaagc atatcgaccg gttgtttgag 720

atcgggtttg aagcggctga gatccttatt acccagatgc aggaaccatc agttgaaaaa

780

ccgcttataa tccgggaaat taagtccact aaaaagagat taaaatga
828

<210> 2
<211> 275
<212> PRT
<213> Unknown

<220>
<223> Obtained from environmental sample

<220>
<221> DOMAIN
<222> (10)...(169)
<223> Patatin-like phospholipase

<220>
<221> SITE
<222> (117)...(120)
<223> N-glycosylation site. Prosite id = PS00001

<220>
<221> SITE
<222> (214)...(217)
<223> N-glycosylation site. Prosite id = PS00001

<400> 2
```
Met Arg Lys Glu Lys Pro Glu Tyr Gly Ile Val Leu Ser Gly Gly Gly
1               5                   10                  15

Ala Arg Gly Ile Ala His Ile Gly Val Leu Ala Ala Leu Glu Lys His
            20                  25                  30

Gly Ile Ser Pro Gly Val Val Ser Gly Ser Ser Met Gly Ala Ile Val
        35                  40                  45

Gly Ala Leu Tyr Ala Gly Gly Val Ser Thr Glu Glu Met Leu Glu Ile
    50                  55                  60

Ala Arg Asn Arg Lys Leu Asn Asp Leu Phe Glu Trp Ser Phe Lys Lys
65                  70                  75                  80

His Gly Gly Met Leu Ser Leu Lys Met Leu Met Gln Val Leu Glu Thr
                85                  90                  95

His Ile Pro Glu Asn Ser Phe Glu Ser Leu Lys Lys Lys Leu Phe Ile
            100                 105                 110

Thr Val Ser Asn Ile Ser Ser Gly Gln Glu Glu Val Ile Ser Glu Gly
        115                 120                 125

Lys Leu Tyr Gln Ala Val Val Ala Ser Ala Ser Ile Pro Ile Ile Phe
    130                 135                 140

Glu Pro Gln Leu Ile Asn Gly Gln Thr Tyr Val Asp Gly Gly Leu Phe
```

145 150 155 160

Asn Asp Leu Pro Val Asp Pro Leu Ile Gly Lys Cys Arg Ser Ile Ile
165 170 175

Ala Ser His Val Asn Tyr Asn Gly Pro Asn Pro Asp Leu Thr Ser Ile
180 185 190

Arg Ala Ile Ala Glu Arg Val Tyr Arg Leu Ala Ile Tyr Gln His Val
195 200 205

His Lys Asn Phe Ser Lys Cys Asp Tyr Ile Ile Asp Pro Pro Ala Leu
210 215 220

Arg Asp His Ser Ile Phe Asp Phe Lys His Ile Asp Arg Leu Phe Glu
225 230 235 240

Ile Gly Phe Glu Ala Ala Glu Ile Leu Ile Thr Gln Met Gln Glu Pro
245 250 255

Ser Val Glu Lys Pro Leu Ile Ile Arg Glu Ile Lys Ser Thr Lys Lys
260 265 270

Arg Leu Lys
275

<210> 3
<211> 1665
<212> DNA
<213> Unknown

<220>
<223> Obtained from environmental sample

<400> 3
atgaagaaaa agatttttac catcgcgggg gttttgattc tcgtgctagt ggcatggcaa
60

ttgtggccgg agataaagac attaccagcg ggcagcgctc ccacagaagc gattcaaacg
120

accaccggcg cgctgcgcgg cttcacccaa aacggattgg atacctacct ggggattccc
180

tacgcggagc cgccggtcgg cagtctgcgt ttcaaatcgc cggtggcgcg cgaaccctgg
240

cagggcacgt tcgaggccta tgaatttggc gcgttctgcc cacaggccta tgacccggtg
300

gtgattgatg atccgcacga agacttgaac aacgaagact gcctgtacct gaacatctgg
360

aagccctccg ccgcgcagga gaaacgcgcc gtcatggtct acatccacgg cggcggattc
420

gtcggcggct cgagcaagga agacctctac aacggcggcg tcatcgccag ggatggcgac

480

gtggtcgtgg tcaccgtgaa ctatcgcgtt ggcattctgg gcttcttcga cttttccgtc
540

atcggcgggt cggagtacac cggcagcgcc gacgcgggga tcgaggatca actcctggcg
600

ttgcgctggg tcaaggacaa catcgcggcg tttggcggcg accccgagaa catcaccgtg
660

ttcggcgagt cggctggagg tgcgagcgtc ctggccctgt tgggcataga ccatccccag
720

gaattgttca agcgcgccat cgtcatgagc gggtcacctt tgcataccgc cgaaaacagc
780

cgggatattg ccaacctgct caaaaccgaa accgggataa cttcgtcgac catctggctc
840

tgggcgccaa cccaggctgt gatgtacatt caggatcagg tgttgacagc cgtcggctcg
900

cctctttcgg atctcatttt tgctcccacc tacggaagcg gctacgtggt caagcaaagt
960

ccgctcgaag ccatcgcctc cggcaacacc aaaggcattg acctgatgat cggcaacatg
1020

gccgacgagt tgagttattg gtcgttctat gatactccgg atagtcacat ctgcgaacag
1080

actctcaagg ataacctctt caccatgatc aatccggaca tcgagccgga actgaaaaaa
1140

ttgtacgatc tgtatgtcaa cgacccggag cgggtctgga aagaagaagg cgatatcatc
1200

ttggccatgg gcgatgacta tgccttccgc gtcgacgcgt tggacatagc gtcagcacaa
1260

gctaccgttg ccaaaaccta ctactaccgc ttcaactatc cggtcaacct gccggagcag
1320

ccctgtcagg atcaccgctc gcctcatgga gcggaactgc ctttcgtgtt cggcagtgtc
1380

aacacacaga ccgggttcga cttcatcggc aagccgcgcg acgagcagga taatgccgct
1440

cgtaaccgcc tcatgcaaca ggcgatcctt gcatggacca acttcgccaa gaccggcgat
1500

cccaatggcg gcgacctgcc cgtctggccg ctgttcgacg ccgggaccca gccgaccatg
1560

gtcttcgccg cggacgtcca cgttgaggac gcgcccttcc tggccgagta caaagccatg
1620

tccgacttcc tgaagatctt caacgtcttc gacgcgttga aatag
1665

<210> 4
<211> 554
<212> PRT
<213> Unknown

<220>
<223> Obtained from environmental sample

<220>
<221> DOMAIN
<222> (10)...(537)
<223> Carboxylesterase

<220>
<221> DOMAIN
<222> (132)...(348)
<223> alpha/beta hydrolase fold

<220>
<221> SITE
<222> (113)...(123)
<223> Carboxylesterases type-B signature 2. Prosite id = PS00941

<220>
<221> SITE
<222> (214)...(229)
<223> Carboxylesterases type-B serine active site. Prosite id = PS00122

<220>
<221> SITE
<222> (220)...(223)
<223> N-glycosylation site. Prosite id = PS00001

<400> 4
```
Met Lys Lys Lys Ile Phe Thr Ile Ala Gly Val Leu Ile Leu Val Leu
1               5                   10                  15

Val Ala Trp Gln Leu Trp Pro Glu Ile Lys Thr Leu Pro Ala Gly Ser
            20                  25                  30

Ala Pro Thr Glu Ala Ile Gln Thr Thr Thr Gly Ala Leu Arg Gly Phe
        35                  40                  45

Thr Gln Asn Gly Leu Asp Thr Tyr Leu Gly Ile Pro Tyr Ala Glu Pro
    50                  55                  60

Pro Val Gly Ser Leu Arg Phe Lys Ser Pro Val Ala Arg Glu Pro Trp
65                  70                  75                  80

Gln Gly Thr Phe Glu Ala Tyr Glu Phe Gly Ala Phe Cys Pro Gln Ala
                85                  90                  95

Tyr Asp Pro Val Val Ile Asp Asp Pro His Glu Asp Leu Asn Asn Glu
            100                 105                 110

Asp Cys Leu Tyr Leu Asn Ile Trp Lys Pro Ser Ala Ala Gln Glu Lys
        115                 120                 125

Arg Ala Val Met Val Tyr Ile His Gly Gly Gly Phe Val Gly Gly Ser
    130                 135                 140
```

```
Ser Lys Glu Asp Leu Tyr Asn Gly Gly Val Ile Ala Arg Asp Gly Asp
145                 150                 155                 160

Val Val Val Val Thr Val Asn Tyr Arg Val Gly Ile Leu Gly Phe Phe
                165                 170                 175

Asp Phe Ser Val Ile Gly Gly Ser Glu Tyr Thr Gly Ser Ala Asp Ala
            180                 185                 190

Gly Ile Glu Asp Gln Leu Leu Ala Leu Arg Trp Val Lys Asp Asn Ile
        195                 200                 205

Ala Ala Phe Gly Gly Asp Pro Glu Asn Ile Thr Val Phe Gly Glu Ser
    210                 215                 220

Ala Gly Gly Ala Ser Val Leu Ala Leu Leu Gly Ile Asp His Pro Gln
225                 230                 235                 240

Glu Leu Phe Lys Arg Ala Ile Val Met Ser Gly Ser Pro Leu His Thr
                245                 250                 255

Ala Glu Asn Ser Arg Asp Ile Ala Asn Leu Leu Lys Thr Glu Thr Gly
            260                 265                 270

Ile Thr Ser Ser Thr Ile Trp Leu Trp Ala Pro Thr Gln Ala Val Met
        275                 280                 285

Tyr Ile Gln Asp Gln Val Leu Thr Ala Val Gly Ser Pro Leu Ser Asp
    290                 295                 300

Leu Ile Phe Ala Pro Thr Tyr Gly Ser Gly Tyr Val Val Lys Gln Ser
305                 310                 315                 320

Pro Leu Glu Ala Ile Ala Ser Gly Asn Thr Lys Gly Ile Asp Leu Met
                325                 330                 335

Ile Gly Asn Met Ala Asp Glu Leu Ser Tyr Trp Ser Phe Tyr Asp Thr
            340                 345                 350

Pro Asp Ser His Ile Cys Glu Gln Thr Leu Lys Asp Asn Leu Phe Thr
        355                 360                 365

Met Ile Asn Pro Asp Ile Glu Pro Glu Leu Lys Lys Leu Tyr Asp Leu
    370                 375                 380

Tyr Val Asn Asp Pro Glu Arg Val Trp Lys Glu Glu Gly Asp Ile Ile
385                 390                 395                 400

Leu Ala Met Gly Asp Asp Tyr Ala Phe Arg Val Asp Ala Leu Asp Ile
```

405 410 415

Ala Ser Ala Gln Ala Thr Val Ala Lys Thr Tyr Tyr Tyr Arg Phe Asn
420 425 430

Tyr Pro Val Asn Leu Pro Glu Gln Pro Cys Gln Asp His Arg Ser Pro
435 440 445

His Gly Ala Glu Leu Pro Phe Val Phe Gly Ser Val Asn Thr Gln Thr
450 455 460

Gly Phe Asp Phe Ile Gly Lys Pro Arg Asp Glu Gln Asp Asn Ala Ala
465 470 475 480

Arg Asn Arg Leu Met Gln Gln Ala Ile Leu Ala Trp Thr Asn Phe Ala
485 490 495

Lys Thr Gly Asp Pro Asn Gly Gly Asp Leu Pro Val Trp Pro Leu Phe
500 505 510

Asp Ala Gly Thr Gln Pro Thr Met Val Phe Ala Ala Asp Val His Val
515 520 525

Glu Asp Ala Pro Phe Leu Ala Glu Tyr Lys Ala Met Ser Asp Phe Leu
530 535 540

Lys Ile Phe Asn Val Phe Asp Ala Leu Lys
545 550

<210> 5
<211> 933
<212> DNA
<213> Alicyclobacillus acidocaldarius ATCC 27009

<400> 5
atgccgctcg atcccgtcat tcagcaggtg ctcgatcaac tcaaccgcat gcctgccccg
60

gactacaaac atctctccgc ccagcaattt cgttcccaac agtcgctgtt tcctcctgtc
120

aagaaggagc ccgtggccga ggtccgagag tttgacatgg atctgcctgg ccgcacgctc
180

aaggtgcgca tgtaccgccc ggagggcgtc gaaccgccct accccgcgct cgtgtattat
240

cacggcggcg gttgggtcgt cggagacctc gagacgcacg atcccgtctg ccgcgtcctc
300

gcgaaagacg gccgcgcggt cgtgttctcc gtcgactacc gcctggcgcc ggagcacaag
360

ttccctgccg ccgtggaaga cgcctacgac gcgcttcagt ggatcgcgga gcgcgcagcg
420

gactttcatc tcgatccagc ccgcatcgcg gtcggcggag acagcgccgg agggaatctt
480

gccgctgtga cgagcatcct tgccaaagag cgcggcgggc cggccatcgc gttccagctg
540

ctcatctacc cttccacggg gtacgatccg gctcatcctc ccgcatctat cgaagaaaat
600

gcggaaggct atctcctgac cggcggcatg atgctctggt tccgggatca atacttgaac
660

agcctggagg aactcacgca tccgtggttt tcacccgtcc tctacccgga cttgagcggc
720

ttgcctccgg cgtacatcgc gacggcgcag tacgatccgc tgcgcgacgt cggcaagctt
780

tacgcggaag cgctgaacaa ggcgggcgtc aaggtcgaga tcgagaactt cgaagatctg
840

atccacggat tcgcacagtt ttacagcctt tcgcctggcg cgacgaaggc gctcgtccgc
900

attgcggaga aacttcgaga cgcgctggcc tga
933

<210> 6
<211> 310
<212> PRT
<213> Alicyclobacillus acidocaldarius ATCC 27009

<220>
<221> DOMAIN
<222> (77)...(286)
<223> alpha/beta hydrolase fold

<400> 6
Met Pro Leu Asp Pro Val Ile Gln Gln Val Leu Asp Gln Leu Asn Arg
1 5 10 15

Met Pro Ala Pro Asp Tyr Lys His Leu Ser Ala Gln Gln Phe Arg Ser
20 25 30

Gln Gln Ser Leu Phe Pro Pro Val Lys Lys Glu Pro Val Ala Glu Val
35 40 45

Arg Glu Phe Asp Met Asp Leu Pro Gly Arg Thr Leu Lys Val Arg Met
50 55 60

Tyr Arg Pro Glu Gly Val Glu Pro Pro Tyr Pro Ala Leu Val Tyr Tyr
65 70 75 80

His Gly Gly Gly Trp Val Val Gly Asp Leu Glu Thr His Asp Pro Val
85 90 95

Cys Arg Val Leu Ala Lys Asp Gly Arg Ala Val Val Phe Ser Val Asp
100 105 110

Tyr Arg Leu Ala Pro Glu His Lys Phe Pro Ala Ala Val Glu Asp Ala
115 120 125

Tyr Asp Ala Leu Gln Trp Ile Ala Glu Arg Ala Ala Asp Phe His Leu
130 135 140

Asp Pro Ala Arg Ile Ala Val Gly Gly Asp Ser Ala Gly Gly Asn Leu
145 150 155 160

Ala Ala Val Thr Ser Ile Leu Ala Lys Glu Arg Gly Gly Pro Ala Ile
165 170 175

Ala Phe Gln Leu Leu Ile Tyr Pro Ser Thr Gly Tyr Asp Pro Ala His
180 185 190

Pro Pro Ala Ser Ile Glu Glu Asn Ala Glu Gly Tyr Leu Leu Thr Gly
195 200 205

Gly Met Met Leu Trp Phe Arg Asp Gln Tyr Leu Asn Ser Leu Glu Glu
210 215 220

Leu Thr His Pro Trp Phe Ser Pro Val Leu Tyr Pro Asp Leu Ser Gly
225 230 235 240

Leu Pro Pro Ala Tyr Ile Ala Thr Ala Gln Tyr Asp Pro Leu Arg Asp
245 250 255

Val Gly Lys Leu Tyr Ala Glu Ala Leu Asn Lys Ala Gly Val Lys Val
260 265 270

Glu Ile Glu Asn Phe Glu Asp Leu Ile His Gly Phe Ala Gln Phe Tyr
275 280 285

Ser Leu Ser Pro Gly Ala Thr Lys Ala Leu Val Arg Ile Ala Glu Lys
290 295 300

Leu Arg Asp Ala Leu Ala
305 310

<210> 7
<211> 780
<212> DNA
<213> Unknown

<220>
<223> Obtained from environmental sample

<400> 7
atgataaaaa acttcgacag agaaaattct agcttagtac tgtccggtgg tggtgctctg
60

ggtattgctc acttgggtgt actgcatgac cttgaaaaac aaaatattgt accaaatgaa
120

attgttggta caagtatggg tggtatcatt ggtgcatcta tggctatcgg gatgaaagag
180

aaagaaatac tcgaagaaat caaaaacttt tccaatgtct tcaactggat aaaattctct
240

ttttccggta attctgttgt cgataacgag aagatcgcta agatatttga tactcttttt
300

aaagacagaa agatgacaga tacggtgatc cctcttaaac tcatcgctac aaacttacat
360

aatggacata aaaaagtatt tactgcttcg gatgatgtac tgatcaaaga tgcaatactc
420

tcaacaatgg caatacccgg tgtatttgaa gaacatatta ttgatggtga aacctatggc
480

gacggttttc tttgtgaaaa ccttggtgtg aatgaggcaa cattcaatga tgttttagct
540

gtagatgtca tgggtgagaa ctcttttgaa aaagcaatgc cggacaactt ctttaaaaca
600

tcaaatgttt tagaaatgtt tgaaaaatca atgcgacttt ttatttacaa ccagacacag
660

acacatatta aaaatgcaaa taaaaatatt tatcttattg aacccgttac caaagagtat
720

aaaacatttc aatttcataa acataaagag atacgtgctt taggcttggg tttactgtga
780

<210> 8
<211> 259
<212> PRT
<213> Unknown

<220>
<223> Obtained from environmental sample

<220>
<221> DOMAIN
<222> (12)...(174)
<223> Patatin-like phospholipase

<220>
<221> SITE
<222> (9)...(12)
<223> N-glycosylation site. Prosite id = PS00001

<220>
<221> SITE
<222> (70)...(73)
<223> N-glycosylation site. Prosite id = PS00001

<220>
<221> SITE
<222> (220)...(223)
<223> N-glycosylation site. Prosite id = PS00001

<400> 8
Met Ile Lys Asn Phe Asp Arg Glu Asn Ser Ser Leu Val Leu Ser Gly
1 5 10 15

Gly Gly Ala Leu Gly Ile Ala His Leu Gly Val Leu His Asp Leu Glu
20 25 30

Lys Gln Asn Ile Val Pro Asn Glu Ile Val Gly Thr Ser Met Gly Gly
35 40 45

Ile Ile Gly Ala Ser Met Ala Ile Gly Met Lys Glu Lys Glu Ile Leu
50 55 60

Glu Glu Ile Lys Asn Phe Ser Asn Val Phe Asn Trp Ile Lys Phe Ser
65 70 75 80

Phe Ser Gly Asn Ser Val Val Asp Asn Glu Lys Ile Ala Lys Ile Phe
85 90 95

Asp Thr Leu Phe Lys Asp Arg Lys Met Thr Asp Thr Val Ile Pro Leu
100 105 110

Lys Leu Ile Ala Thr Asn Leu His Asn Gly His Lys Lys Val Phe Thr
115 120 125

Ala Ser Asp Asp Val Leu Ile Lys Asp Ala Ile Leu Ser Thr Met Ala
130 135 140

Ile Pro Gly Val Phe Glu Glu His Ile Ile Asp Gly Glu Thr Tyr Gly
145 150 155 160

Asp Gly Phe Leu Cys Glu Asn Leu Gly Val Asn Glu Ala Thr Phe Asn
165 170 175

Asp Val Leu Ala Val Asp Val Met Gly Glu Asn Ser Phe Glu Lys Ala
180 185 190

Met Pro Asp Asn Phe Phe Lys Thr Ser Asn Val Leu Glu Met Phe Glu
195 200 205

Lys Ser Met Arg Leu Phe Ile Tyr Asn Gln Thr Gln Thr His Ile Lys
210 215 220

Asn Ala Asn Lys Asn Ile Tyr Leu Ile Glu Pro Val Thr Lys Glu Tyr
225 230 235 240

Lys Thr Phe Gln Phe His Lys His Lys Glu Ile Arg Ala Leu Gly Leu
245 250 255

Gly Leu Leu

<210> 9

<211> 927
<212> DNA
<213> Archaeoglobus fulgidus-VC16

<400> 9
atgccaatcg accctgttta ctaccagctt gctgagtatt tcgacagtct gccgaagttc
60

gaccagtttt cctcggccag agagtacagg gaggcgataa atcgaatata cgaggagaga
120

aaccggcagc tgagccagca tgagagggtt gaaagagttg aggacaggac gattaagggg
180

aggaacggag acatcagagt cagagtttac cagcagaagc ccgattcccc ggttctggtt
240

tactatcacg gtggtggatt tgtgatttgc agcatcgagt cgcacgacgc cttatgcagg
300

agaattgcga gactttcaaa ctctaccgta gtctccgtgg attacaggct cgctcctgag
360

cacaagtttc ccgccgcagt ttatgattgc tacgatgcga ccaagtgggt tgctgagaac
420

gccgaggagc tgaggattga cccgtcaaaa atcttcgttg ggggggacag tgcgggaggg
480

aatcttgccg cggcggtttc aataatggcg agagacagcg gagaagattt cataaagcat
540

caaattctaa tttaccccgt tgtgaacttt gtagcccccca caccatcgct tctggagttt
600

ggagaggggc tgtggattct cgaccagaag ataatgagtt ggttctcgga gcagtacttc
660

tccagagagg aagataagtt caaccccctc gcctccgtaa tctttgcgga ccttgagaac
720

ctacctcctg cgctgatcat aaccgccgaa tacgacccgc tgagagatga aggagaagtt
780

ttcgggcaga tgctgagaag agccggtgtt gaggcgagca tcgtcagata cagaggcgtg
840

cttcacggat tcatcaatta ctatcccgtg ctgaaggctg cgagggatgc gataaaccag
900

attgccgctc ttcttgtgtt cgactag
927

<210> 10
<211> 308
<212> PRT
<213> Archaeoglobus fulgidus-VC16

<220>
<221> DOMAIN
<222> (79)...(286)
<223> alpha/beta hydrolase fold

<220>
<221> SITE
<222> (108)...(111)

<223> N-glycosylation site. Prosite id = PS00001

<400> 10

```
Met Pro Ile Asp Pro Val Tyr Tyr Gln Leu Ala Glu Tyr Phe Asp Ser
1               5                   10                  15

Leu Pro Lys Phe Asp Gln Phe Ser Ser Ala Arg Glu Tyr Arg Glu Ala
            20                  25                  30

Ile Asn Arg Ile Tyr Glu Glu Arg Asn Arg Gln Leu Ser Gln His Glu
        35                  40                  45

Arg Val Glu Arg Val Glu Asp Arg Thr Ile Lys Gly Arg Asn Gly Asp
    50                  55                  60

Ile Arg Val Arg Val Tyr Gln Gln Lys Pro Asp Ser Pro Val Leu Val
65                  70                  75                  80

Tyr Tyr His Gly Gly Gly Phe Val Ile Cys Ser Ile Glu Ser His Asp
                85                  90                  95

Ala Leu Cys Arg Arg Ile Ala Arg Leu Ser Asn Ser Thr Val Val Ser
            100                 105                 110

Val Asp Tyr Arg Leu Ala Pro Glu His Lys Phe Pro Ala Ala Val Tyr
        115                 120                 125

Asp Cys Tyr Asp Ala Thr Lys Trp Val Ala Glu Asn Ala Glu Glu Leu
    130                 135                 140

Arg Ile Asp Pro Ser Lys Ile Phe Val Gly Gly Asp Ser Ala Gly Gly
145                 150                 155                 160

Asn Leu Ala Ala Ala Val Ser Ile Met Ala Arg Asp Ser Gly Glu Asp
                165                 170                 175

Phe Ile Lys His Gln Ile Leu Ile Tyr Pro Val Val Asn Phe Val Ala
            180                 185                 190

Pro Thr Pro Ser Leu Leu Glu Phe Gly Glu Gly Leu Trp Ile Leu Asp
        195                 200                 205

Gln Lys Ile Met Ser Trp Phe Ser Glu Gln Tyr Phe Ser Arg Glu Glu
    210                 215                 220

Asp Lys Phe Asn Pro Leu Ala Ser Val Ile Phe Ala Asp Leu Glu Asn
225                 230                 235                 240

Leu Pro Pro Ala Leu Ile Ile Thr Ala Glu Tyr Asp Pro Leu Arg Asp
                245                 250                 255
```

Glu Gly Glu Val Phe Gly Gln Met Leu Arg Arg Ala Gly Val Glu Ala
260 265 270

Ser Ile Val Arg Tyr Arg Gly Val Leu His Gly Phe Ile Asn Tyr Tyr
275 280 285

Pro Val Leu Lys Ala Ala Arg Asp Ala Ile Asn Gln Ile Ala Ala Leu
290 295 300

Leu Val Phe Asp
305

<210> 11
<211> 747
<212> DNA
<213> Unknown

<220>
<223> Obtained from environmental sample

<400> 11
atgaaaatta gacaaccaca accatttaca tttgaagcag gaaaaagggc cgttttatta
60

ttgcatggat ttacaggtca ttcggcagat gtacgcatgc ttggtcgcta tttagaggaa
120

aaaggttata cctctcatgc gccaatttat cgtggccatg gtcttccacc tgagcagctc
180

atggaaacaa atccaaacat gtggtgggag gatgttgtac aagcatatga acatttacgt
240

caactcggtt atgaagaaat agcagtggcc ggcctatcgt taggtggaat gatggcatta
300

aagttagcga cagagacaaa aataaaagct gttatatcga tgtgtgttcc tatgatattt
360

gatgataaaa ataatttaac agagtctttt gagcgttttg tacgacaata taaacagttt
420

gaaaaaaagg ataaagcgac gattgatcat gaaacagaac aaataatgga acaatccgtt
480

gatttgtttg aggaaattaa agcatttaat ttatcggtta aagaagtcat tgatttgatt
540

tatgcaccaa cacttgttat acaagcacgt aacgatgagg ttattaatcc tgaaagtgca
600

caatatatat atgattcagt tgaaacagat gaaaaagaaa taaagtggta tgaagaatca
660

ggccatgtta tcacattagg tgaagaacgc gaccaattac atgaagatat ttatatgttt
720

ctcgaacagt tagaatggga acaataa
747

<210> 12

```
<211> 248
<212> PRT
<213> Unknown

<220>
<223> Obtained from environmental sample

<220>
<221> SITE
<222> (126)...(129)
<223> N-glycosylation site. Prosite id = PS00001

<220>
<221> SITE
<222> (172)...(175)
<223> N-glycosylation site. Prosite id = PS00001

<400> 12
Met Lys Ile Arg Gln Pro Gln Pro Phe Thr Phe Glu Ala Gly Lys Arg
1               5                   10                  15

Ala Val Leu Leu Leu His Gly Phe Thr Gly His Ser Ala Asp Val Arg
            20                  25                  30

Met Leu Gly Arg Tyr Leu Glu Glu Lys Gly Tyr Thr Ser His Ala Pro
        35                  40                  45

Ile Tyr Arg Gly His Gly Leu Pro Pro Glu Gln Leu Met Glu Thr Asn
    50                  55                  60

Pro Asn Met Trp Trp Glu Asp Val Val Gln Ala Tyr Glu His Leu Arg
65                  70                  75                  80

Gln Leu Gly Tyr Glu Glu Ile Ala Val Ala Gly Leu Ser Leu Gly Gly
                85                  90                  95

Met Met Ala Leu Lys Leu Ala Thr Glu Thr Lys Ile Lys Ala Val Ile
            100                 105                 110

Ser Met Cys Val Pro Met Ile Phe Asp Asp Lys Asn Asn Leu Thr Glu
        115                 120                 125

Ser Phe Glu Arg Phe Val Arg Gln Tyr Lys Gln Phe Glu Lys Lys Asp
    130                 135                 140

Lys Ala Thr Ile Asp His Glu Thr Glu Gln Ile Met Glu Gln Ser Val
145                 150                 155                 160

Asp Leu Phe Glu Glu Ile Lys Ala Phe Asn Leu Ser Val Lys Glu Val
                165                 170                 175

Ile Asp Leu Ile Tyr Ala Pro Thr Leu Val Ile Gln Ala Arg Asn Asp
            180                 185                 190
```

```
Glu Val Ile Asn Pro Glu Ser Ala Gln Tyr Ile Tyr Asp Ser Val Glu
        195                 200                 205

Thr Asp Glu Lys Glu Ile Lys Trp Tyr Glu Glu Ser Gly His Val Ile
    210                 215                 220

Thr Leu Gly Glu Glu Arg Asp Gln Leu His Glu Asp Ile Tyr Met Phe
225                 230                 235                 240

Leu Glu Gln Leu Glu Trp Glu Gln
                245
```

<210> 13
<211> 747
<212> DNA
<213> Unknown

<220>
<223> Obtained from environmental sample

<400> 13

```
atgaaaatta agttgccaga accattcaca ttcgaagcag gaaatcgagc agttttatta
60

ttacatggat ttactggcca ttcagcagat gtaagaatgc ttggtcgatt tctagaaaag
120

aaaggttata caactcatgc accaatttat agagggcatg gtcaagaacc agaagcatta
180

ttaaaatctt cacctgatga atggtgggag gatattttat cagcttataa tcatttgaaa
240

aatctaggat ataatgaaat tgctgttgct ggtctttcaa tgggtggtgc tttagcaatt
300

aaactagcta ctaagcatga aataaaaggt gtcattccaa tgtgtacacc aatgtacttt
360

gataatcaaa aacaattaac tcaagcattt ttaaattttg cacgacaatt taaaaaattt
420

gagaaaaaag atgaagaaac aattaaaaaa gaaatagatt tactacaaca aaattcagca
480

gaattattta atgagatcgg cacttttgta gaacaagtca atggtataat agatagagtt
540

gacgtaccta cattcgtagt tcaagcaagt aaagatgaaa taatcaatcc tgaaagtgcg
600

acatttattt atgaaaacat taaaaatgaa aaaaaagatt taaaatggta taaaaattca
660

actcacttga taacatttgg cgatgaaaaa gatgtcttac atgaagatat atatcatttt
720

ttagaaacat tagattggaa caactaa
747
```

<210> 14
<211> 248

```
<212> PRT
<213> Unknown

<220>
<223> Obtained from environmental sample

<220>
<221> SITE
<222> (222)...(225)
<223> N-glycosylation site. Prosite id = PS00001

<400> 14
Met Lys Ile Lys Leu Pro Glu Pro Phe Thr Phe Glu Ala Gly Asn Arg
1               5                   10                  15

Ala Val Leu Leu Leu His Gly Phe Thr Gly His Ser Ala Asp Val Arg
            20                  25                  30

Met Leu Gly Arg Phe Leu Glu Lys Lys Gly Tyr Thr Thr His Ala Pro
        35                  40                  45

Ile Tyr Arg Gly His Gly Gln Glu Pro Glu Ala Leu Leu Lys Ser Ser
    50                  55                  60

Pro Asp Glu Trp Trp Glu Asp Ile Leu Ser Ala Tyr Asn His Leu Lys
65                  70                  75                  80

Asn Leu Gly Tyr Asn Glu Ile Ala Val Ala Gly Leu Ser Met Gly Gly
                85                  90                  95

Ala Leu Ala Ile Lys Leu Ala Thr Lys His Glu Ile Lys Gly Val Ile
            100                 105                 110

Pro Met Cys Thr Pro Met Tyr Phe Asp Asn Gln Lys Gln Leu Thr Gln
        115                 120                 125

Ala Phe Leu Asn Phe Ala Arg Gln Phe Lys Lys Phe Glu Lys Lys Asp
    130                 135                 140

Glu Glu Thr Ile Lys Lys Glu Ile Asp Leu Leu Gln Gln Asn Ser Ala
145                 150                 155                 160

Glu Leu Phe Asn Glu Ile Gly Thr Phe Val Glu Gln Val Asn Gly Ile
                165                 170                 175

Ile Asp Arg Val Asp Val Pro Thr Phe Val Val Gln Ala Ser Lys Asp
            180                 185                 190

Glu Ile Ile Asn Pro Glu Ser Ala Thr Phe Ile Tyr Glu Asn Ile Lys
        195                 200                 205

Asn Glu Lys Lys Asp Leu Lys Trp Tyr Lys Asn Ser Thr His Leu Ile
    210                 215                 220
```

Thr Phe Gly Asp Glu Lys Asp Val Leu His Glu Asp Ile Tyr His Phe
225 230 235 240

Leu Glu Thr Leu Asp Trp Asn Asn
245

<210> 15
<211> 924
<212> DNA
<213> Unknown

<220>
<223> Obtained from environmental sample

<400> 15
atggcttcca ttcaatctcg tattttcact gggttattgc gtattgcgtc aggctaccgc
60

tctcgaagcg atcgcacctc cgcgcaaaca gccgtgaaca agtcccgtgc ctttacgcgg
120

ctgatgacct tgccccagct tccgggaacg cgatccatcc acgccgacat tccgaacctt
180

cgcgcaacgt ggcatgtggg gaagcacgta cgggacgatg tgcgcatcgt ctatttccac
240

ggcggagcct ataccgcagg gtctgtcgcg acgcatcgac acttcacctc gatgctcgca
300

aaactcagcc atatccccgt gctttctgtc gactatcgcc tcgcaccaga acatcctttt
360

cctgcgggct ttgatgatgc aattgccgcc tatcaatggg ccaaagagca cgggccagta
420

ggaaagagcg aagcgcaaca tatcgtaatt gcaggtgatt ctgcgggcgg aggcctcagt
480

gcggcgatcg cgatgcaact gcgcgatgca ggacagctcc tagacggcgg cctcatgctg
540

ctttcgccgt ggatggatct gacctgtagc tccggcgcag aacatcgcat cggacatctt
600

gatgccatgc tcaatgcgga ccacgcgcgt atcatggcga gtctttacgc aggaacccat
660

gacttgaccg acccacgact ttcgccgctt tatggagact ttgcgggtct tccgcccatg
720

ttcgtcgcca ttggcggacg agaaatcgtg ctcgatgagg tggttcatgc ccttgaaaaa
780

gcgcgcgcag cgggcgtgga agttaccctc gaacgtaatg aagaaatgtt ccacgtttgg
840

ccagtgatgt tccatctcgt ccccgaagga cgcgcaagct tagagcgcat ggtcgattgg
900

ctgaattgga agtttcctgc atag
924

<210> 16
<211> 307
<212> PRT
<213> Unknown

<220>
<223> Obtained from environmental sample

<220>
<221> DOMAIN
<222> (76)...(282)
<223> alpha/beta hydrolase fold

<220>
<221> SITE
<222> (33)...(36)
<223> N-glycosylation site. Prosite id = PS00001

<220>
<221> SITE
<222> (77)...(93)
<223> Lipolytic enzymes "G-D-X-G" family, putative histidine active site. Prosite id = PS01173

<400> 16
```
Met Ala Ser Ile Gln Ser Arg Ile Phe Thr Gly Leu Leu Arg Ile Ala
1               5                   10                  15

Ser Gly Tyr Arg Ser Arg Ser Asp Arg Thr Ser Ala Gln Thr Ala Val
            20                  25                  30

Asn Lys Ser Arg Ala Phe Thr Arg Leu Met Thr Leu Pro Gln Leu Pro
        35                  40                  45

Gly Thr Arg Ser Ile His Ala Asp Ile Pro Asn Leu Arg Ala Thr Trp
    50                  55                  60

His Val Gly Lys His Val Arg Asp Asp Val Arg Ile Val Tyr Phe His
65                  70                  75                  80

Gly Gly Ala Tyr Thr Ala Gly Ser Val Ala Thr His Arg His Phe Thr
                85                  90                  95

Ser Met Leu Ala Lys Leu Ser His Ile Pro Val Leu Ser Val Asp Tyr
            100                 105                 110

Arg Leu Ala Pro Glu His Pro Phe Pro Ala Gly Phe Asp Asp Ala Ile
        115                 120                 125

Ala Ala Tyr Gln Trp Ala Lys Glu His Gly Pro Val Gly Lys Ser Glu
    130                 135                 140

Ala Gln His Ile Val Ile Ala Gly Asp Ser Ala Gly Gly Gly Leu Ser
145                 150                 155                 160
```

Ala Ala Ile Ala Met Gln Leu Arg Asp Ala Gly Gln Leu Leu Asp Gly
165 170 175

Gly Leu Met Leu Leu Ser Pro Trp Met Asp Leu Thr Cys Ser Ser Gly
180 185 190

Ala Glu His Arg Ile Gly His Leu Asp Ala Met Leu Asn Ala Asp His
195 200 205

Ala Arg Ile Met Ala Ser Leu Tyr Ala Gly Thr His Asp Leu Thr Asp
210 215 220

Pro Arg Leu Ser Pro Leu Tyr Gly Asp Phe Ala Gly Leu Pro Pro Met
225 230 235 240

Phe Val Ala Ile Gly Gly Arg Glu Ile Val Leu Asp Glu Val Val His
245 250 255

Ala Leu Glu Lys Ala Arg Ala Ala Gly Val Glu Val Thr Leu Glu Arg
260 265 270

Asn Glu Glu Met Phe His Val Trp Pro Val Met Phe His Leu Val Pro
275 280 285

Glu Gly Arg Ala Ser Leu Glu Arg Met Val Asp Trp Leu Asn Trp Lys
290 295 300

Phe Pro Ala
305

<210> 17
<211> 1050
<212> DNA
<213> Unknown

<220>
<223> Obtained from environmental sample

<400> 17
atgctctggt ttctgctcgc tgtcgttact cttgttgtcc tgatcacact gctgatcttc 60

cgggtaaagg atctttccga gtacgacggc caggactggg ttatcaagga agttgctcca 120

aacccggctc atgatgaggt catcgaacgc atcaaggaca tggggcgcgc ctcccgcgga 180

ctgaagggca aagcccggct gatcgccctc cgcaaccatc tggacagcct gggtgaaggc 240

gtggatatcg aatcggacat ccggcatcag gatcatccac gtggcgaatg gatattggcg 300

cccggggccg attcccgccg acgtgtgctc tatattcacg gcggtgcctg ggctgctggc 360

agccctcgca gtcaccgggc gattaccgac cgtttttccc ggattgccaa tgctgccgtt
420

tttgcggttg attaccggct catgcccgaa caccggttca tggatggcat tcgggactgc
480

cgccaggcct atgcatggct gctgaatcat ggcccggacg gcgaagcgcc cgtcgacttt
540

atggtggttg ccggagactc ggcgggcggc agccataccc tgtcactgct ggcgtggatt
600

cgcgacaatg gcctgcggca ggccgatgcc gccgttgccc tgtcaccctc cacggatctc
660

acgctgacgg cgcctagcaa tcgcgagaat attcgcacgg acccactcct gggcccggtg
720

tttggtggtt tgtccaaaat cccgctgccc gtgttgtggt ggggaacact tgccgcgttc
780

agactctctc ccaccaaccc ggttgcatca cctctgcggg gtgagcttaa caagctgcca
840

cccgtgctta tccacgcaag caccacggaa atgctgctgg acaacgccac gcgttacgcc
900

gccaaggcaa aagcccaggg ttcaccggtg gagcttcaca cctggcagaa catggtgcat
960

gtatggcaca tcttcacgcc actgctgcct gaggcagacg aggcattcga cgacatcgcg
1020

gcttttctga agaaagtgga acaaccttag
1050

<210> 18
<211> 349
<212> PRT
<213> Unknown

<220>
<223> Obtained from environmental sample

<220>
<221> DOMAIN
<222> (109)...(324)
<223> alpha/beta hydrolase fold

<220>
<221> SITE
<222> (299)...(302)
<223> N-glycosylation site. Prosite id = PS00001

<400> 18
Met Leu Trp Phe Leu Leu Ala Val Val Thr Leu Val Val Leu Ile Thr
1 5 10 15

Leu Leu Ile Phe Arg Val Lys Asp Leu Ser Glu Tyr Asp Gly Gln Asp
20 25 30

Trp Val Ile Lys Glu Val Ala Pro Asn Pro Ala His Asp Glu Val Ile
35 40 45

```
Glu Arg Ile Lys Asp Met Gly Arg Ala Ser Arg Gly Leu Lys Gly Lys
    50                  55                  60

Ala Arg Leu Ile Ala Leu Arg Asn His Leu Asp Ser Leu Gly Glu Gly
65                  70                  75                  80

Val Asp Ile Glu Ser Asp Ile Arg His Gln Asp His Pro Arg Gly Glu
                85                  90                  95

Trp Ile Leu Ala Pro Gly Ala Asp Ser Arg Arg Arg Val Leu Tyr Ile
            100                 105                 110

His Gly Gly Ala Trp Ala Ala Gly Ser Pro Arg Ser His Arg Ala Ile
        115                 120                 125

Thr Asp Arg Phe Ser Arg Ile Ala Asn Ala Ala Val Phe Ala Val Asp
    130                 135                 140

Tyr Arg Leu Met Pro Glu His Arg Phe Met Asp Gly Ile Arg Asp Cys
145                 150                 155                 160

Arg Gln Ala Tyr Ala Trp Leu Leu Asn His Gly Pro Asp Gly Glu Ala
                165                 170                 175

Pro Val Asp Phe Met Val Val Ala Gly Asp Ser Ala Gly Gly Ser His
            180                 185                 190

Thr Leu Ser Leu Leu Ala Trp Ile Arg Asp Asn Gly Leu Arg Gln Ala
        195                 200                 205

Asp Ala Ala Val Ala Leu Ser Pro Ser Thr Asp Leu Thr Leu Thr Ala
    210                 215                 220

Pro Ser Asn Arg Glu Asn Ile Arg Thr Asp Pro Leu Leu Gly Pro Val
225                 230                 235                 240

Phe Gly Gly Leu Ser Lys Ile Pro Leu Pro Val Leu Trp Trp Gly Thr
                245                 250                 255

Leu Ala Ala Phe Arg Leu Ser Pro Thr Asn Pro Val Ala Ser Pro Leu
            260                 265                 270

Arg Gly Glu Leu Asn Lys Leu Pro Pro Val Leu Ile His Ala Ser Thr
        275                 280                 285

Thr Glu Met Leu Leu Asp Asn Ala Thr Arg Tyr Ala Ala Lys Ala Lys
    290                 295                 300
```

Ala Gln Gly Ser Pro Val Glu Leu His Thr Trp Gln Asn Met Val His
305 310 315 320

Val Trp His Ile Phe Thr Pro Leu Leu Pro Glu Ala Asp Glu Ala Phe
325 330 335

Asp Asp Ile Ala Ala Phe Leu Lys Lys Val Glu Gln Pro
340 345

<210> 19
<211> 2022
<212> DNA
<213> Unknown

<220>
<223> Obtained from environmental sample

<400> 19
atgaacaaca acgaacaacc gacaaaacgc cgcggcataa cggtggaaga tttgcttcgc
60

ctccgctcgg tgcgcgaccc gcactacgcg ccggacggga cgcgcgccgt gtttgtggaa
120

aaatcgattg atgaggaaaa acaataccgc tcccatttat ggatatgggc ggcagacggc
180

tccgtccgtc aatggacgtt cggccgctgg cgcgacatga agccgcgttt ttccccaaga
240

ggtgaaatga tcgccttttt atctgaccgc tccggacgca cacagctttg gcttttgccc
300

gccaatggcg gtgaggcgcg ccaactgacg tttttcaaaa acggtgtgcg cgattacgtt
360

tggtcgccgg acgggacgtt tctcatcgcc ctaacgacgc ttggcgacga agaaacgatc
420

gaagaccgag aagagcagaa aacggcggaa caaaaaccgg ccgattcgaa gccgcgcgtt
480

gtggaacggc tctattacaa atcagacgcg tcgggttttc ttgacggcaa gcaggacgtg
540

ctggtgcgca tcgatgcggc gtcaggaaaa agcgaagcgt tgacgggccg cgaggaagaa
600

atcggttcgt ttgccgtttc cccaaccgga cggacgctcg cctttgtcgc caaccgaaac
660

gatgatccag acacaacctt tacgcgcgac attgttttgc ttgatctcga atcgaaagcg
720

gaaacgaact tgacgaacgg atgcggcaca ttcgcttcgc tcgcctggtc gccggacgga
780

acgaagctcg ccgccatcgg ccatgatctt gcttatctcg ggctacgct tcaccggctt
840

tacgtctttg agccggagcg tggaacgaag cgggtgttga ccgccgattg ggacgtccat
900

ctcggcgatg ccatggtcgg cgacacacac gctgatgcga aagggccggg accgatttgg
960

gcgagcgacg gaagcggcct gtatgtcacc gcctcggagc gcggacgcgt caacttgtat
1020

ttcgtcccgc tcgacggccc gatcgttccg gtgatcgaag gcaacttcca tttatacggt
1080

ttagccatcc acccaagcga acagcaagcc atcgccgcca tgagcgagcc gacggtcatt
1140

ggcgatttgt atataatctc gcttgaagac ggaacgaaaa cacggcttac gcgtgccaat
1200

gaagcgctcg aaaacgaagt ggtgttcgcc gatgccgaac cgtttacgta tcgatcggcg
1260

gatggttggg agattcaagg ctggatcatg aaaccgcccg ggcttggcga aggggaaaag
1320

gcgccgcttg tcgtcgaaat tcacggcggg ccgcatgcga tgtacgggtt taccttttc
1380

catgaatttc aactgctcgt ttcgcgtggt tatgcggtct tgttcacgaa cccgcgcggc
1440

agccacggat atggacaggc attcgtcaat gccgtgcgcg gcgactatgg cggcatggat
1500

tatgaagaca ttatggccgg cgtcgacgcc gcgatcagca agttcgactt cattgacgag
1560

acatggcttg gcgtcactgg cggcagctac ggcgggttta tgaccaattg gatcgtcggg
1620

cataccgacc ggttcaaagc ggctgtcacc cagcgttcca tttccaactg gctcagtttc
1680

tccggagtga gtgacatcgg ctactttttc acaaaatggg aggtcggctg cgacatctgg
1740

gaagacgcgg agcggctttg gcatcattca ccgctcaaat acgtccaaaa catgcgcaca
1800

ccgctcttaa tcttgcatag cgagcgcgat taccgctgcc cgatcgagca ggcggaacag
1860

ctgtttatcg cattgaaaca actcgggcgg gaaacgaaac ttgtccgctt cccggacgcc
1920

aaccacgatt tgtcgcgcac cggcaacccg gcgcttcgcc tcgaacgtct tcgccaaatc
1980

gtcggttggt ttgatcatta tttgaaagga ccactggatt aa
2022

<210> 20
<211> 673
<212> PRT
<213> Unknown

<220>
<223> Obtained from environmental sample

<220>

```
<221> DOMAIN
<222> (63)...(98)
<223> WD40-like Beta Propeller Repeat

<220>
<221> DOMAIN
<222> (457)...(671)
<223> Prolyl oligopeptidase family

<220>
<221> SITE
<222> (246)...(249)
<223> N-glycosylation site. Prosite id = PS00001

<400> 20
Met Asn Asn Asn Glu Gln Pro Thr Lys Arg Arg Gly Ile Thr Val Glu
1               5                   10                  15

Asp Leu Leu Arg Leu Arg Ser Val Arg Asp Pro His Tyr Ala Pro Asp
            20                  25                  30

Gly Thr Arg Ala Val Phe Val Glu Lys Ser Ile Asp Glu Glu Lys Gln
        35                  40                  45

Tyr Arg Ser His Leu Trp Ile Trp Ala Ala Asp Gly Ser Val Arg Gln
    50                  55                  60

Trp Thr Phe Gly Arg Trp Arg Asp Met Lys Pro Arg Phe Ser Pro Arg
65                  70                  75                  80

Gly Glu Met Ile Ala Phe Leu Ser Asp Arg Ser Gly Arg Thr Gln Leu
                85                  90                  95

Trp Leu Leu Pro Ala Asn Gly Gly Glu Ala Arg Gln Leu Thr Phe Phe
            100                 105                 110

Lys Asn Gly Val Arg Asp Tyr Val Trp Ser Pro Asp Gly Thr Phe Leu
        115                 120                 125

Ile Ala Leu Thr Thr Leu Gly Asp Glu Glu Thr Ile Glu Asp Arg Glu
    130                 135                 140

Glu Gln Lys Thr Ala Glu Gln Lys Pro Ala Asp Ser Lys Pro Arg Val
145                 150                 155                 160

Val Glu Arg Leu Tyr Tyr Lys Ser Asp Ala Ser Gly Phe Leu Asp Gly
                165                 170                 175

Lys Gln Asp Val Leu Val Arg Ile Asp Ala Ala Ser Gly Lys Ser Glu
            180                 185                 190

Ala Leu Thr Gly Arg Glu Glu Glu Ile Gly Ser Phe Ala Val Ser Pro
        195                 200                 205
```

```
Thr Gly Arg Thr Leu Ala Phe Val Ala Asn Arg Asn Asp Asp Pro Asp
    210                 215                 220

Thr Thr Phe Thr Arg Asp Ile Val Leu Leu Asp Leu Glu Ser Lys Ala
225                 230                 235                 240

Glu Thr Asn Leu Thr Asn Gly Cys Gly Thr Phe Ala Ser Leu Ala Trp
                245                 250                 255

Ser Pro Asp Gly Thr Lys Leu Ala Ala Ile Gly His Asp Leu Ala Tyr
            260                 265                 270

Leu Gly Ala Thr Leu His Arg Leu Tyr Val Phe Glu Pro Glu Arg Gly
        275                 280                 285

Thr Lys Arg Val Leu Thr Ala Asp Trp Asp Val His Leu Gly Asp Ala
    290                 295                 300

Met Val Gly Asp Thr His Ala Asp Ala Lys Gly Pro Gly Pro Ile Trp
305                 310                 315                 320

Ala Ser Asp Gly Ser Gly Leu Tyr Val Thr Ala Ser Glu Arg Gly Arg
                325                 330                 335

Val Asn Leu Tyr Phe Val Pro Leu Asp Gly Pro Ile Val Pro Val Ile
            340                 345                 350

Glu Gly Asn Phe His Leu Tyr Gly Leu Ala Ile His Pro Ser Glu Gln
        355                 360                 365

Gln Ala Ile Ala Ala Met Ser Glu Pro Thr Val Ile Gly Asp Leu Tyr
    370                 375                 380

Ile Ile Ser Leu Glu Asp Gly Thr Lys Thr Arg Leu Thr Arg Ala Asn
385                 390                 395                 400

Glu Ala Leu Glu Asn Glu Val Val Phe Ala Asp Ala Glu Pro Phe Thr
                405                 410                 415

Tyr Arg Ser Ala Asp Gly Trp Glu Ile Gln Gly Trp Ile Met Lys Pro
            420                 425                 430

Pro Gly Leu Gly Glu Gly Glu Lys Ala Pro Leu Val Val Glu Ile His
        435                 440                 445

Gly Gly Pro His Ala Met Tyr Gly Phe Thr Phe Phe His Glu Phe Gln
    450                 455                 460

Leu Leu Val Ser Arg Gly Tyr Ala Val Leu Phe Thr Asn Pro Arg Gly
```

465 470 475 480

Ser His Gly Tyr Gly Gln Ala Phe Val Asn Ala Val Arg Gly Asp Tyr
485 490 495

Gly Gly Met Asp Tyr Glu Asp Ile Met Ala Gly Val Asp Ala Ala Ile
500 505 510

Ser Lys Phe Asp Phe Ile Asp Glu Thr Trp Leu Gly Val Thr Gly Gly
515 520 525

Ser Tyr Gly Gly Phe Met Thr Asn Trp Ile Val Gly His Thr Asp Arg
530 535 540

Phe Lys Ala Ala Val Thr Gln Arg Ser Ile Ser Asn Trp Leu Ser Phe
545 550 555 560

Ser Gly Val Ser Asp Ile Gly Tyr Phe Phe Thr Lys Trp Glu Val Gly
565 570 575

Cys Asp Ile Trp Glu Asp Ala Glu Arg Leu Trp His His Ser Pro Leu
580 585 590

Lys Tyr Val Gln Asn Met Arg Thr Pro Leu Leu Ile Leu His Ser Glu
595 600 605

Arg Asp Tyr Arg Cys Pro Ile Glu Gln Ala Glu Gln Leu Phe Ile Ala
610 615 620

Leu Lys Gln Leu Gly Arg Glu Thr Lys Leu Val Arg Phe Pro Asp Ala
625 630 635 640

Asn His Asp Leu Ser Arg Thr Gly Asn Pro Ala Leu Arg Leu Glu Arg
645 650 655

Leu Arg Gln Ile Val Gly Trp Phe Asp His Tyr Leu Lys Gly Pro Leu
660 665 670

Asp

<210> 21
<211> 930
<212> DNA
<213> Unknown

<220>
<223> Obtained from environmental sample

<400> 21
gtggctgaag ttctgcgcct gcatgccaag gcgggacaag ccccgtacga gaccatcgga
60

gccaatgccg cgcgcaaggc ttttgacaag gccagccgtg tgctcgagat tggctcacgc 120

cccgtgcatc ggattgatca tgtcgagatt cccgggcgtg ccgggccggt gcatggccgg 180

ctgatctggc cccatgaacc gagctgggtc gatcgacttc ctgtgctgct gctgttgcac 240

ggcggcggtt tcacggtggg ctcttccgcg accatcgaac acattgcgcg catgctcgcc 300

atggaggtgc attgcgccat actggcgctg gactaccgtc tggcgcccga gcaccggttt 360

ccagccgcct tcgaggatgc ctgggatgcg ctggtctggc tgcatgagga agcgggcgca 420

ctggggctcg acgcttcgcg cattgccgtt ggaggcgact cggccggggg gaccctggcg 480

gccgcctgtg cattgcaggc ccgcgacgca ggcttgccat tgcgtctgca gtggctggtc 540

tacccgggga ccggcgcgca ccaggacacc gcctctcacc tgcgtctggc tgaaggctac 600

ctcatcacac gcaagaccat tctctggttc tttgagcaat acctcgccca cccggcacaa 660

cgtgaagact ggcgcttcgc cccattgctc gcaactgacc tccgcggcct ggcgccggcc 720

tggatagcgg tggccgaatt cgatccgctg gtcgatgaag gcgtggcata tgcacggcgc 780

atgcaacagt cgggggtgga agtccacctc gatttctatc cgggcatggt gcatgcgttc 840

ttcaacatgg ggggctatgt caccatggcc aggcgcgcac atgcccaggg tgtggcagca 900

ttgcgcgcag cattcacgat tcagggctga 930

<210> 22
<211> 309
<212> PRT
<213> Unknown

<220>
<223> Obtained from environmental sample

<220>
<221> DOMAIN
<222> (76)...(282)
<223> alpha/beta hydrolase fold

<400> 22
Met Ala Glu Val Leu Arg Leu His Ala Lys Ala Gly Gln Ala Pro Tyr
1 5 10 15

Glu Thr Ile Gly Ala Asn Ala Ala Arg Lys Ala Phe Asp Lys Ala Ser
20 25 30

```
Arg Val Leu Glu Ile Gly Ser Arg Pro Val His Arg Ile Asp His Val
        35                  40                  45

Glu Ile Pro Gly Arg Ala Gly Pro Val His Gly Arg Leu Ile Trp Pro
    50                  55                  60

His Glu Pro Ser Trp Val Asp Arg Leu Pro Val Leu Leu Leu Leu His
65                  70                  75                  80

Gly Gly Gly Phe Thr Val Gly Ser Ser Ala Thr Ile Glu His Ile Ala
                85                  90                  95

Arg Met Leu Ala Met Glu Val His Cys Ala Ile Leu Ala Leu Asp Tyr
            100                 105                 110

Arg Leu Ala Pro Glu His Arg Phe Pro Ala Ala Phe Glu Asp Ala Trp
        115                 120                 125

Asp Ala Leu Val Trp Leu His Glu Glu Ala Gly Ala Leu Gly Leu Asp
    130                 135                 140

Ala Ser Arg Ile Ala Val Gly Gly Asp Ser Ala Gly Gly Thr Leu Ala
145                 150                 155                 160

Ala Ala Cys Ala Leu Gln Ala Arg Asp Ala Gly Leu Pro Leu Arg Leu
                165                 170                 175

Gln Trp Leu Val Tyr Pro Gly Thr Gly Ala His Gln Asp Thr Ala Ser
            180                 185                 190

His Leu Arg Leu Ala Glu Gly Tyr Leu Ile Thr Arg Lys Thr Ile Leu
        195                 200                 205

Trp Phe Phe Glu Gln Tyr Leu Ala His Pro Ala Gln Arg Glu Asp Trp
    210                 215                 220

Arg Phe Ala Pro Leu Leu Ala Thr Asp Leu Arg Gly Leu Ala Pro Ala
225                 230                 235                 240

Trp Ile Ala Val Ala Glu Phe Asp Pro Leu Val Asp Glu Gly Val Ala
                245                 250                 255

Tyr Ala Arg Arg Met Gln Gln Ser Gly Val Glu Val His Leu Asp Phe
            260                 265                 270

Tyr Pro Gly Met Val His Ala Phe Phe Asn Met Gly Gly Tyr Val Thr
        275                 280                 285
```

Met Ala Arg Arg Ala His Ala Gln Gly Val Ala Ala Leu Arg Ala Ala
290 295 300

Phe Thr Ile Gln Gly
305

<210> 23
<211> 1152
<212> DNA
<213> Unknown

<220>
<223> Obtained from environmental sample

<400> 23
atgacaacag ttccagaaca gcttgacgcc aaagtcgctg acgcgcttga cccgatcatt
60

gatgcagcac tcaccgaagg tcgattggca ggcggcgtcg cactggtggc aaagcgtgga
120

aagttggtct atgcacgcgc tgctggtttt gccgatattg agagcaagcg tcccatgacc
180

cgagacgcca tcttccttgc ggcctcgctc accaagccga ttgtgacagc cgcgttcctc
240

tcattggtcg aggacggcgt gatgagcatg gatgagccga tcacaaaata cctccccgac
300

ttcacaccgg cgtttgaggg taagacacag cagatcacgt tgcgccaact actcactcat
360

acgtctggtc tttcctatgg tttcctgcaa ccggcagacg gtccctatct gcgcctcggc
420

gtttcggatg ggttgaacga accgggtcgg tcctttgctg acaacttggc gcggattgtc
480

gaagccggtc tgttctttcc gccgggcggg gcttggctct actcggtcag catggatgtg
540

ttgggtgcgg cgatggaagt ggcgaccggc aagagcttgc cgcaagtcgt aagcgagcgt
600

gtgtccagca agctcggcat ggcagatagc gggtttgcaa tcacggatcg cgcgaggctc
660

gcaacacctt atgcagatgg tccgccgccg gtgcccatgg gaaacgattt cgttttcccc
720

ttcgccgaac tttcaggcat tcgcttcacg cccggccgca ttttcgatca gacgtcgttt
780

ccatcgggcg gcggcggcat ggcgtgcagc gcgcctgatc tgctgacctt tctggaagcc
840

atccgcactg gtggcgcacc gattgtgaag gctgagacag caaaggcgat gatgaccaac
900

cagactggct cgcacgcctt gctactgaac ggccccggtt ggggctttgg gtatggtggc
960

gcggtgttgc tggacccgac gccaaccgct tcgcgcctgc cagtgggcac atggacctgg

1020

ggtggtgttt acggtcacag ctggtccgtc gatccaacag gagagacgag ctgtgtcttg
1080

atgaccaaca ccacggtcga agggatggcg ggtgcattgt ctgtcgattt gatggctgca
1140

gcattggcat ga
1152

<210> 24
<211> 383
<212> PRT
<213> Unknown

<220>
<223> Obtained from environmental sample

<220>
<221> DOMAIN
<222> (16)...(383)
<223> Beta-lactamase

<220>
<221> SITE
<222> (304)...(307)
<223> N-glycosylation site. Prosite id = PS00001

<220>
<221> SITE
<222> (368)...(371)
<223> N-glycosylation site. Prosite id = PS00001

<400> 24
Met Thr Thr Val Pro Glu Gln Leu Asp Ala Lys Val Ala Asp Ala Leu
1 5 10 15

Asp Pro Ile Ile Asp Ala Ala Leu Thr Glu Gly Arg Leu Ala Gly Gly
20 25 30

Val Ala Leu Val Ala Lys Arg Gly Lys Leu Val Tyr Ala Arg Ala Ala
35 40 45

Gly Phe Ala Asp Ile Glu Ser Lys Arg Pro Met Thr Arg Asp Ala Ile
50 55 60

Phe Leu Ala Ala Ser Leu Thr Lys Pro Ile Val Thr Ala Ala Phe Leu
65 70 75 80

Ser Leu Val Glu Asp Gly Val Met Ser Met Asp Glu Pro Ile Thr Lys
85 90 95

Tyr Leu Pro Asp Phe Thr Pro Ala Phe Glu Gly Lys Thr Gln Gln Ile
100 105 110

Thr Leu Arg Gln Leu Leu Thr His Thr Ser Gly Leu Ser Tyr Gly Phe
115 120 125

Leu Gln Pro Ala Asp Gly Pro Tyr Leu Arg Leu Gly Val Ser Asp Gly
    130                 135                 140

Leu Asn Glu Pro Gly Arg Ser Phe Ala Asp Asn Leu Ala Arg Ile Val
145                 150                 155                 160

Glu Ala Gly Leu Phe Phe Pro Pro Gly Gly Ala Trp Leu Tyr Ser Val
                165                 170                 175

Ser Met Asp Val Leu Gly Ala Ala Met Glu Val Ala Thr Gly Lys Ser
            180                 185                 190

Leu Pro Gln Val Val Ser Glu Arg Val Ser Ser Lys Leu Gly Met Ala
        195                 200                 205

Asp Ser Gly Phe Ala Ile Thr Asp Arg Ala Arg Leu Ala Thr Pro Tyr
    210                 215                 220

Ala Asp Gly Pro Pro Pro Val Pro Met Gly Asn Asp Phe Val Phe Pro
225                 230                 235                 240

Phe Ala Glu Leu Ser Gly Ile Arg Phe Thr Pro Gly Arg Ile Phe Asp
                245                 250                 255

Gln Thr Ser Phe Pro Ser Gly Gly Gly Gly Met Ala Cys Ser Ala Pro
            260                 265                 270

Asp Leu Leu Thr Phe Leu Glu Ala Ile Arg Thr Gly Gly Ala Pro Ile
        275                 280                 285

Val Lys Ala Glu Thr Ala Lys Ala Met Met Thr Asn Gln Thr Gly Ser
    290                 295                 300

His Ala Leu Leu Leu Asn Gly Pro Gly Trp Gly Phe Gly Tyr Gly Gly
305                 310                 315                 320

Ala Val Leu Leu Asp Pro Thr Pro Thr Ala Ser Arg Leu Pro Val Gly
                325                 330                 335

Thr Trp Thr Trp Gly Gly Val Tyr Gly His Ser Trp Ser Val Asp Pro
            340                 345                 350

Thr Gly Glu Thr Ser Cys Val Leu Met Thr Asn Thr Thr Val Glu Gly
        355                 360                 365

Met Ala Gly Ala Leu Ser Val Asp Leu Met Ala Ala Ala Leu Ala
    370                 375                 380

<210> 25

<211> 669
<212> DNA
<213> Unknown

<220>
<223> Obtained from environmental sample

<400> 25
atgttccgaa tcgacccgca aaacccattc aaaggccccc atcagggcct tgacccggca
60

acggccggaa cagatccagg ggaagccgaa gcggcgatga tcctggtgca cggccgggga
120

gcctctgccc agagcatcct catgctggcc gatgagctca atgagccgca agttcattat
180

gttgcccctc aagcctcaca attcacctgg tatccgcact ccttcctggc gccgaccgag
240

aggaatcagc cgggactcaa ctccgggctt caggcgattc atgacctgat tgaggagctg
300

ggagaaaggg gcctcaaccg agaaaaaatc atcctggccg gattctcaca aggggcctgc
360

ctggcctcgg aatttgcagc ccggcatccg gcccggtacg gcgccgtcgt tgcattgagc
420

ggcgggttga tcggagatgc cgtatccgac gaaaactaca gcggatcgat ggaaggtaca
480

ccggtattcc tcggctgcag tgacattgac ccgcacatcc cggtagaacg ggtttatgaa
540

accgaacgtg tattcaggaa actggaggct gaagtcagca gaaaaatcta tcccggaatg
600

gggcacacgg tgaacgagga ggaattgaat catatgaaaa acctgattca aaccgtgcga
660

tcgggttga
669

<210> 26
<211> 222
<212> PRT
<213> Unknown

<220>
<223> Obtained from environmental sample

<220>
<221> DOMAIN
<222> (17)...(220)
<223> Phospholipase/Carboxylesterase

<220>
<221> SITE
<222> (154)...(157)
<223> N-glycosylation site. Prosite id = PS00001

<400> 26
Met Phe Arg Ile Asp Pro Gln Asn Pro Phe Lys Gly Pro His Gln Gly
1 5 10 15

Leu Asp Pro Ala Thr Ala Gly Thr Asp Pro Gly Glu Ala Glu Ala Ala
20 25 30

Met Ile Leu Val His Gly Arg Gly Ala Ser Ala Gln Ser Ile Leu Met
35 40 45

Leu Ala Asp Glu Leu Asn Glu Pro Gln Val His Tyr Val Ala Pro Gln
50 55 60

Ala Ser Gln Phe Thr Trp Tyr Pro His Ser Phe Leu Ala Pro Thr Glu
65 70 75 80

Arg Asn Gln Pro Gly Leu Asn Ser Gly Leu Gln Ala Ile His Asp Leu
85 90 95

Ile Glu Glu Leu Gly Glu Arg Gly Leu Asn Arg Glu Lys Ile Ile Leu
100 105 110

Ala Gly Phe Ser Gln Gly Ala Cys Leu Ala Ser Glu Phe Ala Ala Arg
115 120 125

His Pro Ala Arg Tyr Gly Ala Val Val Ala Leu Ser Gly Gly Leu Ile
130 135 140

Gly Asp Ala Val Ser Asp Glu Asn Tyr Ser Gly Ser Met Glu Gly Thr
145 150 155 160

Pro Val Phe Leu Gly Cys Ser Asp Ile Asp Pro His Ile Pro Val Glu
165 170 175

Arg Val Tyr Glu Thr Glu Arg Val Phe Arg Lys Leu Glu Ala Glu Val
180 185 190

Ser Arg Lys Ile Tyr Pro Gly Met Gly His Thr Val Asn Glu Glu Glu
195 200 205

Leu Asn His Met Lys Asn Leu Ile Gln Thr Val Arg Ser Gly
210 215 220

<210> 27
<211> 408
<212> DNA
<213> Unknown

<220>
<223> Obtained from environmental sample

<400> 27
atggcgctga tcgtcctcgt ccatggagcg tggggaacgc cggccgagct cgccccgatc
60

gaaccggcac tcacggcggc ggggcacgag gtccggttcg tcgacctgcc ctgcaccgat

120

cccgacgcca cgttcgacga ctacgtcgag gcggtgatcg cccagttgga cccgcccgag
180

cgcgaccccg agaccctgct ggtggggcac tcgttcggcg gcgccaccat cggtctggtc
240

cgcgagcggc gacccgatgt ggcactggtg tacgtcacgg ccgtggtgcc ggaaccggcc
300

agtcgctgct cgagatgctg ctcggctccg atcccttcga cgaccccgac gtcgacgatc
360

cgtgggcggc cctcggcgga ctcgtgctgg acgcggggcc ggggttga
408

<210> 28
<211> 135
<212> PRT
<213> Unknown

<220>
<223> Obtained from environmental sample

<400> 28
Met Ala Leu Ile Val Leu Val His Gly Ala Trp Gly Thr Pro Ala Glu
1               5                   10                  15

Leu Ala Pro Ile Glu Pro Ala Leu Thr Ala Ala Gly His Glu Val Arg
            20                  25                  30

Phe Val Asp Leu Pro Cys Thr Asp Pro Asp Ala Thr Phe Asp Asp Tyr
        35                  40                  45

Val Glu Ala Val Ile Ala Gln Leu Asp Pro Pro Glu Arg Asp Pro Glu
    50                  55                  60

Thr Leu Leu Val Gly His Ser Phe Gly Gly Ala Thr Ile Gly Leu Val
65                  70                  75                  80

Arg Glu Arg Arg Pro Asp Val Ala Leu Val Tyr Val Thr Ala Val Val
                85                  90                  95

Pro Glu Pro Ala Ser Arg Cys Ser Arg Cys Cys Ser Ala Pro Ile Pro
            100                 105                 110

Ser Thr Thr Pro Thr Ser Thr Ile Arg Gly Arg Pro Ser Ala Asp Ser
        115                 120                 125

Cys Trp Thr Arg Gly Arg Gly
    130                 135

<210> 29
<211> 1245
<212> DNA
<213> Unknown

<220>
<223> Obtained from environmental sample

<400> 29
atgttctggt tggcacttat ctgggccctg ctggccctga atctgtatta cccctggaag 60

ggcaaacccg aggtcggtgg cgtgatcagc ttcgccttcg ggctgttggt gggcgaactc 120

gccttgcatg tgattgcgct gcaagttctc actaccctgc tgtttatcac cttctgcggc 180

ctgtcaggct tcggcgatgc gctggggctg atgctcgcca tggccacctg gctggcgatg 240

gcctggttct acgcacgcag cgagcgcgcg gggcccgcga tggagagcgg cattcgccag 300

gccctgggcg aagactattc aaaactgatt tcggccgagc gcagcgactg cctgcaccgc 360

gacatagatt atcggcagct gcttaacccc tttgccttca gcaaacccga agtgacccga 420

caccgcaatc tgccctacgc ggaagtggac ggcaaaaacc tttatttgga tatcttccac 480

caccgcgaca agccacaaaa cgcgccagta ctgctgcaaa ttcacggcgg cgcctggctg 540

cagaatctgg gcaacaagga tcagcaggcg ctgccgctga tgaatgaact ggccagccgc 600

ggctgggtgt gcgtgacggt gcaatatcgg ctgagtcccg gcgtcaccat gcccacccat 660

attattgact gcaagcgcgc gctggcctgg gcgaaggagc atatcgctga ttacggtggc 720

gatccgaact tcattatcgt caccggcggt tccgctggcg ggcatctgtc agcgatggtg 780

gcgctcaccg ccaaccagcc cgaattccag ccgggtttcg aggacacgga tacccgcgta 840

caaggtgccg tacctttcta cggtatccac gactttacca acagccgtgg tcagcgcgag 900

cataaggggc tggagaactt catcgcgcaa cgcgtaatga aagtgacccg ctacgataac 960

cccaaactgt gggagcaatg ctctcccctg tttcaggtaa aagacgacgc gcctccgatg 1020

ctgttggtac acggcgaggc cgataccctg gcaccgatca gcgaatcgca ggccttgctg 1080

gaggttttgc gggaacacga cgtcaaagcg gggctggcgg cactgcccga tgcgcagcac 1140

gccttcgaac tgatgtactc gctgcgcacc ctgtatatga ttaatgccgt ggagcgcttc 1200

ggcaacgcac tgcacggcaa gtatctacag gatggcgcag cttaa

1245

<210> 30
<211> 414
<212> PRT
<213> Unknown

<220>
<223> Obtained from environmental sample

<220>
<221> DOMAIN
<222> (171)...(384)
<223> alpha/beta hydrolase fold

<220>
<221> DOMAIN
<222> (189)...(403)
<223> Prolyl oligopeptidase family

<400> 30
Met Phe Trp Leu Ala Leu Ile Trp Ala Leu Leu Ala Leu Asn Leu Tyr
1               5                   10                  15

Tyr Pro Trp Lys Gly Lys Pro Glu Val Gly Gly Val Ile Ser Phe Ala
            20                  25                  30

Phe Gly Leu Leu Val Gly Glu Leu Ala Leu His Val Ile Ala Leu Gln
        35                  40                  45

Val Leu Thr Thr Leu Leu Phe Ile Thr Phe Cys Gly Leu Ser Gly Phe
    50                  55                  60

Gly Asp Ala Leu Gly Leu Met Leu Ala Met Ala Thr Trp Leu Ala Met
65                  70                  75                  80

Ala Trp Phe Tyr Ala Arg Ser Glu Arg Ala Gly Pro Ala Met Glu Ser
                85                  90                  95

Gly Ile Arg Gln Ala Leu Gly Glu Asp Tyr Ser Lys Leu Ile Ser Ala
            100                 105                 110

Glu Arg Ser Asp Cys Leu His Arg Asp Ile Asp Tyr Arg Gln Leu Leu
        115                 120                 125

Asn Pro Phe Ala Phe Ser Lys Pro Glu Val Thr Arg His Arg Asn Leu
    130                 135                 140

Pro Tyr Ala Glu Val Asp Gly Lys Asn Leu Tyr Leu Asp Ile Phe His
145                 150                 155                 160

His Arg Asp Lys Pro Gln Asn Ala Pro Val Leu Leu Gln Ile His Gly
                165                 170                 175

Gly Ala Trp Leu Gln Asn Leu Gly Asn Lys Asp Gln Gln Ala Leu Pro

```
            180                 185                 190

Leu Met Asn Glu Leu Ala Ser Arg Gly Trp Val Cys Val Thr Val Gln
        195                 200                 205

Tyr Arg Leu Ser Pro Gly Val Thr Met Pro Thr His Ile Ile Asp Cys
    210                 215                 220

Lys Arg Ala Leu Ala Trp Ala Lys Glu His Ile Ala Asp Tyr Gly Gly
225                 230                 235                 240

Asp Pro Asn Phe Ile Ile Val Thr Gly Gly Ser Ala Gly Gly His Leu
                245                 250                 255

Ser Ala Met Val Ala Leu Thr Ala Asn Gln Pro Glu Phe Gln Pro Gly
            260                 265                 270

Phe Glu Asp Thr Asp Thr Arg Val Gln Gly Ala Val Pro Phe Tyr Gly
        275                 280                 285

Ile His Asp Phe Thr Asn Ser Arg Gly Gln Arg Glu His Lys Gly Leu
    290                 295                 300

Glu Asn Phe Ile Ala Gln Arg Val Met Lys Val Thr Arg Tyr Asp Asn
305                 310                 315                 320

Pro Lys Leu Trp Glu Gln Cys Ser Pro Leu Phe Gln Val Lys Asp Asp
                325                 330                 335

Ala Pro Pro Met Leu Leu Val His Gly Glu Ala Asp Thr Leu Ala Pro
            340                 345                 350

Ile Ser Glu Ser Gln Ala Leu Leu Glu Val Leu Arg Glu His Asp Val
        355                 360                 365

Lys Ala Gly Leu Ala Ala Leu Pro Asp Ala Gln His Ala Phe Glu Leu
    370                 375                 380

Met Tyr Ser Leu Arg Thr Leu Tyr Met Ile Asn Ala Val Glu Arg Phe
385                 390                 395                 400

Gly Asn Ala Leu His Gly Lys Tyr Leu Gln Asp Gly Ala Ala
                405                 410
```

<210> 31
<211> 1245
<212> DNA
<213> Unknown

<220>
<223> Obtained from environmental sample

<400> 31
atgttctggt tggcacttat ctgggccctg ctggccctga atctgtatta cccctggaag 60

ggcaaacccg aggtcggtgg cgtgatcagc ttcgccttcg ggctgttggt gggcgaactc 120

gccttgcatg tgattgcact gcaagtcctc actaccctgc tgtttatcac cttctgcggc 180

ctctcgggct tcggcgatgc gttgggcctg atgctcgcca tggccacctg gctggcgatg 240

gcctggttct acgcacgcag cgagcgcgcg gggccggcga tggagaacgg cattcgccag 300

gccctgggcg aagactattc aaaactgatt tcggccgagc gcagcgactg cctgcaccgc 360

gacatagatt accggcagct gcttaacccc tttgccttca gcaaacccga agtgacccga 420

caccgcaatc tgccctacgc ggaagtggac ggcaaaaacc ttcatctgga tatcttccac 480

caccgcgaca agccacaaaa cgcgccagta ctgctgcaaa ttcacggcgg cgcctggctg 540

cagaatctgg gcaacaagga tcagcaggca ctgccgctga tgaatgaact ggccagccgc 600

ggctgggttt gcgtaacagt gcaataccgg ctgagccccg gcgtcgccat gcccacccat 660

attattgact gcaagcgcgc actggtctgg gcgaaggagc atatcgccga ttacggtggc 720

gatccgaact tcattatcgt caccggcggt tccgctggcg gccatctgtc agcgatggtg 780

gcgctcaccg ccaaccaacc cgaattccag ccgggtttcg aggacacgga tacccgcgta 840

caaggtgccg tacctttcta cggtatccac gactttacca acagccgtgg tcagcgcgag 900

cataaggggc tggagaactt catcgcgcaa cgcgtaatga aagtgacccg ctacgataac 960

cccaaactgt gggagcaatg ctctcccctg tttcaggtaa aagacgacgc gcctccaatg 1020

ctgttggtac acggcgaggc cgataccctg gcaccgatca gcgaatcgca ggccttgctg 1080

gaggttttgc gggaacacga cgtcaaagcg ggtctggcgg cactccccga tgcgcagcac 1140

gccttcgaac tgatgtactc gctgcgcacc ctgtatatga ttaacgccgt ggagcgcttc 1200

ggcaacgcgc tgcacggcaa gtatctacag gatggcgcag cttaa 1245

<210> 32

<211> 414
<212> PRT
<213> Unknown

<220>
<223> Obtained from environmental sample

<220>
<221> DOMAIN
<222> (171)...(384)
<223> alpha/beta hydrolase fold

<220>
<221> DOMAIN
<222> (189)...(403)
<223> Prolyl oligopeptidase family

<400> 32

```
Met Phe Trp Leu Ala Leu Ile Trp Ala Leu Leu Ala Leu Asn Leu Tyr
1               5                   10                  15

Tyr Pro Trp Lys Gly Lys Pro Glu Val Gly Gly Val Ile Ser Phe Ala
            20                  25                  30

Phe Gly Leu Leu Val Gly Glu Leu Ala Leu His Val Ile Ala Leu Gln
        35                  40                  45

Val Leu Thr Thr Leu Leu Phe Ile Thr Phe Cys Gly Leu Ser Gly Phe
    50                  55                  60

Gly Asp Ala Leu Gly Leu Met Leu Ala Met Ala Thr Trp Leu Ala Met
65                  70                  75                  80

Ala Trp Phe Tyr Ala Arg Ser Glu Arg Ala Gly Pro Ala Met Glu Asn
                85                  90                  95

Gly Ile Arg Gln Ala Leu Gly Glu Asp Tyr Ser Lys Leu Ile Ser Ala
            100                 105                 110

Glu Arg Ser Asp Cys Leu His Arg Asp Ile Asp Tyr Arg Gln Leu Leu
        115                 120                 125

Asn Pro Phe Ala Phe Ser Lys Pro Glu Val Thr Arg His Arg Asn Leu
    130                 135                 140

Pro Tyr Ala Glu Val Asp Gly Lys Asn Leu His Leu Asp Ile Phe His
145                 150                 155                 160

His Arg Asp Lys Pro Gln Asn Ala Pro Val Leu Leu Gln Ile His Gly
                165                 170                 175

Gly Ala Trp Leu Gln Asn Leu Gly Asn Lys Asp Gln Gln Ala Leu Pro
            180                 185                 190
```

```
Leu Met Asn Glu Leu Ala Ser Arg Gly Trp Val Cys Val Thr Val Gln
        195                 200                 205

Tyr Arg Leu Ser Pro Gly Val Ala Met Pro Thr His Ile Ile Asp Cys
    210                 215                 220

Lys Arg Ala Leu Val Trp Ala Lys Glu His Ile Ala Asp Tyr Gly Gly
225                 230                 235                 240

Asp Pro Asn Phe Ile Ile Val Thr Gly Gly Ser Ala Gly Gly His Leu
                245                 250                 255

Ser Ala Met Val Ala Leu Thr Ala Asn Gln Pro Glu Phe Gln Pro Gly
            260                 265                 270

Phe Glu Asp Thr Asp Thr Arg Val Gln Gly Ala Val Pro Phe Tyr Gly
        275                 280                 285

Ile His Asp Phe Thr Asn Ser Arg Gly Gln Arg Glu His Lys Gly Leu
    290                 295                 300

Glu Asn Phe Ile Ala Gln Arg Val Met Lys Val Thr Arg Tyr Asp Asn
305                 310                 315                 320

Pro Lys Leu Trp Glu Gln Cys Ser Pro Leu Phe Gln Val Lys Asp Asp
                325                 330                 335

Ala Pro Pro Met Leu Leu Val His Gly Glu Ala Asp Thr Leu Ala Pro
            340                 345                 350

Ile Ser Glu Ser Gln Ala Leu Leu Glu Val Leu Arg Glu His Asp Val
        355                 360                 365

Lys Ala Gly Leu Ala Ala Leu Pro Asp Ala Gln His Ala Phe Glu Leu
    370                 375                 380

Met Tyr Ser Leu Arg Thr Leu Tyr Met Ile Asn Ala Val Glu Arg Phe
385                 390                 395                 400

Gly Asn Ala Leu His Gly Lys Tyr Leu Gln Asp Gly Ala Ala
                405                 410
```

<210> 33
<211> 900
<212> DNA
<213> Unknown

<220>
<223> Obtained from environmental sample

<400> 33
atgaaggcaa gctgccgcga catcgaaatc gaatacgaaa cattcggcca tccggacgac

60

ccggccatcg tgctgatcat gggactcggt ggccagctca tcctgtggcc ggaagcgttc
120

tgccgcatgc tggccgacgc tggtcactac gtggtgcgct tcgacaaccg cgacatcggc
180

ctgtcgacgc atctcgatca ccttccccgc cccaacctgc cgctcgccgc gctccgccag
240

gcgctgcgcc tgccagttcg cgccagttac acgctcgacg acatggcgga cgacgtcgcg
300

ggcctgcttg atgcactgaa catcaagcag gcgcacgtcg tcggcgtgtc gatgggcggc
360

atgatcgccc agctgctggc cgcacggcac gcgacccgcg tgcgcagcct gaccttgctg
420

atgaccacca gcggcgcgcg caacgttccg ggcccctcac tcggcatgcg catggaaatg
480

atccgtcgac cgcgcgacac ctcgcgcgag gggctgatcc gccatggtat gcgtacctgg
540

cggatcatcg gcagcccgac gtacccgaag ccggaagccg aactgcgccg catcgtcgcc
600

gagggctttg accgcgcgtt tcacccggcc ggtttcatgc gccagctgca cgccgttctc
660

gcggcaccga gccgcgcacc cctgctgccg cgcatcaaac agccggccga cgtcattcac
720

ggcgacgccg acctgctggt accagtggca gcggcacgtg atctggtgcg ccgcctgccg
780

aacgccacgc tcgacatcgt gccgggcatg ggcatgact tcccgaccga gatcatgccg
840

cgtatcgcgc gccgcattgt cgaaaccgcg gcacgcgacc cgcagcggct cgccgcctag
900

<210> 34
<211> 299
<212> PRT
<213> Unknown

<220>
<223> Obtained from environmental sample

<220>
<221> DOMAIN
<222> (49)...(288)
<223> alpha/beta hydrolase fold

<220>
<221> SITE
<222> (265)...(268)
<223> N-glycosylation site. Prosite id = PS00001

<400> 34
Met Lys Ala Ser Cys Arg Asp Ile Glu Ile Glu Tyr Glu Thr Phe Gly
1 5 10 15

```
His Pro Asp Asp Pro Ala Ile Val Leu Ile Met Gly Leu Gly Gly Gln
            20                  25                  30

Leu Ile Leu Trp Pro Glu Ala Phe Cys Arg Met Leu Ala Asp Ala Gly
        35                  40                  45

His Tyr Val Val Arg Phe Asp Asn Arg Asp Ile Gly Leu Ser Thr His
    50                  55                  60

Leu Asp His Leu Pro Arg Pro Asn Leu Pro Leu Ala Ala Leu Arg Gln
65                  70                  75                  80

Ala Leu Arg Leu Pro Val Arg Ala Ser Tyr Thr Leu Asp Asp Met Ala
                85                  90                  95

Asp Asp Val Ala Gly Leu Leu Asp Ala Leu Asn Ile Lys Gln Ala His
            100                 105                 110

Val Val Gly Val Ser Met Gly Gly Met Ile Ala Gln Leu Leu Ala Ala
        115                 120                 125

Arg His Ala Thr Arg Val Arg Ser Leu Thr Leu Leu Met Thr Thr Ser
    130                 135                 140

Gly Ala Arg Asn Val Pro Gly Pro Ser Leu Gly Met Arg Met Glu Met
145                 150                 155                 160

Ile Arg Arg Pro Arg Asp Thr Ser Arg Glu Gly Leu Ile Arg His Gly
                165                 170                 175

Met Arg Thr Trp Arg Ile Ile Gly Ser Pro Thr Tyr Pro Lys Pro Glu
            180                 185                 190

Ala Glu Leu Arg Arg Ile Val Ala Glu Gly Phe Asp Arg Ala Phe His
        195                 200                 205

Pro Ala Gly Phe Met Arg Gln Leu His Ala Val Leu Ala Ala Pro Ser
    210                 215                 220

Arg Ala Pro Leu Leu Pro Arg Ile Lys Gln Pro Ala Asp Val Ile His
225                 230                 235                 240

Gly Asp Ala Asp Leu Leu Val Pro Val Ala Ala Ala Arg Asp Leu Val
                245                 250                 255

Arg Arg Leu Pro Asn Ala Thr Leu Asp Ile Val Pro Gly Met Gly His
            260                 265                 270

Asp Phe Pro Thr Glu Ile Met Pro Arg Ile Ala Arg Arg Ile Val Glu
```

275 280 285

Thr Ala Ala Arg Asp Pro Gln Arg Leu Ala Ala
290 295

<210> 35
<211> 942
<212> DNA
<213> Unknown

<220>
<223> Obtained from environmental sample

<400> 35
atgaagctcg acgacgaagc cgccatggtg ctggacctga tgaagaaatc gggccggccg 60

gcgctggaga cgctcgacgc ggcggcggcg cgcgcccagg ccgacgggct gtcggagaaa 120

agcgagatcg atccgccgga ggtggcgaag gtcgtcgacg gcacgctgcc cggacccggc 180

ggcccgatcc ggttccgccg ctactggccg atcggggccg gcggcgcgac cctgccgacg 240

ctgatctact accacggcgg cggcttcgtg atcggcaatc tggagaccca cgactcgacc 300

tgcaggcgga tcgccaatgt cagccagtgc caggtcgtcg ccatcgacta tcgcctcgcg 360

cccgagcatc ccttccccgc gccggtcgac gacgccatcg ccgctttccg ccacatcgcc 420

gccaatgccg cgcagttcga cacggaagcc gatgcgctcg cggtcggcgg cgattcggcc 480

ggcggcaatc tcgctgcggt gatctgccag gcgcagaggg aaacgggcgg ccagatgccc 540

tcgttccagc tgctgatcta cccggccacc gacaaccgca acgaaagccc gtcgcgacgc 600

atgctgggcg acggctattt cctcaccacg gcgctgatga aatggttctt cgaccactac 660

gcccccgcag gcgtcgatcg caccgatccg cgcatctcgc cgctgttcgc caaggactat 720

tccggcctgc cgccggcctt cgtgctgacc gccggtttcg atccgctgcg cgacgagggc 780

cgcgactacg ccaaccggct gatcgacgcg ggcgtcaagg tcacctattc caactatccc 840

ggcaccatcc acggtttctt ctcgatgacg cgtttcctca agcagggctt gcgcgccaac 900

gacgaggccg cctgcgcgct gaaggtgcat ttcggcatct ga 942

<210> 36
<211> 313

<212> PRT
<213> Unknown

<220>
<223> Obtained from environmental sample

<220>
<221> DOMAIN
<222> (81)...(288)
<223> alpha/beta hydrolase fold

<220>
<221> SITE
<222> (107)...(110)
<223> N-glycosylation site. Prosite id = PS00001

<400> 36
```
Met Lys Leu Asp Asp Glu Ala Ala Met Val Leu Asp Leu Met Lys Lys
1               5                   10                  15

Ser Gly Arg Pro Ala Leu Glu Thr Leu Asp Ala Ala Ala Ala Arg Ala
            20                  25                  30

Gln Ala Asp Gly Leu Ser Glu Lys Ser Glu Ile Asp Pro Pro Glu Val
        35                  40                  45

Ala Lys Val Val Asp Gly Thr Leu Pro Gly Pro Gly Gly Pro Ile Arg
    50                  55                  60

Phe Arg Arg Tyr Trp Pro Ile Gly Ala Gly Gly Ala Thr Leu Pro Thr
65                  70                  75                  80

Leu Ile Tyr Tyr His Gly Gly Gly Phe Val Ile Gly Asn Leu Glu Thr
                85                  90                  95

His Asp Ser Thr Cys Arg Arg Ile Ala Asn Val Ser Gln Cys Gln Val
            100                 105                 110

Val Ala Ile Asp Tyr Arg Leu Ala Pro Glu His Pro Phe Pro Ala Pro
        115                 120                 125

Val Asp Asp Ala Ile Ala Ala Phe Arg His Ile Ala Ala Asn Ala Ala
    130                 135                 140

Gln Phe Asp Thr Glu Ala Asp Ala Leu Ala Val Gly Gly Asp Ser Ala
145                 150                 155                 160

Gly Gly Asn Leu Ala Ala Val Ile Cys Gln Ala Gln Arg Glu Thr Gly
                165                 170                 175

Gly Gln Met Pro Ser Phe Gln Leu Leu Ile Tyr Pro Ala Thr Asp Asn
            180                 185                 190

Arg Asn Glu Ser Pro Ser Arg Arg Met Leu Gly Asp Gly Tyr Phe Leu
```

195 200 205

Thr Thr Ala Leu Met Lys Trp Phe Phe Asp His Tyr Ala Pro Ala Gly
210 215 220

Val Asp Arg Thr Asp Pro Arg Ile Ser Pro Leu Phe Ala Lys Asp Tyr
225 230 235 240

Ser Gly Leu Pro Pro Ala Phe Val Leu Thr Ala Gly Phe Asp Pro Leu
245 250 255

Arg Asp Glu Gly Arg Asp Tyr Ala Asn Arg Leu Ile Asp Ala Gly Val
260 265 270

Lys Val Thr Tyr Ser Asn Tyr Pro Gly Thr Ile His Gly Phe Phe Ser
275 280 285

Met Thr Arg Phe Leu Lys Gln Gly Leu Arg Ala Asn Asp Glu Ala Ala
290 295 300

Cys Ala Leu Lys Val His Phe Gly Ile
305 310

<210> 37
<211> 957
<212> DNA
<213> Unknown

<220>
<223> Obtained from environmental sample

<400> 37
atgaggatcc gtgcgctgac cacctgcttc gccctgctcg ccgcagggct gctcctgtcg
60

ccacccgcga tggcggaggg tgaccccgag tacagcgtcc ccctggagct gctgcgcggc
120

tcggtggact gcaatgccga gttcgtccat ctcgaccggg agccggtcct gctcgtgcac
180

gggacgtccg gcacgcccga ggaagcctgg gcctggaact acatcgagaa cctcccccgc
240

caggggttcg acgtgtgctg ggtccgcctc cccaaccggg cgctcagcga ccagcaggag
300

tcggcagagt acgtggtgta cgcgctgcga ctcatgcacg agcgggcgga gggacgcaag
360

atcgacgtga taggccacag ccagggtggg ctgctgcccc gctgggcgct gcgttggtgg
420

ccgagcctgc gcgacatcgt ggacgacttc gtcccgctcg ccgcgcccgc ccacggcgcc
480

agcggcgcag agatgttctg tccgacgagc tgcgcgcccg cggtccagca gatgaagccc
540

gactcccgct acctcgccgc cctcaactca gtggacgaaa cccccggcga cgtggactac
600

acgagcatct acagcatgac cgacgagctc gtgcagccct acaccagccc tccgctggaa
660

ggcgggacca acatcgcgat ccaggacgtc tgtcccggcc gggtcgtgca tcactacggg
720

atcgtctacg acgcggtcgc ctacgccctg gtggtcgatg cgctcgtcaa cccgggcccg
780

gccgacccgg cccggctccc cgccgacatc tgcctacaga cgtggatgcc gggtgtgaac
840

gagatcgacg tcgtggccgg gaacatgatg gcttacggga acgcctacct ggccttcggt
900

gcctacccac ccagcgattc cgagcccgag ccgaagccct acgtggacga ggactga
957

<210> 38
<211> 318
<212> PRT
<213> Unknown

<220>
<223> Obtained from environmental sample

<220>
<221> SIGNAL
<222> (1)...(25)

<220>
<221> DOMAIN
<222> (53)...(257)
<223> Lipase (class 2)

<220>
<221> SITE
<222> (122)...(131)
<223> Lipases, serine active site. Prosite id = PS00120

<400> 38
Met Arg Ile Arg Ala Leu Thr Thr Cys Phe Ala Leu Leu Ala Ala Gly
1               5                   10                  15

Leu Leu Leu Ser Pro Pro Ala Met Ala Glu Gly Asp Pro Glu Tyr Ser
            20                  25                  30

Val Pro Leu Glu Leu Leu Arg Gly Ser Val Asp Cys Asn Ala Glu Phe
        35                  40                  45

Val His Leu Asp Arg Glu Pro Val Leu Leu Val His Gly Thr Ser Gly
    50                  55                  60

Thr Pro Glu Glu Ala Trp Ala Trp Asn Tyr Ile Glu Asn Leu Pro Arg
65                  70                  75                  80

Gln Gly Phe Asp Val Cys Trp Val Arg Leu Pro Asn Arg Ala Leu Ser

85 90 95

Asp Gln Gln Glu Ser Ala Glu Tyr Val Val Tyr Ala Leu Arg Leu Met
100 105 110

His Glu Arg Ala Glu Gly Arg Lys Ile Asp Val Ile Gly His Ser Gln
115 120 125

Gly Gly Leu Leu Pro Arg Trp Ala Leu Arg Trp Trp Pro Ser Leu Arg
130 135 140

Asp Ile Val Asp Asp Phe Val Pro Leu Ala Ala Pro Ala His Gly Ala
145 150 155 160

Ser Gly Ala Glu Met Phe Cys Pro Thr Ser Cys Ala Pro Ala Val Gln
165 170 175

Gln Met Lys Pro Asp Ser Arg Tyr Leu Ala Ala Leu Asn Ser Val Asp
180 185 190

Glu Thr Pro Gly Asp Val Asp Tyr Thr Ser Ile Tyr Ser Met Thr Asp
195 200 205

Glu Leu Val Gln Pro Tyr Thr Ser Pro Pro Leu Glu Gly Gly Thr Asn
210 215 220

Ile Ala Ile Gln Asp Val Cys Pro Gly Arg Val Val His His Tyr Gly
225 230 235 240

Ile Val Tyr Asp Ala Val Ala Tyr Ala Leu Val Val Asp Ala Leu Val
245 250 255

Asn Pro Gly Pro Ala Asp Pro Ala Arg Leu Pro Ala Asp Ile Cys Leu
260 265 270

Gln Thr Trp Met Pro Gly Val Asn Glu Ile Asp Val Val Ala Gly Asn
275 280 285

Met Met Ala Tyr Gly Asn Ala Tyr Leu Ala Phe Gly Ala Tyr Pro Pro
290 295 300

Ser Asp Ser Glu Pro Glu Pro Lys Pro Tyr Val Asp Glu Asp
305 310 315

<210> 39
<211> 1092
<212> DNA
<213> Unknown

<220>
<223> Obtained from environmental sample

<400> 39
atgaccacat ctacacaaca catcagcgag ctacctctct taccaggccg tctcggcgac 60

cccgatcgcg tcttgaagac tgatccccgt gccgatcctc ggttggtcgc cgcctgcgcc 120

ccctttgctt tagacgttgc gcccccaccc gcaccggtga acgcgcactc gcccttgcag 180

gacaagctcg actacagtgc ggccaacgag gcgggcatgg aaaccgtgtt tgccgccctg 240

ttcgccgacc tcccgccgat gacgaacgtg gagcggcgga ccgaggtcat caagggcgtg 300

gacggtaacg acatcaagct gtatatccat acgccccagc acgtttctgg cccgctgccc 360

tgcgtgtatc atatacacgg cggcggcatg gtcattctga cggccgctgg ccctacctat 420

gtacgctggc gggacgagct tgccgccctg ggcatggtcg tggtgggtgt ggaattccgt 480

aatggcgccg gcaagcttgg caaccatccc tttccagccg ggctcaacga ctgcatgagt 540

ggcttacagt ggacgtttga ccacaaaacc accctgggga tctcgaagat aatcgtgtcc 600

ggtgagtctg gtggaggcaa tctctccctg gccgtgtgcc tcaaggccaa gaaggacaaa 660

cgccttgagc agattcatgg tgtctacgcc ttgtgtccgt acatttatgg cgcatgggcg 720

cagaagagca aagatctccc ttccctatac gaaaacgacg gctacttgat caactgtagc 780

ctgatggagg tgctggcaag cgtctatgac cctgaaggca aaaacgccac caatccgctg 840

tgctggccat actgggccac acgtgaggac ctgcaaggtc tgcctccgca tgtgatctcg 900

gtcaacgagt tagacccccct gcgagacgag gggctgaaat actatcagag gctcctggcg 960

gctggggtgc gtgtatacag ccggaccgtc aacggcacgt gccatgcggg cgacgtcctg 1020

ttccgcaaag cgctcccgga cgtgtacgca gccaccctcc gcgatatcaa ggggttcgca 1080

gactcgctgt ag 1092

<210> 40
<211> 363
<212> PRT
<213> Unknown

<220>
<223> Obtained from environmental sample

<220>
<221> DOMAIN
<222> (122)...(340)
<223> alpha/beta hydrolase fold

<220>
<221> SITE
<222> (210)...(213)
<223> N-glycosylation site. Prosite id = PS00001

<220>
<221> SITE
<222> (261)...(264)
<223> N-glycosylation site. Prosite id = PS00001

<220>
<221> SITE
<222> (279)...(282)
<223> N-glycosylation site. Prosite id = PS00001

<220>
<221> SITE
<222> (336)...(339)
<223> N-glycosylation site. Prosite id = PS00001

<400> 40
```
Met Thr Thr Ser Thr Gln His Ile Ser Glu Leu Pro Leu Leu Pro Gly
1               5                   10                  15

Arg Leu Gly Asp Pro Asp Arg Val Leu Lys Thr Asp Pro Arg Ala Asp
            20                  25                  30

Pro Arg Leu Val Ala Ala Cys Ala Pro Phe Ala Leu Asp Val Ala Pro
        35                  40                  45

Pro Pro Ala Pro Val Asn Ala His Ser Pro Leu Gln Asp Lys Leu Asp
    50                  55                  60

Tyr Ser Ala Ala Asn Glu Ala Gly Met Glu Thr Val Phe Ala Ala Leu
65                  70                  75                  80

Phe Ala Asp Leu Pro Pro Met Thr Asn Val Glu Arg Arg Thr Glu Val
                85                  90                  95

Ile Lys Gly Val Asp Gly Asn Asp Ile Lys Leu Tyr Ile His Thr Pro
            100                 105                 110

Gln His Val Ser Gly Pro Leu Pro Cys Val Tyr His Ile His Gly Gly
        115                 120                 125

Gly Met Val Ile Leu Thr Ala Ala Gly Pro Thr Tyr Val Arg Trp Arg
    130                 135                 140

Asp Glu Leu Ala Ala Leu Gly Met Val Val Val Gly Val Glu Phe Arg
145                 150                 155                 160
```

Asn Gly Ala Gly Lys Leu Gly Asn His Pro Phe Pro Ala Gly Leu Asn
165 170 175

Asp Cys Met Ser Gly Leu Gln Trp Thr Phe Asp His Lys Thr Thr Leu
180 185 190

Gly Ile Ser Lys Ile Ile Val Ser Gly Glu Ser Gly Gly Gly Asn Leu
195 200 205

Ser Leu Ala Val Cys Leu Lys Ala Lys Lys Asp Lys Arg Leu Glu Gln
210 215 220

Ile His Gly Val Tyr Ala Leu Cys Pro Tyr Ile Tyr Gly Ala Trp Ala
225 230 235 240

Gln Lys Ser Lys Asp Leu Pro Ser Leu Tyr Glu Asn Asp Gly Tyr Leu
245 250 255

Ile Asn Cys Ser Leu Met Glu Val Leu Ala Ser Val Tyr Asp Pro Glu
260 265 270

Gly Lys Asn Ala Thr Asn Pro Leu Cys Trp Pro Tyr Trp Ala Thr Arg
275 280 285

Glu Asp Leu Gln Gly Leu Pro Pro His Val Ile Ser Val Asn Glu Leu
290 295 300

Asp Pro Leu Arg Asp Glu Gly Leu Lys Tyr Tyr Gln Arg Leu Leu Ala
305 310 315 320

Ala Gly Val Arg Val Tyr Ser Arg Thr Val Asn Gly Thr Cys His Ala
325 330 335

Gly Asp Val Leu Phe Arg Lys Ala Leu Pro Asp Val Tyr Ala Ala Thr
340 345 350

Leu Arg Asp Ile Lys Gly Phe Ala Asp Ser Leu
355 360

<210> 41
<211> 1005
<212> DNA
<213> Unknown

<220>
<223> Obtained from environmental sample

<400> 41
atgagaatca gccttattca cgttgctttg ctgctcggcc cgcttttccc ggttagcgca
60

ctttccgccg ccgaccagct gcgccgcggc gaacgcaagg aaacaaccaa ggccgaagcc

120

ccgcgcaagg aggagaagaa ggacgagccg gcgcaatcgc ccgtcgagat catcaagaac
180

atttcctacc gggacgatga ggccgccgac ccgacgcgcc acaagctcga cctgtacctg
240

ccgcgcggcg ccaaggactt cccgattgtc tttttcgtcc acggcggcgc ctggcgagcc
300

ggcaacaagg atgagtatcc cagacttggc gaattctttg ccaccgaggg cattggctgc
360

gtcgtcatca attaccgcct ctcccccaag gtgcagcatc ccgcccatat tgaagacgtc
420

gcccgtgcct tcgcctggac cgtcgccaac atcgaaaaat acggcggccg taaagatcgc
480

atttcgccg tgggccactc ggccggcggc cacctggtcg ccctgctggc caccgatgcc
540

acgtacctca agaacgaagg actggcgctg accgacattc gcggcgtgat ccccattagc
600

ggcgtgcatc agatcaactc ggccctgccg atgttccgta acattttcag cagggacaag
660

gaggagtgcc ggcttgcctc gcccatcacc catgtcagca atggccaccc gcctttcctg
720

ctcctttacg ctgagaacga cctgccgctc ttgggcagga tggccgagga catgagcgag
780

gtgctgaaga agaatcagtg cgatgtcgaa tgcctgaaga ttgaccagcg caaccacatg
840

acgatcatca cccagctttc ccaggaaaat gacccgacgc ggcaagcggt cttcgacttc
900

atcggccggc atagcgactg gcagcccgcg gccagggccg aggctgccaa cggcaataag
960

cgcgaagccg gcacgacgga cgccgctatg aaggacgaca aatga
1005

<210> 42
<211> 334
<212> PRT
<213> Unknown

<220>
<223> Obtained from environmental sample

<220>
<221> SIGNAL
<222> (1)...(23)

<220>
<221> DOMAIN
<222> (90)...(283)
<223> alpha/beta hydrolase fold

<220>
<221> SITE

<222> (60)...(63)
<223> N-glycosylation site. Prosite id = PS00001

<400> 42
```
Met Arg Ile Ser Leu Ile His Val Ala Leu Leu Leu Gly Pro Leu Phe
1               5                   10                  15

Pro Val Ser Ala Leu Ser Ala Ala Asp Gln Leu Arg Arg Gly Glu Arg
            20                  25                  30

Lys Glu Thr Thr Lys Ala Glu Ala Pro Arg Lys Glu Glu Lys Lys Asp
        35                  40                  45

Glu Pro Ala Gln Ser Pro Val Glu Ile Ile Lys Asn Ile Ser Tyr Arg
    50                  55                  60

Asp Asp Glu Ala Ala Asp Pro Thr Arg His Lys Leu Asp Leu Tyr Leu
65                  70                  75                  80

Pro Arg Gly Ala Lys Asp Phe Pro Ile Val Phe Phe Val His Gly Gly
                85                  90                  95

Ala Trp Arg Ala Gly Asn Lys Asp Glu Tyr Pro Arg Leu Gly Glu Phe
            100                 105                 110

Phe Ala Thr Glu Gly Ile Gly Cys Val Val Ile Asn Tyr Arg Leu Ser
        115                 120                 125

Pro Lys Val Gln His Pro Ala His Ile Glu Asp Val Ala Arg Ala Phe
    130                 135                 140

Ala Trp Thr Val Ala Asn Ile Glu Lys Tyr Gly Gly Arg Lys Asp Arg
145                 150                 155                 160

Ile Phe Ala Val Gly His Ser Ala Gly Gly His Leu Val Ala Leu Leu
                165                 170                 175

Ala Thr Asp Ala Thr Tyr Leu Lys Asn Glu Gly Leu Ala Leu Thr Asp
            180                 185                 190

Ile Arg Gly Val Ile Pro Ile Ser Gly Val His Gln Ile Asn Ser Ala
        195                 200                 205

Leu Pro Met Phe Arg Asn Ile Phe Ser Arg Asp Lys Glu Glu Cys Arg
    210                 215                 220

Leu Ala Ser Pro Ile Thr His Val Ser Asn Gly His Pro Pro Phe Leu
225                 230                 235                 240

Leu Leu Tyr Ala Glu Asn Asp Leu Pro Leu Leu Gly Arg Met Ala Glu
                245                 250                 255
```

Asp Met Ser Glu Val Leu Lys Lys Asn Gln Cys Asp Val Glu Cys Leu
260 265 270

Lys Ile Asp Gln Arg Asn His Met Thr Ile Ile Thr Gln Leu Ser Gln
275 280 285

Glu Asn Asp Pro Thr Arg Gln Ala Val Phe Asp Phe Ile Gly Arg His
290 295 300

Ser Asp Trp Gln Pro Ala Ala Arg Ala Glu Ala Ala Asn Gly Asn Lys
305 310 315 320

Arg Glu Ala Gly Thr Thr Asp Ala Ala Met Lys Asp Asp Lys
325 330

<210> 43
<211> 1044
<212> DNA
<213> Unknown

<220>
<223> Obtained from environmental sample

<400> 43
atgaaaagat atttctattt gttcttatat cacctcctta ccatacagtt agttttcagt
60

atatcactgg aagcaaaacc gctgcttctt aaaaatgaat ataaagttgc tagggatgta
120

gaatgggcaa aacctgatgg attcagtctg acaatggata tctatacgcc tgaaggtgat
180

aaagataatt atccggtgct gcttatattt cacggcggtg gttggctggc aaacgataaa
240

tctataatga atgatatgag tcagtatgtg gtccgacatg gagactatgt agtgtgtaat
300

attaattata gattgctcaa agaccagaac aataccgtta ccatggacca gatagtggaa
360

gatgccttcg gagcactgct atgggtcaga gagcatgtca gtcaatatgg aggtgatcct
420

gaccggatag ccgtatccgg tgatagtgcc gggggcacc tggcggccat gatcgtaaat
480

gccggtccta atttaagtga gtcgggcctt aaagatggag agaagggatt cacgcccagc
540

tacattcctg aagggatggg tctggatgac ataagggata agggctggat ggaggttcag
600

ggggcagtat tgaattacgc cgctttagat atatatacct tggcgaggaa gggatatgaa
660

gaacaaaaca atagattttg gcagggagca ggagtagaac cccgtggcat tttcggcgat
720

gattataatg tagaggataa tcctgagctg tatcaggcgg tgtctcccgt tcatactctc
780

ccaagcgcta cagagagaag attgcctccc caactactga ctgttggaac agaagatgag
840

cttataccac cgacacaaat cagagagtat aaaaggcttt tggaagaggc agggcatccg
900

gtagagtact gggagcacga aggtagacct catgcttttc tggattcagg agaaaatcct
960

cgtttgggta tcagctttga agaggatgcc cccgaggctt tggataggat tatagctttt
1020

ctagatgcta ttttttacca atag
1044

<210> 44
<211> 347
<212> PRT
<213> Unknown

<220>
<223> Obtained from environmental sample

<220>
<221> DOMAIN
<222> (67)...(315)
<223> alpha/beta hydrolase fold

<220>
<221> SITE
<222> (111)...(114)
<223> N-glycosylation site. Prosite id = PS00001

<220>
<221> SITE
<222> (145)...(157)
<223> Lipolytic enzymes "G-D-X-G" family, putative serine active site. Prosite id = PS01174

<220>
<221> SITE
<222> (166)...(169)
<223> N-glycosylation site. Prosite id = PS00001

<400> 44
Met Lys Arg Tyr Phe Tyr Leu Phe Leu Tyr His Leu Leu Thr Ile Gln
1               5                   10                  15

Leu Val Phe Ser Ile Ser Leu Glu Ala Lys Pro Leu Leu Leu Lys Asn
            20                  25                  30

Glu Tyr Lys Val Ala Arg Asp Val Glu Trp Ala Lys Pro Asp Gly Phe
        35                  40                  45

Ser Leu Thr Met Asp Ile Tyr Thr Pro Glu Gly Asp Lys Asp Asn Tyr
    50                  55                  60

Pro Val Leu Leu Ile Phe His Gly Gly Gly Trp Leu Ala Asn Asp Lys

65 70 75 80

Ser Ile Met Asn Asp Met Ser Gln Tyr Val Val Arg His Gly Asp Tyr
85 90 95

Val Val Cys Asn Ile Asn Tyr Arg Leu Leu Lys Asp Gln Asn Asn Thr
100 105 110

Val Thr Met Asp Gln Ile Val Glu Asp Ala Phe Gly Ala Leu Leu Trp
115 120 125

Val Arg Glu His Val Ser Gln Tyr Gly Gly Asp Pro Asp Arg Ile Ala
130 135 140

Val Ser Gly Asp Ser Ala Gly Gly His Leu Ala Ala Met Ile Val Asn
145 150 155 160

Ala Gly Pro Asn Leu Ser Glu Ser Gly Leu Lys Asp Gly Glu Lys Gly
165 170 175

Phe Thr Pro Ser Tyr Ile Pro Glu Gly Met Gly Leu Asp Asp Ile Arg
180 185 190

Asp Lys Gly Trp Met Glu Val Gln Gly Ala Val Leu Asn Tyr Ala Ala
195 200 205

Leu Asp Ile Tyr Thr Leu Ala Arg Lys Gly Tyr Glu Glu Gln Asn Asn
210 215 220

Arg Phe Trp Gln Gly Ala Gly Val Glu Pro Arg Gly Ile Phe Gly Asp
225 230 235 240

Asp Tyr Asn Val Glu Asp Asn Pro Glu Leu Tyr Gln Ala Val Ser Pro
245 250 255

Val His Thr Leu Pro Ser Ala Thr Glu Arg Arg Leu Pro Pro Gln Leu
260 265 270

Leu Thr Val Gly Thr Glu Asp Glu Leu Ile Pro Pro Thr Gln Ile Arg
275 280 285

Glu Tyr Lys Arg Leu Leu Glu Glu Ala Gly His Pro Val Glu Tyr Trp
290 295 300

Glu His Glu Gly Arg Pro His Ala Phe Leu Asp Ser Gly Glu Asn Pro
305 310 315 320

Arg Leu Gly Ile Ser Phe Glu Glu Asp Ala Pro Glu Ala Leu Asp Arg
325 330 335

Ile Ile Ala Phe Leu Asp Ala Ile Phe Tyr Gln
340 345

<210> 45
<211> 885
<212> DNA
<213> Unknown

<220>
<223> Obtained from environmental sample

<400> 45
atgaaaacaa ccatacaccc aaagctggag cgaacggata tcgtcctgat ggatgacgtc
60

gtctattctc agccgctggc gtattttggt agaagaccac acccgcttaa gatgatgctc
120

ctccgcccga ttgagtggtg gggcgagacg cagctcctgc cgcttccgac gatcctctgg
180

gtcgtgggcg gcgcgtggca gcagacgttt ccctcctggc gcatggcaga gtttagttat
240

ttggcctatg cggggtatca ggtaacggca atcgactacc gaacgatcaa tgaagcggcg
300

tttcccgcgc aagtggagga ctgcaaagct gcggttcgct tcctccgcgc aaacgcaaaa
360

cgctatggcg tagatccgga gcgcatcggc atcatgggcg actcggcggg cggctatctc
420

gctgctatga tcggtgcaac gggagatgat cccgcgtttg aaaccgacga atgggcggga
480

tattccagcg cggtgcaggc tgttgcggac tggtatggcc ctatcgatat gctgcgtatg
540

gggcagtatg ccaaggagca aaacccggat gcgggagaag aggacacgct gctcttccgc
600

ctctttgcgg gctgcccgat cacggaggag aaccataagt ttcttgagag catgagtccg
660

gagcgctatg tttcggcaaa tacgccgccg catctgattt tgcacggcac ggcggacgag
720

atggttgatg tacgcgagag cgagcgttat tacgaggcgc tgacgaaggc gggcgtgcct
780

gccgatttgg ttctgctcga aggggcaggg cattgcgacc aagcattttc acagccggaa
840

gtgcagaata tcatacttga gtttttcacc aaacatttaa agtaa
885

<210> 46
<211> 294
<212> PRT
<213> Unknown

<220>
<223> Obtained from environmental sample

<220>
<221> DOMAIN
<222> (58)...(275)
<223> alpha/beta hydrolase fold

<220>
<221> DOMAIN
<222> (71)...(294)
<223> Prolyl oligopeptidase family

<400> 46
```
Met Lys Thr Thr Ile His Pro Lys Leu Glu Arg Thr Asp Ile Val Leu
1               5                   10                  15

Met Asp Asp Val Val Tyr Ser Gln Pro Leu Ala Tyr Phe Gly Arg Arg
            20                  25                  30

Pro His Pro Leu Lys Met Met Leu Leu Arg Pro Ile Glu Trp Trp Gly
        35                  40                  45

Glu Thr Gln Leu Leu Pro Leu Pro Thr Ile Leu Trp Val Val Gly Gly
    50                  55                  60

Ala Trp Gln Gln Thr Phe Pro Ser Trp Arg Met Ala Glu Phe Ser Tyr
65                  70                  75                  80

Leu Ala Tyr Ala Gly Tyr Gln Val Thr Ala Ile Asp Tyr Arg Thr Ile
                85                  90                  95

Asn Glu Ala Ala Phe Pro Ala Gln Val Glu Asp Cys Lys Ala Ala Val
            100                 105                 110

Arg Phe Leu Arg Ala Asn Ala Lys Arg Tyr Gly Val Asp Pro Glu Arg
        115                 120                 125

Ile Gly Ile Met Gly Asp Ser Ala Gly Gly Tyr Leu Ala Ala Met Ile
    130                 135                 140

Gly Ala Thr Gly Asp Asp Pro Ala Phe Glu Thr Asp Glu Trp Ala Gly
145                 150                 155                 160

Tyr Ser Ser Ala Val Gln Ala Val Ala Asp Trp Tyr Gly Pro Ile Asp
                165                 170                 175

Met Leu Arg Met Gly Gln Tyr Ala Lys Glu Gln Asn Pro Asp Ala Gly
            180                 185                 190

Glu Glu Asp Thr Leu Leu Phe Arg Leu Phe Ala Gly Cys Pro Ile Thr
        195                 200                 205

Glu Glu Asn His Lys Phe Leu Glu Ser Met Ser Pro Glu Arg Tyr Val
    210                 215                 220
```

Ser Ala Asn Thr Pro Pro His Leu Ile Leu His Gly Thr Ala Asp Glu
225 230 235 240

Met Val Asp Val Arg Glu Ser Glu Arg Tyr Tyr Glu Ala Leu Thr Lys
245 250 255

Ala Gly Val Pro Ala Asp Leu Val Leu Leu Glu Gly Ala Gly His Cys
260 265 270

Asp Gln Ala Phe Ser Gln Pro Glu Val Gln Asn Ile Ile Leu Glu Phe
275 280 285

Phe Thr Lys His Leu Lys
290

<210> 47
<211> 960
<212> DNA
<213> Unknown

<220>
<223> Obtained from environmental sample

<400> 47
atgcgcggtt tcgcgcgttc aatcactttt cgtttggtgt tcgctgcgac tgcgttcgtc
60

gttgcgctgg caacgttggc ccaggcgcaa aacgacaaga tgacacccat cgccacaccg
120

gcgcagccca atgcgatcga gatcggaacc ggtccgctgc ccgatgcgaa gaaccccgag
180

tcctggcata gccagtacgg cagcaagttt gcccgtaacg taaccatagc gacgctgaca
240

ccatttctgc cggatgctgc cagggcaagc ggcgccgcag tcatcgtcgc gcccggcggc
300

ggtttccgca ccttgtcgat ggaaaatgaa ggctggaacg ttgcgcgcgc cctggctgaa
360

aagggcgtcg ctgcgttcgt cctgaagtat cgattgaatc agaccccggc ggacatgccg
420

ggcttcgagc agtccatgcg agaaatgttt tcgggaacag cgcgaccgcc gcgccccgac
480

ccggcgaagg ctattgccgg tcttgctccg caaatcgccg acgcccgcgc ggcatttgca
540

ttaatccgca agcgttcttc cgaatggcac gtcgatccta accggatcgg catggttggt
600

ttttccgccg gcgcgatgtt gaccatggcg accgcgctcg cgggcgagga tgcgaagccg
660

gccttcatcg gcaacatcta cggtccgctc gcgtcagtca ccgtgccggc tgacgcgccc
720

ccattgttca tcgcgcttgc tgccgacgat cctttcttcg cgaacggcgg ctatgggctc 780

atcgatagct ggaagacggc gaagcggccc gttgaattcc atctttatga acagggtggg 840

catggcttcg ggatgtatcc gaaggaaacg acgagcaccg gctggttcga agccttcgtg 900

cggtggatag gaatgcacgg catgctgaag ccggcagcgt caggaagcgg cgcgaagtag 960

<210> 48
<211> 319
<212> PRT
<213> Unknown

<220>
<223> Obtained from environmental sample

<220>
<221> SIGNAL
<222> (1)...(29)

<220>
<221> DOMAIN
<222> (94)...(285)
<223> alpha/beta hydrolase fold

<220>
<221> SITE
<222> (74)...(77)
<223> N-glycosylation site. Prosite id = PS00001

<400> 48
Met Arg Gly Phe Ala Arg Ser Ile Thr Phe Arg Leu Val Phe Ala Ala
1 5 10 15

Thr Ala Phe Val Val Ala Leu Ala Thr Leu Ala Gln Ala Gln Asn Asp
20 25 30

Lys Met Thr Pro Ile Ala Thr Pro Ala Gln Pro Asn Ala Ile Glu Ile
35 40 45

Gly Thr Gly Pro Leu Pro Asp Ala Lys Asn Pro Glu Ser Trp His Ser
50 55 60

Gln Tyr Gly Ser Lys Phe Ala Arg Asn Val Thr Ile Ala Thr Leu Thr
65 70 75 80

Pro Phe Leu Pro Asp Ala Ala Arg Ala Ser Gly Ala Ala Val Ile Val
85 90 95

Ala Pro Gly Gly Gly Phe Arg Thr Leu Ser Met Glu Asn Glu Gly Trp
100 105 110

Asn Val Ala Arg Ala Leu Ala Glu Lys Gly Val Ala Ala Phe Val Leu
115 120 125

Lys Tyr Arg Leu Asn Gln Thr Pro Ala Asp Met Pro Gly Phe Glu Gln
    130                 135                 140

Ser Met Arg Glu Met Phe Ser Gly Thr Ala Arg Pro Pro Arg Pro Asp
145                 150                 155                 160

Pro Ala Lys Ala Ile Ala Gly Leu Ala Pro Gln Ile Ala Asp Ala Arg
                165                 170                 175

Ala Ala Phe Ala Leu Ile Arg Lys Arg Ser Ser Glu Trp His Val Asp
            180                 185                 190

Pro Asn Arg Ile Gly Met Val Gly Phe Ser Ala Gly Ala Met Leu Thr
        195                 200                 205

Met Ala Thr Ala Leu Ala Gly Glu Asp Ala Lys Pro Ala Phe Ile Gly
    210                 215                 220

Asn Ile Tyr Gly Pro Leu Ala Ser Val Thr Val Pro Ala Asp Ala Pro
225                 230                 235                 240

Pro Leu Phe Ile Ala Leu Ala Ala Asp Asp Pro Phe Phe Ala Asn Gly
                245                 250                 255

Gly Tyr Gly Leu Ile Asp Ser Trp Lys Thr Ala Lys Arg Pro Val Glu
            260                 265                 270

Phe His Leu Tyr Glu Gln Gly Gly His Gly Phe Gly Met Tyr Pro Lys
        275                 280                 285

Glu Thr Thr Ser Thr Gly Trp Phe Glu Ala Phe Val Arg Trp Ile Gly
    290                 295                 300

Met His Gly Met Leu Lys Pro Ala Ala Ser Gly Ser Gly Ala Lys
305                 310                 315

<210> 49
<211> 897
<212> DNA
<213> Unknown

<220>
<223> Obtained from environmental sample

<400> 49
atgccgcaac aaaacccgcc gccggaaacc cccattgaca aatcggcccg ggaacgcatc
60

ggcgaaacca ggaaagcatt cctgaatatg acggcggagc ttgcgacttc gctggcggac
120

gttccgacgg cggagtatga ccaggagggt atccggggaa gctggatcat gcccgataaa

180

gcggattccg aacgcgtgct tttgtatctt cacggcggcg gttacatagt tggaagcgat
240

gaaacgcctc gcgcgataac cgcatttctg gcgcgcgagg caaaagtgcg gtgtttttcc
300

ctggattatc cgcttgctcc cgagcatcca tttccggctg cgctcgatag cgcggtgcga
360

tcgtatgaaa tgctgttgga aaaggggttc agccccggaa acatcgtgct gggcggcgac
420

tcggccggcg gcggcctggc gcttgccctg ttgctcgcca tccgggaaca cgggctgccg
480

atgccggcgg gagcgtatct cctttccccg tggactgatt tgacgcagag ttttcccacg
540

catttacgca aggccgatgt cgaaatcagt attgcccccg agctgctcga ggaagcggca
600

gccagctatg cgggcgattc cgatagaacc aatccgctta tctcccccgc attcggcgag
660

ttccggggat tcccgccgct cctgatacaa gttggttccc atgaacgtct gttggacgat
720

tccctgacgg tggcgcgcaa tgccgccctc gccgatgtgc cgacgacatt gaaggtatgg
780

cccggctatc cccatgtgtt tcaactctat catcagaatc tcgacggcgc caaaaaggcg
840

ttgagggaag ccgcggaatt catcaccgga gcgatggata aagagttgct tcaataa
897

<210> 50
<211> 298
<212> PRT
<213> Unknown

<220>
<223> Obtained from environmental sample

<220>
<221> DOMAIN
<222> (67)...(269)
<223> alpha/beta hydrolase fold

<220>
<221> SITE
<222> (29)...(32)
<223> N-glycosylation site. Prosite id = PS00001

<400> 50
Met Pro Gln Gln Asn Pro Pro Pro Glu Thr Pro Ile Asp Lys Ser Ala
1 5 10 15

Arg Glu Arg Ile Gly Glu Thr Arg Lys Ala Phe Leu Asn Met Thr Ala
20 25 30

Glu Leu Ala Thr Ser Leu Ala Asp Val Pro Thr Ala Glu Tyr Asp Gln

```
        35                  40                  45

Glu Gly Ile Arg Gly Ser Trp Ile Met Pro Asp Lys Ala Asp Ser Glu
    50                  55                  60

Arg Val Leu Leu Tyr Leu His Gly Gly Gly Tyr Ile Val Gly Ser Asp
65                  70                  75                  80

Glu Thr Pro Arg Ala Ile Thr Ala Phe Leu Ala Arg Glu Ala Lys Val
                85                  90                  95

Arg Cys Phe Ser Leu Asp Tyr Pro Leu Ala Pro Glu His Pro Phe Pro
            100                 105                 110

Ala Ala Leu Asp Ser Ala Val Arg Ser Tyr Glu Met Leu Leu Glu Lys
        115                 120                 125

Gly Phe Ser Pro Gly Asn Ile Val Leu Gly Gly Asp Ser Ala Gly Gly
    130                 135                 140

Gly Leu Ala Leu Ala Leu Leu Leu Ala Ile Arg Glu His Gly Leu Pro
145                 150                 155                 160

Met Pro Ala Gly Ala Tyr Leu Leu Ser Pro Trp Thr Asp Leu Thr Gln
                165                 170                 175

Ser Phe Pro Thr His Leu Arg Lys Ala Asp Val Glu Ile Ser Ile Ala
            180                 185                 190

Pro Glu Leu Leu Glu Glu Ala Ala Ala Ser Tyr Ala Gly Asp Ser Asp
        195                 200                 205

Arg Thr Asn Pro Leu Ile Ser Pro Ala Phe Gly Glu Phe Arg Gly Phe
    210                 215                 220

Pro Pro Leu Leu Ile Gln Val Gly Ser His Glu Arg Leu Leu Asp Asp
225                 230                 235                 240

Ser Leu Thr Val Ala Arg Asn Ala Ala Leu Ala Asp Val Pro Thr Thr
                245                 250                 255

Leu Lys Val Trp Pro Gly Tyr Pro His Val Phe Gln Leu Tyr His Gln
            260                 265                 270

Asn Leu Asp Gly Ala Lys Lys Ala Leu Arg Glu Ala Ala Glu Phe Ile
        275                 280                 285

Thr Gly Ala Met Asp Lys Glu Leu Leu Gln
    290                 295
```

<210> 51
<211> 1071
<212> DNA
<213> Unknown

<220>
<223> Obtained from environmental sample

<400> 51
atgacagcat ttaatcaaaa aattcaaaca ttgctagaaa agggccaagg gcctgctgct
60

cgagctcttg ataagttacc tcgattagct caagaatcac ttgctaaaat tcttggatat
120

tcttaccaat atcccgatct cgactcattt actaaatgta tgatggctgt acaaattaaa
180

cagggtcata tcggtttcat tggttcagac ccaattgaat ctagaaggca gttcgatacg
240

caaatgctgg ctatacgcca caagtcaaca gagatcgact tagtagaaga tatccgttta
300

cctctccaaa gtggaaccgt ttttgcgaga cactatcacc cagcaccgaa taagaaatta
360

cctcttattg ttttctatca tggaggagga tttgttgttg gtggtttaga tactcatgat
420

gaggtttgtc gtatattggc aaaatatgca aaagtacaag tcctcagtat cgattaccct
480

ctagcacctg aggtttctcc tcagcattta atccaatctt gtgaagatgc tttagcttgg
540

gtatatcaaa atcgtagaca gttaaaaata ttaaaaggtc gaattgcggt agcgggggat
600

agtgctggtg gaaacattag tacagtcgtg gctcaaagat cagtaaataa accttatgct
660

cctcaggccc aacttcttat ttatcctgcc gtcgatttca agagtcgaca tccttccttc
720

tatgcatata acgaaggatt ggttttaacg gataacgata tcaataatgt cactcaatat
780

tatgcaaccc agcataacgt agaactagat gatccaatta tttctccaac ttatggcaac
840

ttgaagaaga atcctccggc ttttgtgatt actgctgggc atgatgtatt acatgatgaa
900

ggcaaaattt atagctataa gctacgccaa aatggtgtca aaatgcatta tgaagaatat
960

tcagatcaaa ctcatggttt tataaatttg acgcctattt ccaaaaaagc caaacggtac
1020

accattgagt tcagtaaaaa ctttagaaaa ttttgggata aaaatagcta a
1071

<210> 52
<211> 356

<212> PRT
<213> Unknown

<220>
<223> Obtained from environmental sample

<220>
<221> DOMAIN
<222> (123)...(329)
<223> alpha/beta hydrolase fold

<220>
<221> SITE
<222> (208)...(211)
<223> N-glycosylation site. Prosite id = PS00001

<220>
<221> SITE
<222> (259)...(262)
<223> N-glycosylation site. Prosite id = PS00001

<400> 52

```
Met Thr Ala Phe Asn Gln Lys Ile Gln Thr Leu Leu Glu Lys Gly Gln
1               5                   10                  15

Gly Pro Ala Ala Arg Ala Leu Asp Lys Leu Pro Arg Leu Ala Gln Glu
            20                  25                  30

Ser Leu Ala Lys Ile Leu Gly Tyr Ser Tyr Gln Tyr Pro Asp Leu Asp
        35                  40                  45

Ser Phe Thr Lys Cys Met Met Ala Val Gln Ile Lys Gln Gly His Ile
    50                  55                  60

Gly Phe Ile Gly Ser Asp Pro Ile Glu Ser Arg Arg Gln Phe Asp Thr
65                  70                  75                  80

Gln Met Leu Ala Ile Arg His Lys Ser Thr Glu Ile Asp Leu Val Glu
                85                  90                  95

Asp Ile Arg Leu Pro Leu Gln Ser Gly Thr Val Phe Ala Arg His Tyr
            100                 105                 110

His Pro Ala Pro Asn Lys Lys Leu Pro Leu Ile Val Phe Tyr His Gly
        115                 120                 125

Gly Gly Phe Val Val Gly Gly Leu Asp Thr His Asp Glu Val Cys Arg
    130                 135                 140

Ile Leu Ala Lys Tyr Ala Lys Val Gln Val Leu Ser Ile Asp Tyr Pro
145                 150                 155                 160

Leu Ala Pro Glu Val Ser Pro Gln His Leu Ile Gln Ser Cys Glu Asp
                165                 170                 175
```

```
Ala Leu Ala Trp Val Tyr Gln Asn Arg Arg Gln Leu Lys Ile Leu Lys
            180                 185                 190

Gly Arg Ile Ala Val Ala Gly Asp Ser Ala Gly Gly Asn Ile Ser Thr
        195                 200                 205

Val Val Ala Gln Arg Ser Val Asn Lys Pro Tyr Ala Pro Gln Ala Gln
    210                 215                 220

Leu Leu Ile Tyr Pro Ala Val Asp Phe Lys Ser Arg His Pro Ser Phe
225                 230                 235                 240

Tyr Ala Tyr Asn Glu Gly Leu Val Leu Thr Asp Asn Asp Ile Asn Asn
                245                 250                 255

Val Thr Gln Tyr Tyr Ala Thr Gln His Asn Val Glu Leu Asp Asp Pro
            260                 265                 270

Ile Ile Ser Pro Thr Tyr Gly Asn Leu Lys Lys Asn Pro Pro Ala Phe
        275                 280                 285

Val Ile Thr Ala Gly His Asp Val Leu His Asp Glu Gly Lys Ile Tyr
    290                 295                 300

Ser Tyr Lys Leu Arg Gln Asn Gly Val Lys Met His Tyr Glu Glu Tyr
305                 310                 315                 320

Ser Asp Gln Thr His Gly Phe Ile Asn Leu Thr Pro Ile Ser Lys Lys
                325                 330                 335

Ala Lys Arg Tyr Thr Ile Glu Phe Ser Lys Asn Phe Arg Lys Phe Trp
            340                 345                 350

Asp Lys Asn Ser
        355

<210> 53
<211> 1047
<212> DNA
<213> Unknown

<220>
<223> Obtained from environmental sample

<400> 53
atgagaaaga aacaagtgag tatcagggga aatctgattc gggaaatgat gcgggatgtg
60

atgggaacgg ctctcggcaa gccgattcag tccggggaga ttagaaggca tccggttgag
120

ccggcctgga tctgtccgcc gggatttatc tatgaactga tcgatatgga acatttcaag
180
```

atggaatatc tgcagcctca ggatgtttat accgggcggg tggtgcttca gctgcatggt 240

ggcggatata ttggcccgat gaagaatatt taccggagct ttgctgtgaa atattcaaga 300

ttaagtatgt atggagatgt tttgacgatt gattaccggg tggcgccgga acatccatat 360

ccggcggcgc tggaggatgc gattgctgcg tatcggtggc ttttggatga aaaaaactat 420

cggccggagc aaatcgtggt agcaggagat tccgccggtg gcggcctggc actggcgttg 480

tgtctgtatc tccgggatca tcagattccg ctgccggccg gtatcatagc catgtcgccc 540

tggacggatc tgacactaag tggggagtct tatacatcta attatgacca cgatccgctg 600

tttggtaata ccagagaaag tatgttgtat aattcaactt atattgggga cagcgatccg 660

acggatccgt atatgtctcc gttgtttgga gagtattttg gattcccgcc ggtgttattg 720

caggtggggg atattgaggt gcttctcagc gatacacttc gggtttctga aaaattgcgg 780

gaggctgggg taaagcagcg gctttcggtt tatgaaggca tgtttcattc gtttcagatg 840

ggactggatc tgattccgga gagccgtgaa gcctgggaag aagtggaacg atttatcggg 900

attgtctgcc agattgagcg gaaagctgaa gggaaaattg ttcgccgggt gaaggggaaa 960

acagagaata aagatgaaaa tcacattggt tacggttgga aaaataaaag aaaaatttta 1020

ccgggaggca atttcggaat acagtaa 1047

<210> 54
<211> 348
<212> PRT
<213> Unknown

<220>
<223> Obtained from environmental sample

<220>
<221> DOMAIN
<222> (75)...(280)
<223> alpha/beta hydrolase fold

<220>
<221> SITE
<222> (147)...(159)
<223> Lipolytic enzymes "G-D-X-G" family, putative serine active site. Prosite id = PS01174

<220>
<221> SITE

<222> (214)...(217)
<223> N-glycosylation site. Prosite id = PS00001

<400> 54
Met Arg Lys Lys Gln Val Ser Ile Arg Gly Asn Leu Ile Arg Glu Met
1               5                   10                  15

Met Arg Asp Val Met Gly Thr Ala Leu Gly Lys Pro Ile Gln Ser Gly
            20                  25                  30

Glu Ile Arg Arg His Pro Val Glu Pro Ala Trp Ile Cys Pro Pro Gly
        35                  40                  45

Phe Ile Tyr Glu Leu Ile Asp Met Glu His Phe Lys Met Glu Tyr Leu
    50                  55                  60

Gln Pro Gln Asp Val Tyr Thr Gly Arg Val Val Leu Gln Leu His Gly
65                  70                  75                  80

Gly Gly Tyr Ile Gly Pro Met Lys Asn Ile Tyr Arg Ser Phe Ala Val
                85                  90                  95

Lys Tyr Ser Arg Leu Ser Met Tyr Gly Asp Val Leu Thr Ile Asp Tyr
            100                 105                 110

Arg Val Ala Pro Glu His Pro Tyr Pro Ala Ala Leu Glu Asp Ala Ile
        115                 120                 125

Ala Ala Tyr Arg Trp Leu Leu Asp Glu Lys Asn Tyr Arg Pro Glu Gln
    130                 135                 140

Ile Val Val Ala Gly Asp Ser Ala Gly Gly Gly Leu Ala Leu Ala Leu
145                 150                 155                 160

Cys Leu Tyr Leu Arg Asp His Gln Ile Pro Leu Pro Ala Gly Ile Ile
                165                 170                 175

Ala Met Ser Pro Trp Thr Asp Leu Thr Leu Ser Gly Glu Ser Tyr Thr
            180                 185                 190

Ser Asn Tyr Asp His Asp Pro Leu Phe Gly Asn Thr Arg Glu Ser Met
        195                 200                 205

Leu Tyr Asn Ser Thr Tyr Ile Gly Asp Ser Asp Pro Thr Asp Pro Tyr
    210                 215                 220

Met Ser Pro Leu Phe Gly Glu Tyr Phe Gly Phe Pro Pro Val Leu Leu
225                 230                 235                 240

Gln Val Gly Asp Ile Glu Val Leu Leu Ser Asp Thr Leu Arg Val Ser
                245                 250                 255

Glu Lys Leu Arg Glu Ala Gly Val Lys Gln Arg Leu Ser Val Tyr Glu
260 265 270

Gly Met Phe His Ser Phe Gln Met Gly Leu Asp Leu Ile Pro Glu Ser
275 280 285

Arg Glu Ala Trp Glu Glu Val Glu Arg Phe Ile Gly Ile Val Cys Gln
290 295 300

Ile Glu Arg Lys Ala Glu Gly Lys Ile Val Arg Arg Val Lys Gly Lys
305 310 315 320

Thr Glu Asn Lys Asp Glu Asn His Ile Gly Tyr Gly Trp Lys Asn Lys
325 330 335

Arg Lys Ile Leu Pro Gly Gly Asn Phe Gly Ile Gln
340 345

<210> 55
<211> 948
<212> DNA
<213> Unknown

<220>
<223> Obtained from environmental sample

<400> 55
atgagtcttc atcctcaagc aaaagctttg cttgcggtgc ttgaatcgag cccgcctatc 60

gaacagcaaa ccgttgaaga agccaggcaa gcatatgcga aaatgtgcag ttattttaat 120

aaacgtgaag atgtttatag agtagaagac aggcaaataa aaggattcca gcagaatatt 180

tcgatacgca tctatacccc agacgataaa gccattcatc cggcgctcgt ttattttcat 240

ggcggcggct gggttatcgg cgacttggaa acccatgatg ccctttgccg ttctttggca 300

aataaatctg gagcggctgt tgtttcagtg gattatagcc tttcacccga atatagattt 360

ccagttgcca tcgaagattc gtatctggca gttaaatggg tggcagatca tgcagaagag 420

cttggaatcg acaaaaccat gattgccgtt ggcggtgaca gtgcaggcgg taatctcgcg 480

actgtcgttt gccatttagc gaatcaaaga aaagcccctg ccatcagcta tcaaatgctg 540

ttttaccctt caaccggata tgagcgaaca ccgtctatcg ataaatatgg tgaaggttat 600

catcttacga attcaacaat gaagtggttt caacaacaat acttatccca acctgaagac

660

ctgcgtaacc ccttggccgc ccctatgtta ctgtcagaat ctgaaattgc cttattaccg
720

ccagcttatg tcttaactgc cgaatatgac cccctatgtg atggcggaga aatgtacgcc
780

aaaaaattgg aacatgcggg agtagaagta acatacgtct gctacccggg aatgatccat
840

ggcttcctga cgatgtcgga gccgttggat gattgcaagc gagcgattaa tgaagcggca
900

caaaacttga aagcccattt tacaaagaca gttgccgtaa acgagtaa
948

<210> 56
<211> 315
<212> PRT
<213> Unknown

<220>
<223> Obtained from environmental sample

<220>
<221> DOMAIN
<222> (76)...(284)
<223> alpha/beta hydrolase fold

<220>
<221> SITE
<222> (59)...(62)
<223> N-glycosylation site. Prosite id = PS00001

<220>
<221> SITE
<222> (102)...(105)
<223> N-glycosylation site. Prosite id = PS00001

<220>
<221> SITE
<222> (207)...(210)
<223> N-glycosylation site. Prosite id = PS00001

<400> 56
Met Ser Leu His Pro Gln Ala Lys Ala Leu Leu Ala Val Leu Glu Ser
1 5 10 15

Ser Pro Pro Ile Glu Gln Gln Thr Val Glu Glu Ala Arg Gln Ala Tyr
20 25 30

Ala Lys Met Cys Ser Tyr Phe Asn Lys Arg Glu Asp Val Tyr Arg Val
35 40 45

Glu Asp Arg Gln Ile Lys Gly Phe Gln Gln Asn Ile Ser Ile Arg Ile
50 55 60

Tyr Thr Pro Asp Asp Lys Ala Ile His Pro Ala Leu Val Tyr Phe His
65 70 75 80

```
Gly Gly Gly Trp Val Ile Gly Asp Leu Glu Thr His Asp Ala Leu Cys
                85                  90                  95

Arg Ser Leu Ala Asn Lys Ser Gly Ala Ala Val Val Ser Val Asp Tyr
            100                 105                 110

Ser Leu Ser Pro Glu Tyr Arg Phe Pro Val Ala Ile Glu Asp Ser Tyr
        115                 120                 125

Leu Ala Val Lys Trp Val Ala Asp His Ala Glu Glu Leu Gly Ile Asp
    130                 135                 140

Lys Thr Met Ile Ala Val Gly Gly Asp Ser Ala Gly Gly Asn Leu Ala
145                 150                 155                 160

Thr Val Val Cys His Leu Ala Asn Gln Arg Lys Ala Pro Ala Ile Ser
                165                 170                 175

Tyr Gln Met Leu Phe Tyr Pro Ser Thr Gly Tyr Glu Arg Thr Pro Ser
            180                 185                 190

Ile Asp Lys Tyr Gly Glu Gly Tyr His Leu Thr Asn Ser Thr Met Lys
        195                 200                 205

Trp Phe Gln Gln Gln Tyr Leu Ser Gln Pro Glu Asp Leu Arg Asn Pro
    210                 215                 220

Leu Ala Ala Pro Met Leu Leu Ser Glu Ser Glu Ile Ala Leu Leu Pro
225                 230                 235                 240

Pro Ala Tyr Val Leu Thr Ala Glu Tyr Asp Pro Leu Cys Asp Gly Gly
                245                 250                 255

Glu Met Tyr Ala Lys Lys Leu Glu His Ala Gly Val Glu Val Thr Tyr
            260                 265                 270

Val Cys Tyr Pro Gly Met Ile His Gly Phe Leu Thr Met Ser Glu Pro
        275                 280                 285

Leu Asp Asp Cys Lys Arg Ala Ile Asn Glu Ala Ala Gln Asn Leu Lys
    290                 295                 300

Ala His Phe Thr Lys Thr Val Ala Val Asn Glu
305                 310                 315
```

<210> 57
<211> 927
<212> DNA
<213> Unknown

<220>

<223> Obtained from environmental sample

<400> 57
atgtcacttg atccacaaac gaaatttgta ttagcccaat tggctgctgc cgatgctcct
60

ccaatggaga ctttgtcacc ggaaatggct agacaggcat tcatattgcc gcaaggtgca
120

gtggaggaag tagggaaggt agaaaatcga acgattcctg gtcctgcaac ggacatccct
180

gtccgtgttt attaccctaa agaactccag cctgaaaatc ccgcgctagt tttctatcat
240

ggcggcggct gggtaattgg caatttggac tctcatgacg atatttgccg tgctttaacc
300

aacctcgcca actgcgtgac catttccgtc gactaccgcc tggctccaga gaataaattt
360

cctgctgcgg ttgaggatgc ttatgctgct gcacaatatg tgtatgacca tgcagaagat
420

ttcaaagtcg acaagacccg tattgccgtt ggcggtgaca gtgccggtgg aaatcttgcc
480

gctgtcgtga cgaatctggc aaaagacaaa aactcacctt ctatttgttt ccaacttctg
540

atttatccaa gcaccaatgc aggtggtgag ccaacagcat caatggttga gaatgcccat
600

ggctatttct tagaaaaagg cacgatggat tggttccgtg actgctacct gaacagtgaa
660

gaagacaaac agaatccgct agtctcaccg atgctttatg atgatttcca aggactgcct
720

ccagcaattg tgattactgc cgagtacgat ccattgcgag atgaaggcga agcatatgcc
780

aaaaagctgg gcgaagcagg ggttgctgtc caaaccatcc gctttgacgg tacgattcat
840

ggctttgtca gcatgtcggc tgtcattagc caagggaagg cggcattgga aaaagcagga
900

gaggctttaa ctaaagcctt tcaataa
927

<210> 58
<211> 308
<212> PRT
<213> Unknown

<220>
<223> Obtained from environmental sample

<220>
<221> DOMAIN
<222> (76)...(284)
<223> alpha/beta hydrolase fold

<220>
<221> SITE

<222> (49)...(52)
<223> N-glycosylation site. Prosite id = PS00001

<400> 58
Met Ser Leu Asp Pro Gln Thr Lys Phe Val Leu Ala Gln Leu Ala Ala
1 5 10 15

Ala Asp Ala Pro Pro Met Glu Thr Leu Ser Pro Glu Met Ala Arg Gln
20 25 30

Ala Phe Ile Leu Pro Gln Gly Ala Val Glu Glu Val Gly Lys Val Glu
35 40 45

Asn Arg Thr Ile Pro Gly Pro Ala Thr Asp Ile Pro Val Arg Val Tyr
50 55 60

Tyr Pro Lys Glu Leu Gln Pro Glu Asn Pro Ala Leu Val Phe Tyr His
65 70 75 80

Gly Gly Gly Trp Val Ile Gly Asn Leu Asp Ser His Asp Asp Ile Cys
85 90 95

Arg Ala Leu Thr Asn Leu Ala Asn Cys Val Thr Ile Ser Val Asp Tyr
100 105 110

Arg Leu Ala Pro Glu Asn Lys Phe Pro Ala Ala Val Glu Asp Ala Tyr
115 120 125

Ala Ala Ala Gln Tyr Val Tyr Asp His Ala Glu Asp Phe Lys Val Asp
130 135 140

Lys Thr Arg Ile Ala Val Gly Gly Asp Ser Ala Gly Gly Asn Leu Ala
145 150 155 160

Ala Val Val Thr Asn Leu Ala Lys Asp Lys Asn Ser Pro Ser Ile Cys
165 170 175

Phe Gln Leu Leu Ile Tyr Pro Ser Thr Asn Ala Gly Gly Glu Pro Thr
180 185 190

Ala Ser Met Val Glu Asn Ala His Gly Tyr Phe Leu Glu Lys Gly Thr
195 200 205

Met Asp Trp Phe Arg Asp Cys Tyr Leu Asn Ser Glu Glu Asp Lys Gln
210 215 220

Asn Pro Leu Val Ser Pro Met Leu Tyr Asp Asp Phe Gln Gly Leu Pro
225 230 235 240

Pro Ala Ile Val Ile Thr Ala Glu Tyr Asp Pro Leu Arg Asp Glu Gly
245 250 255

Glu Ala Tyr Ala Lys Lys Leu Gly Glu Ala Gly Val Ala Val Gln Thr
260 265 270

Ile Arg Phe Asp Gly Thr Ile His Gly Phe Val Ser Met Ser Ala Val
275 280 285

Ile Ser Gln Gly Lys Ala Ala Leu Glu Lys Ala Gly Glu Ala Leu Thr
290 295 300

Lys Ala Phe Gln
305

<210> 59
<211> 1071
<212> DNA
<213> Unknown

<220>
<223> Obtained from environmental sample

<400> 59
atgggtgcga ataatccggt cacaaaattt gtaatggata aggggcaggg cactgcagca
60

cgtttgctgg attgcctgcc gagctttgca caagaacgtc tggtaaaagc attggattat
120

ccttatgact atcctgattt ggatccttat ataaaatgct tgatggcgat tcagattaaa
180

cagggagagc acagttttat tagtgcagat gcggtgcagt cacgtcaact gtttgatgaa
240

cgcatgaaag ctattcaggc gaaaccgacg ccggtcaagg cagtcgagga tctgcgtttg
300

ccattgcaaa atggcactat ctttgcccga cattatcatc cggcaccaca gaaaaaattg
360

ccgatggtca ttttctatca tggtggtgca tttatagtgg gtggtctgga tacgcatgat
420

gagttctgcc gtttattggc ggtgcatgcc aaggtgcagg tactcagcgt ggcttatccg
480

ctaacgcccg aatacagtcc tttgcagatg gtacaggtct gtgaagatgc tctggcttgg
540

gtacatcaaa acatcaagca gttgaaaatc tataaaaacc agattgtagt ggcaggggat
600

agtgcaggtg gaaatctggc ggcagtggtt gcgcagcgga gtgccgataa aatctatgca
660

cctcgcgcac agttattgct ttacccggct gtcgatttta aaagccgcca tccttctttt
720

tatgcataca atcagggcct ggtactttca gctcaggata ttgatctggt gaccaagcta
780

tatgctgaaa cacatcaggt cgaactggat gatccgctga tttcaccgac ctatggtgaa
840

ctgaagaatc tgccgcctgc ctatgtgatt accgcccgtc atgatgtgtt gcatgatgag
900

ggttcaatct atgcgctgaa gttacgtgag aatggggtgc gagtgtatta tcaggagtat
960

acggatcagg cccacggttt tatcaatctg accccaattc ataaacgttc gaaaaagcag
1020

gtcattgaac tcagcaagaa tttccgtaaa ttcctggata aaaagatctg a
1071

<210> 60
<211> 356
<212> PRT
<213> Unknown

<220>
<223> Obtained from environmental sample

<220>
<221> DOMAIN
<222> (123)...(329)
<223> alpha/beta hydrolase fold

<220>
<221> SITE
<222> (105)...(108)
<223> N-glycosylation site. Prosite id = PS00001

<400> 60
Met Gly Ala Asn Asn Pro Val Thr Lys Phe Val Met Asp Lys Gly Gln
1 5 10 15

Gly Thr Ala Ala Arg Leu Leu Asp Cys Leu Pro Ser Phe Ala Gln Glu
20 25 30

Arg Leu Val Lys Ala Leu Asp Tyr Pro Tyr Asp Tyr Pro Asp Leu Asp
35 40 45

Pro Tyr Ile Lys Cys Leu Met Ala Ile Gln Ile Lys Gln Gly Glu His
50 55 60

Ser Phe Ile Ser Ala Asp Ala Val Gln Ser Arg Gln Leu Phe Asp Glu
65 70 75 80

Arg Met Lys Ala Ile Gln Ala Lys Pro Thr Pro Val Lys Ala Val Glu
85 90 95

Asp Leu Arg Leu Pro Leu Gln Asn Gly Thr Ile Phe Ala Arg His Tyr
100 105 110

His Pro Ala Pro Gln Lys Lys Leu Pro Met Val Ile Phe Tyr His Gly
115 120 125

```
Gly Ala Phe Ile Val Gly Gly Leu Asp Thr His Asp Glu Phe Cys Arg
    130                 135                 140

Leu Leu Ala Val His Ala Lys Val Gln Val Leu Ser Val Ala Tyr Pro
145                 150                 155                 160

Leu Thr Pro Glu Tyr Ser Pro Leu Gln Met Val Gln Val Cys Glu Asp
                165                 170                 175

Ala Leu Ala Trp Val His Gln Asn Ile Lys Gln Leu Lys Ile Tyr Lys
            180                 185                 190

Asn Gln Ile Val Val Ala Gly Asp Ser Ala Gly Gly Asn Leu Ala Ala
        195                 200                 205

Val Val Ala Gln Arg Ser Ala Asp Lys Ile Tyr Ala Pro Arg Ala Gln
    210                 215                 220

Leu Leu Leu Tyr Pro Ala Val Asp Phe Lys Ser Arg His Pro Ser Phe
225                 230                 235                 240

Tyr Ala Tyr Asn Gln Gly Leu Val Leu Ser Ala Gln Asp Ile Asp Leu
                245                 250                 255

Val Thr Lys Leu Tyr Ala Glu Thr His Gln Val Glu Leu Asp Asp Pro
            260                 265                 270

Leu Ile Ser Pro Thr Tyr Gly Glu Leu Lys Asn Leu Pro Pro Ala Tyr
        275                 280                 285

Val Ile Thr Ala Arg His Asp Val Leu His Asp Glu Gly Ser Ile Tyr
    290                 295                 300

Ala Leu Lys Leu Arg Glu Asn Gly Val Arg Val Tyr Tyr Gln Glu Tyr
305                 310                 315                 320

Thr Asp Gln Ala His Gly Phe Ile Asn Leu Thr Pro Ile His Lys Arg
                325                 330                 335

Ser Lys Lys Gln Val Ile Glu Leu Ser Lys Asn Phe Arg Lys Phe Leu
            340                 345                 350

Asp Lys Lys Ile
        355
```

<210> 61
<211> 750
<212> DNA
<213> Unknown

<220>

<223> Obtained from environmental sample

<400> 61
atgtcaggca agtacactgt tttggaaggc gctgaaccgt tttattttga aggaagcaga 60

gttgggattc ttgtgtcgca tgggtttacc ggcagcaccc aaagcatgag gccgcttggg 120

gaagcctatg caaaggcagg ttatacggtt tgcggcccgc gtctgaaggg gcatggcacc 180

cattacgagg agatggaaca gactacatat aacgattgga ttgattcagt cgaggaaggc 240

taccagtggc tgaaggaacg ctgcgataca atatttgtaa ctggtttgtc catgggcgga 300

acccttaccc tgtatatggc tgaaaatcat cctgaaatta aggcaatcat accaattaat 360

gcagctattc aaatttccga catggccgct gcagccaacc ttgatggagt gcgattcctt 420

gatgcgattg gttccgacat taagaagcag ggtgaaaaag agctcgctta cgagaaaact 480

cctgtaaagt cccttggtga gatcgtagcg ttaatggaaa aggttaaagc tgacctttcc 540

gatatcactt gtccagcact tatttttgtt tcaaaggaag atcacgttgt gccgccagat 600

aactcaagga ttatcaagga acaaatccgt tctgccgata agagcctgat tgagctgtca 660

gaaagctatc atgttgccac actggacaat gaccaggaga tcattatcaa ggaaacatta 720

aaattcattg aggaacagct tgggaaataa 750

<210> 62
<211> 249
<212> PRT
<213> Unknown

<220>
<223> Obtained from environmental sample

<400> 62
Met Ser Gly Lys Tyr Thr Val Leu Glu Gly Ala Glu Pro Phe Tyr Phe
1               5                   10                  15

Glu Gly Ser Arg Val Gly Ile Leu Val Ser His Gly Phe Thr Gly Ser
            20                  25                  30

Thr Gln Ser Met Arg Pro Leu Gly Glu Ala Tyr Ala Lys Ala Gly Tyr
        35                  40                  45

Thr Val Cys Gly Pro Arg Leu Lys Gly His Gly Thr His Tyr Glu Glu
    50                  55                  60

Met Glu Gln Thr Thr Tyr Asn Asp Trp Ile Asp Ser Val Glu Glu Gly
65 70 75 80

Tyr Gln Trp Leu Lys Glu Arg Cys Asp Thr Ile Phe Val Thr Gly Leu
85 90 95

Ser Met Gly Gly Thr Leu Thr Leu Tyr Met Ala Glu Asn His Pro Glu
100 105 110

Ile Lys Ala Ile Ile Pro Ile Asn Ala Ala Ile Gln Ile Ser Asp Met
115 120 125

Ala Ala Ala Ala Asn Leu Asp Gly Val Arg Phe Leu Asp Ala Ile Gly
130 135 140

Ser Asp Ile Lys Lys Gln Gly Glu Lys Glu Leu Ala Tyr Glu Lys Thr
145 150 155 160

Pro Val Lys Ser Leu Gly Glu Ile Val Ala Leu Met Glu Lys Val Lys
165 170 175

Ala Asp Leu Ser Asp Ile Thr Cys Pro Ala Leu Ile Phe Val Ser Lys
180 185 190

Glu Asp His Val Val Pro Pro Asp Asn Ser Arg Ile Ile Lys Glu Gln
195 200 205

Ile Arg Ser Ala Asp Lys Ser Leu Ile Glu Leu Ser Glu Ser Tyr His
210 215 220

Val Ala Thr Leu Asp Asn Asp Gln Glu Ile Ile Ile Lys Glu Thr Leu
225 230 235 240

Lys Phe Ile Glu Glu Gln Leu Gly Lys
245

<210> 63
<211> 945
<212> DNA
<213> Unknown

<220>
<223> Obtained from environmental sample

<400> 63
atggctgatt tgcacccaaa aatgaaattt ttcctggaga atcttttgcc aatccaggaa
60

ggagctgtac ctacccttga ggaaattcgc gcaagaagtg ctgcaatgcc ttcgggtacg
120

gtggaggagg tccaggaagt gtcagacaga cttatccctg gctctgaatc cgacatccca
180

gttcgcattt atactcctga aggagaggga ccatttccgg taaccgtatt tttccacggc
240

ggaggatttg tatacggggg acttgatacc catgatgcgg tctgcagaag catcgtgaag
300

gcttctggcc agatggttgt tgcggtagaa taccggcttg ctccggaaaa tccgttccct
360

gcagcacctg aagactgctt tgcagccgca aattgggtaa atgagaatgc agaaaagcta
420

aacgctgacc gaactaagct aagcgtcgcg ggagacagtg ccggagggaa tttggcaact
480

gtagtctgcc tgcttgcgag agagagggga ggcctgtcta tcagcaagca ggttctgatc
540

taccctgtta cagataaata ccagcctgga aaatatccgt cctatgaaga gaatggaagc
600

ggctatttcc tcacaactga ctcgatggca ttatttgcag ccctttacat tcaatctgaa
660

gagggacgtg acaatccgct ttcggctccg atactggcca agaacttaag cgggctgccg
720

cctgcactgg tcattacggc cgagtatgac ccactaaggg atgaaggcga agcctatgct
780

gaaaaactcc gtgacgcggg agttgaagtg attttaaaaa gggaagcagg ccagattcac
840

ggctttttca atctcttcag cattatggat tccaaggatg atttaaagga cgtatatgcc
900

tatatcggcg agttcctaaa tagtgaaatc gccgcagttc tatag
945

<210> 64
<211> 314
<212> PRT
<213> Unknown

<220>
<223> Obtained from environmental sample

<220>
<221> DOMAIN
<222> (75)...(284)
<223> alpha/beta hydrolase fold

<220>
<221> SITE
<222> (149)...(161)
<223> Lipolytic enzymes "G-D-X-G" family, putative serine active site. Prosite id = PS01174

<220>
<221> SITE
<222> (201)...(204)
<223> N-glycosylation site. Prosite id = PS00001

<220>
<221> SITE

<222> (238)...(241)
<223> N-glycosylation site. Prosite id = PS00001

<400> 64
```
Met Ala Asp Leu His Pro Lys Met Lys Phe Phe Leu Glu Asn Leu Leu
1               5                   10                  15

Pro Ile Gln Glu Gly Ala Val Pro Thr Leu Glu Glu Ile Arg Ala Arg
            20                  25                  30

Ser Ala Ala Met Pro Ser Gly Thr Val Glu Glu Val Gln Glu Val Ser
        35                  40                  45

Asp Arg Leu Ile Pro Gly Ser Glu Ser Asp Ile Pro Val Arg Ile Tyr
    50                  55                  60

Thr Pro Glu Gly Glu Gly Pro Phe Pro Val Thr Val Phe Phe His Gly
65                  70                  75                  80

Gly Gly Phe Val Tyr Gly Gly Leu Asp Thr His Asp Ala Val Cys Arg
                85                  90                  95

Ser Ile Val Lys Ala Ser Gly Gln Met Val Val Ala Val Glu Tyr Arg
            100                 105                 110

Leu Ala Pro Glu Asn Pro Phe Pro Ala Ala Pro Glu Asp Cys Phe Ala
        115                 120                 125

Ala Ala Asn Trp Val Asn Glu Asn Ala Glu Lys Leu Asn Ala Asp Arg
    130                 135                 140

Thr Lys Leu Ser Val Ala Gly Asp Ser Ala Gly Gly Asn Leu Ala Thr
145                 150                 155                 160

Val Val Cys Leu Leu Ala Arg Glu Arg Gly Gly Leu Ser Ile Ser Lys
                165                 170                 175

Gln Val Leu Ile Tyr Pro Val Thr Asp Lys Tyr Gln Pro Gly Lys Tyr
            180                 185                 190

Pro Ser Tyr Glu Glu Asn Gly Ser Gly Tyr Phe Leu Thr Thr Asp Ser
        195                 200                 205

Met Ala Leu Phe Ala Ala Leu Tyr Ile Gln Ser Glu Glu Gly Arg Asp
    210                 215                 220

Asn Pro Leu Ser Ala Pro Ile Leu Ala Lys Asn Leu Ser Gly Leu Pro
225                 230                 235                 240

Pro Ala Leu Val Ile Thr Ala Glu Tyr Asp Pro Leu Arg Asp Glu Gly
                245                 250                 255
```

Glu Ala Tyr Ala Glu Lys Leu Arg Asp Ala Gly Val Glu Val Ile Leu
260 265 270

Lys Arg Glu Ala Gly Gln Ile His Gly Phe Phe Asn Leu Phe Ser Ile
275 280 285

Met Asp Ser Lys Asp Asp Leu Lys Asp Val Tyr Ala Tyr Ile Gly Glu
290 295 300

Phe Leu Asn Ser Glu Ile Ala Ala Val Leu
305 310

<210> 65
<211> 930
<212> DNA
<213> Unknown

<220>
<223> Obtained from environmental sample

<400> 65
atgaaaaaaa cagcacgtat ccttaccgtt ttgagcctgc tcgcattatc agttccatct
60

atggcgcaaa cagaaaccat gaacctttgg ccggaaggca aagtccccaa ttttaaaaag
120

agtgatgtag aagagaaatc ggtcaccgac gcacagggca tcctgcggat cagcggcgta
180

acggttccta cgattacagc ttacattgca ccgaaagata aagcaaccgg agcggcggtg
240

atgatctgcc cgggcggcgg ttacggcata ttggcggctt cgcacgaagg cagtgatttt
300

gccaaatggt tcaatgaacg tggtatttcg gcatttgtcc tgaaataccg cctacccaac
360

gaaaaagcga tggctcatca gcacgaagtc ccattaatgg acgccatgca gggcatgaaa
420

ctgatccgcc agaatgccgg caaatggggc atcgacgtga acaaaattgg agtgatgggt
480

ttttcagcgg gcggccacct ggcagccacg atttcgacac attacaatat gggtgaaaaa
540

gcttcccccg acggcaaacc aaacttttcg atactcattt acccggtgat ctcgcttaca
600

cccgccctcg cacacggagg ctcacgcgac aacttactcg gacctgaaaa atcggacgag
660

ctgatcaaat actattccaa cgaattgcag gtatccgatc agaccccgcc ggcattcctc
720

gttcacgcta tggacgacac gggcgtcccc gtcgagaaca gcatcgaata ttacctggcg
780

ctgaaaaaga aaaagatccc ggccgaaatg cacttgtacc cgaaaggtgg ccatggctac
840

ggcatgcgca ccgaaggtaa aggatctctc ggtggttggc ccgccgcaat ggaaggctgg
900

ctgaaagcga tgggatacca gaaaaattga
930

<210> 66
<211> 309
<212> PRT
<213> Unknown

<220>
<223> Obtained from environmental sample

<220>
<221> SIGNAL
<222> (1)...(22)

<220>
<221> DOMAIN
<222> (85)...(282)
<223> alpha/beta hydrolase fold

<220>
<221> DOMAIN
<222> (98)...(307)
<223> Prolyl oligopeptidase family

<220>
<221> SITE
<222> (190)...(193)
<223> N-glycosylation site. Prosite id = PS00001

<400> 66
Met Lys Lys Thr Ala Arg Ile Leu Thr Val Leu Ser Leu Leu Ala Leu
1 5 10 15

Ser Val Pro Ser Met Ala Gln Thr Glu Thr Met Asn Leu Trp Pro Glu
20 25 30

Gly Lys Val Pro Asn Phe Lys Lys Ser Asp Val Glu Glu Lys Ser Val
35 40 45

Thr Asp Ala Gln Gly Ile Leu Arg Ile Ser Gly Val Thr Val Pro Thr
50 55 60

Ile Thr Ala Tyr Ile Ala Pro Lys Asp Lys Ala Thr Gly Ala Ala Val
65 70 75 80

Met Ile Cys Pro Gly Gly Gly Tyr Gly Ile Leu Ala Ala Ser His Glu
85 90 95

Gly Ser Asp Phe Ala Lys Trp Phe Asn Glu Arg Gly Ile Ser Ala Phe
100 105 110

Val Leu Lys Tyr Arg Leu Pro Asn Glu Lys Ala Met Ala His Gln His

115 120 125

Glu Val Pro Leu Met Asp Ala Met Gln Gly Met Lys Leu Ile Arg Gln
130 135 140

Asn Ala Gly Lys Trp Gly Ile Asp Val Asn Lys Ile Gly Val Met Gly
145 150 155 160

Phe Ser Ala Gly Gly His Leu Ala Ala Thr Ile Ser Thr His Tyr Asn
165 170 175

Met Gly Glu Lys Ala Ser Pro Asp Gly Lys Pro Asn Phe Ser Ile Leu
180 185 190

Ile Tyr Pro Val Ile Ser Leu Thr Pro Ala Leu Ala His Gly Gly Ser
195 200 205

Arg Asp Asn Leu Leu Gly Pro Glu Lys Ser Asp Glu Leu Ile Lys Tyr
210 215 220

Tyr Ser Asn Glu Leu Gln Val Ser Asp Gln Thr Pro Pro Ala Phe Leu
225 230 235 240

Val His Ala Met Asp Asp Thr Gly Val Pro Val Glu Asn Ser Ile Glu
245 250 255

Tyr Tyr Leu Ala Leu Lys Lys Lys Lys Ile Pro Ala Glu Met His Leu
260 265 270

Tyr Pro Lys Gly Gly His Gly Tyr Gly Met Arg Thr Glu Gly Lys Gly
275 280 285

Ser Leu Gly Gly Trp Pro Ala Ala Met Glu Gly Trp Leu Lys Ala Met
290 295 300

Gly Tyr Gln Lys Asn
305

<210> 67
<211> 1137
<212> DNA
<213> Unknown

<220>
<223> Obtained from environmental sample

<400> 67
atggcccgca agttcctcta tcttatcgcc ctgcttgccg tgatggtcat tgcagcgatc
60

ttcgcgctgc gcatctggca gaacgagctc acccgcttcg cattcgtacc gcgcgtggct
120

tacgcccgcc tcgatccgct gcctgcagat gcctatgccg gcaaccggat gtggctcgcc
180

cgcccggggc tcggtgatag cgatcccgca cagtggctcc ctgcaggagt aaggaagagt
240

gccggtccgg gcacatctgc cgcagtgttc ttcattcatc cgaccagcta tctcgcgcgc
300

gaaagctgga acggtccgct cgacgatacc gatacgaatc gccgggccac ctatttcatc
360

caaggtatgg cctctgcctt caacgggcag gcacaggtct gggcaccgcg ctatcggcaa
420

gccgccttcg gcgcattcct caccgacaag gccgagggcg agctggccct ggactctgcc
480

tacctcgatg tccggcaggc cttcgacgcc ttccttgcgg cgacaccgcc cgatgcaccg
540

atcgtgctgg cagggcacag ccagggcgcg ctccacctga tgcgcctctt gaaggaccgg
600

attgccggaa caccactcgc tggccgggtg gtagcggcct acccgatcgg atggccgatc
660

tcggtcgagc atgatcttcc cccgcttggc cttccggctt gcgacaagag cgatgctact
720

ggctgcatca tgacgtggtc gagtttcgct gagcccgccg aaccgcaact tgtgttggaa
780

gcgtaccgca cgcgccccgc gcttgatggg aaagccaagg atgcaaagcc ggttctgtgc
840

accaacccgc ttactggaag catcggcgga caggcacccg cgtcggccaa ccttggcacg
900

ctaaagccca gcaaggacct gaagagtggt gagttgatcg ccaaggctgt tccggcccgc
960

tgcgatacca gcaccggctt gctgatgatc ggggacccac cggaccttgg gccgtttgtc
1020

ctgccaggca acaactacca cgtctatgac atccccctgt tctgggcgaa cctgcgcgca
1080

gatgtcggcc ggcggaccgc aacgtgggag aaggcacgcg gagcggccgc ccggtga
1137

<210> 68
<211> 378
<212> PRT
<213> Unknown

<220>
<223> Obtained from environmental sample

<220>
<221> SITE
<222> (182)...(195)
<223> SRP54-type proteins GTP-binding domain signature. Prosite id = PS00300

<400> 68

```
Met Ala Arg Lys Phe Leu Tyr Leu Ile Ala Leu Leu Ala Val Met Val
1               5                   10                  15

Ile Ala Ala Ile Phe Ala Leu Arg Ile Trp Gln Asn Glu Leu Thr Arg
            20                  25                  30

Phe Ala Phe Val Pro Arg Val Ala Tyr Ala Arg Leu Asp Pro Leu Pro
        35                  40                  45

Ala Asp Ala Tyr Ala Gly Asn Arg Met Trp Leu Ala Arg Pro Gly Leu
    50                  55                  60

Gly Asp Ser Asp Pro Ala Gln Trp Leu Pro Ala Gly Val Arg Lys Ser
65                  70                  75                  80

Ala Gly Pro Gly Thr Ser Ala Ala Val Phe Phe Ile His Pro Thr Ser
                85                  90                  95

Tyr Leu Ala Arg Glu Ser Trp Asn Gly Pro Leu Asp Asp Thr Asp Thr
            100                 105                 110

Asn Arg Arg Ala Thr Tyr Phe Ile Gln Gly Met Ala Ser Ala Phe Asn
        115                 120                 125

Gly Gln Ala Gln Val Trp Ala Pro Arg Tyr Arg Gln Ala Ala Phe Gly
    130                 135                 140

Ala Phe Leu Thr Asp Lys Ala Glu Gly Glu Leu Ala Leu Asp Ser Ala
145                 150                 155                 160

Tyr Leu Asp Val Arg Gln Ala Phe Asp Ala Phe Leu Ala Ala Thr Pro
                165                 170                 175

Pro Asp Ala Pro Ile Val Leu Ala Gly His Ser Gln Gly Ala Leu His
            180                 185                 190

Leu Met Arg Leu Leu Lys Asp Arg Ile Ala Gly Thr Pro Leu Ala Gly
        195                 200                 205

Arg Val Val Ala Ala Tyr Pro Ile Gly Trp Pro Ile Ser Val Glu His
    210                 215                 220

Asp Leu Pro Pro Leu Gly Leu Pro Ala Cys Asp Lys Ser Asp Ala Thr
225                 230                 235                 240

Gly Cys Ile Met Thr Trp Ser Ser Phe Ala Glu Pro Ala Glu Pro Gln
                245                 250                 255

Leu Val Leu Glu Ala Tyr Arg Thr Arg Pro Ala Leu Asp Gly Lys Ala
            260                 265                 270
```

Lys Asp Ala Lys Pro Val Leu Cys Thr Asn Pro Leu Thr Gly Ser Ile
        275                 280                 285

Gly Gly Gln Ala Pro Ala Ser Ala Asn Leu Gly Thr Leu Lys Pro Ser
    290                 295                 300

Lys Asp Leu Lys Ser Gly Glu Leu Ile Ala Lys Ala Val Pro Ala Arg
305                 310                 315                 320

Cys Asp Thr Ser Thr Gly Leu Leu Met Ile Gly Asp Pro Pro Asp Leu
                325                 330                 335

Gly Pro Phe Val Leu Pro Gly Asn Asn Tyr His Val Tyr Asp Ile Pro
            340                 345                 350

Leu Phe Trp Ala Asn Leu Arg Ala Asp Val Gly Arg Arg Thr Ala Thr
        355                 360                 365

Trp Glu Lys Ala Arg Gly Ala Ala Ala Arg
    370                 375

<210> 69
<211> 807
<212> DNA
<213> Unknown

<220>
<223> Obtained from environmental sample

<400> 69
gtgaatacag ccgacctatt gaagccacca cccgcaagca tgacagttct cgaggcgaga
60

gcgctgctgg acatatgcaa gatgagcgcc ccattggcgc gcttgctatt caaaaagaac
120

tcgccctggc gcaaacaacg ggttctcgta atacctggct ttggcgctga tgatcgctac
180

acctggccgt tgcgcaattt cgtccaggca cagggctatg ccacgactgg ctggggcctg
240

ggcaccaaca aggcaggtct caatatgccg catcaactat ccgacgtcca ccccagatgg
300

aagctaaaac ccaagacgcc gtaccgtggt gaggcgggcg taccttacgt gattgaccgc
360

ttgatcgaac ggtttgacga attggcatcg acggatccgc aacccatcgc acttataggt
420

tggagtctgg gtggtttcat ggcccgtgaa gttgcccgag agcgcccaaa ccaggtgagt
480

caggttatta ccctcggttc tcctgtcatc ggaggcccaa aatacaccct cgctgcatcg
540

gctttcatcc ggcgcaaata cgatttggac tgggtggagc aagtgatcgc ggagcgggaa
600

gatcgcccca ttactgttcc tattacagca atagtcagcc agtctgatgg catcgtcgga
660

tattcagcgg caatcgatca ccacagtccc gctgtgcagc atttacatat ggatgttgcc
720

catttgggct ttccttacaa cacgagggtt tggtcagaaa tcgccaatgc gctcaactct
780

ttagaggtgg agaaggagcg tgtttag
807

<210> 70
<211> 268
<212> PRT
<213> Unknown

<220>
<223> Obtained from environmental sample

<220>
<221> SITE
<222> (138)...(147)
<223> Lipases, serine active site. Prosite id = PS00120

<400> 70
Met Asn Thr Ala Asp Leu Leu Lys Pro Pro Pro Ala Ser Met Thr Val
1 5 10 15

Leu Glu Ala Arg Ala Leu Leu Asp Ile Cys Lys Met Ser Ala Pro Leu
20 25 30

Ala Arg Leu Leu Phe Lys Lys Asn Ser Pro Trp Arg Lys Gln Arg Val
35 40 45

Leu Val Ile Pro Gly Phe Gly Ala Asp Asp Arg Tyr Thr Trp Pro Leu
50 55 60

Arg Asn Phe Val Gln Ala Gln Gly Tyr Ala Thr Thr Gly Trp Gly Leu
65 70 75 80

Gly Thr Asn Lys Ala Gly Leu Asn Met Pro His Gln Leu Ser Asp Val
85 90 95

His Pro Arg Trp Lys Leu Lys Pro Lys Thr Pro Tyr Arg Gly Glu Ala
100 105 110

Gly Val Pro Tyr Val Ile Asp Arg Leu Ile Glu Arg Phe Asp Glu Leu
115 120 125

Ala Ser Thr Asp Pro Gln Pro Ile Ala Leu Ile Gly Trp Ser Leu Gly
130 135 140

Gly Phe Met Ala Arg Glu Val Ala Arg Glu Arg Pro Asn Gln Val Ser

145 150 155 160

Gln Val Ile Thr Leu Gly Ser Pro Val Ile Gly Gly Pro Lys Tyr Thr
165 170 175

Leu Ala Ala Ser Ala Phe Ile Arg Arg Lys Tyr Asp Leu Asp Trp Val
180 185 190

Glu Gln Val Ile Ala Glu Arg Glu Asp Arg Pro Ile Thr Val Pro Ile
195 200 205

Thr Ala Ile Val Ser Gln Ser Asp Gly Ile Val Gly Tyr Ser Ala Ala
210 215 220

Ile Asp His His Ser Pro Ala Val Gln His Leu His Met Asp Val Ala
225 230 235 240

His Leu Gly Phe Pro Tyr Asn Thr Arg Val Trp Ser Glu Ile Ala Asn
245 250 255

Ala Leu Asn Ser Leu Glu Val Glu Lys Glu Arg Val
260 265

<210> 71
<211> 1098
<212> DNA
<213> Unknown

<220>
<223> Obtained from environmental sample

<400> 71
atgacgtttg ttttgacgat actgggcatc ctggctgcgg tcgttggcgt gtttctgctt
60

ctcgcctggc tttttcgtcg cggcgagaat ctgtcccgtt acgacacgcc cgtcgatccg
120

gccgcgctcg aatcctgcgg tgatccggac ggccccagcg cgggccatgc cggcgccgtc
180

gcggcgatcg aaaagctgcg cccgcaggcc gaagggctct cccgcggcgg aatgatccgg
240

ttcgcccggc aattcatgga ggatataccg accggccgtc ggttcgattg cgagtttcgg
300

ccggtcgaag ccgctggcgt tcgctgcgaa tgggtgctgg cgcccggcgc ggatccggaa
360

cgccgtgtac tttacctgca cggcggcgca ttcatcgccg gcagccctca cagccaccga
420

acggcgacct gccggttttc gaccgtcgcc cgagcggcgg tgctggcggt cgactaccga
480

ctgatgccgg agcattcgcg gcaggacctc attcaggact gccaggcggc ataccgctgg
540

ctgctcgacc acgggccgca cgggccggct gcggctcaac gggtgtattt cggcggcgac
600

tccgccggcg gcaatctcgc gctgacgctg tccaactgga cgcgggacgc cgggctgcgc
660

cagcccgacg ccgtcgtggc cctgtcgccg ttgacggatt cgacttacag cagcccgagc
720

atccgcggca acctggccac cgataccctg atctcgaccc tgttcgggcg gctggcgcgc
780

gtgccgcaaa gcgtgctgac ctggatgttc gtgctggata accgcatccg cccggtcgat
840

cccctggtct cgccgtcgcg gggcgacctg tcgggcctgc cacccacgct gcttcaggtg
900

agcgaatccg agatgctgta cgacgacgcc cgtcgctacg tgaacaaggc cgtggcggct
960

ggttcaccgg cccgtctgca gagctggccg ggactgctgc acgtctggca gctgttctac
1020

cccgagatcc ccgaggccgg gcaggcctgg cgccgggtcg gcgagttcat cgaggcggtc
1080

gacgcggaaa gcgcttag
1098

<210> 72
<211> 365
<212> PRT
<213> Unknown

<220>
<223> Obtained from environmental sample

<220>
<221> SIGNAL
<222> (1)...(22)

<220>
<221> DOMAIN
<222> (123)...(338)
<223> alpha/beta hydrolase fold

<220>
<221> SITE
<222> (30)...(33)
<223> N-glycosylation site. Prosite id = PS00001

<220>
<221> SITE
<222> (215)...(218)
<223> N-glycosylation site. Prosite id = PS00001

<400> 72
Met Thr Phe Val Leu Thr Ile Leu Gly Ile Leu Ala Ala Val Val Gly
1               5                   10                  15

Val Phe Leu Leu Leu Ala Trp Leu Phe Arg Arg Gly Glu Asn Leu Ser
            20                  25                  30

Arg Tyr Asp Thr Pro Val Asp Pro Ala Ala Leu Glu Ser Cys Gly Asp
        35                  40                  45

Pro Asp Gly Pro Ser Ala Gly His Ala Gly Ala Val Ala Ala Ile Glu
    50                  55                  60

Lys Leu Arg Pro Gln Ala Glu Gly Leu Ser Arg Gly Gly Met Ile Arg
65                  70                  75                  80

Phe Ala Arg Gln Phe Met Glu Asp Ile Pro Thr Gly Arg Arg Phe Asp
                85                  90                  95

Cys Glu Phe Arg Pro Val Glu Ala Ala Gly Val Arg Cys Glu Trp Val
            100                 105                 110

Leu Ala Pro Gly Ala Asp Pro Glu Arg Arg Val Leu Tyr Leu His Gly
        115                 120                 125

Gly Ala Phe Ile Ala Gly Ser Pro His Ser His Arg Thr Ala Thr Cys
    130                 135                 140

Arg Phe Ser Thr Val Ala Arg Ala Ala Val Leu Ala Val Asp Tyr Arg
145                 150                 155                 160

Leu Met Pro Glu His Ser Arg Gln Asp Leu Ile Gln Asp Cys Gln Ala
                165                 170                 175

Ala Tyr Arg Trp Leu Leu Asp His Gly Pro His Gly Pro Ala Ala Ala
            180                 185                 190

Gln Arg Val Tyr Phe Gly Gly Asp Ser Ala Gly Gly Asn Leu Ala Leu
        195                 200                 205

Thr Leu Ser Asn Trp Thr Arg Asp Ala Gly Leu Arg Gln Pro Asp Ala
    210                 215                 220

Val Val Ala Leu Ser Pro Leu Thr Asp Ser Thr Tyr Ser Ser Pro Ser
225                 230                 235                 240

Ile Arg Gly Asn Leu Ala Thr Asp Thr Leu Ile Ser Thr Leu Phe Gly
                245                 250                 255

Arg Leu Ala Arg Val Pro Gln Ser Val Leu Thr Trp Met Phe Val Leu
            260                 265                 270

Asp Asn Arg Ile Arg Pro Val Asp Pro Leu Val Ser Pro Ser Arg Gly
        275                 280                 285

Asp Leu Ser Gly Leu Pro Pro Thr Leu Leu Gln Val Ser Glu Ser Glu

290 295 300

Met Leu Tyr Asp Asp Ala Arg Arg Tyr Val Asn Lys Ala Val Ala Ala
305 310 315 320

Gly Ser Pro Ala Arg Leu Gln Ser Trp Pro Gly Leu Leu His Val Trp
325 330 335

Gln Leu Phe Tyr Pro Glu Ile Pro Glu Ala Gly Gln Ala Trp Arg Arg
340 345 350

Val Gly Glu Phe Ile Glu Ala Val Asp Ala Glu Ser Ala
355 360 365

<210> 73
<211> 930
<212> DNA
<213> Unknown

<220>
<223> Obtained from environmental sample

<400> 73
atgaggaatt gggcaatcgg cgggctggcg ctgggcgcgg gtgcgctggg agcgggcctg
60

tacaccagcc gcaaggcacg ggaggccgag gccgcggtgc cgatggacgg gcgcatggta
120

gaggccggcg ggcaccgctt ccatgtcgcg gagcaggggc agggccggcc gctgctgctg
180

atccatgggc tcgccgggca gatgcgcaac ttcgggcagc cgatgctcga cgacctggcg
240

cgcgactatc gggtcatccg gatcgaccgg ccggggtccg gctactcgcc gcggctggcg
300

agcgggcgcg ggcatctgtc gggacaggcc gacgccatcg ccgcgctgat cgacgcgctt
360

ggcctggaga aaccggtgct ggtggggcat tcgctgggtg gtgcggtgag cctcgcgacg
420

gccatgcgcc atcccgacaa ggtcggcgcg ctggcactga tcgctccggc gacccagccg
480

atccagagca ttcccgaggt gttcaaggga ctggtcgtcc cgcccgcttt gctgcccctg
540

gtcgcctgga ccatcgccgt tcccgtgggc gtcgcgacca aggacaaggt gctgcagcag
600

gtgttcgcgc ccgagccggt gccggcgaac tttctggtgg atggcggcgg catgctcggg
660

ttccggccca agaacttcga gggcgcggca accgacctcg ccgacgccag cagcgaggcc
720

gagaccctgc tgccgggcta cgccgggctg aagctgccgg tcggcatctt ctacgggcgc
780

ggggacaatc tgctcgacta tcgactgcat ggcgagaaga ccgcatccga gatcccgggc
840

gcccggctga cgctgaccga gggcggccac atgctgccct tcacccaacc ggaggcgacc
900

gcccgcttcg tccggagcgt gatcgagtaa
930

<210> 74
<211> 309
<212> PRT
<213> Unknown

<220>
<223> Obtained from environmental sample

<220>
<221> DOMAIN
<222> (83)...(306)
<223> alpha/beta hydrolase fold

<400> 74
Met Arg Asn Trp Ala Ile Gly Gly Leu Ala Leu Gly Ala Gly Ala Leu
1               5                   10                  15

Gly Ala Gly Leu Tyr Thr Ser Arg Lys Ala Arg Glu Ala Glu Ala Ala
            20                  25                  30

Val Pro Met Asp Gly Arg Met Val Glu Ala Gly Gly His Arg Phe His
        35                  40                  45

Val Ala Glu Gln Gly Gln Gly Arg Pro Leu Leu Leu Ile His Gly Leu
    50                  55                  60

Ala Gly Gln Met Arg Asn Phe Gly Gln Pro Met Leu Asp Asp Leu Ala
65                  70                  75                  80

Arg Asp Tyr Arg Val Ile Arg Ile Asp Arg Pro Gly Ser Gly Tyr Ser
                85                  90                  95

Pro Arg Leu Ala Ser Gly Arg Gly His Leu Ser Gly Gln Ala Asp Ala
            100                 105                 110

Ile Ala Ala Leu Ile Asp Ala Leu Gly Leu Glu Lys Pro Val Leu Val
        115                 120                 125

Gly His Ser Leu Gly Gly Ala Val Ser Leu Ala Thr Ala Met Arg His
    130                 135                 140

Pro Asp Lys Val Gly Ala Leu Ala Leu Ile Ala Pro Ala Thr Gln Pro
145                 150                 155                 160

Ile Gln Ser Ile Pro Glu Val Phe Lys Gly Leu Val Val Pro Pro Ala
                165                 170                 175

Leu Leu Pro Leu Val Ala Trp Thr Ile Ala Val Pro Val Gly Val Ala
180 185 190

Thr Lys Asp Lys Val Leu Gln Gln Val Phe Ala Pro Glu Pro Val Pro
195 200 205

Ala Asn Phe Leu Val Asp Gly Gly Gly Met Leu Gly Phe Arg Pro Lys
210 215 220

Asn Phe Glu Gly Ala Ala Thr Asp Leu Ala Asp Ala Ser Ser Glu Ala
225 230 235 240

Glu Thr Leu Leu Pro Gly Tyr Ala Gly Leu Lys Leu Pro Val Gly Ile
245 250 255

Phe Tyr Gly Arg Gly Asp Asn Leu Leu Asp Tyr Arg Leu His Gly Glu
260 265 270

Lys Thr Ala Ser Glu Ile Pro Gly Ala Arg Leu Thr Leu Thr Glu Gly
275 280 285

Gly His Met Leu Pro Phe Thr Gln Pro Glu Ala Thr Ala Arg Phe Val
290 295 300

Arg Ser Val Ile Glu
305

<210> 75
<211> 906
<212> DNA
<213> Unknown

<220>
<223> Obtained from environmental sample

<400> 75
atgagactga tcgtgaaaag acgcgctcac gccgccctgc tgatgttcgc agcggcgctg
60

ttgaatgttg ttggcgtggc tcaggccgct gtcccgttcc gctccgaccg gatgagcgtc
120

gaggtccgcg ggagcggccc cgacgtggtg ctgatccccg ggctcgcctc ctcgccggag
180

gtctggcagg cgaccgcgga ccggctcgac gacacccatc gcgtacatct ggtgcaggtg
240

aacggcttcg ccggcgcgcc cgccggggcg aacgccgaag gcgcggtggt cgcgccgctg
300

gtcggggagc tgaaccgcta catcgccgag accgggctga agcggccggc cctgatcggc
360

cactcgctgg gcggcttcgc ggcgctgcgg ttggcggccg agcatccgca ggcggtgggc

420

cgcgtgctgg tggtggactc gctgcccttc ttcccgctgc tgttcagccc ggcggcgact
480

gtggaggcgg tgaccccgca ggcgaacgcc atgcgggacg gcatgctggc catgccggcc
540

gagcgcttcc cggcggtgca ggcggccggg atgggacggc tggtcaagaa ccccgaagca
600

cgcagcaagc acgtccgcat cggcggcgcc tccgaccctg cggtggtcgc gcgcgccatg
660

cacgagatca tgaccaccga cctgcggccc cagctgcgcg ggatcgttgc gcccgtcacc
720

gttctctacg cctgggaccc cgccatggga ctcacggtcg aggcctacga ccagctctgg
780

cgcggcgcct acgccggact gaagggggcg agcctgacgc gggtggacgg cagcttccac
840

ttcatcatgg acgaccagcc ggagcgcttc tccgcggagg tcgagcggtt cctggcggcc
900

gagtaa
906

<210> 76
<211> 301
<212> PRT
<213> Unknown

<220>
<223> Obtained from environmental sample

<220>
<221> SIGNAL
<222> (1)...(29)

<220>
<221> DOMAIN
<222> (73)...(296)
<223> alpha/beta hydrolase fold

<400> 76
Met Arg Leu Ile Val Lys Arg Arg Ala His Ala Ala Leu Leu Met Phe
1 5 10 15

Ala Ala Ala Leu Leu Asn Val Val Gly Val Ala Gln Ala Ala Val Pro
20 25 30

Phe Arg Ser Asp Arg Met Ser Val Glu Val Arg Gly Ser Gly Pro Asp
35 40 45

Val Val Leu Ile Pro Gly Leu Ala Ser Ser Pro Glu Val Trp Gln Ala
50 55 60

Thr Ala Asp Arg Leu Asp Asp Thr His Arg Val His Leu Val Gln Val
65 70 75 80

```
Asn Gly Phe Ala Gly Ala Pro Ala Gly Ala Asn Ala Glu Gly Ala Val
                85                  90                  95

Val Ala Pro Leu Val Gly Glu Leu Asn Arg Tyr Ile Ala Glu Thr Gly
            100                 105                 110

Leu Lys Arg Pro Ala Leu Ile Gly His Ser Leu Gly Gly Phe Ala Ala
        115                 120                 125

Leu Arg Leu Ala Ala Glu His Pro Gln Ala Val Gly Arg Val Leu Val
    130                 135                 140

Val Asp Ser Leu Pro Phe Phe Pro Leu Leu Phe Ser Pro Ala Ala Thr
145                 150                 155                 160

Val Glu Ala Val Thr Pro Gln Ala Asn Ala Met Arg Asp Gly Met Leu
                165                 170                 175

Ala Met Pro Ala Glu Arg Phe Pro Ala Val Gln Ala Ala Gly Met Gly
            180                 185                 190

Arg Leu Val Lys Asn Pro Glu Ala Arg Ser Lys His Val Arg Ile Gly
        195                 200                 205

Gly Ala Ser Asp Pro Ala Val Val Ala Arg Ala Met His Glu Ile Met
    210                 215                 220

Thr Thr Asp Leu Arg Pro Gln Leu Arg Gly Ile Val Ala Pro Val Thr
225                 230                 235                 240

Val Leu Tyr Ala Trp Asp Pro Ala Met Gly Leu Thr Val Glu Ala Tyr
                245                 250                 255

Asp Gln Leu Trp Arg Gly Ala Tyr Ala Gly Leu Lys Gly Ala Ser Leu
            260                 265                 270

Thr Arg Val Asp Gly Ser Phe His Phe Ile Met Asp Asp Gln Pro Glu
        275                 280                 285

Arg Phe Ser Ala Glu Val Glu Arg Phe Leu Ala Ala Glu
    290                 295                 300
```

<210> 77
<211> 936
<212> DNA
<213> Unknown

<220>
<223> Obtained from environmental sample

<400> 77

atgctcaaga gtttggctgc agccgccgtg ctgctgacgg cggtgggttg cgcgacggcg 60

gacgaggcgg cggccgccgc cgcgccggcg ggtctggtgc agctgcaggg cgcccagccc 120

agccgcttcg ccagcaaccg tatcgccgtg caggtgatcg gaagcgggcc ggacgtggtg 180

ctgatccccg ggctgggctc ctcgccccgc gcctggacca gcaccatcgc cgccaacccg 240

gggcgccggt accacgtggt gcaactgaac ggcttcgccg gcctgccggt cggcggcgcc 300

accgaaggac cggtggccgc tccggcggcc gaggagctcg cccgctacat ccgcgagaac 360

cggctgaaca ggccggcgct ggtcggccac tccatgggcg gcaccatcgg catgatggtg 420

gcggcgcgcc atcccgagct ggtcgggcgg ctcatggtgg tcgacatgtt cccct tcatg 480

ggggcgatgt tcgggggccc caacgcgacg ccggagagcg tgcggccgat cgccgaccag 540

atccgcgacg gcatccgcgg cggcagcggc cccgggcggg aggagcagct gaagcagacc 600

atcgccggca tggttcgcac cgaggcgctg cgcgcggagc cgctcgcgga cagcatggcc 660

agcgactcgg acgtctccgg gcgcgccttc cacgagctgg tggtcaccga cctgcggccc 720

gagctgccga agatcaccgc gcccacgacc gtgctctacg tgacgcctac gggcgcgccg 780

ctcaccgacg cgcagatgga cggcttctac cgcgcctcct acgccgcgct taagggcgcc 840

cagctgaagc ggatccccga cagcgcgcac ttcatcatgc tcgatgcgcc ggagcggttc 900

agtcaggagc tgcgcacctt cctcgccggc tcgtga 936

<210> 78
<211> 311
<212> PRT
<213> Unknown

<220>
<223> Obtained from environmental sample

<220>
<221> SIGNAL
<222> (1)...(20)

<220>
<221> DOMAIN
<222> (82)...(306)
<223> alpha/beta hydrolase fold

<400> 78

```
Met Leu Lys Ser Leu Ala Ala Ala Ala Val Leu Leu Thr Ala Val Gly
1               5                   10                  15

Cys Ala Thr Ala Asp Glu Ala Ala Ala Ala Ala Ala Pro Ala Gly Leu
            20                  25                  30

Val Gln Leu Gln Gly Ala Gln Pro Ser Arg Phe Ala Ser Asn Arg Ile
        35                  40                  45

Ala Val Gln Val Ile Gly Ser Gly Pro Asp Val Val Leu Ile Pro Gly
    50                  55                  60

Leu Gly Ser Ser Pro Arg Ala Trp Thr Ser Thr Ile Ala Ala Asn Pro
65                  70                  75                  80

Gly Arg Arg Tyr His Val Val Gln Leu Asn Gly Phe Ala Gly Leu Pro
                85                  90                  95

Val Gly Gly Ala Thr Glu Gly Pro Val Ala Ala Pro Ala Ala Glu Glu
            100                 105                 110

Leu Ala Arg Tyr Ile Arg Glu Asn Arg Leu Asn Arg Pro Ala Leu Val
        115                 120                 125

Gly His Ser Met Gly Gly Thr Ile Gly Met Met Val Ala Ala Arg His
    130                 135                 140

Pro Glu Leu Val Gly Arg Leu Met Val Val Asp Met Phe Pro Phe Met
145                 150                 155                 160

Gly Ala Met Phe Gly Gly Pro Asn Ala Thr Pro Glu Ser Val Arg Pro
                165                 170                 175

Ile Ala Asp Gln Ile Arg Asp Gly Ile Arg Gly Gly Ser Gly Pro Gly
            180                 185                 190

Arg Glu Glu Gln Leu Lys Gln Thr Ile Ala Gly Met Val Arg Thr Glu
        195                 200                 205

Ala Leu Arg Ala Glu Pro Leu Ala Asp Ser Met Ala Ser Asp Ser Asp
    210                 215                 220

Val Ser Gly Arg Ala Phe His Glu Leu Val Val Thr Asp Leu Arg Pro
225                 230                 235                 240

Glu Leu Pro Lys Ile Thr Ala Pro Thr Thr Val Leu Tyr Val Thr Pro
                245                 250                 255

Thr Gly Ala Pro Leu Thr Asp Ala Gln Met Asp Gly Phe Tyr Arg Ala
            260                 265                 270
```

Ser Tyr Ala Ala Leu Lys Gly Ala Gln Leu Lys Arg Ile Pro Asp Ser
275 280 285

Ala His Phe Ile Met Leu Asp Ala Pro Glu Arg Phe Ser Gln Glu Leu
290 295 300

Arg Thr Phe Leu Ala Gly Ser
305 310

<210> 79
<211> 960
<212> DNA
<213> Unknown

<220>
<223> Obtained from environmental sample

<400> 79
atgcaatcgg ccgctagcat ccttgccctg gcgctggccc tactggccgg cggggtgtgg 60

ctcgtggact ggcgcgccga ccggcgcgag gcggaagcgc tcgcgcagtg gccgccggag 120

ggcatttttg tccaggttga ggaccgccga gtccacgccg tcacgcgggg gcaaggcccc 180

gacctcgtcc tgatccacgg cgcctcgggc aatgcgcggg atatgacctt ccgctttatg 240

ggcctgctaa cggaccgcta ccgtgtcacc gcgttcgacc gtccgggcct gggctacacc 300

gaccccgcgc cggccgattg ggcgagagc ccgaaggagc aggccgcctt cctacaggcc 360

gcagcgcggg agctgcatat cgagagcccc atcgtcctgg gccactcctt cggcggggct 420

gtggcgctgg cctggggatt gagcgacccg gaggggaccg ccgccgtcgt ggacgtttcc 480

ggtgtggcga tgccctggcc cggccgcctg ggctggctct accgcatcaa tggcccgtgg 540

ctcggtacgg cccttgtgcc gcctctcgtt gctgcctttg cgccccaacc gctcctagac 600

cgggcgctcg cggcgacgtt cgcgcccgat cccgtccccg aaggctatgc cgagcacttc 660

ggagccagcc tctcgatccg acgcgcctcg tttcggacca acgcccgtca ggtgaacgcg 720

ctgcgggcct cggtcatgga gatgtcccgg gactattcaa ccttctccgt gccccttgaa 780

atcgtccacg gcgaagccga caccattgtc cccctcgagg tccattcctg ccctctgtcg 840

cgaatcattc ctggcgcgaa ccttacggtg ctgcccggcg tcggccatat gccgcaccag

900

acgcaccccg aggcggtggt cgccgctgtc gatcgggcgc gagcccgcgc tgggctctga
960

<210> 80
<211> 319
<212> PRT
<213> Unknown

<220>
<223> Obtained from environmental sample

<220>
<221> SIGNAL
<222> (1)...(21)

<220>
<221> DOMAIN
<222> (87)...(312)
<223> alpha/beta hydrolase fold

<220>
<221> SITE
<222> (291)...(294)
<223> N-glycosylation site. Prosite id = PS00001

<400> 80
Met Gln Ser Ala Ala Ser Ile Leu Ala Leu Ala Leu Ala Leu Leu Ala
1 5 10 15

Gly Gly Val Trp Leu Val Asp Trp Arg Ala Asp Arg Arg Glu Ala Glu
20 25 30

Ala Leu Ala Gln Trp Pro Pro Glu Gly Ile Phe Val Gln Val Glu Asp
35 40 45

Arg Arg Val His Ala Val Thr Arg Gly Gln Gly Pro Asp Leu Val Leu
50 55 60

Ile His Gly Ala Ser Gly Asn Ala Arg Asp Met Thr Phe Arg Phe Met
65 70 75 80

Gly Leu Leu Thr Asp Arg Tyr Arg Val Thr Ala Phe Asp Arg Pro Gly
85 90 95

Leu Gly Tyr Thr Asp Pro Ala Pro Ala Asp Trp Gly Glu Ser Pro Lys
100 105 110

Glu Gln Ala Ala Phe Leu Gln Ala Ala Ala Arg Glu Leu His Ile Glu
115 120 125

Ser Pro Ile Val Leu Gly His Ser Phe Gly Gly Ala Val Ala Leu Ala
130 135 140

Trp Gly Leu Ser Asp Pro Glu Gly Thr Ala Ala Val Val Asp Val Ser
145 150 155 160

Gly Val Ala Met Pro Trp Pro Gly Arg Leu Gly Trp Leu Tyr Arg Ile
165 170 175

Asn Gly Pro Trp Leu Gly Thr Ala Leu Val Pro Pro Leu Val Ala Ala
180 185 190

Phe Ala Pro Gln Pro Leu Leu Asp Arg Ala Leu Ala Ala Thr Phe Ala
195 200 205

Pro Asp Pro Val Pro Glu Gly Tyr Ala Glu His Phe Gly Ala Ser Leu
210 215 220

Ser Ile Arg Arg Ala Ser Phe Arg Thr Asn Ala Arg Gln Val Asn Ala
225 230 235 240

Leu Arg Ala Ser Val Met Glu Met Ser Arg Asp Tyr Ser Thr Phe Ser
245 250 255

Val Pro Leu Glu Ile Val His Gly Glu Ala Asp Thr Ile Val Pro Leu
260 265 270

Glu Val His Ser Cys Pro Leu Ser Arg Ile Ile Pro Gly Ala Asn Leu
275 280 285

Thr Val Leu Pro Gly Val Gly His Met Pro His Gln Thr His Pro Glu
290 295 300

Ala Val Val Ala Ala Val Asp Arg Ala Arg Ala Arg Ala Gly Leu
305 310 315

<210> 81
<211> 969
<212> DNA
<213> Unknown

<220>
<223> Obtained from environmental sample

<400> 81
atgacgcttc cgcaccgacg ttcgcacctg cgcccgattg atgagaggct ctttgcccgc
60

tggctagcgg ccggcgccgt cgcaatgctg ctcagtgtca ctgcttgcgc cgcgcaaccg
120

gcgaaaagcg agcgaccagc ggccgctgac gtggttcggg tctggccggg ggcggctccc
180

ggcacggaga gttggaccgg agcggaggtc gagctggacg ccgatttgcc ggccggcaaa
240

gtccgggtcg tcaccaatgt caccgcgccg gcgctgacga tcgtgcgccc caagccgggc
300

accgccaacg ggaccgcgat gctggttcta cccggcggtg cattccgggc gctcgcctgg
360

gacctcgaag gaaccgaagc cgcgcaacgg ctggccggcc gcggcatcac ggcctttgtc
420

ctcaaatatc gcgtccggcc cccggtcgga gcgcccgccg ggccggaatc tttcgacgcc
480

ttcaccgttc gaacgcagcc agcgagagac attgcggtcg ccgacgcccg gcaggcgctt
540

cgcctgatcc gggcacgggc gaaagactat ggcatctcgg ccgatcggat cggaatgatc
600

ggcttttcag ctggagccat ggcggtaatg gagctggcgc tggcgcaaga ggtgacggat
660

cgcccgaact tcgcagcgtc catctatgga gcggcgccca ccgagcggca accggccgcc
720

ggcgccccgc ccttgttcct tgtggctgct caggacgatc cccaggtccc ctccgaccga
780

agcgtcgatc tgtatcgccg ctggacccaa gcgggcctgc ccgccgagct gcacctctac
840

gaaaagggcg ggcacggctt cggcacgcgc gcccgcaacc tccccgcgga ccagtggctt
900

gaagcgctcg aggcctggct catctcccgc cgcctggcgc caccacccgc agcggccaag
960

cacaaataa
969

<210> 82
<211> 322
<212> PRT
<213> Unknown

<220>
<223> Obtained from environmental sample

<220>
<221> SIGNAL
<222> (1)...(37)

<220>
<221> DOMAIN
<222> (107)...(289)
<223> alpha/beta hydrolase fold

<220>
<221> SITE
<222> (87)...(90)
<223> N-glycosylation site. Prosite id = PS00001

<220>
<221> SITE
<222> (104)...(107)
<223> N-glycosylation site. Prosite id = PS00001

<400> 82
Met Thr Leu Pro His Arg Arg Ser His Leu Arg Pro Ile Asp Glu Arg
1 5 10 15

```
Leu Phe Ala Arg Trp Leu Ala Ala Gly Ala Val Ala Met Leu Leu Ser
            20                  25                  30

Val Thr Ala Cys Ala Ala Gln Pro Ala Lys Ser Glu Arg Pro Ala Ala
        35                  40                  45

Ala Asp Val Val Arg Val Trp Pro Gly Ala Ala Pro Gly Thr Glu Ser
    50                  55                  60

Trp Thr Gly Ala Glu Val Glu Leu Asp Ala Asp Leu Pro Ala Gly Lys
65                  70                  75                  80

Val Arg Val Val Thr Asn Val Thr Ala Pro Ala Leu Thr Ile Val Arg
                85                  90                  95

Pro Lys Pro Gly Thr Ala Asn Gly Thr Ala Met Leu Val Leu Pro Gly
            100                 105                 110

Gly Ala Phe Arg Ala Leu Ala Trp Asp Leu Glu Gly Thr Glu Ala Ala
        115                 120                 125

Gln Arg Leu Ala Gly Arg Gly Ile Thr Ala Phe Val Leu Lys Tyr Arg
    130                 135                 140

Val Arg Pro Pro Val Gly Ala Pro Ala Gly Pro Glu Ser Phe Asp Ala
145                 150                 155                 160

Phe Thr Val Arg Thr Gln Pro Ala Arg Asp Ile Ala Val Ala Asp Ala
                165                 170                 175

Arg Gln Ala Leu Arg Leu Ile Arg Ala Arg Ala Lys Asp Tyr Gly Ile
            180                 185                 190

Ser Ala Asp Arg Ile Gly Met Ile Gly Phe Ser Ala Gly Ala Met Ala
        195                 200                 205

Val Met Glu Leu Ala Leu Ala Gln Glu Val Thr Asp Arg Pro Asn Phe
    210                 215                 220

Ala Ala Ser Ile Tyr Gly Ala Ala Pro Thr Glu Arg Gln Pro Ala Ala
225                 230                 235                 240

Gly Ala Pro Pro Leu Phe Leu Val Ala Ala Gln Asp Asp Pro Gln Val
                245                 250                 255

Pro Ser Asp Arg Ser Val Asp Leu Tyr Arg Arg Trp Thr Gln Ala Gly
            260                 265                 270
```

Leu Pro Ala Glu Leu His Leu Tyr Glu Lys Gly Gly His Gly Phe Gly
275 280 285

Thr Arg Ala Arg Asn Leu Pro Ala Asp Gln Trp Leu Glu Ala Leu Glu
290 295 300

Ala Trp Leu Ile Ser Arg Arg Leu Ala Pro Pro Pro Ala Ala Ala Lys
305 310 315 320

His Lys

<210> 83
<211> 918
<212> DNA
<213> Sulfolobus solfataricus P1

<400> 83
atgcccctag atcctagaat taaaaagtta ctagaatcag ctcttactat accaattggt 60

aaagccccag tagaagaggt aagaaagata tttaggcaat tagcgtcggc agctcccaaa 120

gtcgaagttg gaaaagtaga agatataaaa ataccaggca gtgaaaccgt tataaacgct 180

agagtgtatt ttccgaagag tagcggtcct tatggtgttc tagtgtatct tcatggaggc 240

ggttttgtaa taggcgatgt ggaatcttat gacccattat gtagagcaat tacaaatgcg 300

tgcaattgcg ttgtagtatc agtggactat aggttagctc cagaatacaa gtttccttct 360

gcagttatcg attcatttga cgctactaat tgggtttata acaatttaga taaatttgat 420

ggaaagatgg gagttgctat tgcgggagat agtgctggag gaaatttggc agcggttgta 480

gctcttcttt caaagggtaa aattaatttg aagtatcaaa tactggttta cccagcggta 540

agtttagata acgtttcaag atccatgata gagtactctg atgggttctt ccttaccaga 600

gagcatatag agtggttcgg ttctcaatac ttacgaagcc ctgcagattt gctagacttt 660

aggttctctc caattctggc gcaagatttc aacggattac ctccagcctt gataataaca 720

gcagaatacg atccactaag ggatcaagga gaagcgtatg caaataaact actacaagct 780

ggagtctcag ttactagtgt gagatttaac aacgttatac acggattcct ctcattcttt 840

ccgttgatgg agcaaggaag agatgctata ggtctgatag ggtctgtgtt aagacgagta 900

```
ttttatgata aaatttaa
918

<210> 84
<211> 305
<212> PRT
<213> Sulfolobus solfataricus P1

<220>
<221> DOMAIN
<222> (74)...(278)
<223> alpha/beta hydrolase fold

<220>
<221> SITE
<222> (147)...(159)
<223> Lipolytic enzymes "G-D-X-G" family, putative serine active
site. Prosite id = PS01174

<220>
<221> SITE
<222> (186)...(189)
<223> N-glycosylation site. Prosite id = PS00001

<400> 84
Met Pro Leu Asp Pro Arg Ile Lys Lys Leu Leu Glu Ser Ala Leu Thr
1               5                   10                  15

Ile Pro Ile Gly Lys Ala Pro Val Glu Glu Val Arg Lys Ile Phe Arg
            20                  25                  30

Gln Leu Ala Ser Ala Ala Pro Lys Val Glu Val Gly Lys Val Glu Asp
        35                  40                  45

Ile Lys Ile Pro Gly Ser Glu Thr Val Ile Asn Ala Arg Val Tyr Phe
    50                  55                  60

Pro Lys Ser Ser Gly Pro Tyr Gly Val Leu Val Tyr Leu His Gly Gly
65                  70                  75                  80

Gly Phe Val Ile Gly Asp Val Glu Ser Tyr Asp Pro Leu Cys Arg Ala
                85                  90                  95

Ile Thr Asn Ala Cys Asn Cys Val Val Val Ser Val Asp Tyr Arg Leu
            100                 105                 110

Ala Pro Glu Tyr Lys Phe Pro Ser Ala Val Ile Asp Ser Phe Asp Ala
        115                 120                 125

Thr Asn Trp Val Tyr Asn Asn Leu Asp Lys Phe Asp Gly Lys Met Gly
    130                 135                 140

Val Ala Ile Ala Gly Asp Ser Ala Gly Gly Asn Leu Ala Ala Val Val
145                 150                 155                 160
```

Ala Leu Leu Ser Lys Gly Lys Ile Asn Leu Lys Tyr Gln Ile Leu Val
165 170 175

Tyr Pro Ala Val Ser Leu Asp Asn Val Ser Arg Ser Met Ile Glu Tyr
180 185 190

Ser Asp Gly Phe Phe Leu Thr Arg Glu His Ile Glu Trp Phe Gly Ser
195 200 205

Gln Tyr Leu Arg Ser Pro Ala Asp Leu Leu Asp Phe Arg Phe Ser Pro
210 215 220

Ile Leu Ala Gln Asp Phe Asn Gly Leu Pro Pro Ala Leu Ile Ile Thr
225 230 235 240

Ala Glu Tyr Asp Pro Leu Arg Asp Gln Gly Glu Ala Tyr Ala Asn Lys
245 250 255

Leu Leu Gln Ala Gly Val Ser Val Thr Ser Val Arg Phe Asn Asn Val
260 265 270

Ile His Gly Phe Leu Ser Phe Phe Pro Leu Met Glu Gln Gly Arg Asp
275 280 285

Ala Ile Gly Leu Ile Gly Ser Val Leu Arg Arg Val Phe Tyr Asp Lys
290 295 300

Ile
305

<210> 85
<211> 885
<212> DNA
<213> Unknown

<220>
<223> Obtained from environmental sample

<400> 85
atgaaggaaa catatcctga gtatagggtt gaacctttttg aattgggtga ttctgaaagc
60

aaagtaggtt gtcttctttt tcacggctac acggggtctc ctctggactt gttcccagtg
120

gccaagatat gcgaaaagta tgggtggcat gttagggctg ttaggcttcc gggccatgga
180

agacccattg aagaactaat caaggtcaag cacactgatt ggttgcgggc tgctgaaaaa
240

gaatacgagg aattcgccaa aaacaaggaa agagttattt tagttggtca ttccttgggt
300

aacgtcttga tttttcatct cgatgtaaag taccgggaag aaaagaagat aaccaatctt
360

gtttctattt cgccccggt tgttattagg aaaggctgga tgcttccttt tgttggtttg
420

gcaaagatat ttgttaggca catagattat tctgagattg cgtttaaaga caagaaaata
480

atgcaacacc cgttgtatga ctacttgagg ttcacgtaca agaaatttcc tttaggtatt
540

attaaggagt tagggaaggc ggttgagtac ggtgtcaagc ttgtgccttt gctcgagact
600

gatgtcattg ttctttttgg taaggaagat gatgtcgtgg atccattgtc gggatacgaa
660

atcttgatga aagcaaagag caggcacaag tccttggtga tgcttgaggg aatgcatgtg
720

ccaatgcttg acattgacta taaagcgttt caagaaagcc tgatctcgta tttcaatggc
780

ataagcgaga aaaaacttgg ctttgaagat actttcgaca tttttgattt gtttgatgag
840

gccgcccgga taactaatat tgctcttggt aagctaaaag gttga
885

<210> 86
<211> 294
<212> PRT
<213> Unknown

<220>
<223> Obtained from environmental sample

<400> 86
Met Lys Glu Thr Tyr Pro Glu Tyr Arg Val Glu Pro Phe Glu Leu Gly
1 5 10 15

Asp Ser Glu Ser Lys Val Gly Cys Leu Leu Phe His Gly Tyr Thr Gly
20 25 30

Ser Pro Leu Asp Leu Phe Pro Val Ala Lys Ile Cys Glu Lys Tyr Gly
35 40 45

Trp His Val Arg Ala Val Arg Leu Pro Gly His Gly Arg Pro Ile Glu
50 55 60

Glu Leu Ile Lys Val Lys His Thr Asp Trp Leu Arg Ala Ala Glu Lys
65 70 75 80

Glu Tyr Glu Glu Phe Ala Lys Asn Lys Glu Arg Val Ile Leu Val Gly
85 90 95

His Ser Leu Gly Asn Val Leu Ile Phe His Leu Asp Val Lys Tyr Arg
100 105 110

Glu Glu Lys Lys Ile Thr Asn Leu Val Ser Ile Ser Pro Pro Val Val

115 120 125

Ile Arg Lys Gly Trp Met Leu Pro Phe Val Gly Leu Ala Lys Ile Phe
130 135 140

Val Arg His Ile Asp Tyr Ser Glu Ile Ala Phe Lys Asp Lys Lys Ile
145 150 155 160

Met Gln His Pro Leu Tyr Asp Tyr Leu Arg Phe Thr Tyr Lys Lys Phe
165 170 175

Pro Leu Gly Ile Ile Lys Glu Leu Gly Lys Ala Val Glu Tyr Gly Val
180 185 190

Lys Leu Val Pro Leu Leu Glu Thr Asp Val Ile Val Leu Phe Gly Lys
195 200 205

Glu Asp Asp Val Val Asp Pro Leu Ser Gly Tyr Glu Ile Leu Met Lys
210 215 220

Ala Lys Ser Arg His Lys Ser Leu Val Met Leu Glu Gly Met His Val
225 230 235 240

Pro Met Leu Asp Ile Asp Tyr Lys Ala Phe Gln Glu Ser Leu Ile Ser
245 250 255

Tyr Phe Asn Gly Ile Ser Glu Lys Lys Leu Gly Phe Glu Asp Thr Phe
260 265 270

Asp Ile Phe Asp Leu Phe Asp Glu Ala Ala Arg Ile Thr Asn Ile Ala
275 280 285

Leu Gly Lys Leu Lys Gly
290

<210> 87
<211> 891
<212> DNA
<213> Unknown

<220>
<223> Obtained from environmental sample

<400> 87
atggatggcg gcacggccaa ggtgggcgac ggcatcgcct atgccgacgg gccgcggcac
60

aagctcgaca tctatgcgcc cgagcaacgc ggcgcgcccg ccccggtcgt gtttttcatc
120

tatggcggcg gctggaaccg gggcgagcgc agcgactatc agttcgtcgg gcgcgccctg
180

gcctcgcgcg gcttcgtcac tgtcattgcc gactatcggc tggtgccgga agtacggttt

240

cccggcttcc tgaccgacag cgccaatgcg ttgcgctggg tgcaggacaa tattgccaat
300

tacggcggcg accccaatcg gctgtttctc gccgggcatt cggccggtgc ctataatgcg
360

gtcatgctgg cgctcgaccc tgcctatcgg caggaattcg gcgtcaccat gccgatcctg
420

gcggtggcgg ccctgtcggg cccctatgat ttctatccgt tcgaatattt cgaggtgcag
480

gaggctttcg gccaggcgcc caatccggaa gggacgcagc cgatcaacct gatcaccgcc
540

gaggcgccac cgatgtatct ggcgaccggc actaccgatc cgatcgtgcg ggtgcagaat
600

accgaacgct ttgccgatcg gctgcgggcc cagggcgtgt gggtgacgac cagatattac
660

gaggggttcg ggcatatgga accggtgatc gccatgggcg cgctgtggcg ttggcgcatg
720

ccggtactcg atgagatggt cggctttttc cagcgtttcg gcgcctttcc cagcggcgtg
780

ccctatctcg ccgtgacgcc ggaagcgcca caaatgctgc ccgattcggt ggcgccgacc
840

gatgaaatcg tccgccagct caacgagatg ttccagccga tcgaggaata g
891

<210> 88
<211> 296
<212> PRT
<213> Unknown

<220>
<223> Obtained from environmental sample

<220>
<221> DOMAIN
<222> (37)...(229)
<223> alpha/beta hydrolase fold

<220>
<221> DOMAIN
<222> (51)...(256)
<223> Prolyl oligopeptidase family

<400> 88
Met Asp Gly Gly Thr Ala Lys Val Gly Asp Gly Ile Ala Tyr Ala Asp
1               5                   10                  15

Gly Pro Arg His Lys Leu Asp Ile Tyr Ala Pro Glu Gln Arg Gly Ala
            20                  25                  30

Pro Ala Pro Val Val Phe Phe Ile Tyr Gly Gly Gly Trp Asn Arg Gly
        35                  40                  45

```
Glu Arg Ser Asp Tyr Gln Phe Val Gly Arg Ala Leu Ala Ser Arg Gly
    50                  55                  60

Phe Val Thr Val Ile Ala Asp Tyr Arg Leu Val Pro Glu Val Arg Phe
65                  70                  75                  80

Pro Gly Phe Leu Thr Asp Ser Ala Asn Ala Leu Arg Trp Val Gln Asp
                85                  90                  95

Asn Ile Ala Asn Tyr Gly Gly Asp Pro Asn Arg Leu Phe Leu Ala Gly
            100                 105                 110

His Ser Ala Gly Ala Tyr Asn Ala Val Met Leu Ala Leu Asp Pro Ala
        115                 120                 125

Tyr Arg Gln Glu Phe Gly Val Thr Met Pro Ile Leu Ala Val Ala Ala
    130                 135                 140

Leu Ser Gly Pro Tyr Asp Phe Tyr Pro Phe Glu Tyr Phe Glu Val Gln
145                 150                 155                 160

Glu Ala Phe Gly Gln Ala Pro Asn Pro Glu Gly Thr Gln Pro Ile Asn
                165                 170                 175

Leu Ile Thr Ala Glu Ala Pro Pro Met Tyr Leu Ala Thr Gly Thr Thr
            180                 185                 190

Asp Pro Ile Val Arg Val Gln Asn Thr Glu Arg Phe Ala Asp Arg Leu
        195                 200                 205

Arg Ala Gln Gly Val Trp Val Thr Thr Arg Tyr Tyr Glu Gly Phe Gly
    210                 215                 220

His Met Glu Pro Val Ile Ala Met Gly Ala Leu Trp Arg Trp Arg Met
225                 230                 235                 240

Pro Val Leu Asp Glu Met Val Gly Phe Phe Gln Arg Phe Gly Ala Phe
                245                 250                 255

Pro Ser Gly Val Pro Tyr Leu Ala Val Thr Pro Glu Ala Pro Gln Met
            260                 265                 270

Leu Pro Asp Ser Val Ala Pro Thr Asp Glu Ile Val Arg Gln Leu Asn
        275                 280                 285

Glu Met Phe Gln Pro Ile Glu Glu
    290                 295
```

<210> 89
<211> 891

<212> DNA
<213> Unknown

<220>
<223> Obtained from environmental sample

<400> 89
atggcaagtg aagcactgac gatgatcgtg aacttgttgc gcagccagcg cccgttgcag
60

gaaccgacgg tggaacaaat gcgtgcgggg ttggaggcga tggcgcaaat gtcgccgcta
120

cctgcggacg tggaattgac caccgtggac gcaggaggtg tgcctggtgc ctgggtgcgc
180

gtgcccgaga gcgaccccga ccgcgtcgtg ctgtaccttc atggaggagg gtacgtcatt
240

gggtcgattc ggacacatcg cgaccttgct cagcgaattg cccgcgctgc ccgttgccgt
300

gtgttgttga tcgactaccg gctcgcaccc gaacacccgc atccggccgc ggtggaagac
360

tcgacgcgag cgtaccgatg gctcctcgag accggaagcg cccccaaacg catggccatt
420

gccggcgatt cggccggcgg cgggctgacg gtggcgacac tggtcgcact gcgagacgcg
480

ggagtgcccc tgcccgctgc cgccgtatgc ttatcgcctt gggtcgactt ggagggaatc
540

ggcgagtcga tgaccacgaa ggccgcagtc gatccgatgg tccaacgaga gccgttgctc
600

cgcatggcgt cgatgtacct ggcgggtcag gacccgcgca cgcctctggc ggctccgttg
660

tacgccgact tgcgagagct gccgcccttg ttgattcagg tgggtactgc ggaaaccctt
720

ctcgacgact cgatgcgcct ggctgagcgc gcacgcgctg cgggcgtaca ggtgacgctc
780

gaaccttggg aagacatgat tcacgtatgg caggcgttcg cggcaatgct tcccgagggc
840

cagcaggcga tcgagcggat tggcgagttt ttacgccagc attggcagta a
891

<210> 90
<211> 296
<212> PRT
<213> Unknown

<220>
<223> Obtained from environmental sample

<220>
<221> DOMAIN
<222> (70)...(272)
<223> alpha/beta hydrolase fold

<220>

<221> SITE
<222> (71)...(87)
<223> Lipolytic enzymes "G-D-X-G" family, putative histidine active site. Prosite id = PS01173

<400> 90

```
Met Ala Ser Glu Ala Leu Thr Met Ile Val Asn Leu Leu Arg Ser Gln
1               5                   10                  15

Arg Pro Leu Gln Glu Pro Thr Val Glu Gln Met Arg Ala Gly Leu Glu
            20                  25                  30

Ala Met Ala Gln Met Ser Pro Leu Pro Ala Asp Val Glu Leu Thr Thr
        35                  40                  45

Val Asp Ala Gly Gly Val Pro Gly Ala Trp Val Arg Val Pro Glu Ser
    50                  55                  60

Asp Pro Asp Arg Val Val Leu Tyr Leu His Gly Gly Gly Tyr Val Ile
65                  70                  75                  80

Gly Ser Ile Arg Thr His Arg Asp Leu Ala Gln Arg Ile Ala Arg Ala
                85                  90                  95

Ala Arg Cys Arg Val Leu Leu Ile Asp Tyr Arg Leu Ala Pro Glu His
            100                 105                 110

Pro His Pro Ala Ala Val Glu Asp Ser Thr Arg Ala Tyr Arg Trp Leu
        115                 120                 125

Leu Glu Thr Gly Ser Ala Pro Lys Arg Met Ala Ile Ala Gly Asp Ser
    130                 135                 140

Ala Gly Gly Gly Leu Thr Val Ala Thr Leu Val Ala Leu Arg Asp Ala
145                 150                 155                 160

Gly Val Pro Leu Pro Ala Ala Ala Val Cys Leu Ser Pro Trp Val Asp
                165                 170                 175

Leu Glu Gly Ile Gly Glu Ser Met Thr Thr Lys Ala Ala Val Asp Pro
            180                 185                 190

Met Val Gln Arg Glu Pro Leu Leu Arg Met Ala Ser Met Tyr Leu Ala
        195                 200                 205

Gly Gln Asp Pro Arg Thr Pro Leu Ala Ala Pro Leu Tyr Ala Asp Leu
    210                 215                 220

Arg Glu Leu Pro Pro Leu Leu Ile Gln Val Gly Thr Ala Glu Thr Leu
225                 230                 235                 240
```

Leu Asp Asp Ser Met Arg Leu Ala Glu Arg Ala Arg Ala Ala Gly Val
245 250 255

Gln Val Thr Leu Glu Pro Trp Glu Asp Met Ile His Val Trp Gln Ala
260 265 270

Phe Ala Ala Met Leu Pro Glu Gly Gln Gln Ala Ile Glu Arg Ile Gly
275 280 285

Glu Phe Leu Arg Gln His Trp Gln
290 295

<210> 91
<211> 1578
<212> DNA
<213> Unknown

<220>
<223> Obtained from environmental sample

<400> 91
atgagatcag cagctcgcat cagcgtggcg gcagttgcct ttctttgcct gctcttgacg 60

actcgggttt ccgcccagat cgtgccggcg atggaatgtg cggatctggc gaatcagcag 120

cttcccaaca cgacgatcac ctcggcccag accgtcacca ccggatcgtt aacgcccccg 180

ggctcgacga atccgatcac cgacctgcct cctttctgcc gtgtcacagg cgccatcgcc 240

ccgacgagcg agtcgcacat cctcttcgag gtctggctgc cgctggataa atggaacggc 300

aagttcgccg gcgtgggcaa cggcggctgg gccggcatca tctccttcgg cgccctcgga 360

agccagctca ggcgcggcta cgcgaccgcc tccacgaata cgggtcacga agcggcgccg 420

gggatgaacg cagccaggtt tgcgttcgag aagccggagc agcttatcga cttcgcctat 480

cgctcccagc acgagacggc cctgaaagcg aaggcgctgg ttcaggcttt ctacgggaag 540

ccgccggaac actcctattt catcgggtgc tcatcgggtg ggtaccaggg cctgatggag 600

gcccaacgat ttccggccga ctacgacggg atcgtcgccg gtatgccggc gaacaactgg 660

acacggctga tggccggcga cttggacgcg atccttgccg tctccgtaga tcctgccagc 720

caccttcccg tctccgcatt gggtctgttg tatcgctcgg tgctcgctgc ctgcgacggc 780

atcgacggtg ttgtagacgg tgttctggag gatccgcgcc gatgccggtt cgacccggcc 840

gtgttgatgt gcaaggcgga tcagaatccc gatggctgcc ttacgccggc tcaggtggaa
900

gcggcacggc gcatatacgg cggtctgaag gatcccaaga ccggcgctca gctctatccg
960

gggctggcgc cgggaagcga gccgttctgg ccgcaccgca atccggcgaa tccgttccct
1020

attccgatcg cgcactacaa gtggctcgtc tttgccgatc caaactggga ttggagaaca
1080

ttcaagttca cggatccggc ggactaccag gctttcctca atgcggaagc cacgtatgcc
1140

cctactctca atgcgaccaa tccggacctc cgggagttca gccggcgcgg cggcaggttg
1200

attcagtacc atggctggaa cgatcagctg attgccccgc aaaacagcat cgactattac
1260

gagagcgtcc tttcgttctt cgggtccggc aaacaggatc gagcgcagac cgtgcgcgag
1320

gttcagagct tctaccggct gttcatggcg ccgggtatgg ctcactgtgg aggcggtaca
1380

ggtccgaact catttgacat gctggatgcc ctcgagaagt gggtggaagg cgggatagcg
1440

ccggaacgag tccttgcgac gcgttccata aacggcgtag tcgaccggct gcgcccgctc
1500

tgtccatatc cgcaggtcgc cgtgtacaag ggtcatgggg atacaaacga cgccgcgaac
1560

ttcgtctgtc gcgattag
1578

<210> 92
<211> 525
<212> PRT
<213> Unknown

<220>
<223> Obtained from environmental sample

<220>
<221> SIGNAL
<222> (1)...(25)

<220>
<221> DOMAIN
<222> (73)...(525)
<223> Tannase and feruloyl esterase

<220>
<221> SITE
<222> (43)...(46)
<223> N-glycosylation site. Prosite id = PS00001

<220>
<221> SITE
<222> (222)...(225)
<223> N-glycosylation site. Prosite id = PS00001

```
<220>
<221> SITE
<222> (389)...(392)
<223> N-glycosylation site. Prosite id = PS00001

<400> 92
Met Arg Ser Ala Ala Arg Ile Ser Val Ala Ala Val Ala Phe Leu Cys
1               5                   10                  15

Leu Leu Leu Thr Thr Arg Val Ser Ala Gln Ile Val Pro Ala Met Glu
            20                  25                  30

Cys Ala Asp Leu Ala Asn Gln Gln Leu Pro Asn Thr Thr Ile Thr Ser
        35                  40                  45

Ala Gln Thr Val Thr Thr Gly Ser Leu Thr Pro Pro Gly Ser Thr Asn
    50                  55                  60

Pro Ile Thr Asp Leu Pro Pro Phe Cys Arg Val Thr Gly Ala Ile Ala
65                  70                  75                  80

Pro Thr Ser Glu Ser His Ile Leu Phe Glu Val Trp Leu Pro Leu Asp
                85                  90                  95

Lys Trp Asn Gly Lys Phe Ala Gly Val Gly Asn Gly Gly Trp Ala Gly
            100                 105                 110

Ile Ile Ser Phe Gly Ala Leu Gly Ser Gln Leu Arg Arg Gly Tyr Ala
        115                 120                 125

Thr Ala Ser Thr Asn Thr Gly His Glu Ala Ala Pro Gly Met Asn Ala
    130                 135                 140

Ala Arg Phe Ala Phe Glu Lys Pro Glu Gln Leu Ile Asp Phe Ala Tyr
145                 150                 155                 160

Arg Ser Gln His Glu Thr Ala Leu Lys Ala Lys Ala Leu Val Gln Ala
                165                 170                 175

Phe Tyr Gly Lys Pro Pro Glu His Ser Tyr Phe Ile Gly Cys Ser Ser
            180                 185                 190

Gly Gly Tyr Gln Gly Leu Met Glu Ala Gln Arg Phe Pro Ala Asp Tyr
        195                 200                 205

Asp Gly Ile Val Ala Gly Met Pro Ala Asn Asn Trp Thr Arg Leu Met
    210                 215                 220

Ala Gly Asp Leu Asp Ala Ile Leu Ala Val Ser Val Asp Pro Ala Ser
225                 230                 235                 240
```

His Leu Pro Val Ser Ala Leu Gly Leu Leu Tyr Arg Ser Val Leu Ala
245 250 255

Ala Cys Asp Gly Ile Asp Gly Val Val Asp Gly Val Leu Glu Asp Pro
260 265 270

Arg Arg Cys Arg Phe Asp Pro Ala Val Leu Met Cys Lys Ala Asp Gln
275 280 285

Asn Pro Asp Gly Cys Leu Thr Pro Ala Gln Val Glu Ala Ala Arg Arg
290 295 300

Ile Tyr Gly Gly Leu Lys Asp Pro Lys Thr Gly Ala Gln Leu Tyr Pro
305 310 315 320

Gly Leu Ala Pro Gly Ser Glu Pro Phe Trp Pro His Arg Asn Pro Ala
325 330 335

Asn Pro Phe Pro Ile Pro Ile Ala His Tyr Lys Trp Leu Val Phe Ala
340 345 350

Asp Pro Asn Trp Asp Trp Arg Thr Phe Lys Phe Thr Asp Pro Ala Asp
355 360 365

Tyr Gln Ala Phe Leu Asn Ala Glu Ala Thr Tyr Ala Pro Thr Leu Asn
370 375 380

Ala Thr Asn Pro Asp Leu Arg Glu Phe Ser Arg Arg Gly Gly Arg Leu
385 390 395 400

Ile Gln Tyr His Gly Trp Asn Asp Gln Leu Ile Ala Pro Gln Asn Ser
405 410 415

Ile Asp Tyr Tyr Glu Ser Val Leu Ser Phe Phe Gly Ser Gly Lys Gln
420 425 430

Asp Arg Ala Gln Thr Val Arg Glu Val Gln Ser Phe Tyr Arg Leu Phe
435 440 445

Met Ala Pro Gly Met Ala His Cys Gly Gly Gly Thr Gly Pro Asn Ser
450 455 460

Phe Asp Met Leu Asp Ala Leu Glu Lys Trp Val Glu Gly Gly Ile Ala
465 470 475 480

Pro Glu Arg Val Leu Ala Thr Arg Ser Ile Asn Gly Val Val Asp Arg
485 490 495

Leu Arg Pro Leu Cys Pro Tyr Pro Gln Val Ala Val Tyr Lys Gly His

500 505 510

Gly Asp Thr Asn Asp Ala Ala Asn Phe Val Cys Arg Asp
515 520 525

<210> 93
<211> 990
<212> DNA
<213> Unknown

<220>
<223> Obtained from environmental sample

<400> 93
atgggcgcgt tcgaggccac cagcgcgcag ggtgacgtcg ccagccgcga cgaactgctg 60

gccgaggcgg ccagcgacga agcggtggcc cagcgggaga tgctcaccgc cttcctcggc 120

atgtgcgaca ccgaggagat cgcgccgtcg gcggggctca cggtctccga gcaccgcgtc 180

gcctctcagc ccgacggcaa caagatcaac atccggttca tccggcccga gggcgacgac 240

gtgctgccgt gcgtgtacta catccacggc ggtggcatgg cgtcgatgtc gtgctatgac 300

ggcaactacc gggcgtgggg ccggatcatc gccgcccagg gcgtggcggt tgccatggtc 360

gacttccgca acgcgctcac accgtcgtcg gccgaggagg tggccccgtt ccccgctggg 420

ctcaacgact gcgtgtcggg gctcaagtgg gtcgcggcca acgccgccga tctgcgcatc 480

gacccggccc gcatcgtcgt ggccggcgag agcggcggcg ggaacctcac cctcgccacc 540

ggcctgaagc tcaagcgcga cggcgacctc gggctgatca agggtctcta tgccctctgc 600

ccctacattg ccggcgagtg gccgctgccg cagaacccgt cgtcgaccga gaacgagggc 660

atcctcctgc acctgcacaa caaccgcggc cgcatgacgt acggcatcga ggagttcgag 720

gcgcggaacc cgctggcgtg gcccggcttc gccactgaag acgacgtcgc cggcctcgtg 780

cccaccatca tcagcgtgaa cgagtgcgac ccgctgcgcg acgagggcgt cggcttctac 840

cgcctcctcc tccgcgcggg tgtgcccacc cggtgccgcc aggtgatggg caccatccac 900

ggcaccgaga tcttcccgat gctgtgcccc gacgtgagcc gggacaccgc ggccagcatc 960

gccgacttcg cccgcaacgc cgggtcctga 990

<210> 94
<211> 329
<212> PRT
<213> Unknown

<220>
<223> Obtained from environmental sample

<220>
<221> DOMAIN
<222> (85)...(304)
<223> alpha/beta hydrolase fold

<220>
<221> SITE
<222> (177)...(180)
<223> N-glycosylation site. Prosite id = PS00001

<400> 94

```
Met Gly Ala Phe Glu Ala Thr Ser Ala Gln Gly Asp Val Ala Ser Arg
1               5                   10                  15

Asp Glu Leu Leu Ala Glu Ala Ala Ser Asp Glu Ala Val Ala Gln Arg
            20                  25                  30

Glu Met Leu Thr Ala Phe Leu Gly Met Cys Asp Thr Glu Glu Ile Ala
        35                  40                  45

Pro Ser Ala Gly Leu Thr Val Ser Glu His Arg Val Ala Ser Gln Pro
    50                  55                  60

Asp Gly Asn Lys Ile Asn Ile Arg Phe Ile Arg Pro Glu Gly Asp Asp
65                  70                  75                  80

Val Leu Pro Cys Val Tyr Tyr Ile His Gly Gly Gly Met Ala Ser Met
                85                  90                  95

Ser Cys Tyr Asp Gly Asn Tyr Arg Ala Trp Gly Arg Ile Ile Ala Ala
            100                 105                 110

Gln Gly Val Ala Val Ala Met Val Asp Phe Arg Asn Ala Leu Thr Pro
        115                 120                 125

Ser Ser Ala Glu Glu Val Ala Pro Phe Pro Ala Gly Leu Asn Asp Cys
    130                 135                 140

Val Ser Gly Leu Lys Trp Val Ala Ala Asn Ala Ala Asp Leu Arg Ile
145                 150                 155                 160

Asp Pro Ala Arg Ile Val Val Ala Gly Glu Ser Gly Gly Gly Asn Leu
                165                 170                 175

Thr Leu Ala Thr Gly Leu Lys Leu Lys Arg Asp Gly Asp Leu Gly Leu
            180                 185                 190
```

Ile Lys Gly Leu Tyr Ala Leu Cys Pro Tyr Ile Ala Gly Glu Trp Pro
195 200 205

Leu Pro Gln Asn Pro Ser Ser Thr Glu Asn Glu Gly Ile Leu Leu His
210 215 220

Leu His Asn Asn Arg Gly Arg Met Thr Tyr Gly Ile Glu Glu Phe Glu
225 230 235 240

Ala Arg Asn Pro Leu Ala Trp Pro Gly Phe Ala Thr Glu Asp Asp Val
245 250 255

Ala Gly Leu Val Pro Thr Ile Ile Ser Val Asn Glu Cys Asp Pro Leu
260 265 270

Arg Asp Glu Gly Val Gly Phe Tyr Arg Leu Leu Leu Arg Ala Gly Val
275 280 285

Pro Thr Arg Cys Arg Gln Val Met Gly Thr Ile His Gly Thr Glu Ile
290 295 300

Phe Pro Met Leu Cys Pro Asp Val Ser Arg Asp Thr Ala Ala Ser Ile
305 310 315 320

Ala Asp Phe Ala Arg Asn Ala Gly Ser
325

<210> 95
<211> 924
<212> DNA
<213> Unknown

<220>
<223> Obtained from environmental sample

<400> 95
gtggatccga aggcccagat cgtcggcgag ttcgtcaagt ccatccgcca gccgggttac
60

tttcccccgc tacccgagct gcgccagcag ctccggacga tggtggcgct gatggacgag
120

ccggcacccg cgctggcgcg catcgaggac cgccgcatcc cgggcccggc cggcgacata
180

ccggtgcgcg tgtacgcctc ggacgtcggc gcctcgcgtc ccgtcgtggc gtactttcac
240

ggcggcggct gggtgcaggg cgacctcgag actcaccacg gcctctgcgc gaggctggcg
300

aagcacgccg ggccctcgt cgtggccgtc gactaccggc tggcgcccga gcacaagttc
360

ccggccgccg tcgaggactg cctggcggcc tatcgctggc tccgagccca cggaggcgac

420

ctcggcgccg acccggggcg cgtggcggtg gccggggact cggcgggcgg caatctctcg
480

gccgtcgtct cgcaggtcgc agccgcggag ggcgctccgg tgcccacgtg ccaggtcctg
540

atctatccgg cggtggactt ctcgctcgac agcgactccc accgagagct ggcggagggc
600

cacatcatcc cgcgcgaccg tgtcctctgg tactcggagc agtacctgag ggcgaggcc
660

gacaagcggg atctgcgcgc ctcgcccctg cgggcgccga agctcgccgg ccagccgccg
720

acgctcgtcg tcaccgccgg gttcgatcct ctccgcgacg agggccgggc gtatgccgat
780

cggctccgcg aggccggggt ggacgtcgtc taccgcgagt acccgggcca gatccatgcc
840

ttcgtgtcgc tgacgaaggc gattccggag ggcatggcct gcacgctgga gattgccgag
900

tacctgcgcc agcgcctcgg ctga
924

<210> 96
<211> 307
<212> PRT
<213> Unknown

<220>
<223> Obtained from environmental sample

<220>
<221> DOMAIN
<222> (76)...(283)
<223> alpha/beta hydrolase fold

<220>
<221> SITE
<222> (160)...(163)
<223> N-glycosylation site. Prosite id = PS00001

<400> 96
Met Asp Pro Lys Ala Gln Ile Val Gly Glu Phe Val Lys Ser Ile Arg
1               5                   10                  15

Gln Pro Gly Tyr Phe Pro Pro Leu Pro Glu Leu Arg Gln Gln Leu Arg
            20                  25                  30

Thr Met Val Ala Leu Met Asp Glu Pro Ala Pro Ala Leu Ala Arg Ile
        35                  40                  45

Glu Asp Arg Arg Ile Pro Gly Pro Ala Gly Asp Ile Pro Val Arg Val
    50                  55                  60

Tyr Ala Ser Asp Val Gly Ala Ser Arg Pro Val Val Ala Tyr Phe His
65                  70                  75                  80

```
Gly Gly Gly Trp Val Gln Gly Asp Leu Glu Thr His His Gly Leu Cys
                85                  90                  95

Ala Arg Leu Ala Lys His Ala Gly Ala Leu Val Val Ala Val Asp Tyr
            100                 105                 110

Arg Leu Ala Pro Glu His Lys Phe Pro Ala Ala Val Glu Asp Cys Leu
        115                 120                 125

Ala Ala Tyr Arg Trp Leu Arg Ala His Gly Gly Asp Leu Gly Ala Asp
    130                 135                 140

Pro Gly Arg Val Ala Val Ala Gly Asp Ser Ala Gly Gly Asn Leu Ser
145                 150                 155                 160

Ala Val Val Ser Gln Val Ala Ala Ala Glu Gly Ala Pro Val Pro Thr
                165                 170                 175

Cys Gln Val Leu Ile Tyr Pro Ala Val Asp Phe Ser Leu Asp Ser Asp
            180                 185                 190

Ser His Arg Glu Leu Ala Glu Gly His Ile Ile Pro Arg Asp Arg Val
        195                 200                 205

Leu Trp Tyr Ser Glu Gln Tyr Leu Arg Gly Glu Ala Asp Lys Arg Asp
    210                 215                 220

Leu Arg Ala Ser Pro Leu Arg Ala Pro Lys Leu Ala Gly Gln Pro Pro
225                 230                 235                 240

Thr Leu Val Val Thr Ala Gly Phe Asp Pro Leu Arg Asp Glu Gly Arg
                245                 250                 255

Ala Tyr Ala Asp Arg Leu Arg Glu Ala Gly Val Asp Val Val Tyr Arg
            260                 265                 270

Glu Tyr Pro Gly Gln Ile His Ala Phe Val Ser Leu Thr Lys Ala Ile
        275                 280                 285

Pro Glu Gly Met Ala Cys Thr Leu Glu Ile Ala Glu Tyr Leu Arg Gln
    290                 295                 300

Arg Leu Gly
305
```

<210> 97
<211> 939
<212> DNA
<213> Unknown

<220>
<223> Obtained from environmental sample

<400> 97
atgccactcg acccaaaagc caaagcgttt ttagaccagg ccgctgcggc tggcgcgcca
60

gcactggacg cgcttcccgt tcgagaagct cgcgaggcga ttcgcggcct gtttgcggcc
120

ataggagaaa aagaacccgt caaacaaata gaagatcgca tgatccctgg cccgggcgga
180

cagatgcctg tgcgtatgta tacgccggca ggtaccacgc cttttcccgt cctcgtttat
240

ttccatggcg gaggatgggt cctgggtgat ctagaaactc acgatgcgac ctgccgggcg
300

ctggcaaatg ctgctggctg catggtgatc tcggttgact atcgtttggc gccggagcac
360

aaattcccta ccgctcccga ggattgctat gcggccacca aatgggcagc gctgaatgcg
420

gcggctcttg gcggagatcc aaccaagatt gccgttggtg gcgatagtgc cggagggaat
480

cttgctgccg cagtggcaca aatggccagt gaccgtgggg ctccttcgct cgggtttcag
540

ttgctcatct atccagtgac caattacgcg ttcgataccg cctcataccg ggcaaatgcg
600

gagggctacc tgctcacaaa aagcgcgatg gggtggttct ggaaccatta tctgcagaac
660

gaaaccgatg gccagaatcc ctatgcttcg cctttacgtg gacaatatct gcgcaatcta
720

cccccagcac ttgtcatcac cgcggagtat gacccgctcc gcgacgaggg agaagcgtat
780

gcagcgcggc tgcgcgaagc aggtgttgct gtcactcaca ctgactacaa aggcatgatt
840

catggcctct tcagcatgac caacgtagtc gatcaggcga agcaagccat caatgaagct
900

ggtgccgcac tgaaggcagc gtttgcggcg aaacgataa
939

<210> 98
<211> 312
<212> PRT
<213> Unknown

<220>
<223> Obtained from environmental sample

<220>
<221> DOMAIN
<222> (78)...(285)
<223> alpha/beta hydrolase fold

<220>
<221> SITE
<222> (223)...(226)
<223> N-glycosylation site. Prosite id = PS00001

<400> 98
Met Pro Leu Asp Pro Lys Ala Lys Ala Phe Leu Asp Gln Ala Ala Ala
1 5 10 15

Ala Gly Ala Pro Ala Leu Asp Ala Leu Pro Val Arg Glu Ala Arg Glu
20 25 30

Ala Ile Arg Gly Leu Phe Ala Ala Ile Gly Glu Lys Glu Pro Val Lys
35 40 45

Gln Ile Glu Asp Arg Met Ile Pro Gly Pro Gly Gly Gln Met Pro Val
50 55 60

Arg Met Tyr Thr Pro Ala Gly Thr Thr Pro Phe Pro Val Leu Val Tyr
65 70 75 80

Phe His Gly Gly Gly Trp Val Leu Gly Asp Leu Glu Thr His Asp Ala
85 90 95

Thr Cys Arg Ala Leu Ala Asn Ala Ala Gly Cys Met Val Ile Ser Val
100 105 110

Asp Tyr Arg Leu Ala Pro Glu His Lys Phe Pro Thr Ala Pro Glu Asp
115 120 125

Cys Tyr Ala Ala Thr Lys Trp Ala Ala Leu Asn Ala Ala Ala Leu Gly
130 135 140

Gly Asp Pro Thr Lys Ile Ala Val Gly Gly Asp Ser Ala Gly Gly Asn
145 150 155 160

Leu Ala Ala Ala Val Ala Gln Met Ala Ser Asp Arg Gly Ala Pro Ser
165 170 175

Leu Gly Phe Gln Leu Leu Ile Tyr Pro Val Thr Asn Tyr Ala Phe Asp
180 185 190

Thr Ala Ser Tyr Arg Ala Asn Ala Glu Gly Tyr Leu Leu Thr Lys Ser
195 200 205

Ala Met Gly Trp Phe Trp Asn His Tyr Leu Gln Asn Glu Thr Asp Gly
210 215 220

Gln Asn Pro Tyr Ala Ser Pro Leu Arg Gly Gln Tyr Leu Arg Asn Leu
225 230 235 240

Pro Pro Ala Leu Val Ile Thr Ala Glu Tyr Asp Pro Leu Arg Asp Glu
245 250 255

Gly Glu Ala Tyr Ala Ala Arg Leu Arg Glu Ala Gly Val Ala Val Thr
260 265 270

His Thr Asp Tyr Lys Gly Met Ile His Gly Leu Phe Ser Met Thr Asn
275 280 285

Val Val Asp Gln Ala Lys Gln Ala Ile Asn Glu Ala Gly Ala Ala Leu
290 295 300

Lys Ala Ala Phe Ala Ala Lys Arg
305 310

<210> 99
<211> 1038
<212> DNA
<213> Unknown

<220>
<223> Obtained from environmental sample

<400> 99
atgaaacgaa tcgcaaccgc cgtgtttttg ctgcatgcca tgacgtcggt ggccgtgtgc
60

gccgacgtcg cgggcgatac ccagaaacag atatggggac gctacctgga aaggagcgaa
120

gaagccgcgc tcacggcaat gataccgcaa caaaccccgc cgccggaaac cccgttgac
180

aaatcggccc gggaacgcat cggcgaaacc aggaaagcgt tcctgaatat gacggcggag
240

ttcgcgactt cgctggcgga cattccgacg acggagtatg accaggaggg tatccgggga
300

agctggatca tgcccaatga tgcggattcc gaacgcgtgc ttttgtatct tcacggcggc
360

ggctacatag tcggaagcga tgaaacgccc cgcgcgataa ccgcatttct ggcgcgcgag
420

gcaaaagtgc ggtgtttttc cctggattat ccgcttgccc ccgagcatcc atttccggct
480

gcgctcgata gcgcggtgcg atcgtatgaa atgctgttgg aaaaagggtt cagccctgga
540

aacatcgtgc tgggcggcga ttcggccggc ggcggcctgg cgcttgccct gttgctcgcc
600

atccgggaac acgggctgcc gatgccggcg ggagcgtatc tcctttcccc gtggactgat
660

ttgacgcaga gttttcccac gcattcacgc aaggccgatg tcgaaatcag cattaccccc
720

gagctgcttg aggaagcggc agccagctat gcgggtgatt ccgacagaac caatccgctt

780

atctcccccg cattcggcga gttccgggga ttcccgccgc tcctgataca agttggttcc
840

catgaacgtc tgttggacga ctccctgacg gtggcgcgca atgccgccct cgccgatgtg
900

ccgacgacat tgaaggtatg gcccggctat ccccatgtgt ttcaagtcta tcaccagaat
960

ctcgacggcg ccaaaaaggc gttgagggaa gccgcggaat tcatcaccgg agcgatggat
1020

aaagagttgc ttcaataa
1038

<210> 100
<211> 345
<212> PRT
<213> Unknown

<220>
<223> Obtained from environmental sample

<220>
<221> SIGNAL
<222> (1)...(21)

<220>
<221> DOMAIN
<222> (114)...(316)
<223> alpha/beta hydrolase fold

<220>
<221> SITE
<222> (77)...(80)
<223> N-glycosylation site. Prosite id = PS00001

<400> 100
Met Lys Arg Ile Ala Thr Ala Val Phe Leu Leu His Ala Met Thr Ser
1 5 10 15

Val Ala Val Cys Ala Asp Val Ala Gly Asp Thr Gln Lys Gln Ile Trp
20 25 30

Gly Arg Tyr Leu Glu Arg Ser Glu Glu Ala Ala Leu Thr Ala Met Ile
35 40 45

Pro Gln Gln Thr Pro Pro Pro Glu Thr Pro Val Asp Lys Ser Ala Arg
50 55 60

Glu Arg Ile Gly Glu Thr Arg Lys Ala Phe Leu Asn Met Thr Ala Glu
65 70 75 80

Phe Ala Thr Ser Leu Ala Asp Ile Pro Thr Thr Glu Tyr Asp Gln Glu
85 90 95

Gly Ile Arg Gly Ser Trp Ile Met Pro Asn Asp Ala Asp Ser Glu Arg
100 105 110

```
Val Leu Leu Tyr Leu His Gly Gly Gly Tyr Ile Val Gly Ser Asp Glu
        115                 120                 125

Thr Pro Arg Ala Ile Thr Ala Phe Leu Ala Arg Glu Ala Lys Val Arg
    130                 135                 140

Cys Phe Ser Leu Asp Tyr Pro Leu Ala Pro Glu His Pro Phe Pro Ala
145                 150                 155                 160

Ala Leu Asp Ser Ala Val Arg Ser Tyr Glu Met Leu Leu Glu Lys Gly
                165                 170                 175

Phe Ser Pro Gly Asn Ile Val Leu Gly Gly Asp Ser Ala Gly Gly Gly
            180                 185                 190

Leu Ala Leu Ala Leu Leu Leu Ala Ile Arg Glu His Gly Leu Pro Met
        195                 200                 205

Pro Ala Gly Ala Tyr Leu Leu Ser Pro Trp Thr Asp Leu Thr Gln Ser
    210                 215                 220

Phe Pro Thr His Ser Arg Lys Ala Asp Val Glu Ile Ser Ile Thr Pro
225                 230                 235                 240

Glu Leu Leu Glu Glu Ala Ala Ala Ser Tyr Ala Gly Asp Ser Asp Arg
                245                 250                 255

Thr Asn Pro Leu Ile Ser Pro Ala Phe Gly Glu Phe Arg Gly Phe Pro
            260                 265                 270

Pro Leu Leu Ile Gln Val Gly Ser His Glu Arg Leu Leu Asp Asp Ser
        275                 280                 285

Leu Thr Val Ala Arg Asn Ala Ala Leu Ala Asp Val Pro Thr Thr Leu
    290                 295                 300

Lys Val Trp Pro Gly Tyr Pro His Val Phe Gln Val Tyr His Gln Asn
305                 310                 315                 320

Leu Asp Gly Ala Lys Lys Ala Leu Arg Glu Ala Ala Glu Phe Ile Thr
                325                 330                 335

Gly Ala Met Asp Lys Glu Leu Leu Gln
            340                 345
```

<210> 101
<211> 906
<212> DNA
<213> Unknown

<220>
<223> Obtained from environmental sample

<400> 101
atgacacata caacagctaa cggaatcgac attgaatatg ataccttcgg agatagaaac
60

cgatccccgc tgcttcttat catgggactc agcagccaga tggttgcctg gcccgagtcg
120

ttttgtcgta aactcgcgcg aagcggccat tgggttctgc gttttgataa tcgcgacgtc
180

ggactttcat ccaaattcga tggcgtcggc ctacccgatc tgatggcggc catggcggcc
240

catctgcagg gacagccggt ggcagcgccg tatacccttt cagacatggc ggctgatgcc
300

gtgcggctga tggatggcct taagctggaa aacgcccatg tgtgcggatt atccatgggt
360

ggcatgatcg ctcaggttat ggcgctagag tatccccagc gcgtaacgag tcttatttcc
420

atggaatcct ccaccggcga tccgacgctg ccgcctgcag acccccagac catggaagcc
480

atgttatccc cgccaccaca ggatcgcgcc gggtacattc agcatgcggt cgaagtgttt
540

cgcgcatttt ccggcggatc cgataaattt gatgaaaccc tggaaaagga actctcggca
600

aactcatatg accgctcgtt ttatccggcc ggctttgttc gtcaactggc ggccatcctg
660

gcctccggcg accgcaccga gagtctggca tccgtgacgg ctcccacgct ggtgattcac
720

ggcgctaatg acccttttggt gccgctggcc catggtcgtg ccacagccag ggccataccg
780

ggagcaaaac tggtggtgat cgagggcctg ggtcacggga tcgcctatcc caccctgtgg
840

gatgagatcg ctgatgctat cacgcagcat acgagcgccg cttccggccg cgagcgtcca
900

gtctaa
906

<210> 102
<211> 301
<212> PRT
<213> Unknown

<220>
<223> Obtained from environmental sample

<220>
<221> DOMAIN
<222> (50)...(285)
<223> alpha/beta hydrolase fold

<400> 102
Met Thr His Thr Thr Ala Asn Gly Ile Asp Ile Glu Tyr Asp Thr Phe
1               5                   10                  15

Gly Asp Arg Asn Arg Ser Pro Leu Leu Leu Ile Met Gly Leu Ser Ser
            20                  25                  30

Gln Met Val Ala Trp Pro Glu Ser Phe Cys Arg Lys Leu Ala Arg Ser
        35                  40                  45

Gly His Trp Val Leu Arg Phe Asp Asn Arg Asp Val Gly Leu Ser Ser
    50                  55                  60

Lys Phe Asp Gly Val Gly Leu Pro Asp Leu Met Ala Ala Met Ala Ala
65                  70                  75                  80

His Leu Gln Gly Gln Pro Val Ala Ala Pro Tyr Thr Leu Ser Asp Met
                85                  90                  95

Ala Ala Asp Ala Val Arg Leu Met Asp Gly Leu Lys Leu Glu Asn Ala
            100                 105                 110

His Val Cys Gly Leu Ser Met Gly Gly Met Ile Ala Gln Val Met Ala
        115                 120                 125

Leu Glu Tyr Pro Gln Arg Val Thr Ser Leu Ile Ser Met Glu Ser Ser
    130                 135                 140

Thr Gly Asp Pro Thr Leu Pro Pro Ala Asp Pro Gln Thr Met Glu Ala
145                 150                 155                 160

Met Leu Ser Pro Pro Pro Gln Asp Arg Ala Gly Tyr Ile Gln His Ala
                165                 170                 175

Val Glu Val Phe Arg Ala Phe Ser Gly Gly Ser Asp Lys Phe Asp Glu
            180                 185                 190

Thr Leu Glu Lys Glu Leu Ser Ala Asn Ser Tyr Asp Arg Ser Phe Tyr
        195                 200                 205

Pro Ala Gly Phe Val Arg Gln Leu Ala Ala Ile Leu Ala Ser Gly Asp
    210                 215                 220

Arg Thr Glu Ser Leu Ala Ser Val Thr Ala Pro Thr Leu Val Ile His
225                 230                 235                 240

Gly Ala Asn Asp Pro Leu Val Pro Leu Ala His Gly Arg Ala Thr Ala
                245                 250                 255

Arg Ala Ile Pro Gly Ala Lys Leu Val Val Ile Glu Gly Leu Gly His

260 265 270

Gly Ile Ala Tyr Pro Thr Leu Trp Asp Glu Ile Ala Asp Ala Ile Thr
275 280 285

Gln His Thr Ser Ala Ala Ser Gly Arg Glu Arg Pro Val
290 295 300

<210> 103
<211> 948
<212> DNA
<213> Unknown

<220>
<223> Obtained from environmental sample

<400> 103
atgaaggagg ttgctccaaa cccggctcat gatgaggtca tagaacgcat caaggacatg
60

gggcgcgcct cccgcggcct gaaaggcaaa gcccggctga tcgccctccg caatcacctg
120

gatagcctgg gtgaaggcgt ggatatcgaa tcggacatcc gtcatcagga tcatccgcgt
180

ggagaatgga tattggcgcc cggggccgat tcccgccgac gtgtgctcta tattcacggc
240

ggtgcctggg ctgctggcag ccctcgcagt caccgggcga ttaccgaccg tttttcccgg
300

attgccaatg ctgccgtttt tgcggttgat taccggctca tgcccgaaca ccggttcatg
360

gatggcattc gggactgccg ccaggcctat gcatggctgc tgaatcatgg cccggacggc
420

gaagcgcccg tcgacttcat ggtggttgcc ggagactcgg cgggcggtag ccataccctg
480

tctctgctgg cgtggattcg cgacaatggc ctgcggcagg ccgatgccgc cgttgccctg
540

tcaccctcca cggatctcac gctgacggcg cctagcaatc gcgagaatat tcgcacggac
600

ccactgctgg gcccggtgtt tggtggtttg tccaaaatcc cgctgcccgt gttgtggtgg
660

ggaacacttg ccgcgttcag aatctctccc accaacccgg ttgcatcacc tctgcggggt
720

gagcttaaca agctgccacc cgtgcttatc cacgcaagca ccacggaaat gctgctggac
780

aacgccacgc gttacgccgc caaggcaaaa gcccagggtt caccggtgga gcttcacacc
840

tggcagaaca tggtgcatgt atggcacatc ttcacgccac ttctgcccga ggcagacgag
900

gcgtttgaag acatcgcggt gtttcttcag aaagtggaac agcaatag
948

<210> 104
<211> 315
<212> PRT
<213> Unknown

<220>
<223> Obtained from environmental sample

<220>
<221> DOMAIN
<222> (75)...(290)
<223> alpha/beta hydrolase fold

<220>
<221> SITE
<222> (265)...(268)
<223> N-glycosylation site. Prosite id = PS00001

<400> 104
Met Lys Glu Val Ala Pro Asn Pro Ala His Asp Glu Val Ile Glu Arg
1 . 5 10 15

Ile Lys Asp Met Gly Arg Ala Ser Arg Gly Leu Lys Gly Lys Ala Arg
20 25 30

Leu Ile Ala Leu Arg Asn His Leu Asp Ser Leu Gly Glu Gly Val Asp
35 40 45

Ile Glu Ser Asp Ile Arg His Gln Asp His Pro Arg Gly Glu Trp Ile
50 55 60

Leu Ala Pro Gly Ala Asp Ser Arg Arg Arg Val Leu Tyr Ile His Gly
65 70 75 80

Gly Ala Trp Ala Ala Gly Ser Pro Arg Ser His Arg Ala Ile Thr Asp
85 90 95

Arg Phe Ser Arg Ile Ala Asn Ala Ala Val Phe Ala Val Asp Tyr Arg
100 105 110

Leu Met Pro Glu His Arg Phe Met Asp Gly Ile Arg Asp Cys Arg Gln
115 120 125

Ala Tyr Ala Trp Leu Leu Asn His Gly Pro Asp Gly Glu Ala Pro Val
130 135 140

Asp Phe Met Val Val Ala Gly Asp Ser Ala Gly Gly Ser His Thr Leu
145 150 155 160

Ser Leu Leu Ala Trp Ile Arg Asp Asn Gly Leu Arg Gln Ala Asp Ala
165 170 175 .

Ala Val Ala Leu Ser Pro Ser Thr Asp Leu Thr Leu Thr Ala Pro Ser
180 185 190

Asn Arg Glu Asn Ile Arg Thr Asp Pro Leu Leu Gly Pro Val Phe Gly
195 200 205

Gly Leu Ser Lys Ile Pro Leu Pro Val Leu Trp Trp Gly Thr Leu Ala
210 215 220

Ala Phe Arg Ile Ser Pro Thr Asn Pro Val Ala Ser Pro Leu Arg Gly
225 230 235 240

Glu Leu Asn Lys Leu Pro Pro Val Leu Ile His Ala Ser Thr Thr Glu
245 250 255

Met Leu Leu Asp Asn Ala Thr Arg Tyr Ala Ala Lys Ala Lys Ala Gln
260 265 270

Gly Ser Pro Val Glu Leu His Thr Trp Gln Asn Met Val His Val Trp
275 280 285

His Ile Phe Thr Pro Leu Leu Pro Glu Ala Asp Glu Ala Phe Glu Asp
290 295 300

Ile Ala Val Phe Leu Gln Lys Val Glu Gln Gln
305 310 315

<210> 105
<211> 747
<212> DNA
<213> Unknown

<220>
<223> Obtained from environmental sample

<400> 105
atgaaaatta agttgccaga accattcaca ttcgaagcag gaaatcgagc agttttatta
60

ttacatggat ttactggcca ttcagcagat gtaagaatgc ttggtcgatt tctagaaaag
120

aaaggttata caactcatgc accaatttat agagggcatg gtcaagagcc agaagcatta
180

ttaaaatctt cacctgatga atggtgggag gatattttat cagcttataa tcatttgaga
240

aatctaggat ataatgaaat tgctgttgct ggtctttcaa tgggtggtgc tttagcaatt
300

aaactagcta ccaagcatga aataaaaggt gtcattccaa tgtgtacacc aatgtacttt
360

gataatcaaa aacaattaac taaagcattt ttaaactttg cacgacaatt taaaaaattt
420

gagaaaaaag atgaacaaac aattaaaaaa gaaatagatt tactacaaca aaattcagca
480

gaattattta atgaaatcgg cacttttgta gaacaagtca atggtataat agatagagtt
540

gacgtaccta cattcgtagt tcaagcaagt aaagatgaaa taataaatcc tgaaagtgcg
600

acatttattt atgaaaacat taaaaatgaa aaaaaagatt taaaatggta taaaaactca
660

actcacttga taacatttgg cgatgaaaaa gatgttttac atgaagatat atatcatttt
720

ttagaaacat tagattggaa caactaa
747

<210> 106
<211> 248
<212> PRT
<213> Unknown

<220>
<223> Obtained from environmental sample

<220>
<221> SITE
<222> (222)...(225)
<223> N-glycosylation site. Prosite id = PS00001

<400> 106
Met Lys Ile Lys Leu Pro Glu Pro Phe Thr Phe Glu Ala Gly Asn Arg
1 5 10 15

Ala Val Leu Leu Leu His Gly Phe Thr Gly His Ser Ala Asp Val Arg
20 25 30

Met Leu Gly Arg Phe Leu Glu Lys Lys Gly Tyr Thr Thr His Ala Pro
35 40 45

Ile Tyr Arg Gly His Gly Gln Glu Pro Glu Ala Leu Leu Lys Ser Ser
50 55 60

Pro Asp Glu Trp Trp Glu Asp Ile Leu Ser Ala Tyr Asn His Leu Arg
65 70 75 80

Asn Leu Gly Tyr Asn Glu Ile Ala Val Ala Gly Leu Ser Met Gly Gly
85 90 95

Ala Leu Ala Ile Lys Leu Ala Thr Lys His Glu Ile Lys Gly Val Ile
100 105 110

Pro Met Cys Thr Pro Met Tyr Phe Asp Asn Gln Lys Gln Leu Thr Lys
115 120 125

Ala Phe Leu Asn Phe Ala Arg Gln Phe Lys Lys Phe Glu Lys Lys Asp
130 135 140

Glu Gln Thr Ile Lys Lys Glu Ile Asp Leu Leu Gln Gln Asn Ser Ala
145 150 155 160

Glu Leu Phe Asn Glu Ile Gly Thr Phe Val Glu Gln Val Asn Gly Ile
165 170 175

Ile Asp Arg Val Asp Val Pro Thr Phe Val Val Gln Ala Ser Lys Asp
180 185 190

Glu Ile Ile Asn Pro Glu Ser Ala Thr Phe Ile Tyr Glu Asn Ile Lys
195 200 205

Asn Glu Lys Lys Asp Leu Lys Trp Tyr Lys Asn Ser Thr His Leu Ile
210 215 220

Thr Phe Gly Asp Glu Lys Asp Val Leu His Glu Asp Ile Tyr His Phe
225 230 235 240

Leu Glu Thr Leu Asp Trp Asn Asn
245

<210> 107
<211> 747
<212> DNA
<213> Unknown

<220>
<223> Obtained from environmental sample

<400> 107
atgaaaatta agttgccaga accattcaca ttcgaagcag gaaatcgagc agttttatta
60

ttacatggat ttactggcca ttcagcagat gtaagaatgc ttggtcgatt tctagaaaag
120

aaaggttata caactcatgc accaatttat agagggcatg gtcaagagcc agaagcatta
180

ttaaaatctt cacctgatga atggtgggag gatattttat cagcttataa tcatttgaga
240

aatctaggat ataatgaaat tgctgttgct ggtctttcaa tgggtggtgc tttagcaatt
300

aaactagcta ccaagcatga aataaaaggt gtcattccaa tgtgtacacc aatgtacttt
360

gataatcaaa aacaattaac tcaagcattt ttaaactttg cacgacaatt taaaaaattt
420

gagaaaaaag atgaacaaac aattaaaaaa gaaatagatt tactacaaca aaattcagca
480

gaattattta atgaaatcgg cacttttgta gaacaagtca atggtataat agatagagtt
540

gacgtaccta cattcgtagt tcaagcaagt aaagatgaaa taataaatcc tgaaagtgcg
600

acatttattt atgaaaacat taaaaatgaa aaaaaagatt taaaatggta taaaaactca
660

actcacttga taacatttgg cgatgaaaaa gatgttttac atgaagatat atatcatttt
720

ttagaaacat tagattggaa caactaa
747

<210> 108
<211> 248
<212> PRT
<213> Unknown

<220>
<223> Obtained from environmental sample

<220>
<221> SITE
<222> (222)...(225)
<223> N-glycosylation site. Prosite id = PS00001

<400> 108
Met Lys Ile Lys Leu Pro Glu Pro Phe Thr Phe Glu Ala Gly Asn Arg
1               5                   10                  15

Ala Val Leu Leu Leu His Gly Phe Thr Gly His Ser Ala Asp Val Arg
            20                  25                  30

Met Leu Gly Arg Phe Leu Glu Lys Lys Gly Tyr Thr Thr His Ala Pro
        35                  40                  45

Ile Tyr Arg Gly His Gly Gln Glu Pro Glu Ala Leu Leu Lys Ser Ser
    50                  55                  60

Pro Asp Glu Trp Trp Glu Asp Ile Leu Ser Ala Tyr Asn His Leu Arg
65                  70                  75                  80

Asn Leu Gly Tyr Asn Glu Ile Ala Val Ala Gly Leu Ser Met Gly Gly
                85                  90                  95

Ala Leu Ala Ile Lys Leu Ala Thr Lys His Glu Ile Lys Gly Val Ile
            100                 105                 110

Pro Met Cys Thr Pro Met Tyr Phe Asp Asn Gln Lys Gln Leu Thr Gln
        115                 120                 125

Ala Phe Leu Asn Phe Ala Arg Gln Phe Lys Lys Phe Glu Lys Lys Asp
    130                 135                 140

Glu Gln Thr Ile Lys Lys Glu Ile Asp Leu Leu Gln Gln Asn Ser Ala
145                 150                 155                 160

Glu Leu Phe Asn Glu Ile Gly Thr Phe Val Glu Gln Val Asn Gly Ile
165 170 175

Ile Asp Arg Val Asp Val Pro Thr Phe Val Val Gln Ala Ser Lys Asp
180 185 190

Glu Ile Ile Asn Pro Glu Ser Ala Thr Phe Ile Tyr Glu Asn Ile Lys
195 200 205

Asn Glu Lys Lys Asp Leu Lys Trp Tyr Lys Asn Ser Thr His Leu Ile
210 215 220

Thr Phe Gly Asp Glu Lys Asp Val Leu His Glu Asp Ile Tyr His Phe
225 230 235 240

Leu Glu Thr Leu Asp Trp Asn Asn
245

<210> 109
<211> 750
<212> DNA
<213> Unknown

<220>
<223> Obtained from environmental sample

<400> 109
atgagagttg tacaacccaa accttttact tttgaaaaag gtccgcgggc cgttttatta
60

ttgcatggtt ttacggggca ttctgccgat gtacggatgc ttggacgctt tttagaagag
120

agaaactata catgccatgc accgatttat cgtggacatg gtgttgcacc agaggagtta
180

ataaagacaa accccaatca gtggtgggaa gacgttgagg aagcttttaa ccacttaaaa
240

gaacgcggtt atgaggaaat tgcagtagcg ggtctttcac ttggtggggt ttttagtttg
300

aaacttgctt atacgaaacc cgtaaaagca gttattccca tgagtacacc gatgtttttc
360

gacaatgaag aacaattaac aaatgggttt cattccttta caagacaata taaaagatta
420

gaaggaaaat caaaagagga aatcgaggat gaaacaaaag cggtcatgga ggattccgga
480

tcgatattca atcaagtggc tgattacatt gctgatgtga ggtcccatgt ggatttaatt
540

tatacgccgg cgatggtcgt ccaggcggga aaagatcaaa tgatcaataa ggacagtgca
600

aattatatct acgaacacct ggaatcagat aaaaaagaaa taaaatggta tgaggaatca
660

ggacatgtca ttacggtcga taaagaaaag gaacagcttt ttgaagatat ctatcaattt 720

ttagagtctt tagactggaa atcatcgtaa 750

<210> 110
<211> 249
<212> PRT
<213> Unknown

<220>
<223> Obtained from environmental sample

<220>
<221> SITE
<222> (42)...(45)
<223> N-glycosylation site. Prosite id = PS00001

<400> 110
Met Arg Val Val Gln Pro Lys Pro Phe Thr Phe Glu Lys Gly Pro Arg
1               5                   10                  15

Ala Val Leu Leu Leu His Gly Phe Thr Gly His Ser Ala Asp Val Arg
            20                  25                  30

Met Leu Gly Arg Phe Leu Glu Glu Arg Asn Tyr Thr Cys His Ala Pro
        35                  40                  45

Ile Tyr Arg Gly His Gly Val Ala Pro Glu Glu Leu Ile Lys Thr Asn
    50                  55                  60

Pro Asn Gln Trp Trp Glu Asp Val Glu Glu Ala Phe Asn His Leu Lys
65                  70                  75                  80

Glu Arg Gly Tyr Glu Glu Ile Ala Val Ala Gly Leu Ser Leu Gly Gly
                85                  90                  95

Val Phe Ser Leu Lys Leu Ala Tyr Thr Lys Pro Val Lys Ala Val Ile
            100                 105                 110

Pro Met Ser Thr Pro Met Phe Phe Asp Asn Glu Glu Gln Leu Thr Asn
        115                 120                 125

Gly Phe His Ser Phe Thr Arg Gln Tyr Lys Arg Leu Glu Gly Lys Ser
    130                 135                 140

Lys Glu Glu Ile Glu Asp Glu Thr Lys Ala Val Met Glu Asp Ser Gly
145                 150                 155                 160

Ser Ile Phe Asn Gln Val Ala Asp Tyr Ile Ala Asp Val Arg Ser His
                165                 170                 175

Val Asp Leu Ile Tyr Thr Pro Ala Met Val Val Gln Ala Gly Lys Asp
            180                 185                 190

Gln Met Ile Asn Lys Asp Ser Ala Asn Tyr Ile Tyr Glu His Leu Glu
195 200 205

Ser Asp Lys Lys Glu Ile Lys Trp Tyr Glu Glu Ser Gly His Val Ile
210 215 220

Thr Val Asp Lys Glu Lys Glu Gln Leu Phe Glu Asp Ile Tyr Gln Phe
225 230 235 240

Leu Glu Ser Leu Asp Trp Lys Ser Ser
245

<210> 111
<211> 747
<212> DNA
<213> Unknown

<220>
<223> Obtained from environmental sample

<400> 111
atgaaaatta agttgccaga accattcaca ttcgaagcag gaaatcgagc agttttatta
60

ttacatggat ttactggcca ttcagcagat gtaagaatgc ttggtcgatt tctagaaaag
120

aaaggttata caactcatgc accaatttat agagggcatg gtcaagagcc agaagcatta
180

ttaaaatctt cacctgatga atggtgggag gatattttat cagcttataa tcatttgaga
240

aatctaggat ataatgaaat tgctgttgct ggtctttcaa tgggtggtgc tttagcaatt
300

aaactagcta ccaagcatga aataaaaggt gtcattccaa tgtgtacacc aatgtacttt
360

gataatcaaa aacaattaac taaagcattt ttaaactttg cacgacaatt taaaaaattt
420

gagaaaaaag atgaacaaac aattaaaaaa gaaatagatt tactacaaca aaattcagca
480

gaattattta atgaaatcgg cacttttgta gaacaagtca atggtataat agatagagtt
540

gacgtaccta cattcgtagt tcaagcaagt aaagatgaaa taataaatcc tgaaagtgcg
600

acatttattt atgaaaacat taaaaatgaa aaaaaagatt taaaatggta taaaaactca
660

actcacttga taacatttgg cgatgaaaaa gatgttttac atgaagatat atatcatttt
720

ttagaaacat tagattggaa caactaa
747

```
<210> 112
<211> 248
<212> PRT
<213> Unknown

<220>
<223> Obtained from environmental sample

<220>
<221> SITE
<222> (222)...(225)
<223> N-glycosylation site. Prosite id = PS00001

<400> 112
Met Lys Ile Lys Leu Pro Glu Pro Phe Thr Phe Glu Ala Gly Asn Arg
1               5                   10                  15

Ala Val Leu Leu Leu His Gly Phe Thr Gly His Ser Ala Asp Val Arg
            20                  25                  30

Met Leu Gly Arg Phe Leu Glu Lys Lys Gly Tyr Thr Thr His Ala Pro
        35                  40                  45

Ile Tyr Arg Gly His Gly Gln Glu Pro Glu Ala Leu Leu Lys Ser Ser
    50                  55                  60

Pro Asp Glu Trp Trp Glu Asp Ile Leu Ser Ala Tyr Asn His Leu Arg
65                  70                  75                  80

Asn Leu Gly Tyr Asn Glu Ile Ala Val Ala Gly Leu Ser Met Gly Gly
                85                  90                  95

Ala Leu Ala Ile Lys Leu Ala Thr Lys His Glu Ile Lys Gly Val Ile
            100                 105                 110

Pro Met Cys Thr Pro Met Tyr Phe Asp Asn Gln Lys Gln Leu Thr Lys
        115                 120                 125

Ala Phe Leu Asn Phe Ala Arg Gln Phe Lys Lys Phe Glu Lys Lys Asp
    130                 135                 140

Glu Gln Thr Ile Lys Lys Glu Ile Asp Leu Leu Gln Gln Asn Ser Ala
145                 150                 155                 160

Glu Leu Phe Asn Glu Ile Gly Thr Phe Val Glu Gln Val Asn Gly Ile
                165                 170                 175

Ile Asp Arg Val Asp Val Pro Thr Phe Val Val Gln Ala Ser Lys Asp
            180                 185                 190

Glu Ile Ile Asn Pro Glu Ser Ala Thr Phe Ile Tyr Glu Asn Ile Lys
        195                 200                 205
```

Asn Glu Lys Lys Asp Leu Lys Trp Tyr Lys Asn Ser Thr His Leu Ile
    210                 215                 220

Thr Phe Gly Asp Glu Lys Asp Val Leu His Glu Asp Ile Tyr His Phe
225                 230                 235                 240

Leu Glu Thr Leu Asp Trp Asn Asn
                245

<210> 113
<211> 747
<212> DNA
<213> Unknown

<220>
<223> Obtained from environmental sample

<400> 113
atgaaaatta agttgccaga accattcaca ttcgaagcag gaaatcgagc agttttatta
60

ttacatggat ttactggcca ttcagcagat gtaagaatgc ttggtcgatt tctagaaaag
120

aaaggttata caactcatgc accaatttat agagggcatg gtcaagaacc agaagcatta
180

ttaaaatctt cacctgatga atggtgggag gatattttat cagcttataa tcatttgaaa
240

aatctaggat ataatgaaat tgctgttgct ggtctttcaa tgggtggtgc tttagcaatt
300

aaactagcta ctaagcatga aataaaaggt gtcattccaa tgtgtacacc aatgtacttt
360

gataatcaaa aacaattaac tcaagcattt ttaaattttg cacgacaatt taaaaaattt
420

gagaaaaaag atgaagaaac aattaaaaaa gaaatagatt tactacaaca aaattcagca
480

gaattattta atgagatcgg cacttttgta gaacaagtca atggtataat agatagagtt
540

gacgtaccta cattcgtagt tcaagcaagt aaagatgaaa taatcaatcc tgaaagtgcg
600

acatttattt atgaaaacat taaaaatgaa aaaaaagatt taaaatggta taaaaattca
660

actcacttga taacatttgg cgatgaaaaa gatgtcttac atgaagatat atatcatttt
720

ttagaaacat tagattggaa caactaa
747

<210> 114
<211> 248
<212> PRT
<213> Unknown

<220>

<223> Obtained from environmental sample

<220>
<221> SITE
<222> (222)...(225)
<223> N-glycosylation site. Prosite id = PS00001

<400> 114
Met Lys Ile Lys Leu Pro Glu Pro Phe Thr Phe Glu Ala Gly Asn Arg
1               5                   10                  15

Ala Val Leu Leu Leu His Gly Phe Thr Gly His Ser Ala Asp Val Arg
            20                  25                  30

Met Leu Gly Arg Phe Leu Glu Lys Lys Gly Tyr Thr Thr His Ala Pro
        35                  40                  45

Ile Tyr Arg Gly His Gly Gln Glu Pro Glu Ala Leu Leu Lys Ser Ser
    50                  55                  60

Pro Asp Glu Trp Trp Glu Asp Ile Leu Ser Ala Tyr Asn His Leu Lys
65                  70                  75                  80

Asn Leu Gly Tyr Asn Glu Ile Ala Val Ala Gly Leu Ser Met Gly Gly
                85                  90                  95

Ala Leu Ala Ile Lys Leu Ala Thr Lys His Glu Ile Lys Gly Val Ile
            100                 105                 110

Pro Met Cys Thr Pro Met Tyr Phe Asp Asn Gln Lys Gln Leu Thr Gln
        115                 120                 125

Ala Phe Leu Asn Phe Ala Arg Gln Phe Lys Lys Phe Glu Lys Lys Asp
    130                 135                 140

Glu Glu Thr Ile Lys Lys Glu Ile Asp Leu Leu Gln Gln Asn Ser Ala
145                 150                 155                 160

Glu Leu Phe Asn Glu Ile Gly Thr Phe Val Glu Gln Val Asn Gly Ile
                165                 170                 175

Ile Asp Arg Val Asp Val Pro Thr Phe Val Val Gln Ala Ser Lys Asp
            180                 185                 190

Glu Ile Ile Asn Pro Glu Ser Ala Thr Phe Ile Tyr Glu Asn Ile Lys
        195                 200                 205

Asn Glu Lys Lys Asp Leu Lys Trp Tyr Lys Asn Ser Thr His Leu Ile
    210                 215                 220

Thr Phe Gly Asp Glu Lys Asp Val Leu His Glu Asp Ile Tyr His Phe
225                 230                 235                 240

Leu Glu Thr Leu Asp Trp Asn Asn
245

<210> 115
<211> 957
<212> DNA
<213> Bacteria

<400> 115
atggatatct gcagaattcg cccttggtgg cgacgactcc tggagcccgt cagtatcggc 60

gggacgtctt tccctgatcg ccatcgcctg cctgctctct gcctgcagcg ccaagggccc 120

tgtgttgcaa ccactgtaca ttgcgccgct cgaactggtg cagccgaaaa aatcgtcatc 180

gacctggcgc tgggcggcgg tgcggcgcgc ggctttgcgc acatcggtgt gatcaaggcg 240

ctcgaatcgc agggcatctt ggccgatgtc gtggtcggca ccagtgccgg cagcgtggtc 300

ggggcgctgt atgcggccgg caactccggt tttgcgctgc aaaaaatggc gctgtccatg 360

gacgaggcgg cgatctcgga ctgggccttg ccgctgttcg ccaggacttc gggcgtactc 420

aagggcgcgg cgctgcaaaa ttacgtcaat agcgcagtac atcaggcgcc gatcgagaaa 480

ttgaagctgc cctttggcgc ggtcgccact gacttgaaaa acggcatgcc catcttgttc 540

agacggggca ataccgggat ggcggtgcgg gcgtcatcgg cggtgccggg tgtgtttcag 600

ccggtcgaga tcggtggtca tagctatgtc gatggcggcc tggtggcgcc ggtgccggtg 660

cgctttgcgc gcgaaatggg cgccgatttc gtaattgcgg tcaacatttc cacgcaaacc 720

gagaagcagg tggcgctcag ttcgctcgaa gtgctgatgc aaactttcag catcatgggg 780

cagcgcatta accaattcga gctcaaggat gccgacatcg tcatcgaacc cagcctggga 840

ttcatgaaaa gcaatgattt caacggtcgc aacctggcca ttctggccgg cgagcaagcg 900

gcgtttgcga tgatgcccga gatcaagcag aaactgaagg tgctgcgcga gcagtaa 957

<210> 116
<211> 318
<212> PRT
<213> Bacteria

<220>

<221> DOMAIN
<222> (62)...(224)
<223> Patatin-like phospholipase

<220>
<221> SITE
<222> (238)...(241)
<223> N-glycosylation site. Prosite id = PS00001

<400> 116
```
Met Asp Ile Cys Arg Ile Arg Pro Trp Trp Arg Arg Leu Leu Glu Pro
1               5                   10                  15

Val Ser Ile Gly Gly Thr Ser Phe Pro Asp Arg His Arg Leu Pro Ala
            20                  25                  30

Leu Cys Leu Gln Arg Gln Gly Pro Cys Val Ala Thr Thr Val His Cys
        35                  40                  45

Ala Ala Arg Thr Gly Ala Ala Glu Lys Ile Val Ile Asp Leu Ala Leu
    50                  55                  60

Gly Gly Gly Ala Ala Arg Gly Phe Ala His Ile Gly Val Ile Lys Ala
65                  70                  75                  80

Leu Glu Ser Gln Gly Ile Leu Ala Asp Val Val Val Gly Thr Ser Ala
                85                  90                  95

Gly Ser Val Val Gly Ala Leu Tyr Ala Ala Gly Asn Ser Gly Phe Ala
            100                 105                 110

Leu Gln Lys Met Ala Leu Ser Met Asp Glu Ala Ala Ile Ser Asp Trp
        115                 120                 125

Ala Leu Pro Leu Phe Ala Arg Thr Ser Gly Val Leu Lys Gly Ala Ala
    130                 135                 140

Leu Gln Asn Tyr Val Asn Ser Ala Val His Gln Ala Pro Ile Glu Lys
145                 150                 155                 160

Leu Lys Leu Pro Phe Gly Ala Val Ala Thr Asp Leu Lys Asn Gly Met
                165                 170                 175

Pro Ile Leu Phe Arg Arg Gly Asn Thr Gly Met Ala Val Arg Ala Ser
            180                 185                 190

Ser Ala Val Pro Gly Val Phe Gln Pro Val Glu Ile Gly Gly His Ser
        195                 200                 205

Tyr Val Asp Gly Gly Leu Val Ala Pro Val Pro Val Arg Phe Ala Arg
    210                 215                 220
```

Glu Met Gly Ala Asp Phe Val Ile Ala Val Asn Ile Ser Thr Gln Thr
225 230 235 240

Glu Lys Gln Val Ala Leu Ser Ser Leu Glu Val Leu Met Gln Thr Phe
245 250 255

Ser Ile Met Gly Gln Arg Ile Asn Gln Phe Glu Leu Lys Asp Ala Asp
260 265 270

Ile Val Ile Glu Pro Ser Leu Gly Phe Met Lys Ser Asn Asp Phe Asn
275 280 285

Gly Arg Asn Leu Ala Ile Leu Ala Gly Glu Gln Ala Ala Phe Ala Met
290 295 300

Met Pro Glu Ile Lys Gln Lys Leu Lys Val Leu Arg Glu Gln
305 310 315

<210> 117
<211> 483
<212> DNA
<213> Unknown

<220>
<223> Obtained from environmental sample

<400> 117
gtgccggagg cggcgattcg ctattatgca gagacggcgg agccgtcggc gcccgactgg
60

caaacgtggc tgcgcatcat gacgtaccgt gtttttgtcg aggggatgct gcggacagcg
120

gacgcccaag cggcccacgg agcggatgtg tacatgtacc ggtttgatta tgagacgccg
180

gtgttcggcg gacagctgaa agcatgccac gcgctcgagc tgccgtttgt gtttcacaat
240

ctccatcagc cgggcgtcgc gaatttcgtc ggcaatcggc cggagcgcga ggcgatcgcc
300

aacgaaatgc attatgcctg gctttcgttc gcccgcaccg gcgacccgaa cggcgatcac
360

ttgccggaag cgtggccggc gtatacaacc gagcgcaagg cggcctttgt cttttcagcc
420

gccagccatg tcaaagatga tccgtttggc cgcgagcgtg cggcgtggat gacgcgcaca
480

taa
483

<210> 118
<211> 160
<212> PRT
<213> Unknown

<220>

<223> Obtained from environmental sample

<400> 118
Met Pro Glu Ala Ala Ile Arg Tyr Tyr Ala Glu Thr Ala Glu Pro Ser
1 5 10 15

Ala Pro Asp Trp Gln Thr Trp Leu Arg Ile Met Thr Tyr Arg Val Phe
20 25 30

Val Glu Gly Met Leu Arg Thr Ala Asp Ala Gln Ala Ala His Gly Ala
35 40 45

Asp Val Tyr Met Tyr Arg Phe Asp Tyr Glu Thr Pro Val Phe Gly Gly
50 55 60

Gln Leu Lys Ala Cys His Ala Leu Glu Leu Pro Phe Val Phe His Asn
65 70 75 80

Leu His Gln Pro Gly Val Ala Asn Phe Val Gly Asn Arg Pro Glu Arg
85 90 95

Glu Ala Ile Ala Asn Glu Met His Tyr Ala Trp Leu Ser Phe Ala Arg
100 105 110

Thr Gly Asp Pro Asn Gly Asp His Leu Pro Glu Ala Trp Pro Ala Tyr
115 120 125

Thr Thr Glu Arg Lys Ala Ala Phe Val Phe Ser Ala Ala Ser His Val
130 135 140

Lys Asp Asp Pro Phe Gly Arg Glu Arg Ala Ala Trp Met Thr Arg Thr
145 150 155 160

<210> 119
<211> 711
<212> DNA
<213> Unknown

<220>
<223> Obtained from environmental sample

<400> 119
gtgtcgttgt caccgttgtt tgaaggtgga catcatgttg tgttgagtgt gacgaccatc
60

gccactcctc atgacgggac gacgcttgtc aacatggttg atttcaccga tcgctttttt
120

gacttgcaaa aagcggtgtt gaaagcggcg gctgtcgcca gcaacgtgcc gtacacgagt
180

caagtatacg attttaagct cgaccaatgg ggactgcgcc gccagccggg tgaatcgttc
240

gaccattatt ttgaacggct caagcgctcc cctgtttgga cgtcgacaga taccgcccgc
300

tacgatttat ccgtttccgg agctgagaag ttgaatcaat gggtgcaagc aagcccgaat
360

acgtattatt tgagctttgc cacagaacgg acgtatcgcg gagcgctcac aggcaactat
420

tatcccgaac tcggaatgaa tgcattcagc gcggtcgtat gcgctccgtt tctcggttcg
480

taccgcaatc cgacgctcgg cattgacgac cgctggcttg aaaacgatgg tattgtcaat
540

acggtttcca tgaacggtcc aaagcgtgga tcaagcgatc ggatcgtacc gtatgacggg
600

gcgttgaaaa aaggggtttg gaatgacatg ggaacgtaca atgtcgacca tttggaaatc
660

atcggcgttg acccgaatcc gtcatttgat attcgcgcct tttatttgcg a
711

<210> 120
<211> 237
<212> PRT
<213> Unknown

<220>
<223> Obtained from environmental sample

<400> 120
Met Ser Leu Ser Pro Leu Phe Glu Gly Gly His His Val Val Leu Ser
1               5                   10                  15

Val Thr Thr Ile Ala Thr Pro His Asp Gly Thr Thr Leu Val Asn Met
            20                  25                  30

Val Asp Phe Thr Asp Arg Phe Phe Asp Leu Gln Lys Ala Val Leu Lys
        35                  40                  45

Ala Ala Ala Val Ala Ser Asn Val Pro Tyr Thr Ser Gln Val Tyr Asp
    50                  55                  60

Phe Lys Leu Asp Gln Trp Gly Leu Arg Arg Gln Pro Gly Glu Ser Phe
65                  70                  75                  80

Asp His Tyr Phe Glu Arg Leu Lys Arg Ser Pro Val Trp Thr Ser Thr
                85                  90                  95

Asp Thr Ala Arg Tyr Asp Leu Ser Val Ser Gly Ala Glu Lys Leu Asn
            100                 105                 110

Gln Trp Val Gln Ala Ser Pro Asn Thr Tyr Tyr Leu Ser Phe Ala Thr
        115                 120                 125

Glu Arg Thr Tyr Arg Gly Ala Leu Thr Gly Asn Tyr Tyr Pro Glu Leu
    130                 135                 140

Gly Met Asn Ala Phe Ser Ala Val Val Cys Ala Pro Phe Leu Gly Ser
145                 150                 155                 160

Tyr Arg Asn Pro Thr Leu Gly Ile Asp Asp Arg Trp Leu Glu Asn Asp
                165                 170                 175

Gly Ile Val Asn Thr Val Ser Met Asn Gly Pro Lys Arg Gly Ser Ser
            180                 185                 190

Asp Arg Ile Val Pro Tyr Asp Gly Ala Leu Lys Lys Gly Val Trp Asn
        195                 200                 205

Asp Met Gly Thr Tyr Asn Val Asp His Leu Glu Ile Ile Gly Val Asp
    210                 215                 220

Pro Asn Pro Ser Phe Asp Ile Arg Ala Phe Tyr Leu Arg
225                 230                 235

<210> 121
<211> 906
<212> DNA
<213> Archaea

<400> 121
atgcccctac atccagaggt aaagaaatta ctttcccagc tacctcccca gggcttctcc
60

agaaacgtgc aggagctgag gaaggcctgg gatttagcct tctcagggag gagggagagc
120

ctggagaggg ttgaggacct tgagataccc actagggacg cacgaatcag ggccagggtc
180

tacacccccct caagtaagga aaacttaccc gtccttgttt actatcacgg cggtggcttc
240

gtgttcggta gcgttgacag ctacgacggc ctcgcatccc ttattgccaa ggaatctggg
300

attgcggtta tctccgtgga gtataggctc gcccctgagc acaagttccc caccgcagtc
360

aacgactcgt gggatgcgct tctctggatc gcggagaacg gaggcaagct ggggctcgac
420

acctcgagac ttgccgtggc tggggatagt gctggaggaa acctgtctgc cgtggtgtcc
480

ctcctggaca gggaccaggg taagggactg gttagttatc aggtcctaat ctacccagca
540

gtgaacatgg tcgataactc cccatccgtc agggagtacg gcgagggata cttcctcacc
600

aggtccatga tgaactggtt cgggaccatg tacttctcct ctggaaggga agcggtatcc
660

ccctacgcct ctccagcctt ggctgaccta cataacctcc caccctcact ggtgatcact
720

gcagagtatg atcccctaag ggatcaggga gagacctact ctcactccct aaacgaggct
780

ggaaacgtat caaccttggt tagatatcaa ggaatgattc acggcttcct gtccttctac
840

gagtggataa ctgccggtaa actagccatt caccacattg ctggggttct gagatctgtc
900

ctttag
906

<210> 122
<211> 301
<212> PRT
<213> Archaea

<220>
<221> DOMAIN
<222> (72)...(278)
<223> alpha/beta hydrolase fold

<220>
<221> SITE
<222> (122)...(125)
<223> N-glycosylation site. Prosite id = PS00001

<220>
<221> SITE
<222> (156)...(159)
<223> N-glycosylation site. Prosite id = PS00001

<220>
<221> SITE
<222> (266)...(269)
<223> N-glycosylation site. Prosite id = PS00001

<400> 122
Met Pro Leu His Pro Glu Val Lys Lys Leu Leu Ser Gln Leu Pro Pro
1               5                   10                  15

Gln Gly Phe Ser Arg Asn Val Gln Glu Leu Arg Lys Ala Trp Asp Leu
            20                  25                  30

Ala Phe Ser Gly Arg Arg Glu Ser Leu Glu Arg Val Glu Asp Leu Glu
        35                  40                  45

Ile Pro Thr Arg Asp Ala Arg Ile Arg Ala Arg Val Tyr Thr Pro Ser
    50                  55                  60

Ser Lys Glu Asn Leu Pro Val Leu Val Tyr Tyr His Gly Gly Gly Phe
65                  70                  75                  80

Val Phe Gly Ser Val Asp Ser Tyr Asp Gly Leu Ala Ser Leu Ile Ala
                85                  90                  95

Lys Glu Ser Gly Ile Ala Val Ile Ser Val Glu Tyr Arg Leu Ala Pro
            100                 105                 110

```
Glu His Lys Phe Pro Thr Ala Val Asn Asp Ser Trp Asp Ala Leu Leu
        115                 120                 125

Trp Ile Ala Glu Asn Gly Gly Lys Leu Gly Leu Asp Thr Ser Arg Leu
    130                 135                 140

Ala Val Ala Gly Asp Ser Ala Gly Gly Asn Leu Ser Ala Val Val Ser
145                 150                 155                 160

Leu Leu Asp Arg Asp Gln Gly Lys Gly Leu Val Ser Tyr Gln Val Leu
                165                 170                 175

Ile Tyr Pro Ala Val Asn Met Val Asp Asn Ser Pro Ser Val Arg Glu
            180                 185                 190

Tyr Gly Glu Gly Tyr Phe Leu Thr Arg Ser Met Met Asn Trp Phe Gly
        195                 200                 205

Thr Met Tyr Phe Ser Ser Gly Arg Glu Ala Val Ser Pro Tyr Ala Ser
    210                 215                 220

Pro Ala Leu Ala Asp Leu His Asn Leu Pro Pro Ser Leu Val Ile Thr
225                 230                 235                 240

Ala Glu Tyr Asp Pro Leu Arg Asp Gln Gly Glu Thr Tyr Ser His Ser
                245                 250                 255

Leu Asn Glu Ala Gly Asn Val Ser Thr Leu Val Arg Tyr Gln Gly Met
            260                 265                 270

Ile His Gly Phe Leu Ser Phe Tyr Glu Trp Ile Thr Ala Gly Lys Leu
        275                 280                 285

Ala Ile His His Ile Ala Gly Val Leu Arg Ser Val Leu
    290                 295                 300


<210> 123
<211> 990
<212> DNA
<213> Unknown

<220>
<223> Obtained from environmental sample

<400> 123
atgcaattct ggcaacgact gcaagccctt ttcaccccgc ggggtcgctc cgcccacccg
60

gaacaggaca ccgcatcgac gtcggcgaac acacacacgt cggcaaaagg ggcctccgtt
120

cccgaacccc aaacgctgcc ccatccacca cgaatcgatt gggtgaactg catcaatacc
180
```

gctggaaccc accgcatgat ctactacgaa tggggcgcgc ccgacaatcc tgaagtcgtc
240

ttttgcgttc acggcttgac gcgaaccggg cgtgatttcg acgcgcttgc ggctcatttg
300

agccgccgct atcgggtgat ctgtcccgac gtcgtcgggc gcggactttc ggattggctt
360

cccaacaaga gcgaatacgc gatcccacgc tacgcgagcg atctcacgac gctcattcag
420

aaaatccgac cccagaaact ctactgggtg ggtacctcca tgggcgggtt gatcgcaatg
480

gcaatcgctg cgcaacccac ttcaccgatc gcaaaactgg tgttgaacga tgttggcgcc
540

gtgctcagtc aggcgtcgtt acaacggatc ggcgcgtacg tcggtgccga gccgcaattt
600

gccacgttcg acgatgccgt tcagtacgta cgtactgtta gcgccccgtt cggcccactc
660

agcgacgcac aatggcgtca tctgacgtgg cacgccgtcc gacaagaact ggacggacgt
720

tgggtgttgc gctacgaccc aggcatcgct gttccgtttc gcatcgcgtt tcctgcccaa
780

gatgtggcct tgtgggagct ctacgacgca atacgctgcc cgacgctcct tttgcgtggc
840

gccgaatcgg acctcttgac gcgggaggtg gccgaagcga tgacgcgccg cggcccgcgg
900

gcgcgactcg tcgaaattcc cggtgtgggt cacgcaccgg cgttgctcga tcgcgcgcag
960

atcgacgaaa tcagcgcatt tctcgattag
990

<210> 124
<211> 329
<212> PRT
<213> Unknown

<220>
<223> Obtained from environmental sample

<220>
<221> DOMAIN
<222> (104)...(326)
<223> alpha/beta hydrolase fold

<220>
<221> SITE
<222> (123)...(126)
<223> N-glycosylation site. Prosite id = PS00001

<400> 124
Met Gln Phe Trp Gln Arg Leu Gln Ala Leu Phe Thr Pro Arg Gly Arg
1 5 10 15

```
Ser Ala His Pro Glu Gln Asp Thr Ala Ser Thr Ser Ala Asn Thr His
            20                  25                  30

Thr Ser Ala Lys Gly Ala Ser Val Pro Glu Pro Gln Thr Leu Pro His
        35                  40                  45

Pro Pro Arg Ile Asp Trp Val Asn Cys Ile Asn Thr Ala Gly Thr His
    50                  55                  60

Arg Met Ile Tyr Tyr Glu Trp Gly Ala Pro Asp Asn Pro Glu Val Val
65                  70                  75                  80

Phe Cys Val His Gly Leu Thr Arg Thr Gly Arg Asp Phe Asp Ala Leu
                85                  90                  95

Ala Ala His Leu Ser Arg Arg Tyr Arg Val Ile Cys Pro Asp Val Val
            100                 105                 110

Gly Arg Gly Leu Ser Asp Trp Leu Pro Asn Lys Ser Glu Tyr Ala Ile
        115                 120                 125

Pro Arg Tyr Ala Ser Asp Leu Thr Thr Leu Ile Gln Lys Ile Arg Pro
    130                 135                 140

Gln Lys Leu Tyr Trp Val Gly Thr Ser Met Gly Gly Leu Ile Ala Met
145                 150                 155                 160

Ala Ile Ala Ala Gln Pro Thr Ser Pro Ile Ala Lys Leu Val Leu Asn
                165                 170                 175

Asp Val Gly Ala Val Leu Ser Gln Ala Ser Leu Gln Arg Ile Gly Ala
            180                 185                 190

Tyr Val Gly Ala Glu Pro Gln Phe Ala Thr Phe Asp Asp Ala Val Gln
        195                 200                 205

Tyr Val Arg Thr Val Ser Ala Pro Phe Gly Pro Leu Ser Asp Ala Gln
    210                 215                 220

Trp Arg His Leu Thr Trp His Ala Val Arg Gln Glu Leu Asp Gly Arg
225                 230                 235                 240

Trp Val Leu Arg Tyr Asp Pro Gly Ile Ala Val Pro Phe Arg Ile Ala
                245                 250                 255

Phe Pro Ala Gln Asp Val Ala Leu Trp Glu Leu Tyr Asp Ala Ile Arg
            260                 265                 270

Cys Pro Thr Leu Leu Leu Arg Gly Ala Glu Ser Asp Leu Leu Thr Arg
        275                 280                 285
```

Glu Val Ala Glu Ala Met Thr Arg Arg Gly Pro Arg Ala Arg Leu Val
290 295 300

Glu Ile Pro Gly Val Gly His Ala Pro Ala Leu Leu Asp Arg Ala Gln
305 310 315 320

Ile Asp Glu Ile Ser Ala Phe Leu Asp
325

<210> 125
<211> 1158
<212> DNA
<213> Unknown

<220>
<223> Obtained from environmental sample

<400> 125
atgtcggttc aaagttccgt cggacggttg tccagccttt tcgatcgccg cctgggaagc
60

ctgctgttgg tgctgctgtt tatctccggg tgcgccagtc ggccgggccc cgaggttctg
120

agaaccgtcg aaccggtggc tgaaagctca catgtgactg tctataccgc cacgacgcgg
180

gcgcgggcac cgcacgaagc tatagagttc acgacagaac gcgcacattc attgacctat
240

gcgcgattta gcgtctccat ccctgccaac catgagccgt caaggatcga atggccaagc
300

ggaacacccg acccgtcgac gagcttcgca gttgtggacc agacgatttt ggacaaacag
360

gcctttatgg aagaggttcg aatggccaac cgagcggcag gggcaccgg ggtcggtatt
420

tttgtgcatg gattcaatca caacttccct gaaacgcttt ttcggctcgc ccagatgtcg
480

gctgatatgg gcttggaggc tccccaaatc gccttctcat ggccatcgca ggctaatctg
540

cgaggctatc tggatgacaa gaactcagcc acgtactcaa gagattacct ggccgaagtg
600

attggcgagc tggcggcacg cgatgacatc ggcgaaatca ccatcgtggg acacagtatg
660

ggcgcctggc tggtcatgga ggcgttgcgc cagttgcgct tgcaaggaca ggatcgtgtc
720

atcgcgcgtc tgagggtggt tctggccgct cccgacatcg acgtcgacgt gtttcgccgg
780

cagctggagg tcatcgggcc tctcccgtca cccctgatgg tgctcgtatc acccgaagat
840

cgggcgctct tgatctctca gtggatagct gcctcgcggc ctcgcatcgg cacgctcgat

900

gttcgagatc ccggagtgca agccgcggcg cagttggcca atgtccagtt tgtcgatatt
960

acagctttgg ccgatgccgg gggggctggg cacgatcgct acatagcact ggccacgacc
1020

attccgcagc acgagacggc cccgggaacc ggtccgctgc gcgatgtccg tcaggcggga
1080

gcgttcgtcc tggatgccgt cggagcgatc atttcggccc cattcggcct ggccggacag
1140

gcgctggcgg gcgagtaa
1158

<210> 126
<211> 385
<212> PRT
<213> Unknown

<220>
<223> Obtained from environmental sample

<220>
<221> DOMAIN
<222> (118)...(340)
<223> Alpha/beta hydrolase of unknown function (DUF900)

<220>
<221> SITE
<222> (216)...(225)
<223> Lipases, serine active site. Prosite id = PS00120

<400> 126
Met Ser Val Gln Ser Ser Val Gly Arg Leu Ser Ser Leu Phe Asp Arg
1 5 10 15

Arg Leu Gly Ser Leu Leu Leu Val Leu Leu Phe Ile Ser Gly Cys Ala
20 25 30

Ser Arg Pro Gly Pro Glu Val Leu Arg Thr Val Glu Pro Val Ala Glu
35 40 45

Ser Ser His Val Thr Val Tyr Thr Ala Thr Thr Arg Ala Arg Ala Pro
50 55 60

His Glu Ala Ile Glu Phe Thr Thr Glu Arg Ala His Ser Leu Thr Tyr
65 70 75 80

Ala Arg Phe Ser Val Ser Ile Pro Ala Asn His Glu Pro Ser Arg Ile
85 90 95

Glu Trp Pro Ser Gly Thr Pro Asp Pro Ser Thr Ser Phe Ala Val Val
100 105 110

Asp Gln Thr Ile Leu Asp Lys Gln Ala Phe Met Glu Glu Val Arg Met
115 120 125

```
Ala Asn Arg Ala Ala Gly Gly Thr Gly Val Gly Ile Phe Val His Gly
    130                 135                 140

Phe Asn His Asn Phe Pro Glu Thr Leu Phe Arg Leu Ala Gln Met Ser
145                 150                 155                 160

Ala Asp Met Gly Leu Glu Ala Pro Gln Ile Ala Phe Ser Trp Pro Ser
                165                 170                 175

Gln Ala Asn Leu Arg Gly Tyr Leu Asp Asp Lys Asn Ser Ala Thr Tyr
            180                 185                 190

Ser Arg Asp Tyr Leu Ala Glu Val Ile Gly Glu Leu Ala Ala Arg Asp
        195                 200                 205

Asp Ile Gly Glu Ile Thr Ile Val Gly His Ser Met Gly Ala Trp Leu
    210                 215                 220

Val Met Glu Ala Leu Arg Gln Leu Arg Leu Gln Gly Gln Asp Arg Val
225                 230                 235                 240

Ile Ala Arg Leu Arg Val Val Leu Ala Ala Pro Asp Ile Asp Val Asp
                245                 250                 255

Val Phe Arg Arg Gln Leu Glu Val Ile Gly Pro Leu Pro Ser Pro Leu
            260                 265                 270

Met Val Leu Val Ser Pro Glu Asp Arg Ala Leu Leu Ile Ser Gln Trp
        275                 280                 285

Ile Ala Ala Ser Arg Pro Arg Ile Gly Thr Leu Asp Val Arg Asp Pro
    290                 295                 300

Gly Val Gln Ala Ala Ala Gln Leu Ala Asn Val Gln Phe Val Asp Ile
305                 310                 315                 320

Thr Ala Leu Ala Asp Ala Gly Gly Ala Gly His Asp Arg Tyr Ile Ala
                325                 330                 335

Leu Ala Thr Thr Ile Pro Gln His Glu Thr Ala Pro Gly Thr Gly Pro
            340                 345                 350

Leu Arg Asp Val Arg Gln Ala Gly Ala Phe Val Leu Asp Ala Val Gly
        355                 360                 365

Ala Ile Ile Ser Ala Pro Phe Gly Leu Ala Gly Gln Ala Leu Ala Gly
    370                 375                 380
```

Glu
385

<210> 127
<211> 852
<212> DNA
<213> Unknown

<220>
<223> Obtained from environmental sample

<400> 127
atgcaggggc aaatagaact tgtgactgat atcgagtacg ttgttcatga tggggttccg
60

ctagttggcg atttgtacct tccacgtgag cgcccccgatc cagttccact gctagtcgga
120

gtccatgggg gaggttggga gcgaggcgac cggaaatcac ttcgctattg gggagagttc
180

tttgcgaata gagggctcgg gttgtttgcg gtgcagtaca gacttgcaaa gtccggggtc
240

aaggcatatc cggaagcacc ttgcgatgtt cgctctgcag ttcaattcat gcgacatagt
300

gcggacagat ttggagtcga tgcccagagg ttggggctgt tcggtctttc cgccggggcg
360

catatagctt cgcttgcggc tttggcgggc gatgttgagc cttttgcgtc gcagtacccg
420

gccgatccat acgccgatgt aagtggcaag gtgaaggttg ttttggcggg gtatgggacc
480

tttgacgccg ctgcacactg gatgcacgat cagatacacc acccggggga cttatctatc
540

gagcggttct tggggaaatc cttattggac gaccgtcagc tatattttga ggcctcgccc
600

ttgagttatg cggtgagaag caatagtaac atttcgtttc ttttggttta cggtcttgag
660

gatgaggtgg tggacgttga cacgcagtct cgtcagtttg ccaaggcact gaaccaagcg
720

ggatttttg tgcggacgga ggcgatgcca ggtgcaggac acttctggat gatggagccg
780

ttggaggagg ttggtagcta tacggggttg gtcgccccga aggtgctgcg ttttcttcaa
840

caacgcctct ag
852

<210> 128
<211> 283
<212> PRT
<213> Unknown

<220>
<223> Obtained from environmental sample

```
<220>
<221> DOMAIN
<222> (38)...(258)
<223> alpha/beta hydrolase fold

<220>
<221> DOMAIN
<222> (52)...(271)
<223> Prolyl oligopeptidase family

<220>
<221> SITE
<222> (213)...(216)
<223> N-glycosylation site. Prosite id = PS00001

<400> 128
Met Gln Gly Gln Ile Glu Leu Val Thr Asp Ile Glu Tyr Val Val His
1               5                   10                  15

Asp Gly Val Pro Leu Val Gly Asp Leu Tyr Leu Pro Arg Glu Arg Pro
            20                  25                  30

Asp Pro Val Pro Leu Leu Val Gly Val His Gly Gly Gly Trp Glu Arg
        35                  40                  45

Gly Asp Arg Lys Ser Leu Arg Tyr Trp Gly Glu Phe Phe Ala Asn Arg
    50                  55                  60

Gly Leu Gly Leu Phe Ala Val Gln Tyr Arg Leu Ala Lys Ser Gly Val
65                  70                  75                  80

Lys Ala Tyr Pro Glu Ala Pro Cys Asp Val Arg Ser Ala Val Gln Phe
                85                  90                  95

Met Arg His Ser Ala Asp Arg Phe Gly Val Asp Ala Gln Arg Leu Gly
            100                 105                 110

Leu Phe Gly Leu Ser Ala Gly Ala His Ile Ala Ser Leu Ala Ala Leu
        115                 120                 125

Ala Gly Asp Val Glu Pro Phe Ala Ser Gln Tyr Pro Ala Asp Pro Tyr
    130                 135                 140

Ala Asp Val Ser Gly Lys Val Lys Val Val Leu Ala Gly Tyr Gly Thr
145                 150                 155                 160

Phe Asp Ala Ala Ala His Trp Met His Asp Gln Ile His His Pro Gly
                165                 170                 175

Asp Leu Ser Ile Glu Arg Phe Leu Gly Lys Ser Leu Leu Asp Asp Arg
            180                 185                 190

Gln Leu Tyr Phe Glu Ala Ser Pro Leu Ser Tyr Ala Val Arg Ser Asn
        195                 200                 205
```

Ser Asn Ile Ser Phe Leu Leu Val Tyr Gly Leu Glu Asp Glu Val Val
    210                 215                 220

Asp Val Asp Thr Gln Ser Arg Gln Phe Ala Lys Ala Leu Asn Gln Ala
225                 230                 235                 240

Gly Phe Phe Val Arg Thr Glu Ala Met Pro Gly Ala Gly His Phe Trp
                245                 250                 255

Met Met Glu Pro Leu Glu Glu Val Gly Ser Tyr Thr Gly Leu Val Ala
            260                 265                 270

Pro Lys Val Leu Arg Phe Leu Gln Gln Arg Leu
        275                 280

<210> 129
<211> 942
<212> DNA
<213> Unknown

<220>
<223> Obtained from environmental sample

<400> 129
ttggacatgc aatttcgcgc aaaaataatc actttcctgt gtttgctgac gctatctttt
60

gctgcttctg ctgcgagctt cgataagcta gttgtgtttg gggatagctt gtctgacaac
120

ggtaactttt atcactatac ctggtctttc ctcccgcaaa atccaccgta ttttcagggc
180

cgattttcaa atggcccaac ctgggctgaa catttagcag aaaaaatgca ttttgacggc
240

gatcaatttg ttgattgtgc ttatggcggt gcaatggcaa aaccagatcg aaatgatctt
300

gattatcagg taaaaaatta tctccaaaaa tttgctgatg aagataaaag tcgtcatttg
360

tttgtggtgt ggattggcag taatgattat ctcacgggta aaggtgaaat tgaaaaagaa
420

actgatatta cgattggttc aatcaaatca cacgtggatg cgttaatcaa aagtggtgcg
480

aaaacaattt tattaataaa tctccctgat cttggccata caccattagc aaaattggcg
540

ggtgcaggat atgcgaaaaa agtcagtcaa tttgtgcgta tacataatgc gaaattgaaa
600

tcattaatcg ccgcagaaaa atcgcagaat ccagatgtga aatttatgtt ctttgatgta
660

accagctatt ttgacgatgc catcgcgcat ccggcgaaat ttggtattgt taatgtgaaa
720

gatgcctgtt atgaaggcga cacctatatg ttttctgtat tggatcagcc taaatatgca
780

gccatccttg ctggtttaaa taaaaatggt gtcgtgagat attgtggtga tgctgataga
840

tatctatttt gggatcacgt gcatccaacc cgtgttgtgc atcaaaaaat tgcagatacg
900

gtgtatgcat tagtacaaaa aaacgctccg cccaacggct aa
942

<210> 130
<211> 313
<212> PRT
<213> Unknown

<220>
<223> Obtained from environmental sample

<220>
<221> SIGNAL
<222> (1)...(24)

<220>
<221> DOMAIN
<222> (30)...(301)
<223> GDSL-like Lipase/Acylhydrolase

<400> 130
Met Asp Met Gln Phe Arg Ala Lys Ile Ile Thr Phe Leu Cys Leu Leu
1 5 10 15

Thr Leu Ser Phe Ala Ala Ser Ala Ala Ser Phe Asp Lys Leu Val Val
20 25 30

Phe Gly Asp Ser Leu Ser Asp Asn Gly Asn Phe Tyr His Tyr Thr Trp
35 40 45

Ser Phe Leu Pro Gln Asn Pro Pro Tyr Phe Gln Gly Arg Phe Ser Asn
50 55 60

Gly Pro Thr Trp Ala Glu His Leu Ala Glu Lys Met His Phe Asp Gly
65 70 75 80

Asp Gln Phe Val Asp Cys Ala Tyr Gly Gly Ala Met Ala Lys Pro Asp
85 90 95

Arg Asn Asp Leu Asp Tyr Gln Val Lys Asn Tyr Leu Gln Lys Phe Ala
100 105 110

Asp Glu Asp Lys Ser Arg His Leu Phe Val Val Trp Ile Gly Ser Asn
115 120 125

Asp Tyr Leu Thr Gly Lys Gly Glu Ile Glu Lys Glu Thr Asp Ile Thr
130 135 140

Ile Gly Ser Ile Lys Ser His Val Asp Ala Leu Ile Lys Ser Gly Ala
145 150 155 160

Lys Thr Ile Leu Leu Ile Asn Leu Pro Asp Leu Gly His Thr Pro Leu
165 170 175

Ala Lys Leu Ala Gly Ala Gly Tyr Ala Lys Lys Val Ser Gln Phe Val
180 185 190

Arg Ile His Asn Ala Lys Leu Lys Ser Leu Ile Ala Ala Glu Lys Ser
195 200 205

Gln Asn Pro Asp Val Lys Phe Met Phe Phe Asp Val Thr Ser Tyr Phe
210 215 220

Asp Asp Ala Ile Ala His Pro Ala Lys Phe Gly Ile Val Asn Val Lys
225 230 235 240

Asp Ala Cys Tyr Glu Gly Asp Thr Tyr Met Phe Ser Val Leu Asp Gln
245 250 255

Pro Lys Tyr Ala Ala Ile Leu Ala Gly Leu Asn Lys Asn Gly Val Val
260 265 270

Arg Tyr Cys Gly Asp Ala Asp Arg Tyr Leu Phe Trp Asp His Val His
275 280 285

Pro Thr Arg Val Val His Gln Lys Ile Ala Asp Thr Val Tyr Ala Leu
290 295 300

Val Gln Lys Asn Ala Pro Pro Asn Gly
305 310

<210> 131
<211> 930
<212> DNA
<213> Unknown

<220>
<223> Obtained from environmental sample

<400> 131
ttggaactga ttctgggcct acttggcctt gccatactcg ttgtgttcta tttgcgtaaa
60

tggctgttgc gcaaggaaat acctcagcag caagcgatcg aattcaaggg cgaactcttt
120

gccgtaggcg ccgccagcct ggcccgccgc ggcccccaac agccccggca gagcgtgctt
180

gtcgtgcatg gttttgtgga aaacttcctc tattttaccg aatactatgc cgacccagac
240

cttgagctga ttctgctgac cagcgcggat taccatgtgc ctgtcaccca ggccacccat
300

cagcaagctg cctgggccaa ggcacccaaa gagcgcctgg gaactattgc ctatgatgct
360

gaggttttaa ttctggggtt ggaacacctg gtcaatggcc aggaagtgcg cgtgcatggc
420

cattcccgcg gtggtgctgt ggtactggaa gccgcccgtc gtcgcccgga tctatttgag
480

caggttgaag tgattcttga ggcacccgtg ttgcctcagg gcaaacctta tgccgaactg
540

ccccccagcg tgcgctggtt cctgcccttt gtactaccct tctggcaaca gcaacccatc
600

tcgccccgca acaaggccat ctgggggccg ctggatgatc ggcgcaaacg ctctctgatc
660

atggggctgc cgttcaaccc caaacattgc agcatcctca tggccaacat gaaggatatg
720

gcggaatgga tgccgggcaa tggcagtgag ctgtatcaga acgttaaacg tggcgccatt
780

ctagtgcctg gcaatgacaa ggtgctggag ccccgcgcca tgctcgccag tgcccggcag
840

gcagaaaatc tgcaagtgat tgaaatcgaa aacggcagtc attttgttat ccccgaccac
900

cctgattcga ttccgccctt gccggcatga
930

<210> 132
<211> 309
<212> PRT
<213> Unknown

<220>
<223> Obtained from environmental sample

<220>
<221> SITE
<222> (250)...(253)
<223> N-glycosylation site. Prosite id = PS00001

<220>
<221> SITE
<222> (295)...(298)
<223> N-glycosylation site. Prosite id = PS00001

<400> 132
Met Glu Leu Ile Leu Gly Leu Leu Gly Leu Ala Ile Leu Val Val Phe
1 5 10 15

Tyr Leu Arg Lys Trp Leu Leu Arg Lys Glu Ile Pro Gln Gln Gln Ala
20 25 30

Ile Glu Phe Lys Gly Glu Leu Phe Ala Val Gly Ala Ala Ser Leu Ala
35 40 45

```
Arg Arg Gly Pro Gln Gln Pro Arg Gln Ser Val Leu Val Val His Gly
    50                  55                  60

Phe Val Glu Asn Phe Leu Tyr Phe Thr Glu Tyr Tyr Ala Asp Pro Asp
65                  70                  75                  80

Leu Glu Leu Ile Leu Leu Thr Ser Ala Asp Tyr His Val Pro Val Thr
                85                  90                  95

Gln Ala Thr His Gln Gln Ala Ala Trp Ala Lys Ala Pro Lys Glu Arg
            100                 105                 110

Leu Gly Thr Ile Ala Tyr Asp Ala Glu Val Leu Ile Leu Gly Leu Glu
        115                 120                 125

His Leu Val Asn Gly Gln Glu Val Arg Val His Gly His Ser Arg Gly
    130                 135                 140

Gly Ala Val Val Leu Glu Ala Ala Arg Arg Arg Pro Asp Leu Phe Glu
145                 150                 155                 160

Gln Val Glu Val Ile Leu Glu Ala Pro Val Leu Pro Gln Gly Lys Pro
                165                 170                 175

Tyr Ala Glu Leu Pro Pro Ser Val Arg Trp Phe Leu Pro Phe Val Leu
            180                 185                 190

Pro Phe Trp Gln Gln Gln Pro Ile Ser Pro Arg Asn Lys Ala Ile Trp
        195                 200                 205

Gly Pro Leu Asp Asp Arg Arg Lys Arg Ser Leu Ile Met Gly Leu Pro
    210                 215                 220

Phe Asn Pro Lys His Cys Ser Ile Leu Met Ala Asn Met Lys Asp Met
225                 230                 235                 240

Ala Glu Trp Met Pro Gly Asn Gly Ser Glu Leu Tyr Gln Asn Val Lys
                245                 250                 255

Arg Gly Ala Ile Leu Val Pro Gly Asn Asp Lys Val Leu Glu Pro Arg
            260                 265                 270

Ala Met Leu Ala Ser Ala Arg Gln Ala Glu Asn Leu Gln Val Ile Glu
        275                 280                 285

Ile Glu Asn Gly Ser His Phe Val Ile Pro Asp His Pro Asp Ser Ile
    290                 295                 300

Pro Pro Leu Pro Ala
```

305

<210> 133
<211> 921
<212> DNA
<213> Unknown

<220>
<223> Obtained from environmental sample

<400> 133
atggacgttg ttttagcttt aggagctggt ggggtaaaag gtttcgctca tatcggtgtg
60

ctgcgggtat tagagcgaca cgggttccgt atcagggcta tcgcagggac gagcattggc
120

ggaattgtcg gctcggtcta ctgtttgggt ctcaacccgg acgagatcga aagcaagctg
180

cgggtgatgg accaaaagaa gttttacggc cggatacccg gagatggacc gtccattatg
240

ggggtatccg ggttggtcga gatcttcaaa gagatcttcg gcgatatgaa cctggagcaa
300

ctgcgtctcc cctttgcggt taatgccgta gatctggaaa cagctgaaga agtcaatatc
360

acccgcggaa gaatcgtcga tgcgatcctg gcgaccaccg cggtaccggg catatttcca
420

cccagaaagg tgaacaacca gactctactg gatggagggg ttctcaaccc cgtacctgtc
480

tcactggcgc gcgcactggc cccagggcta ccggttgtgg cagtcgtgct atctccatca
540

ttacaaggct gggtagcccg gccttcgccg cgcctgctgg gaggattacc gcttatgggg
600

agctatatct cccagctgcg cccagcccag gccttggaaa tcttcttccg ctcggttgat
660

atggccagct gcttaatggc agatctgcgc ttggagaagg agcagccgga agtgattatc
720

cgaccggcga tcgatgggat cgggttgatc gacgaagtga atgtggagga gatggtcaaa
780

aaaggcgaaa ctgctgctga gctggccata ccagagctac gatgggccgt cagttggcga
840

gcgcgaataa gccgccgctt cccctgggaa ggtatgcggc gggctacgag ccatcctgtt
900

caaacgcaca atgggtcttg a
921

<210> 134
<211> 306
<212> PRT
<213> Unknown

<220>

<223> Obtained from environmental sample

<220>
<221> DOMAIN
<222> (5)...(164)
<223> Patatin-like phospholipase

<220>
<221> SITE
<222> (120)...(123)
<223> N-glycosylation site. Prosite id = PS00001

<220>
<221> SITE
<222> (148)...(151)
<223> N-glycosylation site. Prosite id = PS00001

<400> 134
```
Met Asp Val Val Leu Ala Leu Gly Ala Gly Gly Val Lys Gly Phe Ala
1               5                   10                  15

His Ile Gly Val Leu Arg Val Leu Glu Arg His Gly Phe Arg Ile Arg
            20                  25                  30

Ala Ile Ala Gly Thr Ser Ile Gly Gly Ile Val Gly Ser Val Tyr Cys
        35                  40                  45

Leu Gly Leu Asn Pro Asp Glu Ile Glu Ser Lys Leu Arg Val Met Asp
    50                  55                  60

Gln Lys Lys Phe Tyr Gly Arg Ile Pro Gly Asp Gly Pro Ser Ile Met
65                  70                  75                  80

Gly Val Ser Gly Leu Val Glu Ile Phe Lys Glu Ile Phe Gly Asp Met
                85                  90                  95

Asn Leu Glu Gln Leu Arg Leu Pro Phe Ala Val Asn Ala Val Asp Leu
            100                 105                 110

Glu Thr Ala Glu Glu Val Asn Ile Thr Arg Gly Arg Ile Val Asp Ala
        115                 120                 125

Ile Leu Ala Thr Thr Ala Val Pro Gly Ile Phe Pro Pro Arg Lys Val
    130                 135                 140

Asn Asn Gln Thr Leu Leu Asp Gly Gly Val Leu Asn Pro Val Pro Val
145                 150                 155                 160

Ser Leu Ala Arg Ala Leu Ala Pro Gly Leu Pro Val Val Ala Val Val
                165                 170                 175

Leu Ser Pro Ser Leu Gln Gly Trp Val Ala Arg Pro Ser Pro Arg Leu
            180                 185                 190
```

Leu Gly Gly Leu Pro Leu Met Gly Ser Tyr Ile Ser Gln Leu Arg Pro
195 200 205

Ala Gln Ala Leu Glu Ile Phe Phe Arg Ser Val Asp Met Ala Ser Cys
210 215 220

Leu Met Ala Asp Leu Arg Leu Glu Lys Glu Gln Pro Glu Val Ile Ile
225 230 235 240

Arg Pro Ala Ile Asp Gly Ile Gly Leu Ile Asp Glu Val Asn Val Glu
245 250 255

Glu Met Val Lys Lys Gly Glu Thr Ala Ala Glu Leu Ala Ile Pro Glu
260 265 270

Leu Arg Trp Ala Val Ser Trp Arg Ala Arg Ile Ser Arg Arg Phe Pro
275 280 285

Trp Glu Gly Met Arg Arg Ala Thr Ser His Pro Val Gln Thr His Asn
290 295 300

Gly Ser
305

<210> 135
<211> 921
<212> DNA
<213> Unknown

<220>
<223> Obtained from environmental sample

<400> 135
ttgaaaggct gttacggaag tatgagtgtt caggcaacga tattgcgaca aggactaagt
60

ctggtacgca atctgattat ttcatctgct gatccggttg atcttcgtga tcgacttgcc
120

aaactggaat cccgtattcc cgcgctgccg agtgatatca aatgcgagca agtcgccatt
180

gaacatattc ccgggttctg gtacataccc cccaacgcac cgactgataa agtcatcctc
240

taccttcacg gtggtggcta tatgttctgc tccgcgggct ctacccaccg cgacctgctg
300

gcacgcttgt gtcgcgccag ccactgccgg attctggcga ttgattaccg cctggctccc
360

gaaaatattt tccctgctgc cgtcgacgat gtggtcacgg cgtaccagtg gctgcttaag
420

cagttcaagg cacagaatat tgccatcggc ggcgattccg ccggtggcgg tctcgcgttt
480

ggcagcgcgc taaaattgcg cgacgacggt attgaattgc ccgccgcact gattggcatg

540

tctccctgga gcgacctcgc catcaccggt gagagcgtgt tgctgaatca aacctacgac
600

aagattctgc ccgccgatgg tcttgaggaa ggtgccgata tttatctggg cgacgccgac
660

cccacccacc cctacgcgtc accgctgtac ggcgatcaca cagggctgcc accagcaatg
720

attcaagtgg gcggcgatga agtgctgctg gatgacagcc gccggctggc ctacaacatg
780

cagaaagccg gctgtcacgt cattctcgaa gagtggccga atatgcagca tgtgtggcag
840

gccttcagcc cctttttacc cgagggccgg gaagcgattg cccgactggg tatatttctg
900

cgatgccacc tgaaaacctg a
921

<210> 136
<211> 306
<212> PRT
<213> Unknown

<220>
<223> Obtained from environmental sample

<220>
<221> DOMAIN
<222> (79)...(281)
<223> alpha/beta hydrolase fold

<220>
<221> SITE
<222> (199)...(202)
<223> N-glycosylation site. Prosite id = PS00001

<400> 136
Met Lys Gly Cys Tyr Gly Ser Met Ser Val Gln Ala Thr Ile Leu Arg
1               5                   10                  15

Gln Gly Leu Ser Leu Val Arg Asn Leu Ile Ile Ser Ser Ala Asp Pro
            20                  25                  30

Val Asp Leu Arg Asp Arg Leu Ala Lys Leu Glu Ser Arg Ile Pro Ala
        35                  40                  45

Leu Pro Ser Asp Ile Lys Cys Glu Gln Val Ala Ile Glu His Ile Pro
    50                  55                  60

Gly Phe Trp Tyr Ile Pro Pro Asn Ala Pro Thr Asp Lys Val Ile Leu
65                  70                  75                  80

Tyr Leu His Gly Gly Gly Tyr Met Phe Cys Ser Ala Gly Ser Thr His
                85                  90                  95

Arg Asp Leu Leu Ala Arg Leu Cys Arg Ala Ser His Cys Arg Ile Leu
100 105 110

Ala Ile Asp Tyr Arg Leu Ala Pro Glu Asn Ile Phe Pro Ala Ala Val
115 120 125

Asp Asp Val Val Thr Ala Tyr Gln Trp Leu Leu Lys Gln Phe Lys Ala
130 135 140

Gln Asn Ile Ala Ile Gly Gly Asp Ser Ala Gly Gly Gly Leu Ala Phe
145 150 155 160

Gly Ser Ala Leu Lys Leu Arg Asp Asp Gly Ile Glu Leu Pro Ala Ala
165 170 175

Leu Ile Gly Met Ser Pro Trp Ser Asp Leu Ala Ile Thr Gly Glu Ser
180 185 190

Val Leu Leu Asn Gln Thr Tyr Asp Lys Ile Leu Pro Ala Asp Gly Leu
195 200 205

Glu Glu Gly Ala Asp Ile Tyr Leu Gly Asp Ala Asp Pro Thr His Pro
210 215 220

Tyr Ala Ser Pro Leu Tyr Gly Asp His Thr Gly Leu Pro Pro Ala Met
225 230 235 240

Ile Gln Val Gly Gly Asp Glu Val Leu Leu Asp Asp Ser Arg Arg Leu
245 250 255

Ala Tyr Asn Met Gln Lys Ala Gly Cys His Val Ile Leu Glu Glu Trp
260 265 270

Pro Asn Met Gln His Val Trp Gln Ala Phe Ser Pro Phe Leu Pro Glu
275 280 285

Gly Arg Glu Ala Ile Ala Arg Leu Gly Ile Phe Leu Arg Cys His Leu
290 295 300

Lys Thr
305

<210> 137
<211> 1509
<212> DNA
<213> Unknown

<220>
<223> Obtained from environmental sample

<400> 137
atgcagaatc cgtccacccc gttggtgaaa acccggcagg ggatactatc cggcacctct

60

gaagagaaca ttcatatctg gcgcggtatt ccctatgccg cgccgcccgt cggcgatctt
120

cgctggcgcg cgccgcagcc tgctgcccgc tggcagggcg tgcgccgggc agagaccttc
180

tcggcctcca gctggcagga tattgaatac tgccgcgagc tcggcggcgg cgatcccggc
240

agcttttccg aagattgcct ctaccttaat gtctggtcgc ccgccgcccg atcgcagccg
300

ctgccggtta tggtctggct gcacggcggg ggctatacca tcggcgcggg aagtttgccg
360

ccctatgacg gtaaagcctt agcttcccgt ggcgtggtag tggtgacggt caactaccgg
420

ctcgggcatc tgggcttttt tgctcatccg gcgctggaag ggaagacgg cgagagtatc
480

tataatttcg cgctactcga tcagatcgcc gccctgcagt gggtgcagga gaacattcac
540

gcctttggcg gcgatgcggc taacgtgacg ctgttcggcg aatccgccgg cgcgcgcagc
600

gtgctgtcgc tgatggtttc gccaaagtcc agaggcttgt tccataaagc gataatccag
660

agcggctata ccttgcccga tctgccgcga gagaaggcgc tggcgaaggg gcagcttatt
720

gcggaacact ttctttgcc gcaggccagc gccgaacagc tgcgggcgat tccggcgcaa
780

gcgttctggt cgttgaccgc gccgttgacc atcggcccgg cgcccgtggt tggcgatgcc
840

gtgctgccgc agccgatgct ggagactttt tttagcggac gtcagcatcc cctgccggtg
900

atgatcggtt cgaacagcga tgaggcgagc gtcatggcgg tgtttggcgt cgatatcgcc
960

gggcagatcc agaagcttcg ccgtgaacgc cgtttcggtc tgggtctaat taaattgctc
1020

tatcccggcg tgaagggaga tgaagcgctg gggcgcgaag tttgccggga tatggccttt
1080

accactctgg gctatgtggt gatgcaggct cagcagcgag tagggcaacc gtgctggcgc
1140

tactggtttg actacgtggc cgaggcggaa caccagacct atccgcacgg cgcgtggcac
1200

ggtaatgaag tggcatacgt ttttgacaat ctcgaccttg ccgagccggt acgaaattat
1260

gccaacgatc gcgacagaga ctttgccgcc cgggtcgccg actattgggc gaacttcgct
1320

cgcttcgccg gcaatgactg tcatgaactc tcagggccgg tacgctggcc cgccagcgtg

1380

cgcggacggg atcgcctgct gcgaatcggg ctgcacaaac gggccggctt taaggtggag
1440

aaccgcttta tgcgcgcgcg tctggcgctg tttcggcggg tcatgaagca tcatgtgacg
1500

ctggattaa
1509

<210> 138
<211> 502
<212> PRT
<213> Unknown

<220>
<223> Obtained from environmental sample

<220>
<221> DOMAIN
<222> (2)...(487)
<223> Carboxylesterase

<220>
<221> SITE
<222> (85)...(95)
<223> Carboxylesterases type-B signature 2. Prosite id = PS00941

<220>
<221> SITE
<222> (190)...(193)
<223> N-glycosylation site. Prosite id = PS00001

<400> 138
Met Gln Asn Pro Ser Thr Pro Leu Val Lys Thr Arg Gln Gly Ile Leu
1 5 10 15

Ser Gly Thr Ser Glu Glu Asn Ile His Ile Trp Arg Gly Ile Pro Tyr
20 25 30

Ala Ala Pro Pro Val Gly Asp Leu Arg Trp Arg Ala Pro Gln Pro Ala
35 40 45

Ala Arg Trp Gln Gly Val Arg Arg Ala Glu Thr Phe Ser Ala Ser Ser
50 55 60

Trp Gln Asp Ile Glu Tyr Cys Arg Glu Leu Gly Gly Gly Asp Pro Gly
65 70 75 80

Ser Phe Ser Glu Asp Cys Leu Tyr Leu Asn Val Trp Ser Pro Ala Ala
85 90 95

Arg Ser Gln Pro Leu Pro Val Met Val Trp Leu His Gly Gly Gly Tyr
100 105 110

Thr Ile Gly Ala Gly Ser Leu Pro Pro Tyr Asp Gly Lys Ala Leu Ala
115 120 125

Ser Arg Gly Val Val Val Val Thr Val Asn Tyr Arg Leu Gly His Leu
130 135 140

Gly Phe Phe Ala His Pro Ala Leu Glu Gly Glu Asp Gly Glu Ser Ile
145 150 155 160

Tyr Asn Phe Ala Leu Leu Asp Gln Ile Ala Ala Leu Gln Trp Val Gln
165 170 175

Glu Asn Ile His Ala Phe Gly Gly Asp Ala Ala Asn Val Thr Leu Phe
180 185 190

Gly Glu Ser Ala Gly Ala Arg Ser Val Leu Ser Leu Met Val Ser Pro
195 200 205

Lys Ser Arg Gly Leu Phe His Lys Ala Ile Ile Gln Ser Gly Tyr Thr
210 215 220

Leu Pro Asp Leu Pro Arg Glu Lys Ala Leu Ala Lys Gly Gln Leu Ile
225 230 235 240

Ala Glu His Phe Ser Leu Pro Gln Ala Ser Ala Glu Gln Leu Arg Ala
245 250 255

Ile Pro Ala Gln Ala Phe Trp Ser Leu Thr Ala Pro Leu Thr Ile Gly
260 265 270

Pro Ala Pro Val Val Gly Asp Ala Val Leu Pro Gln Pro Met Leu Glu
275 280 285

Thr Phe Phe Ser Gly Arg Gln His Pro Leu Pro Val Met Ile Gly Ser
290 295 300

Asn Ser Asp Glu Ala Ser Val Met Ala Val Phe Gly Val Asp Ile Ala
305 310 315 320

Gly Gln Ile Gln Lys Leu Arg Arg Glu Arg Arg Phe Gly Leu Gly Leu
325 330 335

Ile Lys Leu Leu Tyr Pro Gly Val Lys Gly Asp Glu Ala Leu Gly Arg
340 345 350

Glu Val Cys Arg Asp Met Ala Phe Thr Thr Leu Gly Tyr Val Val Met
355 360 365

Gln Ala Gln Gln Arg Val Gly Gln Pro Cys Trp Arg Tyr Trp Phe Asp
370 375 380

Tyr Val Ala Glu Ala Glu His Gln Thr Tyr Pro His Gly Ala Trp His

385 390 395 400

Gly Asn Glu Val Ala Tyr Val Phe Asp Asn Leu Asp Leu Ala Glu Pro
405 410 415

Val Arg Asn Tyr Ala Asn Asp Arg Asp Arg Asp Phe Ala Ala Arg Val
420 425 430

Ala Asp Tyr Trp Ala Asn Phe Ala Arg Phe Ala Gly Asn Asp Cys His
435 440 445

Glu Leu Ser Gly Pro Val Arg Trp Pro Ala Ser Val Arg Gly Arg Asp
450 455 460

Arg Leu Leu Arg Ile Gly Leu His Lys Arg Ala Gly Phe Lys Val Glu
465 470 475 480

Asn Arg Phe Met Arg Ala Arg Leu Ala Leu Phe Arg Arg Val Met Lys
485 490 495

His His Val Thr Leu Asp
500

<210> 139
<211> 900
<212> DNA
<213> Unknown

<220>
<223> Obtained from environmental sample

<400> 139
atgataccgc aacaaacccc gccgccggaa acccccgttg acaaatcggc ccgggaacgc
60

atcggcgaaa ccaggaaagc gttcctgaat atgacggcgg agttcgcgac ttcgctggcg
120

gacattccga cgacggagta tgaccaggag ggtatccggg gaagctggat catgcccaat
180

gatgcggatt ccgaacgcgt gcttttgtat cttcacggcg gcggctacat agtcggaagc
240

gatgaaacgc cccgcgcgat aaccgcattt ctggcgcgcg aggcaaaagt gcggtgtttt
300

tccctggatt atccgcttgc cccgagcat ccatttccgg ctgcgctcga tagcgcggtg
360

cgatcgtatg aaatgctgtt ggaaaaaggg ttcagccctg gaaacatcgt gctgggcggc
420

gattcggccg gcggcggcct ggcgcttgcc ctgttgctcg ccatccggga acacgggctg
480

ccgatgccgg cgggagcgta tctcctttcc ccgtggactg atttgacgca gagttttccc
540

acgcattcac gcaaggccga tgtcgaaatc agcattaccc ccgagctgct tgaggaagcg
600

gcagccagct atgcgggtga ttccgacaga accaatccgc ttatctcccc cgcattcggc
660

gagttccggg gattcccgcc gctcctgata caagttggtt cccatgaacg tctgttggac
720

gactccctga cggtggcgcg caatgccgcc ctcgccgatg tgccgacgac attgaaggta
780

tggcccggct atccccatgt gtttcaagtc tatcaccaga atctcgacgg cgccaaaaag
840

gcgttgaggg aagccgcgga attcatcacc ggagcgatgg ataaagagtt gcttcaataa
900

<210> 140
<211> 299
<212> PRT
<213> Unknown

<220>
<223> Obtained from environmental sample

<220>
<221> DOMAIN
<222> (68)...(270)
<223> alpha/beta hydrolase fold

<220>
<221> SITE
<222> (30)...(33)
<223> N-glycosylation site. Prosite id = PS00001

<400> 140
Met Ile Pro Gln Gln Thr Pro Pro Pro Glu Thr Pro Val Asp Lys Ser
1 5 10 15

Ala Arg Glu Arg Ile Gly Glu Thr Arg Lys Ala Phe Leu Asn Met Thr
20 25 30

Ala Glu Phe Ala Thr Ser Leu Ala Asp Ile Pro Thr Thr Glu Tyr Asp
35 40 45

Gln Glu Gly Ile Arg Gly Ser Trp Ile Met Pro Asn Asp Ala Asp Ser
50 55 60

Glu Arg Val Leu Leu Tyr Leu His Gly Gly Gly Tyr Ile Val Gly Ser
65 70 75 80

Asp Glu Thr Pro Arg Ala Ile Thr Ala Phe Leu Ala Arg Glu Ala Lys
85 90 95

Val Arg Cys Phe Ser Leu Asp Tyr Pro Leu Ala Pro Glu His Pro Phe
100 105 110

Pro Ala Ala Leu Asp Ser Ala Val Arg Ser Tyr Glu Met Leu Leu Glu
115 120 125

Lys Gly Phe Ser Pro Gly Asn Ile Val Leu Gly Gly Asp Ser Ala Gly
130 135 140

Gly Gly Leu Ala Leu Ala Leu Leu Leu Ala Ile Arg Glu His Gly Leu
145 150 155 160

Pro Met Pro Ala Gly Ala Tyr Leu Leu Ser Pro Trp Thr Asp Leu Thr
165 170 175

Gln Ser Phe Pro Thr His Ser Arg Lys Ala Asp Val Glu Ile Ser Ile
180 185 190

Thr Pro Glu Leu Leu Glu Glu Ala Ala Ala Ser Tyr Ala Gly Asp Ser
195 200 205

Asp Arg Thr Asn Pro Leu Ile Ser Pro Ala Phe Gly Glu Phe Arg Gly
210 215 220

Phe Pro Pro Leu Leu Ile Gln Val Gly Ser His Glu Arg Leu Leu Asp
225 230 235 240

Asp Ser Leu Thr Val Ala Arg Asn Ala Ala Leu Ala Asp Val Pro Thr
245 250 255

Thr Leu Lys Val Trp Pro Gly Tyr Pro His Val Phe Gln Val Tyr His
260 265 270

Gln Asn Leu Asp Gly Ala Lys Lys Ala Leu Arg Glu Ala Ala Glu Phe
275 280 285

Ile Thr Gly Ala Met Asp Lys Glu Leu Leu Gln
290 295

<210> 141
<211> 936
<212> DNA
<213> Unknown

<220>
<223> Obtained from environmental sample

<400> 141
gtgccgaata ccgtcagccc tgaaatacag acgttgattg gcgcaacccc ttttcctttt
60

tggaatctgc accccaaaag caatgctgaa tggaagacat ttgtgcaaaa ggtggcacga
120

gctggcgagg ccgctctgcc gaaactgcgg gaacagatgg gggttgcggt cacgccggga
180

actatggcgg gtgtaccggt attcaccgtc acaccgcgca acctccggcc tgaaaatgcc
240

aagcgtattc tgttgcactt ccacggcggc ggctatgtgc tcaatccagg cgaggccgga
300

acccaggaag ccattcttat ggcgggtata ggtggattca tggtgctttc cgtggactac
360

agaatggccc cggattttcc ttacccggca gctatggatg atgccattgc cgtgtaccat
420

gaattgctga aaaccactcc agccgaaaga attggggtgt tcggcacgtc taccggcggt
480

ggaatgacat tggcgctagc cctccgagcc aaagccgagc ggcttccctt gcccgccgct
540

ctggccccag gaacaccctg gacggatttg accaagaccg gcgacagcta tttcgtcaac
600

gacagggtag acaatattct ggtcagctat gacggctggc ttggggatgc ggcaaagctc
660

tatgctgccg ggcatgacct caaagacccc atgctttcac ccgtctatgg cgatgttagc
720

ggatttcccc cgaccttact taccacggga acacgcgacc tcttcttgag caacaccgtc
780

cgtatgcacc tgagactgcg ccaagccggg gtggatgccg acctgattgt atttgagggt
840

atgtcccacg cgcactatca catgaacccg gacgcgccag aaacacactt tcatttcagc
900

gagttgggga agttctttcg ccgcaatttg cagtag
936

<210> 142
<211> 311
<212> PRT
<213> Unknown

<220>
<223> Obtained from environmental sample

<220>
<221> DOMAIN
<222> (84)...(287)
<223> alpha/beta hydrolase fold

<400> 142
Met Pro Asn Thr Val Ser Pro Glu Ile Gln Thr Leu Ile Gly Ala Thr
1               5                   10                  15

Pro Phe Pro Phe Trp Asn Leu His Pro Lys Ser Asn Ala Glu Trp Lys
            20                  25                  30

Thr Phe Val Gln Lys Val Ala Arg Ala Gly Glu Ala Ala Leu Pro Lys
        35                  40                  45

Leu Arg Glu Gln Met Gly Val Ala Val Thr Pro Gly Thr Met Ala Gly

50 55 60

Val Pro Val Phe Thr Val Thr Pro Arg Asn Leu Arg Pro Glu Asn Ala
65 70 75 80

Lys Arg Ile Leu Leu His Phe His Gly Gly Gly Tyr Val Leu Asn Pro
85 90 95

Gly Glu Ala Gly Thr Gln Glu Ala Ile Leu Met Ala Gly Ile Gly Gly
100 105 110

Phe Met Val Leu Ser Val Asp Tyr Arg Met Ala Pro Asp Phe Pro Tyr
115 120 125

Pro Ala Ala Met Asp Asp Ala Ile Ala Val Tyr His Glu Leu Leu Lys
130 135 140

Thr Thr Pro Ala Glu Arg Ile Gly Val Phe Gly Thr Ser Thr Gly Gly
145 150 155 160

Gly Met Thr Leu Ala Leu Ala Leu Arg Ala Lys Ala Glu Arg Leu Pro
165 170 175

Leu Pro Ala Ala Leu Ala Pro Gly Thr Pro Trp Thr Asp Leu Thr Lys
180 185 190

Thr Gly Asp Ser Tyr Phe Val Asn Asp Arg Val Asp Asn Ile Leu Val
195 200 205

Ser Tyr Asp Gly Trp Leu Gly Asp Ala Ala Lys Leu Tyr Ala Ala Gly
210 215 220

His Asp Leu Lys Asp Pro Met Leu Ser Pro Val Tyr Gly Asp Val Ser
225 230 235 240

Gly Phe Pro Pro Thr Leu Leu Thr Thr Gly Thr Arg Asp Leu Phe Leu
245 250 255

Ser Asn Thr Val Arg Met His Leu Arg Leu Arg Gln Ala Gly Val Asp
260 265 270

Ala Asp Leu Ile Val Phe Glu Gly Met Ser His Ala His Tyr His Met
275 280 285

Asn Pro Asp Ala Pro Glu Thr His Phe His Phe Ser Glu Leu Gly Lys
290 295 300

Phe Phe Arg Arg Asn Leu Gln
305 310

<210> 143
<211> 987
<212> DNA
<213> Unknown

<220>
<223> Obtained from environmental sample

<400> 143
atggcctggg agacggaccg gccagaagcc ttccacctct cccgccacac ggtggagctt 60

gcctatcgcg aggtcttcgc cggctttcgc ccatcgatcc gccttcagcc gcgagccgag 120

cccggcctga ccatccgcgc ccgccatgcc gcagcgcagc cgctgccccga gacacttgtc 180

gacggctaca cgctgcgcga cctcggccgg caatacagcc cgcgcctgca ggccgacatg 240

aaggcactgt tccggcaatg gagccgcgag ggcgaggcct tccgcgcccg ccatggcgcc 300

gccgatctcg cctatggccc cggccgtttc gaaaggctcg acctctaccg tccggccggc 360

gtgaaccgcg cgccgatctt cgtcttcatc catggcggct actggcaggc ctcggacaag 420

gtccagcacg cccagttcgc gcaagggctg ctcgattcag gcttcgccgt cgccatgccg 480

aactacggtc tggcgccgga tacgccgctg gaaacaagcc tcgcccagag cgtcgccgcc 540

ctgaatttcc tcgtccgcga agccgaagcg ctcggcctcg atgccgacag actgcatatc 600

tcaggccatt ccgccggcgg gcacctcgcc gccatgacgc tctgcgcgcc aaatgctccg 660

ccgatcacct cggccctgct gctttccggc ctttaccacc tcaacccgct cggccatctc 720

ccgctcggcc gcttgctcgg actcgacgat gccgcgcgcg ccacaaggct cagccccatc 780

gcccagccaa gcccggaaac gacacggatc gcggtcgcgg tcggcgaagg cgagagcgac 840

gccttcaagg cgcaatcggc cgcactcgcc gcgacatggc aagcgccggc gccgctgatc 900

tgccccggcc atcatttcag catgctcgaa ggcctgaacg gcggtgccct actcgatctc 960

gccctgcgaa cggcggaagg ccgatga 987

<210> 144
<211> 328
<212> PRT
<213> Unknown

<220>
<223> Obtained from environmental sample

<220>
<221> DOMAIN
<222> (127)...(308)
<223> alpha/beta hydrolase fold

<400> 144
Met Ala Trp Glu Thr Asp Arg Pro Glu Ala Phe His Leu Ser Arg His
1               5                   10                  15

Thr Val Glu Leu Ala Tyr Arg Glu Val Phe Ala Gly Phe Arg Pro Ser
            20                  25                  30

Ile Arg Leu Gln Pro Arg Ala Glu Pro Gly Leu Thr Ile Arg Ala Arg
        35                  40                  45

His Ala Ala Ala Gln Pro Leu Pro Glu Thr Leu Val Asp Gly Tyr Thr
    50                  55                  60

Leu Arg Asp Leu Gly Arg Gln Tyr Ser Pro Arg Leu Gln Ala Asp Met
65                  70                  75                  80

Lys Ala Leu Phe Arg Gln Trp Ser Arg Glu Gly Glu Ala Phe Arg Ala
                85                  90                  95

Arg His Gly Ala Ala Asp Leu Ala Tyr Gly Pro Gly Arg Phe Glu Arg
            100                 105                 110

Leu Asp Leu Tyr Arg Pro Ala Gly Val Asn Arg Ala Pro Ile Phe Val
        115                 120                 125

Phe Ile His Gly Gly Tyr Trp Gln Ala Ser Asp Lys Val Gln His Ala
    130                 135                 140

Gln Phe Ala Gln Gly Leu Leu Asp Ser Gly Phe Ala Val Ala Met Pro
145                 150                 155                 160

Asn Tyr Gly Leu Ala Pro Asp Thr Pro Leu Glu Thr Ser Leu Ala Gln
                165                 170                 175

Ser Val Ala Ala Leu Asn Phe Leu Val Arg Glu Ala Glu Ala Leu Gly
            180                 185                 190

Leu Asp Ala Asp Arg Leu His Ile Ser Gly His Ser Ala Gly Gly His
        195                 200                 205

Leu Ala Ala Met Thr Leu Cys Ala Pro Asn Ala Pro Pro Ile Thr Ser
    210                 215                 220

Ala Leu Leu Leu Ser Gly Leu Tyr His Leu Asn Pro Leu Gly His Leu

225 230 235 240

Pro Leu Gly Arg Leu Leu Gly Leu Asp Asp Ala Ala Arg Ala Thr Arg
245 250 255

Leu Ser Pro Ile Ala Gln Pro Ser Pro Glu Thr Thr Arg Ile Ala Val
260 265 270

Ala Val Gly Glu Gly Glu Ser Asp Ala Phe Lys Ala Gln Ser Ala Ala
275 280 285

Leu Ala Ala Thr Trp Gln Ala Pro Ala Pro Leu Ile Cys Pro Gly His
290 295 300

His Phe Ser Met Leu Glu Gly Leu Asn Gly Gly Ala Leu Leu Asp Leu
305 310 315 320

Ala Leu Arg Thr Ala Glu Gly Arg
325

<210> 145
<211> 498
<212> DNA
<213> Archaea

<400> 145
gtggcgcctc caattagaga tgtaaaggac ggctatgaga gaggcctcaa agagctacca
60

gtgtcagtgg ccgcaggcca cagcatgggc gggactgtcg ccctgctctt agccgctaaa
120

aacctgggtt ctgtcaagtg cgttatagca gtggcggcgc cagtggatag gaggctacag
180

ttgcaatacc tctcccagtc cagcgatccc tatcttaaga aactggccaa cgacttggcg
240

tccctcggga gtaaattgga acagacttcg ccgtctcgtt ttattggcaa cggaatgccg
300

ccagtgcttt acataagagg ctctgaggat tacgtagtgc cgaggactca tattgatatt
360

ctcgccggcc tctccgacag atttaacttc cccctggagg tggtggaggt gcagggcatg
420

ggccactcgc ccaagacaga ggagcacgtc aaggctgtcg cagaggctgt gaggaaattc
480

gcggcacgtt gtttataa
498

<210> 146
<211> 165
<212> PRT
<213> Archaea

<400> 146

Met Ala Pro Pro Ile Arg Asp Val Lys Asp Gly Tyr Glu Arg Gly Leu
1               5                   10                  15

Lys Glu Leu Pro Val Ser Val Ala Ala Gly His Ser Met Gly Gly Thr
            20                  25                  30

Val Ala Leu Leu Leu Ala Ala Lys Asn Leu Gly Ser Val Lys Cys Val
        35                  40                  45

Ile Ala Val Ala Ala Pro Val Asp Arg Arg Leu Gln Leu Gln Tyr Leu
    50                  55                  60

Ser Gln Ser Ser Asp Pro Tyr Leu Lys Lys Leu Ala Asn Asp Leu Ala
65                  70                  75                  80

Ser Leu Gly Ser Lys Leu Glu Gln Thr Ser Pro Ser Arg Phe Ile Gly
                85                  90                  95

Asn Gly Met Pro Pro Val Leu Tyr Ile Arg Gly Ser Glu Asp Tyr Val
            100                 105                 110

Val Pro Arg Thr His Ile Asp Ile Leu Ala Gly Leu Ser Asp Arg Phe
        115                 120                 125

Asn Phe Pro Leu Glu Val Val Glu Val Gln Gly Met Gly His Ser Pro
    130                 135                 140

Lys Thr Glu Glu His Val Lys Ala Val Ala Glu Ala Val Arg Lys Phe
145                 150                 155                 160

Ala Ala Arg Cys Leu
                165

<210> 147
<211> 987
<212> DNA
<213> Unknown

<220>
<223> Obtained from environmental sample

<400> 147
atgaagcggc ttttagccct cctttcgcta ttgggtttgg ctttggccca agggcttgcc
60

ctctggcagg cggtggaggt gccgggcggg gtgtgcgccg acggctcccc ctaccgcttc
120

tacgtgagcc cgggggaccc caagaaggtg gtcttggact tccaaggggg tggggcctgt
180

tgggacgccg ccacctgcgg cccggaaagc cgcacctacc gcaagcgggt ggacacccag
240

gagctcgccc tcgcccaggg catctataac cgcctgagcg tggccaaccc cttccacggc

300

tggacccacg tcttcgtgcc ctactgcacc ggggacctcc acgtggggag ggccacggtg
360

gactacgggg gctaccgggt gcaccaccag ggggcgcgga acgtgcaggc ggtgctggag
420

taccttttca agaaccacac caacccggag cgggttttcg tcaccgggtg tagcgccggg
480

gcctacgggg cggtcttctg ggcggacaag gtgctggcca cctacaaaaa cgcccaggtg
540

gcggtctgcg gcgatgccgg ggtgggggtg cgcacggagg gcttcccggg ctttgcccgc
600

tggaacccgc gcctgcccga cctgccaggc ctttccccga accctgaggt ttcggagatc
660

tacctggcct tggcccgggc ctacccgaag gcggtggtcg cccagtacac caccgtcctg
720

gacggcacgc agatcttctt ctatggcctc atgaagggcg aggccacccc ttccgaggcc
780

accgctcggg aatgggccct aaaggcccag gaggcggcgc tccgtccggc ccaggcggca
840

aactacacct tctacctggc cccagggagc cagcactgca tcctgccgag gcccgagctc
900

tacaccctga aggtggggga ggtgagcttc ctggactggc ttaggggttt ggcggagggg
960

aaggcgccgc ctcgggtgcg gccttga
987

<210> 148
<211> 328
<212> PRT
<213> Unknown

<220>
<223> Obtained from environmental sample

<220>
<221> SIGNAL
<222> (1)...(16)

<220>
<221> SITE
<222> (147)...(150)
<223> N-glycosylation site. Prosite id = PS00001

<220>
<221> SITE
<222> (285)...(288)
<223> N-glycosylation site. Prosite id = PS00001

<400> 148
Met Lys Arg Leu Leu Ala Leu Leu Ser Leu Leu Gly Leu Ala Leu Ala
1               5                   10                  15

Gln Gly Leu Ala Leu Trp Gln Ala Val Glu Val Pro Gly Gly Val Cys

20 25 30

Ala Asp Gly Ser Pro Tyr Arg Phe Tyr Val Ser Pro Gly Asp Pro Lys
35 40 45

Lys Val Val Leu Asp Phe Gln Gly Gly Gly Ala Cys Trp Asp Ala Ala
50 55 60

Thr Cys Gly Pro Glu Ser Arg Thr Tyr Arg Lys Arg Val Asp Thr Gln
65 70 75 80

Glu Leu Ala Leu Ala Gln Gly Ile Tyr Asn Arg Leu Ser Val Ala Asn
85 90 95

Pro Phe His Gly Trp Thr His Val Phe Val Pro Tyr Cys Thr Gly Asp
100 105 110

Leu His Val Gly Arg Ala Thr Val Asp Tyr Gly Gly Tyr Arg Val His
115 120 125

His Gln Gly Ala Arg Asn Val Gln Ala Val Leu Glu Tyr Leu Phe Lys
130 135 140

Asn His Thr Asn Pro Glu Arg Val Phe Val Thr Gly Cys Ser Ala Gly
145 150 155 160

Ala Tyr Gly Ala Val Phe Trp Ala Asp Lys Val Leu Ala Thr Tyr Lys
165 170 175

Asn Ala Gln Val Ala Val Cys Gly Asp Ala Gly Val Gly Val Arg Thr
180 185 190

Glu Gly Phe Pro Gly Phe Ala Arg Trp Asn Pro Arg Leu Pro Asp Leu
195 200 205

Pro Gly Leu Ser Pro Asn Pro Glu Val Ser Glu Ile Tyr Leu Ala Leu
210 215 220

Ala Arg Ala Tyr Pro Lys Ala Val Val Ala Gln Tyr Thr Thr Val Leu
225 230 235 240

Asp Gly Thr Gln Ile Phe Phe Tyr Gly Leu Met Lys Gly Glu Ala Thr
245 250 255

Pro Ser Glu Ala Thr Ala Arg Glu Trp Ala Leu Lys Ala Gln Glu Ala
260 265 270

Ala Leu Arg Pro Ala Gln Ala Ala Asn Tyr Thr Phe Tyr Leu Ala Pro
275 280 285

Gly Ser Gln His Cys Ile Leu Pro Arg Pro Glu Leu Tyr Thr Leu Lys
290 295 300

Val Gly Glu Val Ser Phe Leu Asp Trp Leu Arg Gly Leu Ala Glu Gly
305 310 315 320

Lys Ala Pro Pro Arg Val Arg Pro
325

<210> 149
<211> 777
<212> DNA
<213> Unknown

<220>
<223> Obtained from environmental sample

<400> 149
atggaagcga aaaccgtaca gacgccgcat gtgcccgtcc gctacttcga gggcgggaaa
60

ggcgagcccc tcgtcttcct gcatggcgcg ggcggcatta ccgccgaaga cccgttcctt
120

gcgaagctcg cggagacata tcacgtctac gcgccgctgc tgccgggcta tggcgactcg
180

gaggaatgcg gcgagttgcg cgagatgctc gacatcacgc tccacacatg ggacgtcgtc
240

gaagcgctcg gccttaagaa cccgatcctt gtcggccatt cgatgggcgg catgatcgcg
300

gcggagatgg ccgccgtcgc gccgaacgac gtgtcgcgcc tcgggctcat ctgcccggcg
360

ggactctggc tcgacgagca tcccattccc gacatcttcg cgatgctgcc atacgagctt
420

ccggcggttc tcttccacga tgtggaagcc ggcacggcgc tgatgacggc gggcgtcgcg
480

ctcgacgatc cggaatggct gaagggcttc ctcgtccaga atgcgcgcca actcggcatg
540

gcggggaaaa tcctcttccc cgtgccggat cgcgggctct cgcaacggct ctaccgcgtg
600

aaggcgaaga cggtgctcgt ctggggcgac ggcgacaagc tgatcgtgcc tgcctatgcc
660

catgcgtgga agaaggcgat caggcaagcg gaactcgtct cgatcccgga ggcgggccac
720

ctgatgacgc tcgaaaagcc ggaacatgtc gcgaaagcga tcgggaaact cggatag
777

<210> 150
<211> 258
<212> PRT
<213> Unknown

<220>
<223> Obtained from environmental sample

<220>
<221> DOMAIN
<222> (47)...(256)
<223> alpha/beta hydrolase fold

<400> 150
```
Met Glu Ala Lys Thr Val Gln Thr Pro His Val Pro Val Arg Tyr Phe
1               5                   10                  15

Glu Gly Gly Lys Gly Glu Pro Leu Val Phe Leu His Gly Ala Gly Gly
            20                  25                  30

Ile Thr Ala Glu Asp Pro Phe Leu Ala Lys Leu Ala Glu Thr Tyr His
        35                  40                  45

Val Tyr Ala Pro Leu Leu Pro Gly Tyr Gly Asp Ser Glu Glu Cys Gly
    50                  55                  60

Glu Leu Arg Glu Met Leu Asp Ile Thr Leu His Thr Trp Asp Val Val
65                  70                  75                  80

Glu Ala Leu Gly Leu Lys Asn Pro Ile Leu Val Gly His Ser Met Gly
                85                  90                  95

Gly Met Ile Ala Ala Glu Met Ala Ala Val Ala Pro Asn Asp Val Ser
            100                 105                 110

Arg Leu Gly Leu Ile Cys Pro Ala Gly Leu Trp Leu Asp Glu His Pro
        115                 120                 125

Ile Pro Asp Ile Phe Ala Met Leu Pro Tyr Glu Leu Pro Ala Val Leu
    130                 135                 140

Phe His Asp Val Glu Ala Gly Thr Ala Leu Met Thr Ala Gly Val Ala
145                 150                 155                 160

Leu Asp Asp Pro Glu Trp Leu Lys Gly Phe Leu Val Gln Asn Ala Arg
                165                 170                 175

Gln Leu Gly Met Ala Gly Lys Ile Leu Phe Pro Val Pro Asp Arg Gly
            180                 185                 190

Leu Ser Gln Arg Leu Tyr Arg Val Lys Ala Lys Thr Val Leu Val Trp
        195                 200                 205

Gly Asp Gly Asp Lys Leu Ile Val Pro Ala Tyr Ala His Ala Trp Lys
    210                 215                 220

Lys Ala Ile Arg Gln Ala Glu Leu Val Ser Ile Pro Glu Ala Gly His
```

225 230 235 240

Leu Met Thr Leu Glu Lys Pro Glu His Val Ala Lys Ala Ile Gly Lys
245 250 . 255

Leu Gly

<210> 151
<211> 891
<212> DNA
<213> Unknown

<220>
<223> Obtained from environmental sample

<400> 151
atgaagatag ggtatttgaa atttttgatt ttaatattct tttgtagtat acaaacttgg
60

agccaaacaa gcgaagtgtt acccctaaaa aatggagaaa ctattgtttg ggttggtaat
120

agtattacac atcagtgtag gtattctcaa tatgtagaaa attatttatt tacacgttat
180

ccaagtcagc attataattt tcgtactgct ggagtatcag gagataaagt gattgatgct
240

ttagaacgtt tcgatttaga tatcgctaaa tacaatccta attacgtaac catacttctg
300

ggtatgaacg atggcatgta tagaaagttt gataagaata cttttgaagc ctatcaaaaa
360

gatatgaata ctttactttt taaaatagat agtataggag caaaacctat cattatgact
420

cctactatgt ttgatgcaaa aatggcagag actacaggtt ggttttcgga tgaatcttat
480

ttttctgaat ataatggtgt aatggcttat tatagtgctt ggcttatgga gaaagcattg
540

gaacgaaatg attattacgt agacttaaga accagtctaa atcagattac ttggaataaa
600

cgaaagcatg acaaaggctt tagtataatt cctgacggaa tacatccaaa tccaataggg
660

ttagcagtaa tggcatctga tattattaga cagctatttg gaataagttc actttcagaa
720

atccaccttt cattacccat ttctaaaacc tcgataccaa tcgtaaaggg tggtgtaata
780

agtaacatta aaatgtctcg taaaaaaata gctttcgatt tttcacctga atcattgcct
840

tgggttatac ccaaaaatgc tcataaagga atcgaattag cagaagggaa t
891

<210> 152

<211> 297
<212> PRT
<213> Unknown

<220>
<223> Obtained from environmental sample

<220>
<221> SIGNAL
<222> (1)...(21)

<220>
<221> DOMAIN
<222> (35)...(228)
<223> GDSL-like Lipase/Acylhydrolase

<400> 152
Met Lys Ile Gly Tyr Leu Lys Phe Leu Ile Leu Ile Phe Phe Cys Ser
1               5                   10                  15

Ile Gln Thr Trp Ser Gln Thr Ser Glu Val Leu Pro Leu Lys Asn Gly
            20                  25                  30

Glu Thr Ile Val Trp Val Gly Asn Ser Ile Thr His Gln Cys Arg Tyr
        35                  40                  45

Ser Gln Tyr Val Glu Asn Tyr Leu Phe Thr Arg Tyr Pro Ser Gln His
    50                  55                  60

Tyr Asn Phe Arg Thr Ala Gly Val Ser Gly Asp Lys Val Ile Asp Ala
65                  70                  75                  80

Leu Glu Arg Phe Asp Leu Asp Ile Ala Lys Tyr Asn Pro Asn Tyr Val
                85                  90                  95

Thr Ile Leu Leu Gly Met Asn Asp Gly Met Tyr Arg Lys Phe Asp Lys
            100                 105                 110

Asn Thr Phe Glu Ala Tyr Gln Lys Asp Met Asn Thr Leu Leu Phe Lys
        115                 120                 125

Ile Asp Ser Ile Gly Ala Lys Pro Ile Ile Met Thr Pro Thr Met Phe
    130                 135                 140

Asp Ala Lys Met Ala Glu Thr Thr Gly Trp Phe Ser Asp Glu Ser Tyr
145                 150                 155                 160

Phe Ser Glu Tyr Asn Gly Val Met Ala Tyr Tyr Ser Ala Trp Leu Met
                165                 170                 175

Glu Lys Ala Leu Glu Arg Asn Asp Tyr Tyr Val Asp Leu Arg Thr Ser
            180                 185                 190

Leu Asn Gln Ile Thr Trp Asn Lys Arg Lys His Asp Lys Gly Phe Ser

195 200 205

Ile Ile Pro Asp Gly Ile His Pro Asn Pro Ile Gly Leu Ala Val Met
210 215 220

Ala Ser Asp Ile Ile Arg Gln Leu Phe Gly Ile Ser Ser Leu Ser Glu
225 230 235 240

Ile His Leu Ser Leu Pro Ile Ser Lys Thr Ser Ile Pro Ile Val Lys
245 250 255

Gly Gly Val Ile Ser Asn Ile Lys Met Ser Arg Lys Lys Ile Ala Phe
260 265 270

Asp Phe Ser Pro Glu Ser Leu Pro Trp Val Ile Pro Lys Asn Ala His
275 280 285

Lys Gly Ile Glu Leu Ala Glu Gly Asn
290 295

<210> 153
<211> 768
<212> DNA
<213> Unknown

<220>
<223> Obtained from environmental sample

<400> 153
atgagcaagc tggacgatta cgccaacaaa tacgagtgcg tgcgcttcga gcgccgcgac
60

ggcatcttgc agatgacctt gcacaccgaa ggccagtcgc tgcgctgggg gtttgggccg
120

catggcgagc tgccggaggc gtttcacgat gtcggcgccg accgcgacaa tcgggtcgtg
180

atcctgaccg ggaccggggc cgagttttcc gggccgcgcg cgaccccggg cacctcgtcc
240

ttcccgaccc gcccgggctg ggagcggatc gaccgcatcc attgggaggg gcggcatctg
300

ctgatgcgcc tcctcgacat cgaggtgccg gtgatcagcg cgatcaacgg cccggcctgg
360

cgccattccg agatcccgct gctatgcgac atcgtgcttg ccgccgacac cgcacagttc
420

caggattcgg cgcatttccc gtcggacgtg gtgccggggg acggcatgca catcgtcatg
480

ccgctactgc tcggcatcaa tcgcgggcgc tattacctgc tgaccgggca gacgcttgat
540

gcgcagaagg cgcatgattt ggggctggtc gccgaggtgc tgccgcccga ccggctgatg
600

gcgcgcgcct gggaactcgc cgagggcctc gccaaaaagc cgaccctggc gctgcgctac
660

acgcgcctca tgctgaccga gtatctgcgc cgccagatgc atgatttgct cggctacggg
720

ctcggcatgg aattgctggc gctgctcgaa aaaccggaag ggcaataa
768

<210> 154
<211> 255
<212> PRT
<213> Unknown

<220>
<223> Obtained from environmental sample

<220>
<221> DOMAIN
<222> (23)...(195)
<223> Enoyl-CoA hydratase/isomerase family

<400> 154
Met Ser Lys Leu Asp Asp Tyr Ala Asn Lys Tyr Glu Cys Val Arg Phe
1               5                   10                  15

Glu Arg Arg Asp Gly Ile Leu Gln Met Thr Leu His Thr Glu Gly Gln
            20                  25                  30

Ser Leu Arg Trp Gly Phe Gly Pro His Gly Glu Leu Pro Glu Ala Phe
        35                  40                  45

His Asp Val Gly Ala Asp Arg Asp Asn Arg Val Val Ile Leu Thr Gly
    50                  55                  60

Thr Gly Ala Glu Phe Ser Gly Pro Arg Ala Thr Pro Gly Thr Ser Ser
65                  70                  75                  80

Phe Pro Thr Arg Pro Gly Trp Glu Arg Ile Asp Arg Ile His Trp Glu
                85                  90                  95

Gly Arg His Leu Leu Met Arg Leu Leu Asp Ile Glu Val Pro Val Ile
            100                 105                 110

Ser Ala Ile Asn Gly Pro Ala Trp Arg His Ser Glu Ile Pro Leu Leu
        115                 120                 125

Cys Asp Ile Val Leu Ala Ala Asp Thr Ala Gln Phe Gln Asp Ser Ala
    130                 135                 140

His Phe Pro Ser Asp Val Val Pro Gly Asp Gly Met His Ile Val Met
145                 150                 155                 160

Pro Leu Leu Leu Gly Ile Asn Arg Gly Arg Tyr Tyr Leu Leu Thr Gly
                165                 170                 175

Gln Thr Leu Asp Ala Gln Lys Ala His Asp Leu Gly Leu Val Ala Glu
180 185 190

Val Leu Pro Pro Asp Arg Leu Met Ala Arg Ala Trp Glu Leu Ala Glu
195 200 205

Gly Leu Ala Lys Lys Pro Thr Leu Ala Leu Arg Tyr Thr Arg Leu Met
210 215 220

Leu Thr Glu Tyr Leu Arg Arg Gln Met His Asp Leu Leu Gly Tyr Gly
225 230 235 240

Leu Gly Met Glu Leu Leu Ala Leu Leu Glu Lys Pro Glu Gly Gln
245 250 255

<210> 155
<211> 906
<212> DNA
<213> Unknown

<220>
<223> Obtained from environmental sample

<400> 155
atgacgctga gtgacaagca tgccgaggtc ctgcgcgccg tgcgcgccgc cggccccgtg
60

attgatccgc cggccgcgaa ggcgttgtac gcgccgctgc tggcggacat gccgcgcggc
120

gcggaaccgg tgcgcgacgt cgtctacggc gccgatgagc gccaccgcct ggacgtgtac
180

ccggccgtcg gcgcagcggg accggcgccc gtggtcgtgt tcctccatgg cgggggcttc
240

atccgcggcg acaaggcgga ccgcgcggcc gtcggccatt acttttcgcg gcacggcatc
300

gtggccgtgc tgccgaatta ccggctcggt ccgcgccacc gctggccggc cggggccgaa
360

gacgcgtcgt ccgtgctcgc gtgggcccgc gcaaatgttg cggcgcatgg cggcgatccg
420

gatcacatcg tgctcgcggg cgaatcggcc ggcgcggcgc atgtggcggc ggcgacgctc
480

gtcaggcgct tccatccggc cgagggcctg aagatcgcgg gcgcattcct cgcgtcgggc
540

gtgtacaacg cggaactgga atacctcgcg cgcgcacaac tgggcattgc gacaccggac
600

ccgcgcaacg aggcctactt cgggacggac tttgcgcgct accgcacgat gtcgaccgta
660

gaactgatcg acgccgcgcc gttcccgctt gcgatcactt atgcggaact ggatccggtc
720

cagatgcagg cgcaggccgg cgagctgttc gcgcggctcg tcacgcgcca cggtttccag
780

ccgcggatcg gggtcgtcgc gaaccataat cacctcagcc aggtctatgc gatcaacagc
840

ggcgacgagg cgctggctgc gcccttgctg gcctttgtgc gtgacagcag caggaaaact
900

ccctaa
906

<210> 156
<211> 301
<212> PRT
<213> Unknown

<220>
<223> Obtained from environmental sample

<220>
<221> DOMAIN
<222> (72)...(275)
<223> alpha/beta hydrolase fold

<400> 156
Met Thr Leu Ser Asp Lys His Ala Glu Val Leu Arg Ala Val Arg Ala
1 5 10 15

Ala Gly Pro Val Ile Asp Pro Pro Ala Ala Lys Ala Leu Tyr Ala Pro
20 25 30

Leu Leu Ala Asp Met Pro Arg Gly Ala Glu Pro Val Arg Asp Val Val
35 40 45

Tyr Gly Ala Asp Glu Arg His Arg Leu Asp Val Tyr Pro Ala Val Gly
50 55 60

Ala Ala Gly Pro Ala Pro Val Val Val Phe Leu His Gly Gly Gly Phe
65 70 75 80

Ile Arg Gly Asp Lys Ala Asp Arg Ala Ala Val Gly His Tyr Phe Ser
85 90 95

Arg His Gly Ile Val Ala Val Leu Pro Asn Tyr Arg Leu Gly Pro Arg
100 105 110

His Arg Trp Pro Ala Gly Ala Glu Asp Ala Ser Ser Val Leu Ala Trp
115 120 125

Ala Arg Ala Asn Val Ala Ala His Gly Gly Asp Pro Asp His Ile Val
130 135 140

Leu Ala Gly Glu Ser Ala Gly Ala Ala His Val Ala Ala Ala Thr Leu
145 150 155 160

Val Arg Arg Phe His Pro Ala Glu Gly Leu Lys Ile Ala Gly Ala Phe
165 170 175

Leu Ala Ser Gly Val Tyr Asn Ala Glu Leu Glu Tyr Leu Ala Arg Ala
180 185 190

Gln Leu Gly Ile Ala Thr Pro Asp Pro Arg Asn Glu Ala Tyr Phe Gly
195 200 205

Thr Asp Phe Ala Arg Tyr Arg Thr Met Ser Thr Val Glu Leu Ile Asp
210 215 220

Ala Ala Pro Phe Pro Leu Ala Ile Thr Tyr Ala Glu Leu Asp Pro Val
225 230 235 240

Gln Met Gln Ala Gln Ala Gly Glu Leu Phe Ala Arg Leu Val Thr Arg
245 250 255

His Gly Phe Gln Pro Arg Ile Gly Val Val Ala Asn His Asn His Leu
260 265 270

Ser Gln Val Tyr Ala Ile Asn Ser Gly Asp Glu Ala Leu Ala Ala Pro
275 280 285

Leu Leu Ala Phe Val Arg Asp Ser Ser Arg Lys Thr Pro
290 295 300

<210> 157
<211> 843
<212> DNA
<213> Cochliobolus heterostrophus ATCC 48331

<400> 157
atgcttcgcc cagcttctct cttcgcactt ggtgcccttc tctttctttc acttctcgac
60

tctgtgtccg ctgcaacagc aggctgtggc aaaaccccca gactgaacag tggtgttcat
120

accatcacag tcaacaacaa gcagcgccgc tacactctcc gcgtgcctca aaactatgac
180

aacaagcgcc aataccgctt catctttggc ctgcactggc tcaacggtga catgaacgcg
240

gtcgctagtg gagctgctcc ctactacggc ctcgctgctc ttgccggtga gagcaccatc
300

tttgtggccc ccgatggtct caacagggga tggggcaacc agaatggaga ggatattgcc
360

ttccttgacg cagtcaggaa gcagattgac gacgaattct gtgtcaacga gaagctccgc
420

ttctccctcg gtttctccta cggcggcgcc atgtccttca gtctggcttg cagcaaggcg
480

gctgacttcc gaggcattgc tgtcttgtct ggtgccactc tctctggatg tgccggtggc
540

aacaccccca ttgcgttcta cggtcaacac ggtgtcaagg acagtgttct gcccatatct
600

tccgggaaac agatccgtga ccgctttgtg cagctgaacg gatgccagtc caagaacgca
660

cccgacccat ctcccaacag ccgacagcgc gtcaagaccg tatacgatgg atgcaggtac
720

ccaacccaat ggacctcctt tgatggagac catgtcgcac ttcccggaga tgcaggaaac
780

gatgttggtg ctcagagctt cacacccaaa gaagtctggg ccttcttcag ccagttcacc
840

taa
843

<210> 158
<211> 280
<212> PRT
<213> Cochliobolus heterostrophus ATCC 48331

<220>
<221> SIGNAL
<222> (1)...(26)

<220>
<221> DOMAIN
<222> (21)...(235)
<223> Glycosyl hydrolase family 62

<400> 158
Met Leu Arg Pro Ala Ser Leu Phe Ala Leu Gly Ala Leu Leu Phe Leu
1 5 10 15

Ser Leu Leu Asp Ser Val Ser Ala Ala Thr Ala Gly Cys Gly Lys Thr
20 25 30

Pro Arg Leu Asn Ser Gly Val His Thr Ile Thr Val Asn Asn Lys Gln
35 40 45

Arg Arg Tyr Thr Leu Arg Val Pro Gln Asn Tyr Asp Asn Lys Arg Gln
50 55 60

Tyr Arg Phe Ile Phe Gly Leu His Trp Leu Asn Gly Asp Met Asn Ala
65 70 75 80

Val Ala Ser Gly Ala Ala Pro Tyr Tyr Gly Leu Ala Ala Leu Ala Gly
85 90 95

Glu Ser Thr Ile Phe Val Ala Pro Asp Gly Leu Asn Arg Gly Trp Gly
100 105 110

Asn Gln Asn Gly Glu Asp Ile Ala Phe Leu Asp Ala Val Arg Lys Gln
115 120 125

```
Ile Asp Asp Glu Phe Cys Val Asn Glu Lys Leu Arg Phe Ser Leu Gly
    130                 135                 140

Phe Ser Tyr Gly Gly Ala Met Ser Phe Ser Leu Ala Cys Ser Lys Ala
145                 150                 155                 160

Ala Asp Phe Arg Gly Ile Ala Val Leu Ser Gly Ala Thr Leu Ser Gly
                165                 170                 175

Cys Ala Gly Gly Asn Thr Pro Ile Ala Phe Tyr Gly Gln His Gly Val
            180                 185                 190

Lys Asp Ser Val Leu Pro Ile Ser Ser Gly Lys Gln Ile Arg Asp Arg
        195                 200                 205

Phe Val Gln Leu Asn Gly Cys Gln Ser Lys Asn Ala Pro Asp Pro Ser
    210                 215                 220

Pro Asn Ser Arg Gln Arg Val Lys Thr Val Tyr Asp Gly Cys Arg Tyr
225                 230                 235                 240

Pro Thr Gln Trp Thr Ser Phe Asp Gly Asp His Val Ala Leu Pro Gly
                245                 250                 255

Asp Ala Gly Asn Asp Val Gly Ala Gln Ser Phe Thr Pro Lys Glu Val
            260                 265                 270

Trp Ala Phe Phe Ser Gln Phe Thr
        275                 280
```

<210> 159
<211> 999
<212> DNA
<213> Unknown

<220>
<223> Obtained from environmental sample

<400> 159
atggcgccac tcaccaggcg gggtttcgtc aaggcgtcgg gcgcgcttgc cgcggccccg
60

gcgctcgcgg cgctcgcggc gcacgcgcag gggcccgcgc cagacacggg gaatctgcgt
120

gtcgagcggg acatcgtgtt cggcaagggc ggagacatgg atctcgcgct cgacgtctat
180

caaccgcccc agggcgtgac gccgaagcgc atggccatca ttcatctgtt cgggggcggc
240

ttcttcgtcg gcaacaagaa cgccggctac atcgtgaacg atgcccgggc gctcggtgcc
300

agagggtaca cgagcatctc ggcgaactac cgcctgcaga gcgaggggtc gtggccggcc
360

cagatccacg acacgaaggc cgcgatccgc tggacccgtg cgaatgcggg ccgcctcggg
420

atcgagaccg accggattgc ggtggccggt tactcggccg gcggcatgct ggcgctgatg
480

gccgccggaa cgaacggccg gcgcgaattc gagggcgacg gcggcaacgc cggcgtgagc
540

tcggatgtca acgcctgcat cggcgtctat ccgctggcga gcgcgcagac cgcgacagga
600

ctctttccac aggggcaggc gacacccgac aacatcgcgg ccgcgtcccc gacgacctac
660

atcagtgagc gcttcgcgcc gacgatcttc attcacggca cgaacgacgg cacagtcccg
720

gcacagtcga gcatcgactt ccacacgaag ctgcacgcgc tcggcgtgcc gtcggcgctg
780

acgctgatcc agggcgccga tcacgccttc gacaacagcg ccctggacgc gatcgagctc
840

atggcgcagt ccatcgatct cttcttcgat cggctgatcg tcaatccgac gccctatccc
900

tcattcggcg ctggcggcgg gggccgcggg ggtcgtggtg gcggccgggg cgccgcaccg
960

gggggccgtg gcgacgcacc cgggggccgc ggtcagtag
999

<210> 160
<211> 332
<212> PRT
<213> Unknown

<220>
<223> Obtained from environmental sample

<220>
<221> SIGNAL
<222> (1)...(29)

<220>
<221> DOMAIN
<222> (50)...(264)
<223> X-Pro dipeptidyl-peptidase (S15 family)

<220>
<221> DOMAIN
<222> (73)...(272)
<223> alpha/beta hydrolase fold

<220>
<221> DOMAIN
<222> (88)...(295)
<223> Prolyl oligopeptidase family

<400> 160
Met Ala Pro Leu Thr Arg Arg Gly Phe Val Lys Ala Ser Gly Ala Leu
1 5 10 15

```
Ala Ala Ala Pro Ala Leu Ala Ala Leu Ala Ala His Ala Gln Gly Pro
            20                  25                  30

Ala Pro Asp Thr Gly Asn Leu Arg Val Glu Arg Asp Ile Val Phe Gly
        35                  40                  45

Lys Gly Gly Asp Met Asp Leu Ala Leu Asp Val Tyr Gln Pro Pro Gln
    50                  55                  60

Gly Val Thr Pro Lys Arg Met Ala Ile Ile His Leu Phe Gly Gly Gly
65                  70                  75                  80

Phe Phe Val Gly Asn Lys Asn Ala Gly Tyr Ile Val Asn Asp Ala Arg
                85                  90                  95

Ala Leu Gly Ala Arg Gly Tyr Thr Ser Ile Ser Ala Asn Tyr Arg Leu
            100                 105                 110

Gln Ser Glu Gly Ser Trp Pro Ala Gln Ile His Asp Thr Lys Ala Ala
        115                 120                 125

Ile Arg Trp Thr Arg Ala Asn Ala Gly Arg Leu Gly Ile Glu Thr Asp
    130                 135                 140

Arg Ile Ala Val Ala Gly Tyr Ser Ala Gly Gly Met Leu Ala Leu Met
145                 150                 155                 160

Ala Ala Gly Thr Asn Gly Arg Arg Glu Phe Glu Gly Asp Gly Gly Asn
                165                 170                 175

Ala Gly Val Ser Ser Asp Val Asn Ala Cys Ile Gly Val Tyr Pro Leu
            180                 185                 190

Ala Ser Ala Gln Thr Ala Thr Gly Leu Phe Pro Gln Gly Gln Ala Thr
        195                 200                 205

Pro Asp Asn Ile Ala Ala Ala Ser Pro Thr Thr Tyr Ile Ser Glu Arg
    210                 215                 220

Phe Ala Pro Thr Ile Phe Ile His Gly Thr Asn Asp Gly Thr Val Pro
225                 230                 235                 240

Ala Gln Ser Ser Ile Asp Phe His Thr Lys Leu His Ala Leu Gly Val
                245                 250                 255

Pro Ser Ala Leu Thr Leu Ile Gln Gly Ala Asp His Ala Phe Asp Asn
            260                 265                 270
```

Ser Ala Leu Asp Ala Ile Glu Leu Met Ala Gln Ser Ile Asp Leu Phe
275 280 285

Phe Asp Arg Leu Ile Val Asn Pro Thr Pro Tyr Pro Ser Phe Gly Ala
290 295 300

Gly Gly Gly Gly Arg Gly Gly Arg Gly Gly Gly Arg Gly Ala Ala Pro
305 310 315 320

Gly Gly Arg Gly Asp Ala Pro Gly Gly Arg Gly Gln
325 330

<210> 161
<211> 747
<212> DNA
<213> Unknown

<220>
<223> Obtained from environmental sample

<400> 161
gtgctcggat tctcgcatct cggcatcctc caggccctcc atgaggcggg aatccgcgtg
60

caccgggtgt ccggcaccag cgcgggcgct attgtcgccg cgctccacgc cttcggggtc
120

ggagcggagc ggatgcggga gctcctcgtg ccgctcacgt ggcgcaaggt ctcgaacttc
180

tccgcctcct ccctcgccct gctctccaac gaggccatcg ccgacctgct gagcgccgag
240

ctcggacccg tccgtatcga ggatgcccga atcccgctgg ccatcgtcgc ggccgacatc
300

cacaccggcg agaccgtcgt gctccgacag ggcccccctcg ccgatgccgt gcgcgccagc
360

gccgccatcc cgggcatcta cgcgcccgta tccctggacg gccggctcct cgtcgatggc
420

ggcatcgtcg ccaacgttcc cgtccgggcc cttcgcgcac tggacgccga tgtcatcgtc
480

gcggccaccc tgggaaaggc gatcgccttc cagaccgtgc gcaccctggt gggcgtcctg
540

agcaatgcct tcctgatcgc cgtcgatacg gccacgcgat tcgacctgat ggagggcgta
600

gacgtcatca tcgagcccga cctccaggtg tacaaccccct gggacttcgg gcagcgcgac
660

gagctcatcg tgaaaggaca cgccgcgggg gaggccgcgg tcccgcggat caggcaagcg
720

ctggcccggt tcagcgccga cggctga
747

<210> 162
<211> 248

<212> PRT
<213> Unknown

<220>
<223> Obtained from environmental sample

<220>
<221> DOMAIN
<222> (1)...(152)
<223> Patatin-like phospholipase

<220>
<221> SITE
<222> (59)...(62)
<223> N-glycosylation site. Prosite id = PS00001

<400> 162
```
Met Leu Gly Phe Ser His Leu Gly Ile Leu Gln Ala Leu His Glu Ala
1               5                   10                  15

Gly Ile Arg Val His Arg Val Ser Gly Thr Ser Ala Gly Ala Ile Val
            20                  25                  30

Ala Ala Leu His Ala Phe Gly Val Gly Ala Glu Arg Met Arg Glu Leu
        35                  40                  45

Leu Val Pro Leu Thr Trp Arg Lys Val Ser Asn Phe Ser Ala Ser Ser
    50                  55                  60

Leu Ala Leu Leu Ser Asn Glu Ala Ile Ala Asp Leu Leu Ser Ala Glu
65                  70                  75                  80

Leu Gly Pro Val Arg Ile Glu Asp Ala Arg Ile Pro Leu Ala Ile Val
                85                  90                  95

Ala Ala Asp Ile His Thr Gly Glu Thr Val Val Leu Arg Gln Gly Pro
            100                 105                 110

Leu Ala Asp Ala Val Arg Ala Ser Ala Ala Ile Pro Gly Ile Tyr Ala
        115                 120                 125

Pro Val Ser Leu Asp Gly Arg Leu Leu Val Asp Gly Gly Ile Val Ala
    130                 135                 140

Asn Val Pro Val Arg Ala Leu Arg Ala Leu Asp Ala Asp Val Ile Val
145                 150                 155                 160

Ala Ala Thr Leu Gly Lys Ala Ile Ala Phe Gln Thr Val Arg Thr Leu
                165                 170                 175

Val Gly Val Leu Ser Asn Ala Phe Leu Ile Ala Val Asp Thr Ala Thr
            180                 185                 190

Arg Phe Asp Leu Met Glu Gly Val Asp Val Ile Ile Glu Pro Asp Leu
```

195 200 205

Gln Val Tyr Asn Pro Trp Asp Phe Gly Gln Arg Asp Glu Leu Ile Val
210 215 220

Lys Gly His Ala Ala Gly Glu Ala Ala Val Pro Arg Ile Arg Gln Ala
225 230 235 240

Leu Ala Arg Phe Ser Ala Asp Gly
245

<210> 163
<211> 747
<212> DNA
<213> Unknown

<220>
<223> Obtained from an environmental sample

<400> 163
atgaaaatta agttgccaga accattcaca ttcgaagcag gaaatcgagc agttttatta
60

ttacatggat ttactggcca ttcagcagat gtaagaatgc ttggtcgatt tctagaaaag
120

aaaggttata caactcatgc accaatttat agagggcatg gtcaagaacc agaagcatta
180

ttaaaatctt cacctgatga atggtgggag gatattttat cagcttataa tcatttgaaa
240

aatctaggat ataatgaaat tgctgttgct ggtctttcaa tgggtggtgc tttagcaatt
300

aaactagcta ctaagcatga aataaaaggt gtcattccaa tgtgtacacc aatgtacttt
360

gataatcaaa aacaattaac tcaagcattt ttaaattttg cacgacaatt taaaaaattt
420

gagaaaaaag atgaagaaac aattaaaaaa gaaatagatt tactacaaca aaattcagca
480

gaattattta atgagatcgg cacttttgta gaacaagtca atggtataat agatagagtt
540

gacgtaccta cattcgtagt tcaagcaagt aaagatgaaa taatcaatcc tgaaagtgcg
600

acatttattt atgaaaacat taaaaatgaa aaaaaagatt taaaatggta taaaaattca
660

actcacttga taacatttgg cgatgaaaaa gatgtcttac atgaagatat atatcatttt
720

ttagaaacat tagattggaa caactaa
747

<210> 164
<211> 248

<212> PRT
<213> Unknown

<220>
<223> Obtained from an environmental sample

<220>
<221> DOMAIN
<222> (16)...(245)
<223> hydrolase alpha/beta fold epoxide

<400> 164

```
Met Lys Ile Lys Leu Pro Glu Pro Phe Thr Phe Glu Ala Gly Asn Arg
1               5                   10                  15

Ala Val Leu Leu Leu His Gly Phe Thr Gly His Ser Ala Asp Val Arg
            20                  25                  30

Met Leu Gly Arg Phe Leu Glu Lys Lys Gly Tyr Thr Thr His Ala Pro
        35                  40                  45

Ile Tyr Arg Gly His Gly Gln Glu Pro Glu Ala Leu Leu Lys Ser Ser
    50                  55                  60

Pro Asp Glu Trp Trp Glu Asp Ile Leu Ser Ala Tyr Asn His Leu Lys
65                  70                  75                  80

Asn Leu Gly Tyr Asn Glu Ile Ala Val Ala Gly Leu Ser Met Gly Gly
                85                  90                  95

Ala Leu Ala Ile Lys Leu Ala Thr Lys His Glu Ile Lys Gly Val Ile
            100                 105                 110

Pro Met Cys Thr Pro Met Tyr Phe Asp Asn Gln Lys Gln Leu Thr Gln
        115                 120                 125

Ala Phe Leu Asn Phe Ala Arg Gln Phe Lys Lys Phe Glu Lys Lys Asp
    130                 135                 140

Glu Glu Thr Ile Lys Lys Glu Ile Asp Leu Leu Gln Gln Asn Ser Ala
145                 150                 155                 160

Glu Leu Phe Asn Glu Ile Gly Thr Phe Val Glu Gln Val Asn Gly Ile
                165                 170                 175

Ile Asp Arg Val Asp Val Pro Thr Phe Val Val Gln Ala Ser Lys Asp
            180                 185                 190

Glu Ile Ile Asn Pro Glu Ser Ala Thr Phe Ile Tyr Glu Asn Ile Lys
        195                 200                 205

Asn Glu Lys Lys Asp Leu Lys Trp Tyr Lys Asn Ser Thr His Leu Ile
```

210 215 220

Thr Phe Gly Asp Glu Lys Asp Val Leu His Glu Asp Ile Tyr His Phe
225 230 235 240

Leu Glu Thr Leu Asp Trp Asn Asn
245

<210> 165
<211> 1911
<212> DNA
<213> Unknown

<220>
<223> Obtained from an environmental sample

<400> 165
atgatcaaac agtcgttgtt tgtaccgctc gcaggatgct tgctcgcgat cgcatgcgcc
60

caggcgaacg ccgcacccaa tccttataca aatttcgttg tcttcgggga cagtctcaac
120

gatgcaggga cctttgccga cacgggcggg cctgccggag cgacacagcg cttcaccaac
180

cggacgggac cggtgtacct ggatggcagc ggcgaagtcc gctcgctcaa ctccacacag
240

atcctgggtg ggaaactggg gttttcaccg gaccagacgg catcttctac ttcagcagtt
300

cgcgccaacg aaggcctgcc tgatggtaac aactgggctg taggcggcta ccgtaccgac
360

cagattcttg attcgattac cagcatctcc gccaccggcg aacgcacccg gtccggctat
420

cttccatcca ataacttccg tgccgacccg aacgcactgt attacctgtc gggcggcggt
480

aacgacttcc tgcaaggacg cgtcaccagc ctgccccaag ccagtgccgc cgccgaccgc
540

ctggccgaca gcgtgcagac cctgcaaacc gctggcgcca gatacgtcat ggtctggttg
600

ctgcccgata tcggctttac cccggcgttc aacggcacgc cgctgcaagc gttcacatct
660

caactcagcg cccagttcaa cactgaactg gtaagccgac tgcaaaacat caatgccgaa
720

gtcatcccgc tcaacattcc ggtactgctc aaagagacgt tcgccaaccc ggcacagttt
780

ggcctggcca ccgaccagaa cctcatcacc acctgcttca gcggcaacgg ttgtaccgag
840

aatgcccgct acggcatcaa cagcgccacg ccggacccga ccaagctgat ctacaacgac
900

tcggtgcacc ccaccgaagc cgggcaacgc ctgatcgccg attacgccta ttccctgctg

960

gccgcaccat gggagctgac cctgctgccg gaaatggccc agggcaccgt gcgtgcgcat
1020

caggatgagt tgcgcaacca gtggctggcc gattgggaaa actggcaagg cgtcggccaa
1080

tggcgagcca tcgtttccgc cggcggacag catcaggact ttgacggcca ggacagcgtt
1140

gcaagcgccg acggcaacgg agccaacctg aacatcggtg gcagctatcg tctcaacgat
1200

gcctggcgtg ttggtctggt ggccggtttc tataaccagg aactcgaagc cggcaacaac
1260

gactccgact acaagctcaa cacttacctg ggcacggcat tcgcccagta ccagcaaaac
1320

cgtgtgtggg ccgacgcggc gctgacggcc gggcatctgg attacgacag cctcaagcgc
1380

aaattccagc tgggcgtcaa cgaacgcggc gaaaaaggcg ataccagcgg ctacgtcgaa
1440

gccttcagcg cacgcgtggg ctacgacatt gcgcaacaag ccagcagtcc ttggcacctg
1500

tcgccgtttg tcagcgccga cttcgccaag gtacaagtcg atggttactc ggaggacgga
1560

aacaattcca cggcgctgac cttcgccgat caggagcgta tttcccggcg cctgggcgct
1620

ggcctgcagg gcaaatacca gatcaccccg caaacccagg tgttcggcga agcggcaatc
1680

gagcgtgagt acaacgacga tacccaggac gtggacatca acctcaacag cctgccaaac
1740

aaccagttca cgctggaagg ctacaccccg caaagtcact tgcaacatgt gaacctgggg
1800

gtgagccaca acctcaccaa ggacctggca ctgcgcgcca gctacaacat ccgcaaggac
1860

gacgacttca cccaacaggg catcaatgta ggtgttgcgc tggacttctg a
1911

<210> 166
<211> 636
<212> PRT
<213> Unknown

<220>
<223> Obtained from an environmental sample

<220>
<221> SIGNAL
<222> (1)...(24)

<220>
<221> DOMAIN
<222> (32)...(316)

<223> GDSL-like Lipase/Acylhydrolase

<220>
<221> DOMAIN
<222> (357)...(627)
<223> Autotransporter beta-domain

<220>
<221> SITE
<222> (32)...(43)
<223> Lipolytic enzymes "G-D-S-L" family, serine active site. Prosite id = PS01098

<220>
<221> SITE
<222> (60)...(63)
<223> N-glycosylation site. Prosite id = PS00001

<220>
<221> SITE
<222> (78)...(81)
<223> N-glycosylation site. Prosite id = PS00001

<220>
<221> SITE
<222> (303)...(306)
<223> N-glycosylation site. Prosite id = PS00001

<220>
<221> SITE
<222> (426)...(429)
<223> N-glycosylation site. Prosite id = PS00001

<220>
<221> SITE
<222> (529)...(532)
<223> N-glycosylation site. Prosite id = PS00001

<220>
<221> SITE
<222> (613)...(616)
<223> N-glycosylation site. Prosite id = PS00001

<400> 166

```
Met Ile Lys Gln Ser Leu Phe Val Pro Leu Ala Gly Cys Leu Leu Ala
1               5                   10                  15

Ile Ala Cys Ala Gln Ala Asn Ala Ala Pro Asn Pro Tyr Thr Asn Phe
            20                  25                  30

Val Val Phe Gly Asp Ser Leu Asn Asp Ala Gly Thr Phe Ala Asp Thr
        35                  40                  45

Gly Gly Pro Ala Gly Ala Thr Gln Arg Phe Thr Asn Arg Thr Gly Pro
    50                  55                  60

Val Tyr Leu Asp Gly Ser Gly Glu Val Arg Ser Leu Asn Ser Thr Gln
65                  70                  75                  80

Ile Leu Gly Gly Lys Leu Gly Phe Ser Pro Asp Gln Thr Ala Ser Ser
```

85 90 95

Thr Ser Ala Val Arg Ala Asn Glu Gly Leu Pro Asp Gly Asn Asn Trp
100 105 110

Ala Val Gly Gly Tyr Arg Thr Asp Gln Ile Leu Asp Ser Ile Thr Ser
115 120 125

Ile Ser Ala Thr Gly Glu Arg Thr Arg Ser Gly Tyr Leu Pro Ser Asn
130 135 140

Asn Phe Arg Ala Asp Pro Asn Ala Leu Tyr Tyr Leu Ser Gly Gly Gly
145 150 155 160

Asn Asp Phe Leu Gln Gly Arg Val Thr Ser Leu Pro Gln Ala Ser Ala
165 170 175

Ala Ala Asp Arg Leu Ala Asp Ser Val Gln Thr Leu Gln Thr Ala Gly
180 185 190

Ala Arg Tyr Val Met Val Trp Leu Leu Pro Asp Ile Gly Phe Thr Pro
195 200 205

Ala Phe Asn Gly Thr Pro Leu Gln Ala Phe Thr Ser Gln Leu Ser Ala
210 215 220

Gln Phe Asn Thr Glu Leu Val Ser Arg Leu Gln Asn Ile Asn Ala Glu
225 230 235 240

Val Ile Pro Leu Asn Ile Pro Val Leu Leu Lys Glu Thr Phe Ala Asn
245 250 255

Pro Ala Gln Phe Gly Leu Ala Thr Asp Gln Asn Leu Ile Thr Thr Cys
260 265 270

Phe Ser Gly Asn Gly Cys Thr Glu Asn Ala Arg Tyr Gly Ile Asn Ser
275 280 285

Ala Thr Pro Asp Pro Thr Lys Leu Ile Tyr Asn Asp Ser Val His Pro
290 295 300

Thr Glu Ala Gly Gln Arg Leu Ile Ala Asp Tyr Ala Tyr Ser Leu Leu
305 310 315 320

Ala Ala Pro Trp Glu Leu Thr Leu Leu Pro Glu Met Ala Gln Gly Thr
325 330 335

Val Arg Ala His Gln Asp Glu Leu Arg Asn Gln Trp Leu Ala Asp Trp
340 345 350

```
Glu Asn Trp Gln Gly Val Gly Gln Trp Arg Ala Ile Val Ser Ala Gly
        355                 360                 365

Gly Gln His Gln Asp Phe Asp Gly Gln Asp Ser Val Ala Ser Ala Asp
    370                 375                 380

Gly Asn Gly Ala Asn Leu Asn Ile Gly Gly Ser Tyr Arg Leu Asn Asp
385                 390                 395                 400

Ala Trp Arg Val Gly Leu Val Ala Gly Phe Tyr Asn Gln Glu Leu Glu
                405                 410                 415

Ala Gly Asn Asn Asp Ser Asp Tyr Lys Leu Asn Thr Tyr Leu Gly Thr
            420                 425                 430

Ala Phe Ala Gln Tyr Gln Gln Asn Arg Val Trp Ala Asp Ala Ala Leu
        435                 440                 445

Thr Ala Gly His Leu Asp Tyr Asp Ser Leu Lys Arg Lys Phe Gln Leu
    450                 455                 460

Gly Val Asn Glu Arg Gly Glu Lys Gly Asp Thr Ser Gly Tyr Val Glu
465                 470                 475                 480

Ala Phe Ser Ala Arg Val Gly Tyr Asp Ile Ala Gln Gln Ala Ser Ser
                485                 490                 495

Pro Trp His Leu Ser Pro Phe Val Ser Ala Asp Phe Ala Lys Val Gln
            500                 505                 510

Val Asp Gly Tyr Ser Glu Asp Gly Asn Asn Ser Thr Ala Leu Thr Phe
        515                 520                 525

Ala Asp Gln Glu Arg Ile Ser Arg Arg Leu Gly Ala Gly Leu Gln Gly
    530                 535                 540

Lys Tyr Gln Ile Thr Pro Gln Thr Gln Val Phe Gly Glu Ala Ala Ile
545                 550                 555                 560

Glu Arg Glu Tyr Asn Asp Asp Thr Gln Asp Val Asp Ile Asn Leu Asn
                565                 570                 575

Ser Leu Pro Asn Asn Gln Phe Thr Leu Glu Gly Tyr Thr Pro Gln Ser
            580                 585                 590

His Leu Gln His Val Asn Leu Gly Val Ser His Asn Leu Thr Lys Asp
        595                 600                 605

Leu Ala Leu Arg Ala Ser Tyr Asn Ile Arg Lys Asp Asp Asp Phe Thr
```

610 615 620

Gln Gln Gly Ile Asn Val Gly Val Ala Leu Asp Phe
625 630 635